(19) Europäisches Patentamt
European Patent Office
Office européen des brevets

(11) EP 1 771 548 B1

(12) # FASCICULE DE BREVET EUROPEEN

(45) Date de publication et mention de la délivrance du brevet:
**27.06.2012 Bulletin 2012/26**

(21) Numéro de dépôt: **05793666.8**

(22) Date de dépôt: **29.07.2005**

(51) Int Cl.:
***A61K 31/80*** *(2006.01)* ***C12N 5/00*** *(2006.01)*
***C07F 9/6521*** *(2006.01)* ***A61K 35/14*** *(2006.01)*
***A61K 31/675*** *(2006.01)* ***A61K 31/662*** *(2006.01)*
***A61K 36/26*** *(2006.01)* ***C07F 9/6581*** *(2006.01)*
***A61K 35/26*** *(2006.01)*

(86) Numéro de dépôt international:
**PCT/FR2005/002003**

(87) Numéro de publication internationale:
**WO 2006/024769 (09.03.2006 Gazette 2006/10)**

(54) **UTILISATION DE DENDRIMERES POUR STIMULER LA CROISSANCE CELLULAIRE**
VERWENDUNG VON DENDRIMEREN ZUR STIMULIERUNG DES ZELLWACHSTUMS
USE OF DENDRIMERS TO STIMULATE CELL GROWTH

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **30.07.2004 FR 0408426**

(43) Date de publication de la demande:
**11.04.2007 Bulletin 2007/15**

(73) Titulaires:
- **CENTRE NATIONAL DE LA RECHERCHE SCIENTIFIQUE (CNRS) 75794 Paris Cedex 16 (FR)**
- **INSTITUT NATIONAL DE LA SANTE ET DE LA RECHERCHE MEDICALE (INSERM) 75654 Paris Cédex 13 (FR)**
- **Rhodia UK Limited Watford, Hertfordshire WD24 4QP (GB)**

(72) Inventeurs:
- **MAJORAL, Jean-Pierre F-31520 Ramonville Saint Agne (FR)**
- **CAMINADE, Anne-Marie, Jeannine, Jacqueline F-31400 Toulouse (FR)**
- **FOURNIE, Jean-Jacques F-31450 Corronsac (FR)**
- **GRIFFE, Laurent F-11000 Carcassonne (FR)**
- **POUPOT-MARSAN, Mary F-31280 Aigrefeuille (FR)**
- **POUPOT, Rémy F-31280 Aigrefeuille (FR)**
- **TURRIN, Cédric-Olivier F-31400 Toulouse (FR)**

(74) Mandataire: **Grosset-Fournier, Chantal Catherine Grosset-Fournier & Demachy 54, rue Saint-Lazare 75009 Paris (FR)**

(56) Documents cités:
**WO-A-01/38335 WO-A-01/91816**
**WO-A-02/067908 WO-A-03/091304**

- **PREVOTE D: "Phosphate-, phosphite-, ylide-, and phosphonate-terminated dendrimers" JOURNAL OF ORGANIC CHEMISTRY, AMERICAN CHEMICAL SOCIETY. EASTON, US, vol. 62, no. 14, 11 juillet 1997 (1997-07-11), pages 4834-4841, XP002144559 ISSN: 0022-3263 cité dans la demande**
- **MURAKI, TAKAHITO ET AL: "Enhancement of thermal stability of polystyrene and poly(methyl methacrylate) by cyclotriphosphazene derivatives" POLYMER DEGRADATION AND STABILITY , 84(1), 87-93 CODEN: PDSTDW; ISSN: 0141-3910, avril 2004 (2004-04), XP002320475**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 771 548 B1

## Description

La présente invention concerne l'utilisation de dendrimères pour stimuler la croissance cellulaire.

Les dendrimères sont des macromolécules constituées de monomères qui s'associent selon un processus arborescent autour d'un coeur central plurifonctionnel.

Les dendrimères, appelées aussi « molécules cascade », sont des polymères fonctionnels hautement ramifiés de structure définie. Ces macromolécules sont effectivement des polymères puisqu'elles sont basées sur l'association d'unités répétitives. Cependant, les dendrimères diffèrent fondamentalement des polymères classiques dans la mesure où ils ont des propriétés propres dues à leur construction en arborescence. Le poids moléculaire et la forme des dendrimères peuvent être précisément contrôlés et toutes les fonctions sont situées à la terminaison des arborescences, formant une surface, ce qui les rend facilement accessibles.

Les dendrimères sont construits étape par étape, par la répétition d'une séquence de réactions permettant la multiplication de chaque unité répétitive et des fonctions terminales. Chaque séquence de réactions forme ce qui est appelé une « nouvelle génération ». La construction arborescente s'effectue par la répétition d'une séquence de réactions qui permet l'obtention à la fin de chaque cycle réactionnel d'une nouvelle génération et d'un nombre croissant de branches identiques. Après quelques générations, le dendrimère prend généralement une forme globulaire hautement ramifiée et plurifonctionnalisée grâce aux nombreuses fonctions terminales présentes en périphérie.

De tels polymères ont notamment été décrits par Launay et al., Angew. Chem. Int. Ed. Engl., 1994, 33, 15/16, 1589-1592, ou encore Launay et al., Journal of Organometallic Chemistry, 1997, 529, 51-58.

A l'heure actuelle, la prolifération de cellules, en particulier de cellules cytotoxiques, notamment du type NK, est essentiellement induite, *in vitro* ou *in vivo,* par des cytokines, telles que IL-2, IL-7, IL-15, IL-18, IL-21 ou IFN$\alpha/\beta$, comme cela est notamment décrit par Vivier et al., Immunologie des Cancers (2003) eds Médecine-Sciences Flammarion. Ces cytokines sont en général utilisées sous la forme de protéines recombinantes. Toutefois, plusieurs obstacles limitent leur utilisation. Ces composés ont tout d'abord un coût de production, par génie génétique, relativement élevé. Par ailleurs, leur utilisation *in vivo,* notamment en thérapie chez l'homme, se heurte à une toxicité élevée, liée à leur action pléïotrope.

Des composés, d'origines végétale ou bactérienne par exemple, pourraient éventuellement être recherchés pour stimuler la croissance des cellules de la lignée lymphoïde. Toutefois, les difficultés d'obtention de molécules naturelles pures en quantités importantes et les problèmes liés à l'utilisation de substances naturelles limiteraient leur utilisation en thérapeutique humaine.

Il est fait aussi réference aux documents suivants: WO 02/067 508, WO 01/91816, WO 01/38335, WO 03/0913304; PREVOTE, "J. Org. Chem. " 1997, 62, 14, papes 4834 - 4841 et Muraki et al., "Polymer Degradation and Stability", 2004, 84(1), popes 87-93.

Ainsi un des buts de la présente invention est de proposer un nouveau procédé de stimulation de la croissance cellulaire à l'aide de composés synthétiques de faibles coûts de production et essentiellement non toxiques.

Un autre but de l'invention est de proposer de nouveaux composés synthétiques, utilisables pour la stimulation de la croissance cellulaire.

Un autre but de l'invention est également de proposer de nouvelles compositions pharmaceutiques contenant les cellules dont la croissance cellulaire a été stimulée par des composés synthétiques de faibles coûts de production et essentiellement non toxiques.

Ainsi, la présente invention concerne l'utilisation de dendrimères à terminaisons monophosphoniques ou bisphosphoniques pour stimuler la croissance de cultures cellulaires ou activer des cellules en culture.

Les dendrimères utilisés selon l'invention sont constitués d'un noyau §, sur lequel viennent se fixer en arborescence des chaînes de liaison, lesdites chaînes de liaison étant elles-mêmes constituées de chaînes de génération, une chaîne intermédiaire étant éventuellement fixée à l'extrémité de chaque chaîne de la génération la plus éloignée du noyau central, des groupements monophosphoniques ou bisphosphoniques étant présents aux extrémités non liées au noyau des chaînes de liaison. A fin de clarté, ces éléments sont schématisés dans la **Figure 9.**

Selon l'invention, les dendrimères à terminaisons monophosphoniques, ou dendrimères monophosphoniques, présentent une fonction terminale $-PO(OX)_2$, et les dendrimères à terminaisons bisphosphoniques, ou dendrimères bisphosphoniques, présentent deux fonctions terminales $-PO(OX)_2$, à la terminaison de chaque arborescence, où X représente -H ou un radical -Alkyle, -Aryle, -Alkaryle ou -Aralkyle, ou les sels correspondants, lesdits sels étant formés de l'association de terminaisons monophosphoniques ou bisphosphoniques et de cations. Pour un groupement phosphonique donné les groupements X peuvent être identiques ou différents.

• Les sels des dendrimères peuvent être préparés *in situ* pendant l'isolement final et la purification des composés. Les sels peuvent être préparés en faisant réagir séparément le composé purifié sous sa forme acide avec une base organique ou inorganique et en isolant le sel ainsi formé. Les sels comprennent les sels aminés et métalliques. Les sels métalliques adaptés comprennent les sels de sodium, potassium, cuivre, calcium, baryum, zinc, magnésium et aluminium. Les sels de sodium et de potassium sont préférés. Les sels inorganiques de base adaptés sont préparés à partir de

bases métalliques qui comprennent hydrure de sodium, hydroxyde de sodium, hydroxyde de potassium, hydroxyde de calcium, hydroxyde d'aluminium, hydroxyde de lithium, hydroxyde de magnésium, hydroxyde de zinc.

Avantageusement, selon un mode de réalisation particulier, les sels des dendrimères monophosphoniques ou bisphosphoniques utilisés dans la présente invention sont pharmaceutiquement acceptables.

Selon l'invention, l'expression « stimuler la croissance de culture cellulaire » signifie que l'ajout des dendrimères mentionnés ci-dessus à une culture cellulaire permet d'obtenir une plus grande quantité de cellules au terme de la culture, que lorsque cette culture est réalisée dans les mêmes conditions en l'absence desdits dendrimères.

Avantageusement, les dendrimères à terminaisons monophosphoniques ou bisphosphoniques peuvent également provoquer, directement ou indirectement, une différenciation cellulaire et conduire à l'obtention de nouvelles cellules, lesdites nouvelles cellules pouvant notamment être caractérisées par une combinaison particulière de marqueurs membranaires et/ou par une combinaison particulière de niveau d'expression de marqueurs membranaires. On nomme ce phénomène « activation des cellules en culture ».

Dans un mode de réalisation particulier de l'utilisation définie ci-dessus, les cellules en question sont des cellules hématopoïétiques, c'est-à-dire des cellules provenant de la différenciation de cellules souches hématopoïétiques.

La présente invention concerne plus particulièrement l'utilisation, telle que définie ci-dessus, de dendrimères à terminaisons monophosphoniques ou bisphosphoniques pour la préparation de compositions cellulaires enrichies en cellules de la lignée lymphoïde exprimant le récepteur NKG2D, à partir d'échantillons biologiques.

Les compositions cellulaires enrichies selon l'invention sont caractérisées en ce qu'elles contiennent une proportion plus importante de cellules de la lignée lymphoïde exprimant le récepteur NKG2D par rapport à la proportion de ces mêmes cellules dans l'échantillon biologique dont elles sont issues.

Toutes les cellules de la lignées lymphoïde n'expriment pas ce récepteur, on le retrouve plus particulièrement à la surface des cellules impliquées dans l'immunité innée et notamment à la surface de cellules de type NK (natural killer), de type lymphocytes T $\gamma\delta$, de type lymphocytes T $\alpha\beta$ CD8 (S. Bauer et al., Science, 1999, 285, 727-729 ; J. Wu, Science, 1999, 285, 730-732).

Selon l'invention, on entend par « échantillon biologique » tout prélèvement de tissu sur un être vivant, en particulier le sang total et les fractionnements cellulaires du sang total, tels que les cellules mononucléées du sang périphérique (PBMC, *peripheral blood mononuclear cells),* sont considérés comme des échantillons biologiques.

Les principaux types cellulaires retrouvés dans les PBMC sont les lymphocytes T et B, les cellules NK, et les cellules de type monocytaire.

Selon un mode de réalisation particulier de l'invention, les dendrimères à terminaisons monophosphoniques ou bisphosphoniques sont de génération n et comprennent un noyau central, ou coeur, § de valence m pouvant établir m-2 liaisons, sous réserve que m soit supérieur à 2, ou m-1 liaisons, sous réserve que m soit supérieur à 1, ou m liaisons avec des chaînes de liaison, de préference identiques entre elles, lesquelles chaînes de liaison sont constituées par :

- des chaînes de génération fixées en arborescence autour du noyau sur chacune des liaisons, au moins une chaîne intermédiaire étant éventuellement fixée à l'extrémité de chaque chaîne de la génération la plus éloignée du noyau central, et un groupe terminal étant fixé à l'extrémité de chaque chaîne de la génération la plus éloignée du noyau central ou le cas échéant à l'extrémité de chaque chaîne intermédiaire, ou
- des chaînes intermédiaires fixées autour du noyau sur chacune des liaisons, un groupe terminal étant fixé à l'extrémité de chaque chaîne intermédiaire ;

ledit groupe terminal étant représenté par la formule :

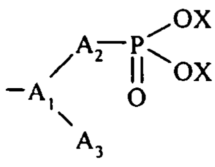

où

$A_1$ représente N ; un groupe P=Y, où Y représente O, S, ou aucun atome ; un groupe N-R; ou un groupe C-R; R représentant H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR'R", un groupement alkoxy de 1 à 16 atomes de carbone, un groupement alkyle de 1 à 16 atomes de carbone, un atome d'halogène, un

groupement $-NO_2$, un groupement -CN, un groupement $-CF_3$, un groupement aryle de 6 à 24 atomes de carbone, un groupement aralkyle de 7 à 16 atomes de carbone, R' et R" représentant indépendamment l'un de l'autre H ou un groupement alkyle de 1 à 16 atomes de carbone, aryle de 6 à 24 atomes de carbone ou aralkyle de 7 à 16 atomes de carbone ;

$A_2$ représente une liaison simple ou une chaîne hydrocarbonée de 1 à 6 chaînons, linéaire, ramifiée ou cyclique, chacun desdits chaînons contenant éventuellement un ou plusieurs hétéroatomes, lesdits hétéroatomes étant préférentiellement choisis parmi un atome de soufre, d'oxygène, de phosphore, ou d'azote, plus préférentiellement d'azote, et étant éventuellement substitués par au moins un substituant choisi parmi H, un groupement alkyle de 1 à 16 atomes de carbone, un halogène, un groupement $-NO_2$, un groupement -NRR', un groupement -CN, un groupement $-CF_3$, un groupement hydroxyle, un groupement alkoxy de 1 à 16 atomes de carbone, un groupement aryle ou hétéroaryle de 1 à 24 atomes de carbone, l'hétéroélément étant préférentiellement choisi parmi l'oxygène, l'azote ou le soufre, un groupement aralkyle de 7 à 16 atomes de carbone, R et R' représentant indépendamment l'un de l'autre H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone ;

$A_3$ représente H, ou une chaîne hydrocarbonée de 1 à 6 chaînons, linéaire, ramifiée ou cyclique, chacun desdits chaînons pouvant éventuellement être choisi parmi un hétéroatome, ledit hétéroatome étant préférentiellement choisi parmi le soufre, l'azote, le phosphore, ou le silicium, plus préférentiellement l'azote, chaque chaînon pouvant être éventuellement substitué par au moins un groupement choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone, ou aralkyle de 7 à 16 atomes de carbone ou

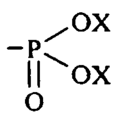 ,

en particulier $A_3$ peut représenter

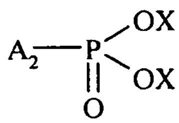 ,

chacun des $A_2$ étant identiques ou différents ;

chaque OX, identique ou différent pour chaque groupement phosphonique, représente OH, OAlkyle, où le groupe alkyle comprend de 1 à 16 atomes de carbone, OAryle, où le groupe aryle comprend de 6 à 24 atomes de carbone, OAralkyle, où le groupe aralkyle comprend de 7 à 24 atome de carbone, OAlkylaryle, où le groupe alkylaryle comprend de 7 à 24 atomes de carbone, $OSiR'_1R'_2R'_3$, où $R'_1$, $R'_2$ et $R'_3$, identiques ou différents, représentent un groupement alkyle de 1 à 16 atomes de carbone, ou $O^-M^+$, où $M^+$ est un cation d'éléments du groupe IA, IB, IIA, IIB ou IIIA, IIIB de la classification périodique des éléments, de préférence $M^+$ est choisi parmi les cations des atomes de sodium, de potassium, de cuivre, de calcium, de baryum, de zinc, de magnésium, de lithium et d'aluminium, ou de groupes hydrocarbonés de 1 à 100 atomes de carbone, ou azotés de 0 à 100 atomes de carbone, tel que $NR_1R_2R_3R_4^+$, où, indépendamment les uns des autres $R_1$, $R_2$, $R_3$ et $R_4$ représentent H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone ;
m représente un nombre entier de 1 à 20, en particulier de 1 à 10 et plus particulièrement de 1 à 8 ;
n représente un nombre entier de 0 à 12 ;
ledit noyau central § représentant un groupe comprenant de 1 à 500 atomes, et contenant éventuellement un ou plusieurs hétéroatomes, lesdits hétéroatomes étant préférentiellement choisis parmi l'oxygène, le soufre, l'azote, le phosphore ou le silicium.

Selon un mode de réalisation particulier de l'invention, les dendrimères à terminaisons monophosphoniques ou bisphosphoniques sont de génération n et comprennent un noyau central § de valence m pouvant établir m-2 liaisons, sous réserve que m soit supérieur à 2, ou m-1 liaisons, sous réserve que m soit supérieur à 1, ou m liaisons avec des chaînes de liaison, de préférence identiques entre elles, m représentant un nombre entier de 1 à 20, en particulier de 1 à 10 et plus particulièrement de 1 à 8, et n représentant un nombre entier de 0 à 12, lesquelles chaînes de liaison sont constituées par :

- des chaînes de génération fixées en arborescence autour du noyau sur chacune des liaisons, lorsque n est supérieur ou égal à 1, une chaine de génération d'une génération donnée étant reliée à

  - une chaîne de génération de la génération immédiatement inférieure à la génération donnée, ou au noyau lorsque la génération donnée vaut 1, et à
  - au moins 2 chaînes de génération de la génération immédiatement supérieure à la génération donnée, ou éventuellement à au moins une chaîne intermédiaire lorsque la génération donnée vaut n,

  un groupe terminal étant fixé à l'extrémité de chaque chaîne de génération de génération n, ou le cas échéant à l'extrémité de chaque chaîne intermédiaire, ou
- des chaînes intermédiaires fixées autour du noyau sur chacune des liaisons, lorsque n vaut 0, un groupe terminal étant fixé à l'extrémité de chaque chaîne intermédiaire ;

ledit groupe terminal étant représenté par la formule :

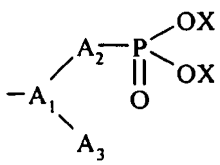

où
$A_1$ représente N ; un groupe P=Y, où Y représente O, S, ou aucun atome ; un groupe N-R ; ou un groupe C-R ; R représentant H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR'R", un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R' et R" représentant indépendamment l'un de l'autre H ou un groupement alkyle de 1 à 16 atomes de carbone, aryle de 6 à 24 atomes de carbone ou aralkyle de 7 à 16 atomes de carbone ;
$A_2$ représente une liaison simple ou une chaîne hydrocarbonée de 1 à 6 chaînons, linéaire, ramifiée ou cyclique, chacun desdits chaînons contenant éventuellement un ou plusieurs hétéroatomes, lesdits hétéroatomes étant préférentiellement choisis parmi un atome de soufre, d'oxygène, de phosphore, ou d'azote, plus préférentiellement d'azote, et étant éventuellement substitués par au moins un substituant choisi parmi H, un groupement alkyle de 1 à 16 atomes de carbone, un halogène, un groupement $-NO_2$, un groupement -NRR', un groupement -CN, un groupement $-CF_3$, un groupement hydroxyle, un groupement alkoxy de 1 à 16 atomes de carbone, un groupement aryle ou hétéroaryle de 1 à 24 atomes de carbone, l'hétéroélément étant préférentiellement choisi parmi l'oxygène, l'azote ou le soufre, un groupement aralkyle de 7 à 16 atomes de carbone, R et R' représentant indépendamment l'un de l'autre H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant

choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone ;

$A_3$ représente H, ou une chaîne hydrocarbonée de 1 à 6 chaînons, linéaire, ramifiée ou cyclique, chacun desdits chaînons pouvant éventuellement être choisi parmi un hétéroatome, ledit hétéroatome étant préférentiellement choisi parmi le soufre, l'azote, le phosphore, ou le silicium, plus préférentiellement l'azote, chaque chaînon pouvant être éventuellement substitué par au moins un groupement choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone, ou aralkyle de 7 à 16 atomes de carbone ou

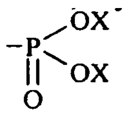

,

en particulier $A_3$ peut représenter

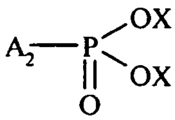

,

chacun des $A_2$ étant identiques ou différents ;

chaque OX, identique ou différent pour chaque groupement phosphonique, représente OH, OAlkyle, où le groupe alkyle comprend de 1 à 16 atomes de carbone, OAryle, où le groupe aryle comprend de 6 à 24 atomes de carbone, OAralkyle, où le groupe aralkyle comprend de 7 à 24 atome de carbone, OAlkylaryle, où le groupe alkylaryle comprend de 7 à 24 atomes de carbone, $OSiR'_1R'_2R'_3$, où $R'_1$, $R'_2$ et $R'_3$, identiques ou différents, représentent un groupement alkyle de 1 à 16 atomes de carbone, ou $O^-M^+$, où $M^+$ est un cation d'éléments du groupe IA, IB, IIA, IIB ou IIIA, IIIB de la classification périodique des éléments, de préférence $M^+$ est choisi parmi les cations des atomes de sodium, de potassium, de cuivre, de calcium, de baryum, de zinc, de magnésium, de lithium et d'aluminium, ou de groupes hydrocarbonés de 1 à 100 atomes de carbone, ou azotés de 0 à 100 atomes de carbone, tel que $NR_1R_2R_3R_4^+$, où, indépendamment les uns des autres $R_1$, $R_2$, $R_3$ et $R_4$ représentent H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone ;

ledit noyau central § représentant un groupe comprenant de 1 à 500 atomes, et contenant éventuellement un ou plusieurs hétéroatomes, lesdits hétéroatomes étant préférentiellement choisis parmi l'oxygène, le soufre, l'azote, le phosphore ou le silicium.

Les chaînes de liaison sont de deux types selon qu'elles comportent ou non des chaînes de génération. Dans une même molécule, toutes les chaînes de liaison sont identiques ou différentes. Les chaînes de génération permettent notamment de multiplier le nombre de fonctions terminales, et de contrôler la taille du dendrimère.

On désigne par « dendrimère de génération n » un dendrimère comprenant n générations de chaînes de génération. Chaque chaîne de génération peut alors être caractérisée par la génération à laquelle elle appartient.

Ainsi, un dendrimère de génération 0 ne comprend aucune chaîne de génération, les chaînes intermédiaires sont directement reliées au noyau.

Un dendrimère de génération 1 ne comprend qu'une génération de chaînes de génération, lesquelles chaînes de génération sont reliées, d'une part, au noyau et, d'autre part, à éventuellement au moins 1 chaîne intermédiaire.

Pour les dendrimères de génération 2, les chaînes de génération de génération 1 sont liées, d'une part, au

noyau et, d'autre part, à au moins 2 chaînes de génération de génération 2, chaque chaîne de génération 2 étant liée à une chaîne de génération 1 et éventuellement à au moins 1 chaîne intermédiaire.

Enfin, d'une manière générale, pour un dendrimère de génération n, n variant de 3 à 12, i étant un nombre entier de 2 à n-1, une chaîne de génération de génération i donnée est reliée à une chaîne de génération de génération i-1, c'est-à-dire de génération immédiatement inférieure à la génération donnée, et à au moins deux chaînes de génération de génération i+1, c'est-à-dire de génération immédiatement supérieure à la génération donnée. Lorsque i vaut n alors la chaîne de génération de génération i est reliée à une chaîne de génération de génération n-1 et éventuellement à au moins 1 chaîne intermédiaire.

Le cas échéant, on désigne, pour une chaîne de génération d'une génération donnée, par « coefficient de divergence » le nombre de chaînes de génération de la génération immédiatement supérieure à la génération donnée qui lui sont liées.

Selon un mode de réalisation préféré de l'invention, le coefficient de divergence est de 2à5.

Selon un mode de réalisation particulier de l'invention, le coefficient de divergence est identique pour une génération donnée de chaînes de génération, en revanche il peut varier d'une génération à l'autre.

Dans un mode de réalisation particulier de l'invention, le coefficient de divergence est identique pour chaque génération d'un dendrimère donné.

Selon un mode de réalisation encore plus particulier de l'invention, le coefficient de divergence est de 2.

Selon un autre mode de réalisation particulier de l'invention, une chaîne de génération de génération n est liée à un nombre de groupements terminaux ou de chaînes intermédiaires correspondant au coefficient de divergence.

Avantageusement, un coefficient de divergence élevé permet de disposer d'un grand nombre de groupements terminaux à la surface d'un dendrimère de génération donnée.

Selon un mode de réalisation particulier de l'invention, les chaînes de génération sont identiques entre elles pour une même génération mais peuvent être différentes d'une génération à l'autre.

Selon un autre mode de réalisation particulier de l'invention, les chaînes de génération sont toutes identiques entre elles.

Selon un autre mode de réalisation particulier de l'invention, le noyau § établit m liaisons avec m chaînes de liaison identiques constituées :

- soit par des chaînes de génération fixées en arborescence autour du noyau sur chacune des liaisons, l'extrémité de chaque chaîne de la génération la plus éloignée du noyau central étant fixée soit à un groupe terminal soit à une chaîne intermédiaire, l'extrémité de chaque chaîne intermédiaire étant fixée à un groupe terminal,
- soit par des chaînes intermédiaires fixées autour du noyau sur chacune des liaisons, l'extrémité de chaque chaîne intermédiaire étant fixée à un groupe terminal.

Selon ce mode de réalisation, pour un dendrimère donné, l'ensemble des chaînes de génération de la génération la plus éloignée du noyau central sont fixées à un substituant identique qui peut être soit à un groupe terminal soit à une chaîne intermédiaire.

Selon un mode de réalisation alternatif de l'invention, le noyau établit m-2 ou m-1 liaisons, m représentant un entier de 3 à 20, en particulier de 3 à 10 et plus particulièrement de 3 à 8, avec respectivement m-2 ou m-1 chaînes de liaison identiques constituées :

- soit par des chaînes de génération fixées en arborescence autour du noyau sur chacune des liaisons, l'extrémité de chaque chaîne de la génération la plus éloignée du noyau central étant fixée soit à un groupe terminal soit à une chaîne intermédiaire, et l'extrémité de chaque chaîne intermédiaire étant fixée à un groupe terminal,
- soit par des chaînes intermédiaires fixées autour du noyau sur chacune des liaisons, l'extrémité de chaque chaîne intermédiaire étant fixée à un groupe terminal ;

les 1 ou 2 liaisons restantes étant fixées à des groupes de liaison, identiques ou différents, éventuellement liés ensemble, notamment par l'intermédiaire d'une liaison covalente, constitués :

- soit d'une partie des chaînes de liaison définies ci-dessus,
- soit d'un atome d'hydrogène,
- soit de groupes hydrocarbonés comprenant de 1 à 500 atomes de carbone, lesdits groupes hydrocarbonés étant notamment constitués de H ou d'une chaîne hydrocarbonée de 1 à 200 chaînons, linéaire, ramifiée ou cyclique, contenant éventuellement une ou plusieurs doubles ou triples liaisons, chacun desdits chaînons pouvant éventuellement être choisi parmi un hétéroatome, ledit hétéroatome étant préférentiellement choisi parmi un atome d'azote, d'oxygène, de phosphore, de silicium ou de soufre, un groupement aryle de 6 à 24 atomes de carbone, un groupement hétéroaryle de 1 à 24 atomes de carbone, un groupement carboxyle, un groupement >C=NR, chaque chaînon

pouvant être éventuellement substitué par au moins un substituant choisi parmi un groupement hydroxyle, un groupement -NR''R''', un groupement alkoxy de 1 à 16 atomes de carbone, un groupement alkyle de 1 à 16 atomes de carbone, un atome d'halogène, un groupement $-NO_2$, un groupement -CN, un groupement - $CF_3$, un groupement aryle de 6 à 24 atomes de carbone, un groupement aralkyle de 7 à 16

atomes de carbone, R'' et R''' représentant indépendamment l'un de l'autre H ou un groupement alkyle de 1 à 16 atomes de carbone, aryle de 6 à 24 atomes de carbone ou aralkyle de 7 à 16 atomes de carbone, le premier chaînon de ladite chaîne hydrocarbonée étant de préférence un oxygène ou un azote.

En particulier les groupes hydrocarbonés comprenant de 1 à 500 atomes de carbone définis ci-dessus peuvent être des fluorophores, ou tout groupement chimique fonctionnel.

Selon un autre mode de réalisation particulier de l'invention, les chaînes de génération sont choisies parmi toute chaîne hydrocarbonée de 1 à 12 chaînons, linéaire, ramifiée ou cyclique, contenant éventuellement une ou plusieurs doubles ou triples liaisons, chacun desdits chaînons pouvant éventuellement être choisi parmi un hétéroatome, ledit hétéroatome étant préférentiellement choisi parmi l'azote, l'oxygène, le soufre, le phosphore ou le silicium, un groupement aryle de 6 à 24 atomes de carbone, un groupement hétéroaryle de 1 à 24 atomes de carbone, l'hétéroélément étant préférentiellement choisi parmi l'oxygène, l'azote ou le soufre, un groupement carboxyle, un groupement >C=NR, chaque chaînon pouvant éventuellement être substitué par au moins un substituant choisi parmi un groupement alkyle de 1 à 16 atomes de carbone, un atome d'halogène, un groupement $-NO_2$, un groupement - NRR', un groupement -CN, un groupement $-CF_3$, un groupement hydroxyle, un groupement alkoxy de 1 à 16 atomes de carbone, un groupement aryle de 6 à 24 atomes de carbone, ou un groupement aralkyle de 7 à 16 atomes de carbone, R et R' représentant indépendamment l'un de l'autre H ou un groupement alkyle de 1 à 16 atomes de carbone, aryle de 6 à 24 atomes de carbone, ou aralkyle de 7 à 16 atomes de carbone.

Selon encore un autre mode de réalisation particulier de l'invention, les chaînes intermédiaires sont choisies parmi les groupes correspondant à la formule :

-J-K-L-

où

- J représente un atome d'oxygène, de soufre, ou un groupement -NR- ;
- K représente un groupement aryle de 6 à 24 atomes de carbone, hétéroaryle de 1 à 24 atomes de carbone, l'hétéroélément étant préférentiellement choisi parmi l'oxygène, l'azote ou le soufre, alkyle de 1 à 16 atomes de carbone, chacun pouvant être éventuellement substitué par un atome d'halogène ou un groupement $-NO_2$, -NRR', - CN, $-CF_3$, -OH, alkyle de 1 à 16 atomes de carbone, aryle de 6 à 24 atomes de carbone, aralkyle de 7 à 16 atomes de carbone ;
- L représente une chaîne hydrocarbonée de 0 à 10 chaînons, notamment de 0 à 6 chaînons, linéaire, ramifiée ou cyclique, contenant éventuellement une ou plusieurs doubles ou triples liaisons, chacun desdits chaînons pouvant éventuellement être un hétéroatome, ledit hétéroatome étant préférentiellement choisi parmi l'oxygène, le soufre, l'azote, le phosphore, le silicium chaque chaînon pouvant être éventuellement substitué par au moins un substituant choisi parmi un groupement alkyle de 1 à 16 atomes de carbone, un halogène, un atome d'oxygène, $-NO_2$, -NRR', -CN, $-CF_3$, -OH, un groupement alkoxy de 1 à 16 atomes de carbone, un groupement aryle de 6 à 24 atomes de carbone, un groupement aralkyle de 7 à 16 atomes de carbone ;

R et R' représentant indépendamment l'un de l'autre H ou un groupement alkyle de 1 à 16 atomes de carbone, aryle de 6 à 24 atomes de carbone, ou aralkyle de 7 à 16 atomes de carbone.

Selon un mode de réalisation plus particulier de l'invention, le noyau est choisi parmi :

- un atome d'azote ou de silicium ;
- un groupement de formule

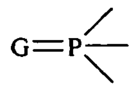 ,

dans lequel G représente un atome d'oxygène, d'azote, de soufre, de sélénium, de tellure ou un groupement =NR, R représentant H ou un groupement alkyle de 1 à 16 atomes de carbone, aryle de 6 à 24 atomes de carbone, ou aralkyle de 7 à 16 atomes de carbone, tel que le groupement thiophosphoryle de formule

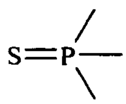 ;

- un groupement bis-phényloxy de formule

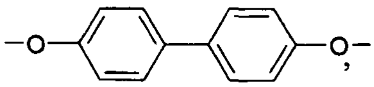 ,

- un groupement 1,2-diamino-éthane de formule

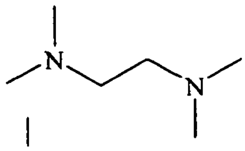 ,

- un groupement 1,4-diamino-butane de formule

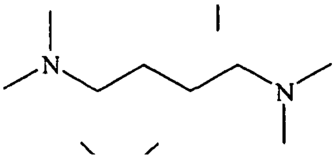 ,

- un groupement cyclotriphosphazène de formule

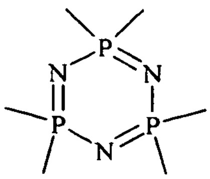 ,

également notée $N_3P_3$ ou $P_3N_3$,

- un groupement cyclotetraphosphazène de formule

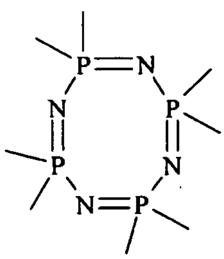 ,

également notée $N_4P_4$ ou $P_4N_4$.

La présente invention concerne en particulier l'utilisation telle que définie ci-dessus, de dendrimères de structure PAMAM, DAB ou PMMH.

Les dendrimères de structure PAMAM sont notamment décrits par D.A. Tomalia, H. Baker, J. Dewald, M. Hall, G. Kallos, S. Martin, J. Roeck, J. Ryder, P.S. Smith, Polym. J. (Tokyo) 1985, 17, 117; D.A. Tomalia, H. Baker, J. Dewald, M. Hall, G. Kallos, S. Martin, J. Roeck, J. Ryder, P.S. Smith, Macromolecules, 1986, 19, 2466.

Les dendrimères de structure DAB sont notamment décrits par E.M.M. de Brabander-van den Berg, E.W.

Meijer Angew. Chem. Int. Ed. Engl. 1993, 32, 1308.

Les dendrimères de structure PMMH sont notamment décrits dans « A general synthetic strategy for neutral phosphorus containing dendrimers » Launay N., Caminade A.M., Lahana R., Majoral J.P., Angew. Chem. 1994, 106, 1682. Angew. Chem. Int. Ed. Engl. 1994, 33, 1589 et dans «Synthesis of bowl-shaped dendrimers from generation 1 to generation 8 » Launay N., Caminade A.M., Majoral J.P., J. Organomet. Chem. 1997, 529, 51

Un exemple de dendrimère de type PAMAM pour lequel n = 4 et m = 4 est représenté ci-dessous :

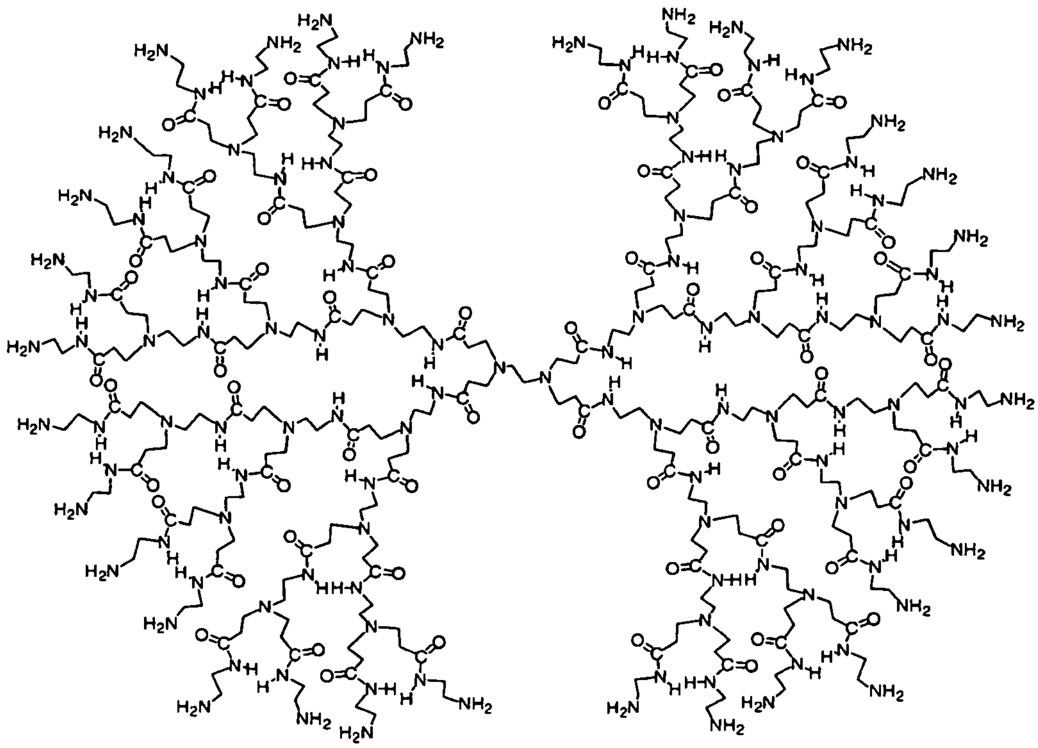

Un exemple de dendrimère de type DAB, pour lequel n = 5 et m = 4 est représenté ci-dessous :

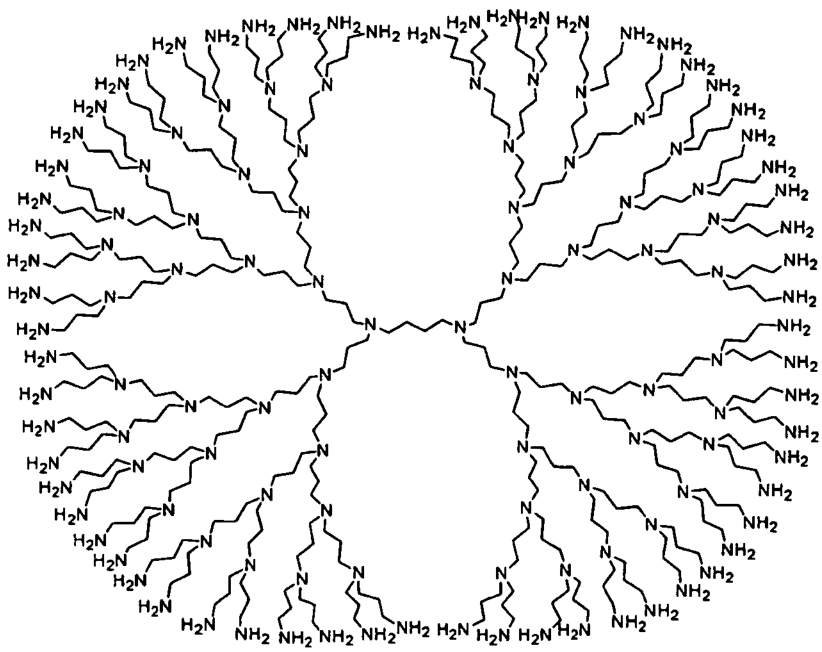

Un exemple de dendrimère de type PMMH à noyau thiophosphoryle, pour lequel n = 4 et m = 3 est représenté ci-dessous :

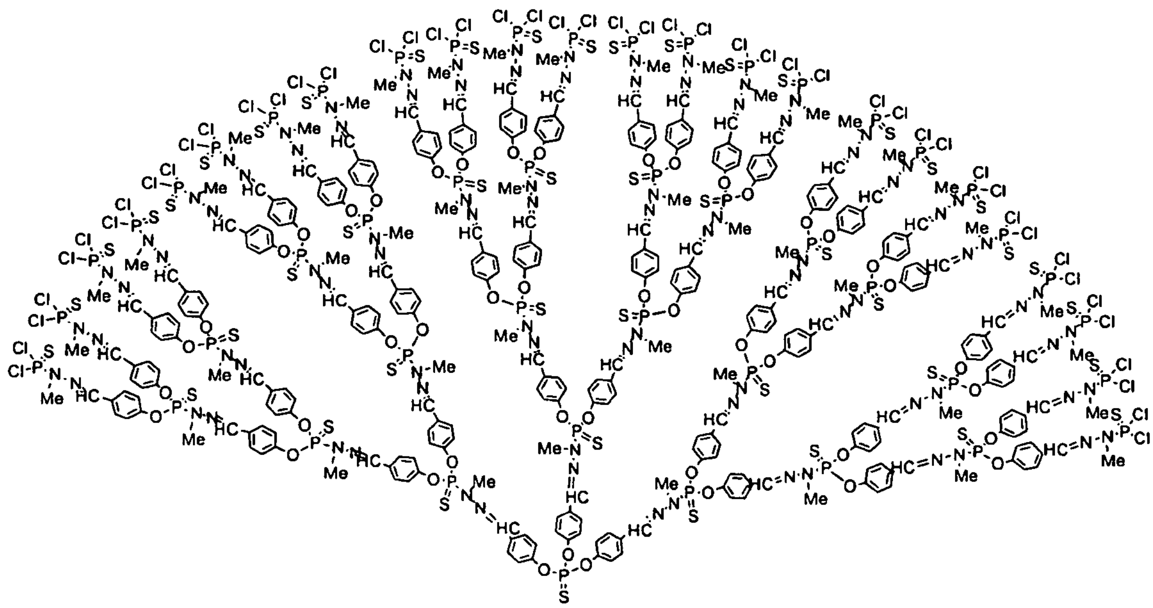

Un exemple de dendrimère de type PMMH à noyau cyclotriphosphazène, pour lequel n = 2 et m = 6, sans chaîne intermédiaire, est représenté ci-dessous :

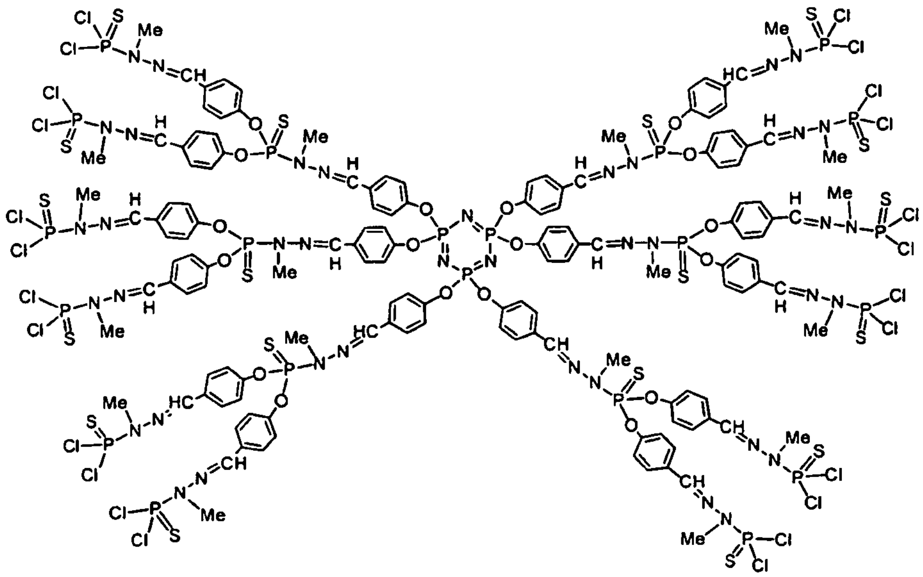

Un autre exemple de dendrimère de type PMMH à noyau cyclotriphosphazène, pour lequel n = 2 et m = 6, avec chaîne intermédiaire, est représenté ci-dessous :

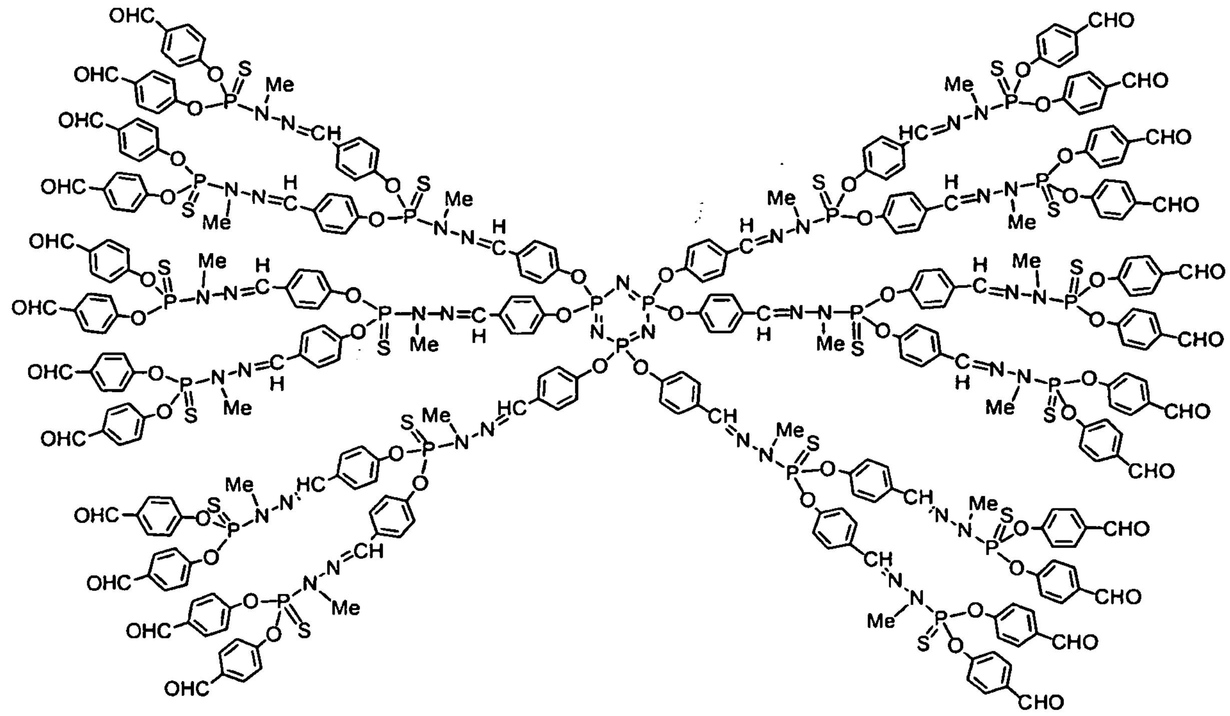

Dans un mode de réalisation particulier, l'invention concerne l'utilisation telle que définie ci-dessus, de dendrimères à terminaisons monophosphoniques ou bisphosphoniques correspondant à la formule générale (1a) suivante :

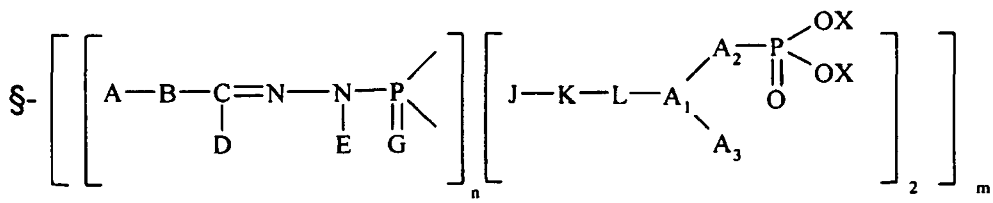

(1a)

où n représente un entier de 0 à 3, à savoir :

- lorsque n = 0, la formule (1a) correspond à la formule (2a) suivante,

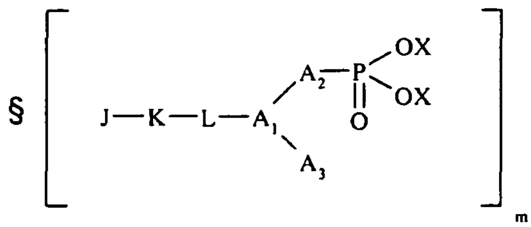

(2a)

- lorsque n = 1, la formule (1a) correspond à la formule (3a) suivante,

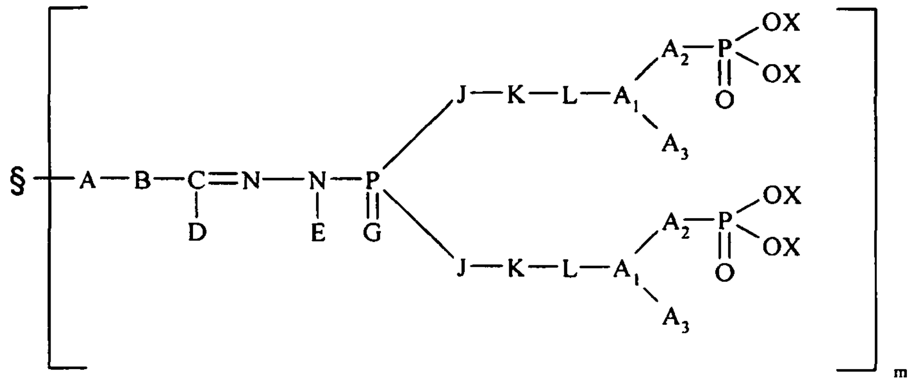

(3a)

- lorsque n = 2, la formule (1a) correspond à la formule (4a) suivante,

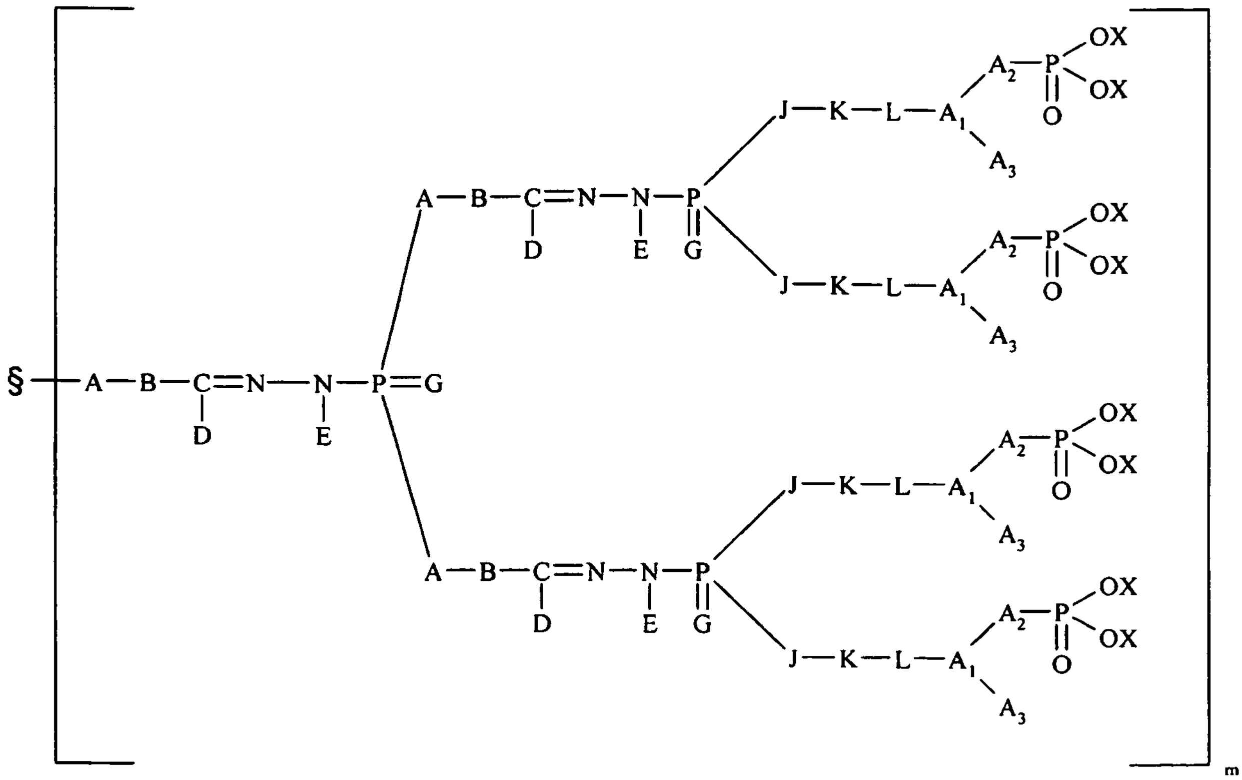

(4a)

- et lorsque n = 3, la formule (1a) correspond à la formule (5a) suivante,

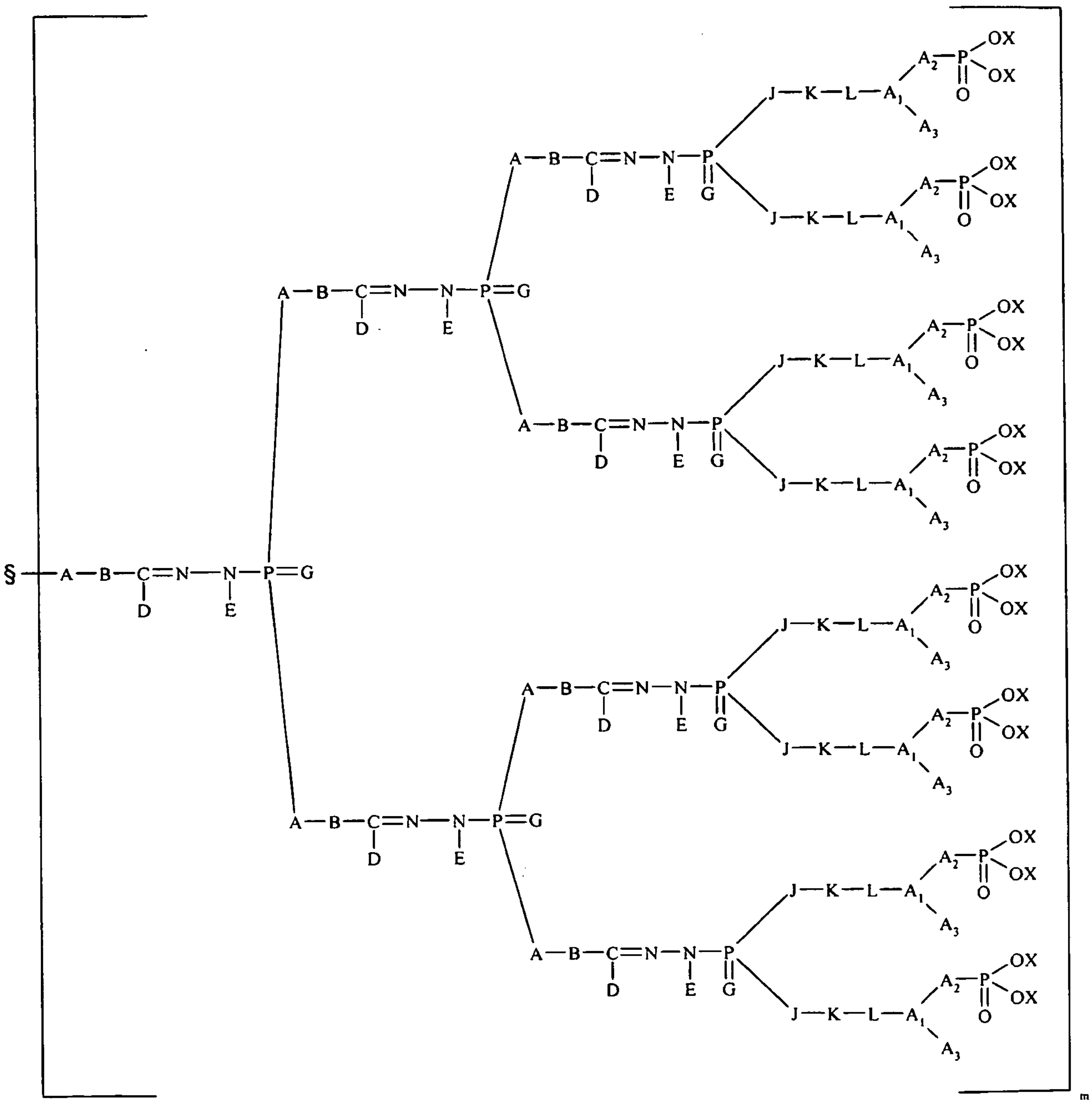

(5a)

et dans lesquelles formules :

- le noyau central § est choisi parmi les groupes suivants :

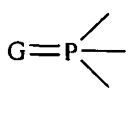 , 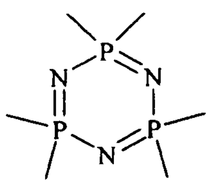 , 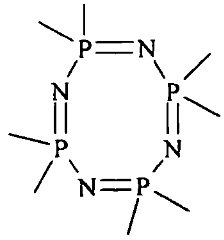 ;

- m représente 3, 6 ou 8 ;
- la chaîne de génération correspond à la formule :

où

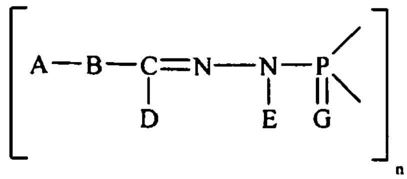

- A représente un atome d'oxygène, de soufre, de phosphore ou un groupement -NR-;
- B représente un groupement aryle de 6 à 24 atomes de carbone, hétéroaryle de 1 à 24 atomes de carbone, alkyle de 1 à 16 atomes de carbone, chacun pouvant être éventuellement substitué par un atome d'halogène ou un groupement $-NO_2$, -NRR', - CN, $-CF_3$, -OH, alkyle de 1 à 16 atomes de carbone, aryle de 6 à 24 atomes de carbone, aralkyle de 7 à 16 atomes de carbone ;
- D représente un atome d'hydrogène, un groupement alkyle de 1 à 16 atomes de carbone, alkoxy de 1 à 16 atomes de carbone, aryle de 6 à 24 atomes de carbone, aralkyle de 7 à 16 atomes de carbone, chacun pouvant être éventuellement substitué par un atome d'halogène ou un groupement $-NO_2$, -NRR', -CN, $-CF_3$, -OH, alkyle de 1 à 16 atomes de carbone, aryle de 6 à 24 atomes de carbone, aralkyle de 7 à 16 atomes de carbone ;
- E représente un atome d'hydrogène, un groupement alkyle de 1 à 16 atomes de carbone, alkoxy de 1 à 16 atomes de carbone, aryle de 6 à 24 atomes de carbone, aralkyle de 7 à 16 atomes de carbone, chacun pouvant être éventuellement substitué par un atome d'halogène ou un groupement $-NO_2$, -NRR', -CN, $-CF_3$, -OH, alkyle de 1 à 16 atomes de carbone, aryle de 6 à 24 atomes de carbone, aralkyle de 7 à 16 atomes de carbone ;
- G représente un atome d'oxygène, d'azote, de soufre, de sélénium, de tellure ou un groupement =NR ;

R et R' représentant indépendamment l'un de l'autre H ou un groupement alkyle de 1 à 16 atomes de carbone, aryle de 6 à 24 atomes de carbone, ou aralkyle de 7 à 16 atomes de carbone ;

• la chaîne intermédiaire correspond à la formule :

-J-K-L-

où

- J représente un atome d'oxygène, de soufre, ou un groupement -NR- ;
- K représente un groupement aryle de 6 à 24 atomes de carbone, hétéroaryle de 1 à 24 atomes de carbone, l'hétéroélément étant préférentiellement choisi parmi l'oxygène, l'azote ou le soufre, alkyle de 1 à 16 atomes de carbone, chacun pouvant être éventuellement substitué par un atome d'halogène ou un groupement $-NO_2$, -NRR', - CN, $-CF_3$, -OH, alkyle de 1 à 16 atomes de carbone, aryle de 6 à 24 atomes de carbone, aralkyle de 7 à 16 atomes de carbone ;
- L représente une chaîne hydrocarbonée de 0 à 10 chaînons, linéaire, ramifiée ou cyclique, contenant éventuellement une ou plusieurs doubles ou triples liaisons, chacun desdits chaînons pouvant éventuellement être un hétéroatome, ledit hétéroatome étant préférentiellement choisi parmi l'oxygène, le soufre, l'azote, le phosphore, le silicium chaque chaînon pouvant être éventuellement substitué par au moins un substituant choisi parmi un groupement alkyle de 1 à 16 atomes de carbone, un halogène, un atome d'oxygène, $-NO_2$, -NRR', -CN, $-CF_3$, -OH, un groupement alkoxy de 1 à 16 atomes de carbone, un groupement aryle de 6 à 24 atomes de carbone, un groupement aralkyle de 7 à 16 atomes de carbone ;

R et R' représentant indépendamment l'un de l'autre H ou un groupement alkyle de 1 à 16 atomes de carbone, aryle de 6 à 24 atomes de carbone, ou aralkyle de 7 à 16 atomes de carbone ;

• le groupe terminal correspond à la formule :

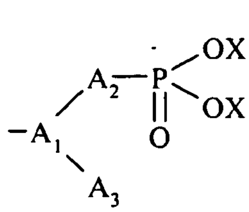

où $A_1$, $A_3$ et X ont été définis précédemment, chacun des X étant identique ou différent.

Dans un mode de réalisation plus particulier, l'invention concernel'utilisation telle que définie ci-dessus d'un dendrimère de formule générale (1a) dans laquelle $A_3$ représente :

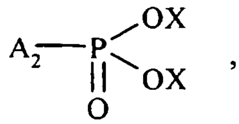

ladite formule générale (1a) correspondant alors à la formule générale (1) suivante :

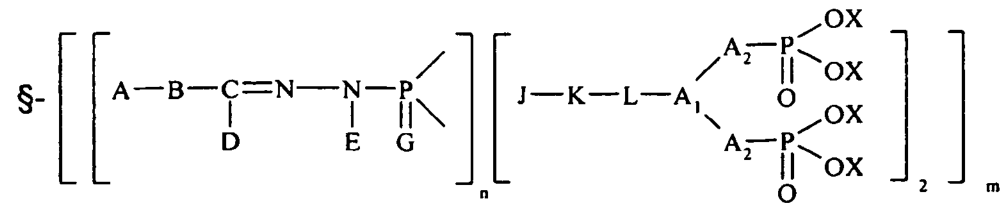

(1)

§, A, B, C, D, E, G, J, K, L, $A_1$, $A_2$, X, m et n étant tels que définis ci-dessus. C'est-à-dire que l'invention concerne plus particulièrement l'utilisation telle que définie ci-dessus de dendrimères à terminaisons bisphosphoniques correspondant à la formule générale (1) suivante :

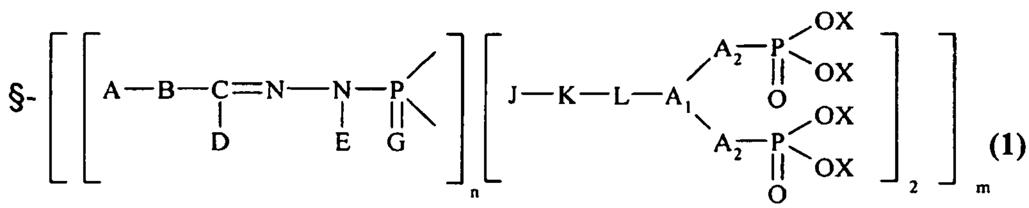

**(1)**

où n représente un entier de 0 à 3, à savoir :

- lorsque n = 0, la formule (1) correspond à la formule (2) suivante,

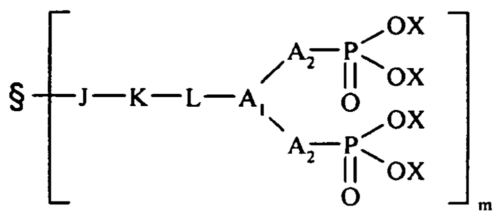

**(2)**

- lorsque n = 1, la formule (1) correspond à la formule (3) suivante,

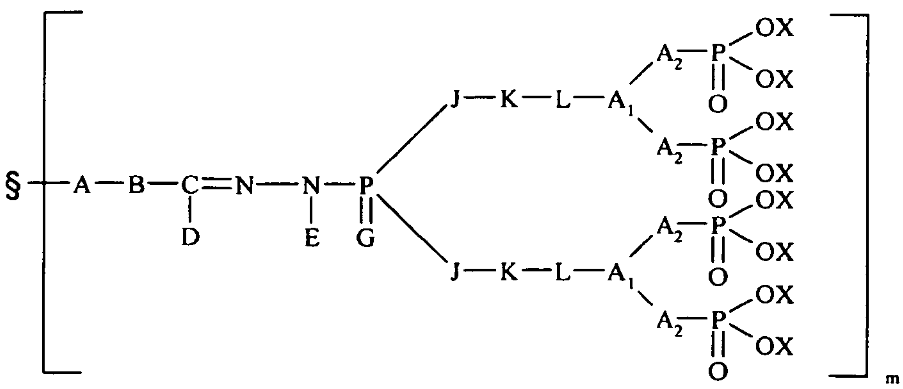

**(3)**

- lorsque n = 2, la formule (1) correspond à la formule (4) suivante,

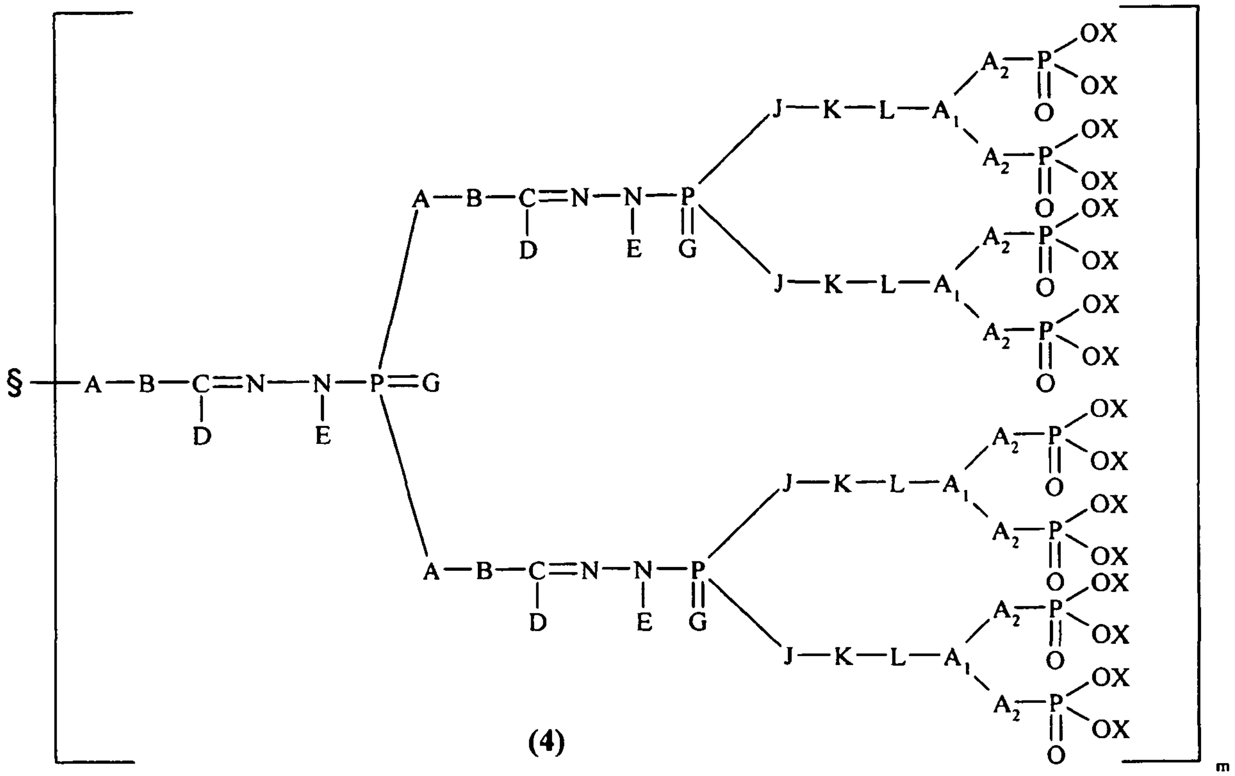

(4)

- et lorsque n = 3, la formule (1) correspond à la formule (5) suivante,

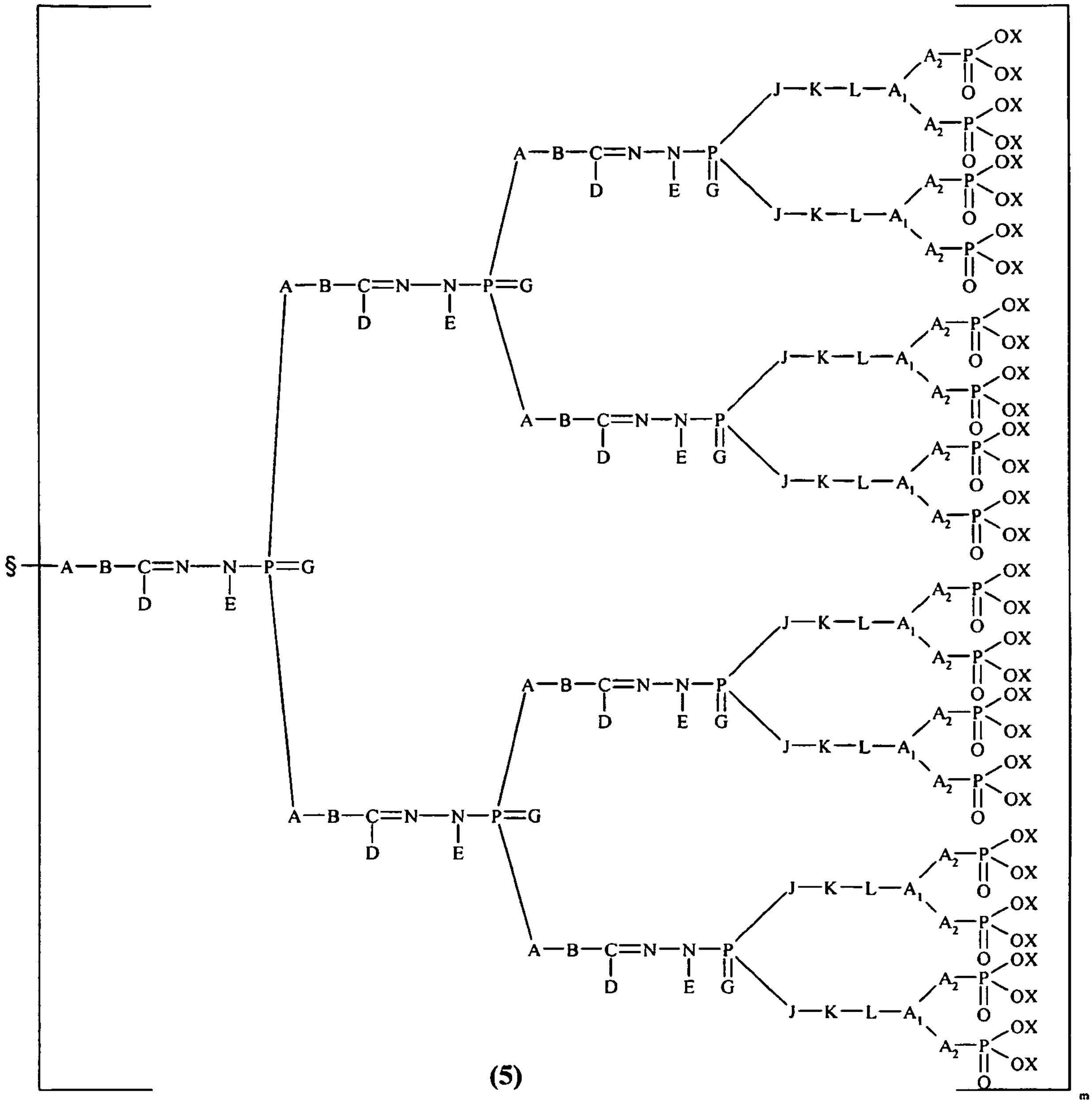

(5)

et dans lesquelles formules :

- le noyau central § est choisi parmi les groupes suivants :

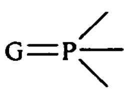 , 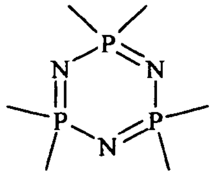 , 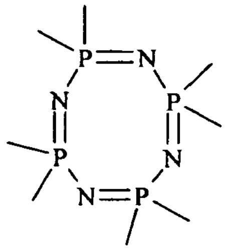 ;

- m représente 3, 6 ou 8 ;
- la chaîne de génération correspond à la formule :

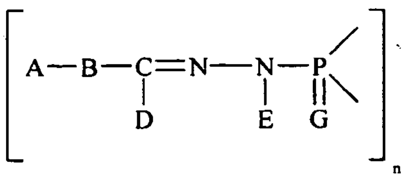

où

- A représente un atome d'oxygène, de soufre, de phosphore ou un groupement -NR-;
- B représente un groupement aryle de 6 à 24 atomes de carbone, hétéroaryle de 1 à 24 atomes de carbone, alkyle de 1 à 16 atomes de carbone, chacun pouvant être éventuellement substitué par un atome d'halogène ou un groupement $-NO_2$, -NRR', - CN, $-CF_3$, -OH, alkyle de 1 à 16 atomes de carbone, aryle de 6 à 24 atomes de carbone, aralkyle de 7 à 16 atomes de carbone ;
- D représente un atome d'hydrogène, un groupement alkyle de 1 à 16 atomes de carbone, alkoxy de 1 à 16 atomes de carbone, aryle de 6 à 24 atomes de carbone, aralkyle de 7 à 16 atomes de carbone, chacun pouvant être éventuellement substitué par un atome d'halogène ou un groupement $-NO_2$, -NRR', -CN, $-CF_3$, -OH, alkyle de 1 à 16 atomes de carbone, aryle de 6 à 24 atomes de carbone, aralkyle de 7 à 16 atomes de carbone ;
- E représente un atome d'hydrogène, un groupement alkyle de 1 à 16 atomes de carbone, alkoxy de 1 à 16 atomes de carbone, aryle de 6 à 24 atomes de carbone, aralkyle de 7 à 16 atomes de carbone, chacun pouvant être éventuellement substitué par un atome d'halogène ou un groupement $-NO_2$, -NRR', -CN, $-CF_3$, -OH, alkyle de 1 à 16 atomes de carbone, aryle de 6 à 24 atomes de carbone, aralkyle de 7 à 16 atomes de carbone ;
- G représente un atome d'oxygène, d'azote, de soufre, de sélénium, de tellure ou un groupement =NR ;

R et R' représentant indépendamment l'un de l'autre H ou un groupement alkyle de 1 à 16 atomes de carbone, aryle de 6 à 24 atomes de carbone, ou aralkyle de 7 à 16 atomes de carbone ;

- la chaîne intermédiaire correspond à la formule :

-J-K-L-

où

- J représente un atome d'oxygène, de soufre, ou un groupement -NR- ;
- K représente un groupement aryle de 6 à 24 atomes de carbone, hétéroaryle de 1 à 24 atomes de carbone, l'hétéroélément étant préférentiellement choisi parmi l'oxygène, l'azote ou le soufre, alkyle de 1 à 16 atomes de carbone, chacun pouvant être éventuellement substitué par un atome d'halogène ou un groupement $-NO_2$, -NRR', - CN, $-CF_3$, -OH, alkyle de 1 à 16 atomes de carbone, aryle de 6 à 24 atomes de carbone, aralkyle de 7 à 16 atomes de carbone ;
- L représente une chaîne hydrocarbonée de 0 à 10 chaînons, notamment de 0 à 6 chaînons, linéaire, ramifiée ou cyclique, contenant éventuellement une ou plusieurs doubles ou triples liaisons, chacun desdits chaînons pouvant éventuellement être un hétéroatome, ledit hétéroatome étant préférentiellement choisi parmi l'oxygène, le soufre, l'azote, le phosphore, le silicium chaque chaînon pouvant être éventuellement substitué par au moins un substituant choisi parmi un groupement alkyle de 1 à 16 atomes de carbone, un halogène, un atome d'oxygène, $-NO_2$, -NRR', -CN, $-CF_3$, -OH, un groupement alkoxy de 1 à 16 atomes de carbone, un groupement aryle de 6 à 24 atomes de carbone, un groupement aralkyle de 7 à 16 atomes de carbone ;

R et R' représentant indépendamment l'un de l'autre H ou un groupement alkyle de 1 à 16 atomes de carbone, aryle de 6 à 24 atomes de carbone, ou aralkyle de 7 à 16 atomes de carbone ;

- le groupe terminal correspond à la formule :

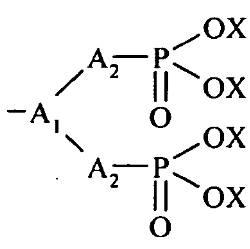

où $A_1$, $A_2$ et X ont été définis précédemment, chacun des X étant identique ou différent.

Selon un mode de réalisation préféré, l'invention concerne l'utilisation telle que définie ci-dessus d'un dendrimère de formule générale (1) de structure PMMH, dans laquelle

§ représente

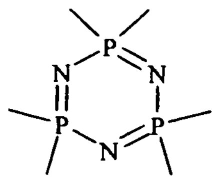 ;

m représente 6 ;
n représente 0, 1, ou 2 ;
A représente un atome d'oxygène ;
B représente un groupement benzénique ;
D représente un hydrogène ;
E représente un groupement méthyle ;
G représente un atome de soufre ;
J représente un atome d'oxygène ;
K représente un groupement benzénique ;
L représente une chaîne hydrocarbonée saturée linéaire non substituée à deux atomes de carbone ;
$A_1$ représente un atome d'azote ;
$A_2$ représente un groupement $CH_2$ ;
X représente un groupement méthyle, ou un atome d'hydrogène ou de sodium ; ledit dendrimère étant désigné GCn, n étant défini ci-dessus.

Selon un mode de réalisation particulièrement préféré, l'invention concerne l'utilisation telle que définie ci-dessus de composés de formules suivantes :

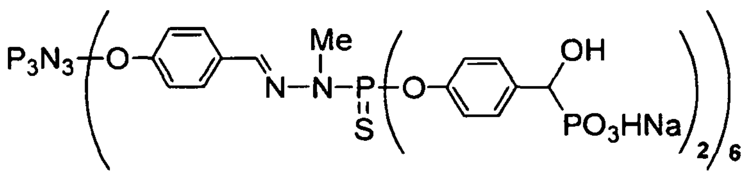

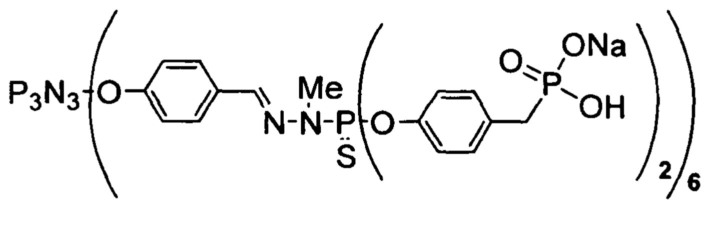

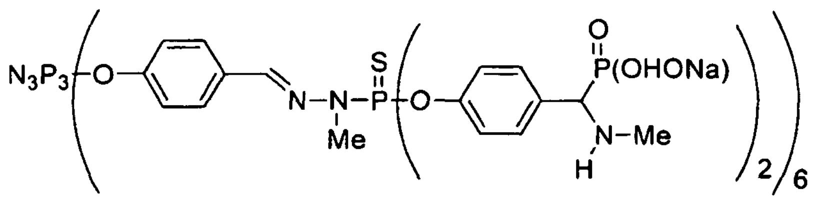

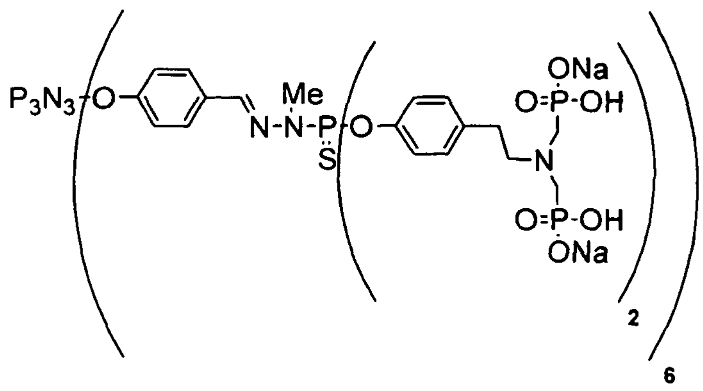

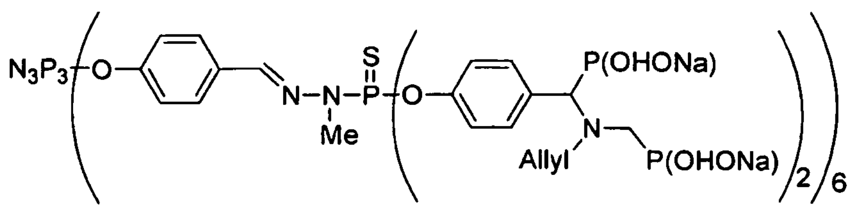

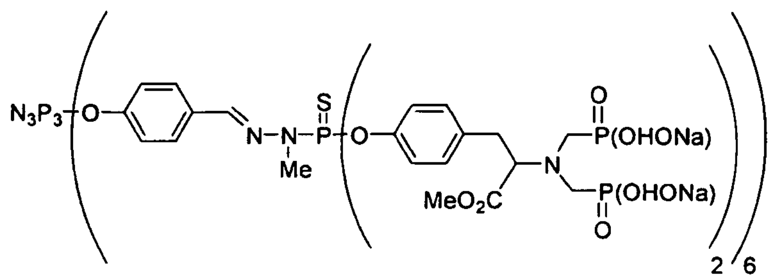

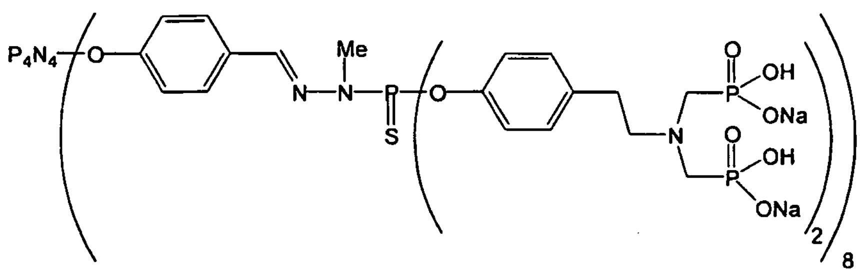

ou de composés GC1 de formules suivantes :

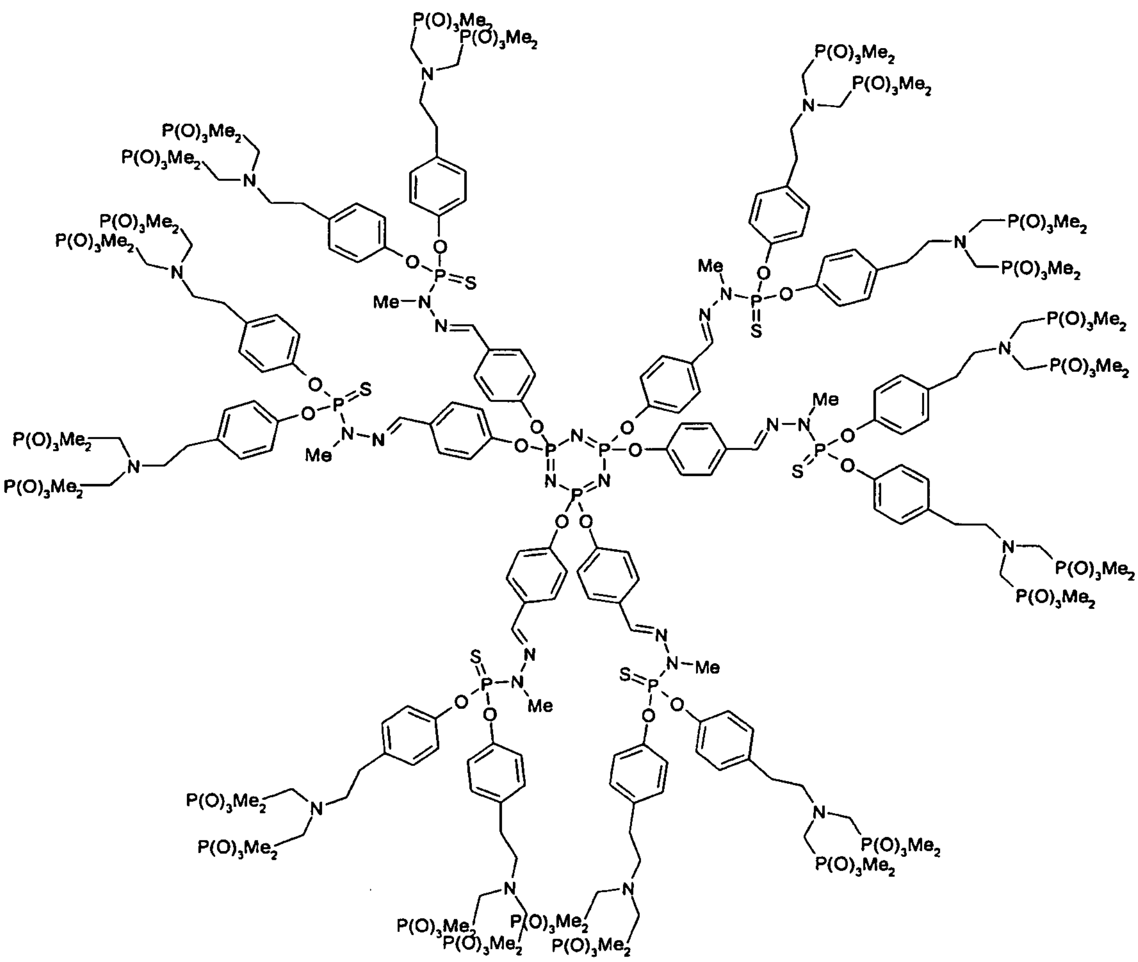

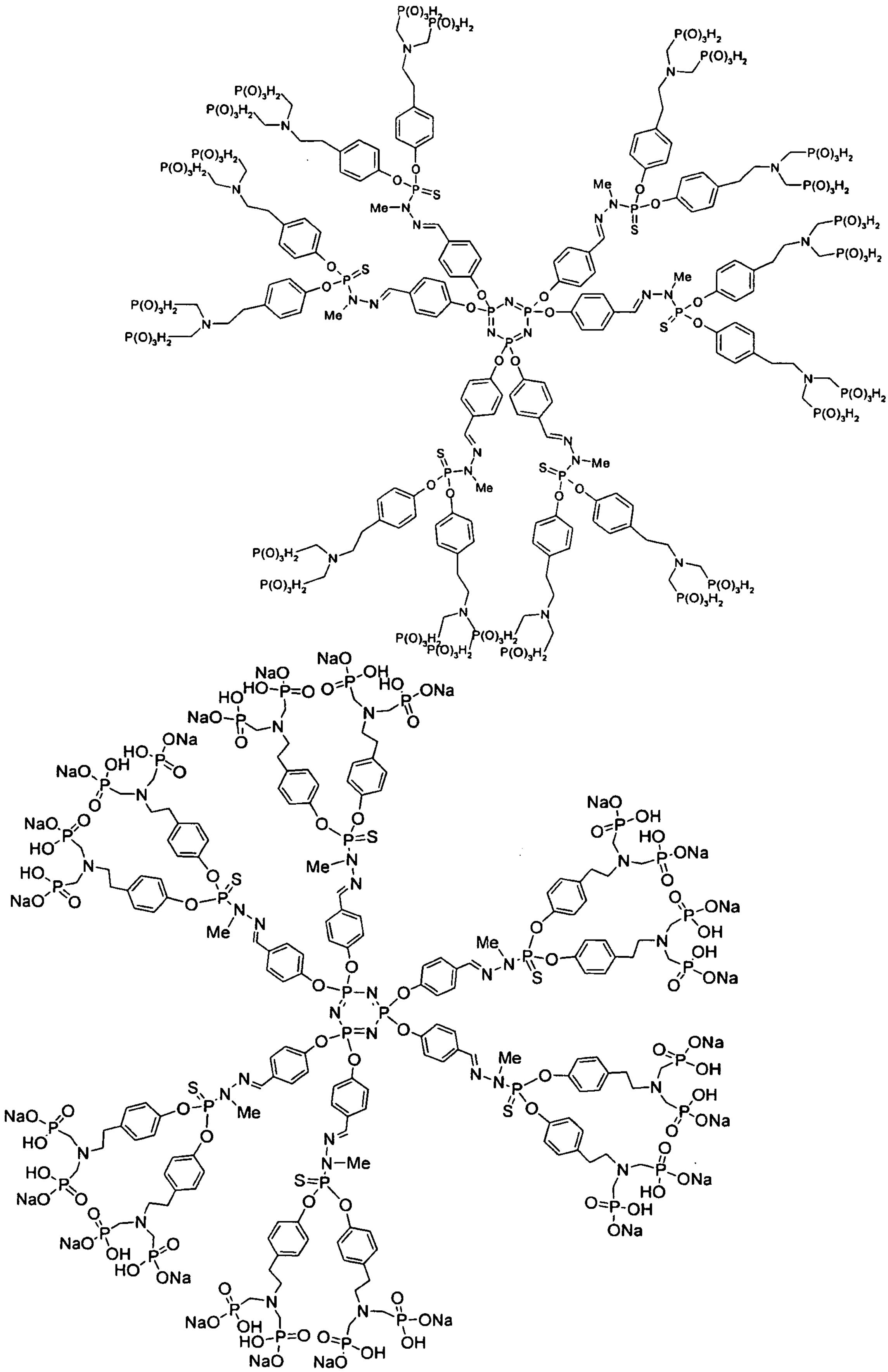

et notamment de formule (6) suivante :

formule (6) suivante :

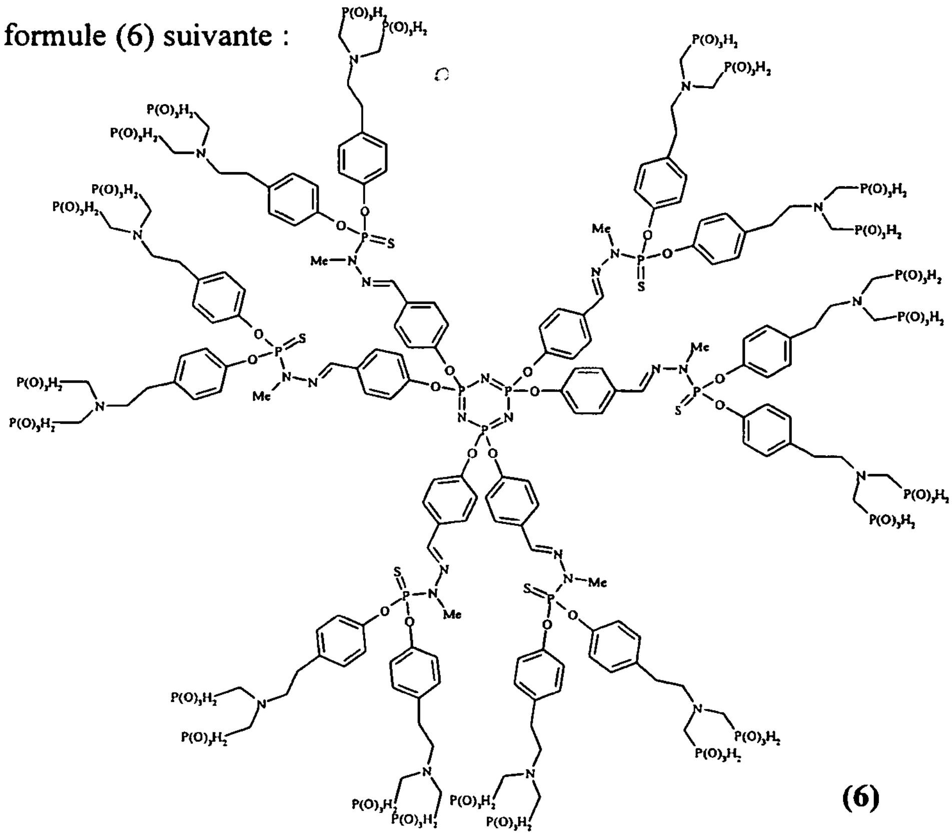

(6)

La présente invention concerne également l'utilisation telle que définie ci-dessus, de dendrimères à terminaisons bisphosphoniques correspondant à la formule générale (7) suivante :

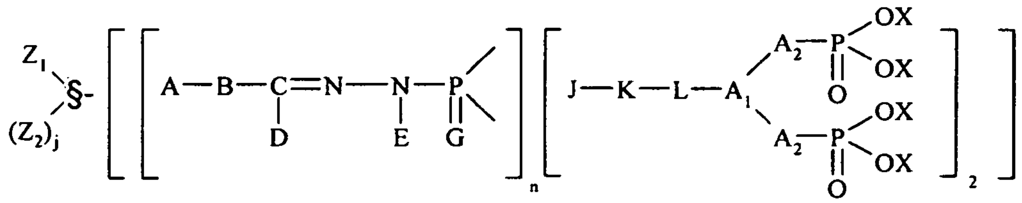

(7)

où n représente un entier de 0 à 3, m représente 3, 6 ou 8, p représente m - 1 ou m - 2, et j représente 0 lorsque p représente m - 1 et 1 lorsque p représente m - 2, à savoir :

- lorsque p = m - 1, la formule (7) correspond à la formule (8) suivante :

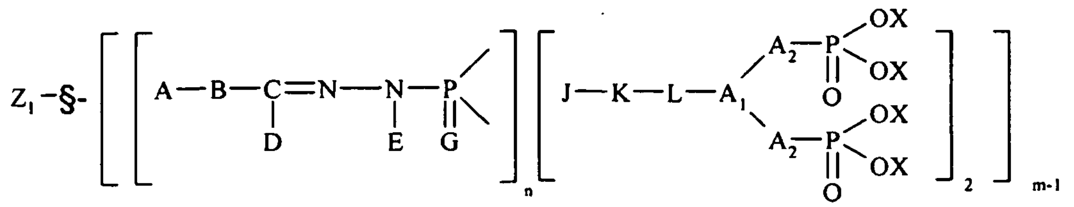

(8)

- lorsque p = m - 2, la formule (7) correspond à la formule (9) suivante :

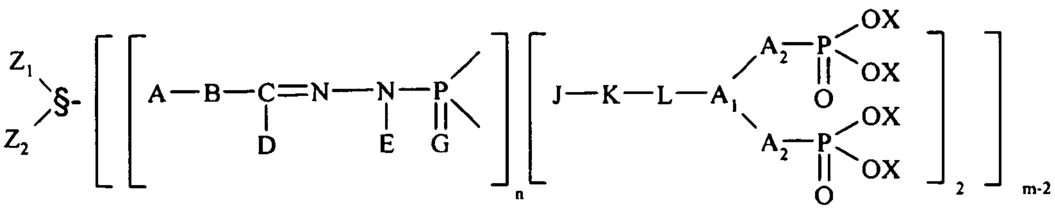 **(9)**

et dans lesquelles formules :

- le noyau central § est choisi parmi les groupes suivants :

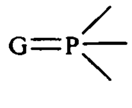 ,

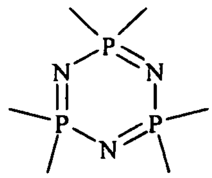 ,

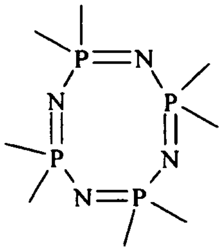 ;

- la chaîne de génération correspond à la formule :

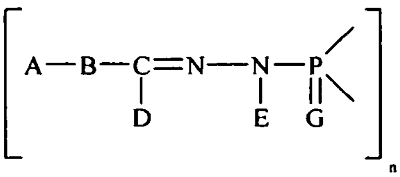

où

- A représente un atome d'oxygène, de soufre, de phosphore ou un groupement -NR-;
- B représente un groupement aryle de 6 à 24 atomes de carbone, hétéroaryle de 1 à 24 atomes de carbone, alkyle de 1 à 16 atomes de carbone, chacun pouvant être éventuellement substitué par un atome d'halogène ou un groupement -$NO_2$, -NRR', - CN, -$CF_3$, -OH, alkyle de 1 à 16 atomes de carbone, aryle de 6 à 24 atomes de carbone, aralkyle de 7 à 16 atomes de carbone ;
- D représente un atome d'hydrogène, un groupement alkyle de 1 à 16 atomes de carbone, alkoxy de 1 à 16 atomes de carbone, aryle de 6 à 24 atomes de carbone, aralkyle de 7 à 16 atomes de carbone, chacun pouvant être éventuellement substitué par un atome d'halogène ou un groupement -$NO_2$, -NRR', -CN, -$CF_3$, -OH, alkyle de 1 à 16 atomes de carbone, aryle de 6 à 24 atomes de carbone, aralkyle de 7 à 16 atomes de carbone ;
- E représente un atome d'hydrogène, un groupement alkyle de 1 à 16 atomes de carbone, alkoxy de 1 à 16 atomes de carbone, aryle de 6 à 24 atomes de carbone, aralkyle de 7 à 16 atomes de carbone, chacun pouvant être éventuellement substitué par un atome d'halogène ou un groupement -$NO_2$, -NRR', -CN, -$CF_3$, -OH, alkyle de 1 à 16 atomes de carbone, aryle de 6 à 24 atomes de carbone, aralkyle de 7 à 16 atomes de carbone ;
- G représente un atome d'oxygène, d'azote, de soufre, de sélénium, de tellure ou un groupement =NR ;
  R et R' représentant indépendamment l'un de l'autre H ou un groupement alkyle de 1 à 16 atomes de carbone, aryle de 6 à 24 atomes de carbone, ou aralkyle de 7 à 16 atomes de carbone ;

- la chaîne intermédiaire correspond à la formule :

  -J-K-L-

  où

- J représente un atome d'oxygène, de soufre, ou un groupement -NR- ;
- K représente un groupement aryle de 6 à 24 atomes de carbone, hétéroaryle de 1 à 24 atomes de carbone, l'hétéroélément étant préférentiellement choisi parmi l'oxygène, l'azote ou le soufre, alkyle de 1 à 16 atomes de

carbone, chacun pouvant être éventuellement substitué par un atome d'halogène ou un groupement $-NO_2$, -NRR', - CN, $-CF_3$, -OH, alkyle de 1 à 16 atomes de carbone, aryle de 6 à 24 atomes de carbone, aralkyle de 7 à 16 atomes de carbone ;

- L représente une chaîne hydrocarbonée de 0 à 10 chaînons, notamment de 0 à 6 chaînons, linéaire, ramifiée ou cyclique, contenant éventuellement une ou plusieurs doubles ou triples liaisons, chacun desdits chaînons pouvant éventuellement être un hétéroatome, ledit hétéroatome étant préférentiellement choisi parmi l'oxygène, le soufre, l'azote, le phosphore, le silicium, chaque chaînon pouvant être éventuellement substitué par au moins un substituant choisi parmi un groupement alkyle de 1 à 16 atomes de carbone, un halogène, un atome d'oxygène, $-NO_2$, -NRR', -CN, $-CF_3$, -OH, un groupement alkoxy de 1 à 16 atomes de carbone, un groupement aryle de 6 à 24 atomes de carbone, un groupement aralkyle de 7 à 16 atomes de carbone ;

  R et R' représentant indépendamment l'un de l'autre H ou un groupement alkyle de 1 à 16 atomes de carbone, aryle de 6 à 24 atomes de carbone, ou aralkyle de 7 à 16 atomes de carbone ;

  • le groupe terminal correspond à la formule :

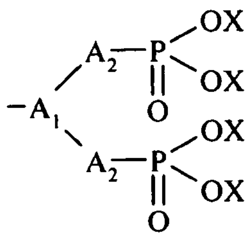

où A1, A2 et X ont été définis précédemment, chacun des X étant identique ou différent ;
$Z_1$ et $Z_2$ étant identiques ou différents, éventuellement liés ensemble, notamment par l'intermédiaire d'une liaison covalente, et représentant H ou une chaîne hydrocarbonée de 1 à 16 chaînons, linéaire, ramifiée ou cyclique, contenant éventuellement une ou plusieurs doubles ou triples liaisons, chacun desdits chaînons pouvant éventuellement être choisi parmi un hétéroatome, ledit hétéroatome étant préférentiellement choisi parmi un atome d'azote, d'oxygène, de phosphore, de silicium ou de soufre, un groupement aryle de 6 à 24 atomes de carbone, un groupement hétéroaryle de 1 à 24 atomes de carbone, un groupement carboxyle, un groupement >C=NR, chaque chaînon pouvant être éventuellement substitué par au moins un substituant choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement - $CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone, le premier chaînon de ladite chaîne hydrocarbonée étant de préférence un oxygène ou un azote.

L'invention concerne plus particulièrement l'utilisation telle que définie ci-dessus, d'un dendrimère de formule générale (8) de structure PMMH, dans laquelle

§ représente

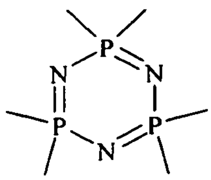 ;

m représente 6 ;
p représente 5 ;
n représente 0, 1, ou 2 ;
A représente un atome d'oxygène ;
B représente un groupement benzénique ;
D représente un hydrogène ;
E représente un groupement méthyle ;
G représente un atome de soufre ;
J représente un atome d'oxygène ;

K représente un groupement benzénique ;
L représente une chaîne hydrocarbonée saturée linéaire non substituée à deux atomes de carbone ;
$A_1$ représente un atome d'azote ;
$A_2$ représente un groupement $CH_2$ ;
X représente un groupement méthyle, ou un atome d'hydrogène ou de sodium ;
$Z_1$ représente un groupement phényloxy ;

ledit dendrimère étant désigné GCn', n étant défini ci-dessus.

De façon préférentielle, la présente invention concerne en particulier l'utilisation telle que définie ci-dessus :

- des composés de formules suivantes :

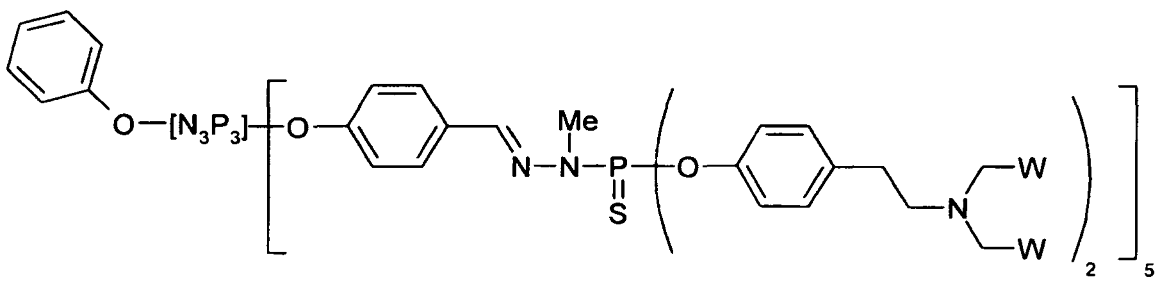

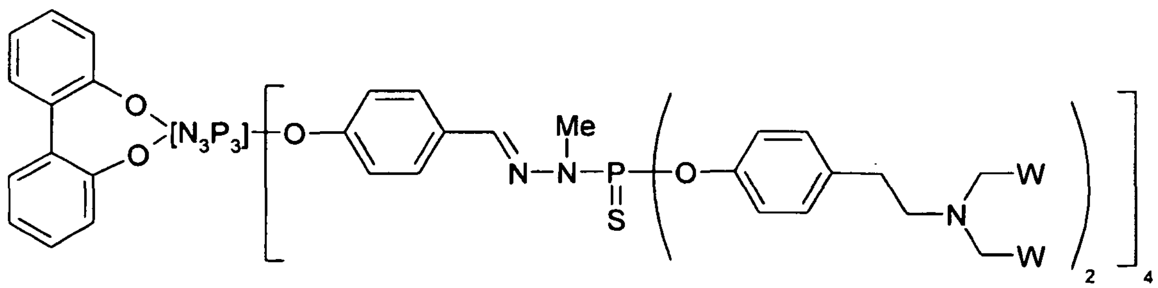

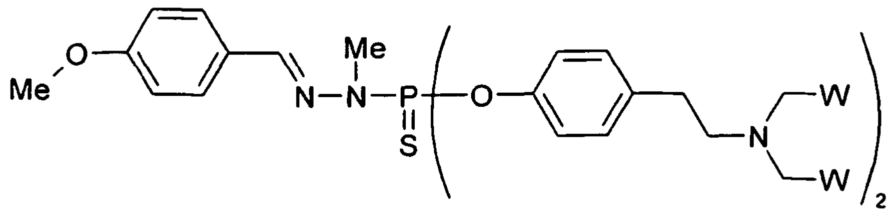

dans lesquelles W représente $PO_3Me_2$, $PO_3HNa$, $PO_3H_2$ lesdits composés correspondant, notamment, au composé GC1' de formule (10) suivante :

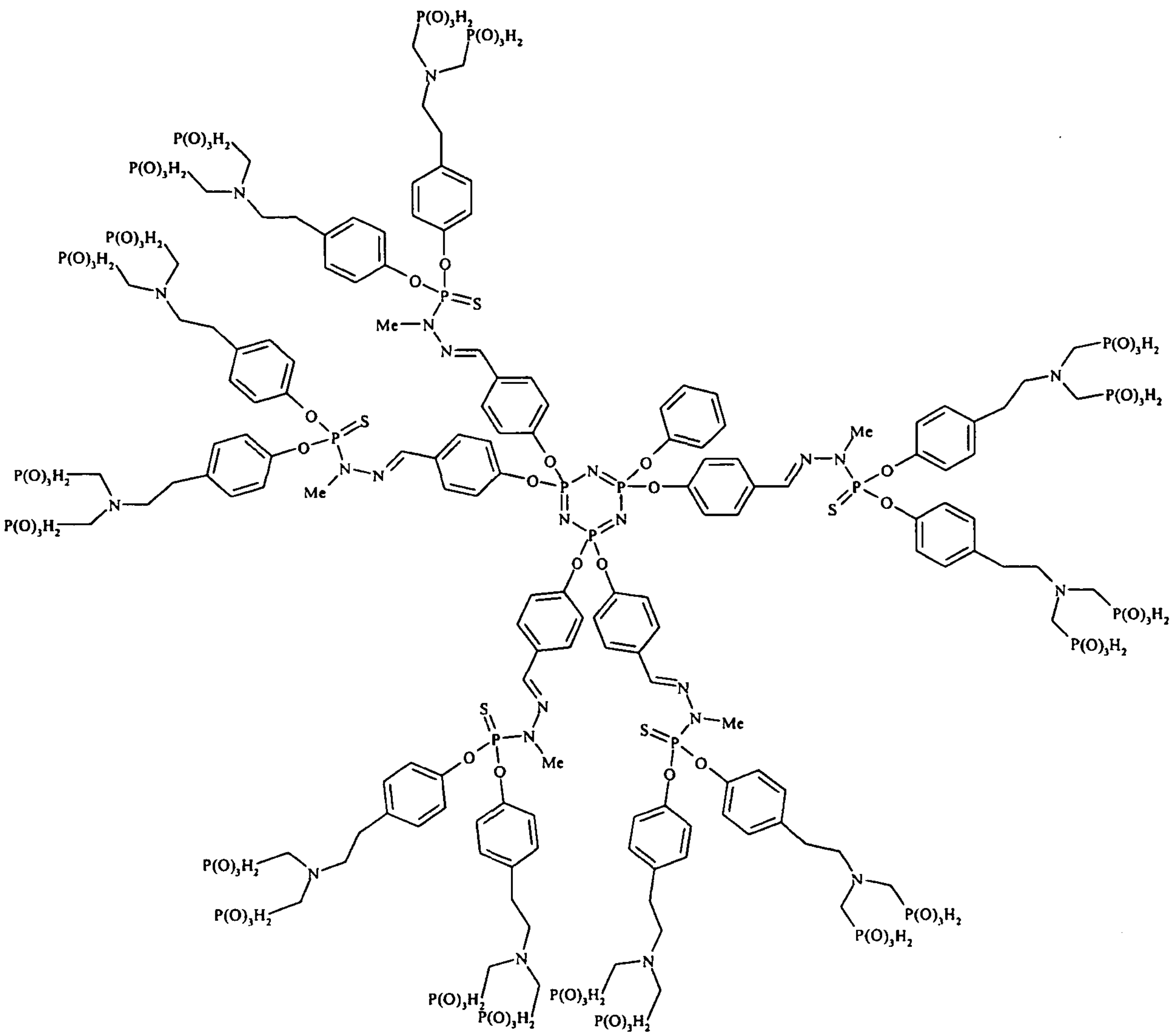

**(10)**

- ou des composés de formule suivante :

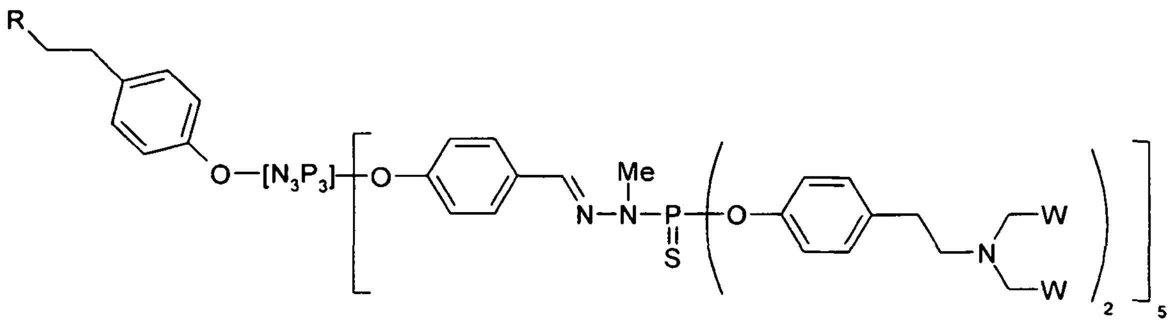

dans laquelle W représente $PO_3Me_2$, $PO_3HNa$, ou $PO_3H_2$ et R représente un groupe fluorescent choisi parmi :

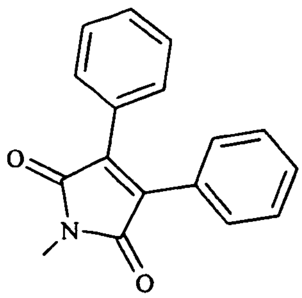 , 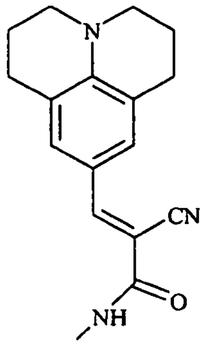 ,

lesdits composés correspondant notamment aux composés de formules suivantes :

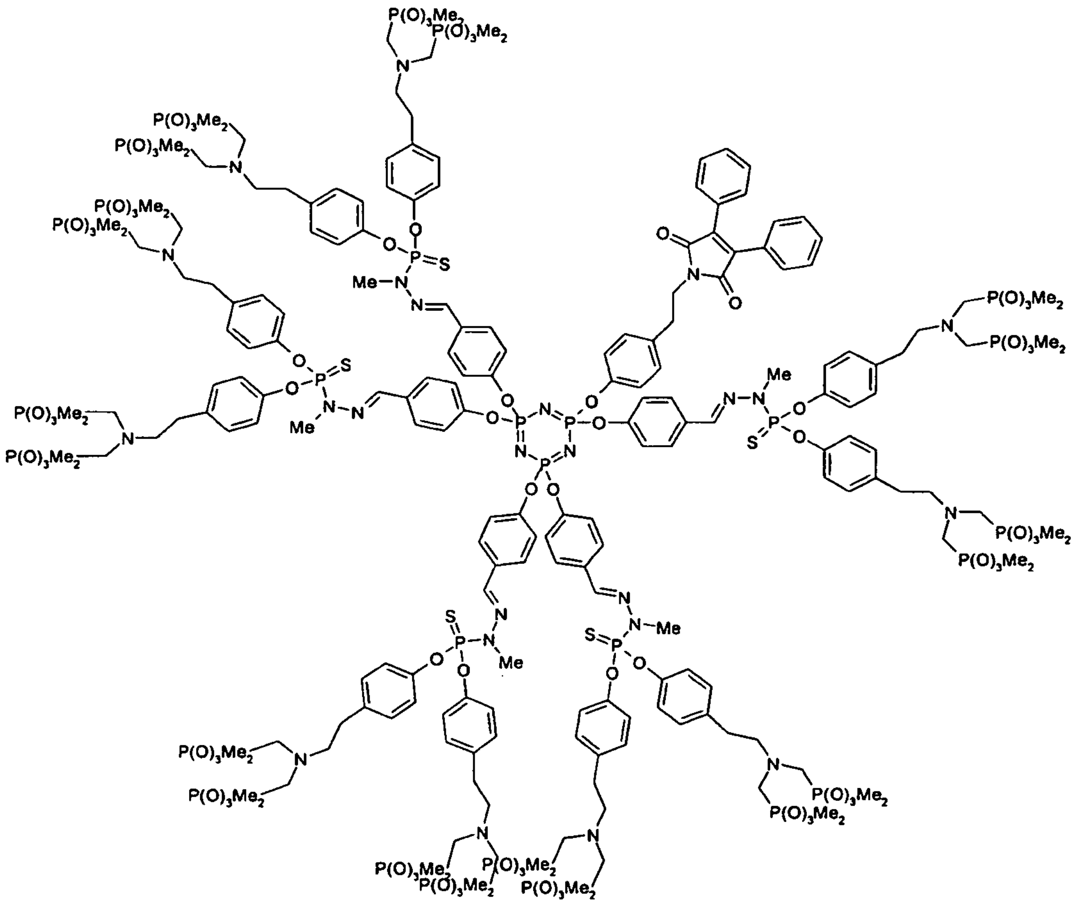

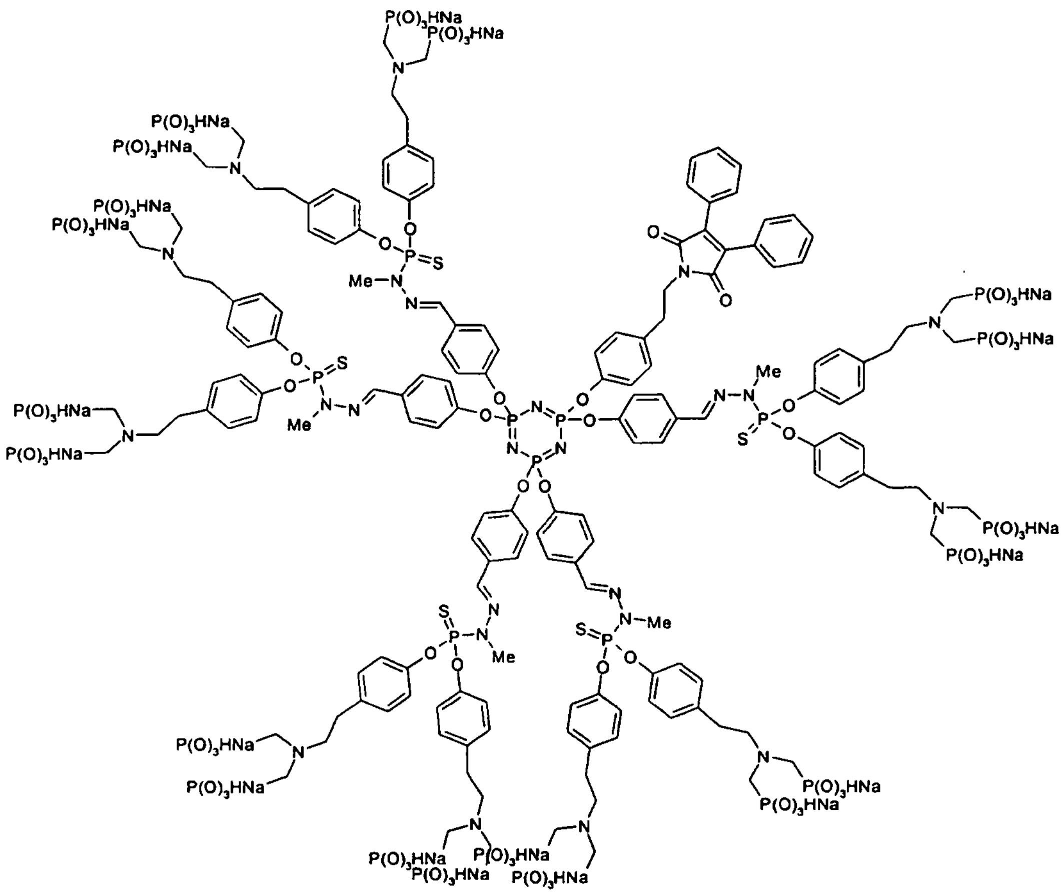

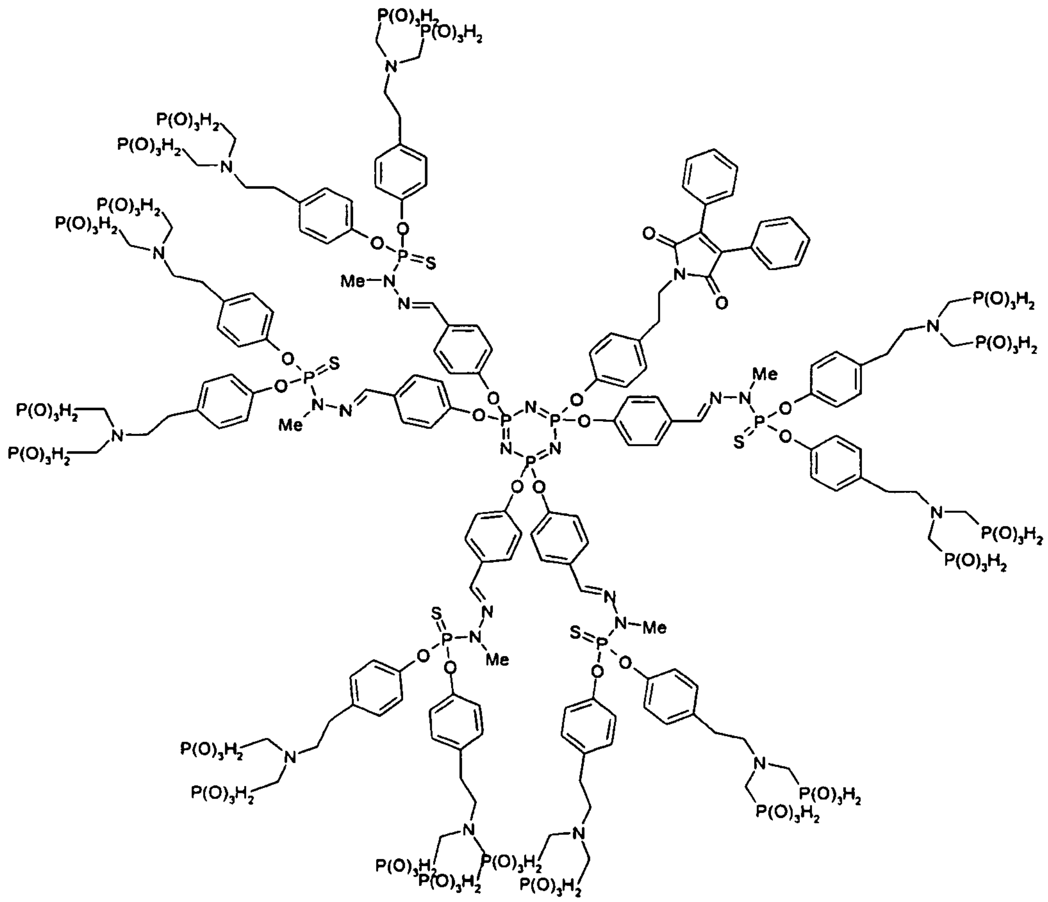

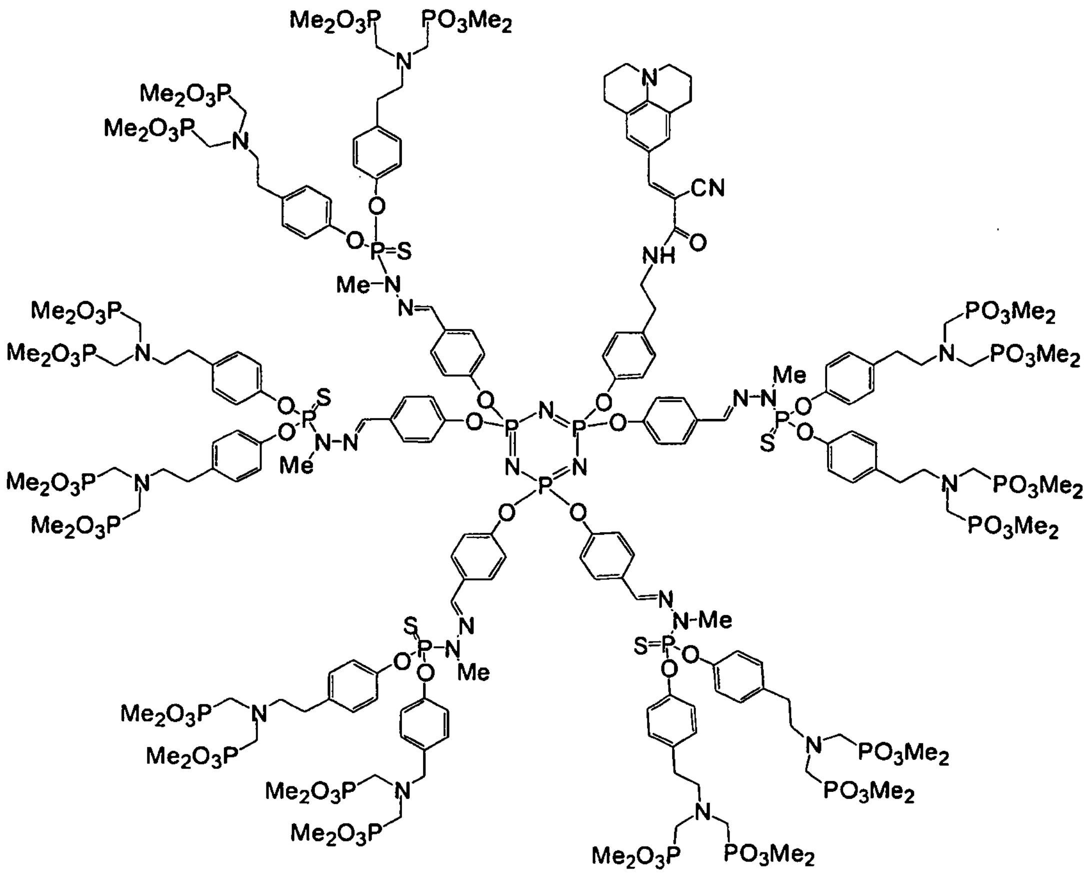

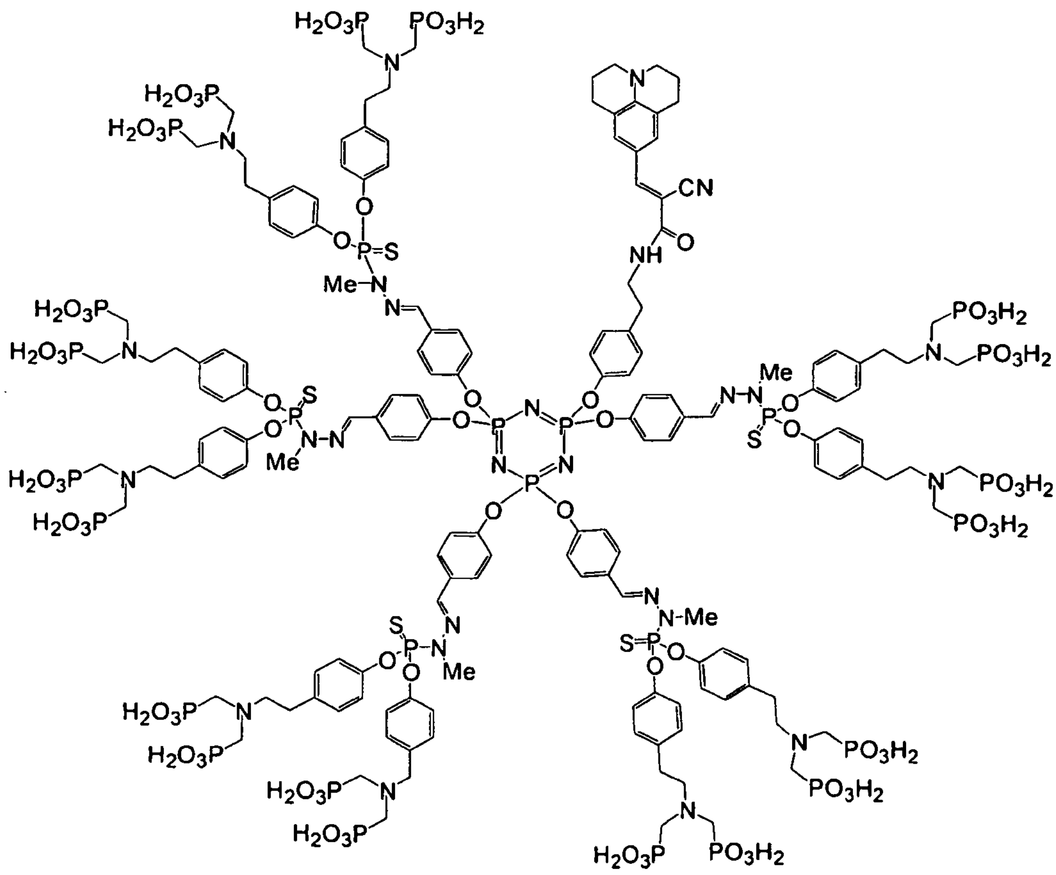

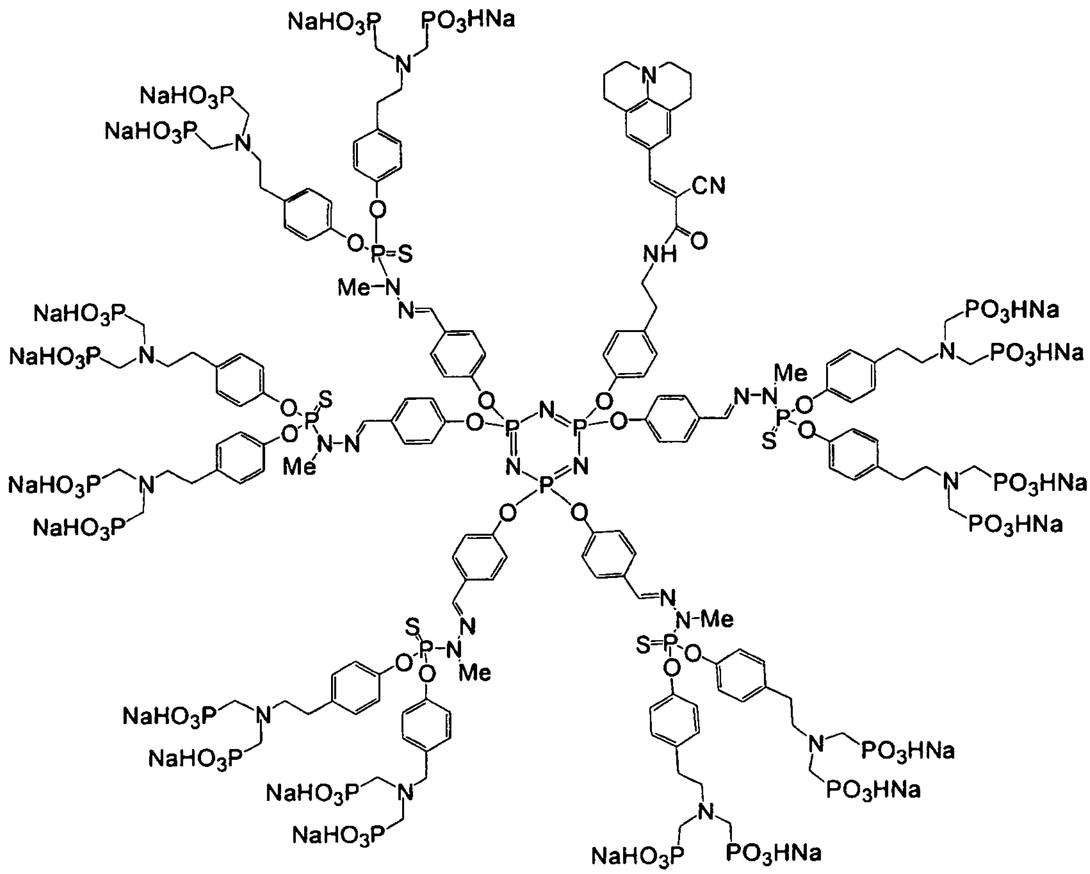

Avantageusement, ces derniers dendrimères sont fluorescents. Ils peuvent être notamment utilisés dans le cadre de la détection de cellules se liant aux dendrimères et/ou pour l'identification du site de liaison des dendrimères à la surface des cellules.

La présente invention concerne également l'utilisation telle que définie ci-dessus de dendrimères à terminaisons bisphophoniques de formule suivante :

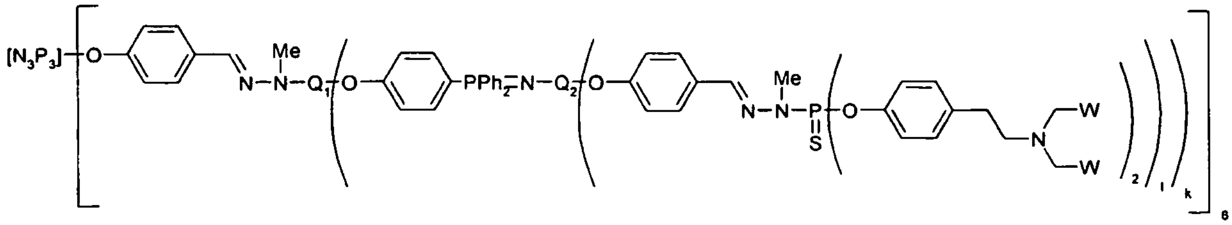

dans laquelle W représente $PO_3Me_2$, $PO_3HNa$, ou $PO_3H_2$, $Q_1$ et $Q_2$, identiques ou différents, représentent P=S ou le cyclotriphosphazène ($N_3P_3$), 1 représente 2 lorsque $Q_2$ représente P=S ou 5 lorsque $Q_2$ représente $N_3P_3$ et k représente 2 lorsque $Q_1$ représente P=S ou 5 lorsque $Q_1$ représente $N_3P_3$, lesdits dendrimères étant notamment représentés par les formules suivantes :

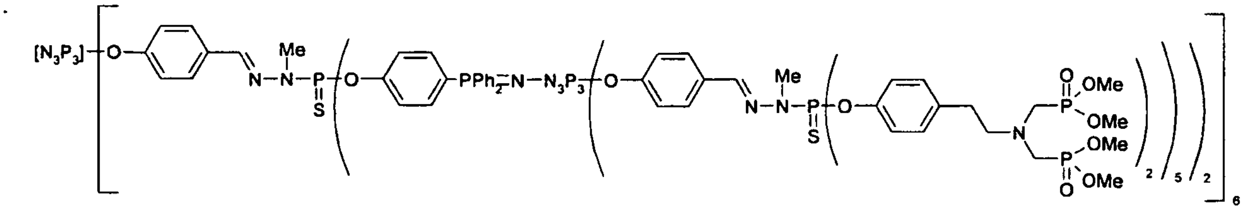

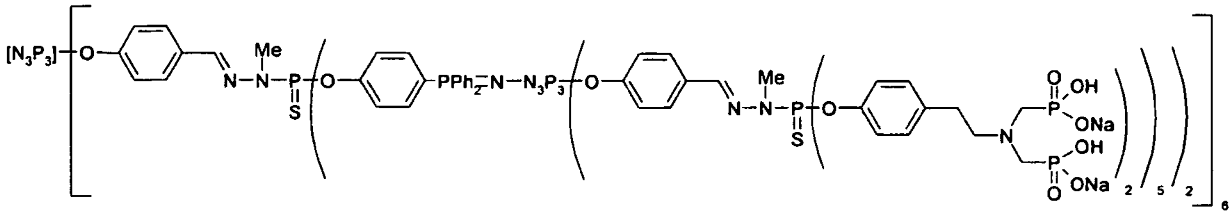

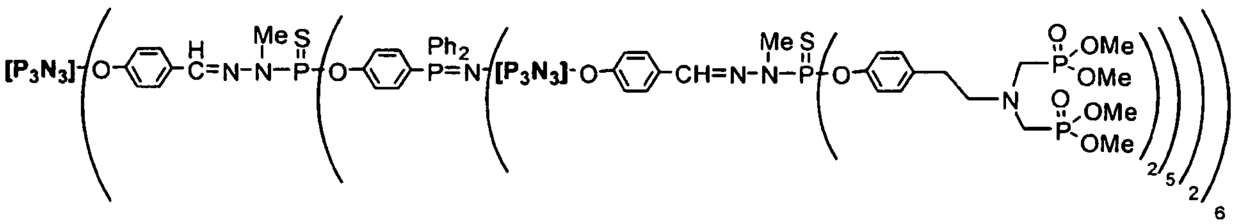

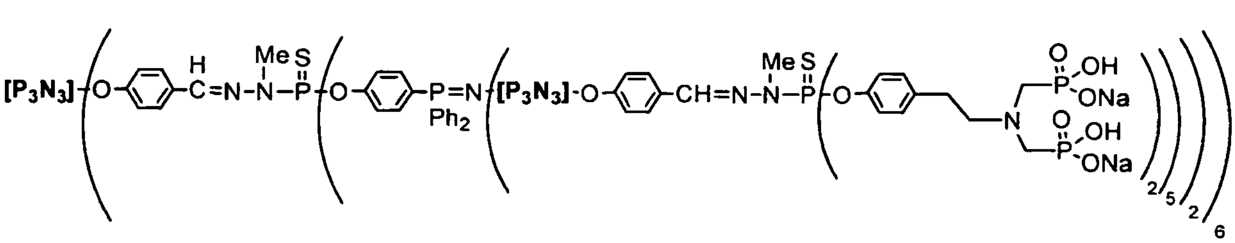

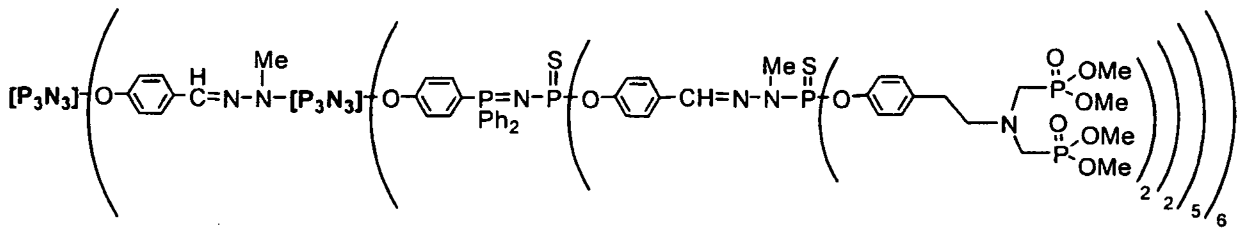

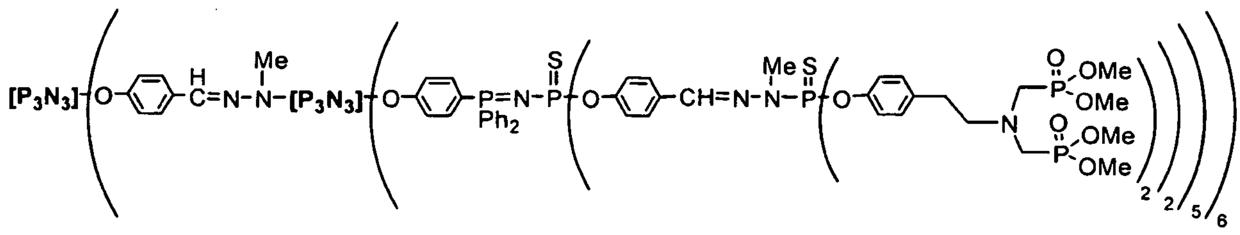

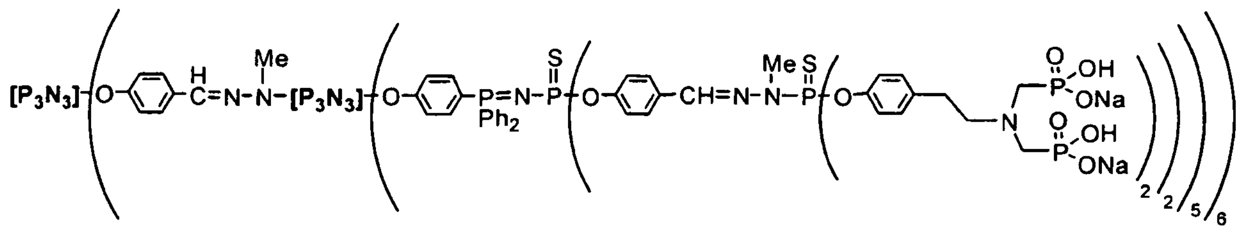

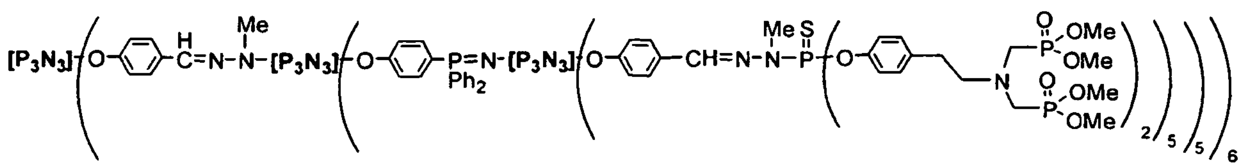

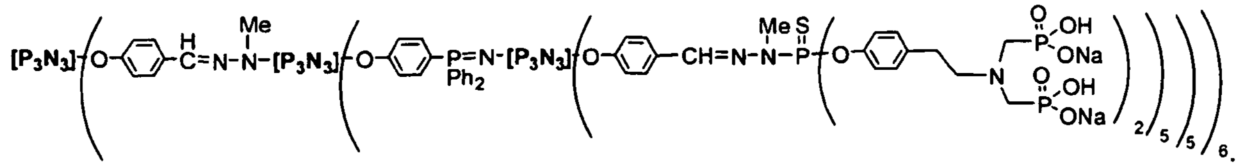

La présente invention concerne également l'utilisation telle que définie ci-dessus de dendrimères à terminaisons monophosphoniques ou bisphosphoniques de formule suivante :

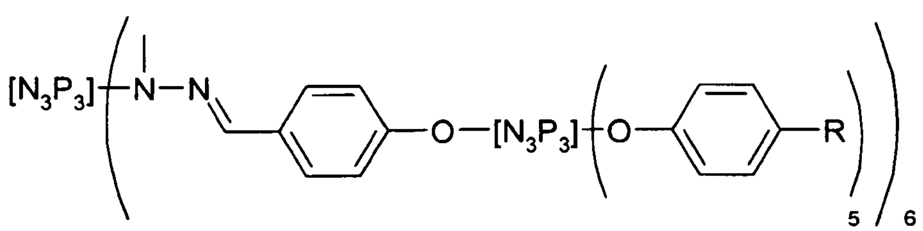

dans laquelle R représente un groupe choisi parmi

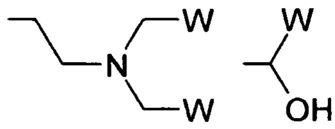

où W représente $PO_3Me_2$, $PO_3HNa$, ou $PO_3H_2$.

La présente invention concerne également l'utilisation telle que définie ci-dessus de dendrimères à terminaisons bisphosphoniques de formule suivante :

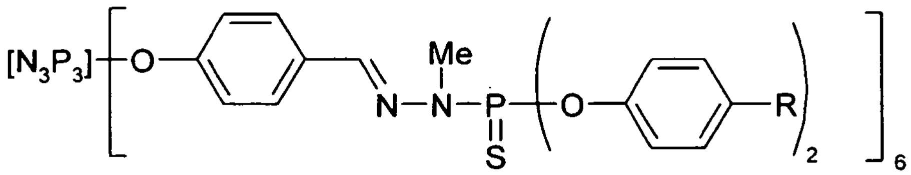

dans laquelle R représente un groupe choisi parmi :

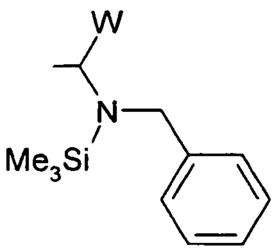 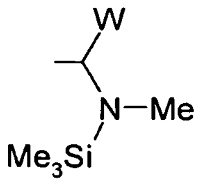 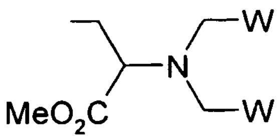 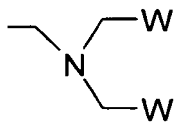

où W représente $PO_3Si_2Me_6$ $PO_3Me_2$, $PO_3HNa$, ou $PO_3H_2$.

La présente invention concerne également l'utilisation telle que définie ci-dessus de dendrimères à terminaisons monophosphoniques de formule suivante :

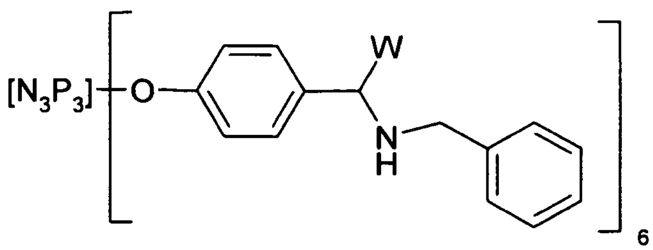

dans laquelle W représente $PO_3Me_2$, $PO_3HNa$, ou $PO_3H_2$.

La présente invention concerne également l'utilisation telle que définie ci-dessus de dendrimères à terminaisons bisphosphoniques de formule suivante :

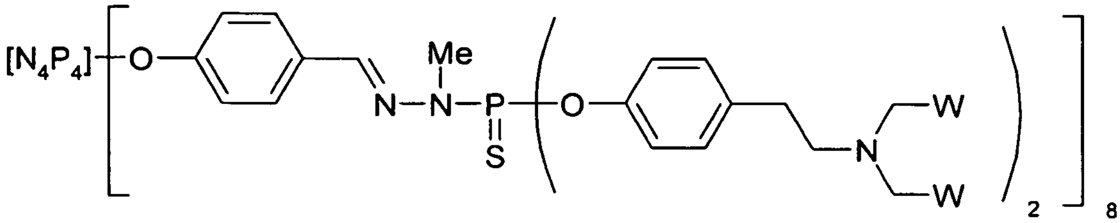

dans laquelle W représente $PO_3Me_2$, $PO_3HNa$, ou $PO_3H_2$.

La présente invention concerne également l'utilisation telle que définie ci-dessus de dendrimères à terminaisons bisphosphoniques de formule suivante :

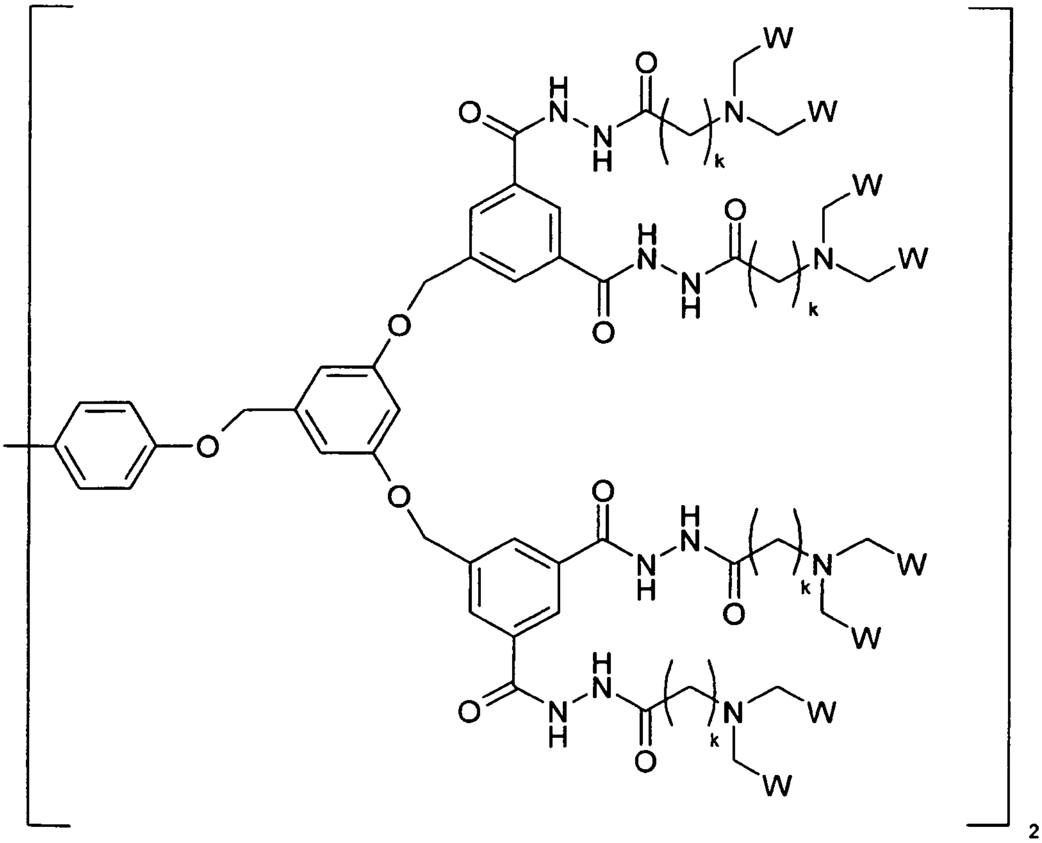

dans laquelle W représente $PO_3Me_2$, $PO_3HNa$, ou $PO_3H_2$ et k représente 1, 2 ou 3

La présente invention concerne également l'utilisation telle que définie ci-dessus de dendrimères à terminaisons bisphosphoniques de formule suivante :

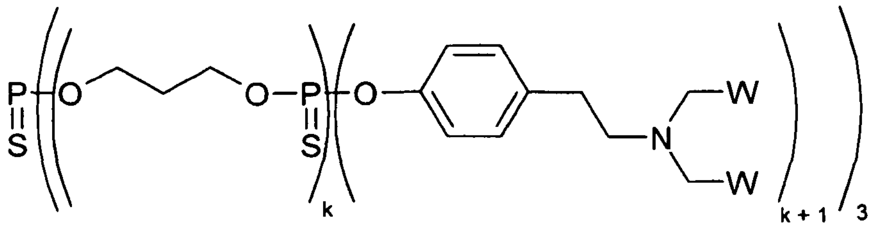

dans laquelle W représente $PO_3Me_2$, $PO_3HNa$, ou $PO_3H_2$ et k représente 0 ou 1.

La présente invention concerne également l'utilisation telle que définie ci-dessus de dendrimères à terminaisons bisphosphoniques de formule suivante :

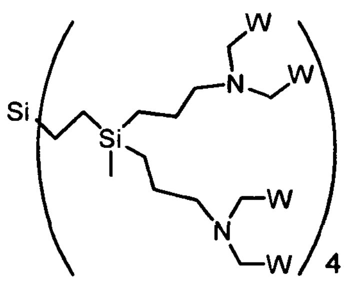

dans laquelle W représente $PO_3Me_2$, $PO_3HNa$ ou $PO_3H_2$.

La présente invention concerne également l'utilisation telle que définie ci-dessus de dendrimères à terminaisons bisphosphoniques de formule suivante :

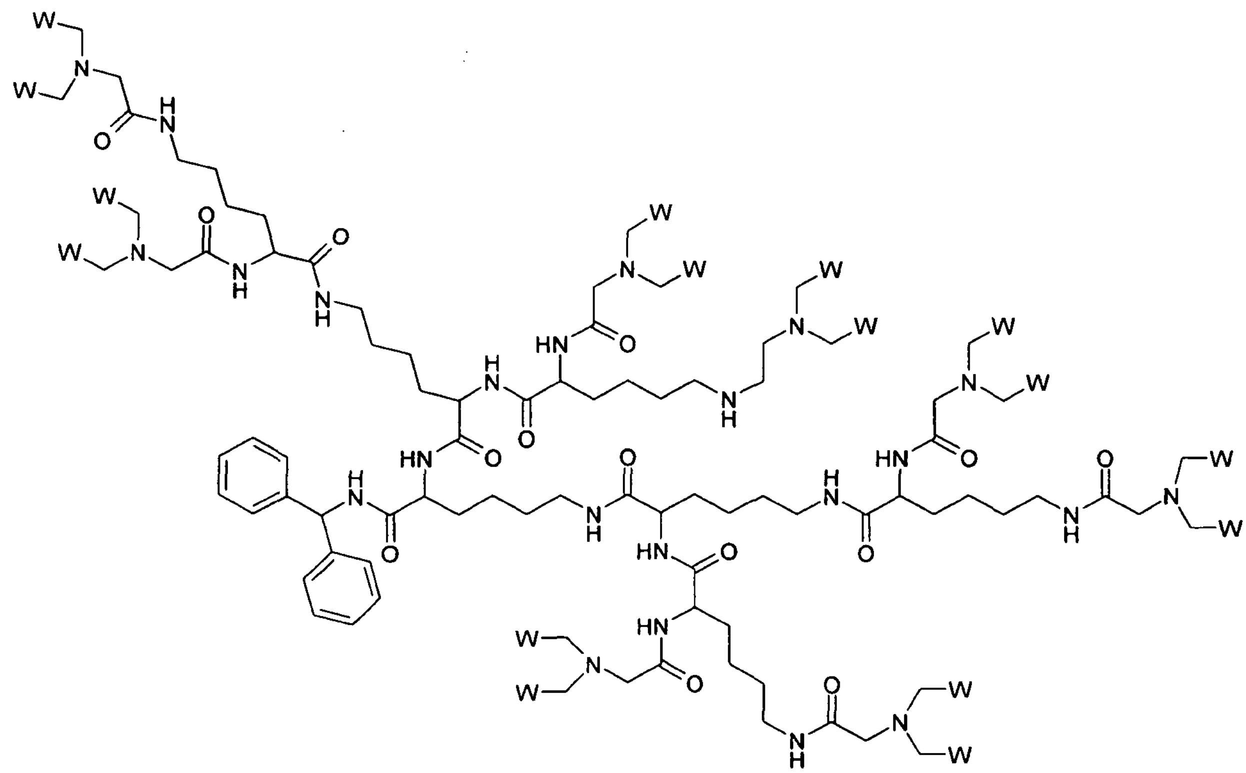

dans laquelle W représente $PO_3Me_2$, $PO_3HNa$, ou $PO_3H_2$.

De manière particulière, la présente invention concerne notamment l'utilisation, telle que définie ci-dessus, des composés de formules suivantes :

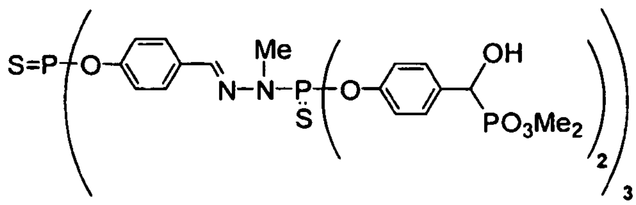

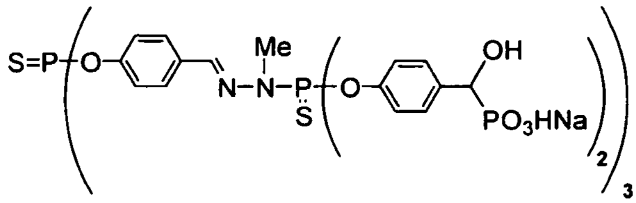

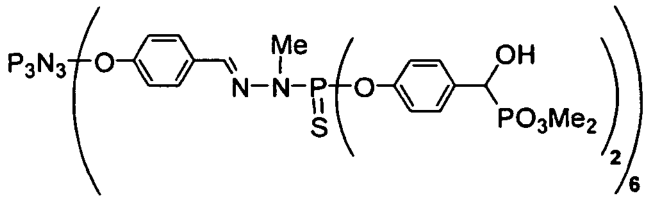

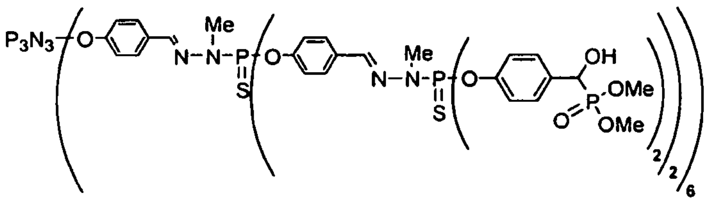

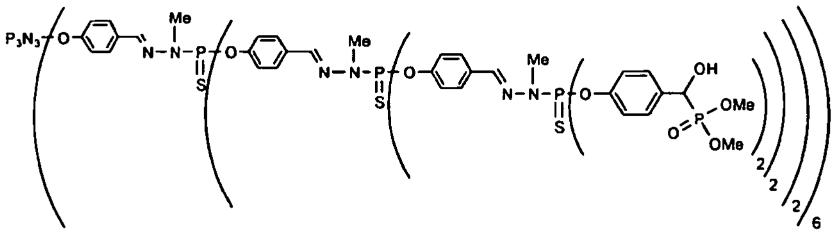

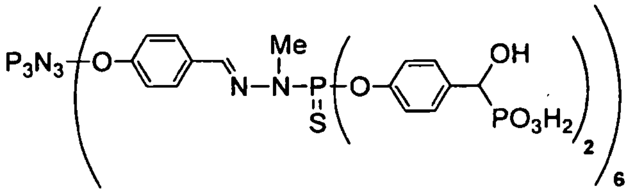

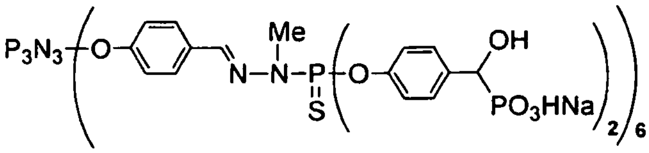

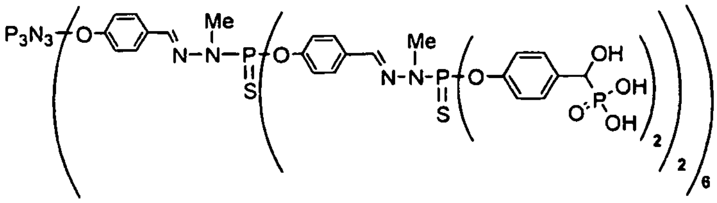

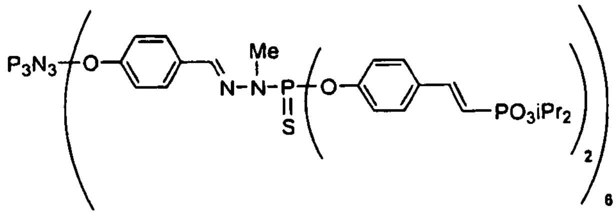

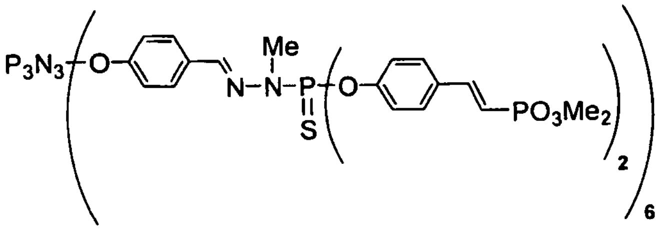

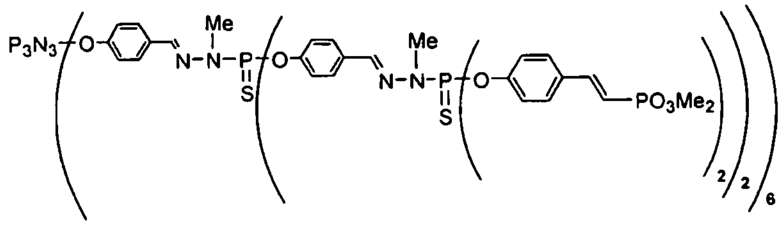

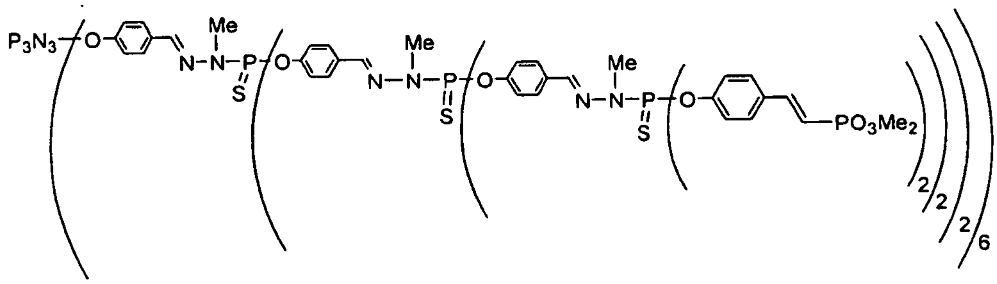

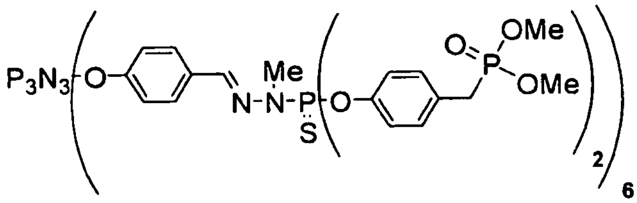

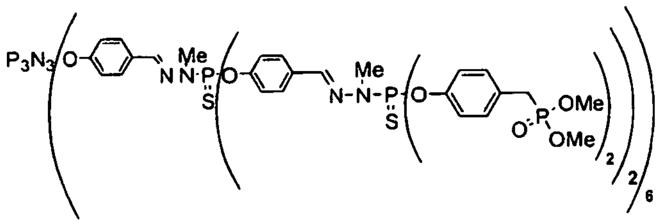

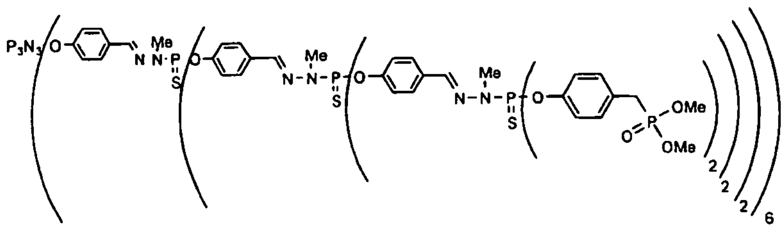

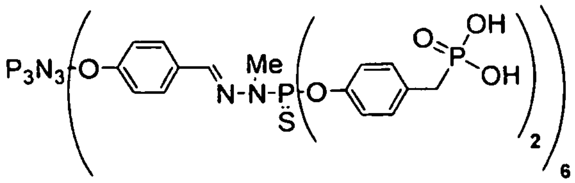

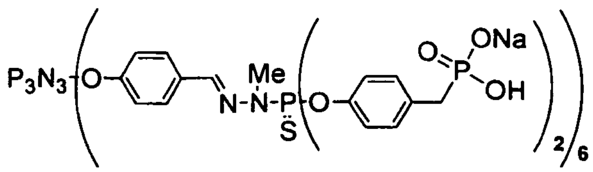

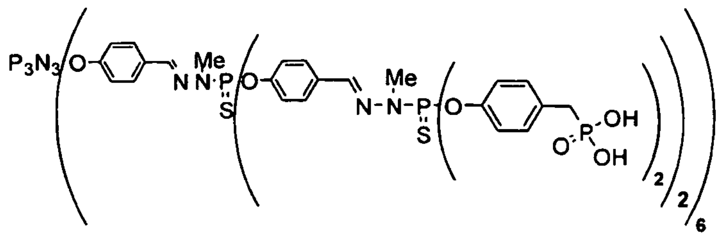

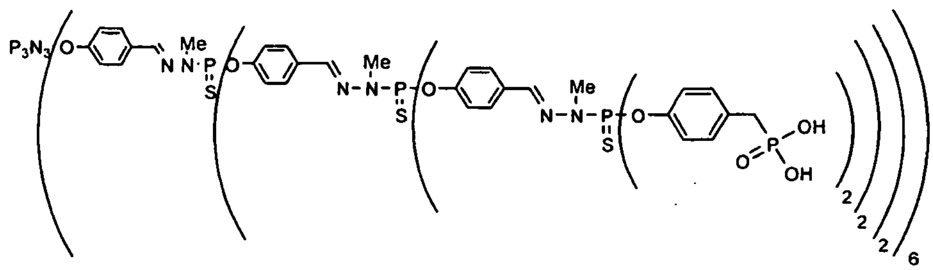

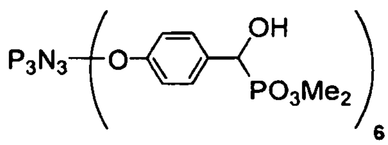

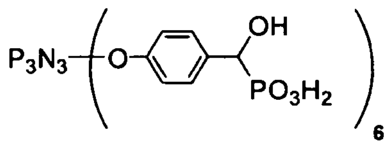

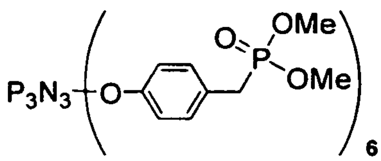

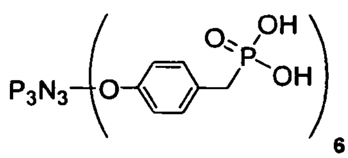

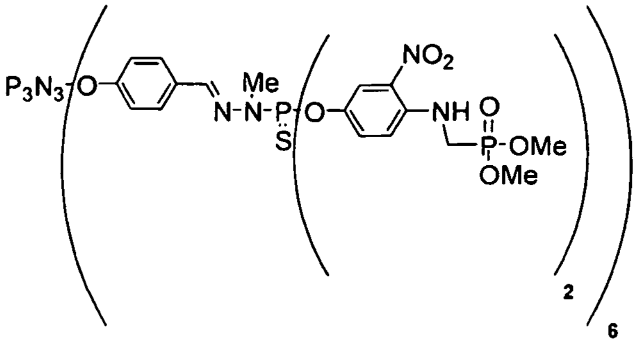

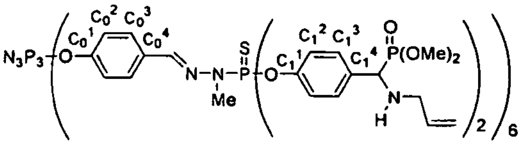

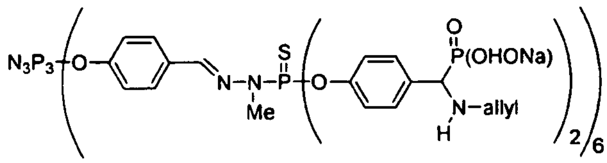

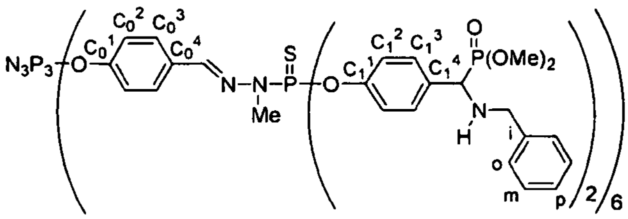

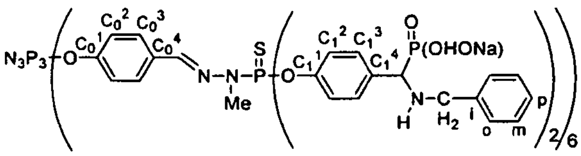

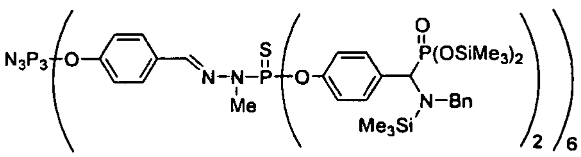

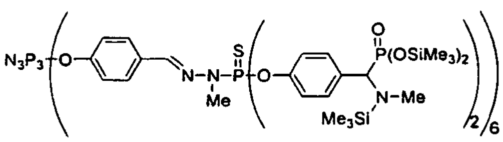

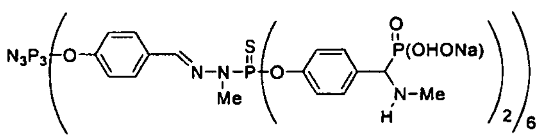

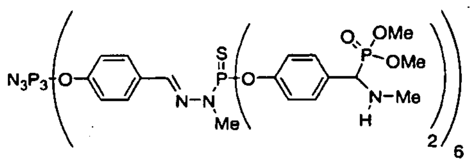

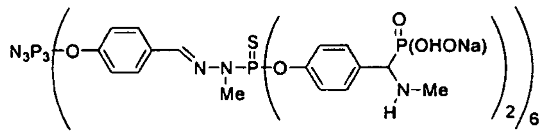

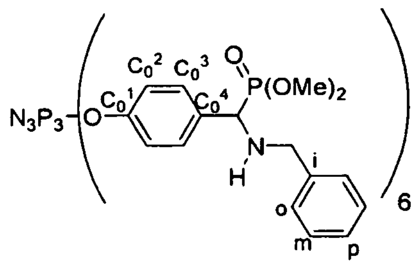

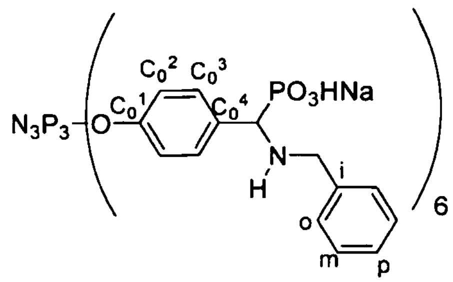

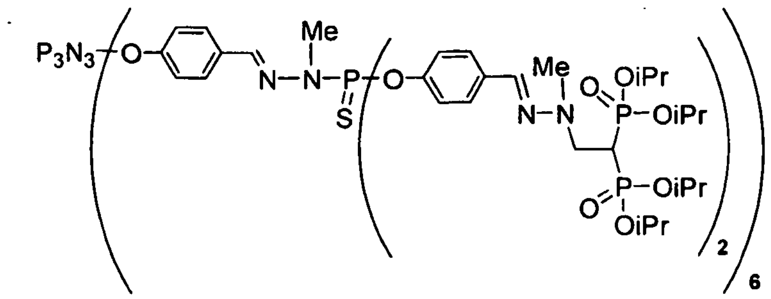

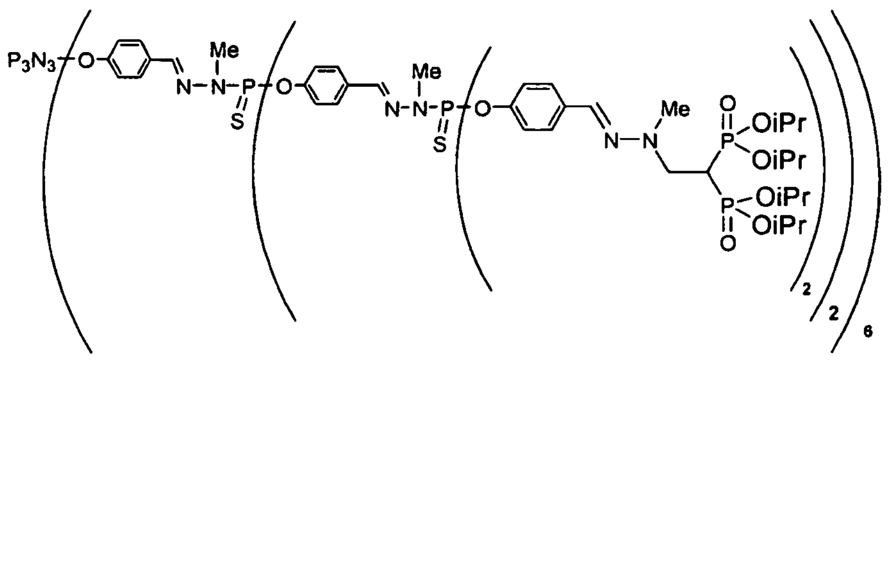

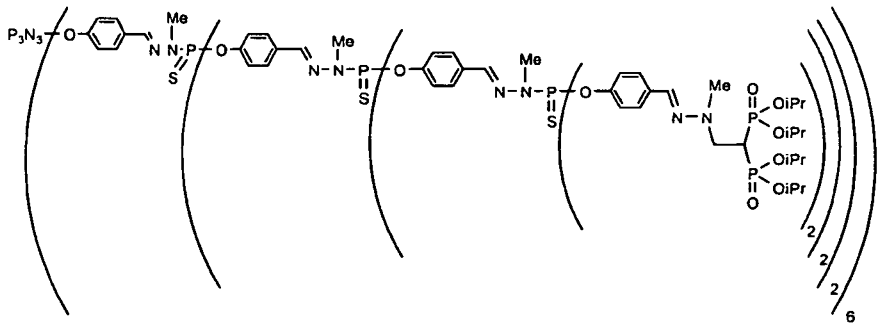

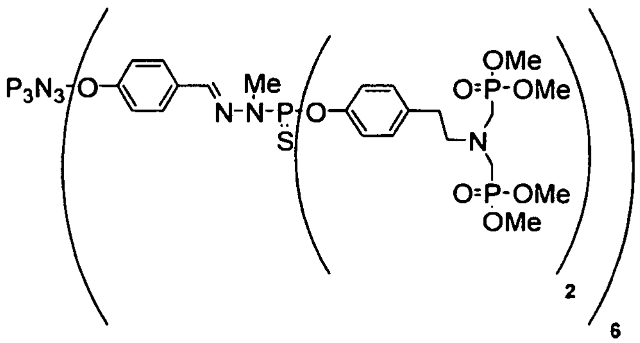

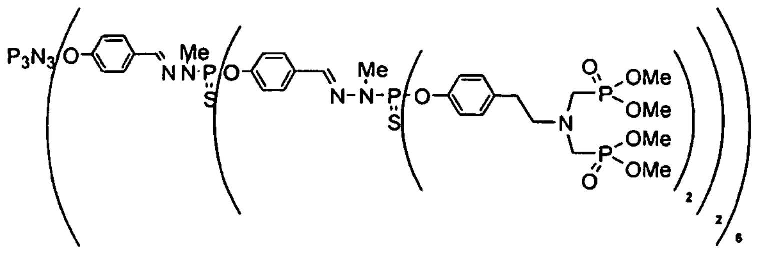

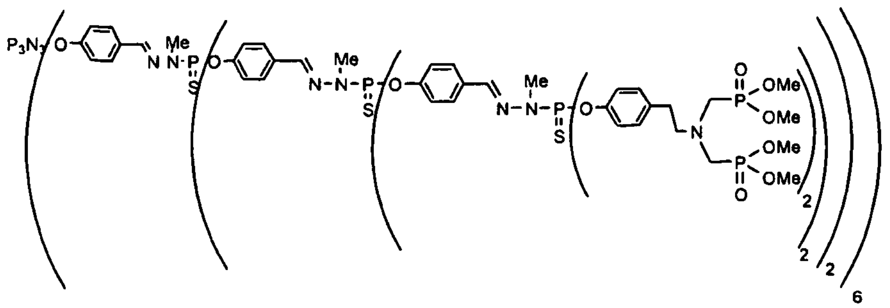

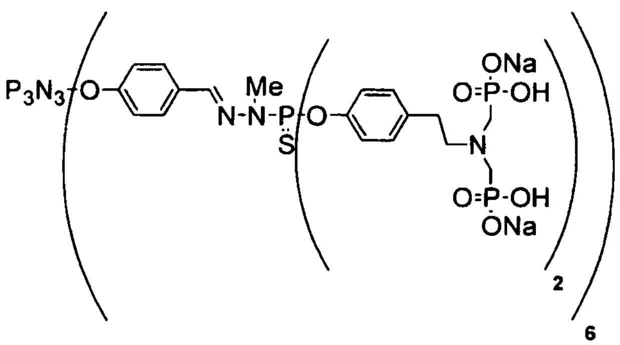

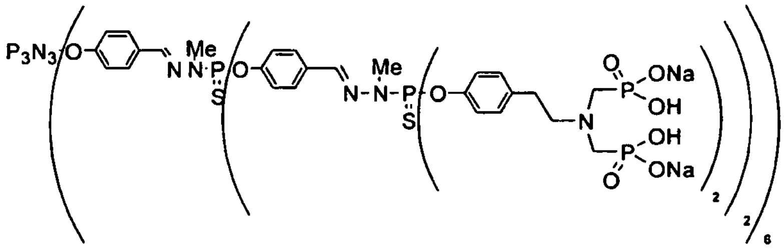

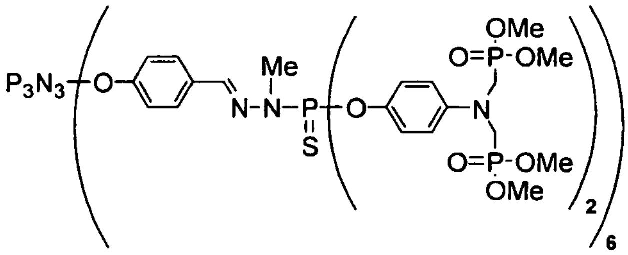

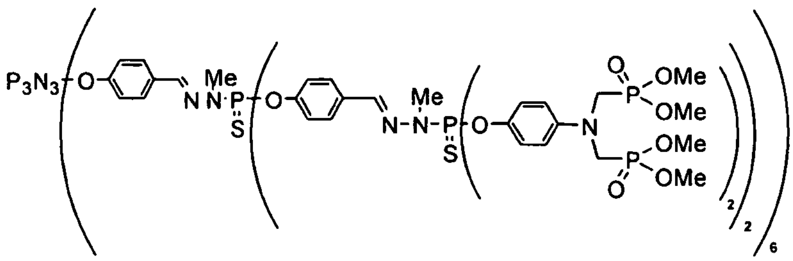

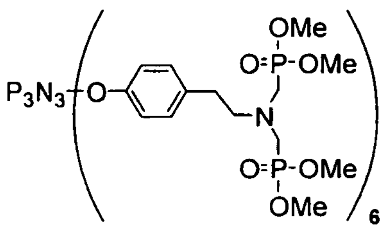

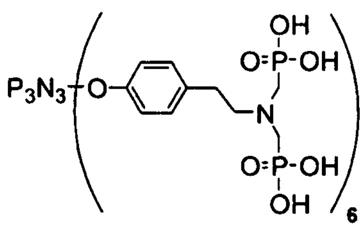

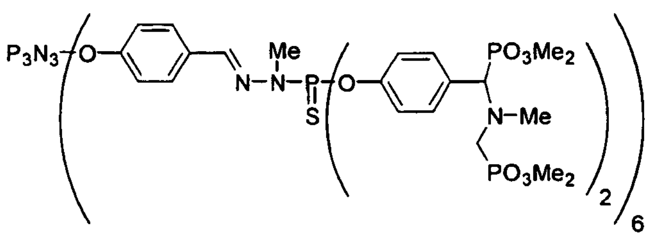

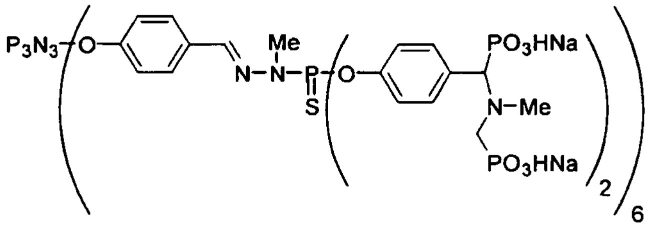

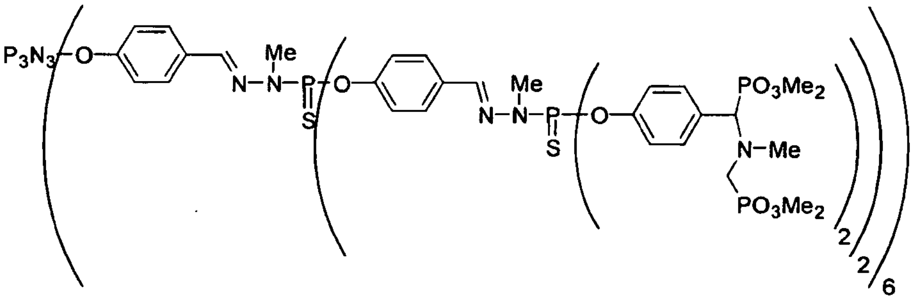

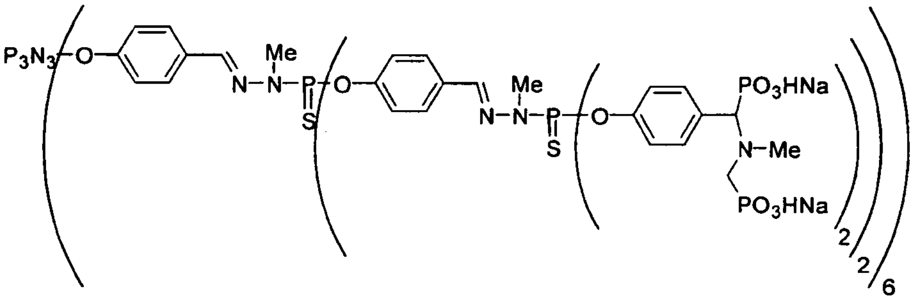

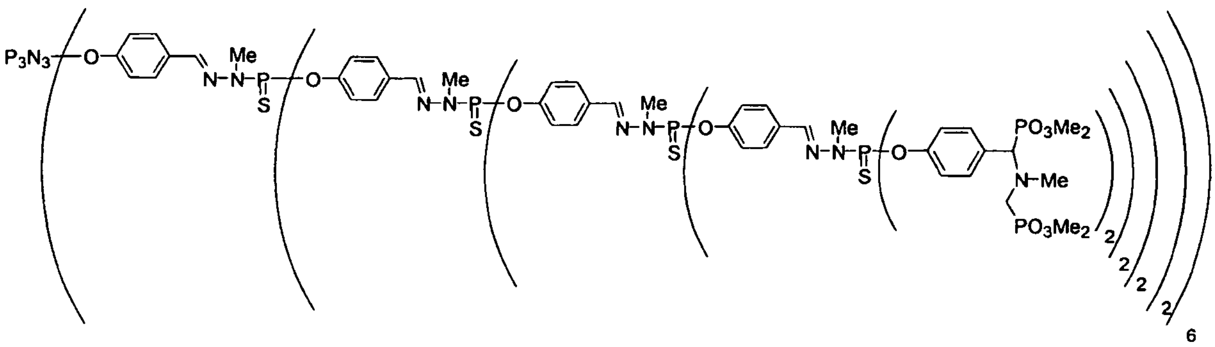

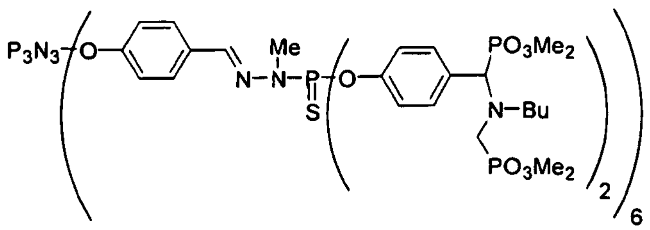

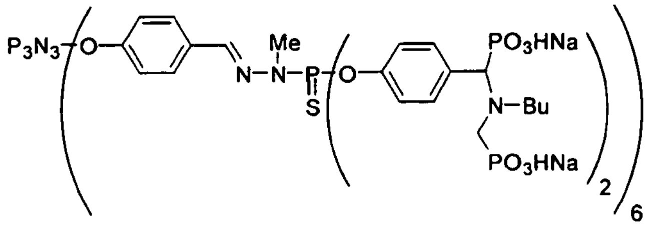

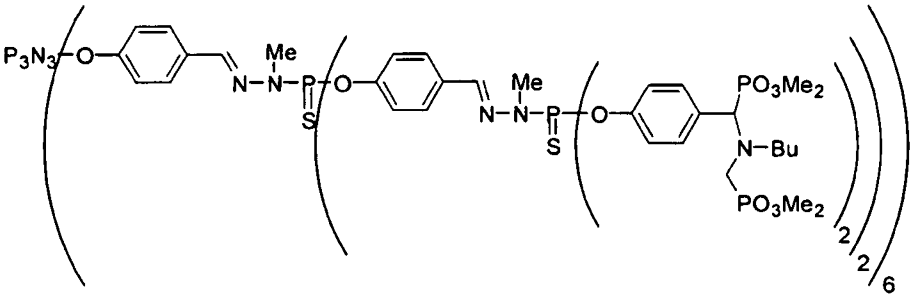

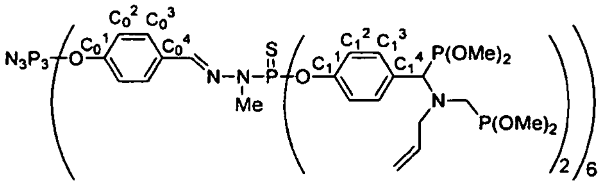

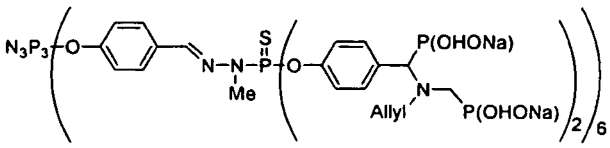

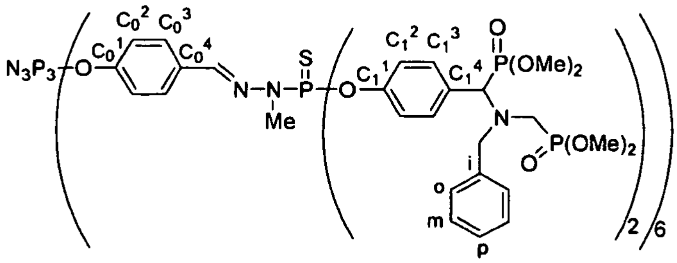

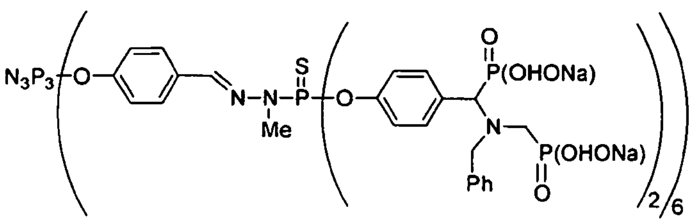

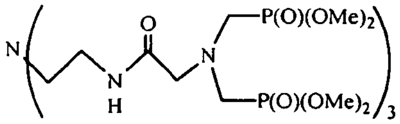

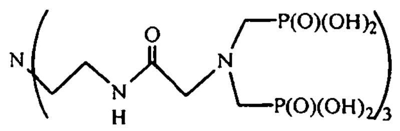

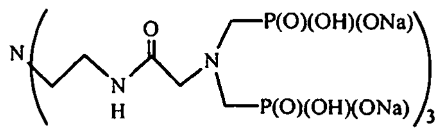

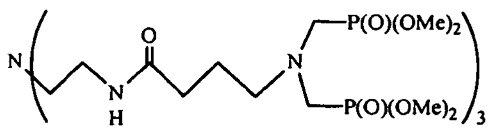

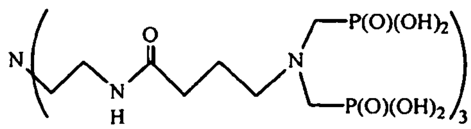

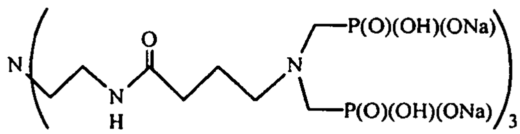

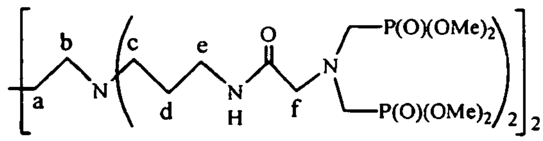

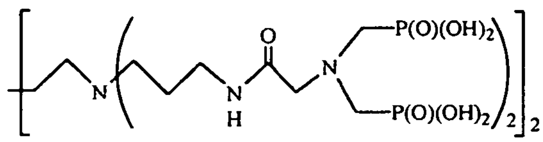

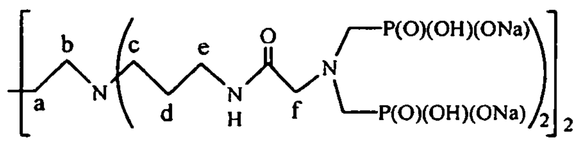

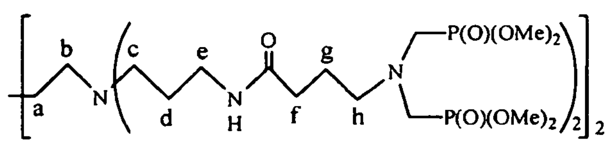

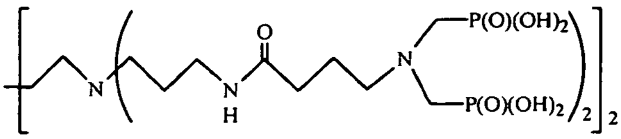

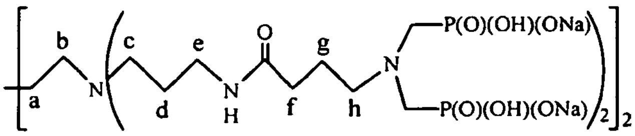

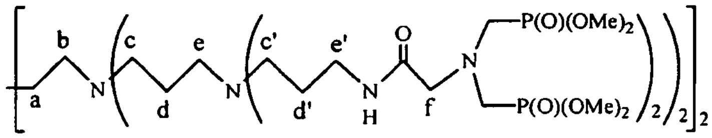

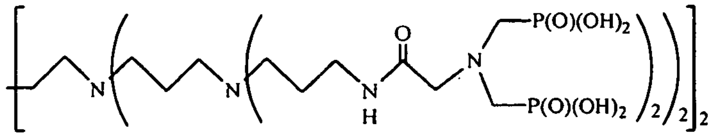

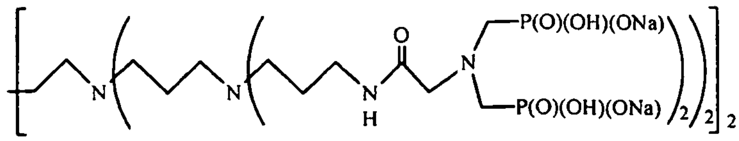

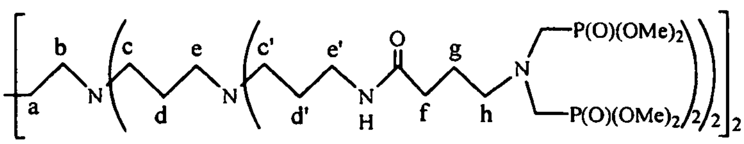

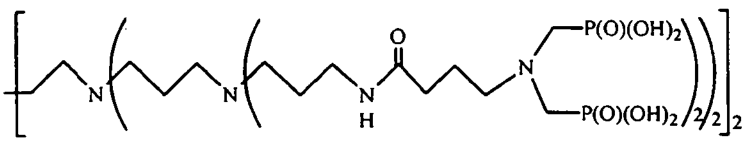

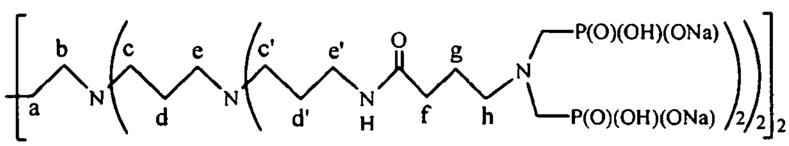

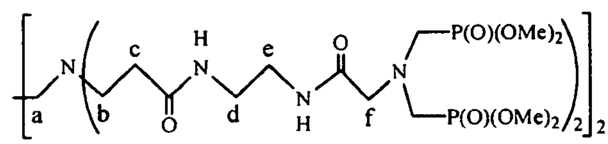

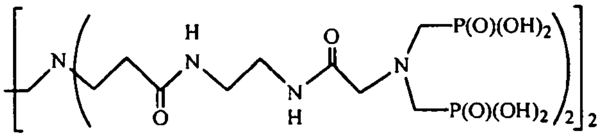

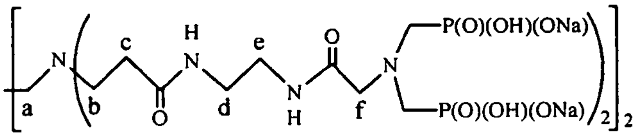

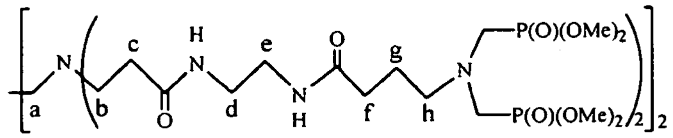

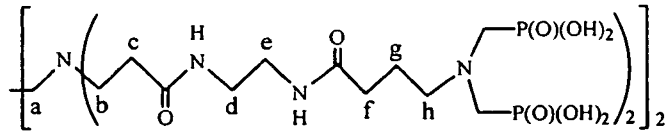

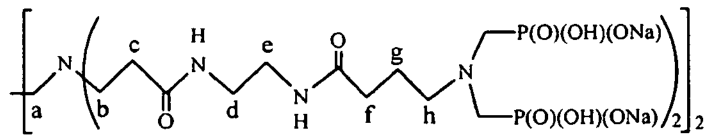

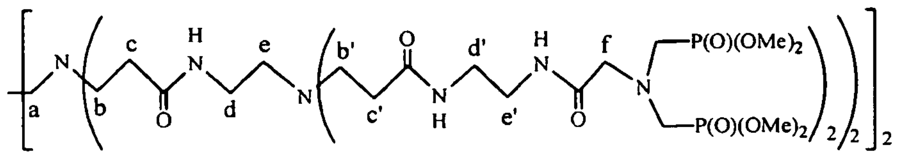

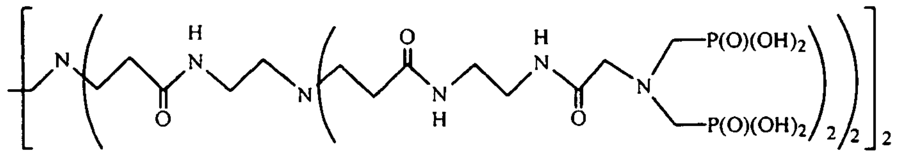

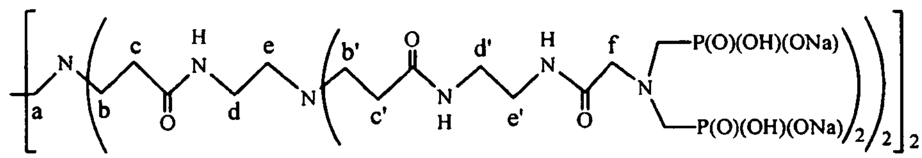

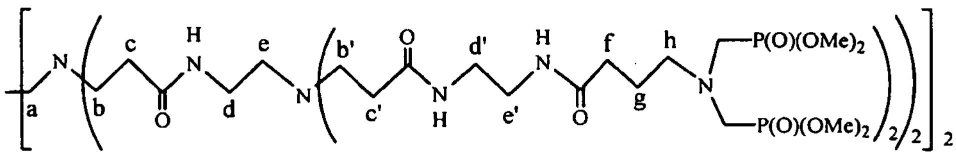

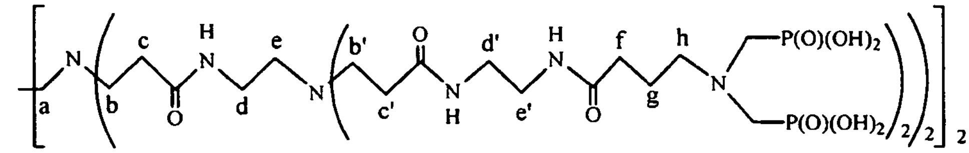

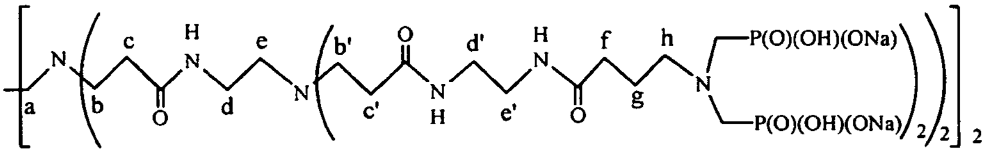

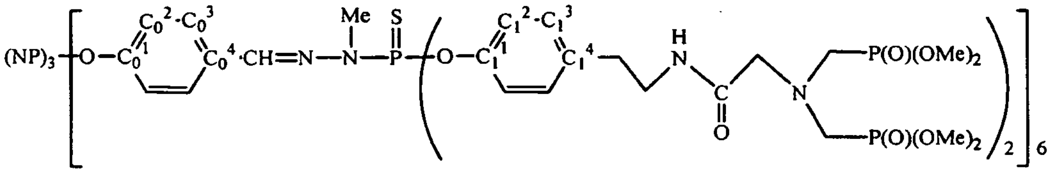

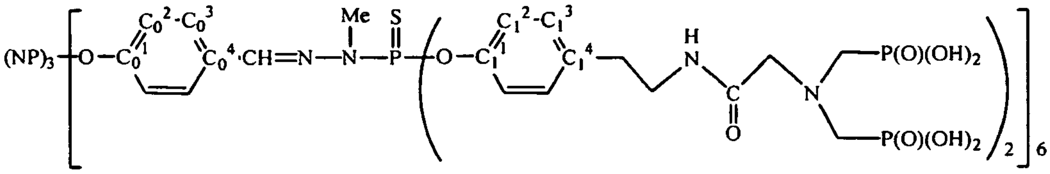

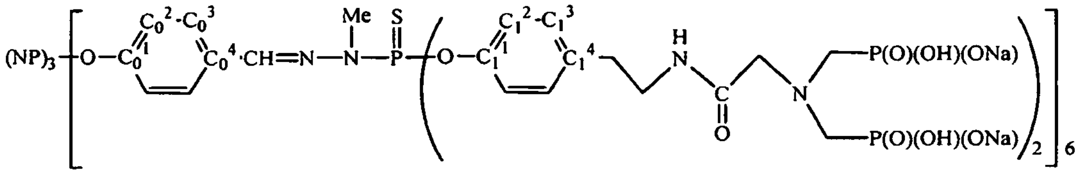

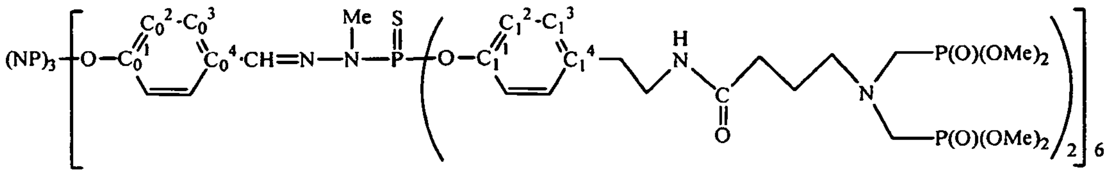

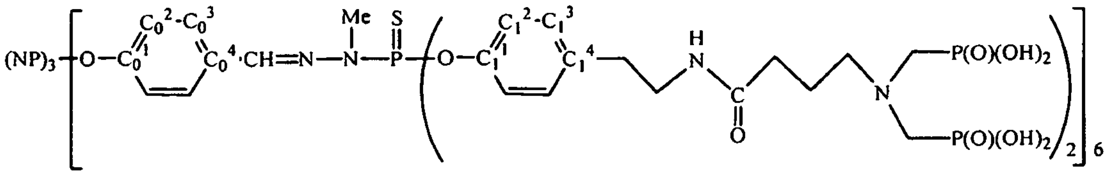

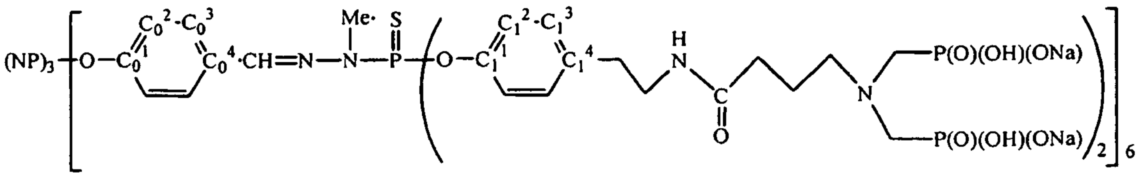

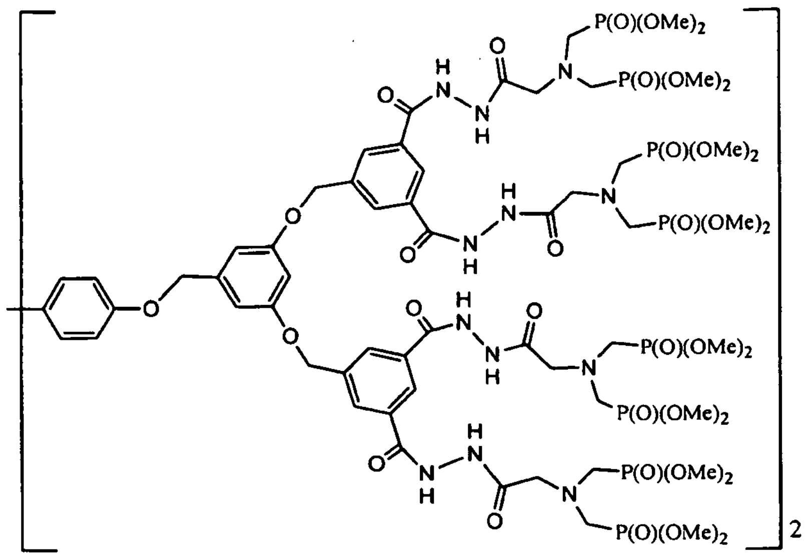

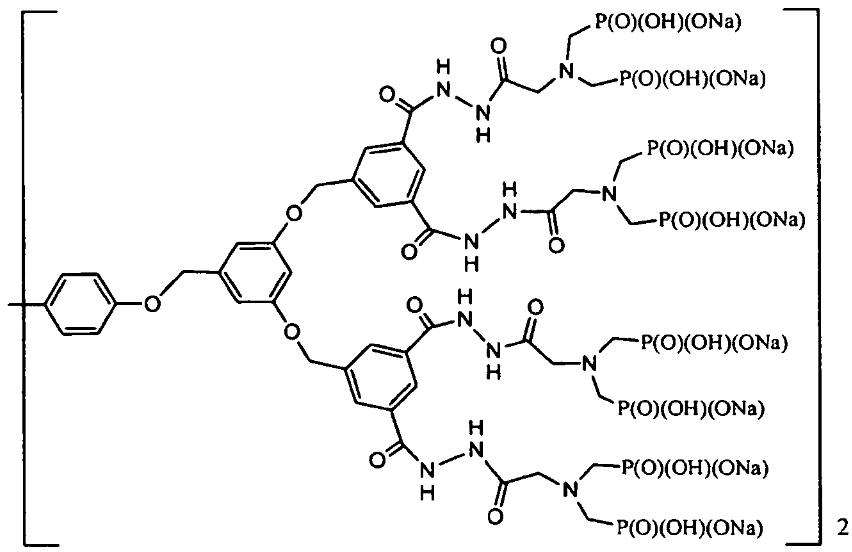

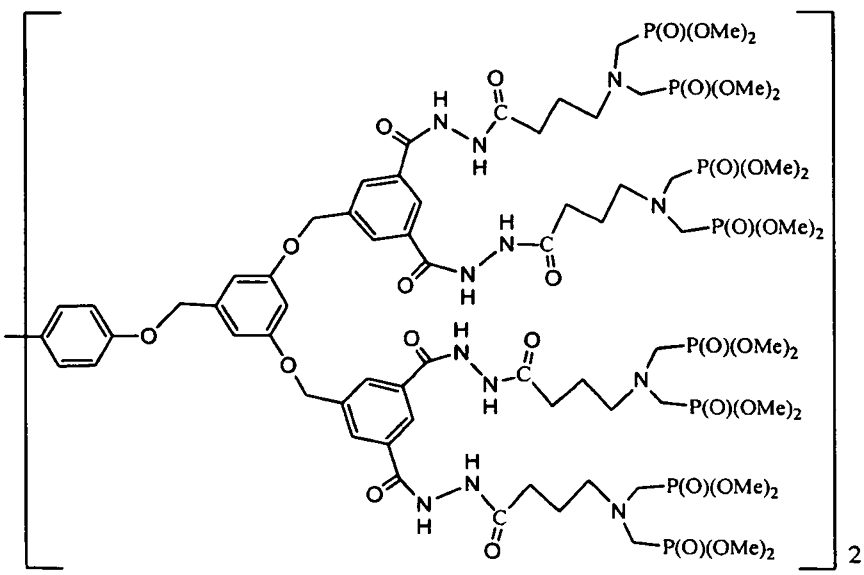

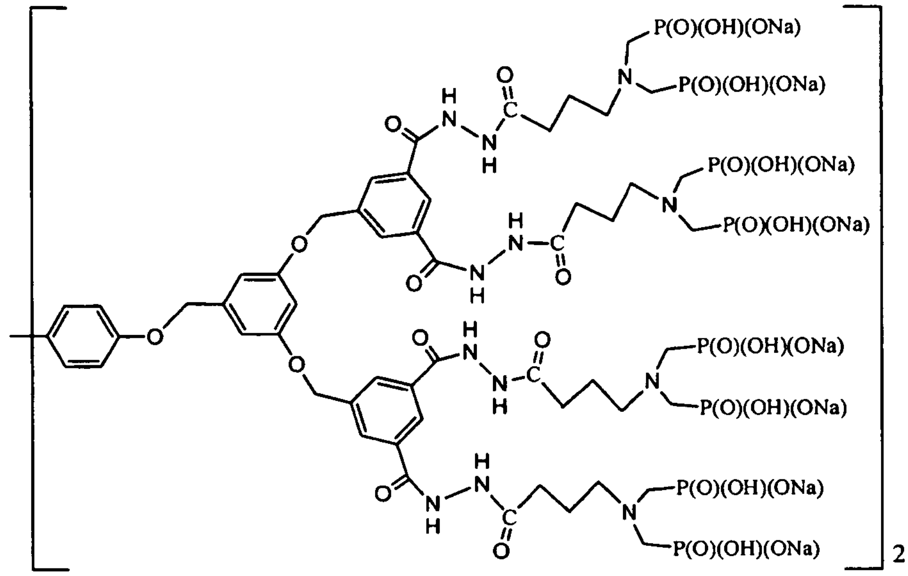

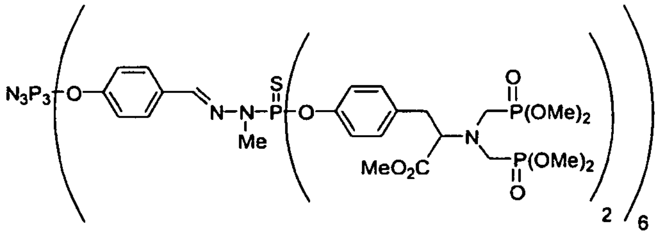

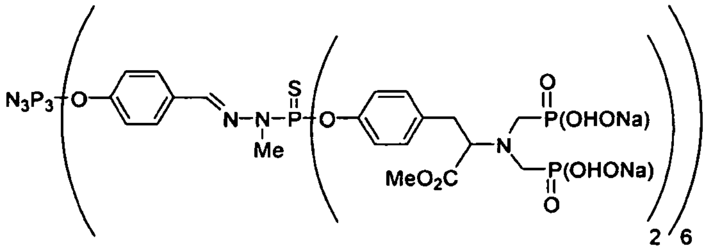

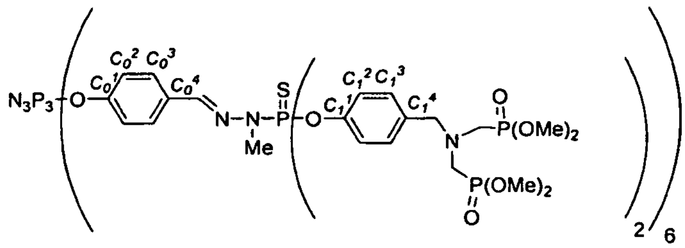

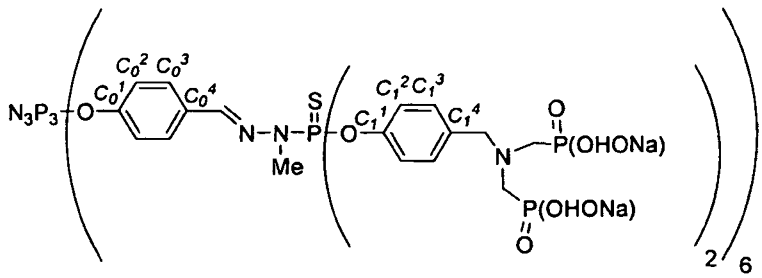

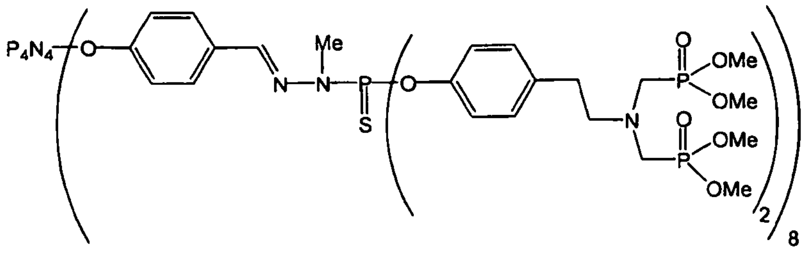

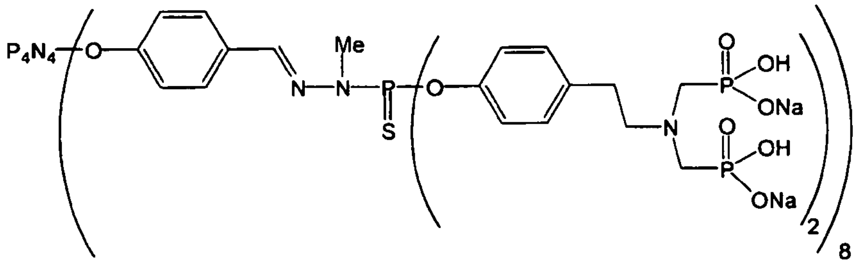

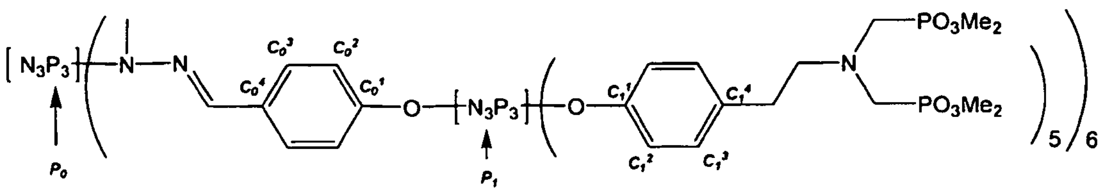

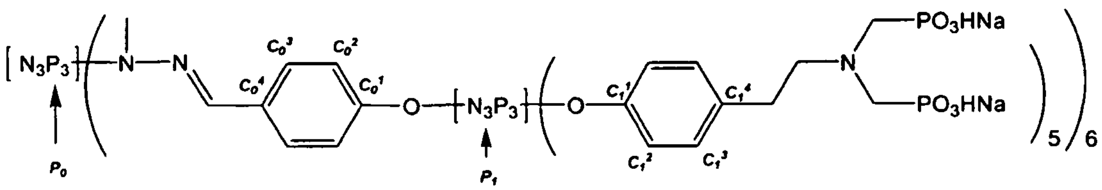

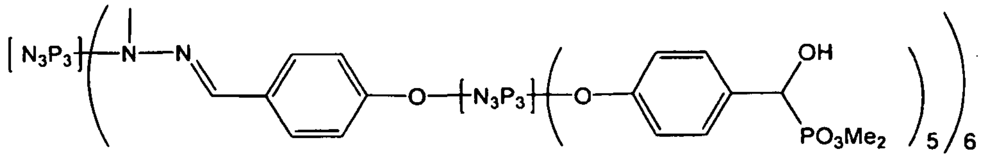

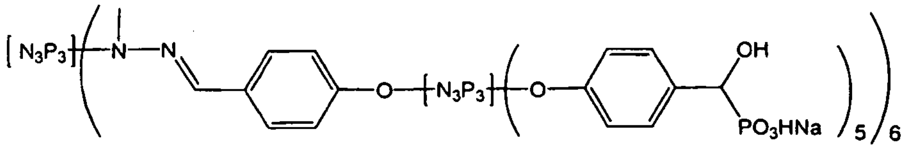

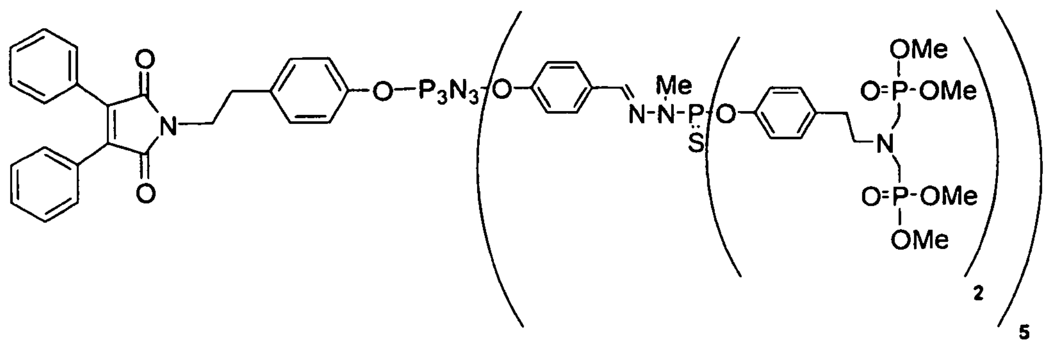

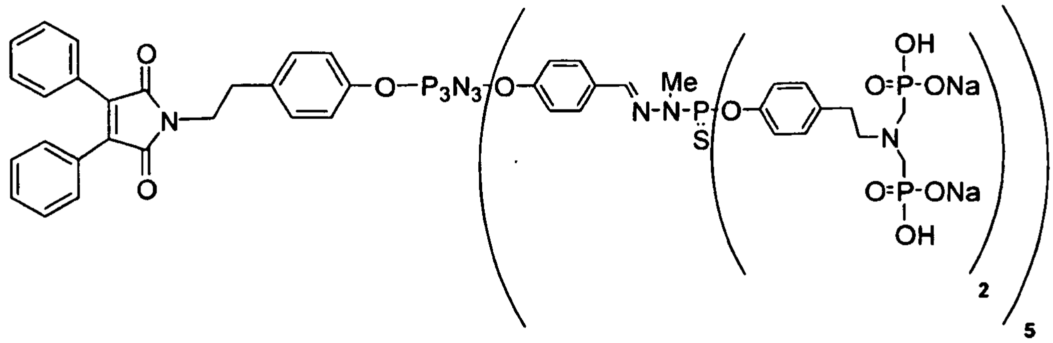

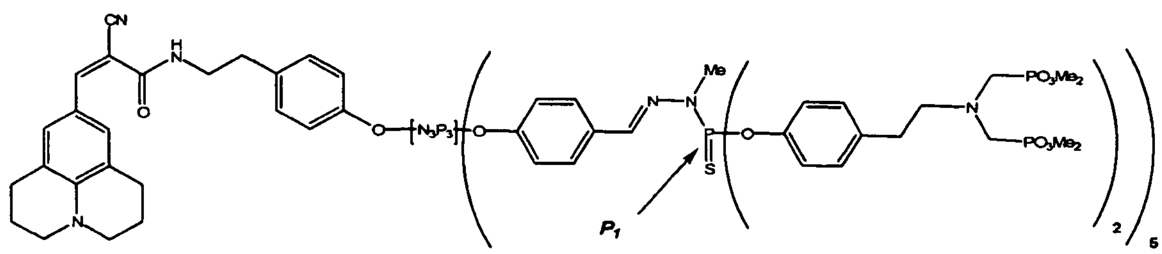

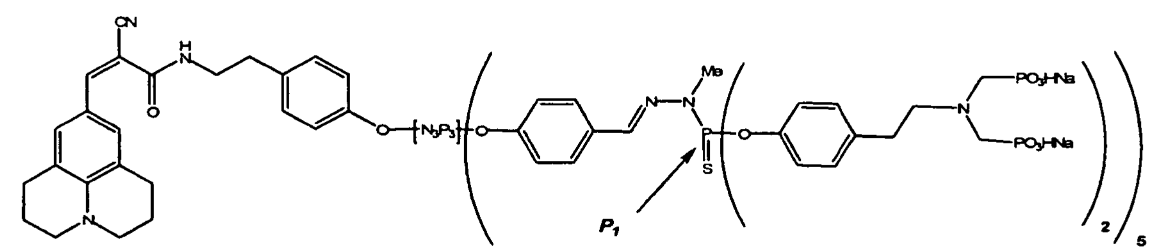

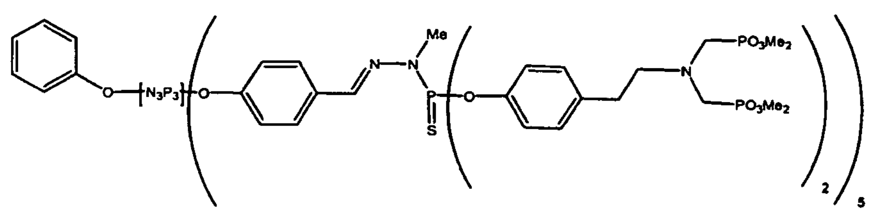

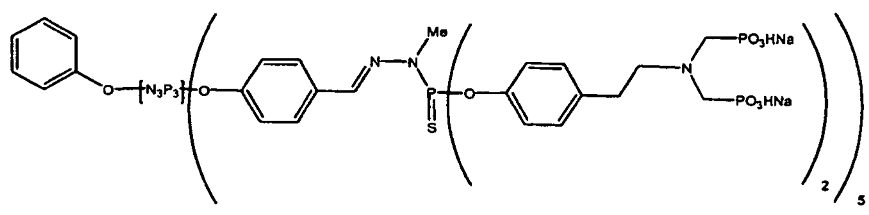

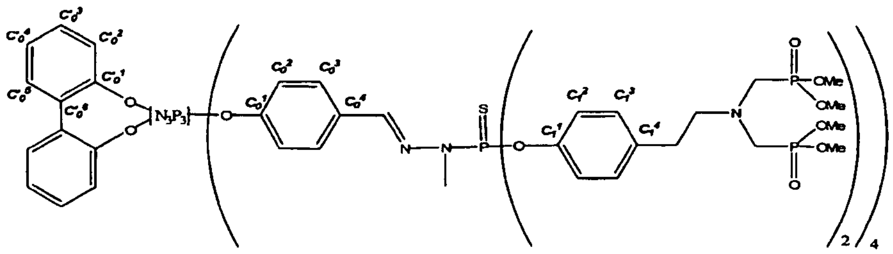

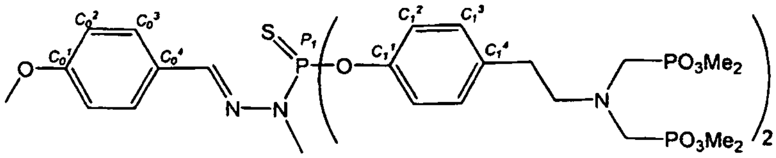

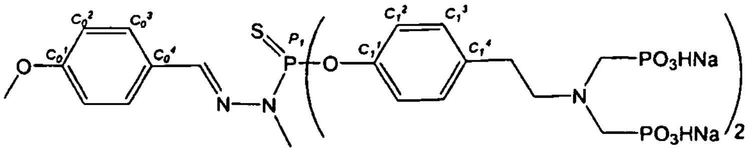

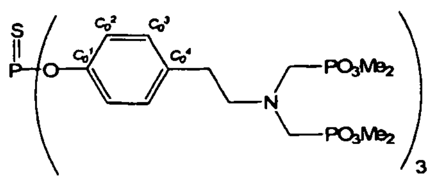

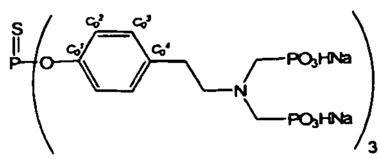

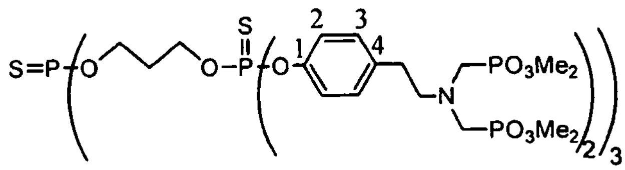

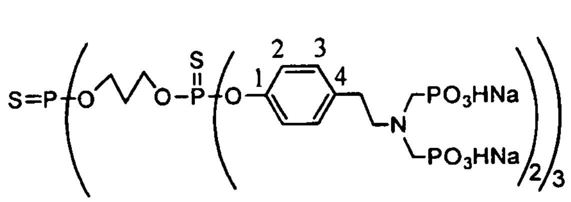

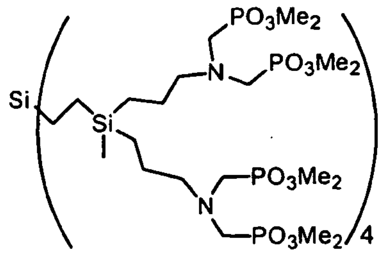

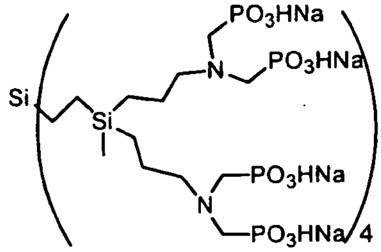

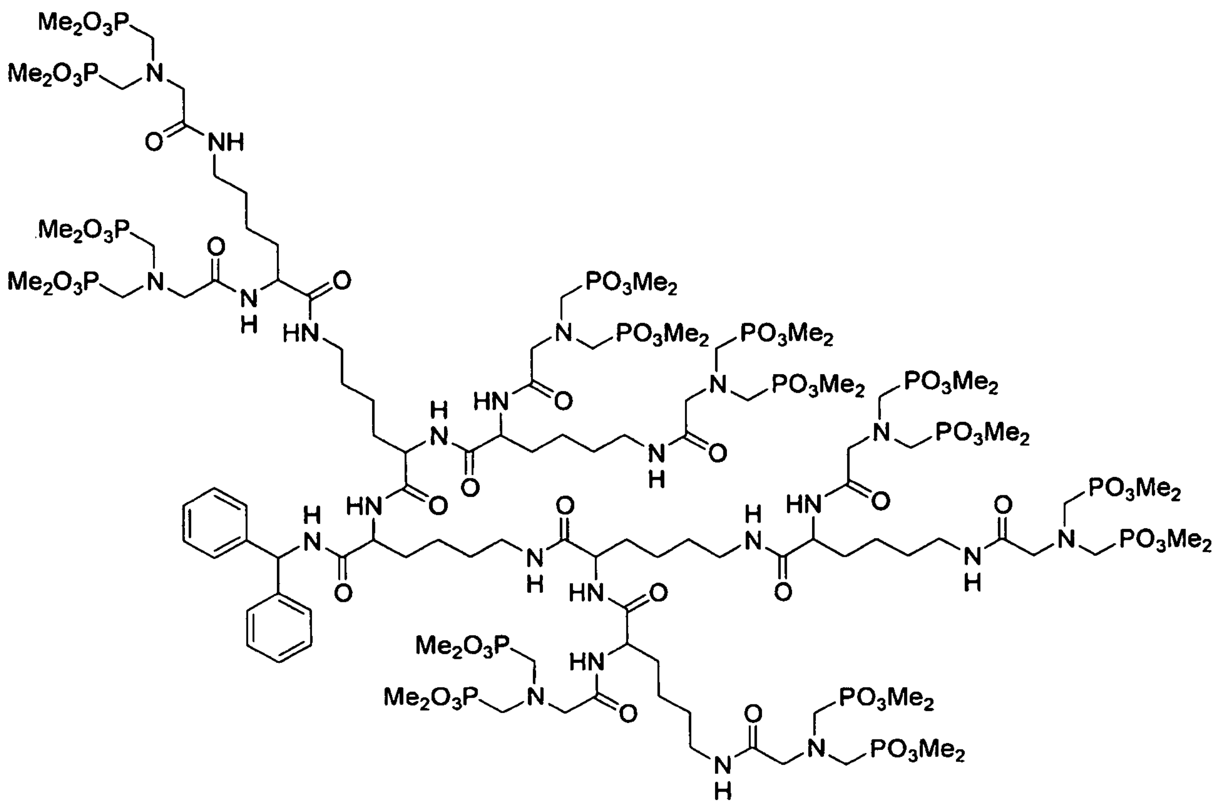

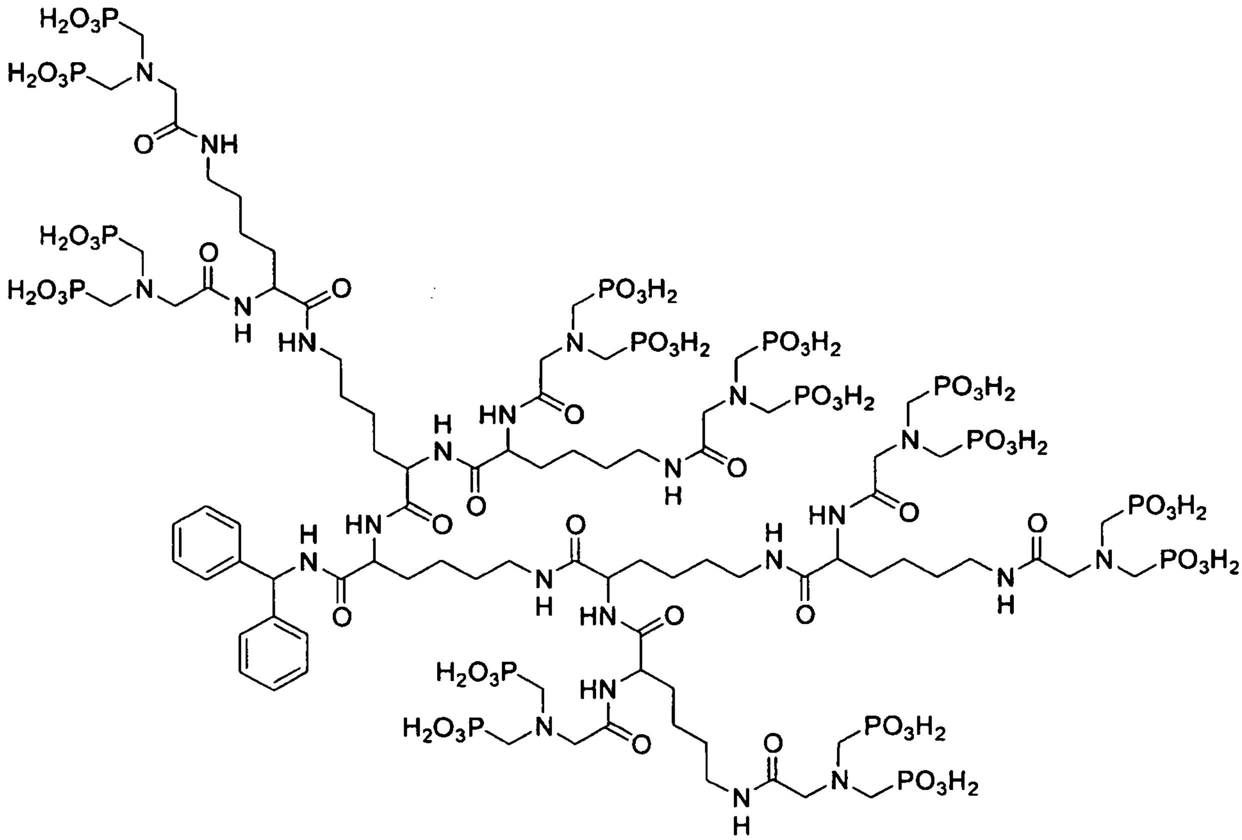

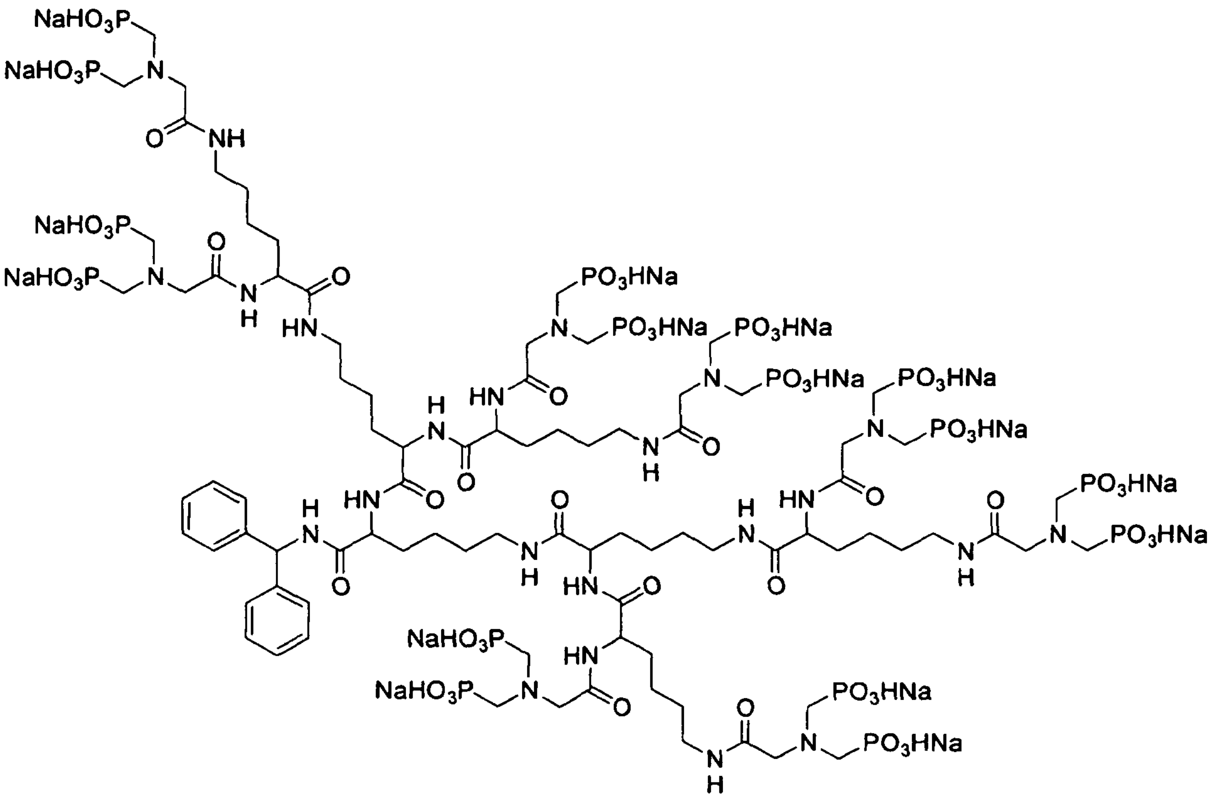

Selon un autre mode de réalisation particulier de l'invention, les cellules de la lignée lymphoïde exprimant le récepteur NKG2D sont dérivées de cellules NK, de lymphocytes T $\alpha\beta$ $CD8^+$ ou de lymphocytes T $\gamma\delta$, notamment de cellules NK.

L'expression « dérivées » signifie que les cellules de l'invention sont notamment obtenues par stimulation de la croissance de cellules NK, de lymphocytes T αβ $CD8^+$ ou de lymphocytes T γδ.

Dans un autre mode de réalisation particulier de l'invention, des cellules de la lignée monocytaire en culture sont activées par les dendrimères à terminaisons monophosphoniques ou bisphosphoniques, l'activation des cellules de la lignée monocytaire correspondant notamment :

- à une augmentation de la taille des cellules activées par rapport aux cellules non activées, et/ou
- à une diminution de l'expression des molécules de CMH de classe I et de classe II, ou de la molécule CD14 par rapport aux cellules non activées, et/ou
- à une augmentation de la translocation nucléaire du facteur NFκB.

Dans un autre mode de réalisation particulier de l'invention, les cellules de la lignée monocytaire en culture présentent une apoptose réduite par rapport à des cellules de la lignée monocytaire cultivées en absence de dendrimère à terminaisons monophosphoniques ou bisphosphoniques.

La présente invention concerne également un milieu de culture cellulaire, caractérisé en ce qu'il contient au moins un composé dendrimérique à terminaisons monophosphoniques ou bisphosphoniques.

On désigne par milieu de culture cellulaire, un milieu, solide ou liquide, contenant l'ensemble des éléments nécessaires à la croissance de cellules (nutriments, facteurs de croissance...), notamment de cellules eucaryotes. De tels milieux sont bien connus de l'homme de l'art.

Selon un mode de réalisation particulier de l'invention, le milieu de culture défini ci-dessus comprend en outre au moins un facteur de croissance et/ou d'activation des cellules NK.

De tels facteurs de croissance et/ou d'activation des cellules NK peuvent être des interleukines, mais également des interférons de type α ou β.

Selon un autre mode de réalisation préféré de l'invention, le milieu de culture défini ci-dessus comprend au moins une interleukine choisie parmi le groupe comprenant : IL-2, IL-7, IL-12, IL-15, IL-18, ou IL-21.

Ces interleukines sont couramment utilisée pour la culture de cellules, notamment de cellules NK. Avantageusement, en présence de dendrimères de l'invention, la concentration de ces composés dans les milieux de culture peut être abaissée par rapport aux milieux de culture standards, ce qui permet notamment de diminuer le coût desdits milieux.

Selon un autre mode de réalisation préféré de l'invention, le milieu de culture défini ci-dessus comprend au moins un composé dendrimérique à terminaisons monophosphoniques ou bisphosphoniques en association avec de l'IL-2.

De préférence, l'IL-2 utilisée est de l'IL-2 humaine recombinante, produite dans un système procaryote (*Escherichia coli* par exemple) ou eucaryote (lignées cellulaires humaines ou d'insectes par exemple).

Selon encore un autre mode de réalisation préféré de l'invention, le composé dendrimérique compris dans le milieu de culture tel que défini ci-dessus correspond aux dendrimères tels que définis ci-dessus.
Selon un mode de réalisation particulièrement préféré de l'invention, le composé dendrimérique compris dans le milieu de culture tel que défini ci-dessus correspond aux dendrimères définis ci-dessous :

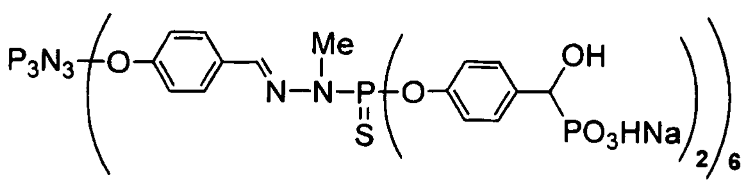

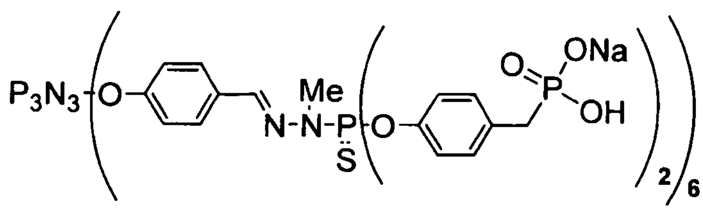

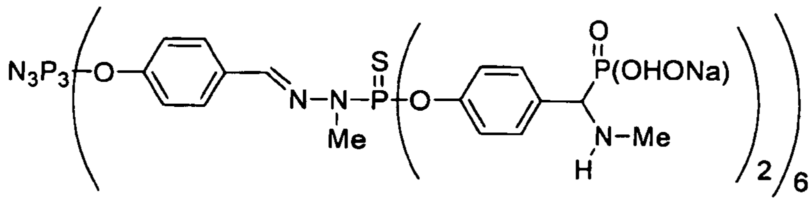

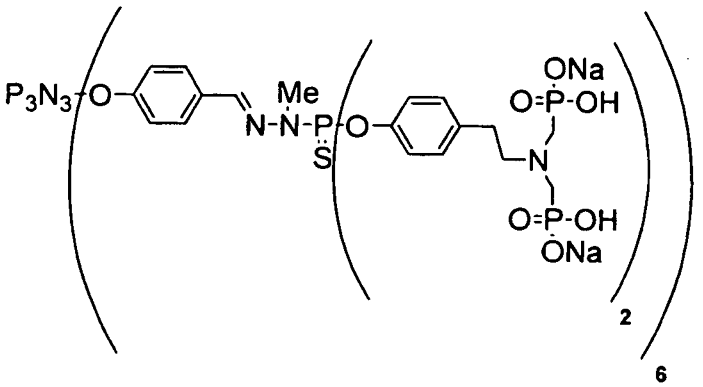

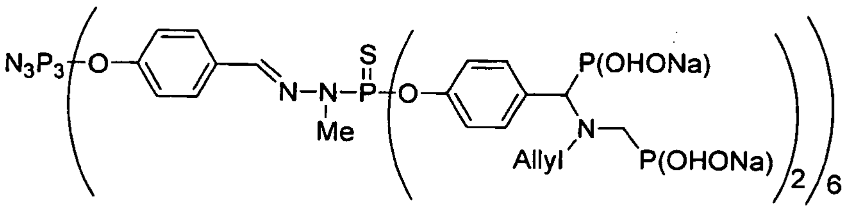

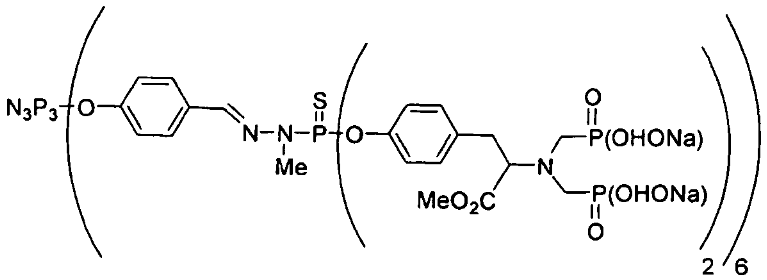

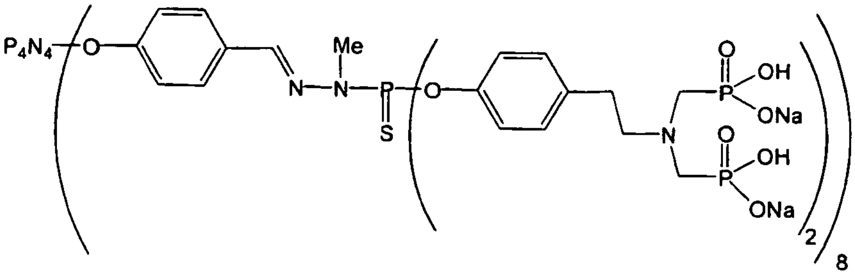

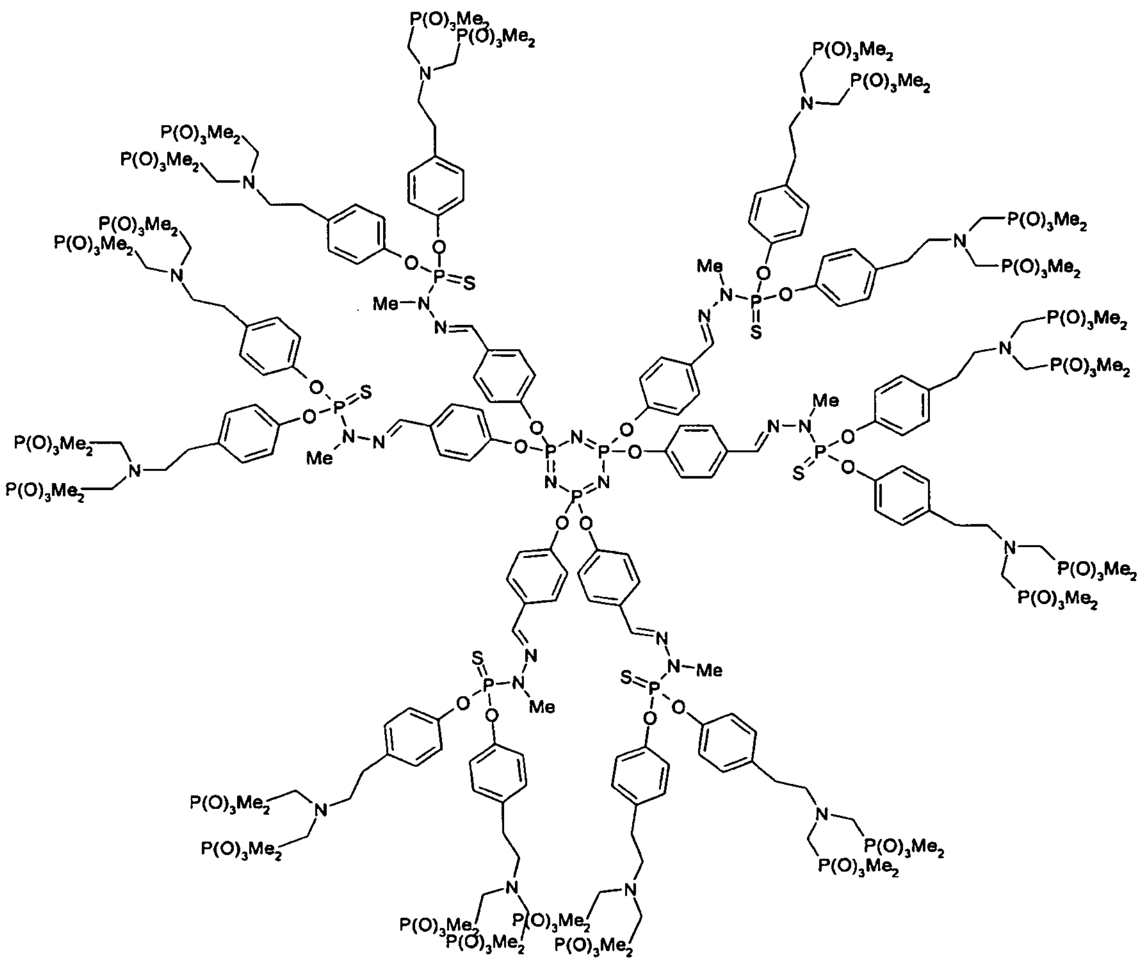

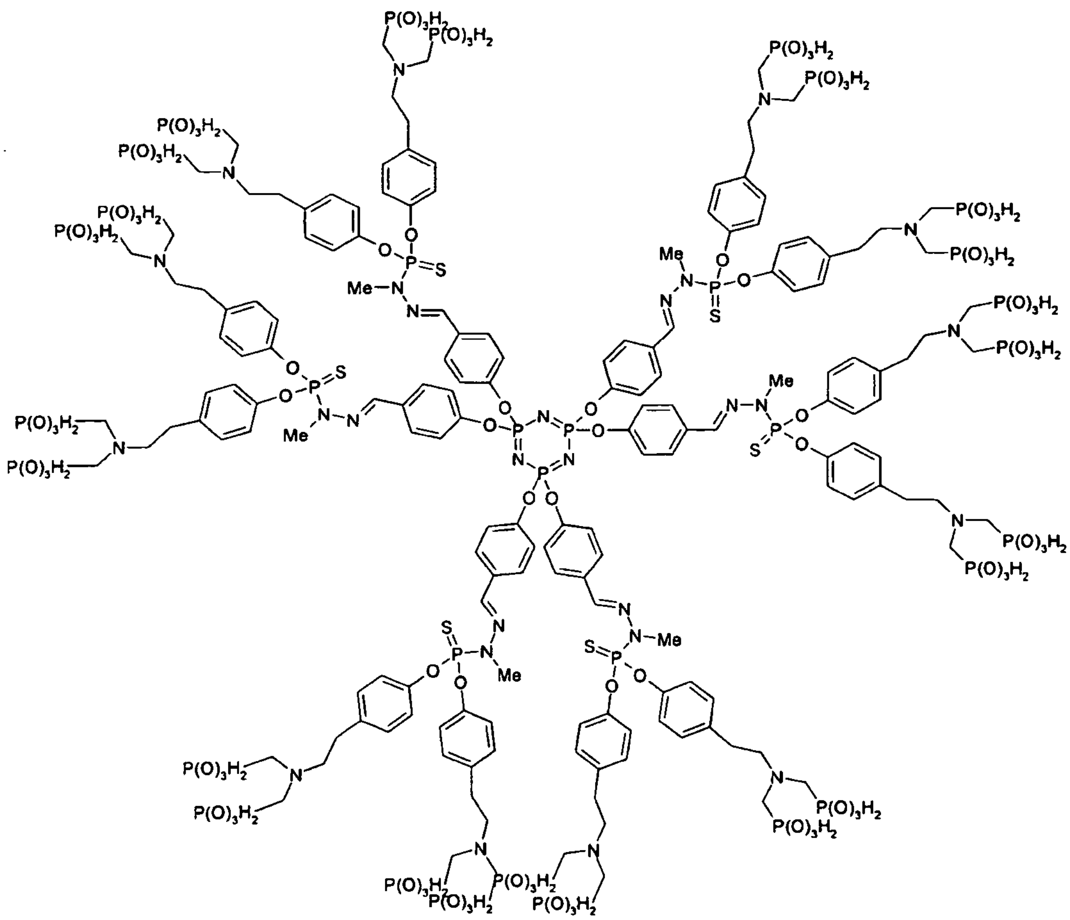

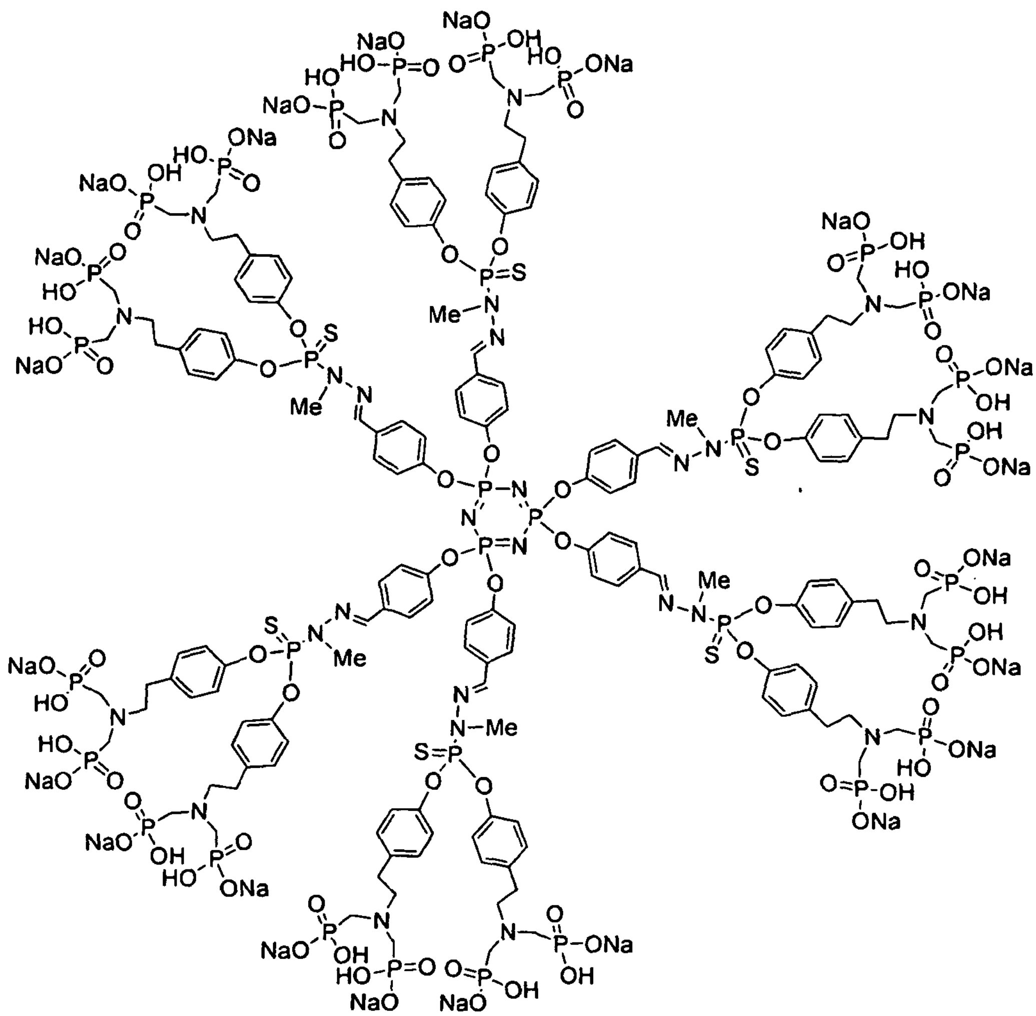

ou à GC0, GC 1 ou GC2, notamment à GC1.

Selon un mode de réalisation tout particulièrement préféré de l'invention, le milieu de culture tel que défini ci-dessus est caractérisé en ce qu'il comprend le composé GC1 à la concentration d'environ 10 à environ 50 μM, notamment environ 20 μM, en association avec de l'IL-2 à la concentration d'environ 100 à environ 1000 unités par ml, correspondant à une concentration d'environ 4 à environ 40 ng par ml, notamment environ 400 unités par ml, correspondant à environ 16 ng/ml, et en ce que l'IL-2 correspond à de l'IL-2 recombinante humaine.

La présente invention concerne également un procédé de préparation de compositions cellulaires enrichies en cellules de la lignée lymphoïde exprimant le récepteur NKG2D, notamment en cellules NK, caractérisé en ce qu'il comprend une étape de mise en présence d'un échantillon biologique avec un dendrimère à terminaisons monophosphoniques ou bisphosphoniques.

Dans un mode de réalisation particulier, le procédé ci-dessus est caractérisé en ce qu'il comprend des cellules de la lignée lymphoïde et/ou des cellules de la lignée monocytaire.

Selon un mode de réalisation préféré du procédé tel que défini ci-dessus, l'échantillon biologique est constitué de sang humain, notamment d'une fraction cellulaire mononucléée d'un échantillon de sang périphérique humain.

Les fractions cellulaires mononucléées de sang périphérique (PBMC) sont préparées selon des méthodes bien connues de l'homme de l'art et notamment par centrifugation sur gradient de densité, comme cela est décrit dans les exemples.

Selon un autre mode de réalisation préféré du procédé tel que défini ci-dessus le dendrimère à terminaisons monophosphoniques ou bisphosphoniques correspond aux dendrimères tels que définis ci-dessus.

Selon un mode de réalisation tout particulièrement préféré du procédé tel que défini ci-dessus le dendrimère à terminaisons monophosphoniques ou bisphosphoniques correspond aux dendrimères de formules suivantes :

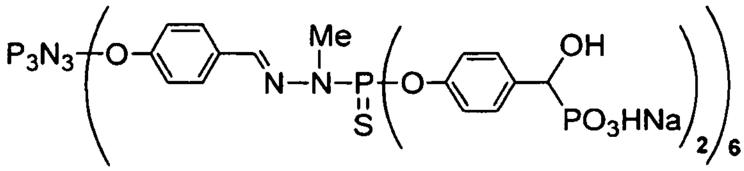

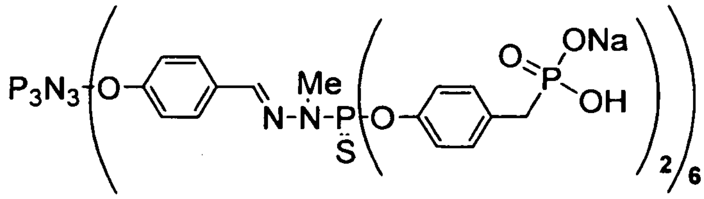

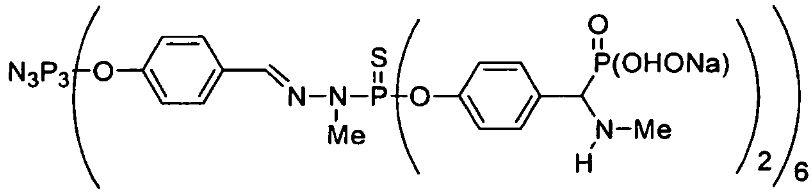

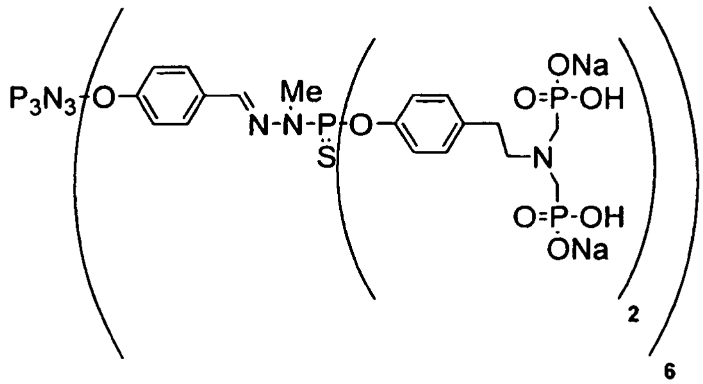

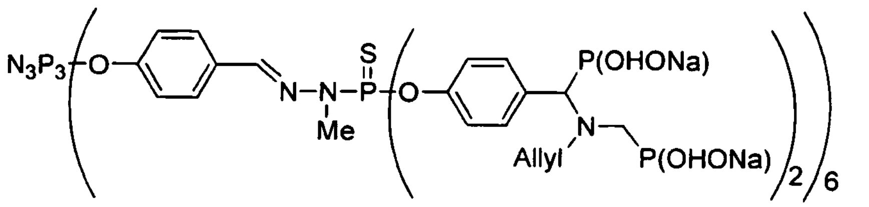

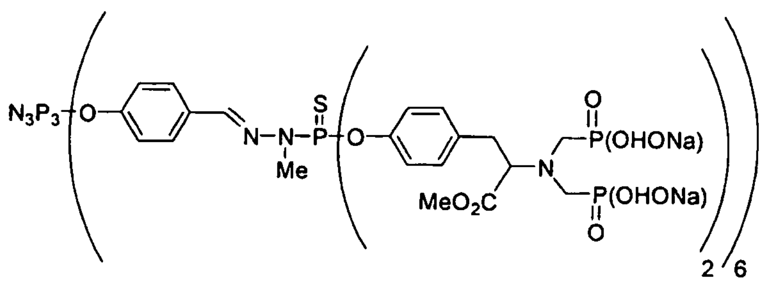

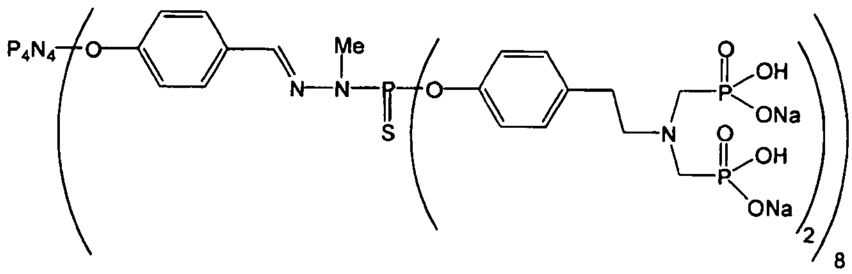

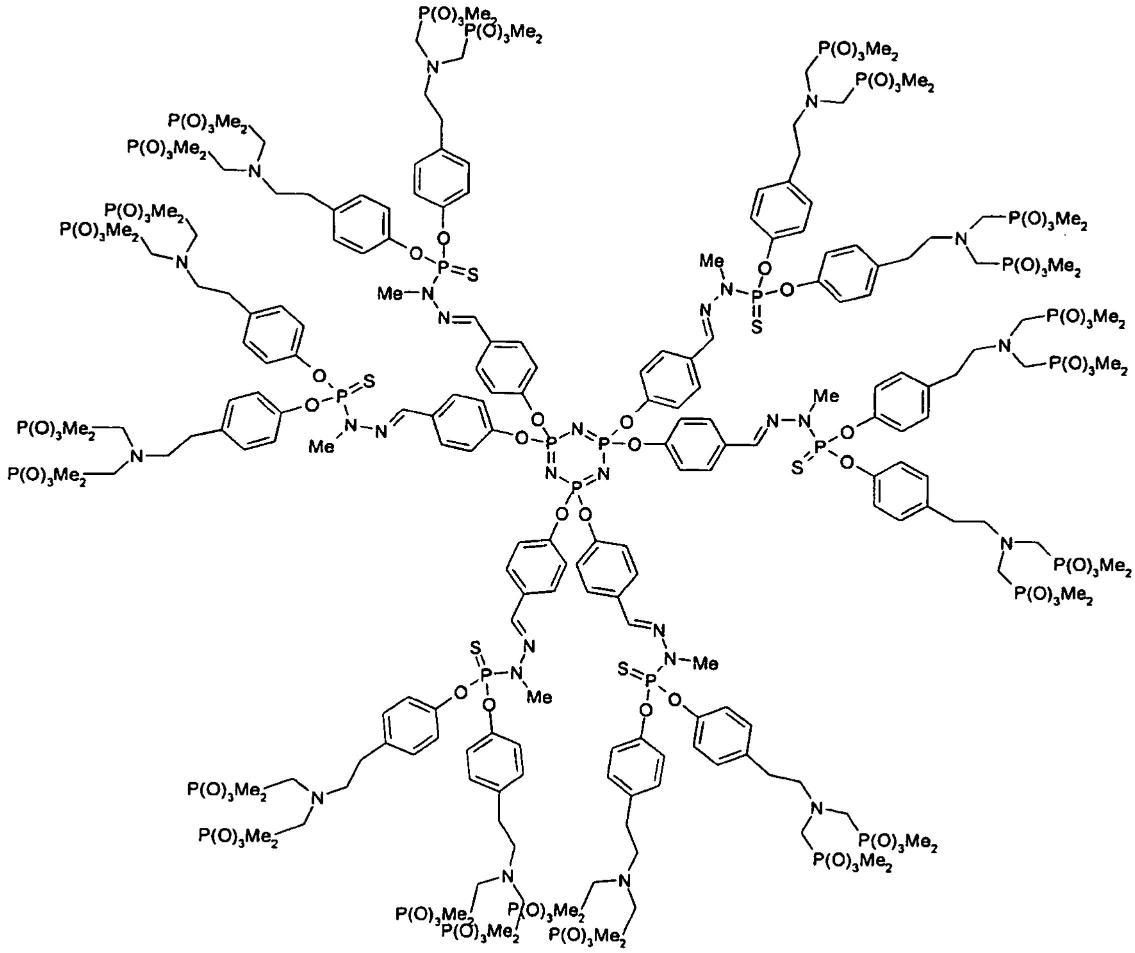

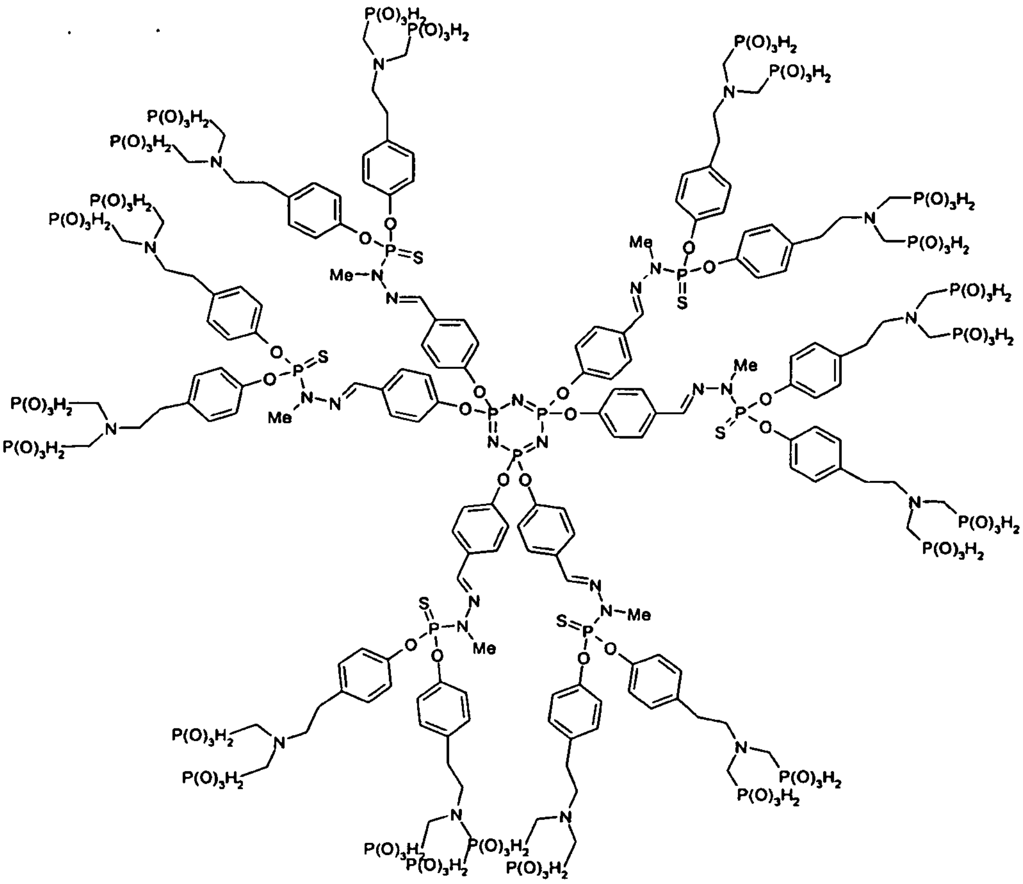

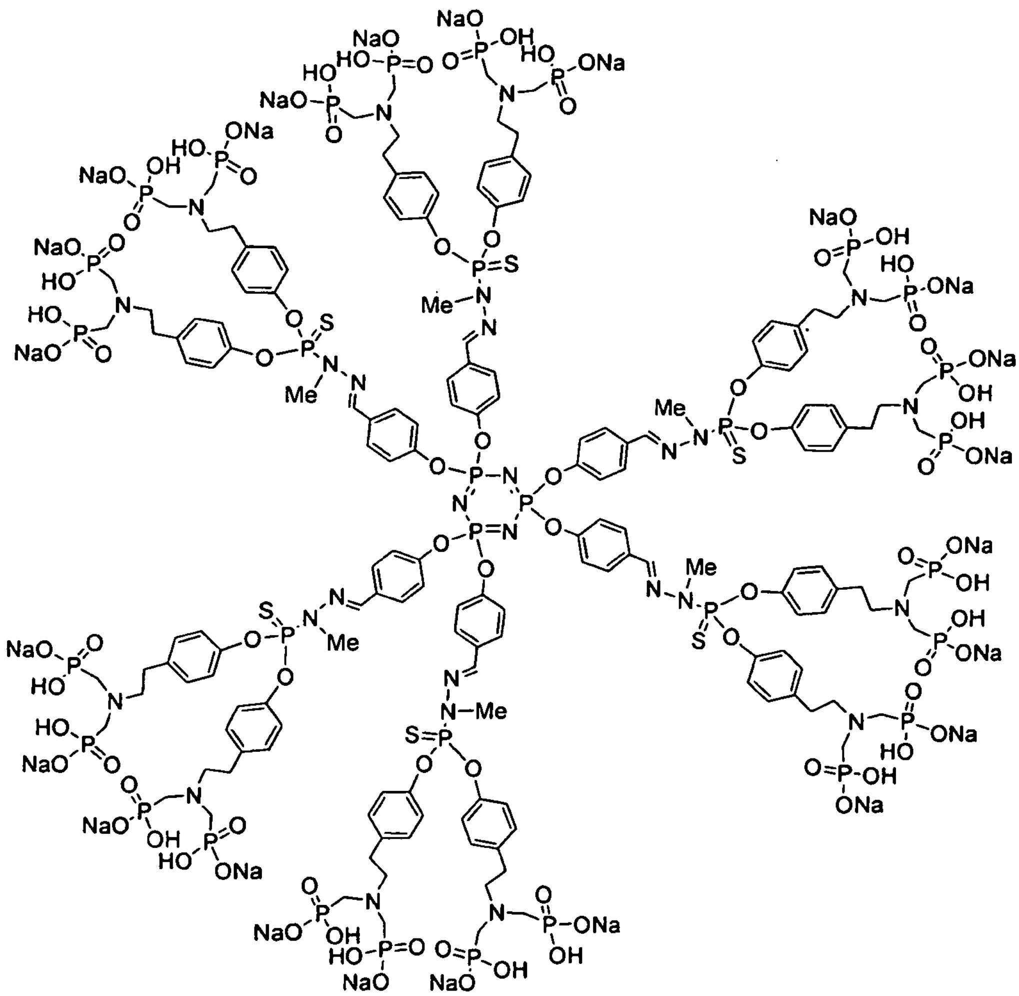

ou à GC0, GC 1 ou GC2, notamment à GC1.

La présente invention concerne également des compositions cellulaires enrichies en cellules de la lignée lymphoïde exprimant le récepteur NKG2D, notamment en cellules NK, telles qu'obtenues par le procédé défini ci-dessus.

Selon un mode de réalisation préféré de l'invention, les compositions cellulaires définies ci-dessus comprennent également un dendrimère à terminaisons monophosphoniques ou bisphosphoniques, notamment GC 1.

La présente invention concerne également un procédé de préparation de monocytes activés ou de compositions cellulaires comprenant des monocytes activés, caractérisé en ce qu'il comprend une étape de mise en présence d'un échantillon biologique comprenant des monocytes avec un dendrimère à terminaisons monophosphoniques ou bisphosphoniques.

Dans un mode de réalisation préféré du procédé de préparation de monocytes activés ou de compositions cellulaires comprenant des monocytes activés ci-dessus, l'échantillon biologique est constitué de sang humain, notamment d'une fraction cellulaire mononucléée d'un échantillon de sang périphérique humain.

Dans un mode de réalisation préféré du procédé de préparation de monocytes activés ou de compositions cellulaires comprenant des monocytes activés ci-dessus, le dendrimère à terminaisons monophosphoniques ou bisphosphoniques correspond aux dendrimères tels que définis ci-dessus.

Dans un mode de réalisation préféré du procédé de préparation de monocytes activés ou de compositions cellulaires comprenant des monocytes activés ci-dessus, le dendrimère à terminaisons monophosphoniques ou bisphosphoniques correspond aux dendrimères de formules suivantes :

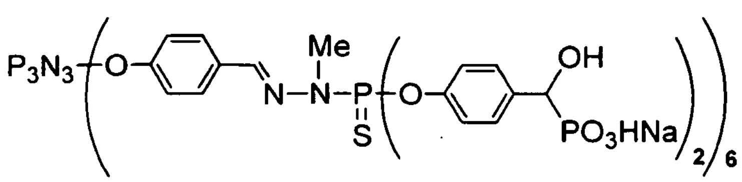

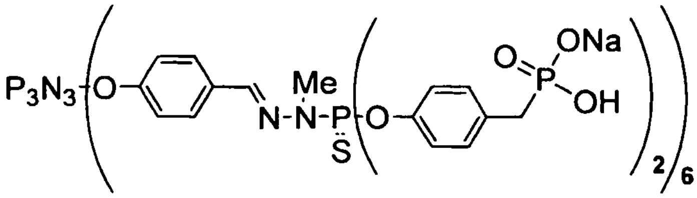

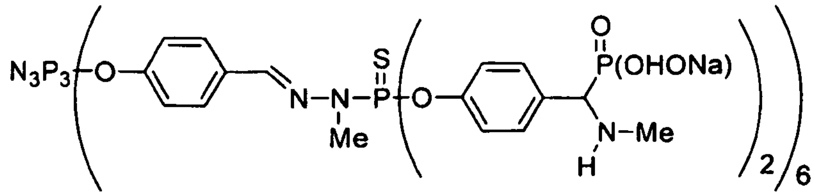

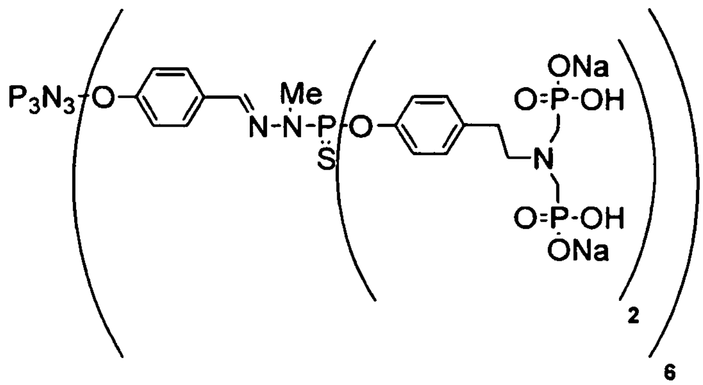

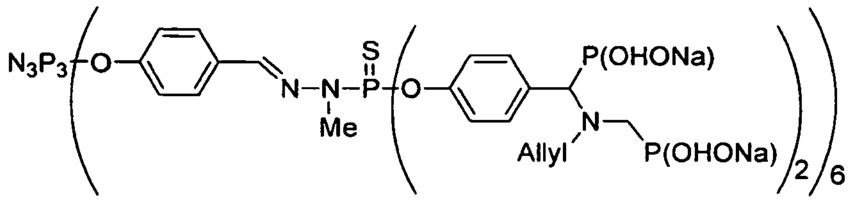

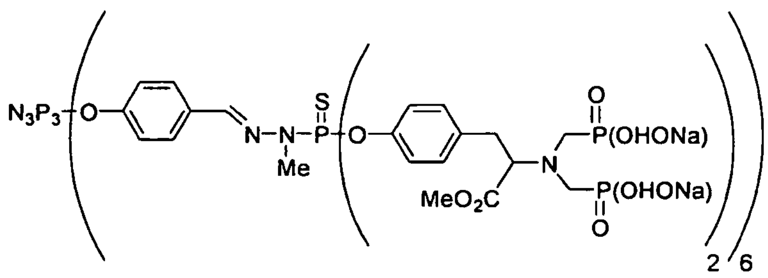

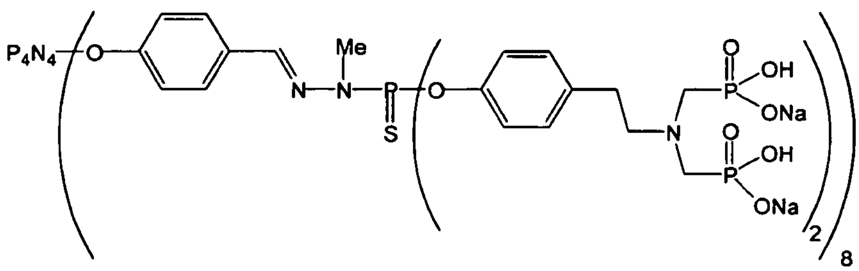

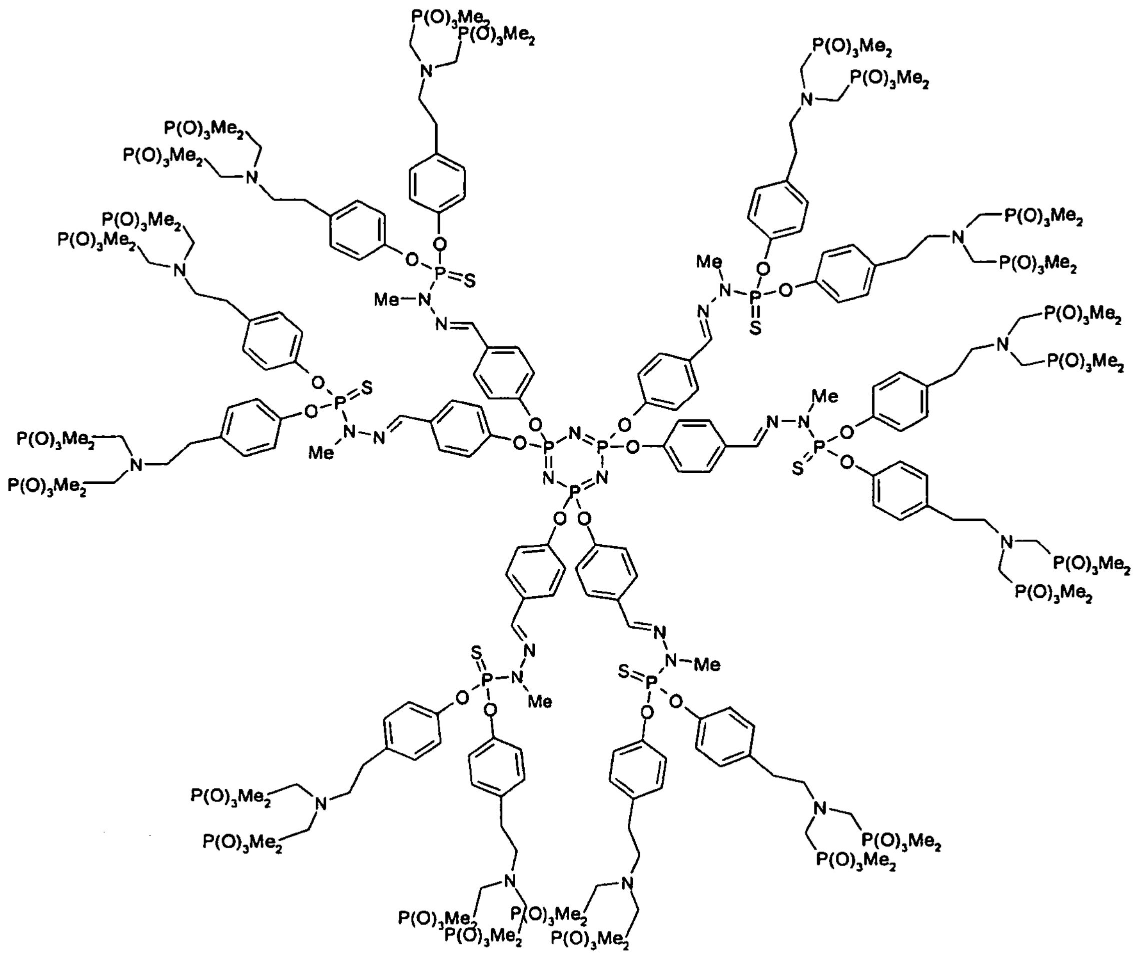

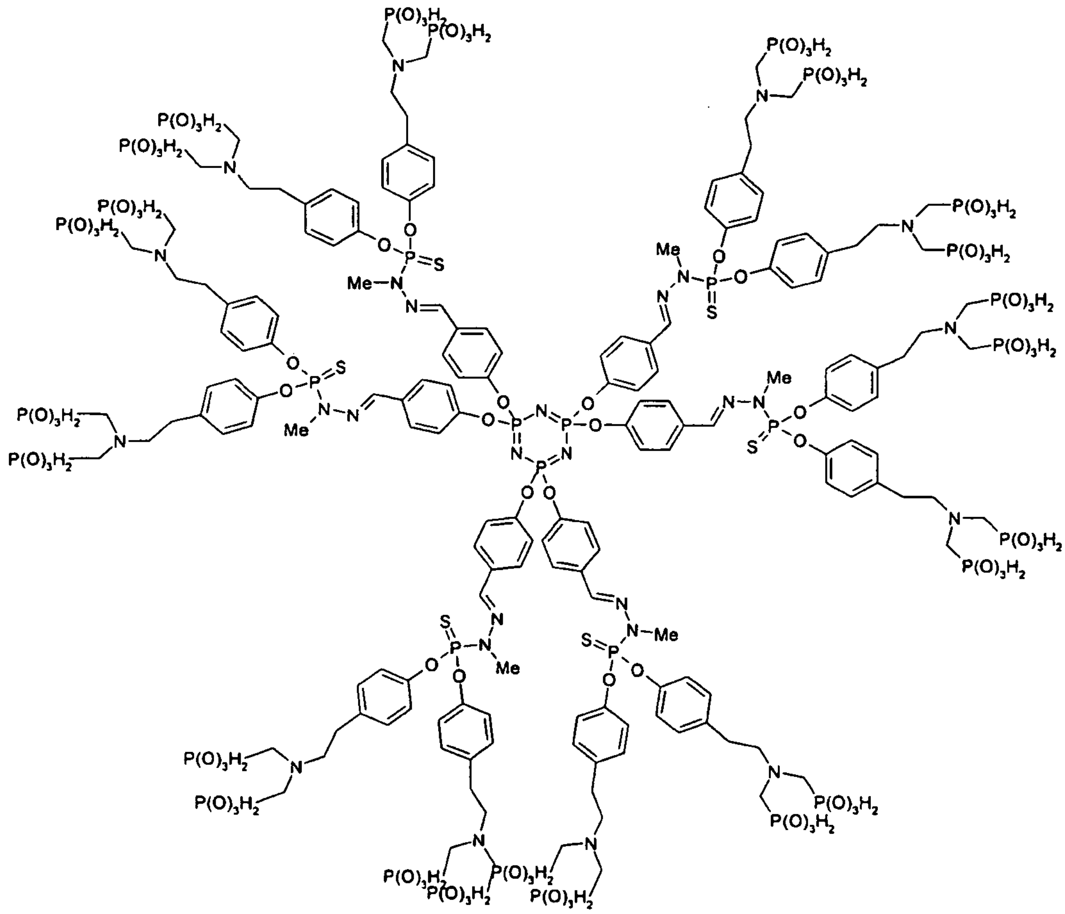

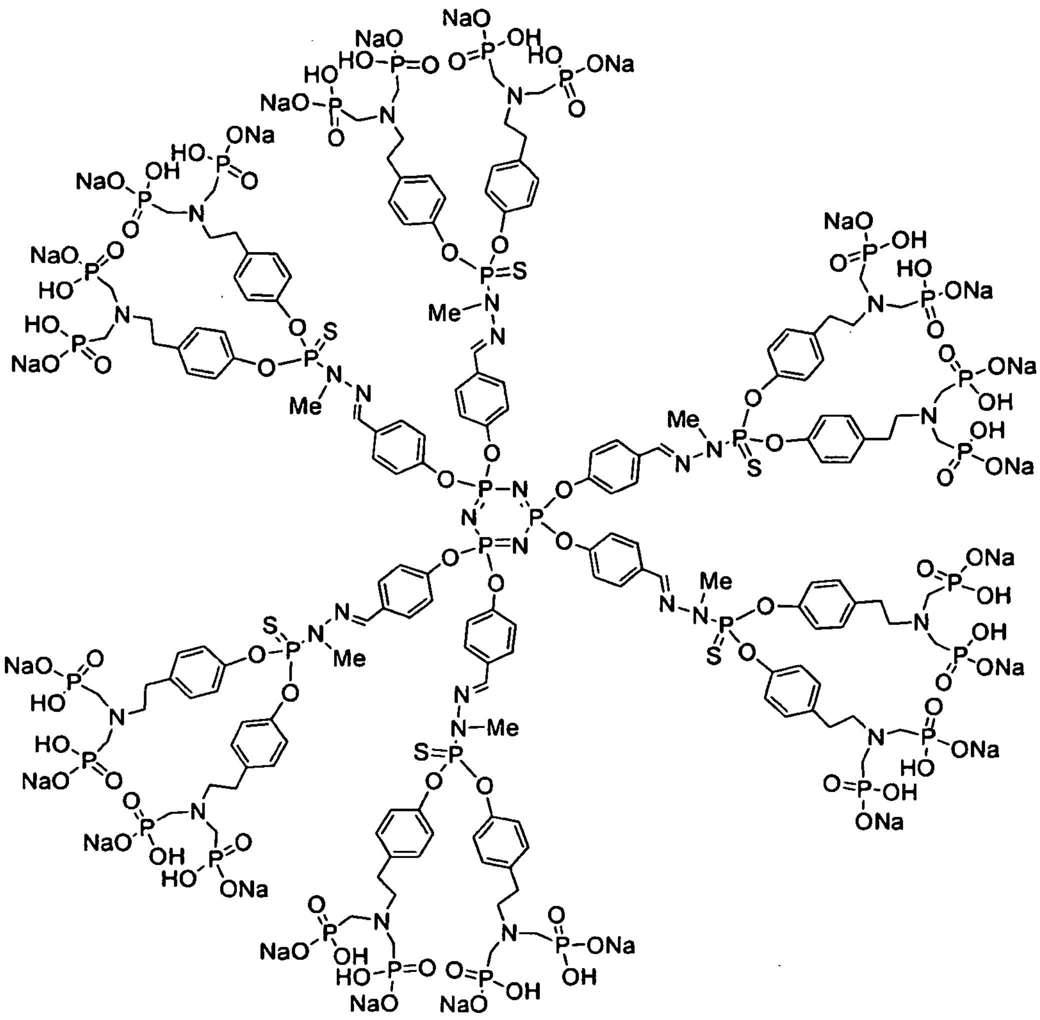

ou à GC0, GC 1 ou GC2, notamment à GC1.

Dans un mode de réalisation particulier, le procédé défini ci-dessus comprend une étape supplémentaire de purification des monocytes activés à partir d'une composition cellulaire comprenant des monocytes activés.

La présente invention concerne également les monocytes activés ou les compositions cellulaires comprenant des monocytes activés tels qu'obtenus par le procédé defini ci-dessus.

La présente invention concerne également une composition pharmaceutique caractérisée en ce qu'elle comprend à titre de substance active des cellules lymphoïdes exprimant le récepteur NKG2D, notamment des cellules NK, et au moins un dendrimère à terminaisons monophosphoniques ou bisphosphoniques, en association avec un véhicule pharmaceutiquement acceptable.

Selon un mode de réalisation préféré de la composition pharmaceutique, le dendrimère à terminaisons monophosphoniques ou bisphosphoniques correspond aux dendrimères tels que définis ci-dessus.

Selon un autre mode de réalisation préféré de la composition pharmaceutique définie ci-dessus, le dendrimère à terminaisons monophosphoniques ou bisphosphoniques correspond aux dendrimères de formules suivantes :

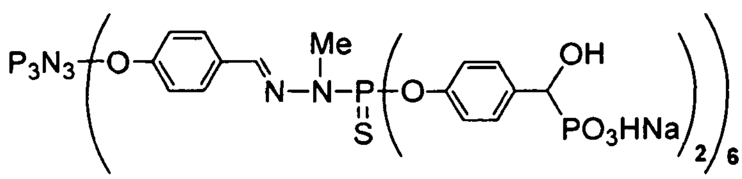

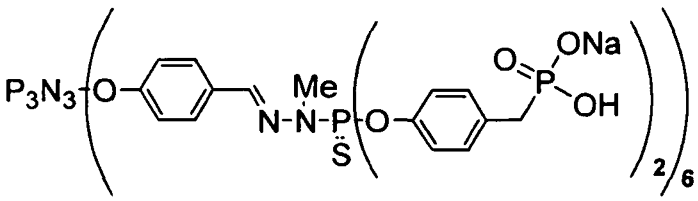

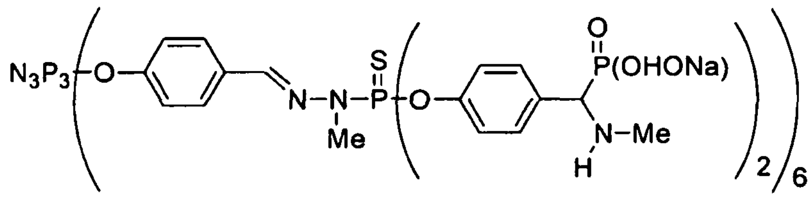

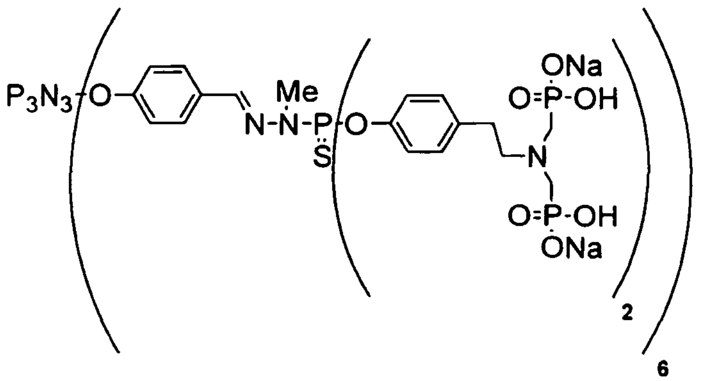

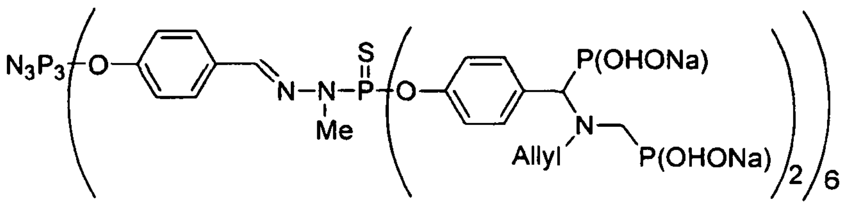

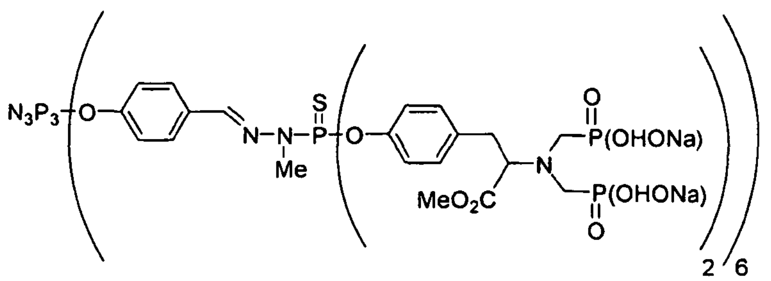

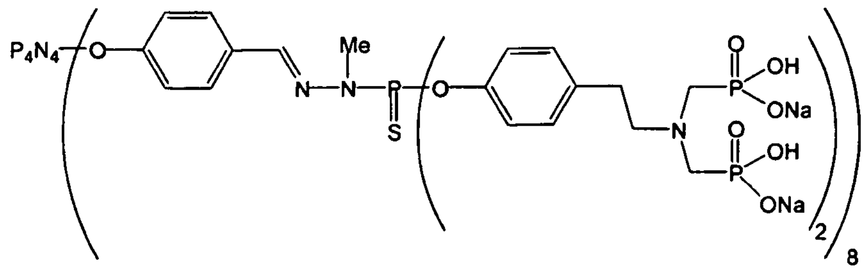

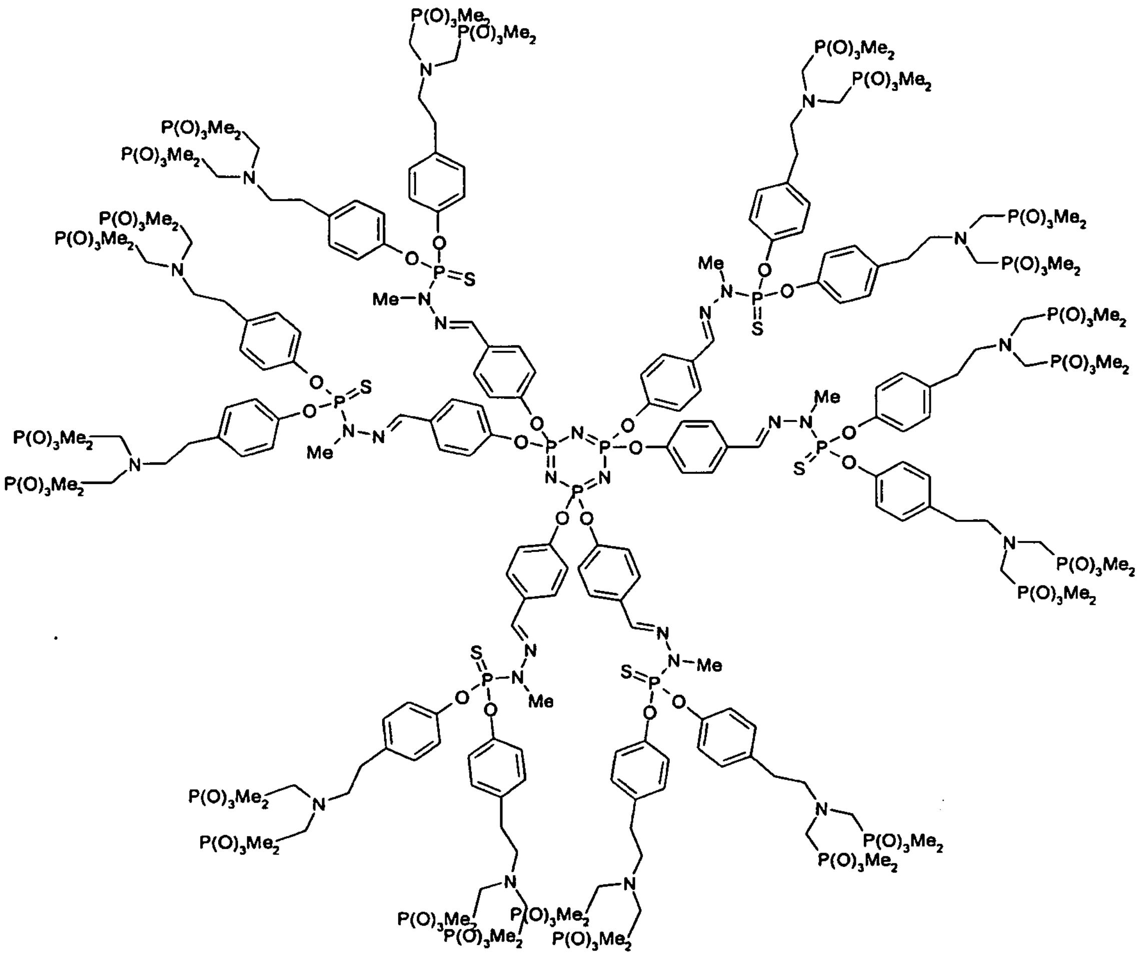

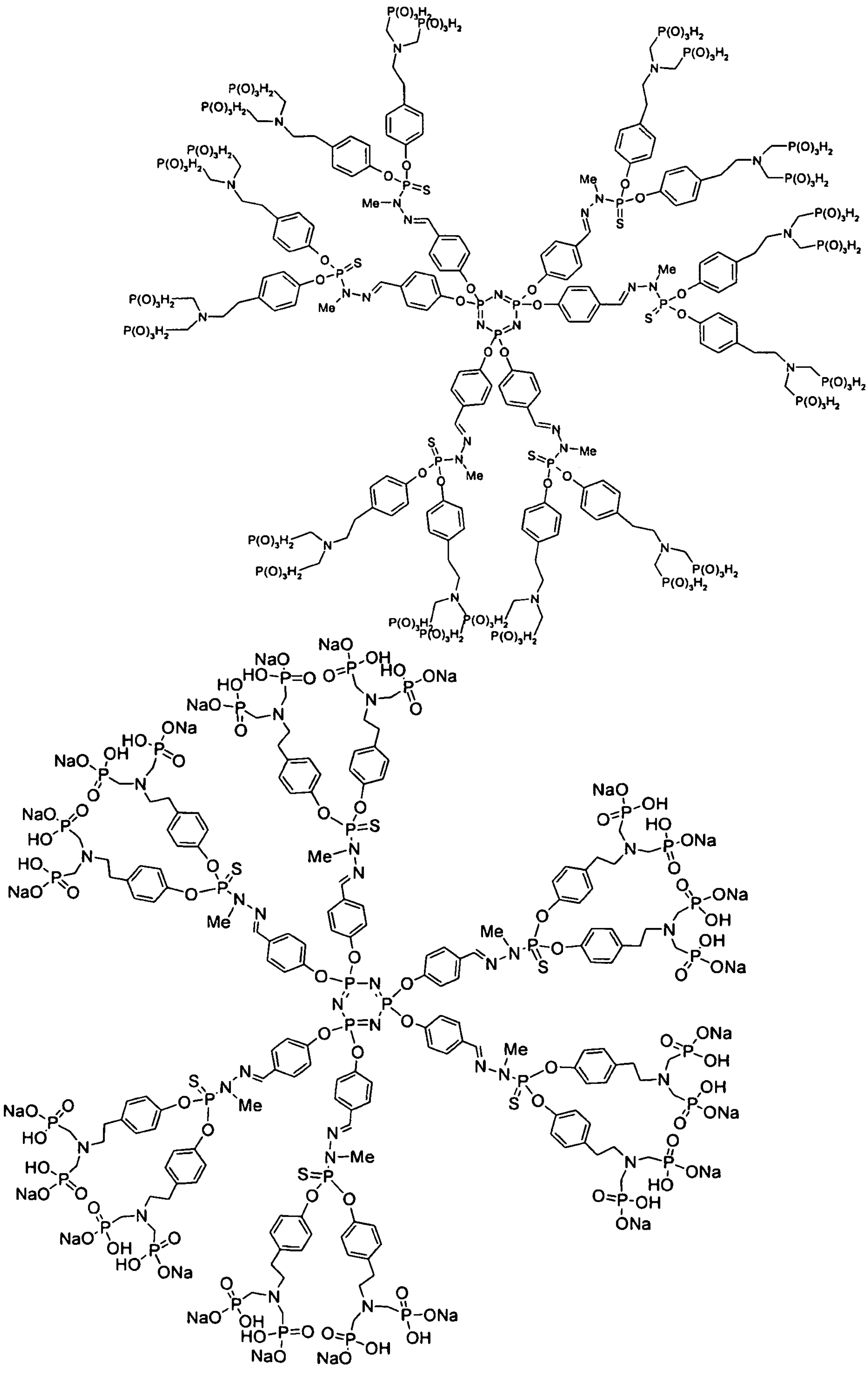

ou à GC0, GC 1 ou GC2, notamment à GC 1.

Selon un autre mode de réalisation préféré de la composition pharmaceutique définie ci-dessus, elle contient à titre de substance active une composition cellulaire enrichie en cellules de la lignée lymphoïde exprimant le récepteur NKG2D telle que définie ci-dessus et/ou des monocytes activés ou une composition cellulaire comprenant des monocytes activés tels que définis ci-dessus, en association avec un véhicule pharmaceutiquement acceptable.

Selon encore un autre mode de réalisation particulier de la composition pharmaceutique définie ci-dessus, elle convient pour l'administration à un individu d'une dose unitaire d'environ $10^5$ à environ $5.10^9$ cellules lymphoïdes exprimant le récepteur NKG2D, notamment de cellules NK.

La présente invention concerne également l'utilisation de cellules lymphoïdes exprimant le récepteur NKG2D, notamment de cellules NK, et de GC 1 pour la préparation de médicaments destinés à traiter et/ou à prévenir les cancers, dont les tumeurs de tissus hématopoïétiques, telles que les leucémies myéloïdes ou les lymphomes anaplasiques, et les mélanomes.

Les cellules selon l'invention possèdent une activité cytolytique dirigée contre les cellules cancéreuses. Ces cellules peuvent donc être administrées à un patient atteint d'un cancer afin de détruire *in vivo* les tumeurs que celui-ci porte. Avantageusement, ces cellules peuvent être préparées, soit à partir d'échantillons biologiques issus du patient lui-même, soit à partir d'un donneur sain.

L'invention concerne plus particulièrement, l'utilisation d'une composition cellulaire enrichie en cellules de la lignée lymphoïde exprimant le récepteur NKG2D telle que définie ci-dessus et/ou des monocytes activés ou une composition cellulaire comprenant des monocytes activés tels que définis ci-dessus pour la préparation de médicaments destinés à traiter et/ou à prévenir les cancers, dont les tumeurs de tissus hématopoïétiques, telles que les leucémies myéloïdes ou les lymphomes anaplasiques, et les mélanomes.

La présente invention concerne également une composition pharmaceutique contenant à titre de substance active au moins un dendrimère à terminaisons monophosphoniques ou bisphosphoniques en association avec un véhicule pharmaceutiquement acceptable.

La présente invention concerne plus particulièrement une composition pharmaceutique contenant à titre de substance active au moins un dendrimère à terminaisons monophosphoniques ou bisphosphoniques telle que définie ci-dessus, caractérisée en ce que le dendrimère à terminaisons monophosphoniques ou bisphosphoniques correspond aux dendrimères tels que définis ci-dessus.

Selon un mode de réalisation préféré de la composition pharmaceutique contenant à titre de substance active au moins un dendrimère à terminaisons monophosphoniques ou bisphosphoniques telle que définie ci-dessus, le dendrimère à terminaisons monophosphoniques ou bisphosphoniques correspond aux dendrimères de formules suivantes :

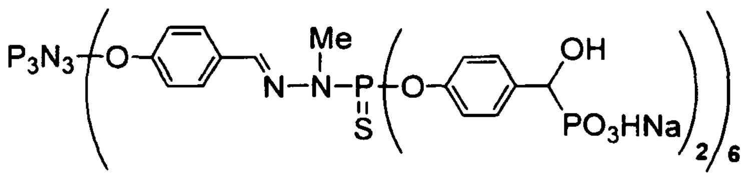

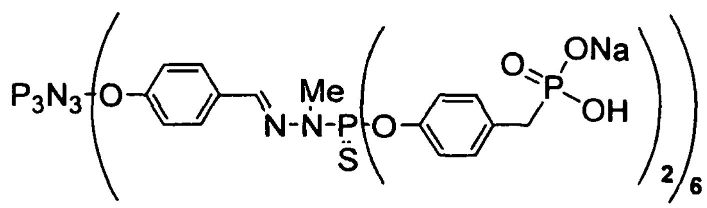

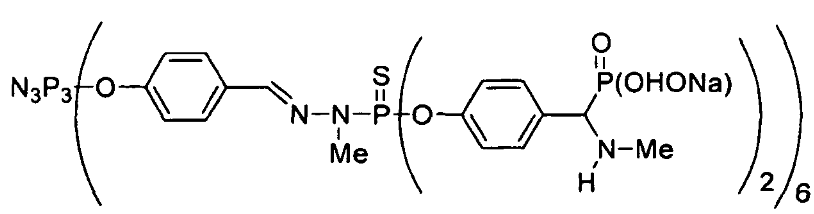

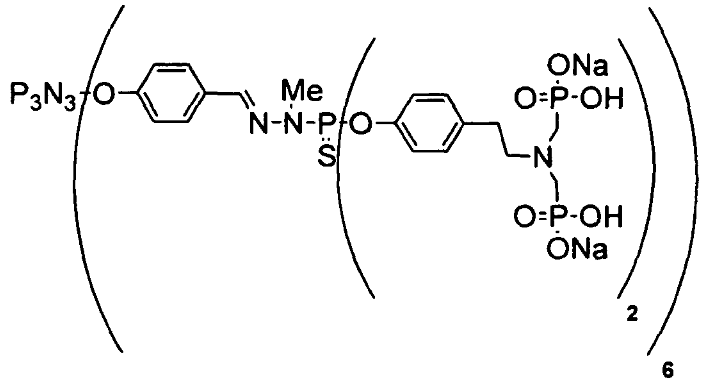

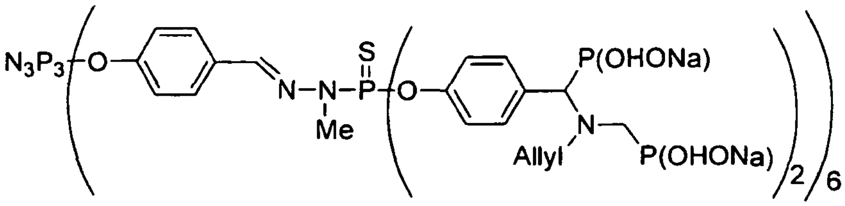

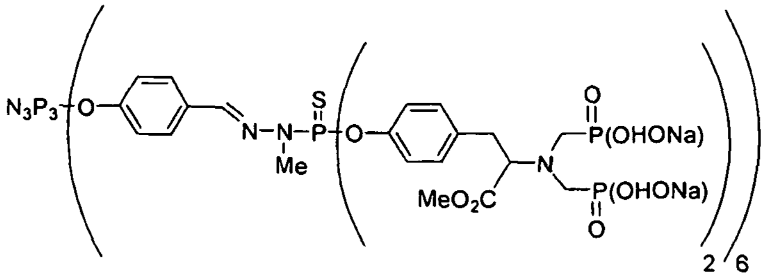

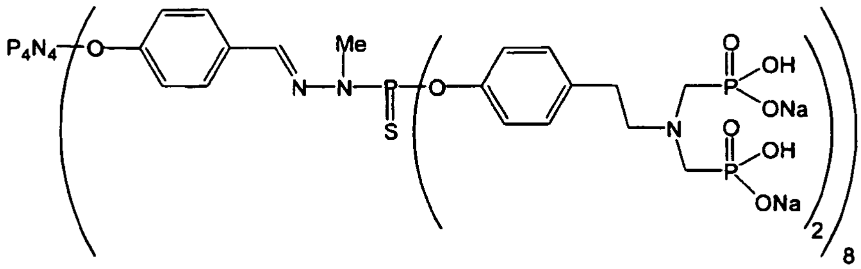

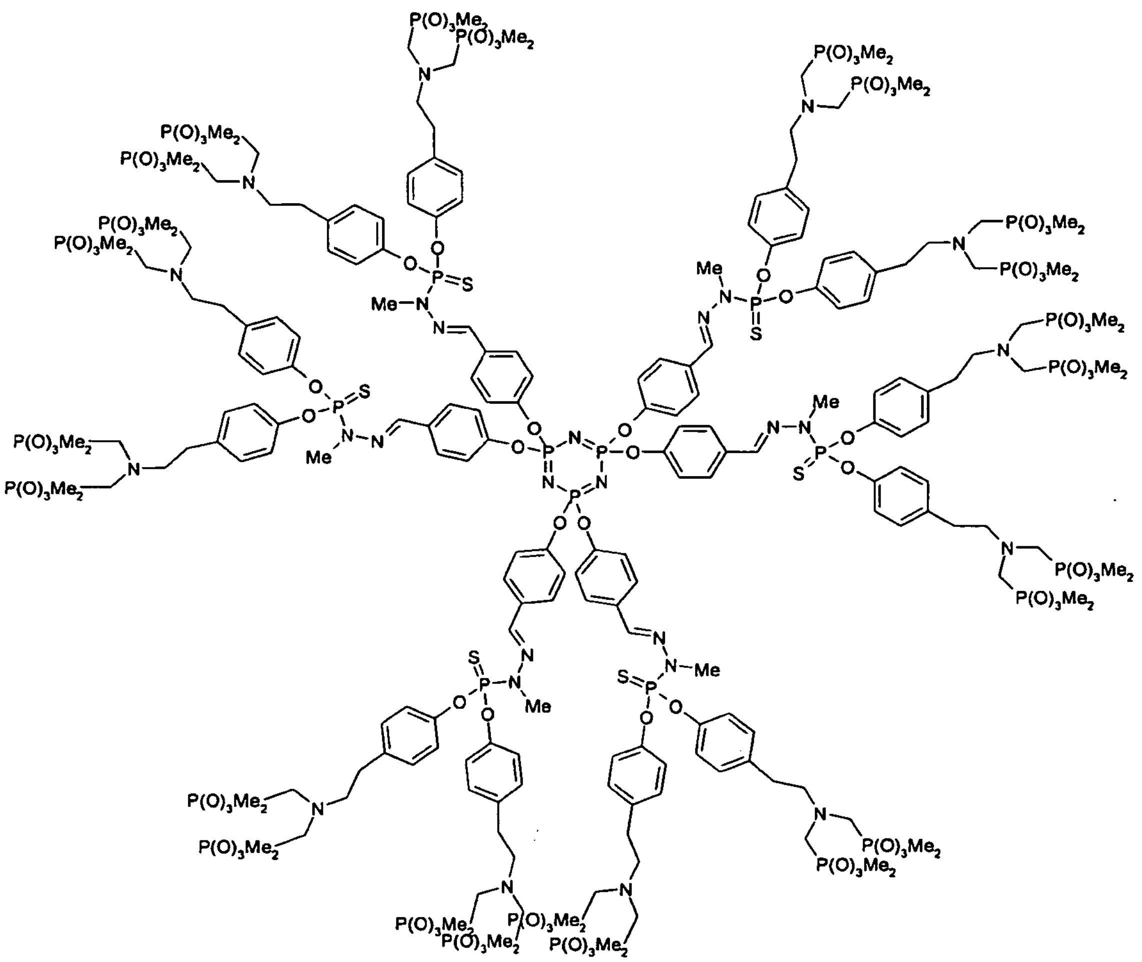

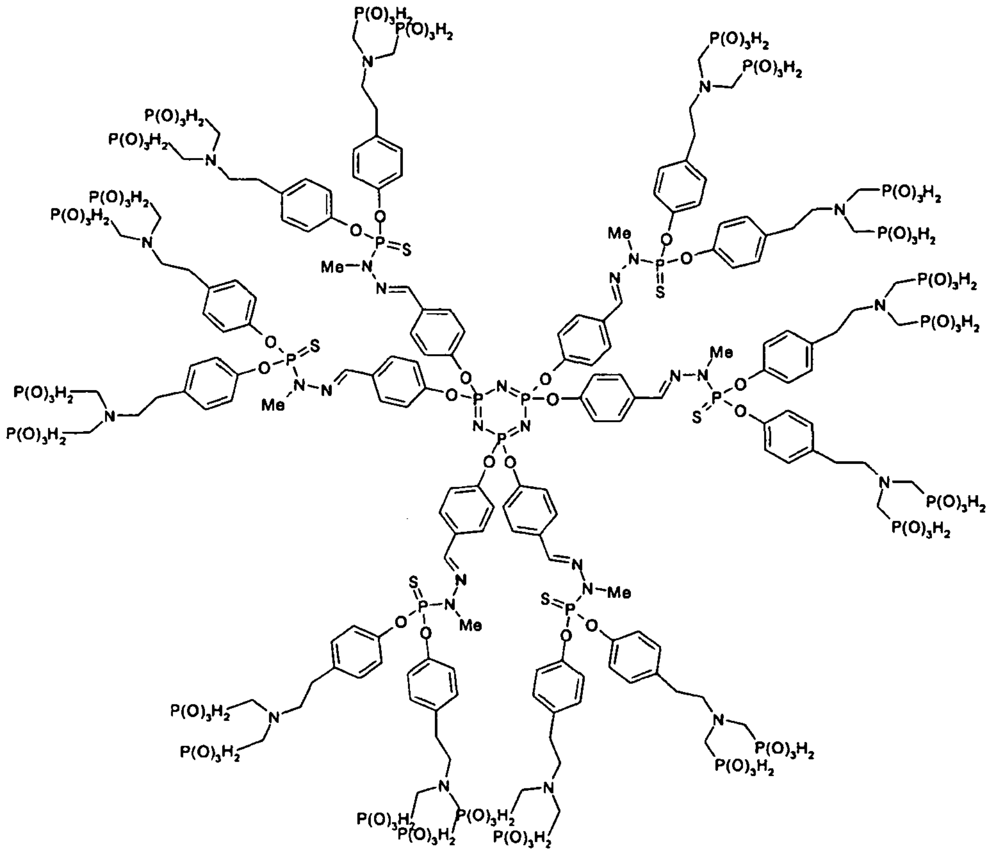

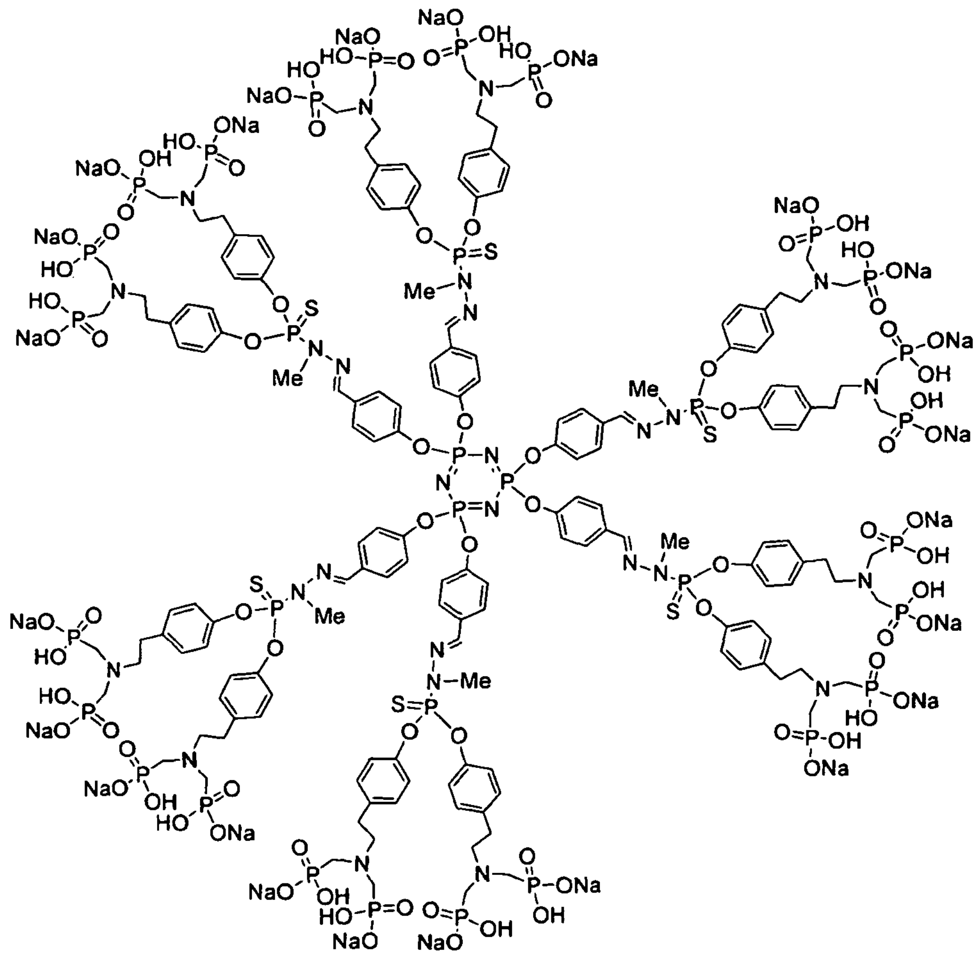

ou à GC0, GC 1 ou GC2, notamment à GC1.

Avantageusement les dendrimères de l'invention peuvent être administrés directement à un patient afin de produire leurs effets *in vivo*. Ces effets peuvent notamment passer par la stimulation sélective de la croissance des cellules NK au sein de l'organisme, permettant ainsi de renforcer les défenses anti-cancéreuses dudit patient.

Les dendrimères utilisés dans le cadre de l'invention et dans lesquels une ou deux liaisons susceptibles d'être établies par le noyau central sont reliées à des groupes de liaison, identiques ou différents, constitués
soit d'une partie des chaînes de liaison définies ci-dessus,
soit d'un atome d'hydrogène,
soit de groupes hydrocarbonés comprenant de 1 à 500 atomes de carbone,
sont nouveaux.

A ce titre, la présente invention concerne également des dendrimères à terminaisons monophosphoniques ou bisphosphoniques de génération n comprenant un noyau central § de valence m, m représentant un entier de 3 à 20, en particulier de 3 à 10 et plus particulièrement de 3 à 8, le noyau établissant m-2 ou m-1 liaisons avec respectivement m-2 ou m-1 chaînes de liaison identiques constituées :

- soit par des chaînes de génération fixées en arborescence autour du noyau sur chacune des liaisons, au moins une chaîne intermédiaire étant éventuellement fixée à l'extrémité de chaque chaîne de la génération la plus éloignée du noyau central, et un groupe terminal étant fixé à l'extrémité de chaque chaîne de la génération la plus éloignée du noyau central ou le cas échéant à l'extrémité de chaque chaîne intermédiaire, ou
- soit par des chaînes intermédiaires fixées autour du noyau sur chacune des liaisons, un groupe terminal étant fixé à l'extrémité de chaque chaîne intermédiaire ;

ledit groupe terminal étant représenté par la formule :

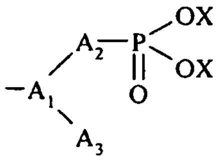

où

$A_1$ représente N ; un groupe P=Y, où Y représente O, S, ou aucun atome ; un groupe N-R ; ou un groupe C-R ; R représentant H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR'R'', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R' et R'' représentant indépendamment l'un de l'autre H ou un groupement alkyle de 1 à 16 atomes de carbone, aryle de 6 à 24 atomes de carbone ou aralkyle de 7 à 16 atomes de carbone ;

$A_2$ représente une liaison simple ou une chaîne hydrocarbonée de 1 à 6 chaînons, linéaire, ramifiée ou cyclique, chacun desdits chaînons contenant éventuellement un ou plusieurs hétéroatomes, lesdits hétéroatomes étant préférentiellement choisis parmi un atome de soufre, d'oxygène, de phosphore, ou d'azote, plus préférentiellement d'azote, et étant éventuellement substitués par au moins un substituant choisi parmi H, un groupement alkyle de 1 à 16 atomes de carbone, un halogène, un groupement $-NO_2$, un groupement -NRR', un groupement -CN, un groupement $-CF_3$, un groupement hydroxyle, un groupement alkoxy de 1 à 16 atomes de carbone, un groupement aryle ou hétéroaryle de 1 à 24 atomes de carbone, l'hétéroélément étant préférentiellement choisi parmi l'oxygène, l'azote ou le soufre, un groupement aralkyle de 7 à 16 atomes de carbone, R et R' représentant indépendamment l'un de l'autre H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR''R''', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R'' et R''' représentant indépendamment l'un de l'autre H ou un groupement alkyle de 1 à 16 atomes de carbone, aryle de 6 à 24 atomes de carbone ou aralkyle de 7 à 16 atomes de carbone ;

$A_3$ représente H, ou une chaîne hydrocarbonée de 1 à 6 chaînons, linéaire, ramifiée ou cyclique, chacun desdits chaînons pouvant éventuellement être choisi parmi un hétéroatome, ledit hétéroatome étant préférentiellement choisi parmi le soufre, l'azote, le phosphore, ou le silicium, plus préférentiellement l'azote, chaque chaînon pouvant être éventuellement substitué par au moins un groupement choisi parmi un groupement hydroxyle, un groupement -NR''R''', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R'' et R''' représentant indépendamment l'un de l'autre H ou un groupement alkyle de 1 à 16 atomes de carbone, aryle de 6 à 24 atomes de carbone, ou aralkyle de 7 à 16 atomes de carbone ou

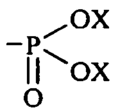

,

en particulier $A_3$ peut représenter

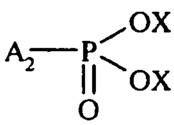

,

chacun des $A_2$ étant identiques ou différents ;

chaque OX, identique ou différent pour chaque groupement phosphonique, représente OH, OAlkyle, où le groupe alkyle

comprend de 1 à 16 atomes de carbone, OAryle, où le groupe aryle comprend de 6 à 24 atomes de carbone, OAralkyle, où le groupe aralkyle comprend de 7 à 24 atome de carbone, OAlkylaryle, où le groupe alkylaryle comprend de 7 à 24 atomes de carbone, $OSiR'_1R'_2R'_3$, où $R'_1$, $R'_2$ et $R'_3$, identiques ou différents, représentent un groupement alkyle de 1 à 16 atomes de carbone, ou $O^-M^+$, où $M^+$ est un cation d'éléments du groupe IA, IB, IIA, IIB ou IIIA, IIIB de la classification périodique des éléments, de préférence $M^+$ est choisi parmi les cations des atomes de sodium, de potassium, de cuivre, de calcium, de baryum, de zinc, de magnésium, de lithium et d'aluminium, ou de groupes hydrocarbonés de 1 à 100 atomes de carbone, ou azotés de 0 à 100 atomes de carbone, tel que $NR_1R_2R_3R_4^+$, où, indépendamment les uns des autres $R_1$, $R_2$, $R_3$ et $R_4$ représentent H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone ;
n représente un nombre entier de 0 à 12 ;
les 1 ou 2 liaisons restantes étant fixées à des groupes de liaison, identiques ou différents, éventuellement liés ensemble, notamment par l'intermédiaire d'une liaison covalente, constitués
soit d'une partie des chaînes de liaison définies ci-dessus,
soit d'un atome d'hydrogène,
soit de groupes hydrocarbonés comprenant de 1 à 500 atomes de carbone,
lesdits groupes hydrocarbonés étant notamment constitués de H ou d'une chaîne hydrocarbonée de 1 à 16 chaînons, linéaire, ramifiée ou cyclique, contenant éventuellement une ou plusieurs doubles ou triples liaisons, chacun desdits chaînons pouvant éventuellement être choisi parmi un hétéroatome, ledit hétéroatome étant préférentiellement choisi parmi un atome d'azote, d'oxygène, de phosphore, de silicium ou de soufre, un groupement aryle de 6 à 24 atomes de carbone, un groupement hétéroaryle de 1 à 24 atomes de carbone, un groupement carboxyle, un groupement >C=NR, chaque chaînon pouvant être éventuellement substitué par au moins un substituant choisi parmi un groupement hydroxyle, un groupement - NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone, le premier chaînon de ladite chaîne hydrocarbonée étant de préférence un oxygène ou un azote.
ledit noyau central § représentant un groupe comprenant de 1 à 500 atomes, et contenant éventuellement un ou plusieurs hétéroatomes, lesdits hétéroatomes étant préférentiellement choisis parmi l'oxygène, le soufre, l'azote, le phosphore ou le silicium,
étant choisi parmi :

- un atome d'azote ou un atome de silicium,

- un groupement de formule

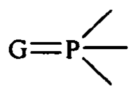 ,

dans lequel G représente un atome d'oxygène, d'azote, de soufre, de sélénium, de tellure ou un groupement =NR, R représentant H ou un groupement alkyle de 1 à 16 atomes de carbone, aryle de 6 à 24 atomes de carbone, ou aralkyle de 7 à 16 atomes de carbone, tel que le groupement thiophosphoryle de formule
;

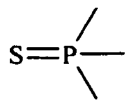

- un groupement bis-phényloxy de formule

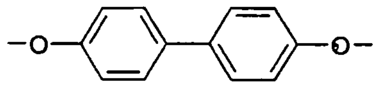

- un groupement 1,2-diamino-éthane de formule

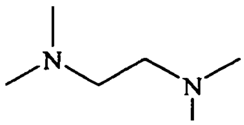 ,

- un groupement 1,4-diamino-butane de formule

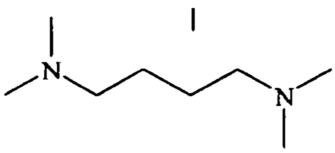 ,

- un groupement cyclotriphosphazène de formule

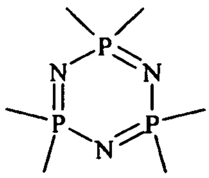 ,

- un groupement cyclotétraphosphazène de formule

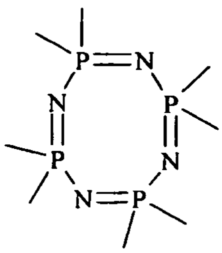 .

En particulier les groupes hydrocarbonés comprenant de 1 à 500 atomes définis ci-dessus peuvent être des fluorophores, ou tout groupement chimique fonctionnel.

La présente invention concerne également des dendrimères à terminaisons monophosphoniques ou bisphosphoniques de génération n, n représentant un nombre entier de 0 à 12, comprenant un noyau central § de valence m, m représentant un entier de 3 à 20, en particulier de 3 à 10 et plus particulièrement de 3 à 8, le noyau établissant m-2 ou m-1 liaisons avec respectivement m-2 ou m-1 chaînes de liaison identiques constituées :

- de chaînes de génération fixées en arborescence autour du noyau sur chacune des liaisons, lorsque n est supérieur ou égal à 1, une chaîne de génération d'une génération donnée étant reliée à

  - une chaîne de génération de la génération immédiatement inférieure à la génération donnée, ou au noyau lorsque la génération donnée vaut 1, et à
  - au moins 2 chaînes de génération de la génération immédiatement supérieure à la génération donnée, ou éventuellement à au moins une chaîne intermédiaire lorsque la génération donnée vaut n,

  un groupe terminal étant fixé à l'extrémité de chaque chaîne de génération de génération n, ou le cas échéant à l'extrémité de chaque chaîne intermédiaire, ou

- de chaînes intermédiaires fixées autour du noyau sur chacune des liaisons, lorsque n vaut 0, un groupe terminal

étant fixé à l'extrémité de chaque chaîne intermédiaire ;

ledit groupe terminal étant représenté par la formule :

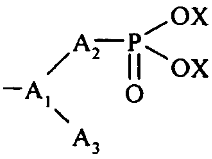

où

$A_1$ représente N ; un groupe P=Y, où Y représente O, S, ou aucun atome ; un groupe N-R ; ou un groupe C-R ; R représentant H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR'R", un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R' et R" représentant indépendamment l'un de l'autre H ou un groupement alkyle de 1 à 16 atomes de carbone, aryle de 6 à 24 atomes de carbone ou aralkyle de 7 à 16 atomes de carbone ;

$A_2$ représente une liaison simple ou une chaîne hydrocarbonée de 1 à 6 chaînons, linéaire, ramifiée ou cyclique, chacun desdits chaînons contenant éventuellement un ou plusieurs hétéroatomes, lesdits hétéroatomes étant préférentiellement choisis parmi un atome de soufre, d'oxygène, de phosphore, ou d'azote, plus préférentiellement d'azote, et étant éventuellement substitués par au moins un substituant choisi parmi H, un groupement alkyle de 1 à 16 atomes de carbone, un halogène, un groupement $-NO_2$, un groupement -NRR', un groupement -CN, un groupement $-CF_3$, un groupement hydroxyle, un groupement alkoxy de 1 à 16 atomes de carbone, un groupement aryle ou hétéroaryle de 1 à 24 atomes de carbone, l'hétéroélément étant préférentiellement choisi parmi l'oxygène, l'azote ou le soufre, un groupement aralkyle de 7 à 16 atomes de carbone, R et R' représentant indépendamment l'un de l'autre H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone ;

$A_3$ représente H, ou une chaîne hydrocarbonée de 1 à 6 chaînons, linéaire, ramifiée ou cyclique, chacun desdits chaînons pouvant éventuellement être choisi parmi un hétéroatome, ledit hétéroatome étant préférentiellement choisi parmi le soufre, l'azote, le phosphore, ou le silicium, plus préférentiellement l'azote, chaque chaînon pouvant être éventuellement substitué par au moins un groupement choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone, ou aralkyle de 7 à 16 atomes de carbone ou

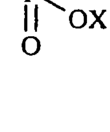 ,

en particulier $A_3$ peut représenter

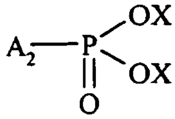

,

chacun des $A_2$ étant identiques ou différents ;
chaque OX, identique ou différent pour chaque groupement phosphonique, représente OH, OAlkyle, où le groupe alkyle comprend de 1 à 16 atomes de carbone, OAryle, où le groupe aryle comprend de 6 à 24 atomes de carbone, OAralkyle, où le groupe aralkyle comprend de 7 à 24 atome de carbone, OAlkylaryle, où le groupe alkylaryle comprend de 7 à 24 atomes de carbone, $OSiR'_1R'_2R'_3$, où $R'_1$, $R'_2$ et $R'_3$, identiques ou différents, représentent un groupement alkyle de 1 à 16 atomes de carbone, ou $O^-M^+$, où $M^+$ est un cation d'éléments du groupe IA, IB, IIA, IIB ou IIIA, IIIB de la classification périodique des éléments, de préférence $M^+$ est choisi parmi les cations des atomes de sodium, de potassium, de cuivre, de calcium, de baryum, de zinc, de magnésium, de lithium et d'aluminium, ou de groupes hydrocarbonés de 1 à 100 atomes de carbone, ou azotés de 0 à 100 atomes de carbone, tel que $NR_1R_2R_3R_4^+$, où, indépendamment les uns des autres $R_1$, $R_2$, $R_3$ et $R_4$ représentent H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR''R''', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R'' et R''' représentant indépendamment l'un de l'autre H ou un groupement alkyle de 1 à 16 atomes de carbone, aryle de 6 à 24 atomes de carbone ou aralkyle de 7 à 16 atomes de carbone ;
les 1 ou 2 liaisons restantes étant fixées à des groupes de liaison, identiques ou différents, éventuellement liés ensemble, notamment par l'intermédiaire d'une liaison covalente, constitués
soit d'une partie des chaînes de liaison définies ci-dessus,
soit d'un atome d'hydrogène,
soit de groupes hydrocarbonés comprenant de 1 à 500 atomes de carbone,
lesdits groupes hydrocarbonés étant notamment constitués de H ou d'une chaîne hydrocarbonée de 1 à 16 chaînons, linéaire, ramifiée ou cyclique, contenant éventuellement une ou plusieurs doubles ou triples liaisons, chacun desdits chaînons pouvant éventuellement être choisi parmi un hétéroatome, ledit hétéroatome étant préférentiellement choisi parmi un atome d'azote, d'oxygène, de phosphore, de silicium ou de soufre, un groupement aryle de 6 à 24 atomes de carbone, un groupement hétéroaryle de 1 à 24 atomes de carbone, un groupement carboxyle, un groupement >C=NR, chaque chaînon pouvant être éventuellement substitué par au moins un substituant choisi parmi un groupement hydroxyle, un groupement - NR''R''', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R'' et R''' représentant indépendamment l'un de l'autre H ou un groupement alkyle de 1 à 16 atomes de carbone, aryle de 6 à 24 atomes de carbone ou aralkyle de 7 à 16 atomes de carbone, le premier chaînon de ladite chaîne hydrocarbonée étant de préférence un oxygène ou un azote ;
ledit noyau central § représentant un groupe comprenant de 1 à 500 atomes, et contenant éventuellement un ou plusieurs hétéroatomes, lesdits hétéroatomes étant préférentiellement choisis parmi l'oxygène, le soufre, l'azote, le phosphore ou le silicium.

En particulier les groupes hydrocarbonés comprenant de 1 à 500 atomes définis ci-dessus peuvent être des fluorophores, ou tout groupement chimique fonctionnel.

Selon un mode de réalisation particulier des dendrimères définis ci-dessus, les chaînes de génération sont choisies parmi toute chaîne hydrocarbonée de 1 à 12 chaînons, linéaire, ramifiée ou cyclique, contenant éventuellement une ou plusieurs doubles ou triples liaisons, chacun desdits chaînons pouvant éventuellement être choisi parmi un hétéroatome, ledit hétéroatome étant préférentiellement choisi parmi l'azote, l'oxygène, le soufre, le phosphore ou le silicium, un groupement aryle de 6 à 24 atomes de carbone, un groupement hétéroaryle de 1 à 24 atomes de carbone, l'hétéroélément étant préférentiellement choisi parmi l'oxygène, l'azote ou le soufre, un groupement carboxyle, un groupement >C=NR, chaque chaînon pouvant éventuellement être substitué par au moins un substituant choisi parmi un groupement alkyle de 1 à 16 atomes de carbone, un atome d'halogène, un groupement $NO_2$, un groupement -NRR', un groupement -CN, un groupement $-CF_3$, un groupement hydroxyle, un groupement alkoxy de 1 à 16 atomes de carbone, un groupement aryle de 6 à 24 atomes de carbone, ou un groupement aralkyle de 7 à 16 atomes de carbone, R et R' représentant indépendamment l'un de l'autre H ou un groupement alkyle de 1 à 16 atomes de carbone, aryle de 6 à 24 atomes de carbone, ou aralkyle de 7 à 16 atomes de carbone.

Selon un autre mode de réalisation préféré des dendrimères définis ci-dessus, les chaînes intermédiaires sont

choisies parmi les groupes correspondant à la formule :

-J-K-L-

où

- J représente un atome d'oxygène, de soufre, ou un groupement -NR- ;
- K représente un groupement aryle de 6 à 24 atomes de carbone, hétéroaryle de 1 à 24 atomes de carbone, l'hétéroélément étant préférentiellement choisi parmi l'oxygène, l'azote ou le soufre, alkyle de 1 à 16 atomes de carbone, chacun pouvant être éventuellement substitué par un atome d'halogène ou un groupement $-NO_2$, -NRR', - CN, $-CF_3$, -OH, alkyle de 1 à 16 atomes de carbone, aryle de 6 à 24 atomes de carbone, aralkyle de 7 à 16 atomes de carbone ;
- L représente une chaîne hydrocarbonée de 0 à 10 chaînons, notamment de 0 à 6 chaînons, linéaire, ramifiée ou cyclique, contenant éventuellement une ou plusieurs doubles ou triples liaisons, chacun desdits chaînons pouvant éventuellement être un hétéroatome, ledit hétéroatome étant préférentiellement choisi parmi l'oxygène, le soufre, l'azote, le phosphore, le silicium chaque chaînon pouvant être éventuellement substitué par au moins un substituant choisi parmi un groupement alkyle de 1 à 16 atomes de carbone, un halogène, un atome d'oxygène, $-NO_2$, -NRR', -CN, $-CF_3$, -OH, un groupement alkoxy de 1 à 16 atomes de carbone, un groupement aryle de 6 à 24 atomes de carbone, un groupement aralkyle de 7 à 16 atomes de carbone ;

R et R' représentant indépendamment l'un de l'autre H ou un groupement alkyle de 1 à 16 atomes de carbone, aryle de 6 à 24 atomes de carbone, ou aralkyle de 7 à 16 atomes de carbone.

Selon un mode de réalisation particulièrement préféré de l'invention, les dendrimères définis ci-dessus sont de structure PMMH, PAMAM ou DAB.

Selon un autre mode de réalisation particulièrement préféré de l'invention, les dendrimères définis ci-dessus sont à terminaisons bisphosphoniques correspondant à la formule générale (7) suivante :

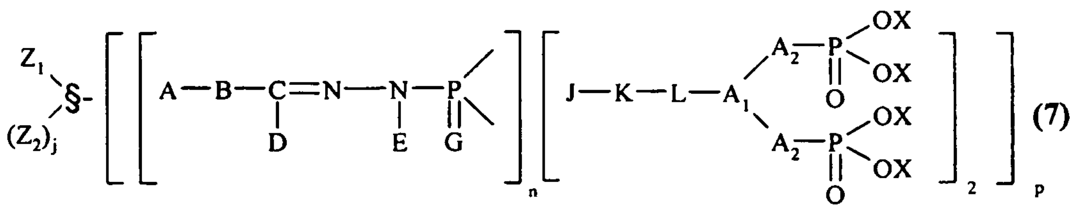

(7)

où n représente un entier de 0 à 3, m représente 3, 6 ou 8, p représente m - 1 ou m - 2, et j représente 0 lorsque p représente m - 1 et 1 lorsque p représente m - 2, à savoir :

- lorsque p = m - 1, la formule (7) correspond à la formule (8) suivante :

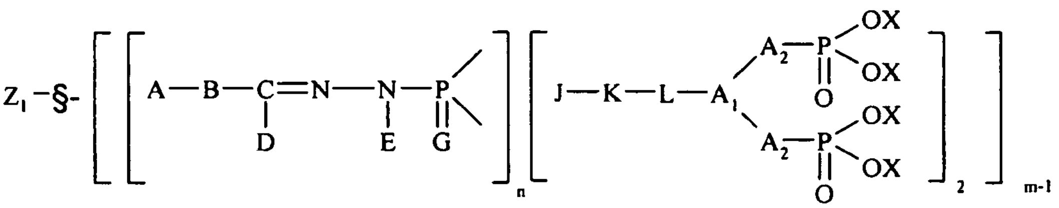

(8)

- lorsque p = m - 2, la formule (7) correspond à la formule (9) suivante

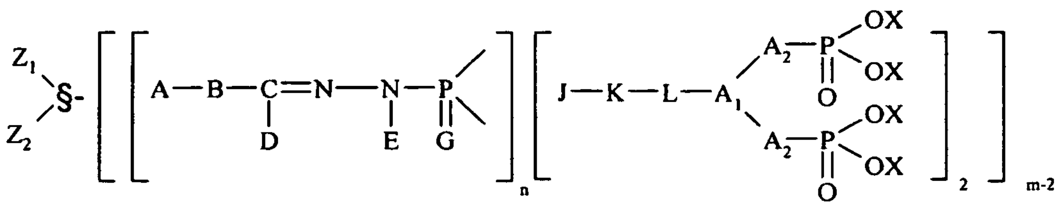

(9)

et dans lesquelles formules :

- le noyau central § est choisi parmi les groupes suivants :

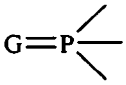 , 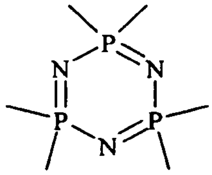 , 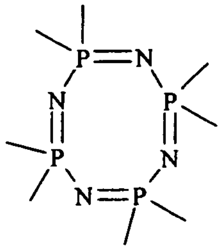 ;

- la chaîne de génération correspond à la formule :

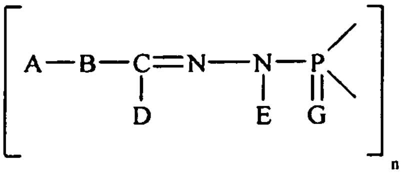

où

- A représente un atome d'oxygène, de soufre, de phosphore ou un groupement -NR-;
- B représente un groupement aryle de 6 à 24 atomes de carbone, hétéroaryle de 1 à 24 atomes de carbone, alkyle de 1 à 16 atomes de carbone, chacun pouvant être éventuellement substitué par un atome d'halogène ou un groupement -$NO_2$, -NRR', - CN, -$CF_3$, -OH, alkyle de 1 à 16 atomes de carbone, aryle de 6 à 24 atomes de carbone, aralkyle de 7 à 16 atomes de carbone ;
- D représente un atome d'hydrogène, un groupement alkyle de 1 à 16 atomes de carbone, alkoxy de 1 à 16 atomes de carbone, aryle de 6 à 24 atomes de carbone, aralkyle de 7 à 16 atomes de carbone, chacun pouvant être éventuellement substitué par un atome d'halogène ou un groupement -$NO_2$, -NRR', -CN, -$CF_3$, -OH, alkyle de 1 à 16 atomes de carbone, aryle de 6 à 24 atomes de carbone, aralkyle de 7 à 16 atomes de carbone ;
- E représente un atome d'hydrogène, un groupement alkyle de 1 à 16 atomes de carbone, alkoxy de 1 à 16 atomes de carbone, aryle de 6 à 24 atomes de carbone, aralkyle de 7 à 16 atomes de carbone, chacun pouvant être éventuellement substitué par un atome d'halogène ou un groupement -$NO_2$, -NRR', -CN, -$CF_3$, -OH, alkyle de 1 à 16 atomes de carbone, aryle de 6 à 24 atomes de carbone, aralkyle de 7 à 16 atomes de carbone ;
- G représente un atome d'oxygène, d'azote, de soufre, de sélénium, de tellure ou un groupement =NR ;
  R et R' représentant indépendamment l'un de l'autre H ou un groupement alkyle de 1 à 16 atomes de carbone, aryle de 6 à 24 atomes de carbone, ou aralkyle de 7 à 16 atomes de carbone ;

- la chaîne intermédiaire correspond à la formule :

-J-K-L-

où

- J représente un atome d'oxygène, de soufre, ou un groupement -NR- ;
- K représente un groupement aryle de 6 à 24 atomes de carbone, hétéroaryle de 1 à 24 atomes de carbone, l'hétéroélément étant préférentiellement choisi parmi l'oxygène, l'azote ou le soufre, alkyle de 1 à 16 atomes de carbone, chacun pouvant être éventuellement substitué par un atome d'halogène ou un groupement -$NO_2$, -NRR', - CN, -$CF_3$, -OH, alkyle de 1 à 16 atomes de carbone, aryle de 6 à 24 atomes de carbone, aralkyle de 7 à 16 atomes de carbone ;
- L représente une chaîne hydrocarbonée de 0 à 10 chaînons, notamment de 0 à 6 chaînons, linéaire, ramifiée ou cyclique, contenant éventuellement une ou plusieurs doubles ou triples liaisons, chacun desdits chaînons pouvant éventuellement être un hétéroatome, ledit hétéroatome étant préférentiellement choisi parmi l'oxygène, le soufre, l'azote, le phosphore, le silicium, chaque chaînon pouvant être éventuellement substitué par au moins un substituant choisi parmi un groupement alkyle de 1 à 16 atomes de carbone, un halogène, un atome d'oxygène, -$NO_2$, -NRR',

-CN, $-CF_3$, -OH, un groupement alkoxy de 1 à 16 atomes de carbone, un groupement aryle de 6 à 24 atomes de carbone, un groupement aralkyle de 7 à 16 atomes de carbone ;
R et R' représentant indépendamment l'un de l'autre H ou un groupement alkyle de 1 à 16 atomes de carbone, aryle de 6 à 24 atomes de carbone, ou aralkyle de 7 à 16 atomes de carbone ;

- le groupe terminal correspond à la formule :

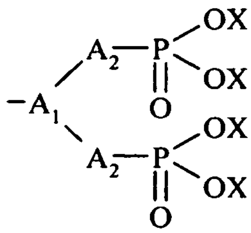

où A1, A2 et X ont été définis précédemment, chacun des X étant identique ou différent ;
$Z_1$ et $Z_2$ étant identiques ou différents, éventuellement liés ensemble, notamment par l'intermédiaire d'une liaison covalente, et représentant H ou une chaîne hydrocarbonée de 1 à 16 chaînons, linéaire, ramifiée ou cyclique, contenant éventuellement une ou plusieurs doubles ou triples liaisons, chacun desdits chaînons pouvant éventuellement être choisi parmi un hétéroatome, ledit hétéroatome étant préférentiellement choisi parmi un atome d'azote, d'oxygène, de phosphore, de silicium ou de soufre, un groupement aryle de 6 à 24 atomes de carbone, un groupement hétéroaryle de 1 à 24 atomes de carbone, un groupement carboxyle, un groupement >C=NR, chaque chaînon pouvant être éventuellement substitué par au moins un substituant choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement - $CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone, le premier chaînon de ladite chaîne hydrocarbonée étant de préférence un oxygène ou un azote.

Selon encore un autre mode de réalisation particulièrement préféré de l'invention, les dendrimères tels que définis ci-dessus sont de structure PMMH, et correspondent à la formule générale (8), dans laquelle

§ représente

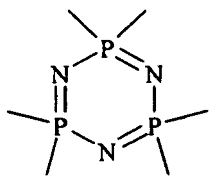 ;

m représente 6 ;
p représente 5 ;
n représente 0, 1, ou 2 ;
A représente un atome d'oxygène ;
B représente un groupement benzénique ;
D représente un hydrogène ;
E représente un groupement méthyle ;
G représente un atome de soufre ;
J représente un atome d'oxygène ;
K représente un groupement benzénique ;
L représente une chaîne hydrocarbonée saturée linéaire non substituée à deux atomes de carbone ;
A, représente un atome d'azote ;
$A_2$ représente un groupement $CH_2$ ;
X représente un groupement méthyle, ou un atome d'hydrogène ou de sodium ;
$Z_1$ représente un groupement phényloxy ;

ledit dendrimère étant désigné GCn', n étant défini ci-dessus.

Selon un mode de réalisation tout particulièrement préféré de l'invention, les dendrimères définis ci-dessus correspondent aux composés de formules suivantes :

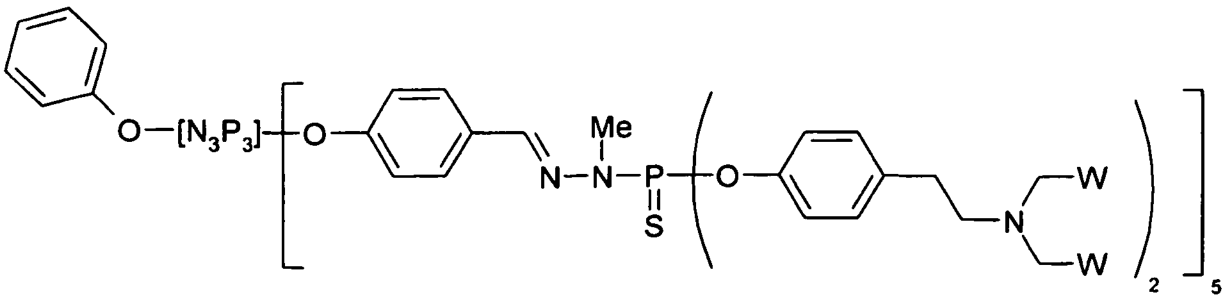

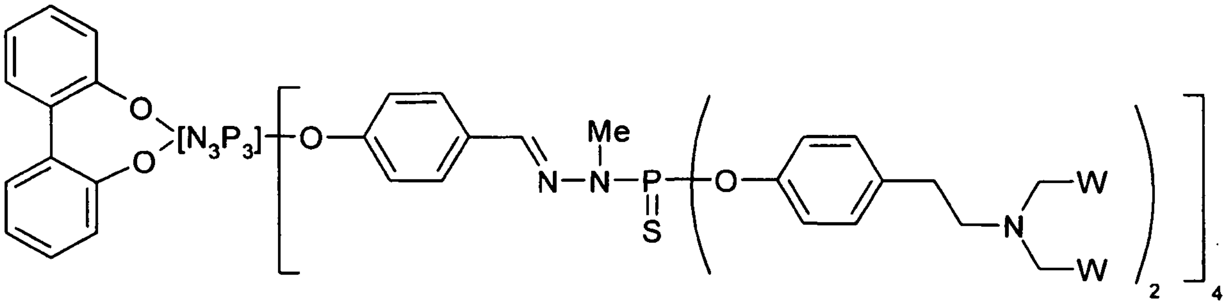

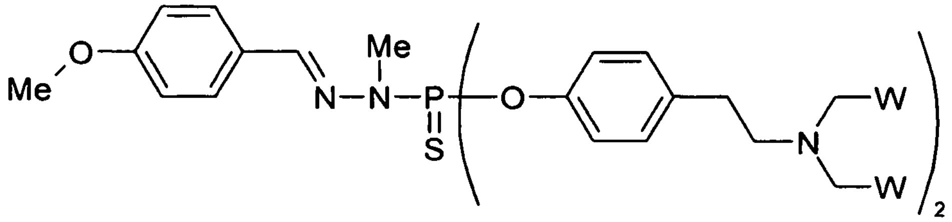

dans lesquelles W représente $PO_3Me_2$, $PO_3HNa$, ou $PO_3H_2$, lesdits dendrimères correspondant notamment au composé GC1' de formule (10) suivante :

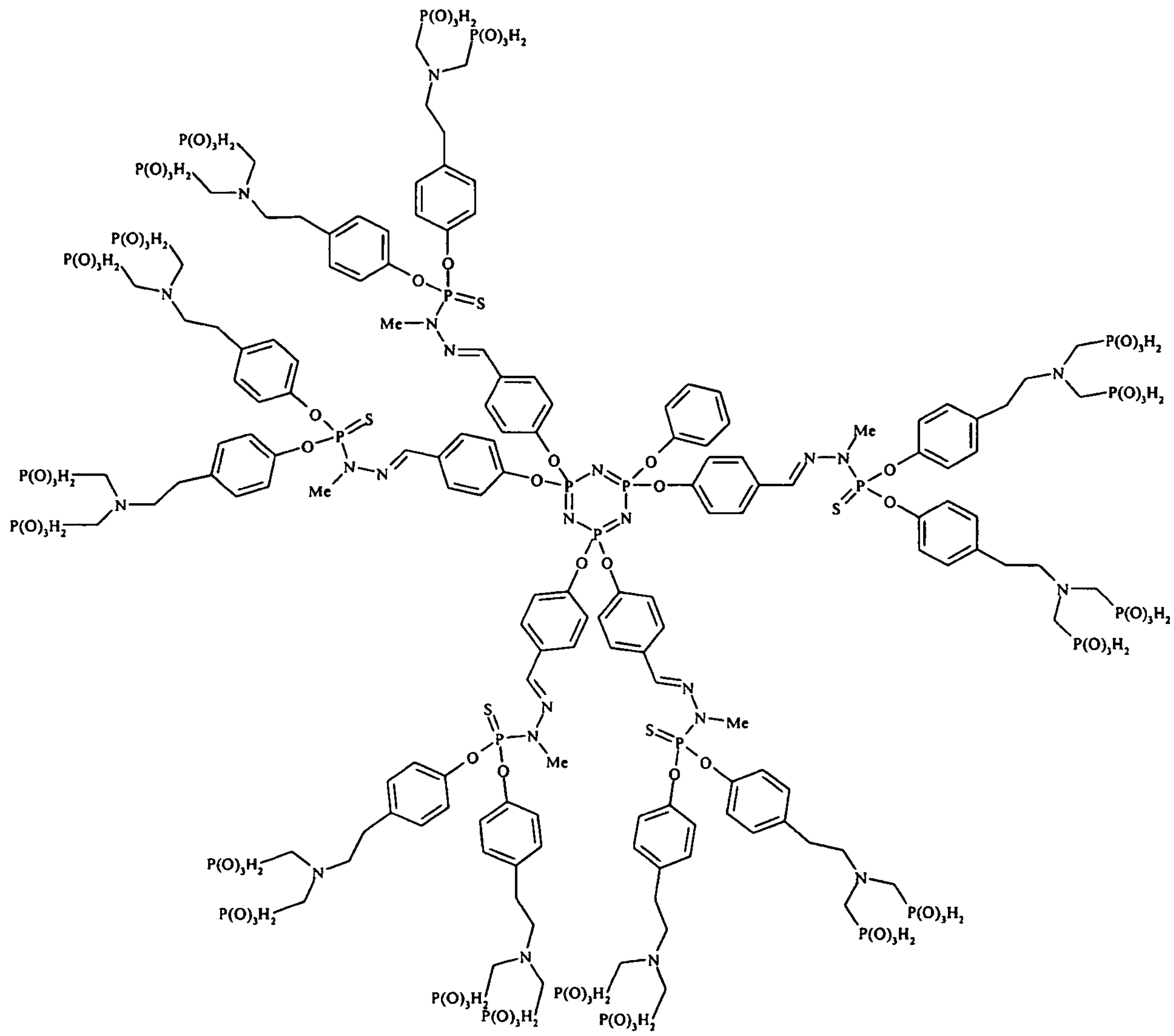

**(10)**

Selon un autre mode de réalisation tout particulièrement préféré de l'invention, les dendrimères définis ci-dessus correspondent au composé de formule suivante :

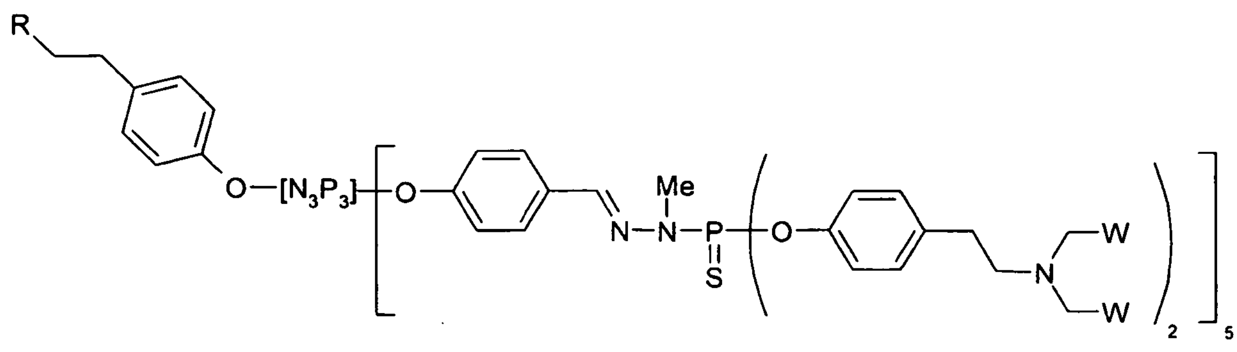

dans laquelle W représente $PO_3Me_2$, $PO_3HNa$, ou $PO_3H_2$ et R représente un groupe fluorescent choisi parmi :

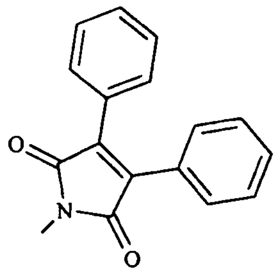

,

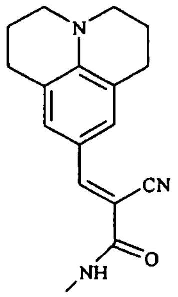

,

lesdits dendrimères correspondant notamment aux composés de formules suivantes :

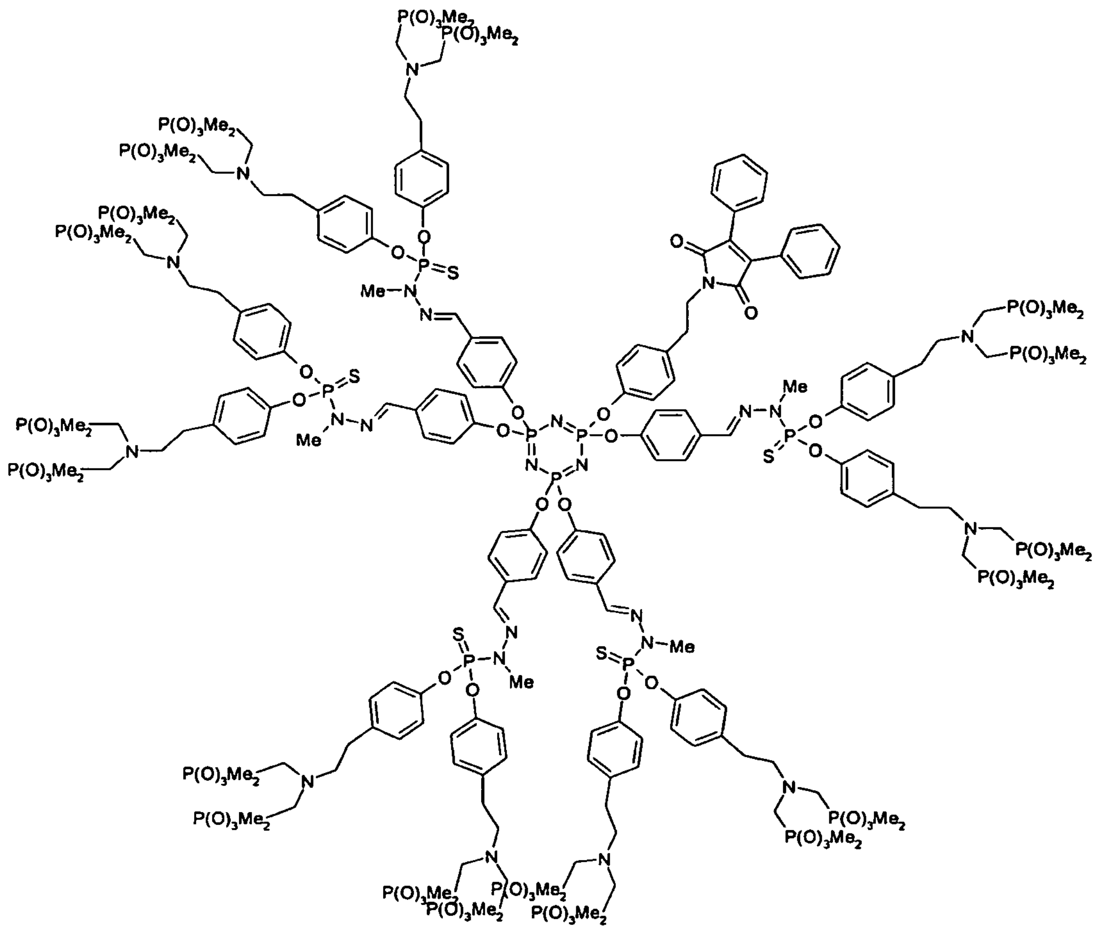

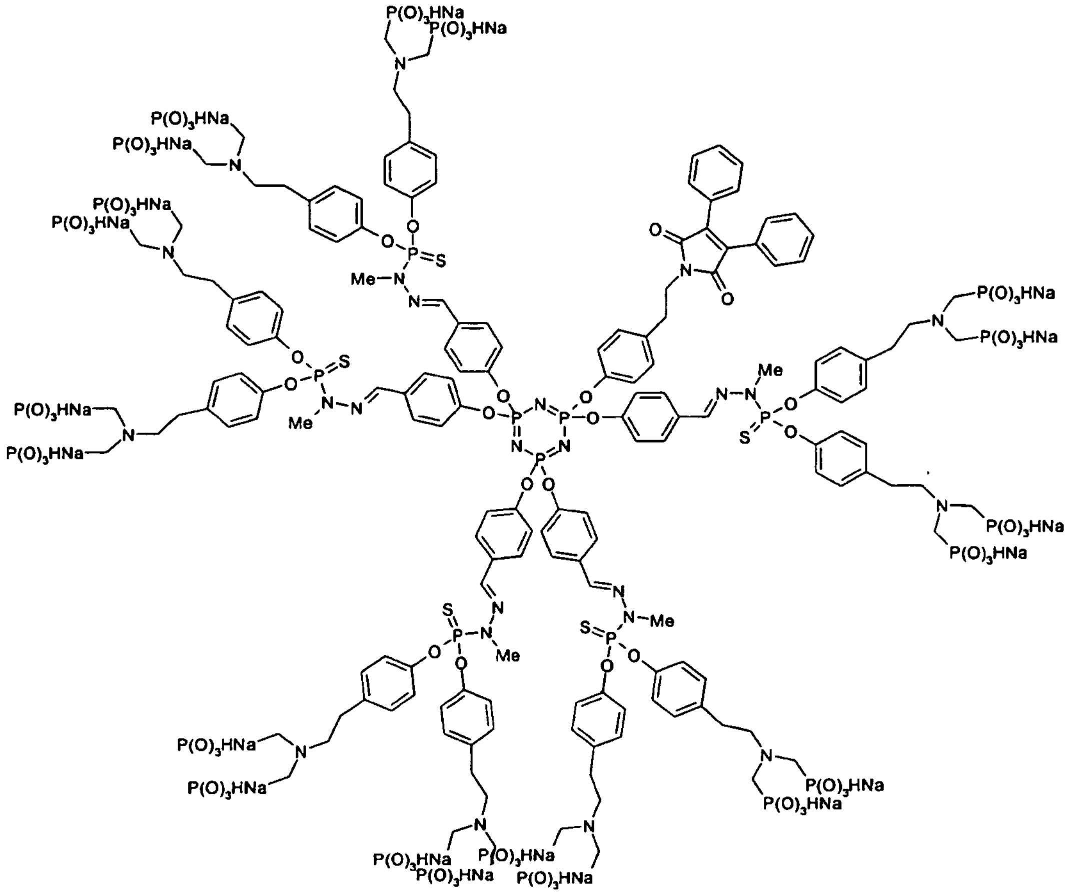

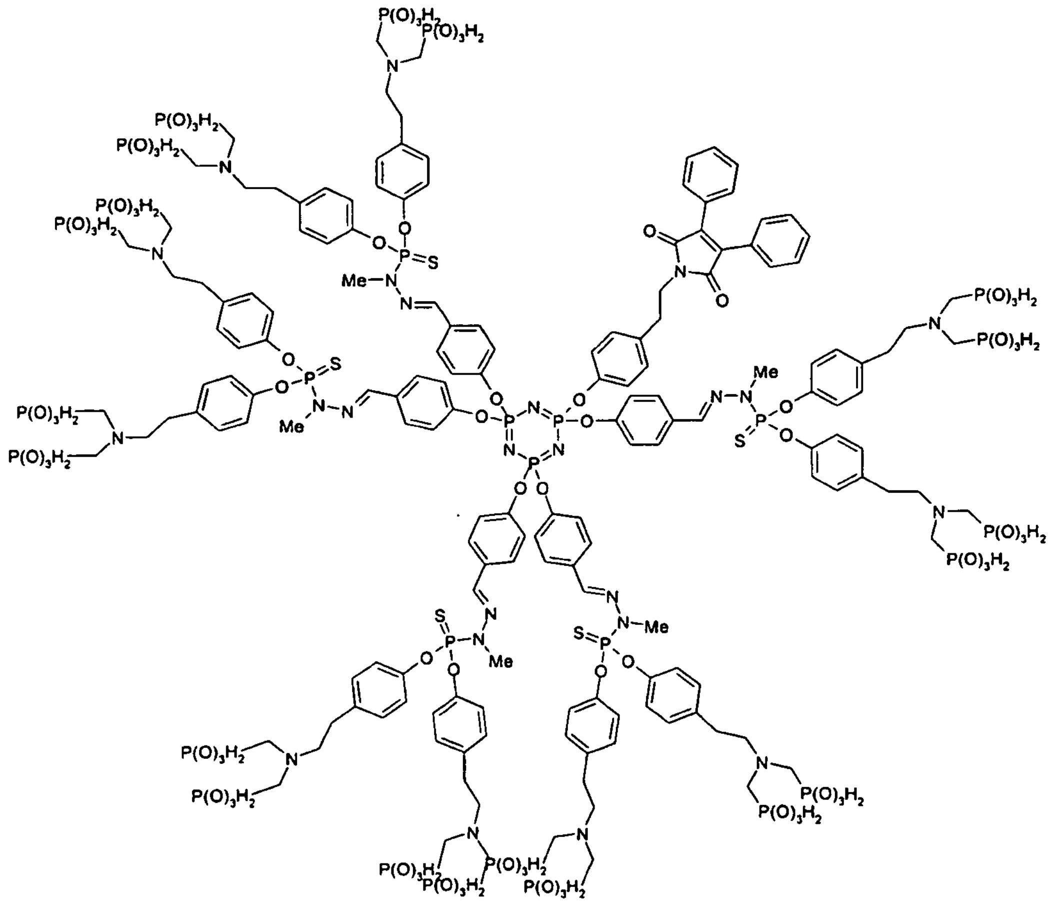

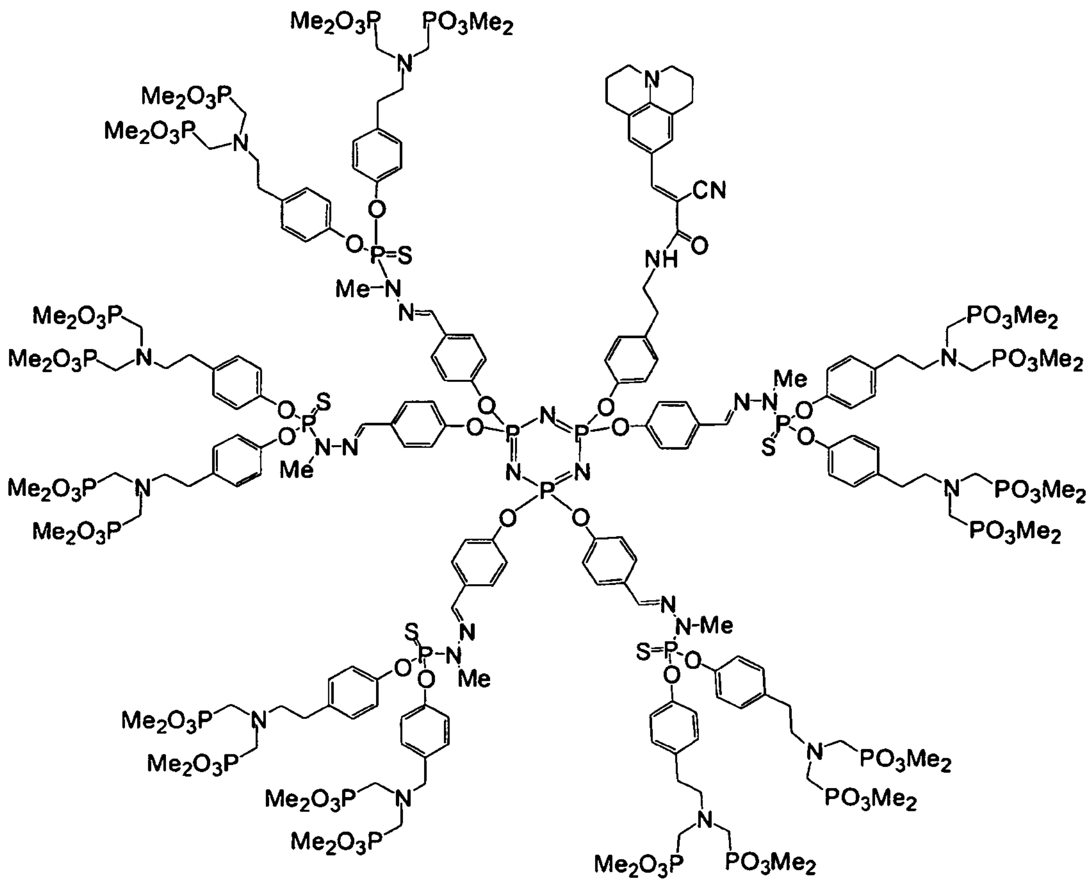

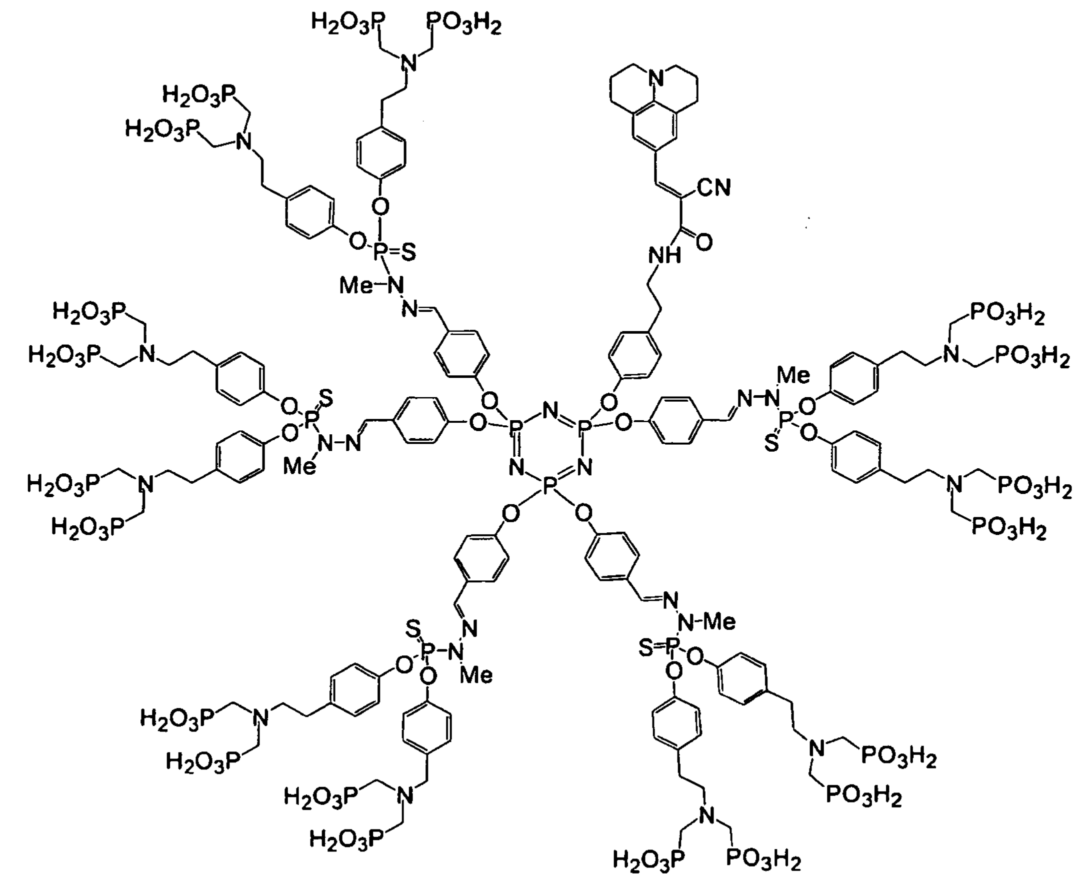

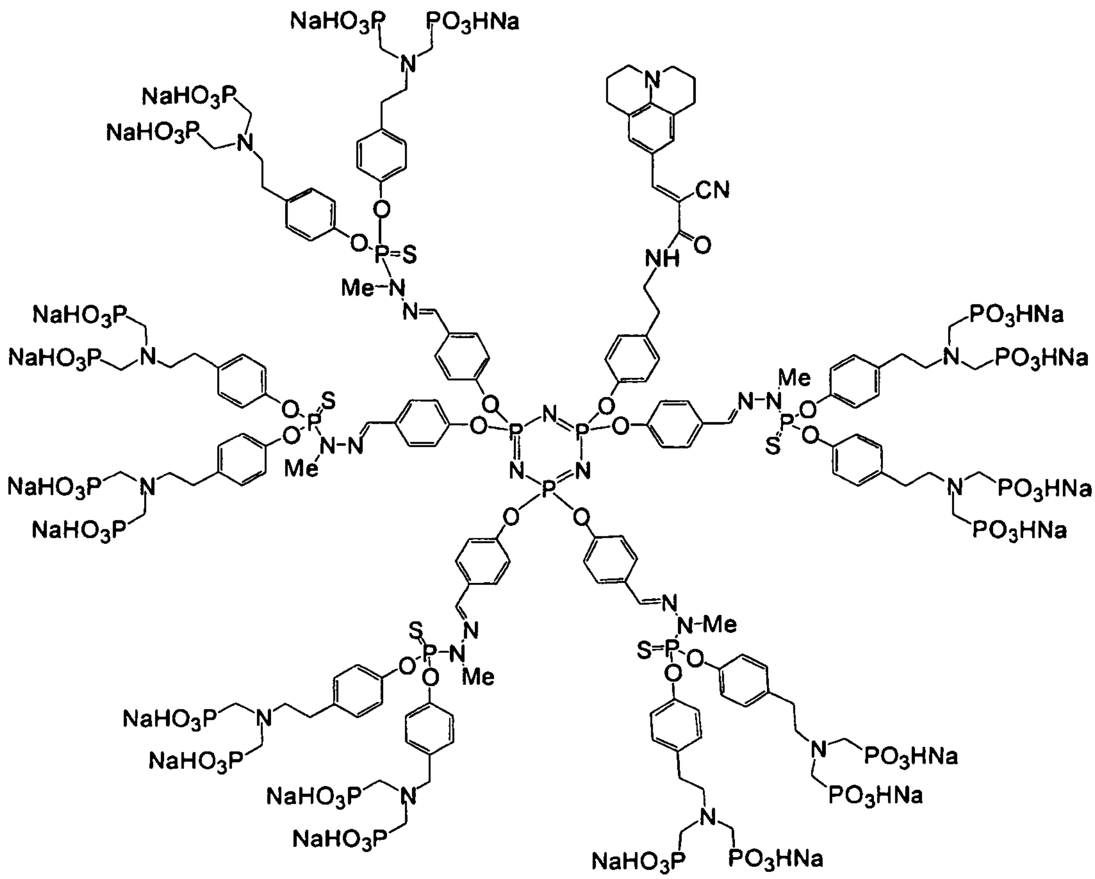

Avantageusement, ces composés sont fluorescents. Leur synthèse est décrite dans les exemples 88 à 90.

La présente invention concerne également les dendrimères à terminaisons bisphosphoniques tels que définis ci-dessus, de formule suivante :

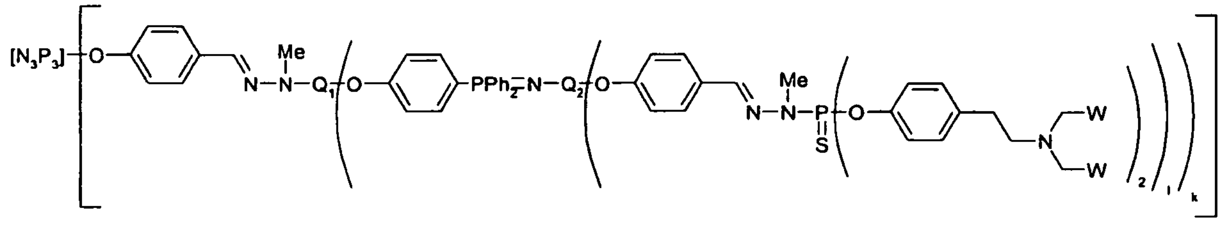

dans laquelle W représente $PO_3Me_2$, $PO_3HNa$, ou $PO_3H_2$, $Q_1$ et $Q_2$, identiques ou différents, représentent P=S ou le cyclotriphosphazène ($N_3P_3$), 1 représente 2 lorsque $Q_2$ représente P=S ou 5 lorsque $Q_2$ représente $N_3P_3$ et k représente 2 lorsque $Q_1$ représente P=S ou 5 lorsque $Q_1$ représente $N_3P_3$, lesdits dendrimères étant notamment représentés par les formules suivantes :

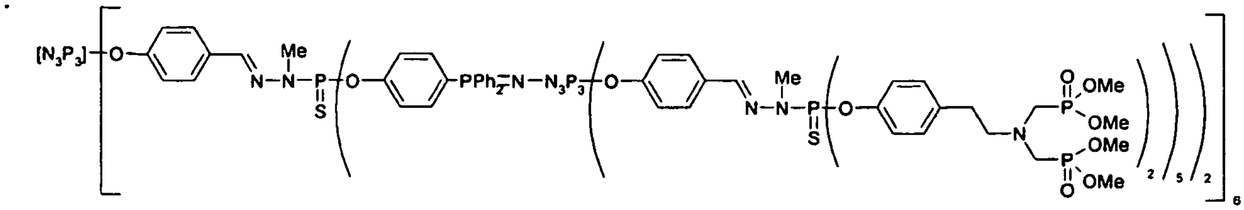

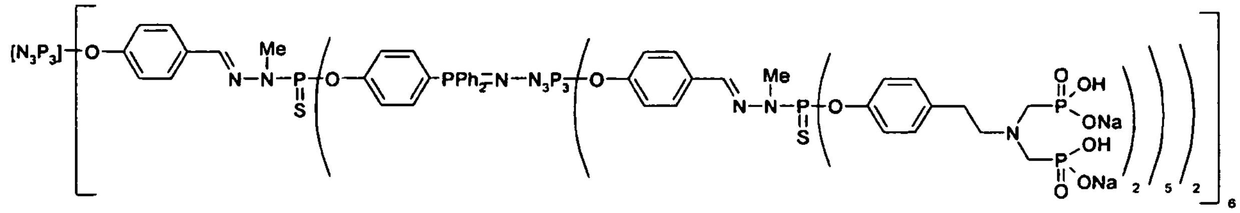

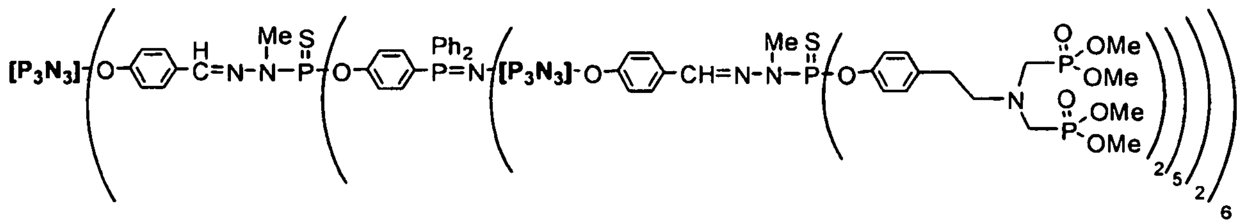

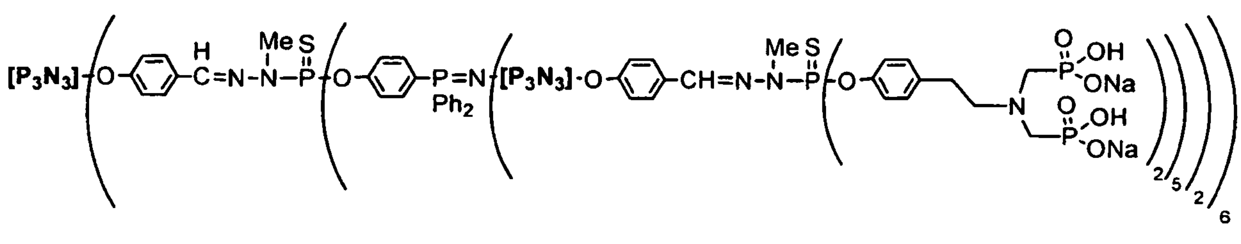

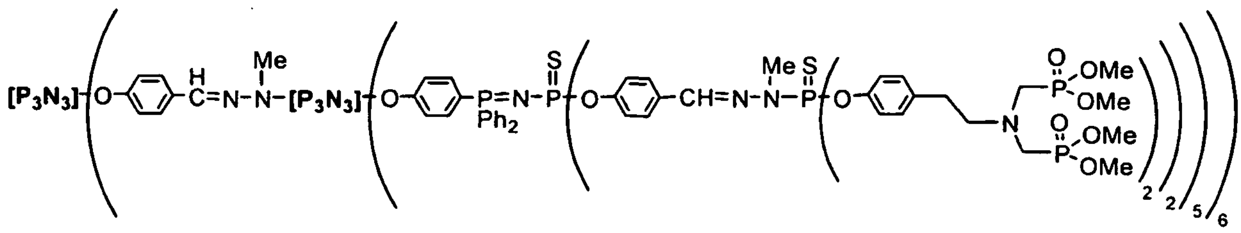

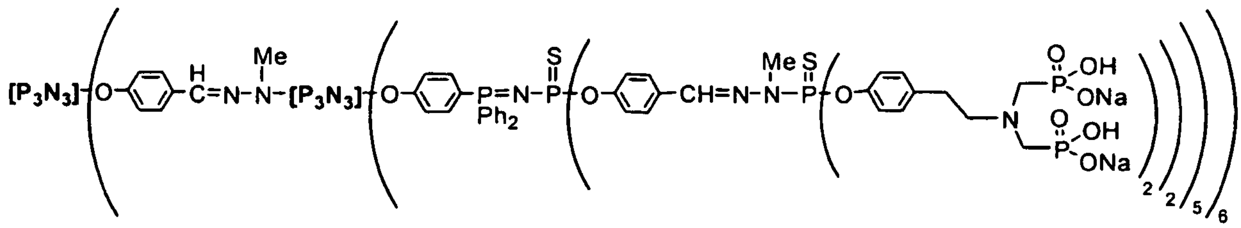

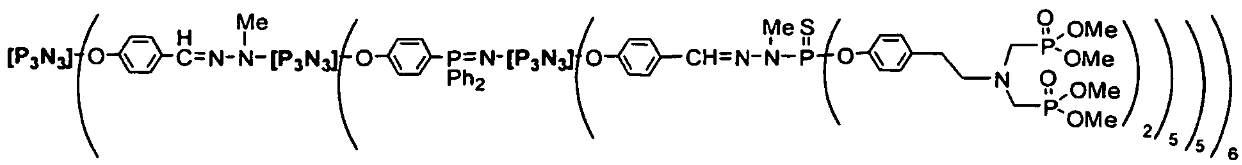

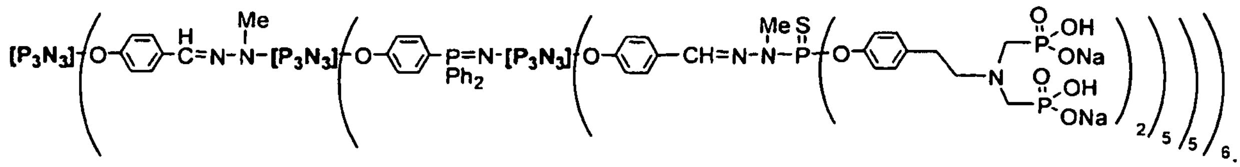

Dans ces formules de dendrimères, $P_3N_3$ représente le noyau cyclotriphosphazène.

Ces dendrimères à terminaisons bisphophoniques sont notamment caractérisés en ce que le coefficient de divergence varie d'une génération à l'autre pour un dendrimère donné. De plus, pour chacun de ces dendrimères, au moins une génération correspond à un coefficient de divergence supérieur à 2.

Les composés de formules :

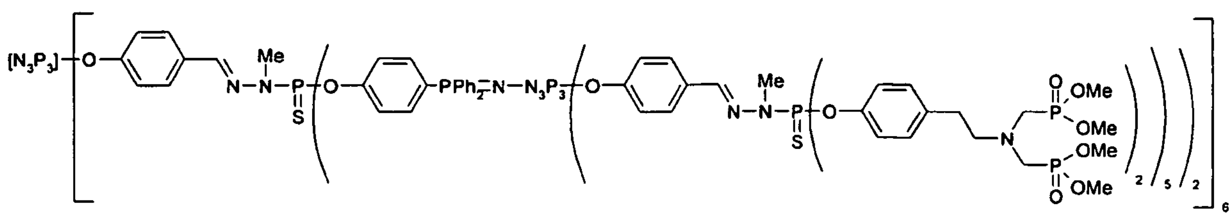

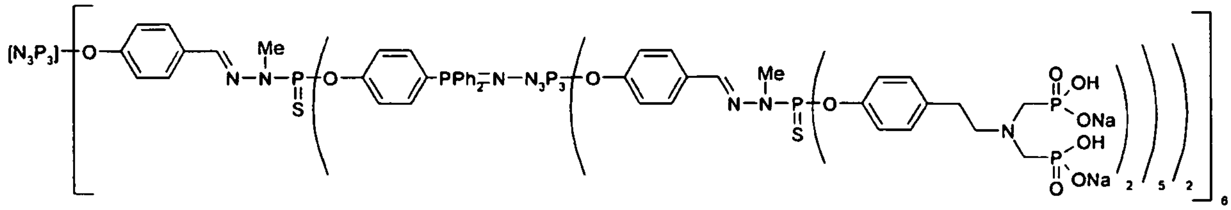

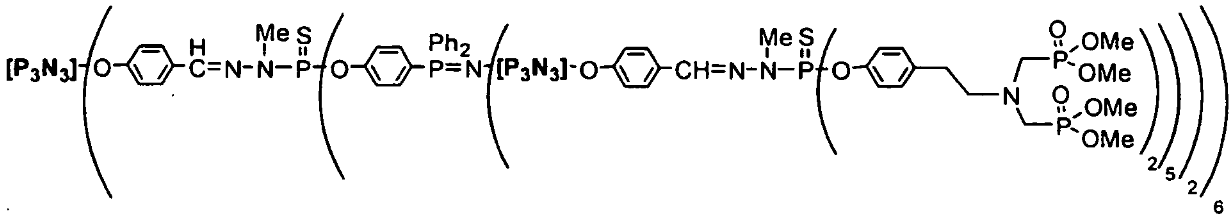

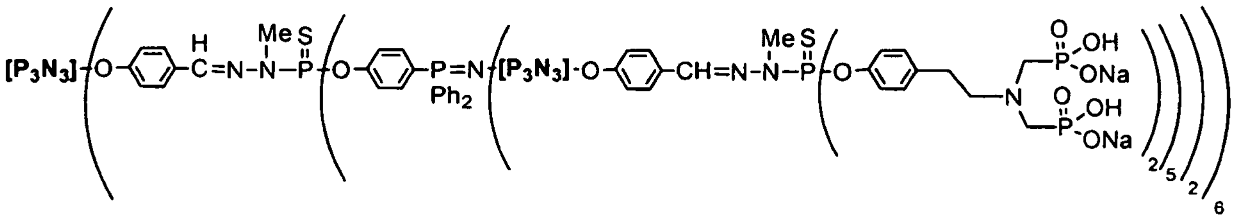

peuvent être synthétisés à partir du composé de formule :

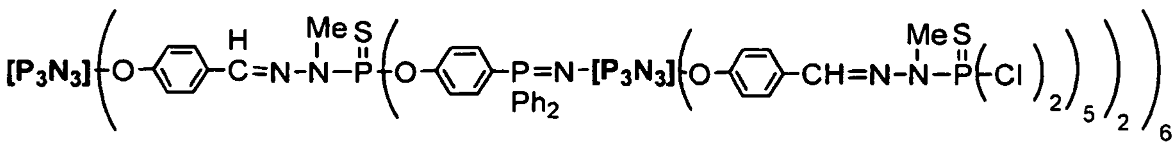

qui peut lui-même être synthétisé à partir du composé 6-2-5 défini dans l'exemple 40 et décrit dans V. Maraval et al. Angew. Chem. Int. Ed. (2003) 42, 1822.

Les composés de formules :

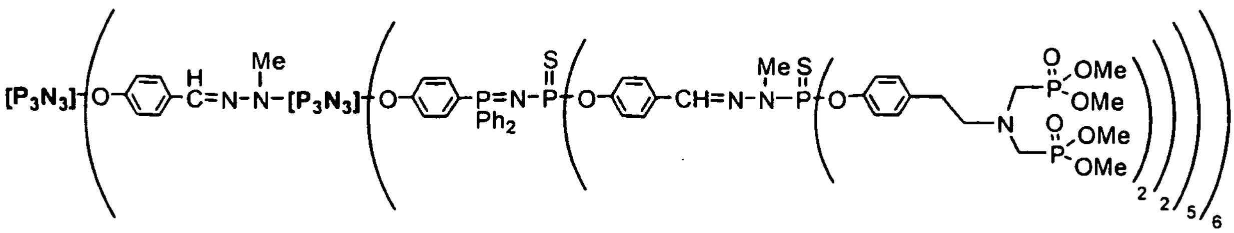

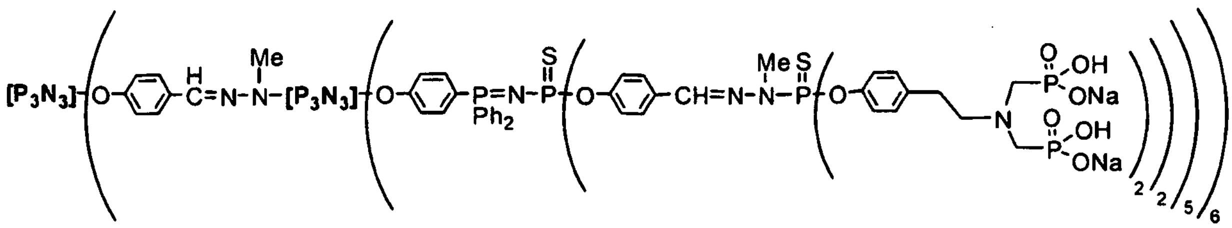

peuvent être synthétisés à partir du composé de formule :

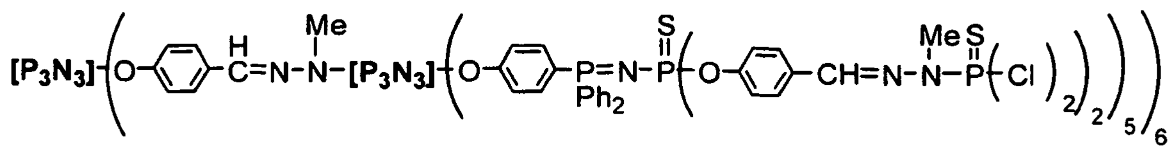

qui peut lui-même être synthétisé à partir du composé 6-5-2 défini dans l'exemple 40 et décrit dans V. Maraval et al. Angew. Chem. Int. Ed. (2003) 42, 1822.

Les composés de formules :

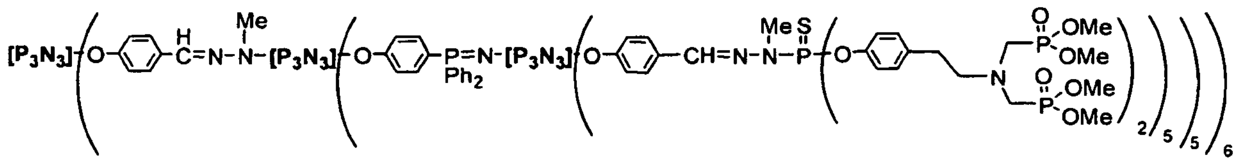

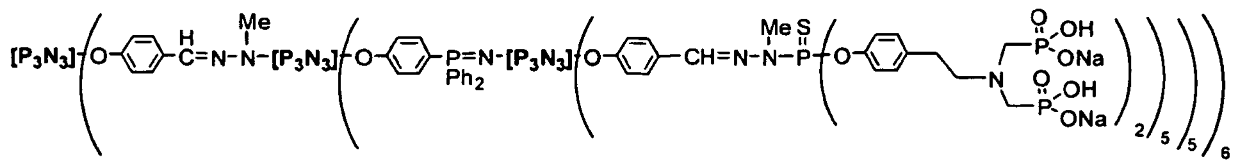

peuvent être synthétisés à partir du composé de formule :

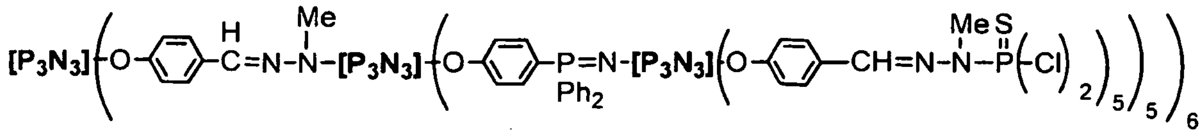

qui peut lui-même être synthétisé à partir du composé 6-5-5 défini dans l'exemple 40 et décrit dans V. Maraval et al. Angew. Chem. Int. Ed. (2003) 42, 1822.

La synthèse des composés de l'invention est plus particulièrement décrite dans les exemples 77 à 85.

La présente invention concerne également, les dendrimères à terminaisons monophosphoniques ou bisphosphoniques, tels que définis ci-dessus, de formule suivante :

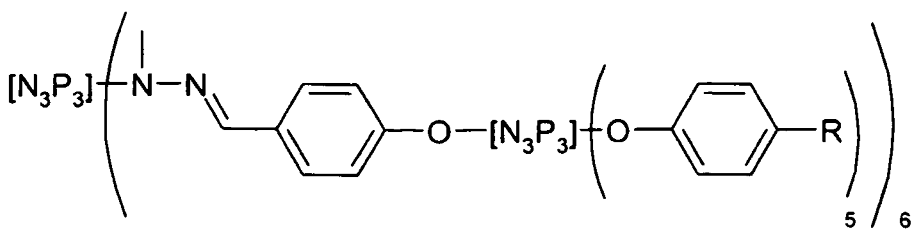

dans laquelle R représente un groupe choisi parmi

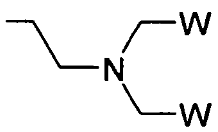 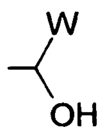

où W représente $PO_3Me_2$, $PO_3HNa$, ou $PO_3H_2$.

La présente invention concerne également les dendrimères à terminaisons bisphosphoniques, tels que définis ci-dessus, de formule suivante :

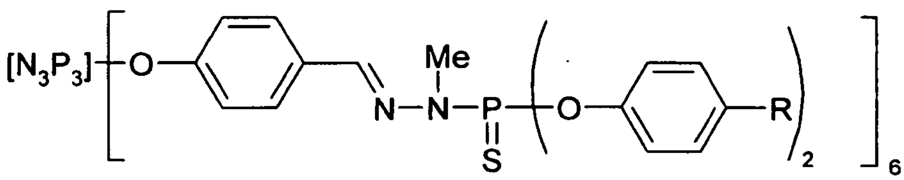

dans laquelle R représente un groupe choisi parmi :

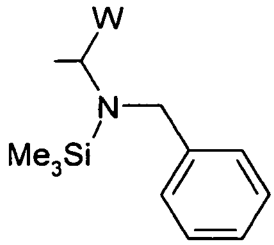

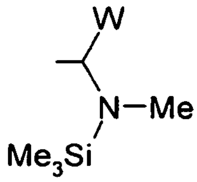

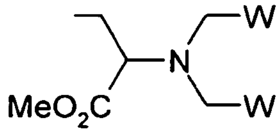

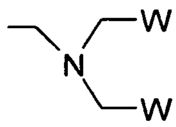

où W représente $PO_3Si_2Me_6$, $PO_3Me_2$, $PO_3HNa$, ou $PO_3H_2$.

La présente invention concerne également les dendrimères à terminaisons monophosphoniques, tels que définis ci-dessus, de formule suivante :

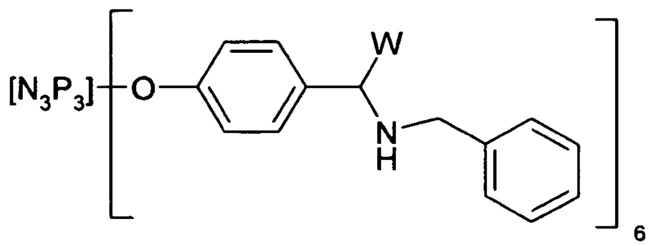

dans laquelle W représente $PO_3Me_2$, $PO_3HNa$, ou $PO_3H_2$.

La présente invention concerne également les dendrimères à terminaisons bisphosphoniques, tels que définis ci-dessus, de formule suivante :

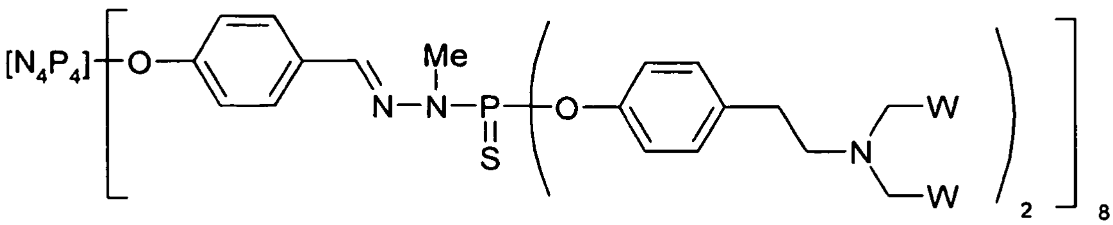

dans laquelle W représente $PO_3Me_2$, $PO_3HNa$, ou $PO_3H_2$.

La présente invention concerne également les dendrimères à terminaisons bisphosphoniques, tels que définis ci-dessus, de formule suivante :

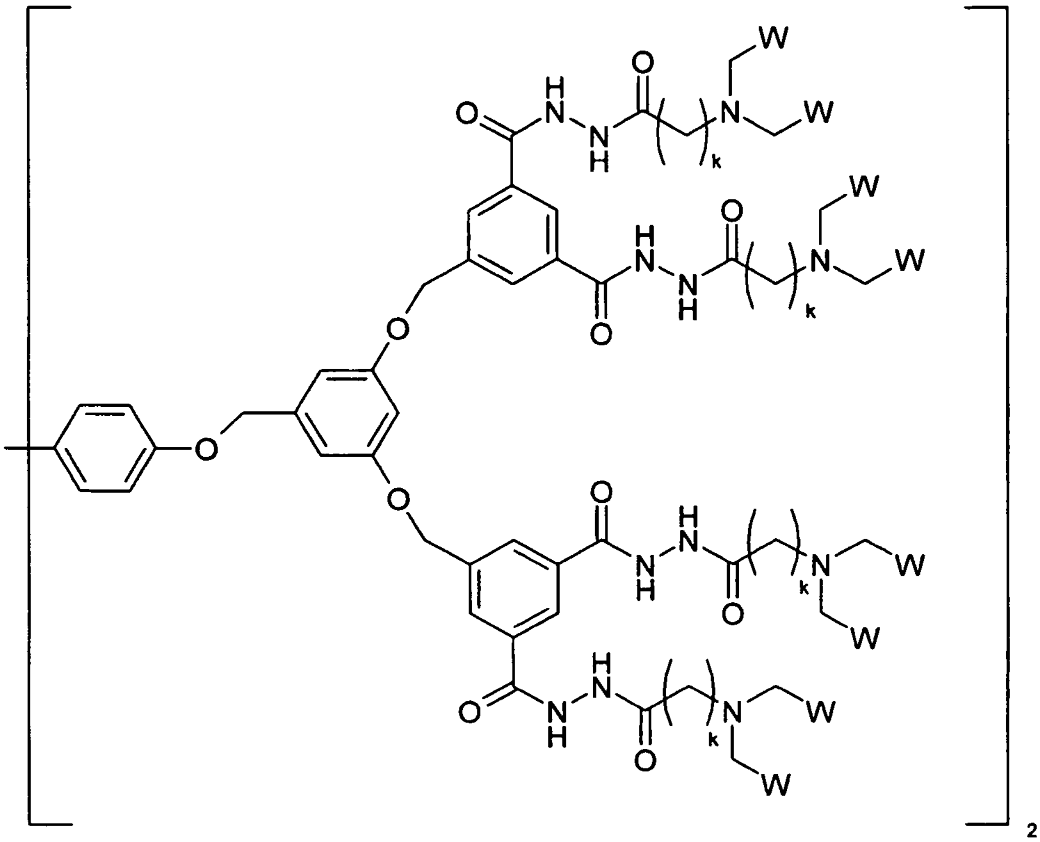

dans laquelle W représente $PO_3Me_2$, $PO_3HNa$, ou $PO_3H_2$ et k représente 1, 2 ou 3

La présente invention concerne également les dendrimères à terminaisons bisphosphoniques, tels que définis ci-dessus, de formule suivante :

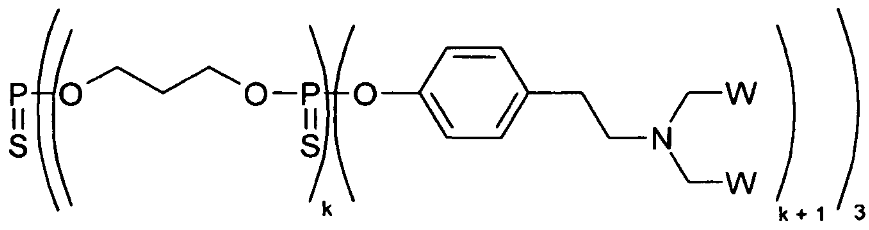

dans laquelle W représente $PO_3Me_2$, $PO_3HNa$, ou $PO_3H_2$ et k représente 0 ou 1.

La présente invention concerne également les dendrimères à terminaisons bisphosphoniques, tels que définis ci-dessus, de formule suivante :

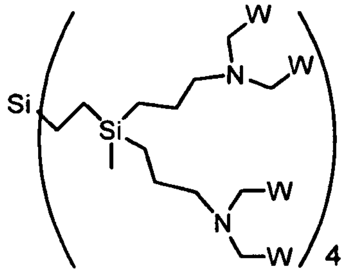

dans laquelle W représente $PO_3Me_2$, $PO_3HNa$, ou $PO_3H_2$.

La présente invention concerne également les dendrimères à terminaisons bisphosphoniques, tels que définis ci-dessus, de formule suivante :

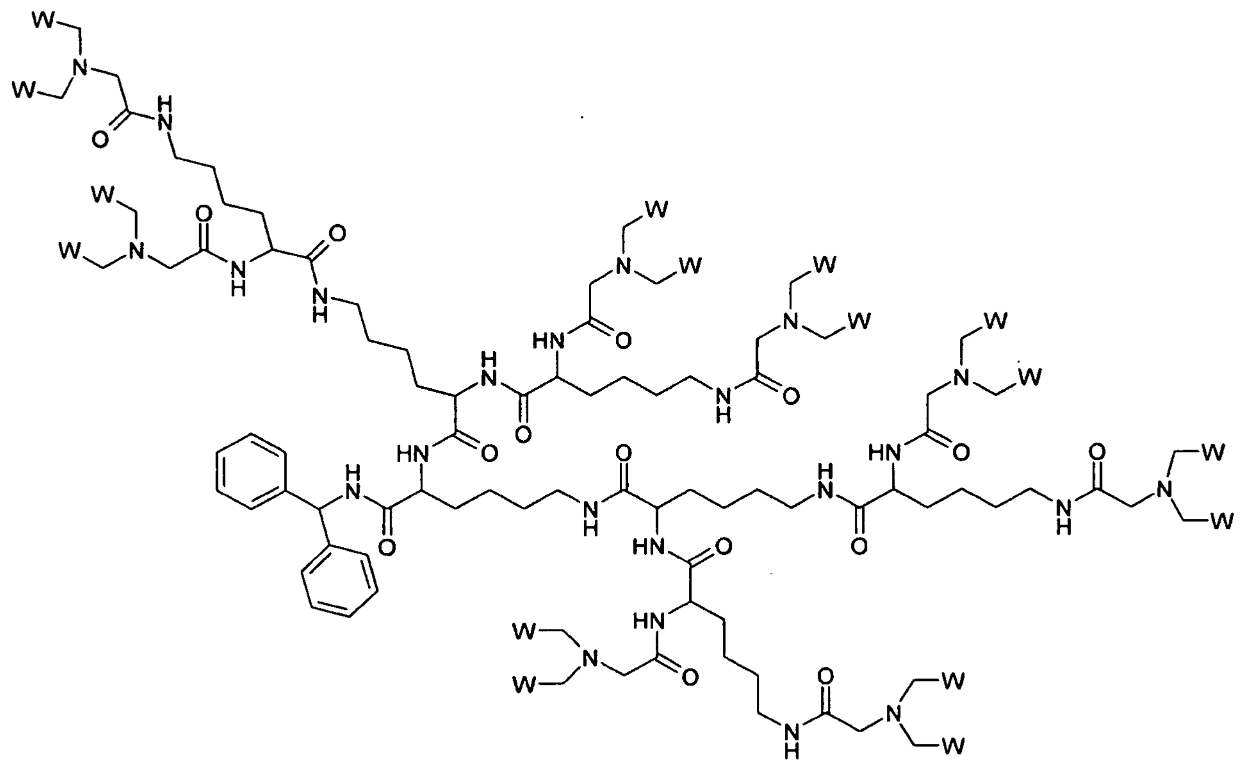

dans laquelle W représente $PO_3Me_2$, $PO_3HNa$, ou $PO_3H_2$.

La présente invention concerne également des cellules de la lignée lymphoïde, caractérisées en ce qu'elles expriment le récepteur NKG2D plus fortement que des cellules de la lignée lymphoïde exprimant le récepteur NKG2D et issues d'échantillons biologiques et/ou cultivées dans des conditions standards.

Selon un mode de réalisation particulier, l'invention concerne en particulier les cellules de la lignée lymphoïde définies ci-dessus, caractérisées en ce que le niveau d'expression du récepteur NKG2D est 2 à 5 fois supérieur à celui des cellules de la lignée lymphoïde exprimant le récepteur NKG2D et issues d'échantillons biologiques et/ou cultivées dans des conditions standards.

Selon un autre mode de réalisation particulier, l'invention concerne en particulier les cellules de la lignée lymphoïde définies ci-dessus, caractérisées en ce que le niveau d'expression du récepteur NKG2D correspond à une intensité de fluorescence moyenne (MPI) comprise d'environ 5 à environ 20 comme mesuré sur un appareil Coulter Epics XL à l'aide de l'anticorps Beckman-Coulter-Immunotech anti-NKG2D (clone ON72) marqué par la phycoérythrine.

Selon encore un autre mode de réalisation particulier de l'invention, les cellules de la lignée lymphoïde définies ci-dessus sont dérivées de cellules NK, de lymphocytes T $\alpha\beta$ $CD8^+$ ou de lymphocytes T $\gamma\delta$.

Selon un mode de réalisation préféré de l'invention, les cellules de la lignée lymphoïde définies ci-dessus sont dérivées de cellules NK.

Selon un autre mode de réalisation préféré de l'invention, les cellules de la lignée lymphoïde définies ci-dessus expriment le récepteur TLR2.

Selon un mode de réalisation particulièrement préféré de l'invention, les cellules de la lignée lymphoïde définies ci-dessus sont caractérisées en ce que le niveau d'expression du récepteur TLR2 correspond à une intensité de fluorescence moyenne (MFI) comprise d'environ 1 à environ 3 comme mesuré sur un appareil Coulter Epics XL à l'aide de l'anticorps BioLegend anti-TLR2 (clone TL2.1) marqué par la phycoérythrine.

La présente invention concerne également une composition pharmaceutique, caractérisée en ce qu'elle comprend, à titre de substance active, des cellules de la lignée lymphoïde telles que définies ci-dessus, en association avec un véhicule pharmaceutiquement acceptable.

Selon un mode de réalisation particulier de l'invention la composition pharmaceutique définie ci-dessus, comprenant à titre de substance active des cellules de la lignée lymphoïde telles que définies ci-dessus, est caractérisée en ce qu'elle convient pour l'administration à un individu d'une dose unitaire d'environ $10^5$ à environ $5.10^9$ cellules de la lignée lymphoïde telles que définies ci-dessus.

La présente invention concerne également l'utilisation de cellules de la lignée lymphoïde telles que définies ci-dessus, pour la préparation de médicaments destinés au traitement et/ou à la prévention de cancers, dont les tumeurs de tissus hématopoïétiques, telles que les leucémies myéloïdes ou les lymphomes anaplasiques, et les mélanomes.

D'une manière générale les dendrimères mono- et bisphosphoniques de l'invention peuvent être préparés comme cela est indiqué respectivement dans les demandes internationales WO 2005/0052032 et WO 2005/0052031.

En particulier, les dendrimères monophosphoniques utilisés dans l'invention peuvent être préparés comme indiqué ci-après. On donne ci-après leur définition afin de faciliter la description des procédés de préparation. Les dendrimères monophosphoniques peuvent être constitués:

- d'un noyau central § de valence m ;
- éventuellement des chaînes de générations en arborescence autour du noyau ;
- une chaîne intermédiaire à l'extrémité de chaque liaison autour du noyau ou éventuellement à l'extrémité de chaque chaîne de génération, le cas échéant ; et
- un groupe terminal en arborescence à l'extrémité de chaque chaîne intermédiaire, de formule :

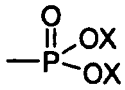

(T)

où chaque X, identique ou différent pour un groupe terminal donné, représente un radical - Me, -H, ou /$M^+$ où $M^+$ est un cation,
n représente la génération du dendrimère considéré ; il représente un entier compris entre 0 et 12.
m représente un entier supérieur ou égal à 1.

Le noyau central § est constitué d'au moins un atome de valence m.

De préférence, le noyau central § présente au moins un atome de phosphore. De préférence le noyau § est choisi parmi les groupes suivants : $SPCl_3$, $P_3N_3Cl_6$, $P_4N_4Cl_8$,

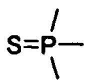 , 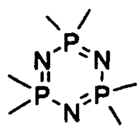 , 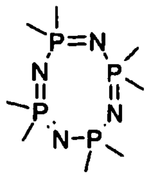 , 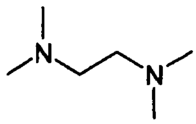 , 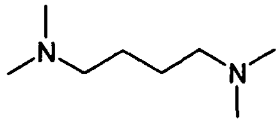

De préférence, le noyau central § est de formule :

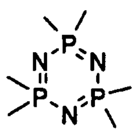

De préférence, n est compris entre 0 et 3.

De préférence, m est choisi parmi 3, 4 et 6.

De préférence, les dendrimères monophosphoniques correspondent aux dendrimères commerciaux auxquels a été greffé le groupe terminal $-P(=O)(OX)_2$.

Lesdits dendrimères commerciaux sont notamment choisis parmi les dendrimères de type DAB-AM, PAMAM (Starbust® notamment) présentant des fonctions terminales $-NH_2$, - OH ou -COOH, ou encore parmi les dendrimères de type PMMH, tels que Cyclophosphazène- ou Thiophosphoryl-PMMH, en particulier :

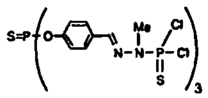

$G_1$

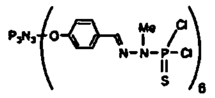

$Gc_1$

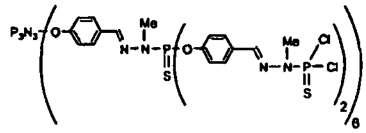

$Gc_2$

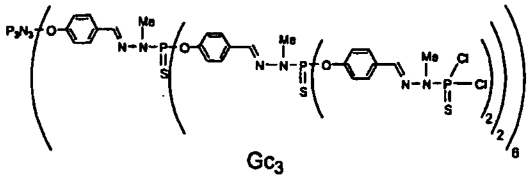

$Gc_3$

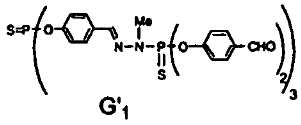

$G'_1$

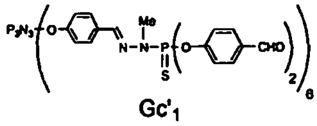

$Gc'_1$

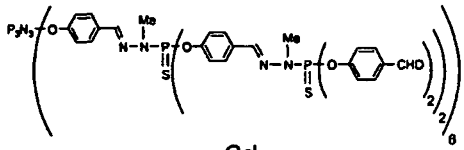

$Gc'_2$

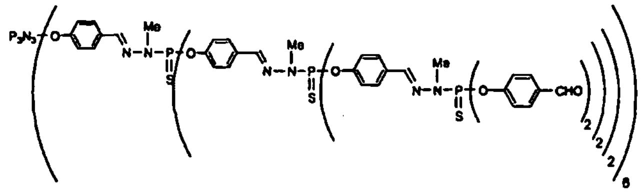

$Gc'_3$

ainsi que les dendrimères de génération ultérieure.

Tous ces dendrimères sont commercialisés par Aldrich.

De préférence, M représente un élément du groupe IA, IB, IIA, IIB ou IIIA, IIIB de la classification périodique ; de préférence, M est choisi parmi les atomes de sodium, potassium, cuivre, calcium, baryum, zinc, magnésium, lithium et aluminium, encore plus préférentiellement le sodium, le lithium et le potassium.

$M^+$ est un cation d'un atome, par exemple un atome de métal, ou un cation dérivé de tout radical susceptible d'être stable sous forme de cation. Ledit cation peut être notamment choisi parmi les sels d'ammonium, seuls ou en mélange, notamment avec les tensio-actifs cationiques.

De préférence, $M^+$ représente le cation d'une base azotée, tel que $HNEt_3^+$.

Les chaînes de génération sont choisies parmi toute chaîne hydrocarbonée de 1 à 12 chaînons, linéaire ou ramifiée, contenant éventuellement une ou plusieurs double ou triple liaisons, chacun desdits chaînons pouvant éventuellement être choisi parmi un hétéroatome, un groupe Aryle, Hétéroaryle, >C=O, >C=NR, chaque chaînon pouvant être éventuellement substitué par un ou plusieurs substituants choisi(s) parmi -Alkyle, -Hal, $-NO_2$, -NRR', -CN, - $CF_3$, -OH, -OAlkyle, -Aryle, -Aralkyle.

où

R et R', identiques ou différents, représentent indépendamment un atome d'hydrogène ou un radical -Alkyle, -Aryle, -Aralkyle ;

De préférence, les chaînes de génération, identiques ou différentes, sont représentées par la formule :

-A-B-C(D)=N-N(E)-(P(=G))<      (C1)

où:

A représente un atome d'oxygène, soufre, phosphore ou un radical -NR- ;

B représente un radical -Aryle-, -Hétéroaryle-, -Alkyle-, chacun pouvant être éventuellement substitué par un atome d'Halogène ou un radical $NO_2$, -NRR', -CN, $-CF_3$, -OH, -Alkyle, -Aryle, -Aralkyle

C représente l'atome de carbone,

D et E, identiques ou différents, représentent indépendamment un atome d'hydrogène, un radical -Alkyle, -OAlkyle, -Aryle, -Aralkyle, chacun pouvant être éventuellement substitué par un atome d'Halogène ou un radical $-NO_2$, -NRR', -CN, $-CF_3$, -OH, -Alkyle, - Aryle, -Aralkyle ;

G représente un atome de soufre, oxygène, sélénium, tellure ou un radical =NR ;

N représente l'atome d'azote ;

P représente l'atome de phosphore ;
< représente les 2 liaisons situées à l'extrémité de chaque chaîne de génération.

De préférence, dans la formule générale (C1) ci-dessus, A représente un atome d'oxygène.
De préférence, dans la formule générale (C1) ci-dessus, B représente un noyau phényle, éventuellement substitué par un atome d'halogène ou un radical $-NO_2$, -NRR', -CN, $-CF_3$, -OH, -Alkyle, -Aryle, -Aralkyle ; encore plus préférentiellement, B représente un noyau phényle non substitué.
De préférence, dans la formule générale (C1) citée ci-dessus, D représente un atome d'hydrogène.
De préférence, dans la formule générale (C1) citée ci-dessus, E représente un radical - Alkyle.
De préférence, dans la formule générale (C1) ci-dessus, G représente un atome de soufre.
Selon un autre aspect préféré, les chaînes de génération sont représentées par la formule :

-A'-(C=O)-N(R)-B'-N< (C1')

où

A' et B' représentent indépendamment un radical -Alkyle, -Alkényle, -Alkynyle, chacun pouvant être éventuellement substitué par un ou plusieurs substituants choisi(s) parmi -Alkyle, -Hal, $-NO_2$, -NRR', -CN, $-CF_3$, -OH, -OAlkyle, -Aryle, -Aralkyle ;
R, R' sont définis comme précédemment.

De préférence, A' représente -Alkyle-, encore plus préférentiellement -Ethyle-.
De préférence, B' représente -Alkyle-, encore plus préférentiellement -Ethyle-.
De préférence, R représente un atome d'hydrogène.
Selon un autre aspect préféré, les chaînes de génération sont représentées par la formule :

-A"-N< (C1")

où

A" représente un radical -Alkyle-, -Alkényle-, -Alkynyle-, chacun pouvant être éventuellement substitué par un ou plusieurs substituant choisi(s) parmi -Alkyle, -Hal, $-NO_2$, -NRR', -CN, $-CF_3$, -OH, -OAlkyle, -Aryle, -Aralkyle, où RR' sont définis comme précédemment.

De préférence, A" représente -Alkyle-, encore plus préférentiellement -Propyle-.
Selon un autre aspect préféré, les dendrimères selon l'invention de génération 0 ne comprennent pas de chaîne de génération. Notamment, dans le cas où la chaîne de génération est représentée par les formules (C1') ou (C1"), les dendrimères correspondants de génération 0 ne comprennent pas de chaîne de génération.
Les chaînes intermédiaires sont choisies parmi toute chaîne hydrocarbonée de 1 à 12 chaînons, linéaire ou ramifiée, contenant éventuellement une ou plusieurs double ou triple liaisons, chacun desdits chaînons pouvant éventuellement être choisi parmi un hétéroatome, un groupe Aryle, Hétéroaryle, >C=O, >C=NR, chaque chaînon pouvant être éventuellement substitué par un ou plusieurs substituants choisi(s) parmi -Alkyle, -Hal, $-NO_2$, -NRR', -CN, - $CF_3$, -OH, -OAlkyle, -Aryle, -Aralkyle,
où R, R' sont définis comme précédemment.
De préférence, les chaînes intermédiaires présentent une simple liaison à leur extrémité.
De préférence, les chaînes intermédiaires, identiques ou différentes, sont représentées par la formule :

-J-K-L- (C2)

où

J représente un atome d'oxygène, soufre, ou un radical -N-R- ;
K représente un radical -Aryle-, -Hétéroaryle-, -Alkyle-, chacun pouvant être éventuellement substitué par un atome d'Halogène ou un radical $-NO_2$, -NRR', -CN, $-CF_3$, -OH, -Alkyle, -Aryle, -Aralkyle ;
L représente une chaîne hydrocarbonée de 1 à 6 chaînons, linéaire ou ramifiée, contenant éventuellement un ou plusieurs hétéroatomes, et/ou contenant éventuellement une ou plusieurs double ou triple liaisons, chacun desdits maillons pouvant éventuellement être substitué par un ou plusieurs substituants choisi(s) parmi -OH, -NRR', -OAlkyle, -Alkyle, - Hal, $-NO_2$,-CN, $-CF_3$, -Aryle, -Aralkyle.

R et R', identiques ou différents, représentent indépendamment un atome d'hydrogène ou un radical -Alkyle, -Aryle, -Aralkyle.

De préférence, dans la formule (C2) ci-dessus, J représente un atome d'oxygène.

De préférence, dans la formule (C2) ci-dessus, K représente un noyau phényle, éventuellement substitué ; encore plus préférentiellement, K représente un noyau phényle non substitué.

De préférence, dans la formule (C2) ci-dessus, L représente un radical -Alkyle-, -Alkényle- ou -Allcynyle-, chacun pouvant être éventuellement substitué par un ou plusieurs substituants choisis parmi -OH, -NRR', -OAlkyle ; encore plus préférentiellement, L représente un radical -Alkyle-, éventuellement substitué par un radical -OH, ou un radical - Alkényle- ; encore plus préférentiellement, L représente un radical -Alkyle- éventuellement substitué par un radical -OH.

Selon un autre aspect préféré, les chaînes intermédiaires peuvent être représentées par la formule (C2') :

-L"- (C2')

où L" représente une chaîne -Alkyle- de 1 à 6 chaînons, éventuellement substituée par un ou plusieurs substituants choisis parmi -OH, -NRR', -OAlkyle ; encore plus préférentiellement, L représente un radical -Alkyle-, de préférence -Méthyle-.

De préférence, les chaînes de génération sont identiques.

De préférence, dans les formules (C1) et (C2) citées ci-dessus, J et K sont respectivement égaux à A, B.

De préférence, les dendrimères peuvent être représentés par la formule (I) suivante :

$$§\text{-}\{\{A\text{-}B\text{-}C(D){=}N\text{-}N(E)\text{-}(P({=}G))<\}^n[J\text{-}K\text{-}L\text{-}PO_3X_2]_2\}_m \qquad \text{(I-1)}$$

dans laquelle :

§, A, B, C, D, E, G, N, P, J, K, L, X, m, n, < sont définis comme précédemment.

Selon un autre aspect préféré, les dendrimères peuvent être représentés par la formule (I-2) suivante :

$$§\text{-}\{\{A'\text{-}(C{=}O)\text{-}N(R)\text{-}B'\text{-}NH\text{-}\}^n[L''\text{-}PO_3X_2]\}_m \qquad \text{(I-2)}$$

dans laquelle :

§, A', B', C, N, P, X, L", m, n sont définis comme précédemment.

Selon un autre aspect préféré, les dendrimères peuvent être représentés par la formule (I-3) suivante :

$$§\text{-}\{\{A''\text{-}NH\text{-}\}^n[L''\text{-}PO_3X_2]\}_m \qquad \text{(I-3)}$$

dans laquelle :

§, A", N, P, X, L", m, n sont définis comme précédemment.

Dans la formule ci-dessus, $\{\}^n$ désigne la structure en arborescence de génération n dudit radical.

Le radical -Alk, -Alkyle ou -Alkyle- représente un radical alkyle, c'est-à-dire un radical hydrocarboné et saturé, en chaîne droite ou ramifiée, de 1 à 20 atomes de carbone, de préférence de 1 à 5 atomes de carbone.

On peut notamment citer, lorsqu'ils sont linéaires, les radicaux méthyle, éthyle, propyle, butyle, pentyle, hexyle, octyle, nonyle, décyle, dodécyle, hexadécyle, et octadécyle.

On peut notamment citer, lorsqu'ils sont ramifiés ou substitués par un ou plusieurs radical alkyle, les radicaux isopropyle, tert-butyle, 2-éthylhexyle, 2-méthylbutyle, 2-méthylpentyle, 1-méthylpentyle et 3-méthylheptyle.

-Alkényle ou -Alkényle- désigne un groupe hydrocarboné aliphatique qui contient au moins une double liaison carbone-carbone et qui peut être linéaire ou ramifié ayant environ 2 à environ 15 atomes de carbone dans la chaîne. Des groupes alcényle préférés ont 2 à environ 12 atomes de carbone dans la chaîne ; et plus encore de préférence environ 2 à environ 4 atomes de carbone dans la chaîne. « Ramifié » signifie qu'un ou plusieurs groupes alkyle inférieurs, tels que le méthyle, l'éthyle ou le propyle, sont liés à une chaîne alkényle linéaire. Des exemples types de groupes alcényle comprennent l'éthényle, le propényle, le *n*-butényle, l'*i*-butényle, le 3-méthylbut-2-ényle, le *n*-pentényle, l'heptényle, l'octényle, le cyclohexylbutényle et le décényle.

Alkynyle ou -Alkynyle- désigne un groupe hydrocarboné aliphatique qui contient au moins une triple liaison carbone-carbone et qui peut être linéaire ou ramifié ayant 2 à environ 15 atomes de carbone dans la chaîne. Des groupes alcynyle préférés ont 2 à environ 12 atomes de carbone dans la chaîne ; et plus encore de préférence environ 2 à environ 4 atomes de carbone dans la chaîne. « Ramifié » signifie qu'un ou plusieurs groupes alkyle inférieurs, tels que le méthyle, l'éthyle ou le propyle, sont liés à une chaîne alcynyle linéaire. Des exemples types de groupes alcynyle comprennent l'éthynyle, le propynyle, le *n*-butynyle, le 2-butynyle, le 3-méthylbutynyle, le *n*-pentynyle, l'heptynyle, l'octynyle et le décynyle.

Parmi les atomes d'Halogène, on cite plus particulièrement les atomes de fluor, de chlore, de brome et d'iode, de préférence le fluor.

Le radical -Aryle ou -Aryle- représente un radical Aryle, c'est-à-dire un système aromatique hydrocarboné, mono ou bicyclique de 6 à 10 atomes de carbone.

Parmi les radicaux Aryle, on peut notamment citer le radical phényle ou naphtyle, plus particulièrement substitué par au moins un atome d'halogène.

Parmi les radicaux -Aralkyle (-AryleAlkyle), on peut notamment citer le radical benzyle ou phénéthyle.

Le terme « Hétéroatome » désigne l'atome d'azote, d'oxygène, silicium, phosphore ou soufre.

-Hétéroaryle ou -Hétéroaryle- désigne un radical Hétéroaryle, c'est-à-dire un système aromatique comprenant un ou plusieurs hétéroatomes choisis parmi l'azote, l'oxygène ou le soufre, mono ou bicyclique, de 5 à 10 atomes de carbone. Parmi les radicaux Hétéroaryles, on pourra citer le pyrazinyle, le thiényle, l'oxazolyle, le furazanyle, le pyrrolyle, le 1,2,4-thiadiazolyle, le naphthyridinyle, le pyridazinyle, le quinoxalinyle, le phtalazinyle, l'imidazo[1,2-a]pyridine, l'imidazo[2,1-b]thiazolyle, le cinnolinyle, le triazinyle, le benzofurazanyle, l'azaindolyle, le benzimidazolyle, le benzothiényle, le thiénopyridyle, le thiénopyrimidinyle, le pyrrolopyridyle, l'imidazopyridyle, le benzoazaindole, le 1,2,4-triazinyle, le benzothiazolyle, le furanyle, l'imidazolyle, l'indolyle, le triazolyle, le tétrazolyle, l'indolizinyle, l'isoxazolyle, l'isoquinolinyle, l'isothiazolyle, l'oxadiazolyle, le pyrazinyle, le pyridazinyle, le pyrazolyle, le pyridyle, le pyrimidinyle, le purinyle, le quinazolinyle, le quinolinyle, l'isoquinolyle, le 1,3,4-thiadiazolyle, le thiazolyle, le triazinyle, l'isothiazolyle, le carbazolyle, ainsi que les groupes correspondants issus de leur fusion ou de la fusion avec le noyau phényle. Les groupes Hétéroaryle préférés comprennent le thiényle, le pyrrolyle, le quinoxalinyle, le furanyle, l'imidazolyle, l'indolyle, l'isoxazolyle, l'isothiazolyle, le pyrazinyle, le pyridazinyle, le pyrazolyle, le pyridyle, le pyrimidinyle, le quinazolinyle, le quinolinyle, le thiazolyle, le carbazolyle, le thiadiazolyle, et les groupes issus de la fusion avec un noyau phényle, et plus particulièrement le quinolynyle, le carbazolyle, le thiadiazolyle.

On entend par « dendrimère correspondant » le dendrimère de même génération possédant les mêmes noyaux, chaînes de génération, chaînes intermédiaires et des groupes terminaux distincts.

Les dendrimères monophosphoniques peuvent être préparés par application ou adaptation de toute méthode connue en soi et/ou à la portée de l'homme du métier permettant le greffage de fonctions $-PO_3X_2$, notamment celles décrites par Larock dans Comprehensive Organic Transformations, VCH Pub., 1989, ou par application ou adaptation des procédés décrits dans les exemples qui suivent.

Dans les réactions décrites ci-après, il peut être nécessaire de protéger les groupes fonctionnels réactifs, par exemples les groupes hydroxy, amino, imino, thio, carboxy, lorsqu'ils sont souhaités dans le produit final, pour éviter leur participation indésirable dans les réactions. Les groupes de protection traditionnels peuvent être utilisés conformément à la pratique standard, pour des exemples voir T.W. Green et P.G.M. Wuts dans Protective Groups in Organic Chemistry , John Wiley and Sons, 1991 ; J.F.W. McOmie in Protective Groups in Organic Chemistry, Plenum Press, 1973.

Le procédé de préparation d'un dendrimère selon l'invention comprenant le groupe terminal $-P(=O)(OX)_2$ comprend :

(i) la réaction du dendrimère correspondant présentant une fonction terminale capable de réagir avec un composé correspondant présentant une fonctionnalité $-PO_3Me_2$. La fonction terminale du dendrimère peut être par exemple: -CHO, -CH=NR, ou $-P(=G)Cl_2$ ;

(ii) suivie éventuellement, lorsque X représente H ou M, de l'étape consistant à transformer le dendrimère obtenu en (i) présentant une terminaison $-PO_3Me_2$ en le dendrimère correspondant présentant une terminaison $-P(=O)(OH)_2$,

(iii) suivie éventuellement, lorsque X représente M, de l'étape consistant à transformer le dendrimère obtenu en (ii) présentant une terminaison $P(=O)(OH)_2$ en le sel du dendrimère correspondant présentant une terminaison $P(=O)(OM)_2$ ou P(=O)(OH)(OM).

L'étape (i) comprend la réaction du dendrimère correspondant de même génération n présentant une fonction terminale -CHO, -CH=NR, ou $-P(=S)Cl_2$
avec
un composé de formule $Z-PO_3Me_2$, où Z représente respectivement :

- soit -H lorsque la fonction est -CHO ou -CH=NR,
- soit la chaîne intermédiaire précédemment définie lorsque ladite fonction représente
- $P(=S)Cl_2$ ;

Selon une première alternative, l'étape (i) comprend l'action de $HPO_3Me_2$ sur le dendrimère correspondant présentant une terminaison -CHO ou -CH=NR par application ou adaptation de la méthode décrite dans J. Org. Chem. 1997, 62, 4834.

Plus précisément, cette réaction est effectuée sous agitation, en solution dans un solvant polaire, aprotique, tel que le THF, le chloroforme, le dichlorométhane, l'acétonitrile, de préférence sans solvant, en présence d'une base organique ou inorganique, de préférence une base azotée, telle que la triéthylamine, à température comprise entre -80°C et 100°C, de préférence à température ambiante.

Le composé de formule $HPO_3Me_2$ est disponible commercialement (Aldrich) ou peut être préparé selon des méthodes connues en soi.

Selon une seconde alternative, l'étape (i) comprend l'action d'un composé de formule $Z\text{-}PO_3Me_2$, où Z représente la chaîne intermédiaire précédemment définie sur un dendrimère de départ présentant la fonction terminale -P(=S)$Cl_2$.

Cette réaction est effectuée sous agitation, en solution dans un solvant polaire, aprotique tel que le THF, le chloroforme, le dichlorométhane, l'acétonitrile, l'acétone, le DMF, de préférence le THF, en présence d'une base organique ou inorganique, de préférence de type carbonate, telle que le carbonate de césium, à température comprise entre -80°C et 100°C, de préférence à température ambiante.

(ii) suivie éventuellement, lorsque X représente H ou M, de l'étape consistant à transformer le dendrimère obtenu en (i) présentant une terminaison $-PO_3Me_2$ en le dendrimère correspondant présentant une terminaison $-PO_3H_2$,

- par action d'halogénure de triméthylsilane, de préférence le bromure de triméthylsilane ($Me_3SiBr$), dans un solvant organique aprotique, polaire, tel que le chloroforme, le dichlorométhane, l'acétonitrile, de préférence l'acétonitrile. De préférence, on opère par ajout lent d'halogénure de triméthylsilane, en maintenant le mélange réactionnel à une température comprise entre -80°C et 100°C, de préférence, à environ 0°C.
- suivie de l'action de MeOH anhydre, ajouté au mélange réactionnel ;

(iii) suivie éventuellement, lorsque X représente M, de l'étape consistant à transformer le dendrimère obtenu en (ii) présentant une terminaison $-PO_3H_2$ en le sel du dendrimère correspondant présentant une terminaison $-PO_3M_2$.

Plus précisément, lorsque le dendrimère est de formule (I-1)

$$\S\text{-}\{\{A\text{-}B\text{-}C(D)\text{=}N\text{-}N(E)\text{-}(P(\text{=}G))\text{<}\}^{n}[J\text{-}K\text{-}L\text{-}PO_3X_2]_2\}_m \qquad \text{(I-1)}$$

dans laquelle §, A, B, C, D, E, G, N, P, J, K, L, X, m, n, < sont définis comme précédemment,
l'étape (i) comprend la réaction sur le dendrimère correspondant n de formule

$$\S\text{-}\{\{A\text{-}B\text{-}C(D)\text{=}N\text{-}N(E)\text{-}(P(\text{=}G))\text{<}\}^{n}\text{-}Y_2\}_m \qquad \text{(II-1)}$$

où Y représente :

- soit -J-K-L', où L' représente un radical -CHO ou -CH=NR ;
- soit -Cl ;

  d'un composé de formule $Z\text{-}PO_3Me_2$, où Z représente respectivement :
- soit H- lorsque Y représente -J-K-L' ;
- soit H-J-K-L- lorsque Y représente Cl ;

(ii) suivie éventuellement, lorsque X représente H ou M, de l'étape consistant à transformer le dendrimère de formule (III-1) obtenu en (i) dans laquelle X représente un radical Méthyle en le dendrimère correspondant de formule (I-1) dans laquelle X représente un atome d'hydrogène, selon le schéma réactionnel suivant :

$$\S\text{-}\{\{A\text{-}B\text{-}C(D){=}N\text{-}N(E)\text{-}(P({=}G))<\}^{n}[J\text{-}K\text{-}L\text{-}PO_3Me_2]_2\}_m \qquad \text{(III-1)}$$

↓

$$\S\text{-}\{\{A\text{-}B\text{-}C(D){=}N\text{-}N(E)\text{-}(P({=}G))<\}^{n}[J\text{-}K\text{-}L\text{-}PO_3H_2]_2\}_m \qquad \text{(IV-1)}$$

dans laquelle §, A, B, C, D, E, G, N, P, J, K, L, n, m, < sont définis comme précédemment,
(iii) suivie éventuellement, lorsque X représente M, de l'étape consistant à transformer le dendrimère de formule (IV-1) obtenu en (ii) en le sel correspondant.

Le produit de formule (III-1) est obtenu selon l'étape (i) par l'une ou l'autre des méthodes suivantes :
Selon une première alternative de l'étape (i), le produit de formule (III-1) est obtenu selon la réaction suivante :

$$\S\text{-}\{\{A\text{-}B\text{-}C(D){=}N\text{-}N(E)\text{-}(P({=}G))<\}^{n}[J\text{-}K\text{-}L']_2\}_m \qquad \text{(V)}$$

↓ + $H\text{-}PO_3Me_2$ (VI)

$$\S\text{-}\{\{A\text{-}B\text{-}C(D){=}N\text{-}N(E)\text{-}(P({=}G))<\}^{n}[J\text{-}K\text{-}L\text{-}PO_3Me_2]_2\}_m \qquad \text{(III-1)}$$

où
§, A, B, C, D, E, G, N, P, J, K, L, L', m, n, < sont définis comme précédemment.
Cette réaction peut être effectuée par application ou adaptation de la méthode décrite dans J. Org. Chem. 1997, 62, 4834.
Plus précisément, cette réaction est effectuée sous agitation, éventuellement en solution dans un solvant polaire, aprotique, tel que le THF, le dichlorométhane, le chloroforme ou l'acétonitrile, de préférence sans solvant, en présence d'une base organique ou inorganique, de préférence azotée, telle que la triéthylamine, à température comprise entre -80°C et 100°C, de préférence à température ambiante.
Le composé de formule (VI) est disponible commercialement (Aldrich) ou peut être préparé selon des méthodes connues en soi.
Les dendrimères de formule (V) sont disponibles commercialement (Aldrich) ou peuvent être préparés selon des méthodes connues en soi.
Selon une seconde alternative, le composé de formule (III-1) est obtenu selon la réaction suivante :

$$\S\text{-}\{\{A\text{-}B\text{-}C(D){=}N\text{-}N(E)\text{-}(P({=}G))<\}^{n}(Cl_2)\}_m \qquad \text{(VII)}$$

↓ + $H\text{-}J\text{-}K\text{-}L\text{-}PO_3Me_2$ (VIII)

$$\S\text{-}\{\{A\text{-}B\text{-}C(D){=}N\text{-}N(E)\text{-}(P({=}G)\ )<\}^{n}\text{-}[J\text{-}K\text{-}L\text{-}PO_3Me_2]_2\}_m \qquad \text{(III-1)}$$

où
§, A, B, C, D, E, G, N, P, J, K, L, m, n sont définis comme précédemment.
Cette réaction est effectuée sous agitation, en solution dans un solvant polaire, aprotique, tel que le THF, le chloroforme, le dichlorométhane, l'acétonitrile, l'acétone, le DMF, de préférence le THF, en présence d'une base organique ou inorganique, de préférence de type carbonate, telle que le carbonate de césium, à température comprise entre -80°C et 100°C, de préférence à température ambiante.
Les dendrimères de formule (VII) sont disponibles commercialement (Aldrich) ou peuvent être préparés selon des méthodes connues en soi.
Les dendrimères de formule (V) et (VII) peuvent notamment être choisis parmi :

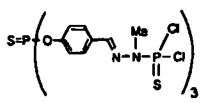
G1

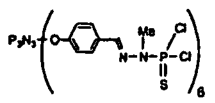
Gc1

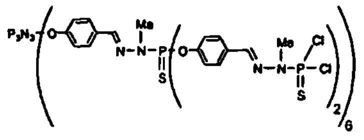
Gc2

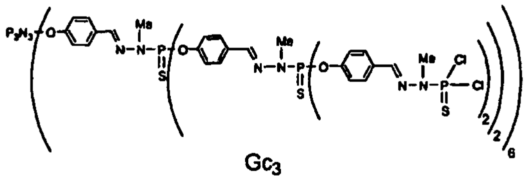
Gc3

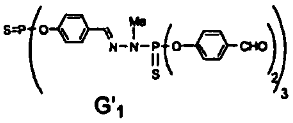
G'1

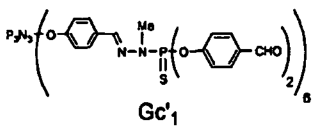
Gc'1

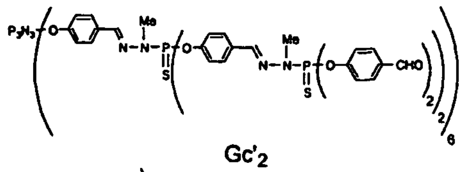
Gc'2

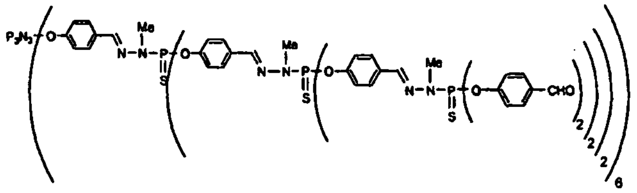
Gc'3

Plus précisément, lorsque les dendrimères selon l'invention sont représentés par la formule (1-2) suivante :

§'{{A'-(C=O)-N(R)-B'-NH-}$^n$[L"-$PO_3X_2$]}$_m$ (I-2)

dans laquelle §, A', B', C, N, P, X, L", m, n sont définis comme précédemment.

Ou la formule (I-3) suivante :

§-{{A"-NH-}$^n$[L"-$PO_3X_2$]$_2$}$_m$ (I-3)

dans laquelle §, A", N, P, X, L", m, n sont définis comme précédemment, le procédé comprend :

l'étape (i) comprenant la réaction sur le dendrimère correspondant n de formule

§-{{A'-(C=O)-N(R)-B'-N=R}$^n$}$_m$ (II-2)

ou §-{{A"-N=R}$^n$}$^m$ (I-3)

où R est un radical >Alkyle,

avec un composé de formule H-$PO_3Me_2$ (VI).

Cette réaction peut être effectuée par application ou adaptation de la méthode décrite dans J. Org. Chem. 1997, 62, 4834.

Plus précisément, cette réaction est effectuée sous agitation, éventuellement en solution dans un solvant polaire, aprotique, tel que le THF, le chloroforme, le dichlorométhane, l'acétonitrile, de préférence sans solvant, en présence d'une base organique ou inorganique, de préférence azotée, telle que la triéthylamine, à température comprise entre -80°C et 100°C, de préférence à température ambiante.

Le composé de formule (VI) est disponible commercialement (Aldrich) ou peut être préparé selon des méthodes connues en soi.

Les dendrimères de formule

§-{{A'-(C=O)-N(R)-B'-N=R}$^n$}$_m$ (II-2)

ou §-{{A"-N-R}$^n$}$_m$ (II-3)

peuvent être obtenus à partir des dendrimères correspondants de formule

§-{{A'-(C=O)-N(R)-B'-$NH_2$}$^n$}$_m$ (XVI)

ou §-{{A"-$NH_2$}$^n$}$_m$ (XVII)

commerciaux, par application ou adaptation de toute réaction connue en soi, permettant de transformer le groupe terminal -$NH_2$ en la fonction terminale -N=R requise. De telles méthodes, à la portée de l'homme de l'art ont notamment été décrites par Larock et al (supra).

Les dendrimères de formule (XVI) et (XVII) sont disponibles commercialement et peuvent notamment être choisis parmi les dendrimères de type DAB ou PAMAM.

(ii) suivie éventuellement, lorsque X représente H ou M, de l'étape consistant à transformer le dendrimère de formule (III-2) ou (III-3) obtenu en (i) dans laquelle X représente un radical Méthyle en le dendrimère correspondant de formule (I) dans laquelle X représente un atome d'hydrogène, selon le schéma réactionnel suivant :

§-{{A'-(C=O)-N(R)-B'-NH-}$^n$[L''-$PO_3Me_2$]}$_m$ (III-2)

ou §-{{A''-NH-}$^n$[L''-$PO_3Me_2$]}$_m$ (III-3)

↓

§-{{A'-(C=O)-N(R)-B'-NH-}$^n$[L''-$PO_3H_2$]}$_m$ (IV-2)

ou §-{{A''-NH-}$^n$[L''-$PO_3H_2$]}$_m$ (IV-3)

(iii) suivie éventuellement, lorsque X représente M, de l'étape consistant à transformer le dendrimère de formule (IV) obtenu en (ii) en le sel correspondant.

Dans tous les cas, la réaction (ii) est effectuée :

- par action d'halogénure de triméthylsilane, de préférence le bromure de triméthylsilane ($Me_3SiBr$), dans un solvant organique aprotique, polaire, tel que l'acétonitrile, le chloroforme ou le dichlorométhane, de préférence l'acétonitrile. De préférence on opère par ajout lent d'halogénure de triméthylsilane, en maintenant le mélange réactionnel à une température comprise entre -80°C et 100°C, de préférence, à environ 0°C.
- suivie de l'action de MeOH anhydre, ajouté au mélange réactionnel.

Dans l'étape (iii), les sels d'acides des dendrimères peuvent être obtenus à partir des dendrimères présentant une chaîne terminale dans laquelle Z représente un atome d'hydrogène, par l'application ou l'adaptation de procédés connus, par addition d'une base. De préférence, on opère en solution, sous agitation, dans un solvant convenable protique ou aprotique, polaire, tel que les alcools, l'eau, le THF, le dichlorométhane, le chloroforme, l'acétonitrile, le DMF, de préférence l'eau, en présence d'une base organique ou inorganique, telle que les hydroxydes, les carbonates, les bases azotées, de préférence l'hydroxyde de sodium, de lithium ou de potassium, selon le sel désiré.

Lorsqu'on utilise des dendrimères de départ présentant des groupes terminaux différents des fonctions terminales décrites plus haut pour les dendrimères de formule (II-1), (II-2) ou (II-3), le procédé comprend l'étape préliminaire supplémentaire permettant de transformer lesdits groupes en lesdites fonctions requises. Par exemple, dans le cas de dendrimères présentant des groupes terminaux de type acide carboxylique ou hydroxyle, il suffit d'effectuer toute réaction permettant de convertir lesdits groupes de type acide carboxylique ou hydroxyle en les fonctions de type -$NH_2$, -CHO, -C=NR ou -$PSCl_2$ correspondantes aux dendrimères de formule (II-1), (II-2) ou (II-3). De telles réactions sont connues de l'homme du métier et/ou peuvent être effectuées par application ou adaptation de celles discutées par Larock et al (*supra*).

Pour obtenir un dendrimère de génération 0, les réactions ci-dessus peuvent être effectuées de la même façon en opérant à partir du noyau, présentant la fonctionnalité requise. Par exemple, les réactions de génération peuvent être effectuées en opérant à partir d'un noyau $PSCl_3$, $P_3N_3Cl_6$, $P_4N_4Cl_8$, ou

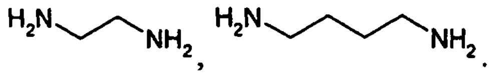

S'agissant des composés de formule (VIII) :

$$Z\text{-}J\text{-}K\text{-}L\text{-}PO_3Me_2 \qquad (VIII)$$

dans laquelle

Z représente H ou un groupe protecteur de la fonction -JH ; ces groupes protecteurs sont connus en soi et peuvent notamment être identifiés dans Green et al ou McOmie et al. cités ci-avant. De préférence, lorsque J représente un atome d'oxygène, Z représente le groupe TBDMS (radical tertio-butyl-diméthyl-silyle).
J représente un atome d'oxygène, soufre, ou un radical -N-R- ;
K représente un radical -Aryle-, -Hétéroaryle-, -Alkyle-, chacun pouvant être éventuellement substitué par un atome d'Halogène ou un radical $-NO_2$, -NRR', -CN, $-CF_3$, -OH, -Alkyle, -Aryle, -Aralkyle ;
L représente une chaîne hydrocarbonée de 1 à 6 chaînons, linéaire ou ramifiée, chacun desdits chaînons pouvant éventuellement être choisis parmi un hétéroatome, de préférence l'azote, et/ou contenant éventuellement une ou plusieurs double ou triple liaisons, chacun desdits maillons pouvant éventuellement être substitué par un ou plusieurs substituants choisi(s) parmi -OH, -NRR', -OAlkyle, -Alkyle, -Hal, $-NO_2$, -CN, $-CF_3$, -Aryle, -Aralkyle.
R, R', identiques ou différents, représentent indépendamment l'un de l'autre un atome d'hydrogène ou un radical -Alkyle, -Aryle, -Aralkyle,
de préférence, dans la formule (VIII) ci-dessus, J représente un atome d'oxygène,
de préférence, dans la formule (VIII) ci-dessus, K représente un noyau phényle, éventuellement substitué ; encore plus préférentiellement, K représente un noyau phényle non substitué,
de préférence, dans la formule (VIII) ci-dessus, L représente un radical -Alkyle-, éventuellement substitué par un radical -OH, ou un radical -Alkényle- ; encore plus préférentiellement, L représente un radical -Alkyle-,
ils peuvent être obtenus de la façon suivante :

$$Z\text{-}J\text{-}K\text{-}L\text{-}Hal\ (IX) \rightarrow Z\text{-}J\text{-}K\text{-}L\text{-}\ PO_3Me_2 \qquad (VIII)$$

où Z, J, K, L sont définis comme précédemment, Hal représente un atome d'halogène, de préférence le brome.

Dans le cas où, dans la formule (VIII), Z=H, le produit de formule (VIII) est obtenu à partir du produit de formule (VIII) où Z est un groupe protecteur, par application ou adaptation de toute méthode connue de déprotection du groupe protecteur Z, notamment celles décrites dans Green et al. ou McOmie et al. (supra). Notamment, dans le cas où J=O et Z=TBDMS, on opère par action de fluorure de tétrabutylammonium, de préférence 2 équivalents, sous agitation, en solution dans un solvant polaire, aprotique, tel que le THF, le chloroforme, le dichlorométhane, l'acétonitrile, le DMF, de préférence le THF, à une température comprise entre -80°C et 100°C, de préférence à température ambiante.

Le produit de formule (VIII) où Z est un groupe protecteur est obtenu à partir du produit de formule (IX) par application ou adaptation de la réaction d'Arbuzow décrite notamment dans B.A. Arbuzow, Pure appl. Chem. 1964, 9, 307, ou toute réaction équivalente. Notamment, on met à réagir le produit de formule (IX) en présence de triméthylphosphite de formule

$$P(OMe)_3 \qquad (X)$$

sous agitation, en solution dans un solvant polaire, aprotique, tel que le THF, le chloroforme, le dichlorométhane, l'acétonitrile, de préférence sans solvant, à une température comprise entre -80°C et 150°C, de préférence à environ 80°C.

Le produit de formule (IX) peut être obtenu par application ou adaptation de la méthode décrite par Olszewski et al dans J. Org. Chem. 1994, 59, 4285-4296.

Notamment, on peut opérer de la façon suivante :

$$H\text{-}J\text{-}K\text{-}L''\text{-}CHO\ (XI) \rightarrow Z\text{-}J\text{-}K\text{-}L''\text{-}CHO\ (XII) \rightarrow Z\text{-}J\text{-}K\text{-}L\text{-}OH\ (XII) \rightarrow Z\text{-}J\text{-}K\text{-}L\text{-}COCF_3\ (XIV) \rightarrow Z\text{-}J\text{-}K\text{-}L\text{-}Hal \qquad (IX)$$

où Z, J, K, L, Hal sont tels que définis plus haut et L" représente un radical correspondant à L où un atome d'hydrogène et de carbone ont été formellement éliminés.

Le produit de formule (IX) est obtenu à partir du produit de formule (XIV) par application ou adaptation de toute

réaction connue de substitution du groupe trifluoroacétate par un atome d'halogène, le brome notamment, par exemple par action de LiBr sous agitation, en solution dans un solvant polaire, aprotique, tel que le THF, le chloroforme, le dichlorométhane, l'acétonitrile, le DMF, de préférence le THF, à reflux, pendant une durée nécessaire à l'obtention d'un rendement acceptable de la réaction, par exemple entre 5 et 20 heures.

Le produit de formule (XIV) est obtenu à partir du produit de formule (XIII) par application ou adaptation de toute réaction connue de substitution de la fonction hydroxy par un radical trifluoroacétate, notamment par action de l'anhydride trifluoroacétique $(CF_3CO)_2O$ sous agitation, en solution dans un solvant polaire, aprotique, tel que le THF, le chloroforme, le dichlorométhane, l'acétonitrile, le DMF, de préférence le THF, à reflux, pendant une durée nécessaire à l'obtention d'un rendement acceptable de la réaction, par exemple entre 5 minutes et 5 heures.

Le produit de formule (XIII) est obtenu à partir du produit de formule (XII) par application ou adaptation de toute réaction connue de réduction de la fonction aldéhyde en fonction hydroxy, notamment par action d'un agent réducteur tel que $NaBH_4$ ou tout agent équivalent, en solution dans un solvant polaire, protique ou aprotique, tel que l'éther, le THF, les alcools, l'eau, de préférence le mélange THF/EtOH (5/1), à reflux, pendant une durée nécessaire à l'obtention d'un rendement acceptable de la réaction, par exemple entre 1 heure et 10 jours.

Le produit de formule (XII) est obtenu à partir du produit de formule (XI) par application ou adaptation de toute réaction connue de protection de la fonction -JH par un groupe protecteur Z ou tout autre groupe protecteur convenable, par application ou adaptation des méthodes décrites par Green et al. ou Wuts et al. cités ci-avant. Dans les cas de la protection par TBDMS, on opère notamment par action de Cl-TBDMS (XV) sous agitation, en solution dans un solvant polaire, aprotique, tel que le THF, le chloroforme, le dichlorométhane, l'acétonitrile, le DMF, de préférence le dichlorométhane, en présence d'une base telle que la triéthylamine (2 équivalents), à température comprise entre -80°C et 100°C, de préférence à température ambiante.

Le produit de formule (XI) est commercial et peut notamment être obtenu auprès de Aldrich.

Dans la description du procédé ci-dessus, deux groupes sont dits « correspondants » lorsque ils sont respectivement inclus dans un produit de départ et d'arrivée, et leur structure est identique et peut se déduire l'une de l'autre.

Eventuellement, ledit procédé peut également comprendre l'étape consistant à isoler le produit obtenu ou le produit final intermédiairement formé à l'issue des étapes (i), (ii) ou (iii).

Le composé ainsi préparé peut être récupéré à partir du mélange de la réaction par les moyens traditionnels. Par exemple, les composés peuvent être récupérés en distillant le solvant du mélange de la réaction ou si nécessaire après distillation du solvant du mélange de la solution, en versant le reste dans de l'eau suivi par une extraction avec un solvant organique immiscible dans l'eau, et en distillant le solvant de l'extrait. En outre, le produit peut, si on le souhaite, être encore purifié par diverses techniques, telles que la recristallisation, la reprécipitation ou les diverses techniques de chromatographie, notamment la chromatographie sur colonne ou la chromatographie en couche mince préparative.

Les produits de base ou les intermédiaires peuvent être préparés par l'application ou l'adaptation de procédés connus, par exemple des procédés tels que décrits dans les Exemples de Référence ou leurs équivalents chimiques évidents.

Les dendrimères bisphosphoniques utilisés dans la présente invention peuvent être préparés comme indiqués ci-après. On donne ci-après leur définition pour faciliter la description des procédés de préparation.

Les dendrimères bisphosphoniques de génération n peuvent être caractérisés comme comprenant :

- un noyau central § de valence m ;
- éventuellement des chaînes de génération en arborescence autour du noyau ;
- une chaîne intermédiaire à l'extrémité de chaque chaîne de génération ou à l'extrémité de chaque liaison autour du noyau, le cas échéant ; et
- un groupe terminal à l'extrémité de chaque chaîne intermédiaire, le cas échéant, caractérisé en ce que ledit groupe terminal est représenté par la formule:

$$-(A1)<[A2-P(=O)(OX)_2]_2 \qquad (T')$$

où

-A1< représente le radical -CR< ou -Hétéroatome< ;

chacun des A2, identiques ou différents représentent indépendamment une liaison simple ou une chaîne hydrocarbonée de 1 à 6 chaînons, linéaire ou ramifiée, chacun desdits chaînons pouvant éventuellement être choisi parmi un hétéroatome, de préférence l'azote, chaque chaînon pouvant être éventuellement substitué par un ou plusieurs substituants choisi(s) parmi -Alkyle, -Hal, $-NO_2$, -NRR', -CN, $-CF_3$, -OH, -OAlkyle, -Aryle, -Aralkyle;

chaque X, identique ou différent pour chacun des groupements phosphoniques, représente un radical -Alkyle,

-Aryle, -H, ou /$M^+$ où $M^+$ est un cation,
m représente un entier supérieur ou égal à 1;
n représente un entier compris entre 0 et 12 ;
< représente deux liaisons situées sur A1.

De préférence, les dendrimères utilisés correspondent aux dendrimères commerciaux auxquels a été greffé le groupe terminal -(A1)<[A2-P(=O)(OX$_2$]$_2$ sur leur surface.
Lesdits dendrimères commerciaux sont notamment choisis parmi ceux mentionnés ci-dessus.
De préférence, A1 représente le radical -CH< ou -N<.
De préférence, les groupes -P(=O)(OX)$_2$ sont en position gem.
De préférence, X représente un radical -Alkyle, tel que -Méthyle.
De préférence, A2 représente -Me-.
Le noyau central § est constitué d'au moins un atome de valence m.
Le noyau central § peut être choisi parmi tout atome ou radical présentant une valence m supérieure ou égale à 1. De préférence, § contient au moins un hétéroatome.
$M^+$ est un cation d'un atome, par exemple un atome de métal, ou un cation dérivé de tout radical susceptible d'être stable sous forme de cation. Ledit cation peut être notamment choisi parmi les sels de base azotée, notamment les sels d'ammonium, seuls ou en mélange, notamment avec les tensio-actifs cationiques.
De préférence, $M^+$ représente un cation d'élément du groupe IA, IB, IIA, IIB ou IIIA, IIIB de la classification périodique ; de préférence, M est choisi parmi les atomes de sodium, potassium, cuivre, calcium, baryum, zinc, magnésium, lithium et aluminium, encore plus préférentiellement le sodium, le lithium et le potassium.
Selon un autre aspect préféré, $M^+$ représente le cation d'une base azotée, tel que $HNEt_3^+$. De préférence, le noyau § est choisi parmi les groupes suivants :

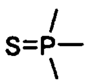 , 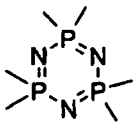 , 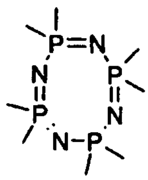 , 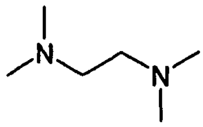 , 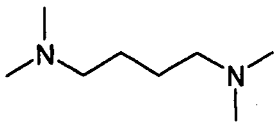

De préférence, le noyau central § est de formule :

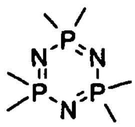

m représente un entier compris entre 1 et 20, en particulier de 1 à 10, plus particulièrement de 1 à 8, encore plus préférentiellement compris entre 3 et 8, et plus particulièrement 3, 4 ou 6 ;
n représente le nombre de générations du dendrimère ; il représente un entier compris entre 0 et 12 ; de préférence compris entre 0 et 3 ;

Les chaînes de génération sont choisies parmi celles définies ci-dessus à propos des définitions données pour les dendrimères monophosphoniques.
De préférence, les chaînes de génération, identiques ou différentes, sont représentées par la formule :

-A-B-C(D)=N-N(E)-(P(=G))< (C1)

où:

A représente un atome d'oxygène, soufre, phosphore ou un radical -N-R- ;
B représente un radical -Aryle-, -Hétéroaryle-, -Alkyle-, chacun pouvant être éventuellement substitué par un atome d'Halogène ou un radical -$NO_2$, -NRR', -CN, -$CF_3$, -OH, -Alkyle, -Aryle, -Aralkyle ;
C représente l'atome de carbone,
D et E, identiques ou différents, représentent indépendamment un atome d'hydrogène, un radical -Alkyle, -OAlkyle,

-Aryle, -Aralkyle, chacun pouvant être éventuellement substitué par un atome d'Halogène ou un radical $-NO_2$, -NRR', -CN, $-CF_3$, -OH, -Alkyle, -Aryle, - Aralkyle ;
G représente un atome de soufre, oxygène, azote, Sélénium, Tellure ou un radical =NR ;
N représente l'atome d'azote ;
P représente l'atome de phosphore.

De préférence, dans la formule générale (C1) ci-dessus, A représente un atome d'oxygène.
De préférence, dans la formule générale (C1) ci-dessus, B représente un noyau phényle, éventuellement substitué par un atome d'halogène ou un radical $-NO_2$, -NRR', -CN, $-CF_3$, - OH, -Alkyle, -Aryle, -Aralkyle ; encore plus préférentiellement, B représente un noyau phényle non substitué.
De préférence, dans la formule générale (C1) citée ci-dessus, D représente un atome d'hydrogène.
De préférence, dans la formule générale (C1) citée ci-dessus, E représente un radical - Alkyle.
De préférence, dans la formule générale (C1) ci-dessus, G représente un atome de soufre.
Selon un autre aspect préféré, les chaînes de génération sont représentées par la formule :

-A'-(C=O)-N(R)-B'-N< (C1')

où

A' et B' représentent indépendamment un radical -Alkyle-, -Alkényle-, -Alkynyle-, chacun éventuellement substitué par un ou plusieurs substituants choisi(s) parmi -Alkyle, - Hal, $-NO_2$, -NRR', -CN, $-CF_3$, -OH, -OAlkyle, -Aryle, -Aralkyle ;
R, R' sont définis comme précédemment.

De préférence, A' représente -Alkyle-, encore plus préférentiellement -Ethyle-. De préférence, B' représente -Alkyle-, encore plus préférentiellement -Ethyle-.
De préférence, R représente un atome d'hydrogène.
Selon un autre aspect préféré, les chaînes de génération sont représentées par la formule :

-A"-N< (C1")

où

A" représente un radical -Alkyle-, -Alkényle-, -Alkynyle-, chacun éventuellement substitué par un ou plusieurs substituants choisi(s) parmi -Alkyle, -Hal, $-NO_2$, -NRR', -CN, - $CF_3$, -OH, -OAlkyle, -Aryle, -Aralkyle, où RR' sont définis comme précédemment.

De préférence, A" représente -Alkyle-, encore plus préférentiellement -Propyle-.
Selon un autre aspect préféré, les dendrimères de génération 0 ne comprennent pas de chaîne de génération. Notamment, dans le cas où la chaîne de génération est représentée par les formules (C1') ou (C1"), les dendrimères correspondants de génération 0 ne comprennent pas de chaîne de génération.
Les chaînes intermédiaires sont choisies parmi celles définies à propos des dendrimères monophosphoniques.
De préférence, les chaînes intermédiaires présentent une simple liaison à leur extrémité.
De préférence, les chaînes intermédiaires, identiques ou différentes, sont représentées par la formule :

-J-K-L- (C2)

définie ci-dessus.
De préférence, dans la formule (C2) ci-dessus, J représente un atome d'oxygène.
De préférence, dans la formule (C2) ci-dessus, K représente un noyau phényle, éventuellement substitué ; encore plus préférentiellement, K représente un noyau phényle non substitué.
De préférence, dans la formule (C2) ci-dessus, L représente un radical $-(Alk)_a-$, ou L représente le radical -C(D)=N-N(E)-$(Alk)_a$-,
où C représente un atome de carbone,

D et E, identiques ou différents, représentent indépendamment un atome d'hydrogène, un radical -Alkyle, -OAlkyle, -Aryle, -Aralkyle, chacun pouvant être éventuellement substitué par un atome d'Halogène ou un radical $-NO_2$, -NRR', -CN, $-CF_3$, -OH, -Alkyle, -Aryle, -Aralkyle ;

a représente 0 ou 1 ;
R, R' sont définis comme précédemment.

Selon un autre aspect préféré, les chaînes intermédiaires sont représentées par la formule

-A'-(C=O)-N(R)-B'- (C2'$_1$)

où A', B', R, R' sont définis comme précédemment.
De préférence, A' représente -Alkyle- ; encore plus préférentiellement -Ethyle-.
De préférence, B' représente -Alkyle- ; encore plus préférentiellement -Ethyle-.
De préférence, R représente un atome d'hydrogène.
Selon un autre aspect préféré, les chaînes intermédiaires sont représentées par la formule

-A"- (C2")

où

A" est défini comme précédemment.

De préférence, A" représente un radical -Alkyle- ; encore plus préférentiellement - Propyle-.
De préférence, les chaînes de génération sont identiques.
De préférence, dans les formules (C1) et (C2) citées ci-dessus, J et K sont respectivement égaux à A, B.
De préférence, les dendrimères bisphosphoniques peuvent être représentés par la formule (I-1i) suivante :

§-{{A-B-C(D)=N-N(E)-(P(=G))<}$^n$[J-K-(Alk)$_a$-N<[A2-P(=O)(OX)$_2$]$_2$]$_2$}$_m$ (I-1i)

dans laquelle :

§, A, B, C, D, E, G, N, P, J, K, X, A2, m, n sont définis comme précédemment, {}$^n$ désigne la structure en arborescence des chaînes de génération n dudit dendrimère, et a représente 0 ou 1 ; de préférence, A2 représente un radical -Alkyle-.

De préférence, les dendrimères bisphosphoniques peuvent être représentés par la formule (I-1ii) suivante :

§-{{A-B-C(D)=N-N(E)-(P(=G))<}$^n$[J-K-C(D)=N-N(E)-(Alk)$_a$-CH<[A2-P(=O)(OX)$_2$]$_2$]$_2$}$_m$ (I-1ii)

dans laquelle :

§, A, B, C, D, E, G, N, P, J, K, X, A2, m, n sont définis comme précédemment, {}$^n$ désigne la structure en arborescence des chaînes de génération n dudit dendrimère, et a représente 0 ou 1 ; de préférence, A2 représente une liaison simple.

Selon un autre aspect préféré, les dendrimères bisphosphoniques peuvent être représentés par la formule (I-2) suivante :

§-{{A'-(C=O)-N(R)-B'-N<}$^n$ [A2-P(=O)(OX)$_2$]$_2$}$_m$ (1-2')

dans laquelle :

§, A', B', C, N, P, X, A2, m, n sont définis comme précédemment et {}$^n$ désigne la structure en arborescence des chaînes de génération n dudit dendrimère.

Selon un autre aspect préféré, les dendrimères bisphosphoniques peuvent être représentés par la formule (I-3) suivante :

§-{{A"-N<}$^n$ [A2-P(=O)(OX)$_2$]$_2$}$_m$ (I-3')

dans laquelle :

§, A", N, P, X, A2, m, n sont définis comme précédemment et $\{\}^n$ désigne la structure en arborescence des chaînes de génération n dudit dendrimère.

Les radicaux -Alk, -Alkyle ou -Alkyle-, -Alkényle ou -Alkényle-, -Alkynyle ou - Alkynyle- ont les significations mentionnées ci-dessus.

Parmi les atomes d'Halogène (Hal), on cite plus particulièrement les atomes de fluor, de chlore, de brome et d'iode, de préférence le fluor.

Les radicaux -Aryle ou -Aryle-, -Aralkyle (-AlkyleAryle), -Hétéroaryle ou - Hétéroaryle-, ont les significations mentionnées ci-dessus.

On entend par « dendrimère correspondant » le dendrimère de même génération possédant les mêmes noyaux, chaînes de génération, chaînes intermédiaires et des groupes terminaux distincts.

Les dendrimères bisphosphoniques peuvent être préparés par application ou adaptation de toute méthode connue en soi et/ou à la portée de l'homme du métier permettant le greffage de fonctions $-PO_3X_2$, particulièrement $-(Al)<[A2-P(=O)(OX)_2]_2$, notamment celles décrites par Larock dans Comprehensive Organic Transformations, VCH Pub., 1989, ou par application ou adaptation des procédés décrits dans les exemples qui suivent.

Dans les réactions décrites ci-après, il peut être nécessaire de protéger les groupes fonctionnels réactifs, par exemple les groupes hydroxy, amino, imino, thio, carboxy, lorsqu'ils sont souhaités dans le produit final, pour éviter leur participation indésirable dans les réactions. Les groupes de protection traditionnels peuvent être utilisés conformément à la pratique standard, pour des exemples voir T.W. Green et P.G.M. Wuts dans Protective Groups in Organic Chemistry, John Wiley and Sons, 1991 ; J.F.W. McOmie in Protective Groups in Organic Chemistry, Plenum Press, 1973.

Le procédé de préparation d'un dendrimère selon l'invention comprenant le groupe terminal $-A_1<[A2-P(=O)(OX)_2]_2$ comprend :

(i) la réaction du dendrimère correspondant présentant une fonction terminale -CHO, - CH=NR, $-NH_2$ ou $-P(=G)Cl_2$ avec un composé correspondant présentant une ou deux fonctionnalités $-PO_3X_2$ ;

(ii) suivie éventuellement, lorsque X représente H ou M, de l'étape consistant à transformer le dendrimère obtenu en (i) présentant une terminaison $-PO_3Me_2$ en le dendrimère correspondant présentant une terminaison $-A1<[A2-P(+O)(OH)_2]_2$,

(iii) suivie éventuellement, lorsque X représente M, de l'étape consistant à transformer le dendrimère obtenu en (ii) présentant une terminaison $-A1<[A2-P(=O)(OH)_2]_2$ en le sel du dendrimère correspondant présentant une terminaison $-Al<[A2-P(=O)(OM)_2]_2$.

Les dendrimères correspondants de départ sont disponibles commercialement (Aldrich) ou peuvent être préparés selon des méthodes connues en soi.

Plus précisément, l'étape (i) peut être opérée selon les alternatives suivantes :

Selon une première alternative, lorsque le dendrimère bisphosphonique est représenté par la formule (I-1i)

$$§-\{\{A-B-C(D)=N-N(E)-(P(=G))<\}^n[J-K-(Alk)_a-N<[A2-P(=O)(OX)_2]_2]_2\}_m \qquad \text{(I-1i)}$$

dans laquelle §, A, B, C, D, E, G, N, P, J, K, A2, Alk, X, a, m, n, < sont définis comme précédemment,
l'étape (i) comprend la réaction sur le dendrimère correspondant de même génération n de formule

$$§-\{A-B-C(D)=N-N(E)-(P(=G))<Y_2\}^n \qquad \text{(II-1i)}$$

où Y représente -Cl;
d'un composé de formule $H-J-K-(Alk)_a-N<[A2-P(=O)(OX)_2]_2$ (III)

Cette réaction est effectuée sous agitation, en solution dans un solvant polaire, aprotique, tel que le THF, l'acétonitrile, le chloroforme, le dichlorométhane, le DMF ou l'acétone, de préférence le THF, en présence d'une base organique ou inorganique, telle que le carbonate de césium, à température comprise entre -80°C et 100°C, de préférence à température ambiante.

De préférence, dans la formule (II-1i), G représente S.

De préférence, les dendrimères de formule (II-1i) sont choisis parmi : $SPCl_3$, $P_3N_3Cl_6$,

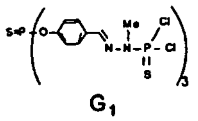

G1

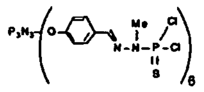

Gc1

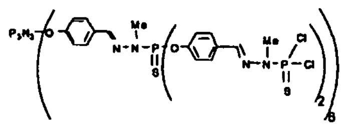

Gc2

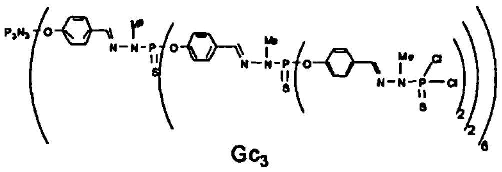

Gc3

Selon une seconde alternative, lorsque le dendrimère est représenté par la formule (I-2') ou (I-3) :

$§\text{-}\{\{A'\text{-}(C{=}O)\text{-}N(R)\text{-}B'\text{-}N{<}\}^{n}\ [A2\text{-}P({=}O)(OX)_2]_2\}_m$ (I-2')

ou

$§\text{-}\{\{A''\text{-}N{<}\}^{n}\ [A2\text{-}P({=}O)(OX)_2]_2\}_m$ (I-3')

dans lesquelles §, A', A", B', B", C, N, P, A2, X, m, n, < sont définis comme précédemment, l'étape (i) comprend la réaction sur le dendrimère correspondant de même génération n de formule

$§\text{-}(\{A'\text{-}(C{=}O)\text{-}N(R)\text{-}B'\text{-}NH_2\}^{n}\}_m$ (II-2')

ou

$§\text{-}\{\{\text{-}A''\text{-}NH_2\}^{n}\}_m$ (II-3)

d'un composé de formule $H\text{-}P({=}O)(OX)_2$ (IV), en présence d'un composé de formule H-A2-(C=O)H (V') correspondant.

Cette réaction est effectuée sous agitation, éventuellement dilué en solution aqueuse, à température comprise entre -5°C et la température de reflux du mélange.

Les composés de formule (IV) et (V') sont disponibles commercialement (Aldrich) ou peuvent être préparés selon des méthodes connues en soi.

Les dendrimères de formule (II-2') et (II-3) sont disponibles commercialement (Aldrich). Ils sont de préférence de type DAB ou PAMAM mentionnés plus haut.

Selon une troisième alternative, lorsque le dendrimère est représenté par la formule (I-1ii)

$§\text{-}\{\{A\text{-}B\text{-}C(D){=}N\text{-}N(E)\text{-}(P({=}G)){<}\}^{n}[J\text{-}K\text{-}C(D){=}N\text{-}N(E)\text{-}(Alk)_a\text{-}CH{<}[A2\text{-}P({=}O)(OX)_2]_2]_2\}_m$ (I-1ii)

dans laquelle :

§, A, B, C, D, E, G, N, P, J, K, L, X, A2, m, n, a sont définis comme précédemment, l'étape (i) comprend la réaction sur le dendrimère correspondant de formule

$§\text{-}\ \{\{A\text{-}B\text{-}C(D){=}N\text{-}N(E)\text{-}(P({=}G)){<}\}^{n}\text{-}[J\text{-}K\text{-}L']_2\}_m$ (II-1ii)

où L' représente un radical -CHO ;
d'un composé de formule $(Alk')_a\text{-}CH\text{-}[A2\text{-}P({=}O)(OX)_2]$ (VI')
où Alk correspondant à Alk défini précédemment dans la formule (I-1ii) représente un radical Alkényle en présence d'un composé de formule

$H_3C\text{-}NH\text{-}NH_2$ (VII')

Cette réaction peut être effectuée par application ou adaptation de la méthode décrite dans J. Org. Chem., 1997, 62, 4834.

De préférence, on opère en milieu solvant aprotique, polaire tel que le THF, le chloroforme, le dichlorométhane, ou l'acétonitrile, de préférence $CH_2Cl_2$, par ajout simultané du (VI') et (VII') au dendrimère à une température comprise entre -80°C et 50°C, de préférence environ 0°C.

Les composés de formule (VI') et (VII') sont disponibles commercialement ou peuvent être préparés selon des méthodes connues en soi.

De préférence, les dendrimères de formule (II-1ii) sont choisis parmi : $SPCl_3$,$P_3N_3Cl_6$,

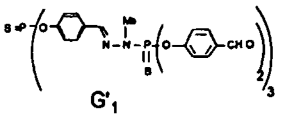

G'1

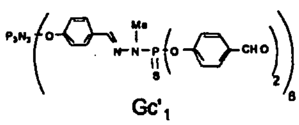

Gc'1

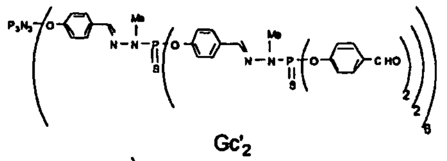

Gc'2

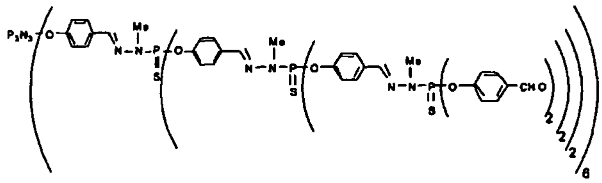

Gc'3

Pour obtenir un composé d'un dendrimère où X=H ou M, l'étape (i) est effectuée de préférence avec un réactif de formule (III), (IV) ou (VI) où X=Me. On effectue ensuite l'étape (ii) à partir du composé de formule (I-1i), (1-2'), (I-3'), (I-1ii) obtenu en (i) où X=Me.

De préférence, l'étape (ii) est effectuée :

- par action d'halogénure de triméthylsilane, de préférence le bromure de triméthylsilane ($Me_3SiBr$), dans un solvant organique aprotique, polaire, tel que l'acétonitrile, le chloroforme, ou le dichlorométhane de préférence l'acétonitrile. De préférence, on opère par ajout lent d'halogénure de triméthylsilane, en maintenant le mélange réactionnel à température comprise entre -80°C et 50°C, de préférence, à environ 0°C.
- suivie de l'action de MeOH anhydre, ajouté au mélange réactionnel.

Dans l'étape (iii), les sels d'acides des dendrimères bisphosphoniques peuvent être obtenus à partir des dendrimères bisphosphoniques présentant un groupe terminal dans lequel X représente un atome d'hydrogène, par l'application ou l'adaptation de procédés connus, par addition d'une base. De préférence, on opère en solution, sous agitation, dans un solvant convenable, protique ou aprotique, polaire, tel que le THF, le chloroforme le dichlorométhane, le DMF, l'acétonitrile, les alcools, l'eau, de préférence l'eau, en présence d'une base organique ou inorganique, telle que l'hydroxyde de sodium, de lithium ou de potassium, selon le sel désiré.

Lorsqu'on utilise des dendrimères de départ présentant des groupes terminaux différents des fonctions terminales décrites plus haut pour les dendrimères de formule (II-1i), (II-1ii), (II-2') ou (II-3), le procédé comprend l'étape préliminaire supplémentaire permettant de transformer lesdits groupes en lesdites fonctions requises. Par exemple, dans le cas de dendrimères présentant des groupes terminaux de type acide carboxylique ou hydroxyle, il suffit d'effectuer toute réaction permettant de convertir lesdits groupes de type acide carboxylique ou hydroxyle en les fonctions de type $-NH_2$, -CHO, -CH=NR ou $-PSCl_2$ correspondant aux dendrimères de formule (II-1i), (II-1ii), (II-2') ou (II-3). De telles réactions sont connues de l'homme du métier et/ou peuvent être effectuées par application ou adaptation de celles discutées par Larock et al (*supra*).

Pour obtenir un dendrimère de génération 0, les réactions ci-dessus peuvent être effectuée de la même façon en opérant à partir du noyau, présentant la fonctionnalité requise. Par exemple, les réactions de génération peuvent être effectuées en opérant à partir d'un noyau $PSCl_3$, $P_3N_3Cl_6$, $P_4N_4Cl_8$, ou

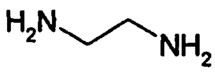,
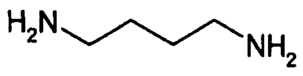.

S'agissant des composés de formule (III) :

$$H\text{-}J\text{-}K\text{-}(Alk)_a\text{-}N<[A2\text{-}P(=O)(OX)_2]_2 \qquad (III)$$

dans laquelle

X représente H, un radical -Alkyle, -Aryle ou $M^+$ où $M^+$ représente un cation ;
J représente un atome d'oxygène, soufre, ou un radical -NR- ;
K représente un radical -Aryle-, -Hétéroaryle-, -Alkyle-, chacun pouvant être éventuellement substitué par un atome d'Halogène ou un radical $-NO_2$, -NRR', -CN, $-CF_3$, -OH, -Alkyle, -Aryle, -Aralkyle ;

chacun des A2, identiques ou différents représentent indépendamment une liaison simple ou une chaîne hydrocarbonée de 1 à 6 chaînons, linéaire ou ramifiée, chacun desdits chaînons pouvant éventuellement être choisi parmi un hétéroatome, de préférence l'azote, chaque chaînon pouvant être éventuellement substitué par un ou plusieurs substituants choisi(s) parmi -Alkyle, -Hal, $-NO_2$, -NRR', -CN, $-CF_3$, -OH, -OAlkyle, -Aryle, -Aralkyle;

-Alk- représente un radical alkyle ;
a représente 0 ou 1 ,

dans lesquels

- de préférence, dans la formule (III) ci-dessus, J représente un atome d'oxygène ;
- de préférence, dans la formule (III) ci-dessus, K représente un noyau phényle, éventuellement substitué ; encore plus préférentiellement, K représente un noyau phényle non substitué ;
- de préférence, dans la formule (III) ci-dessus, -Alk- représente un radical -Ethyle- ;
- de préférence, dans la formule (III) ci-dessus, A2 représente un radical -Alkyle-, encore plus préférentiellement, -Méthyle- ;
- de préférence, dans la formule (III) ci-dessus, X représente -H ou -Me ;

ils peuvent être obtenus à partir d'un composé de formule (VIII') de la façon suivante :

$$H\text{-}J\text{-}K\text{-}(Alk)_a\text{-}NH_2 \ (VIII') + H\text{-}A2'\text{-}(C{=}O)H \ (V') + H\text{-}P({=}O)(OX)_2 \ (IV) \rightarrow H\text{-}J\text{-}K\text{-}(Alk)_a\text{-}N{<}[A2\text{-}PO_3X_2]_2 \ (III)$$

où, dans la formule (V'), -A2'- est un radical correspondant à A2.

Cette réaction est effectuée par application ou adaptation de méthode connue en soi, notamment décrite dans I. Linzaga et al., Tetrahedron 2002, 58, 8973-8978. On opère notamment par ajout lent, des composés (VIII') et (IV), puis du composé (V'), éventuellement dilué en solution aqueuse, de préférence à température comprise entre -5 et 25°C, de préférence à environ 0°C. Puis on laisse le mélange réactionnel s'ajuster à la température ambiante, puis éventuellement réagir sous reflux.

Dans la description du procédé ci-dessus, deux groupes sont dits « correspondants » lorsque ils sont respectivement inclus dans un produit de départ et d'arrivée, et leur structure est identique et peut se déduire l'une de l'autre.

Eventuellement, ledit procédé peut également comprendre l'étape consistant à isoler le produit obtenu à l'issu des étapes (i), (ii), et/ou (iii).

Le composé ainsi préparé peut être récupéré à partir du mélange de la réaction par les moyens traditionnels. Par exemple, les composés peuvent être récupérés en distillant le solvant du mélange de la réaction ou si nécessaire après distillation du solvant du mélange de la solution, en versant le reste dans de l'eau suivi par une extraction avec un solvant organique immiscible dans l'eau, et en distillant le solvant de l'extrait. En outre, le produit peut, si on le souhaite, être encore purifié par diverses techniques, telles que la recristallisation, la reprécipitation ou les diverses techniques de chromatographie, notamment la chromatographie sur colonne ou la chromatographie en couche mince préparative.

Il sera apprécié que les composés utiles selon la présente invention puissent contenir des centres asymétriques. Ces centres asymétriques peuvent être indépendamment en configuration R ou S. Il apparaîtra à l'homme du métier que certains composés utiles selon l'invention peuvent également présenter une isomérie géométrique. On doit comprendre que la présente invention comprend des isomères géométriques individuels et des stéréoisomères et des mélanges de ceux-ci, incluant des mélanges racémiques, de composés de formule (I) ci-dessus. Ces isomères peuvent être séparés de leurs mélanges, par l'application ou l'adaptation de procédés connus, par exemple des techniques de chromatographie ou des techniques de recristallisation, ou ils sont préparés séparément à partir des isomères appropriés de leurs intermédiaires.

Aux fins de ce texte, il est entendu que les formes tautomériques sont comprises dans la citation d'un groupe donné, par exemple thio/mercapto ou oxo/hydroxy.

Les composés utiles selon la présente invention peuvent être facilement préparés, ou formés pendant le processus de l'invention, sous forme de solvates (par exemple hydrates). Les hydrates des composés utiles selon la présente invention peuvent être facilement préparés par la recristallisation dans un mélange de solvants aqueux/organique, en utilisant des solvants organiques tels que dioxane, tétrahydrofuranne ou méthanol.

Les produits de base ou les intermédiaires peuvent être préparés par l'application ou l'adaptation de procédés connus, par exemple des procédés tels que décrits dans les Exemples de Référence ou leurs équivalents chimiques évidents.

Les dendrimères monophosphoniques ou bisphosphoniques selon l'invention pour lesquels une ou deux liaisons du coeur ne sont pas occupées par des chaînes de génération, c'est-à-dire des dendrimères monophosphoniques ou bisphosphoniques ayant une ou deux branches manquantes, sont préparés tels que cela est décrit dans l'**Exemple 91**. Brièvement, un ou deux groupes ne permettant pas l'élongation des chaînes de générations (comme le phénol par exemple) sont fixés sur le noyau avant la mise en oeuvre du processus d'élongation du dendrimère déjà décrit ci-dessus pour les dendrimères monophosphoniques ou bisphosphoniques.

## DESCRIPTION DES FIGURES

### Figure 1A, Figure 1B, Figure 1C, Figure 1D, Figure 1E, Figure 1F, Figure 1G

La Figure 1A représente l'intensité de fluorescence de cellules séparées par cytométrie de flux d'une population initiale de PBMC marquées par un anticorps anti-CD3-FITC (axe des abscisses) et par un anticorps anti-CD56-PC5 (axe des ordonnées). Le panneau 1 correspond aux cellules CD3-CD56$^+$ (23%), le panneau 2 correspond aux cellules CD3$^+$CD56$^+$ (6%), le panneau 3 correspond aux cellules CD3$^-$CD56$^-$ et le panneau 4 correspond aux cellules CD3$^+$CD56$^-$ (50%).

La Figure 1B représente l'intensité de fluorescence de cellules séparées par cytométrie de flux issues d'une population initiale de PBMC cultivées en présence de dendrimère GC1, marquées par un anticorps anti-CD3-FITC (axe des abscisses) et par un anticorps anti-CD56-PC5 (axe des ordonnées). Le panneau 1 correspond aux cellules CD3$^-$CD56$^+$ (76%), le panneau 2 correspond aux cellules CD3$^+$CD56$^+$ (14%), le panneau 3 correspond aux cellules CD3$^-$CD56$^-$ et le panneau 4 correspond aux cellules CD3$^+$CD56$^-$ (8%).

La Figure 1C représente l'intensité de fluorescence de cellules séparées par cytométrie de flux issues d'une population initiale de PBMC cultivées en présence de dendrimère GC1, marquées par un anticorps anti-CD16-PE (axe des abscisses) et par un anticorps anti-CD56-PC5 (axe des ordonnées). Le panneau 1 correspond aux cellules CD16$^-$CD56$^+$, le panneau 2 correspond aux cellules CD16$^+$CD56$^+$, le panneau 3 correspond aux cellules CD16$^-$CD56$^-$ et le panneau 4 correspond aux cellules CD16$^+$CD56$^-$.

La Figure 1D représente l'intensité de fluorescence de cellules séparées par cytométrie de flux issues d'une population initiale de PBMC cultivées en présence de dendrimère GC1, marquées par un anticorps anti-NKG2D révélé par un anticorps GAM (Goat Anti-Mouse)-FITC (axe des abscisses) et par un anticorps anti-CD56-PC5 (axe des ordonnées). Le panneau 1 correspond aux cellules NKG2D$^-$CD56$^+$, le panneau 2 correspond aux cellules NKG2D$^+$CD56$^+$, le panneau 3 correspond aux cellules NKG2D$^-$CD56$^-$ et le panneau 4 correspond aux cellules NKG2D$^+$CD56$^-$.

La Figure 1E représente l'intensité de fluorescence de cellules séparées par cytométrie de flux issues d'une population initiale de PBMC cultivées en présence de dendrimère GC1, marquées par un anticorps anti-NKp30 révélé par un anticorps GAM (Goat Anti-Mouse)-FITC (axe des abscisses) et par un anticorps anti-CD56-PC5 (axe des ordonnées). Le panneau 1 correspond aux cellules NKp30$^-$CD56$^+$, le panneau 2 correspond aux cellules NKp30$^+$CD56$^+$, le panneau 3 correspond aux cellules NKp30$^-$CD56$^-$ et le panneau 4 correspond aux cellules NKp30$^+$CD56$^-$.

La Figure 1F représente l'intensité de fluorescence de cellules séparées par cytométrie de flux issues d'une population initiale de PBMC cultivées en présence de dendrimère GC1, marquées par un anticorps anti-NKp44-PE (axe des abscisses) et par un anticorps anti-CD56-PC5 (axe des ordonnées). Le panneau 1 correspond aux cellules NKp44$^-$CD56$^+$, le panneau 2 correspond aux cellules NKp44$^+$CD56$^+$, le panneau 3 correspond aux cellules NKp44$^-$CD56$^-$ et le panneau 4 correspond aux cellules NKp44$^+$CD56$^-$.

La Figure 1G représente l'intensité de fluorescence de cellules séparées par cytométrie de flux issues d'une population initiale de PBMC cultivées en présence de dendrimère GC1, marquées par un anticorps anti-CD85j (ILT2) révélé par un anticorps GAM (Goat Anti-Mouse)-FITC (axe des abscisses) et par un anticorps anti-CD56-PC5 (axe des ordonnées). Le panneau 1 correspond aux cellules $CD85j^{-}CD56^{+}$, le panneau 2 correspond aux cellules $CD85j^{+}CD56^{+}$, le panneau 3 correspond aux cellules $CD85j^{-}CD56^{-}$ et le panneau 4 correspond aux cellules $CD85j^{+}CD56^{-}$.

**Figure 2A, Figure 2B, Figure 2C, Figure 2D**

Les Figures 2A, 2B, 2C et 2D représentent respectivement les compositions lymphocytaires de cultures de PBMC en présence de GC1 issues de quatre donneurs différents. L'axe vertical représente le nombre de cellules en millions. La barre J0 représente le nombre de cellule au début de l'expérience, la barre GC1 représente le nombre de cellules présentes après 15 jours de culture en présence de GC1 (cellules NK (traits verticaux), cellules T $\gamma\delta$ (blanc) et cellules T $\alpha\beta$ (pointillés)), et la barre contrôle représente le nombre de cellules présentes après 15 jours de culture en présence d'un milieu de culture standard (cellules NK (traits verticaux), cellules T $\gamma\delta$ (blanc) et cellules T $\alpha\beta$ (pointillés)).

**Figure 3**

La Figure 3 représente le nombre de cellules NK obtenues après culture de PBMC de 4 donneurs différents (cercle blanc, cercle noir, triangle blanc, triangle noir) en présence (+GC1) ou en absence (-GC1) de GC1. L'axe des abscisses représente le nombre de cellules en millions et l'axe des ordonnées représente le temps de culture (en semaines).

**Figure 4A, Figure 4B**

La Figure 4A représente le nombre de cellules NK obtenues (axe des ordonnées, en millions) par culture de PBMC en présence de concentrations variables de GC1 (axe des abscisses, en $\mu$M).
La Figure 4B représente le nombre de cellules NK obtenues (axe des ordonnées, en millions) par culture de PBMC de donneurs différents (cercle blanc, cercle noir) en présence d'un milieu de culture standard (0), de GC0, de GC1, de GC2 ou d'un groupement azabisphosphonique seul (monomère).

**Figure 5A, Figure 5B, Figure 5C**

Les Figures 5A et 5B représentent la fluorescence transmise à des cellules NK obtenues à l'aide de GC1 (intensité de fluorescence, axe des ordonnées) par des cellules de lymphome B (Figure 5A) ou de carcinome colique (Figure 5B) marquées par un marqueur membranaire fluorescent, en fonction du temps (axe des abscisses, en minutes).
La Figure 5C représente une photographie prise en microscopie confocale dans laquelle la trogocytose (flèches) de cellules cancéreuses (cible) par une cellule NK est visible.

**Figure 6A, Figure 6B**

La Figure 6A représente schématiquement l'expérience de lyse redirigée dans laquelle des cellules NK provoquent la lyse de cellules cibles (P815) suite à la stimulation de récepteurs des cellules NK réalisée par des anticorps (Ig) fixés par l'intermédiaire du FcR sur lesdites cellules cibles.
La Figure 6B représente les résultats d'une expérience de lyse redirigée effectuée à l'aide de cellules NK obtenues avec GC1 (axe des ordonnées, pourcentage de lyse spécifique) en fonction du rapport E:T (nombre de cellules effectrices:nombre de cellules cibles) (axe des abscisses) en présence d'aucun anticorps (triangles blancs), d'un

anticorps contrôle (triangles noirs), d'un anticorps anti-NKG2D (cercles noirs) ou d'un anticorps anti-NKp30 (cercles blancs).

**Figure 7A, Figure 7B, Figure 7C**

Les Figures 7A-7C représentent la lyse par des cellules NK obtenues avec GC1 (pourcentage de lyse spécifique, axe des ordonnées) de cellule de lymphome de Burkitt (Figure 7A), de cellules LMC K562 (Figure 7B) ou de PBMC autologues (Figure 7C) en fonction du rapport E:T (axe des abscisses).

**Figure 8**

La Figure 8 représente la cytotoxicité exercée par des cellules NK1 obtenues avec GC1 (axe des abscisses, pourcentage de lyse spécifique) vis-à-vis de cellules de lignée cellulaires de leucémies ou de carcinomes en fonction du rapport E:T de 1:1 (barres blanches) ou de 10:1 (barres grises).

**Figure 9**

La Figure 9 représente des exemples schématisés de trois dendrimères, un dendrimère de génération 4, pour lequel n = 4 et m = 3 (à gauche, A), un dendrimère de génération 3, pour lequel n = 3 et m = 6 (à droite, B) et un dendrimère de génération 0, pour lequel n = 0 et m = 6 (en bas, C). Les éléments constitutifs d'un dendrimère sont représentés : un noyau (§) sur lequel viennent se fixer des chaînes de génération (D) pour les dendrimères de génération supérieure ou égale à 1, ou directement des chaînes intermédiaires (E) pour les dendrimères de génération 0, des chaînes intermédiaires (E) fixées à l'extrémité des chaînes de génération, les chaînes intermédiaires étant également reliées à des groupes terminaux (f), l'ensemble des groupes terminaux formant la surface du dendrimère. Les chaînes de liaison sont constituées d'un groupe de chaînes de génération comprenant une chaîne de génération fixée au noyau et l'ensemble des autres chaînes de génération qui sont reliées à cette chaîne soit directement, soit par l'intermédiaire d'autres chaînes de génération.

**Figure 10A et Figure 10B**

La Figure 10A représente une population de lymphocyte totaux séparés en cytométrie de flux en fonction de leur taille (axe des abscisses, FS lin) et de leur granularité (PMT1 lin), ainsi que ceux sélectionnés pour être incubés en présence de dendrimères fluorescents (cercle).

La Figure 10B représente les résultats de cultures des lymphocytes ci-dessus en présence d'IL2, ou de dendrimère fluorescent (GC1F) et d'IL2, ou de GC1 et d'IL2, après 4 h (A), 24 h (B) ou 15 jours (C). L'axe des abscisses représente l'intensité de fluorescence (unités arbitraires) et l'axe des ordonnées le nombre de cellules (unités arbitraires).

**Figure 11**

La Figure 11 représente le nombre de cellules NK (axe des ordonnées, en millions) obtenues après culture, en présence de GC1 pendant 2,5 semaines, de PBMC issus de donneurs sains et de patients cancéreux atteints de Myélome Multiple (cercles pleins) par rapport au nombre de cellules NK présentes au début de la culture (cercles vides).

**Figure 12A et Figure 12B**

La Figure 12A représente le nombre de cellules NK obtenues (axe des ordonnées, en millions) en fonction de la durée de culture (axe des abscisses, en jours) de PBMC de donneurs sains en présence d'IL2 (carrés vides), d'IL15 (triangles vides), d'IL2 + IL15 (cercles vides), d'IL2 + GC1 (carrés pleins), d'IL15 + GC1 (triangles pleins) et d'IL2 + IL15 + GC1 (cercles pleins).

La Figure 12B représente le pourcentage de cellules NK (axe des ordonnées) dans une culture de PBMC de donneurs sains en fonction de la durée de la culture (axe des abscisses, en jours), en présence d'IL2 (carrés vides), d'IL15 (triangles vides), d'IL2 + IL15 (cercles vides), d'IL2 + GC1 (carrés pleins), d'IL15 + GC1 (triangles pleins) et d'IL2 + IL15 + GC1 (cercles pleins).

**Figure 13**

La figure 13 représente l'effet de dendrimères phosphorés de type PMMH à extrémités sel de Na sur l'amplification de cellules NK. 0 correspond à l'amplification en milieu de culture par l'IL2 seule (sans dendrimère). Les autres numéros correspondent aux numéros des exemples des dendrimères en question, sous leur forme sels de sodium.

**Figure 14A, Figure 14B, Figure 14C et Figure 14D**

La figure 14A représente la photographie de lames, observées en microscopie optique de monocytes cultivés en absence (à gauche) ou en présence (à droite) du dendrimère GC1. La barre horizontale représente 30 μm.

La figure 14B représente l'analyse en cytométrie de flux (axe des abscisses, moyenne d'intensité de fluorescence (MIF)) de l'expression des marqueurs HLA-A, B, C, CD14 et HLA-DR sur des monocytes en culture en présence (barres noires) ou en absence de GC1 (barres grises). Le symbole étoile (***) représente une différence de MIF significative en test de student ($p < 0,001$). Les résultats sont représentatifs de 3 donneurs.

La figure 14C représente la translocation nucléaire de NFκB p50 (axe des ordonnées, RLUx$10^6$) (RLU : Unité de Luminescence Relative) au sein de monocytes cultivés en absence (triangles) ou en présence (cercles) de GC1.

La figure 14D représente le nombre de monocytes viables (axe des ordonnées, en millions) en fonction de la durée de culture (axe des abcisses, en jours) de monocytes cultivés en absence (barres grises) ou en présence (barres noires) de GC1 (premier graphe en partant de gauche) et le pourcentage de monocytes positifs à l'annexine-V (axe des ordonnées) en fonction de la durée de culture de monocytes cultivés en absence (barres grises) ou en présence (barres noires) de GC1 (deuxième graphe en partant de gauche). Le pourcentage de monocytes positifs à l'annexine-V est déterminé par cytométrie de flux à l'aide d'un anticorps anti-annexine-V marqué au FITC. Des exemples de détermination du pourcentage de monocytes positifs à l'annexine-V pour des monocytes cultivés pendant 6 jours en absence de GC1 (troisième graphe en partant de gauche : 35% de monocytes positifs à l'annexine-V) et pour des monocytes cultivés pendant 6 jours en présence de GC1 (quatrième graphe en partant de la gauche : 5% de monocytes positifs à l'annexine-V).

## EXEMPLES

### GENERALITES

Les réactions ont été réalisées sous atmosphère d'argon sec (argon U, Air Liquide). Les solvants suivants ont été séchés et distillés sous argon immédiatement avant usage selon les techniques décrites par Perrin et al, Purification of Laboratory Chemicals, Third Edition; Press, P., Ed.: Oxford, 1988 : tétrahydrofuranne, dichlorométhane, acétonitrile, pentane, toluène, éther diéthylique, chloroforme, triéthylamine, pyridine.

Les chromatographies sur couche mince ont été réalisées sur des plaques d'aluminium enduites de silice de type Merck Kieselgel $60F_{254}$.

Les spectres de RMN ont été enregistrés sur des appareils Brüker (AC200, AM250, DPX 300). Les déplacements chimiques sont exprimés en parties par million (ppm) par rapport à l'acide phosphorique à 85 % dans l'eau pour la RMN $^{31}P$ et par rapport au tétraméthylsilane pour la RMN $^{1}H$ et $^{13}C$. Les abréviations suivantes ont été utilisées pour exprimer la multiplicité des signaux : s (singulet), d (doublet), d1 (doublet large), dd (doublet dédoublé), syst.AB (système AB), t (triplet), td (triplet dédoublé), q (quadruplet), hept (heptuplet), m (multiplet non résolu).

La spectroscopie vibrationnelle dans l'infrarouge a été réalisée sur un spectromètre Perkin Elmer FT 1725x. La spectroscopie UV-visible a été réalisée sur un appareil HP 4852A. Les mesures thermogravimétriques ont été réalisées sur un appareil Netzch DSC 204 ou Setaram TGA 92-16.18.

*Numérotation utilisée pour l'attribution en RMN :*

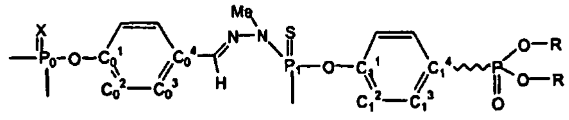

R = Me, H, Na

Exemple de numérotation pour un dendrimère de première génération

*Structures des différents dendrimères utilisés comme produit de départ*

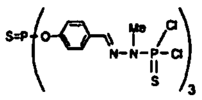

$G_1$

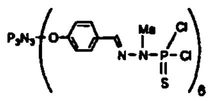

$Gc_1$

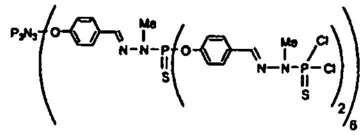

$Gc_2$

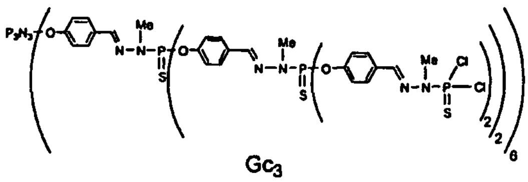

$Gc_3$

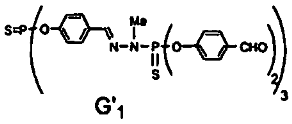

$G'_1$

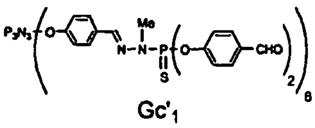

$Gc'_1$

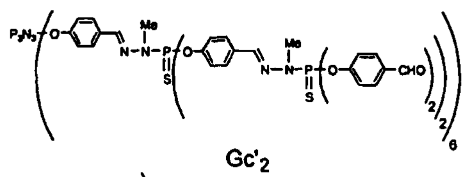

$Gc'_2$

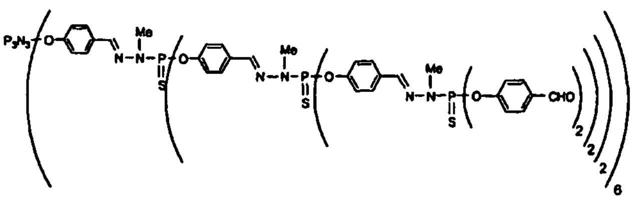

$Gc'_3$

**Exemple 1 : Synthèse du dendrimère de première génération (coeur P=S) à extrémités acide α-hydroxy-diméthylphosphonique**

*Etape 1 : Synthèse du dendrimère de première génération (coeur P=S) à extrémités α-hydroxy-diméthylphosphonate*

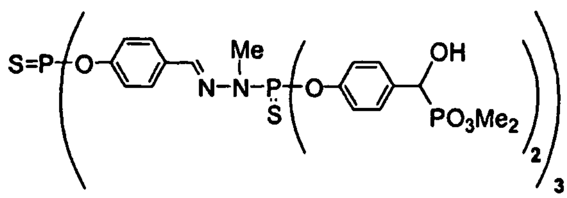

Le dendrimère G'$_1$ (0,14 mmol, 200 mg) est mis en solution dans 0,2 ml de THF avec de la triéthylamine distillée (0,126 mmol, 4,5 $\mu$L), et du diméthylphosphite (1,26 mmol, 115 $\mu$L). On laisse le mélange pendant 12 heures avec une agitation magnétique. La pâte obtenue est ensuite lavée avec un mélange THF/$Et_2O$: 1/1, pour donner une poudre blanche. Le produit final est isolé avec un rendement de 72%.

RMN $^{31}P-\{^1H\}$ (DMSO d6) : $\delta$ = 27,10 (s, $P(O)(O-CH_3)_2$), 56,10 (s, $P_0$), 65,91 (s, $P_1$) ppm. RMN $^1H$ (DMSO d6) : $\delta$ = 3,34 (d, $^3J_{HP}$ = 9,8 Hz, 9H, $CH_3$-N-$P_1$), 3,52 (d, 18H, $^3J_{HP}$= 10,3 Hz, P(O)-O-$CH_3$), 3,57 (d, 18H, $^3J_{HP}$ = 11,6 Hz, P(O)-O-$CH_3$), 5,01 (dd, $^3J_{HH}$ = 4,5 Hz, $^2J_{HP}$ = 13,0 Hz, 6H, CH-P(O)), 6,33 (dd, $^3J_{HH}$ = 5,6 Hz, $^3J_{Hp}$ = 15,7 Hz, 6H, OH), 7,18-7,93 (m, 39H, $H_{arom}$, CH=N) ppm.

RMN $^{13}C-\{^1H\}$ (DMSO d6) : $\delta$ = 33,9 (d, $^2J_{CP}$ = 12,1 Hz, $CH_3$-N-$P_1$), 53,7 (d, $^2J_{CP}$ = 6,8 Hz, $CH_3$-O-P(O)), 54,2 (d, $^2J_{CP}$= 7,0 Hz, $CH_3$-O-P(O)), 70,0 (d, $^1J_{CP}$ = 162,8 Hz, C-OH), 121,3 (s large, $C_1^2$), 122,4 (d, $^3J_{CP}$= 3,8 Hz, $C_0^2$), 129, 4 (s, $C_0^3$), 129,8 (d, $^3J_{CP}$= 5,6 Hz, $C_1^3$), 133,6 (s, $C_0^4$), 136,4 (s, $C_1^4$), 141,4 (d, $^3J_{CP}$ = 14,5 Hz, CH=N), 150,3 (dd, $^5J_{CP}$= 3,4 Hz, $^2J_{CP}$ = 6,6 Hz, $C_1^1$), 151,4 (d, $^2J_{CP}$ = 8,0 Hz, $C_0^1$) ppm.

*Etape 2 : Synthèse du dendrimère G'$_1$ à extrémités acide $\alpha$-hydroxy-phosphonique (sel de Na)*

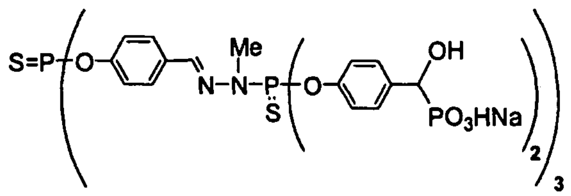

A une solution de dendrimère G'$_1$ de première génération à extrémités $\alpha$-hydroxy-diméthyl-phosphonate, (0,8 mmol, 1g) obtenu à l'étape 1 à 0°C dans de l'acétonitrile (5 mL) avec de la triéthylamine distillée (4,8 mmol) on additionne lentement du bromotriméthylsilane (15,8 mmol). Une fois l'addition terminée on laisse le mélange revenir à température ambiante pendant 12 heures. Le mélange est alors évaporé à sec puis on additionne 1 mL de méthanol anhydre à température ambiante et on laisse le mélange une heure sous agitation. Après évaporation à sec, le résidu est lavé plusieurs fois à l'éther. Le produit étant totalement insoluble dans les solvants organiques il est transformé en son mono sel de sodium en présence d'une solution d'hydroxyde de sodium préalablement titrée. La solution résultante est lyophilisée pour donner le dendrimère sous forme d'une poudre blanche. Le produit final est isolé avec un rendement de 48%.

RMN $^{31}P-\{^1H\}$ ($D_2O/CD_3CN$) : $\delta$ = 10,1 (s, P(O)(OH)(ONa)), 56,10 (s, $P_0$), 66,91 (s, $P_1$) ppm.

**Exemple 2 : Synthèse du dendrimère de première génération (coeur $P_3N_3$) à extrémités $\alpha$-hydroxy-diméthyl-phosphonate**

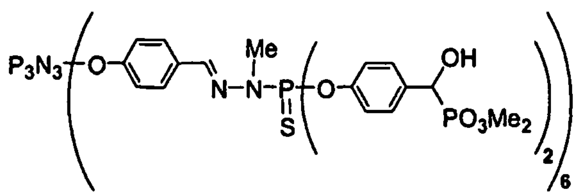

On met 1 g de Gc'$_1$ (0,35 mmol) en solution dans 1 ml de THF puis on additionne la triéthylamine distillée (10 $\mu$l soit 0,84.10$^{-3}$ mol), et le diméthylphosphite (382 $\mu$L soit 4,2.10$^{-3}$ mol) (1 équiv par -CHO). On laisse le mélange pendant 12 heures sous agitation. La pâte obtenue est ensuite lavée avec un mélange THF/$Et_2O$ : 1/1, pour donner une poudre blanche. Le produit final est isolé avec un rendement de 72%.

RMN $^{31}P-\{^1H\}$ (DMSO d6) : $\delta$ =11,46 (s, $P_0$), 27,10 (s, $P(O)(O-CH_3)_2$), 66,07 (s, $P_1$) ppm.

RMN $^1H$ (DMSO d6) : $\delta$ = 3,35 (d, $^3J_{HP}$ = 10,5 Hz, 18H, $CH_3$-N-$P_1$), 3,54 (d, $^3J_{HP}$ = 10,3 Hz, 36H, P(O)-O-$CH_3$), 3,59 (d, $^3J_{HP}$ = 10,4 Hz, 36H, P(O)-O-$CH_3$), 5,01 (dd, $^3J_{HH}$ = 5,2 Hz, $^2J_{HP}$ = 13,5 Hz, 12H, CH-P(O)), 6,41 (dd, $^3J_{HH}$ = 5,6 Hz, $^3J_{HP}$= 15,5 Hz, 12H, OH), 7,18-7,93 (m, 78H, $H_{arom}$, CH=N) ppm.

RMN $^{13}C-\{^1H\}$ (DMSO d6) : $\delta$ = 32,8 (d, $^2J_{CP}$ = 11,9 Hz, $CH_3$-N-$P_1$), 52,7 (d, $^2J_{CP}$ = 6,9 Hz, $CH_3$-O-P(O)), 53,2 (d, $^2J_{CP}$ = 6,9 Hz, $CH_3$-O-P(O)), 68,2 (d, $^1J_{CP}$ = 162,3 Hz, C-OH), 120,4 (s large, $C_1^2$), 120,8 (s, $C_0^2$), 128,2 (s, $C_0^3$), 128,7

(d, $^3J_{CP}$= 5,7 Hz, $C_1^3$), 132,0 (s, $C_0^4$), 135,5 (s, $C_1^4$), 140,2 (d, $^3J_{CP}$=13,8 Hz, CH=N), 149,4 (d, $^2J_{CP}$= 6,3 Hz, $C_1^1$), 150,5 (s, $C_0^1$) ppm. IR: Absence de υ(CHO) à 1670 $cm^{-1}$; υ(OH) à 3271 $cm^{-1}$.

**Exemple 3 : Synthèse du dendrimère de deuxième génération à extrémités α-hydroxy-diméthylphosphonate**

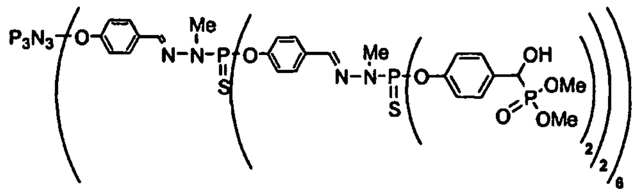

Le dendrimère Gc'$_2$ (0,146 mmol, 1g) est mis en solution dans 1 ml de THF avec de la triéthylamine distillée (1,3 mmol, 15 μL), et du diméthylphosphite (3,5 mmol, 319 μL). On laisse le mélange pendant 12 heures avec une agitation magnétique. La pâte obtenue est ensuite lavée avec un mélange THF/$Et_2O$ : 1/1, pour donner une poudre blanche. Le produit final est isolé avec un rendement de 80%.
RMN $^{31}P$-{$^1H$} (DMSO d6) : δ = 11,7 (s, $P_0$), 27,10 (s, $P(O)(O-CH_3)_2$), 66,1 (s large, $P_{1,2}$) ppm.
RMN $^1H$ (DMSO d6) : δ = 3,29 (d large, $^3J_{HP}$ = 9,2 Hz, 54H, $CH_3$-N-$P_1$ , $CH_3$-N-$P_2$), 3,49 (d, $^1J_{CP}$=10,9 Hz, 72H, P(O)-O-$CH_3$), 3,55 (d, $^2J_{CP}$= 10,6 Hz, 72H, P(O)-O-$CH_3$), 5,00 (dd, $^3J_{HH}$ = 5,4 Hz, $^2J_{HP}$= 15,7 Hz, 24H, CH-P(O)), 6,30 (dd, $^3J_{HH}$ = 5,4 Hz, $^2J_{HP}$ = 15,7 Hz, 24H, OH), 7,0-8,0 (m, 186H, $H_{arom}$, CH=N) ppm.
RMN $^{13}C$-{$^1H$} (DMSO d6) : δ = 32,8 (d large, $^2J_{CP}$ = 11,3 Hz, $CH_3$-N-$P_{1,2}$), 52,7 (d, $^2J_{CP}$ = 6,2 Hz, $CH_3$-O-P(O)), 53,2 (d, $^2J_{CP}$ = 6,3 Hz, $CH_3$-O-P(O)), 68,2 (d, $^1J_{CP}$ = 163,0 Hz, C-OH), 120,4 (s large, $C_2^2$), 120,8 (s large, $C_0^2$), 121,4 (s, $C_1^2$), 128,2 (s, $C_0^3$), 128,2 (s, $C_1^3$), 128,7 (d, $^3J_{CP}$= 3,7 Hz, $C_2^3$), 132,1 (s, $C_0^4$), 132,1 (s, $C_1^4$), 135,4 (s, $C_2^4$), 140,2 (s large, <u>CH</u>=N-N(Me)-$P_{1,2}$), 149,4 (d, $^2J_{CP}$= 3,8 Hz, $C_2^1$), 150,4 (s, $C_0^1$) 150,7 (d, $^2J_{CP}$= 6,4 Hz, $C_1^1$) ppm.
IR: Absence de υ(CHO) à 1670 $cm^{-1}$; υ(OH) à 3271 $cm^{-1}$.

**Exemple 4 : Synthèse du dendrimère de troisième génération à extrémités α-hydroxy-diméthylphosphonate**

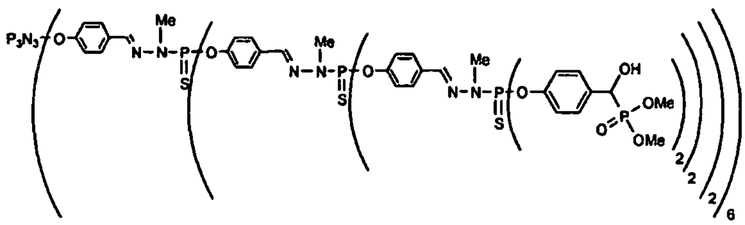

Le dendrimère Gc'$_3$ (1,35.10$^{-2}$ mmol, 0,2 g) est mis en solution dans 0,2 ml de THF avec de la triéthylamine distillée (0,8 mmol, 10 μL), et du diméthylphosphite (0,648 mmol, 59 μL). On laisse le mélange pendant 12 heures avec une agitation magnétique. La pâte obtenue est ensuite lavée avec un mélange THF/$Et_2O$: 1/1, pour donner une poudre blanche. Le produit final est isolé avec un rendement de 85%.
RMN $^{31}P$-{$^1H$} (DMSO d6) : δ= 11,7 (s, $P_0$), 28,6 (s, $P(O)(O-CH_3)_2$), 66,4 (s large, $P_{1,2,3}$) ppm.
RMN $^1H$ (DMSO d6) : δ = 3,40 (d large, $^3J_{HP}$= 10,7 Hz, 126H, $CH_3$-N-$P_1$, $CH_3$-N-$P_2$, CH3-N-$P_3$), 3,60 (d, $^2J_{CP}$=13,15 Hz, 144H, P(O)-O-$CH_3$), 3,65 (d, $^2J_{CP}$= 13,16 Hz, 144H, P(O)-O-$CH_3$), 5,10 (dd, $^3J_{HH}$ = 4,3 Hz, $^2J_{HP}$ = 15,3 Hz, 48H, CH-P(O)), 6,4 (dd, $^3J_{HH}$ = 4,3 Hz , $^2J_{HP}$= 15,3 Hz, 48H, OH), 7,0-8,1 (m, 402H, $H_{arom}$, CH=N) ppm.
RMN $^{13}C$-{$^1H$} (DMSO d6) : δ = 32,8 (s large, $CH_3$-N-$P_{1,2,3}$), 52,7 (d, $^2J_{CP}$ = 6,3 Hz, $CH_3$-O-P(O)), 53,2 (d, $^2J_{CP}$= 7,4 Hz, $CH_3$-O-P(O)), 68,1 (d, $^1J_{CP}$ = 162,8 Hz, C-OH), 119,5 (s, $C_1^2$), 120,4 (s large, $C_3^2$, $C_0^2$), 121,4 (s, $C_2^2$), 128,3 (s large, $C_0^3$, $C_1^3$, $C_2^3$), 128,6 (d, $^3J_{CP}$ = 4,2 Hz, $C_3^3$), 132,1 (s, $C_0^4$, $C_1^4$, $C_2^4$), 135,5 (s, $C_3^4$), 140,2 (s large, <u>CH</u>=N-N (Me)-$P_{1,2,3}$), 149,4 (d, $^2J_{CP}$= 8,3 Hz, $C_3^1$), 150,6 (s large, $C_0^1$, $C_1^1$, $C_2^1$) ppm.
IR: Absence de υ(CHO) à 1670 $cm^{-1}$; υ(OH) à 3271 $cm^{-1}$.

**Exemple 5 : Synthèse du dendrimère de première génération à extrémités acide α-hydroxy-phosphonique**

*Etape 1 : Dendrimère à extrémités acide α-hydroxy-phosphonique*

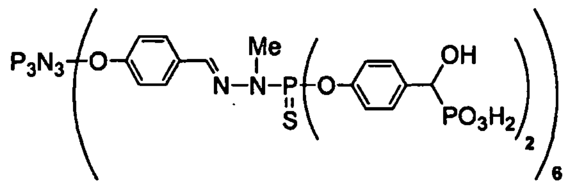

Le dendrimère de première génération (4,78.10$^{-2}$ mmol, 200 mg) à extrémités α-hydroxy-diméthylphosphonate obtenu dans l'**Exemple 2** est mis en suspension dans l'acétonitrile (4 mL) avec de la triéthylamine (0,575 mmol, 20,5 μL) à 0°C. Puis le bromure de triméthylsilane (1,72 mmol, 229 μL) est additionné lentement à 0°C, l'ensemble revient lentement à température ambiante pendant 6 heures. Puis on ajoute du méthanol anhydre (1 mL). Après 2 heures d'agitation le mélange réactionnel est séché sous pression réduite. Ensuite la poudre est mise en suspension dans un minimum d'eau pendant 30 minutes avec une forte agitation. Après filtration, le produit est séché puis lavé abondamment à l'éther. De préférence, pour obtenir un dendrimère soluble, le dendrimère, final ne doit pas être totalement désolvaté. Le produit final est isolé avec un rendement de 51 %.
RMN $^{31}$P-{$^1$H} (DMSO d6) : δ = 11,40 (s, $P_0$), 22,0 (m, $P(O)(OH)_2$), 66,05 (s, $P_1$) ppm. RMN $^1$H (DMSO d6) : δ = 3,29 (d, $^3J_{HP}$= 10,5 Hz, 18H, $CH_3$-N-$P_1$), 4,67 (d, $^3J_{HP}$= 13,9 Hz, 12H, -CH-OH), 4,7-5,7 (m, 36H, -OH), 7,0-8,0 (m, 78H, $H_{arom}$, CH=N) ppm.
RMN $^{13}$C-{$^1$H} (DMSO d6) : δ = 32,9 (d, $^2J_{CP}$ = 15,7 Hz, $CH_3$-N-$P_1$), 69,5 (d, $^1J_{CP}$ = 163,5 Hz, C-OH), 120,0 (s large, $C_1^2$), 120,7 (s, $C_0^2$), 128,2 (s, $C_0^3$), 128,6 (s, $C_1^3$), 132,0 (s, $C_0^4$), 137,1 (s, $C_1^4$), 140,2 (s large, CH=N), 148,8 (s, $C_1^1$), 150,4 (s, $C_0^1$) ppm.

IR: Absence de υ(CHO) à 1670 cm$^{-1}$; υ(OH) à 3271 cm$^{-1}$.

*Etape 2 : Dendrimère à extrémités sel de sodium d'acide α-hydroxy phosphonique*

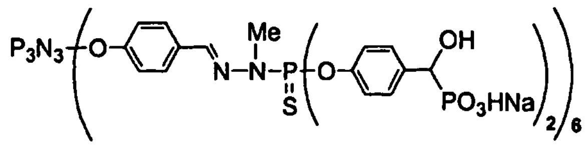

Le dendrimère obtenu à l'étape précédente est dissous dans une solution de soude titrée (0,1966 M, 12 éq.). La solution obtenue est filtrée sur filtre millipore puis lyophilisée. Le dendrimère de première génération à extrémités mono sel de sodium de l'acide alpha-hydroxyphosphonique est obtenu sous forme d'une poudre blanche avec un rendement de 82%.
RMN $^{31}$P{$^1$H} ($D_2O/CD_3CN$, 81 MHz) : δ = 12,5 (s 1, $N_3P_3$), 17,8 (s 1, P=O), 67,5 (s 1, P=S).

**Exemple 6 : Synthèse du dendrimère de deuxième génération à extrémités acide α-hydroxy-phosphonique**

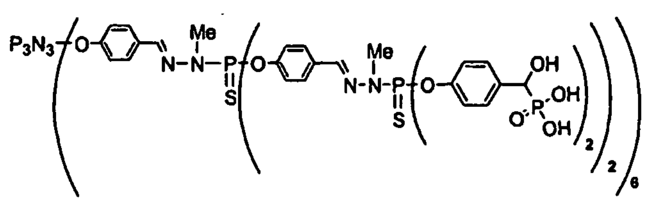

Le dendrimère de deuxième génération (3,16.10$^{-2}$ mmol, 300 mg) à extrémités α hydroxy-diméthylphosphonate

obtenu dans l'**Exemple 3** est mis en suspension dans l'acétonitrile (1,5 mL) avec de la triéthylamine (0,86 mmol, 30 µL) à 0°C. Puis le bromure de triméthylsilane (2,3 mmol, 304 µL) est additionné lentement à 0°C, l'ensemble revient lentement à température ambiante pendant 6 heures. Puis on ajoute du méthanol anhydre (1 mL). Après 2 heures d'agitation le mélange réactionnel est séché sous pression réduite. Ensuite la poudre est mise en suspension dans un minimum d'eau pendant 30 minutes avec une forte agitation. Après filtration, le produit est séché puis lavé abondamment à l'éther. De préférence, si un produit soluble est désiré, le dendrimère final ne doit pas être totalement désolvaté. Le produit final est isolé avec un rendement de 62%.

RMN $^{31}P-\{^1H\}$ (DMSO d6) : δ = 11,9 (s, $P_0$), 21,5 (m, $P(O)(OH)_2$), 66,00 (s large, $P_{1,2}$) ppm. RMN $^1H$ (DMSO d6) : δ = 3,06 (s large, 54H, $CH_3$-N-$P_{1,2}$), 4,66 (d, $^3J_{HP}$ = 14,0 Hz, 24H, - CH-OH), 3,7-5,2 (m, 72H, -OH), 6,7-8,0 (m, 186H, $H_{arom}$, CH=N) ppm.

RMN $^{13}C-\{^1H\}$ (DMSO d6) : δ = 33,6 (s large, $CH_3$-N-$P_{1,2}$), 70,2 (d, $^1J_{CP}$= 158,5 Hz, C-OH), 121,0 (s large, $C_2^2$, $C_0^2$), 122,0 (s, $C_1^2$), 129,5 (s large, $C_0^3$, $C_1^3$, $C_2^3$), 132,8 (s, $C_0^4$, $C_1^4$), 137,4 (s, $C_2^4$), 141,0 (s large, CH=N), 149,8 (s large, $C_2^1$), 151,2 (s large, $C_0^1$, $C_1^1$) ppm.

IR: Absence de υ(CHO) à 1670 $cm^{-1}$; ν(OH) à 3271 $cm^{-1}$.

Les dérivés à terminaisons acide phosphonique peuvent être obtenus par application ou adaptation de cette méthode à partir des composés des exemples 1 à 5 et 8 à 10 présentant un groupe diméthyl phosphonate. Cette réaction ne fonctionne pas à partir du composé présentant un groupe diisopropyl phosphonate de l'**Exemple 7**.

**Exemple 7 : Synthèse du dendrimère de première génération à terminaisons vinyl-diisopropyl-phosphonate**

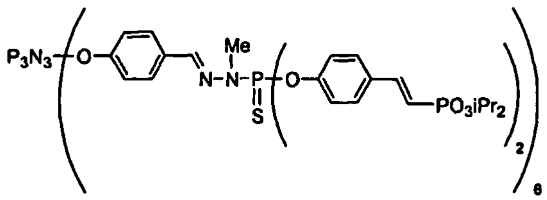

Le tétraisopropyl-méthylène-gem-diphosphonate (3 mmol) ainsi que de l'hydrure de sodium (3 mmol, 75 mg) sont mis dans 2 mL de THF distillé. Ce mélange est laissé sous une forte agitation pendant 2 heures à température ambiante. Une fois le dégagement d'hydrogène fini, il est additionné lentement sur le dendrimère $Gc'_1$ (0,17 mmol, 500 mg) qui a été préalablement mis en solution dans 3 mL de THF distillé. L'addition est effectuée à 0°C puis on laisse le mélange revenir à température ambiante pendant une nuit. Après évaporation du THF, le solide blanc est lavé avec un mélange pentane / éther 1/1 de manière à retirer l'excès de tétraisopropyl-méthylène-gem-diphosphonate. Ensuite le dendrimère est mis en suspension dans le minimum d'eau, la solution trouble obtenue est centrifugée. Une poudre blanche est récupérée après centrifugation mais il peut parfois être nécessaire de répéter une deuxième fois l'opération (centrifugation) toujours avec le minimum d'eau. Le produit final est isolé avec un rendement de 55%.

RMN $^{31}P-\{^1H\}$ ($CDCl_3$) : δ = 11,66 (s, $P_0$); 65,73 (s, $P_1$); 20,31 (s, P=O) ppm.

RMN $^1H$ ($CDCl_3$) : δ = 1,26 (d, $^3J_{HH}$ = 6,2 Hz, 72H, $CH_3$-CH); 1,32 (d, $^3J_{HH}$ = 6,2 Hz, 72H $CH_3$-CH); 3,27 (d, $^3J_{HP}$= 10,4 Hz, 18H, N-Me); 4,66 (hept, $^3J_{HH}$ = 5,9 Hz, 24H, O-CH-$(CH_3)_2$); 6,14 (dd, $^3J_{HH\ trans}$ = $^2J_{HP(O)}$ = 17,1 Hz, 12H, -CH=CH-P (O)); 6,9-7,7 (m, 90H, $CH_{arom}$, CH=N, -CH=CH-P(O)) ppm.

RMN $^{13}C-\{^1H\}$ ($CDCl_3$) : δ = 24,0 (d, $^3J_{CP}$ = 5,0 Hz, $CH_3$-CH); 32,9 (d, $^2J_{CP}$= 12 Hz, $CH_3$-N-$P_1$); 70,5 (d, $^2J_{CP}$= 5,0 Hz, -O-CH-$CH_3$); 116,1 (d, $^1J_{CP}$= 192,52 Hz, -CH=CH-P(O)$(OiPr)_2$); 121,4 (s large, $C_0^2$); 121,8 (d, $^3J_{CP}$ = 4,9 Hz, $C_1^2$); 128,3 (s, $C_0^3$); 129,0 (s, $C_1^3$); 132,2 (d, $^3J_{CP}$ = 18,7 Hz, $C_1^4$); 132,7 (s, $C_0^4$); 139,0 (d, $^3J_{CP}$ = 14,46 Hz, CH=N); 146,3 (d, $^2J_{CP}$ = 6,3 Hz, - CH=CH-P(O)$(OiPr)_2$); 151,3 (s large, $C_0^1$); 151,6 (d, $^2J_{CP}$= 5,7 Hz, $C_1^1$) ppm.

**Exemple 8 : Synthèse du dendrimère de première génération à terminaisons vinyl-diméthyl-phosphonate**

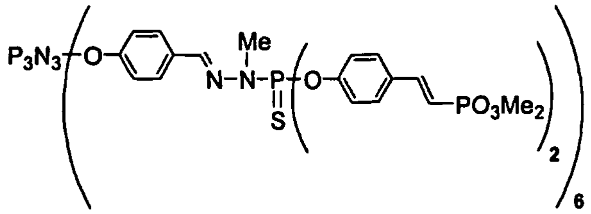

Le tétraméthyl-méthylène-gem-diphosphonate (11,7 mmol, 2,7 g) ainsi que de l'hydrure de sodium (11,7 mmol, 281 mg) sont mis dans 10 mL de THF distillé. Ce mélange est laissé sous une forte agitation pendant 2 heures à température ambiante. Une fois le dégagement d'hydrogène fini, il est additionné lentement sur le dendrimère Gc'$_2$ (0,7 mmol, 1 g) qui a été préalablement mis en solution dans 5 mL de THF distillé. L'addition est effectuée à 0°C puis on laisse le mélange revenir à température ambiante pendant une nuit. Après évaporation du THF, le solide blanc est lavé avec un mélange pentane / éther 1/1 de manière à retirer l'excès de tétraméthyl-méthylène-gem-diphosphonate. Ensuite le dendrimère est mis en suspension dans le minimum d'eau, la solution trouble obtenue est centrifugée. Une poudre blanche est récupérée après centrifugation mais il peut parfois être nécessaire de répéter une deuxième fois l'opération (centrifugation) toujours avec le minimum d'eau. Le produit final est isolé avec un rendement de 63%.
RMN $^{31}$P-{$^1$H} (CDCl$_3$) : $\delta$ = 11,7 (s, P$_0$); 65,5 (s, P$_1$); 25,43 (s, P=O) ppm.
RMN $^1$H (CDCl$_3$) : $\delta$ = 3,27 (d, $^3J_{HP}$ = 9,5 Hz, 18H, N-Me); 3,72 (d, $^3J_{HP}$ = 10,6 Hz, 72H, O-CH$_3$); 6,08 (dd, $^3J_{HH\ trans}$ = $^2J_{HP}$(O) = 16,9 Hz, 12H, -CH=CH-P(O)); 6,9-7,8 (m, 90H, CH$_{arom}$, CH=N, -CH=CH-P(O)) ppm.
RMN $^{13}$C-{$^1$H} (CDCl$_3$) : $\delta$ = 32,9 (d, $^2J_{CP}$=12,13 Hz, CH$_3$-N-P$_1$); 52,4 (d, $^2J_{HP}$= 5,6 Hz, -O-CH$_3$); 112,7 (d, $^1J_{CP}$= 191,64 Hz, -CH=CH-P(O)(OMe)$_2$); 121,3 (s large, C$_0^2$); 121,7 (d, $^3J_{CP}$ = 3,2 Hz, C$_1^2$); 128,2 (s, C$_0^3$); 129,1 (s, C$_1^3$); 131,9 (s, C$_0^4$); 132,1 (d, $^3J_{CP}$= 16,9 Hz, C$_1^4$); 139,0 (d, $^3J_{CP}$ = 13,4 Hz, CH=N); 148,03 (d, $^2J_{CP}$ = 6,8 Hz, -CH=CH-P(O)(OMe)$_2$); 151,2 (s large, C$_0^1$); 151,8 (d, $^2J_{CP}$ = 6,3 Hz, C$_1^1$) ppm.

**Exemple 9 : Synthèse du dendrimère de seconde génération à terminaisons vinyl-diméthyl-phosphonate**

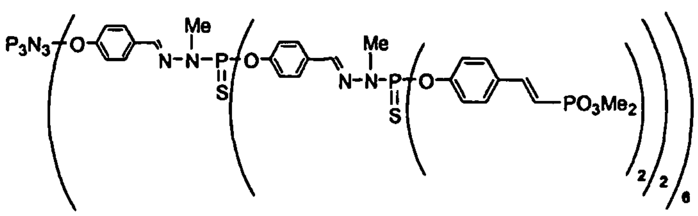

Le tétraméthyl-méthylène-gem-diphosphonate (0,77 mmol, 0,18 g) ainsi que de l'hydrure de sodium (0,78 mmol, 19 mg) sont mis dans 4 mL de THF distillé, ce mélange est laissé sous une forte agitation pendant 2 heures à température ambiante. Une fois le dégagement d'hydrogène fini, il est additionné lentement sur le dendrimère Gc'$_2$ (2,9.10$^{-2}$ mmol, 0,2 mg) qui a été préalablement mis en solution dans 2 mL de THF distillé. L'addition est effectuée à 0°C puis on laisse le mélange revenir à température ambiante pendant une nuit. Après évaporation du THF, le solide blanc est lavé avec un mélange pentane / éther 1/1 de manière à retirer l'excès de tétraméthyl-méthylène-gem-diphosphonate. Ensuite, le dendrimère est mis en suspension dans le minimum d'eau, la solution trouble obtenue est centrifugée. Une poudre blanche est récupérée après centrifugation mais il peut parfois être nécessaire de répéter une deuxième fois l'opération (centrifugation) toujours avec le minimum d'eau. Le produit final est isolé avec un rendement de 68%.
RMN $^{31}$P-{$^1$H} (CDCl$_3$) : $\delta$ = 11,8 (s, P$_0$); 65,4 (s, P$_2$); 65,9 (s, P$_1$); 25,4 (s, P=O) ppm. RMN $^1$H (CDCl$_3$) : $\delta$ = 3,26 (d large, $^3J_{HP}$ = 10,2 Hz, 54H, N-Me); 3,66 (d, $^3J_{HP}$ = 10,4 Hz, 144H, O-CH$_3$); 6,06 (dd, $^3J_{HH}$ *trans* = $^2J_{HP(O)}$ = 16,9 Hz, 24H, -CH=CH-P(O)); 6,9-7,8 (m, 210H, CH$_{arom}$, CH=N, -CH=CH-P(O)) ppm.
RMN $^{13}$C-{$^1$H} (CDCl$_3$): $\delta$ = 33,0 (d, $^2J_{CP}$ = 12,5 Hz, CH$_3$-N-P$_{1,2}$); 52,5 (d, $^2J_{CP}$= 5,3 Hz, -O-CH$_3$); 112,6 (d, $^1J_{CP}$= 192,08 Hz, -CH=CH-P(O)(OMe)$_2$); 121,4 (s large, C$_0^2$); 121,9 (s large, C$_1^2$, C$_2^2$); 128,4 (s large, C$_0^3$, C$_1^3$); 129,2 (s, C$_2^3$); 132,0 (s, C$_1^4$); 132,4 (s large, C$_0^4$, C$_2^4$); 139,2 (d, $^3J_{CP}$ = 13,6 Hz, CH=N); 148,1 (d, $^2J_{CP}$ = 5,4 Hz, -CH=CH-P(O)(OMe)$_2$); 151,2 (s, C$_0^1$); 151,3 (d, $^2J_{CP}$= 6,9 Hz, C$_1^1$); 151,8 (d, $^2J_{CP}$= 6,4 Hz, C$_2^1$) ppm.

**Exemple 10 : Synthèse du dendrimère de troisième génération à terminaisons vinyl-diméthyl-phosphonate**

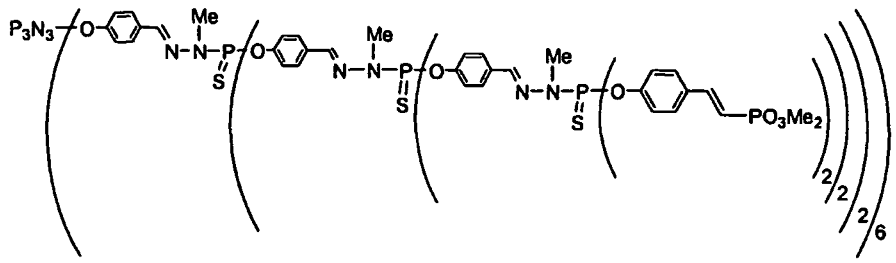

Le tétraméthyl-méthylène-gem-diphosphonate (0,71 mmol, 165 mg) ainsi que de l'hydrure de sodium (0,71 mmol, 17,1 mg) sont mis dans 5 mL de THF distillé, ce mélange est laissé sous une forte agitation pendant 2 heures à température ambiante. Une fois le dégagement d'hydrogène fini, il est additionné lentement sur le dendrimère Gc'$_3$ (1,35.10$^{-2}$ mmol, 200 mg) qui a été préalablement mis en solution dans 3 mL de THF distillé. L'addition est effectuée à 0°C puis on laisse le mélange revenir à température ambiante pendant une nuit. Après évaporation du THF, le solide blanc est lavé avec un mélange pentane / éther 1/1 de manière à retirer l'excès de tétraméthyl-méthylène-gem-diphosphonate. Ensuite, le dendrimère est mis en suspension dans le minimum d'eau, la solution trouble obtenue est centrifugée. Une poudre blanche est récupérée après centrifugation mais il peut parfois être nécessaire de répéter une deuxième fois l'opération (centrifugation) toujours avec le minimum d'eau. Le produit final est isolé avec un rendement de 72%. RMN $^{31}$P-{$^1$H} ($CDCl_3$) : $\delta$ = 11,7 (s, $P_0$); 65,3 (s, $P_3$); 66,0 (s, $P_{1,2}$); 25,5 (s, P=O) ppm. RMN $^1$H ($CDCl_3$) : $\delta$ = 3,29 (s large, 126H, N-Me); 3,68 (d, $^3J_{HP}$ = 7,7 Hz, 288H, O-$CH_3$); 6,08 (dd, $^3J_{HH\ trans}$ = $^2J_{HP(O)}$ = 17,6 Hz, 48H, -CH=CH-P(O)); 6,9-7,8 (m, 450H, $CH_{arom}$, CH=N, -CH=CH-P(O)) ppm.
RMN $^{13}$C-{$^1$H} ($CDCl_3$) : $\delta$ = 33,0 (d, $^2J_{CP}$ = 13,1 Hz, $CH_3$-N-$P_{1,2,3}$); 52,5 (d, $^2J_{CP}$ = 5,5 Hz, - O-$CH_3$); 112,6 (d, $^1J_{CP}$= 192,2 Hz, -CH=CH-P(O)(OMe)$_2$); 121,9 (d large, $^3J_{CP}$ = 2,7 Hz, $C_0^2$, $C_1^2$, $C_2^2$, $C_3^2$); 128,3 (s large, $C_0^3$, $C_1^3$, $C_2^3$); 129,1 (s, $C_3^3$); 131,9 (s large, $C_0^4$, $C_2^4$); 132,1 (s, $C_1^4$); 132,2 (s, $C_3^4$); 139,2 (d, $^3J_{CP}$ = 13,2 Hz, CH=N); 148,3 (s large, -CH=CH-P(O)(OMe)$_2$); 151,3 (s, $C_1^1$, $C_0^1$); 151,8 (s, $C_3^1$); 152,0 (s, $C_2^1$) ppm.

**Exemple 11 : Synthèse 4-hydroxybenzyl-diméthyl-phosphonate**

Les étapes a) à d) de cette synthèse multi-étapes étaient déjà décrites par J.D. Olsjewski et al J. Org. Chem. 1994, 59, 4285-4296.

*a) Synthèse du 4-tertiobutyldiméthylsiloxy-benzaldéhyde*

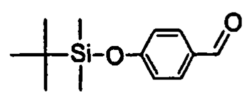

Le 4-hydroxy-benzaldéhyde (10 g, 0,082 mol) est mis en solution dans 100 mL de dichlorométhane. On additionne à cette solution à température ambiante le chlorotriméthylsilane (11,72 g, 0,078 mol) ainsi que de la N,N-diméthylaminopyridine (1g, 0,008 mol) et de la triéthylamine (23 mL, 0,164 mol). L'ensemble est laissé à température ambiante 48 heures avec une agitation magnétique puis le solvant est évaporé sous pression réduite. Le solide obtenu est agité dans le pentane pur (3x200 mL), et on extrait ainsi le produit silylé.

*b) Synthèse du 4-tertiobutyldiméthylsiloxy-benzyl alcool*

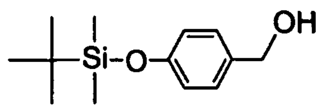

Le 4-tertiobutyldiméthylsiloxy-benzaldéhyde (28 g, 0,118 mol) est mis en solution dans un mélange THF/éthanol (50 mL/10 mL). On additionne sur cette solution à température ambiante du borohydrure de sodium (9 g, 0,237 mol) cette suspension est agitée sous argon et à température ambiante pendant 4 jours. Puis on évapore sous pression

réduite tous les solvants du milieu réactionnel et on obtient ainsi un gel blanc très compact. Ce dernier est mis en suspension dans de l'éther puis on ajoute très lentement une solution de chlorhydrate d'ammonium saturée, jusqu'à obtention d'une solution plus homogène dans les deux phases. Lorsque les deux phases sont homogènes on sépare le produit final par simple décantation eau/éther. La phase éthérée est évaporée puis le produit obtenu est repris dans le pentane et lavé une fois à l'eau.

*c) Synthèse du 4-tertiobutyldiméthylsiloxy-benzyl trifluoroacétate*

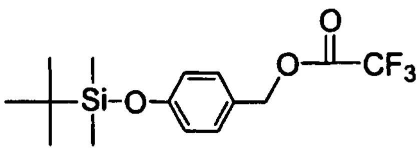

L'alcool benzylique obtenu en b) (27 g, 0,113 mol) est mis en solution dans du THF (100 mL); sur cette solution on additionne l'anhydride trifluoroacétique (19,2 mL, 0,136 mol) à température ambiante. Puis l'ensemble est mis au reflux du THF pendant une heure. On laisse ensuite le mélange revenir lentement à température ambiante, et on évapore 75 % du THF puis on reprend l'ensemble dans de l'éther et on lave dans un premier temps avec une solution d'hydrogénocarbonate de sodium (2x100 mL) et une fois à l'eau (100 mL).

*d) Synthèse du 4-tertiobutyldiméthylsiloxy-benzyl bromure*

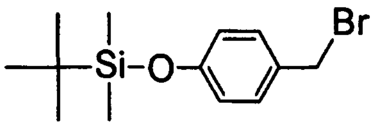

Le trifluoroacétate obtenu en c) (35 g, 0,105 mol) est mis en solution dans du THF (100 mL), sur cette solution on additionne du bromure de lithium (11 g, 0,126 mol) l'ensemble est mis au reflux du THF pendant 18 heures. On évapore le THF sous pression réduite puis on reprend le produit dans 40 mL d'acétonitrile et on fait une décantation à l'hexane (4x100 mL). L'hexane est évaporé et on obtient une huile blanchâtre contenant des cristaux blancs. Il faut alors récupérer l'huile en utilisant à nouveau l'hexane mais en filtrant ces cristaux. Le produit est isolé avec un rendement de 84 %.

*e) Synthèse du 4-tertiobutyldiméthylsiloxy-benzyl-diméthyl-phosphonate*

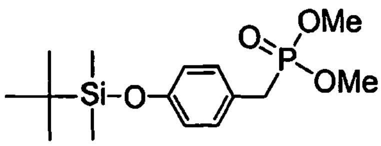

Le bromure de benzyle du 4-tertiobutyldiméthylsiloxane obtenu en d) (2,72 mmol, 800 mg), est additionné au triméthylphosphite (4 mmol, 0,47 mL). Le triméthylphosphite est ajouté en plusieurs fois : dans un premier temps le premier équivalent est ajouté (0,32 mL) puis l'ensemble est porté à 80°C avec agitation et sans solvant. La réaction libère du bromure de méthyle qui doit être éliminé sous pression réduite pour permettre à la réaction d'être totale. Après 4 heures de reflux l'excès de triméthylphosphite est additionné (0,15 mL). Le mélange réactionnel est à nouveau porté à 80°C pendant 2 heures. Le mélange final contient des traces de triméthylphosphite et de méthyl-diméthylphosphite, sous-produit dû à la formation de bromure de méthyle, qui peuvent être éliminés sous pression réduite à 80°C. Le produit final est obtenu sous forme d'une huile, avec un rendement de 90%.
RMN $^{31}P$-{$^{1}H$} ($CDCl_3$) : $\delta$ = 32,6 (s, P) ppm.

RMN$^1$H ($CDCl_3$) : δ = 0,17 (s, 6H, Si-$CH_3$); 0,97 (s, 9H, Si-tBu); 3,09 (d, $^2J_{HP}$ = 21,2 Hz, 2H, -$CH_2$-); 3,63 (d, $^2J_{HP}$ = 10,7 Hz, 6H, O-Me); 6,78 (d, $^3J_{HH}$ = 8,5 Hz, 2H, $CH_{arom}$); 7,15 (dd, $^3J_{HH}$ = 8,5 Hz, $^4J_{HP}$ = 2,5 Hz, 2H, $CH_{arom}$) ppm.

*f) Synthèse du 4-hydroxy-benzyl-diméthyl-phosphonate*

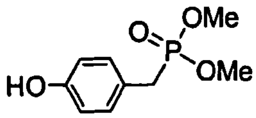

Le siloxane obtenu en e) (2,72 mmol) est placé en solution dans du THF anhydre 5 mL, puis le fluorure de tétrabutylammonium en solution anhydre à 1 M dans le THF est additionné (5,4 mmol, 5,5 mL). Le mélange est laissé 48 heures à température ambiante. Sur ce mélange réactionnel sont additionnées quelques gouttes d'eau; après 1 heure d'agitation le produit est lavé au pentane. Le produit est ensuite purifié par filtration sur silice en utilisant un gradient de solvant : éther pur dans un premier temps, puis avec un mélange Pentane/THF 1/1.

Le produit final est recristallisé dans le dichlorométhane, pour cela on le dissout dans un minimum de dichlorométhane à chaud puis on le laisse revenir très lentement à température ambiante. On refroidit à -20°C, et le produit est récupéré totalement pur, sous forme de cristaux blancs avec un rendement de 50%.
RMN $^{31}P$-{$^1H$} ($CDCl_3$) : δ = 32,4 (s, P) ppm.
RMN $^1H$ ($CDCl_3$) : δ = 3,07 (d, $^2J_{HP}$ = 22,0 Hz, 2H, -$CH_2$-); 3,68 (d, $^2J_{HP}$ = 10,8 Hz, 6H, O-Me); 6,64 (dd, $^3J_{HH}$ = 8,6 Hz, $^5J_{HP}$ = 0,78 Hz, 2H, $CH_{arom}$); 7,04 (dd, $^3J_{HH}$ = 8,6 Hz, $^4J_{HP}$ = 2,8 Hz, 2H, $CH_{arom}$); 7,68 (s large, 1H, OH) ppm.
RMN $^{13}C$-{$^1H$} ($CDCl_3$) : δ = 31,7 (d, $^1J_{CP}$ = 139,6 Hz, -$CH_2$-P); 53,1 (d, $^2J_{CP}$ = 6,9 Hz, - OMe); 116,0 (s, $C_2$); 120,8 (d, $^2J_{CP}$ = 8,17 Hz, $C_4$); 130,7 (d, $^3J_{CP}$ = 7,54 Hz, $C_3$), 156,0 (d, $^5J_{CP}$ = 3,14 Hz, $C_1$) ppm.

**Exemple 12 : Synthèse d'un dendrimère de première génération à extrémités benzyl-diméthyl-phosphonate**

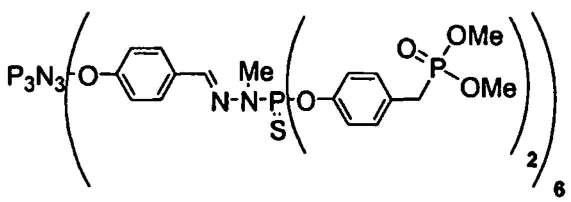

Le dendrimère de première génération à extrémités dichlorothiophosphine $Gc_1$ (0,109 mmol, 200 mg) est mis en solution dans du THF (2 mL). Sur cette solution sont additionnés du carbonate de césium (2,6 mmol, 853 mg) et le 4-hydroxy-benzyl-diméthyl-phosphonate (1,3 mmol, 282 mg). On laisse le mélange sous agitation pendant 24 heures à température ambiante. Après filtration, on place l'échantillon sous vide jusqu'à obtention d'une poudre blanche qui est lavée avec un mélange pentane / éther (1/1). Le produit final est isolé avec un rendement de 73%.
RMN $^{31}P$-{$^1H$} ($CDCl_3$) : δ = 66,2 (s, $P_1$); 31,9 (s, P(O)$(OMe)_2$); 12,3 (s, $P_0$) ppm.
RMN $^1H$ ($(CD_3)_2CO$) : δ = 3,15 (d, $^2J_{HP}$ = 21,5 Hz, 24H, $CH_2$); 3,32 (d, $^3J_{HP}$ = 10,5 Hz, 18H, $CH_3$-N-$P_1$); 3,58 (d, $^3J_{HP}$ = 10,9 Hz, 72H, P(O)-O-$CH_3$); 6,90-7,90 (m, 78H, $H_{arom}$, CH=N) ppm.
RMN $^{13}C$-{$^1H$} ($(CD_3)_2CO$) : δ = 31,85 (d, $^1J_{CP}$ = 136,4 Hz, -$\underline{CH_2}$-P(O)$(OMe)_2$); 33,4 (d, $^2J_{CP}$ = 16,8 Hz, $CH_3$-N-$P_1$); 52,8 (d, $^2J_{CP}$ = 6,8 Hz, -O-$CH_3$); 121,9 (s large, $C_0^2$ et $C_1^2$); 129,1 (s, $C_0^3$); 130,3 (d, $^2J_{CP}$ = 8,6 Hz, $C_1^4$); 131,8 (d, $^2J_{CP}$ = 6,18 Hz, $C_1^3$); 133,3 (s, $C_0^4$); 140,4 (d, $^3J_{CP}$ = 14,04 Hz, CH=N); 150,3 (d large, $^2J_{CP}$ = 3,8 Hz, $C_1^1$); 152,0 (d large, $C_0^1$) ppm.

**Exemple 13 : Synthèse d'un dendrimère de deuxième génération à extrémités benzyl-diméthyl-phosphonate**

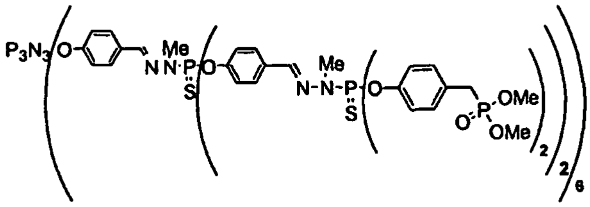

Le dendrimère de deuxième génération à extrémités dichlorothiophosphine $Gc_2$ (0,02 mmol, 100 mg) est mis en solution dans du THF (2 mL). Dans cette solution sont additionnés du carbonate de césium (1,5 mmol, 490 mg) et le 4-hydroxy-benzyl-diméthyl-phosphonate (0,53 mmol, 113 mg). On laisse le mélange sous agitation pendant 24 heures à température ambiante. Après filtration, on place l'échantillon sous vide jusqu'à obtention d'une poudre blanche qui est lavée avec un mélange pentane / éther (1/1). Le produit final est isolé avec un rendement de 78%.
RMN $^{31}P-\{^1H\}$ ($CDCl_3$) : $\delta$ = 66,0 (s, $P_2$); 65,9 (s, $P_1$); 31,8 (s, $P(O)(OMe)_2$); 11,8 (s, $P_0$) ppm. RMN $^1H$ ($(CD_3)_2CO$) : $\delta$ = 3,15 (d, $^2J_{HP}$ = 22,2 Hz, 48H, $CH_2$); 3,25 (d, $^3J_{HP}$ = 11,2 Hz, 54H, $CH_3$-N-$P_{1,2}$); 3,55 (d, $^3J_{HP}$ = 10,8 Hz, 144H, P(O)-O-$CH_3$); 6,70-7,90 (m, 186H, $H_{arom}$, CH=N) ppm.
RMN $^{13}C-\{^1H\}$ ($CDCl_3$) : $\delta$ = 32,1 (d, $^1J_{CP}$ = 138,6 Hz, -$\underline{CH_2}$-$P(O)(OMe)_2$); 33,0 (d, $^2J_{CP}$ = 12,2 Hz, $CH_3$-N-$P_{1,2}$); 52,9 (d, $^2J_{CP}$ = 6,3 Hz, -O-$CH_3$); 121,4 (s, $C_0^2$); 121,5 (s, $C_1^2$); 121,6 (d large, $^3J_{CP}$ = 3,5 Hz, $C_2^2$); 128,3 (s, $C_0^3$, $C_1^3$); 128,4 (d, $^2J_{CP}$ = 8,9 Hz, $C_2^4$); 130,8 (d, $^2J_{CP}$ = 6,5 Hz, $C_2^3$); 132,1 (s, $C_0^4$); 132,3 (s, $C_1^4$); 138,6 (d, $^3J_{CP}$ = 13,7 Hz, $\underline{CH}$=N-N(Me)-$P_2$); 139,1 (d, $^3J_{CP}$ = 12,8 Hz, $\underline{CH}$=N-N(Me)-$P_1$); 149,6 (dd, $^2J_{CP}$ = 6,1 Hz, $^5J_{CP}$ = 3,8 Hz, $C_2^1$); 151,1 (s, $C_0^1$); 151,2 (d, $^2J_{CP}$ = 7,6 Hz, $C_1^1$) ppm.

**Exemple 14 : Synthèse d'un dendrimère de troisième génération à extrémités benzyl-diméthyl-phosphonate**

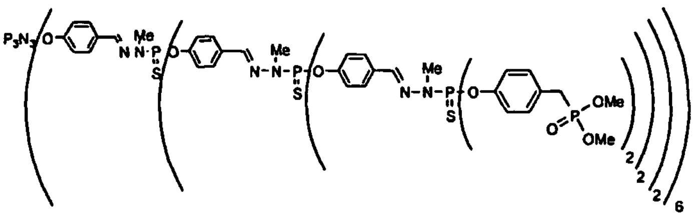

Le dendrimère de troisième génération à extrémités dichlorothiophosphine $Gc_3$ (0,014 mmol, 150 mg) est mis en solution dans du THF (2 mL). Dans cette solution sont additionnés du carbonate de césium (1,4 mmol, 460 mg) et le 4-hydroxy-benzyl-diméthyl-phosphonate (0,71 mmol, 153 mg). On laisse le mélange sous agitation pendant 24 heures à température ambiante. Après filtration, on place l'échantillon sous vide jusqu'à obtention d'une poudre blanche qui est lavée avec un mélange pentane / éther (1/1). Le produit final est isolé avec un rendement de 80%.
RMN $^{31}P-\{^1H\}$ ($CDCl_3$) : $\delta$ = 66,0 (s, $P_{1,2,3}$); 31,9 (s, $P(O)(OMe)_2$); 11,5 (s, $P_0$) ppm.
RMN $^1H$ ($CDCl_3$) : $\delta$ = 3,03 (d, $^2J_{HP}$ = 21,5 Hz, 96H, $CH_2$); 3,23 (d, $^3J_{HP}$ = 9,7 Hz, 126H, $CH_3$-N-$P_{1,2,3}$); 3,54 (d, $^3J_{HP}$ = 10,8 Hz, 288H, P(O)-O-$CH_3$); 6,70-7,90 (m, 402H, $H_{arom}$, CH=N) ppm.
RMN $^{13}C-\{^1H\}$ ($CDCl_3$) : ô = 31,8 (d, $^1J_{CP}$ = 122,5 Hz, -$\underline{CH_2}$-$P(O)(OMe)_2$); 33,1 (s, $CH_3$-N-$P_{1,2,3}$); 52,9 (d, $^2J_{CP}$ = 7,4 Hz, -O-$CH_3$); 121,2 (s, $C_1^2$); 121,5 (s, $C_3^2$); 121,7 (s large, $C_2^2$, $C_0^2$); 128,3 (s, $C_0^3$, $C_1^3$, $C_2^3$); 128,4 (d, $^2J_{CP}$ = 10,1 Hz, $C_3^4$); 130,5 (s, $C_0^4$); 130,6 (s, $C_1^4$); 130,8 (d, $^2J_{CP}$ = 6,2 Hz, $C_3^3$) 132,2 (s, $C_2^4$); 138,7 (d large, $^3J_{CP}$ = 13,6 Hz, $\underline{CH}$=N-N(Me)-$P_{1,2,3}$); 148,3 (s large, $C_0^1$); 149,6 (dd, $^2J_{CP}$ = 4,3 Hz, $^5J_{CP}$ = 4,3 Hz, $C_3^1$); 151,2 (d, $^2J_{CP}$ = 7,1 Hz, $C_1^1$, $C_2^1$) ppm.

**Exemple 15 : Synthèse d'un dendrimère de première génération à extrémités benzylphosphonique**

*Etape 1 : dendrimère à extrémités acide benzyl phosphonique*

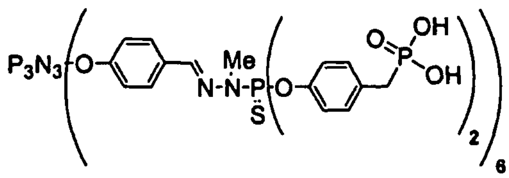

Le dendrimère de première génération à extrémités benzyl-diméthyl-phosphonate obtenu dans **l'Exemple 12** (400 mg, 0,1 mmol) est mis en solution dans de l'acétonitrile (1 mL). Le mélange est refroidi à 0°C puis on additionne lentement le bromotriméthylsilane (386 μl, 2,9 mmol) soit 1,2 équivalents de silane par extrémité méthyle. Le mélange est laissé pendant 16 heures à température ambiante. Puis on met l'échantillon sous vide pendant deux heures. Après obtention d'une poudre on additionne du méthanol anhydre (1 mL), on agite la suspension pendant 2 heures, enfin on remet le produit sous vide pendant 1 heure. La poudre obtenue est lavée plusieurs fois à l'eau et à l'éther. Le produit final est isolé avec un rendement de 70%.
RMN $^{31}P$-{$^1H$} (DMSO $d^6$) : δ = 66,1 (s, $P_1$); 25,2 (s, $P(O)(OH)_2$); 11,7 (s, $P_0$) ppm.
RMN $^1H$ (DMSO $d^6$) : δ = 2,89 (d, $^2J_{HP}$ = 20,7 Hz, 24H, $CH_2$); 3,22 (d, $^3J_{HP}$ = 10,6 Hz, 18H, $CH_3$-N-$P_1$); 4,0-5,2 (m, 24H, -$PO_3H_2$); 6,70-7,90 (m, 78H, $H_{arom}$, CH=N) ppm.
RMN $^{13}C$-{$^1H$} (DMSO $d^6$) : δ = 32,8 (d, $^2J_{CP}$ = 11,3 Hz, $CH_3$-N-P); 34,3 (d, $^1J_{CP}$ = 132,7 Hz, -$CH_2$-P(O)(OH)$_2$); 120,4 (s, $C_1^2$); 120,9 (s, $C_0^2$); 128,2 (s, $C_0^3$); 130,9 (d, $C_1^3$, $^2J_{CP}$ = 6,8 Hz); 131,1 (s, $C_1^4$); 132,1 (s, $C_0^4$); 139,8 (d large, $^3J_{CP}$ = 10,8 Hz, CH=N); 148,3 (d, $^2J_{CP}$ = 7,2 Hz, $C_1^1$); 150,4 (s, $C_0^1$) ppm.

*Etape 2 : dendrimère à extrémités sel de sodium d'acide benzyl phosphonique*

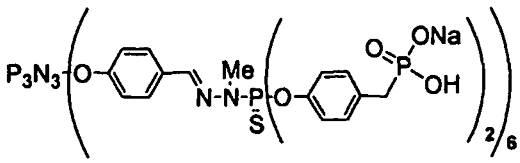

Le dendrimère obtenu à l'étape précédente est dissous dans une solution de soude titrée (0,1966 M, 12 éq.). La solution obtenue est filtrée sur filtre millipore puis lyophilisée. Le dendrimère de première génération à extrémités mono sel de sodium de l'acide benzylphosphonique est obtenu sous forme d'une poudre blanche avec un rendement de 89%. RMN $^{31}P${$^1H$} ($D_2O/CD_3CN$, 81 MHz) : δ = 12,0 (s 1, $N_3P_3$), 21,3 (s 1, P=O), 65,8 (m, P=S).

**Exemple 16 : Synthèse d'un dendrimère de seconde génération à extrémités benzylphosphonique**

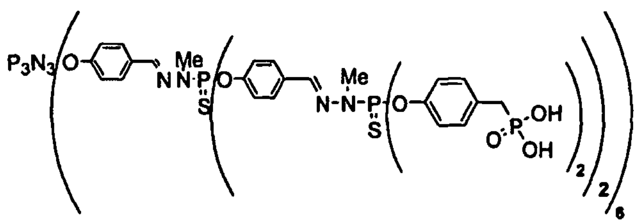

Le dendrimère de deuxième génération à extrémités benzyl-diméthyl-phosphonate obtenu dans **l'Exemple 13** (130 mg, 0,014 mmol) est mis en solution dans de l'acétonitrile (1 mL). Le mélange est refroidi à 0°C puis on additionne lentement le bromotriméthylsilane (101 μl, 0,76 mmol) soit 1,2 équivalents de silane par extrémité méthyle. Le mélange est laissé pendant 16 heures à température ambiante. Puis on met l'échantillon sous vide pendant deux heures. Après obtention d'une poudre on additionne du méthanol anhydre (1 mL), on agite la suspension pendant 2 heures, enfin on remet le produit sous vide pendant une heure. La poudre obtenue est lavée plusieurs fois à l'eau et à l'éther. Le produit final est isolé avec un rendement de 63%.
RMN $^{31}P$-{$^1H$} (DMSO d6/$D_2O$) : δ = 66,1 (s, $P_{1,2}$); 25,5 (s, $P(O)(OH)_2$); 11,9 (s, $P_0$) ppm. RMN $^1H$ (DMSO d6/$D_2O$) : δ

= 2,95 (s large, 48H, $CH_2$); 3,40-3,75 (m, 54H, $CH_3$-N-$P_{1,2}$); 6,50-7,30 (m, 186H, $H_{arom}$, CH=N) ppm.
RMN $^{13}C-\{^1H\}$ (DMSO d6/$D_2O$) : δ = 32,9 (d, $^2J_{CP}$ = 11,5 Hz, $CH_3$-N-$P_{1,2}$); 34,5 (d, $^1J_{CP}$ = 133,7 Hz, -$\underline{CH_2}$-P(O)(OH)$_2$); 119,5 (s, $C_0^2$); 120,4 (s, $C_2^2$); 121,4 (s, $C_1^2$); 128,2 (s, $C_0^3$, $C_1^3$); 131,0 (s large, $C_2^4$, $C_2^3$); 132,1 (s, $C_0^4$, $C_1^4$); 140,3 (d large, $^3J_{CP}$ = 11,1 Hz, CH=N); 148,3 (d, $^2J_{CP}$ = 3,6 Hz, $C_2^1$); 150,3 (s, $C_0^1$); 150,7 (s, $^2J_{CP}$ = 6,0 Hz, $C_1^1$) ppm.

**Exemple 17 : Synthèse d'un dendrimère de troisième génération à extrémités benzylphosphonique**

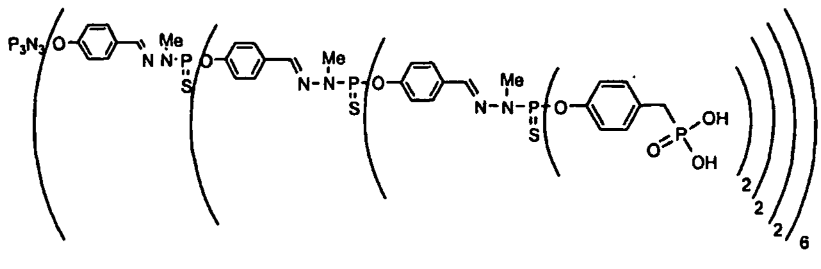

Le dendrimère de troisième génération à extrémités benzyl-diméthyl-phosphonate obtenu dans **<u>l'Exemple 14</u>** (200 mg, 0,01 mmol) est mis en solution dans de l'acétonitrile (1 mL). Le mélange est refroidi à 0°C puis on additionne lentement le bromotriméthylsilane (146 μl, 1,09 mmol) soit 1,1 équivalents de silane par extrémité méthyle. Le mélange est laissé pendant 16 heures à température ambiante. Puis, on met l'échantillon sous vide pendant deux heures. Après obtention d'une poudre on additionne du méthanol anhydre (1 mL), on agite la suspension pendant 2 heures, enfin on remet le produit sous vide pendant une heure. La poudre obtenue est lavée plusieurs fois à l'eau et à l'éther. Le produit final est isolé avec un rendement de 81%.
RMN $^{31}P-\{^1H\}$ (DMSO d6) : δ = 66,1 (s, $P_{1,2,3}$); 25,1 (s, P(O)(OH)$_2$); 11,7 (s, $P_0$) ppm. RMN $^1H$ (DMSO d6) : δ = 2,94 (d, $^2J_{HP}$ = 23,1 Hz, 96H, $CH_2$); 3,10-3,40 (m, 126H, $CH_3$-N-$P_{1,2,3}$); 5,2-6,2 (m, 96H, -$PO_3H_2$); 7,00-8,10 (m, 402H, $H_{arom}$, CH=N) ppm.
RMN $^{13}C-\{^1H)$ (DMSO d6) : δ = 32,9 (d, $^2J_{CP}$ = 12,2 Hz, $CH_3$-N-$P_{1,2,3}$); 34,5 (d, $^1J_{CP}$ = 132,1 Hz, -$\underline{CH_2}$-P(O)(OH)$_2$); 119,5 (s, $C_0^2$); 120,4 (s, $C_3^2$); 120,6 (s, $C_2^2$); 121,4 (s, $C_1^2$); 128,3 (s, $C_0^3$, $C_1^3$, $C_2^3$); 131,0 (s large, $C_3^4$, $C_3^3$); 132,1 (s large, $C_0^4$, $C_1^4$, $C_2^4$); 140,3 (d large, $^3J_{CP}$ = 10,4 Hz, CH=N); 148,3 (d, $^2J_{CP}$ = 6,2 Hz, $C_31$); 150,6 (d large, $^2J_{CP}$ = 7,2 Hz, $C_0^1$, $C_1^1$, $C_2^1$) ppm.

**Exemple 18 : Synthèse d'un dendrimère génération zéro (coeur $P_3N_3$) à extrémités α-hydroxy-diméthylphosphonate**

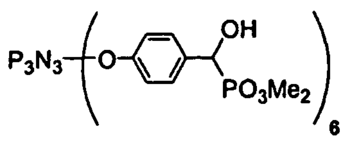

A 301 mg de $P_3N_3(OC_6H_4\text{-}CHO)_6$ (0,35 mmol) en solution dans 1 ml de THF on additionne de la triéthylamine distillée (5 μl soit 0,42.10$^{-3}$ mol), et du diméthylphosphite (196 μL soit 2,1.10$^{-3}$ mol) (1 équiv par -CHO). On laisse le mélange pendant 12 heures sous agitation. La pâte obtenue est ensuite lavée avec un mélange THF/$Et_2O$ : 1/1, pour donner une poudre blanche. Le produit final est isolé avec un rendement de 78%.
RMN $^{31}P-\{^1H\}$ (DMSO d6) : δ = 11,5 (s, $P_0$), 27,2 (s, P(O)(O-$CH_3$)$_2$) ppm.

**Exemple 19 : Synthèse d'un dendrimère génération zéro à extrémités acide α-hydroxy-phosphonique**

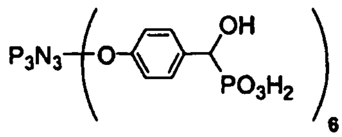

Le dendrimère de génération zéro de **l'Exemple 18** (4,78.$10^{-2}$ mmol, 72 mg) à extrémités α-hydroxy-diméthyl-phosphonate est mis en suspension dans l'acétonitrile (4 mL) avec de la triéthylamine (0,288 mmol, 10,25 μL) à 0°C. Puis le bromure de triméthylsilane (0,86 mmol, 115 μL) est additionné lentement à 0°C, l'ensemble revient lentement à température ambiante pendant 6 heures, puis on ajoute du méthanol anhydre (1 mL). Après 2 heures d'agitation le mélange réactionnel est séché sous pression réduite. Ensuite la poudre est mise en suspension dans un minimum d'eau pendant 30 minutes avec une forte agitation. Après filtration, le produit est séché puis lavé abondamment à l'éther. De préférence, pour obtenir un dendrimère soluble, le dendrimère final ne doit pas être totalement désolvaté. Le produit final est isolé avec un rendement de 60%.
RMN $^{31}P-\{^1H\}$ (DMSO d6) : δ = 11,3 (s, $P_0$), 22,0 (m, $P(O)(OH)_2$) ppm.
IR: Absence de υ(CHO) à 1670 $cm^{-1}$; u(OH) à 3271 $cm^{-1}$.

**Exemple 20 : Synthèse d'un dendrimère de génération zéro à extrémités benzyl-diméthyl-phosphonate**

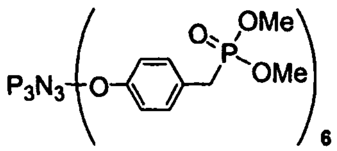

A une solution d'hexachlorocyclotriphosphazène (0,109 mmol, 38 mg) dans 2 mL de THF sont ajoutés du carbonate de césium (1,3 mmol, 427 mg) et du 4-hydroxy-benzyl-diméthyl-phosphonate (0,65 mmol, 141 mg). On laisse le mélange sous agitation pendant 24 heures à température ambiante. Après filtration, on place l'échantillon sous vide jusqu'à obtention d'une poudre blanche qui est lavée avec un mélange pentane / éther (1/1). Le produit final est isolé avec un rendement de 61%.
RMN $^{31}P-\{^1H\}$ ($CDCl_3$) : δ = 12,2 (s, $P_0$); 31,8 (s, $P(O)(OMe)_2$) ppm.

**Exemple 21 : Synthèse d'un dendrimère de génération zéro à extrémités benzylphosphonique**

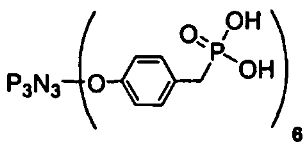

Le dendrimère de génération zéro à extrémités benzyl-diméthyl-phosphonate de **l'Exemple 20** (126 mg, 0,1 mmol) est mis en solution dans de l'acétonitrile (1 mL). Le mélange est refroidi à 0°C puis on additionne lentement le bromotriméthylsilane (198 μl, 1,45 mmol) soit 1,2 équivalents de silane par extrémité méthyle. Le mélange est laissé pendant 16 heures à température ambiante, puis on met l'échantillon sous vide pendant deux heures. Après obtention d'une poudre on additionne du méthanol anhydre (1 mL), on agite la suspension pendant 2 heures, enfin on remet le produit sous vide pendant 1 heure. La poudre obtenue est lavée plusieurs fois à l'eau et à l'éther. Le produit final est isolé avec un rendement de 79%.
RMN $^{31}P-\{^1H\}$ (DMSO $d^6$) : δ = 11,8 (s, $P_0$); 25,1 (m, $P(O)(OH)_2$) ppm.

**Exemple 22 : Synthèse du (4-hydroxy-2-nitrophénylamino)méthyl-diméthylphosphonate**

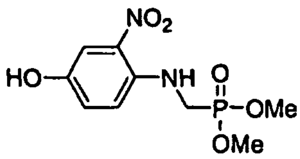

A température ambiante sont mélangés 500 mg de 2-nitro-4-hydroxy-aniline, 1 mL de formaldéhyde en solution à 37% dans l'eau et 1,2 mL de diméthylphosphite. La solution rouge résultante est agitée à température ambiante pendant 96 heures. Le résidu brut est directement purifié par chromatographie sur gel de silice (éluant: diéthyl éther puis acétate d'éthyle). Le résidu rouge obtenu après chromatographie sur colonne est dilué dans 300 mL d'acétate d'éthyle puis lavé avec 50 mL d'eau. La phase organique est ensuite séchée sur sulfate de magnésium puis évaporée à sec pour donner le produit attendu sous forme d'une poudre rouge avec un rendement de 71%.
RMN $^{31}P-\{^1H\}$ (Acétone) : $\delta$ = 28,7 (s, $P(O)(OMe)_2$) ppm.
RMN $^1H$ (Acétone) : $\delta$ = 3,8 (d, $^3J_{HP}$ = 10,8 Hz, 6H, -OMe); 3,90 (d, $^2J_{HP}$ = 12,6 Hz, 2H, N-$\underline{CH_2}$-P); 5,9 (s large, 1 H, -NH); 7,0-8,0 (m, 3H, $CH_{arom}$) ppm.
RMN $^{13}C-\{^1H\}$ (Acétone) : $\delta$ = 38,5 (d, $^1J_{CP}$ = 154,7 Hz, $CH_2$); 52,9 (d, $^2J_{CP}$ = 6,0 Hz, OMe); 110,1 (s, $C_{arom}$); 116,5 (s, $C_{arom}$); 126,8 (s, $C_{arom}$); 132,6 (s, $C_{arom}$); 139,8 (s, $C_{arom}$); 148,3 (s, $C_{arom}$) ppm.
$[M+Na]^+$ = 299,2 $g.mol^{-1}$.

**Exemple 23 : Synthèse d'un dendrimère de première génération à coeur cyclotriphosphazène et à surface 2-nitrophénylaminométhyl-diméthylphosphonate**

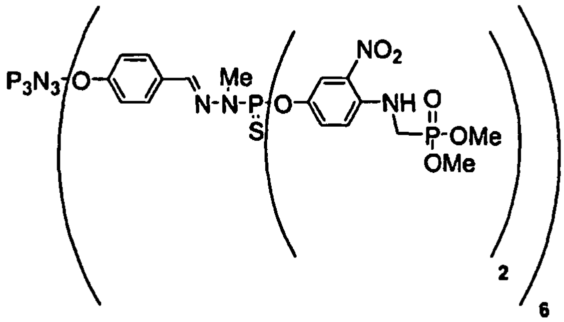

A une suspension de NaH (7 mg) dans le THF est additionnée à température ambiante 90 mg de phénol (4-hydroxy-2-nitrophénylamino)méthyl-diméthyl- phosphonate de **l'Exemple 22.** Après une heure d'agitation, 30 mg de dendrimère de $Gc_1$ en solution dans 5 mL de THF anhydre sont additionnés. On laisse le mélange sous agitation pendant 24 heures à température ambiante puis on filtre sur célite et on centrifuge le mélange final de manière à séparer les sels. Enfin le produit final est lavé par précipitation dans du pentane et isolé avec un rendement de 82 %.
RMN $^{31}P-\{^1H\}$ ($CDCl_3$) : $\delta$ = 66,1 (s, $P_1$); 28,6 (s, $P(O)(OMe)_2$); 11,9 (s, $P_0$) ppm.
RMN $^1H$ ($CDC1_3$) : $\delta$ = 3,6 (d, $^3J_{HP}$ = 14,6 Hz, 18H, $CH_3$-N-$P_1$); 3,78 (d, $^3J_{HP}$ = 10,7 Hz, 72H, -P(O)(O-$\underline{CH_3}$)$_2$); 3,9 (d, $^2J_{HP}$ = 12,6 Hz, 24H, -$\underline{CH_2}$-$P(O)(OCH_3)_2$); 7,0-8,1 (m, 66H, $CH_{arom}$, CH=N) ppm.

**Exemple 24 : Synthèse d'un dendrimère à coeur cyclotriphosphazene de première génération à surface aza-mono-phosphonique dérivé de l'allylamine**

*Etape 1 : Allylimine en surface de dendrimères phosphorés*

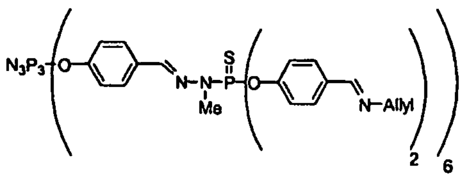

A une solution de Gc'1 (230 mg, 80,5 μmol) dans le $CH_2Cl_2$ (10 mL) sont ajoutés l'allylamine (400 μL, 66 éq., 5,33 mmol) et quelques grammes de $MgSO_4$. La suspension est agitée 24 heures à température ambiante puis diluée avec 10 mL de $CH_2Cl_2$ et filtrée. Le solide est rincé avec 10 mL de $CH_2Cl_2$ et la solution est évaporée sous pression réduite. Le résidu visqueux est lavé à l'éther puis avec un mélange THF/pentane et à l'acétonitrile. Le solide obtenu est solubilisé dans le $CH_2Cl_2$, la solution est ensuite filtrée et concentrée sous pression réduite pour donner le dendrimère à surface N-allylamine sous forme d'une poudre jaune pâle avec un rendement de 75 %.
RMN $^{31}P\{^1H\}$ ($CDCl_3$, 81,01 MHz) : δ = 11,7 (s, $N_3P_3$); 65,3 (s, P=S).
$^1H$ ($CDCl_3$, 200,13 MHz) : δ = 3,25 (d, 18H, $^3J_{HP}$ = 10,5 Hz, $NCH_3$); 4,19 (d, 24H, $^3J_{HH}$ = 5,6 Hz, $CH_2CH$=); 5,21 (m, 24H, $CH_2$=); 6,02 (m, 12H, CH=); 6,98 (d, 12H, $^3J_{HH}$ = 8,5 Hz, $C_0^2$-H); 7,21 (m, 24H, $C_1^2$-H); 7,60 (m, 42H, $C_0^3$-H, $C_1^3$-H et CH=NN); 8,17 (sl, 12H, CH=N).

*Etape 2 : Dendrimère phosphoré de première génération à extrémités monophosphonate*

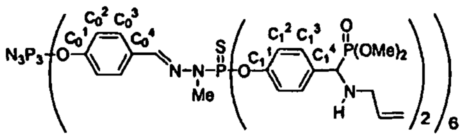

Le phosphite de diméthyle (1 mL, 10,9 mmol) en large excès (205 éq.) est additionné sur le dendrimère à terminaisons N-allylimine (200 mg, 60,4 μmol) sans solvant. La solution obtenue est agitée 72 heures à température ambiante puis diluée avec 2 mL de THF. Le dendrimère est précipité par ajout d'un grand volume de pentane et le solide obtenu est lavé 2 fois avec un mélange THF / pentane puis 3 fois à l'éther puis séché pour donner une poudre blanche avec un rendement de 70 %.
RMN $^{31}P\{^1H\}$ ($CDCl_3$, 81,01 MHz) : δ = 11,5 (s, $N_3P_3$); 28,9 (s, P=O); 65,6 (s, P=S).
RMN $^1H$ ($CDCl_3$, 250,13 MHz) : δ = 2,90-3,01 (m, 12H, CHHN); 3,10-3,20 (m, 12H, CHHN); 3,30 (d, 18H, $^3J_{HP}$ = 10,1 Hz, $NCH_3$); 3,68 (d, 36H, $^3J_{HP}$ = 10,5 Hz, POMe); 3,78 (d, 36H, $^3J_{HP}$ = 11,9 Hz, POMe); 4,06 (d, 12H, $^2J_{HP}$ = 19,8 Hz, PCH); 5,08 (m, 24H, $CH_2$=); 5,78 (m, 12H, CH=); 7,04 (d, 12H, $^3J_{HH}$ = 8,3 Hz, $C_0^2$-H); 7,19 (d, 24H, $^3J_{HH}$ = 7,9 Hz, $C_1^2$-H); 7,35 (d, 24H, $^3J_{HH}$ = 6,9 Hz, $C_1^3$-H); 7,61 (m, 18H, $C_0^3$-H, CH=N).

*Etape 3 : Dendrimère phosphoré de première génération à extrémités acide monophosphonique (sel de Na)*

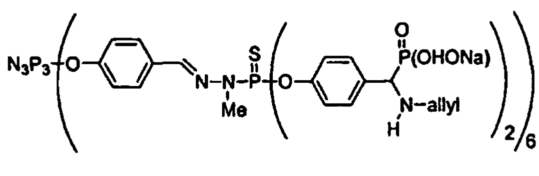

A une solution de dendrimère à terminaisons phosphonate de diméthyle (220 mg, 46,9 μmol) dans l'acétonitrile est additionné à 0°C sous flux d'argon 13 équivalents d'HCl 1M dans l'éther. La solution hétérogène est agitée 2 heures à température ambiante puis concentrée sous pression réduite. Le résidu est mis en suspension dans l'acétonitrile distillé et 30 équivalents de bromo-triméthylsilane sont ajoutés sous argon à 0°C. La solution est agitée 20 heures à température ambiante puis concentrée sous pression réduite. 5 mL de méthanol sont ajoutés et le mélange est vigoureusement agité pendant 2 heures. Le méthanol est éliminé sous pression réduite et le résidu est lavé avec de l'éther distillé, puis de l'eau et du méthanol. Le solide est séché sous pression réduite jusqu'à obtention d'une poudre. Une solution de soude (0,1966 M, 12 éq.) est additionnée lentement sur le solide. La solution obtenue est filtrée puis lyophilisée. Le dendrimère à terminaisons acide amino phosphonique est obtenu sous forme d'une poudre blanche avec un rendement de 65 %.

RMN $^{31}P\{^1H\}$ ($D_2O/CD_3CN$, 81,01 MHz) : 12,1 (s, $N_3P_3$), 14,5 (s, P=O), 66,7 (s, P=S).

*Etape 3bis: le même composé peut être obtenu selon la procédure alternative suivante :*

A une solution de dendrimère à terminaisons N-allylphosphonate de diméthyle obtenu à l'étape 2 (110 mg, 23,5 μmol) dans un mélange $CH_3CN/CH_2Cl_2$ (5/5 mL) est additionné à 0°C sous flux d'argon 36 équivalents de $N(Et)_3$ (118 μL, 0,846 mmol) puis 90 équivalents (280 μL, 2,11 mmol) de bromotriméthylsilane. La solution est agitée 20 heures à température ambiante puis concentrée sous pression réduite. 3 mL de méthanol sont ajoutés et le mélange est vigoureusement agité pendant 2 heures. Le méthanol est éliminé sous pression réduite et le résidu est lavé avec de l'éther distillé, puis de l'eau et du méthanol. Le solide est séché sous pression réduite jusqu'à obtention d'une poudre. Une solution de soude (0,1966 M, 12 éq.) est additionnée lentement sur le solide. La solution obtenue est filtrée puis lyophilisée. Le dendrimère à terminaisons acide amino phosphonique est obtenu sous forme d'une poudre blanche.

**Exemple 25 : Synthèse d'un dendrimère à coeur cyclotriphosphazène de première génération à surface aza-mono-phosphonique dérivé de la benzylamine**

*Etape 1 : Benzylimine en surface de dendrimères phosphorés*

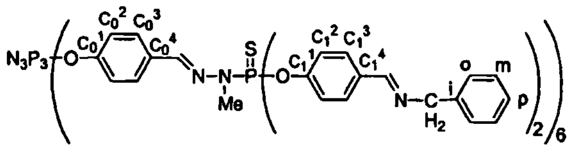

A une solution de Gc'1 (310 mg, 108 μmol) dans le $CH_2Cl_2$ (15 mL) sont ajoutés la benzylamine (450 μL, 38 éq, 4,1 mmol) et 4 grammes de $MgSO_4$. La suspension est agitée 24 heures à température ambiante puis diluée avec 10 mL de $CH_2Cl_2$ et filtrée. Le solide est rincé avec 10 mL de $CH_2Cl_2$ et la solution est évaporée sous pression réduite. Le résidu visqueux est lavé à l'éther puis avec un mélange THF/pentane et à l'acétonitrile. Le solide obtenu est solubilisé dans le $CH_2Cl_2$, puis la solution résultante est filtrée et concentrée sous pression réduite pour donner un solide jaune pâle avec un rendement de 92 %.
RMN $^{31}P\{^1H\}$ ($CDCl_3$, 81,01 MHz) : $\delta$ = 11,8 (s, $N_3P_3$); 65,3 (s, P=S).
RMN $^1H$ ($CDCl_3$, 200,13 MHz) : $\delta$ = 3,20 (d, 18H, $^3J_{HP}$ = 10,4 Hz, $NCH_3$); 4,75 (s, 24H, $\underline{CH_2}Ph$); 6,98 (d, 12H, $^3J_{HH}$ = 8,6 Hz, $C_0^2$-H); 7,30 (m, 84H, $C_1^2$-H et $C_6H_5$); 7,64 (s, 6H, CH=NN); 7,65 (d, 12H, $^3J_{HH}$ = 8,8 Hz, $C_0^3$-H); 7,68 (d, 24H, $^3J_{HH}$ = 8,6 Hz, $C_1^3$-H); 8,27 (s, 12H, CH=N).
RMN $^{13}C\{^1H\}$ ($CDCl_3$, 50,3 MHz) : $\delta$ = 32,9 (d, $^2J_{CP}$ = 12,1 Hz, NMe); 64,0 (s, $NCH_2$); 121,5 (d, $^2J_{CP}$ = 4,5 Hz, $C_0^2$ et $C_1^2$); 127,1 (s, $C_p$); 127,9 (s, $C_m$); 128,3 (s, $C_0^3$); 128,5 (s, $C_o$); 129,6 (s, $C_1^3$); 132,0 (s, $C_0^4$); 133,6 (s, $C_1^4$); 138, 8 (sl, CH=N-N); 139,1 (s, $C_i$); 151,2 (sl, $C_0^1$); 152,3 (d, $^2J_{CP}$ = 7,5 Hz, $C_1^1$); 160,5 (s, CH=N).

*Etape 2 : dendrimère à surface benzylamino mono-phosphonate de diméthyle*

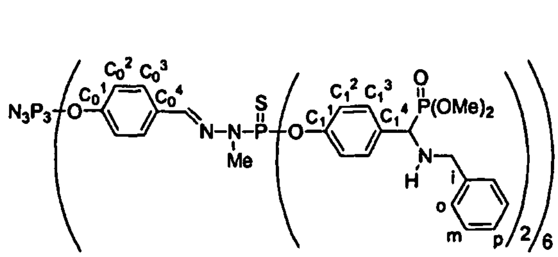

Le phosphite de diméthyle (1 mL, 10,9 mmol) en large excès (195 éq) est ensuite additionné sur le dendrimère à terminaisons N-benzylimine décrit ci-dessus (210 mg, 53 μmol) sans solvant. La solution obtenue est agitée 72 heures à température ambiante puis diluée avec 2 mL de THF. Le dendrimère est précipité par ajout d'un grand volume de pentane et le solide obtenu est lavé 2 fois avec un mélange THF / pentane puis 3 fois à l'éther. Après séchage sous pression réduite, un solide blanc est obtenu avec un rendement de 61 %.
RMN $^{31}P\{^1H\}$ ($CDCl_3$, 81,01 MHz) : $\delta$ = 11,5 (s, $N_3P_3$); 28,7 (s, P=O); 65,6 (s, P=S).

RMN $^1H$ ($CDCl_3$, 200,13 MHz) : δ = 2,17 (sl, 12H, NH); 3,30 (d, 18H, $^3J_{HP}$ = 10,2 Hz, NMe); 3,46 (d, 12H, $^2J_{HH}$ = 13,6 Hz, NCHH); 3,47 (d, 36H, $^3J_{HP}$ = 10,6 Hz, POMe); 3,65 (d, 36H, $^3J_{HP}$ = 10,6 Hz, POMe); 3,71 (d, 12H, $^2J_{HH}$ = 13,6 Hz, NCHH); 3,98 (d, 12H, $^2J_{HP}$ = 20,8 Hz, PCH); 7,04 (d, 12H, $^3J_{HH}$ = 8,5 Hz, $C_0^2$-H); 7,25 (m, 84H, $C_1^2$-H et $C_6H_5$); 7,31 (d, 24H, $^3J_{HH}$ = 6,8 Hz, $C_1^3$-H); 7,60 (s, 6H, CH=N); 7,61 (d, 12H, $^3J_{HH}$ = 8,5 Hz, $C_0^3$-H).
RMN $^{13}C\{^1H\}$ ($CDCl_3$, 62,89 MHz) : δ = 32,9 (d, $^2J_{CP}$ = 12,3 Hz, NMe); 51,1 (d, $^3J_{CP}$ = 17,0 Hz, $NCH_2$); 53,5 (d, $^2J_{CP}$ = 6,9 Hz, POMe); 53,8 (d, $^2J_{CP}$ = 7,2 Hz, POMe); 58,5 (d, $^1J_{CP}$ = 154,2 Hz, PCH); 121,2 (sl, $C_0^2$); 121,4 (sl, $C_1^2$); 127,2 (s, $C_p$); 128,3 (s, $C_0^3$ et $C_m$); 128,4 (s, $C_o$); 129,8 (d, $^3J_{CP}$ = 5,5 Hz, $C_1^3$); 132,1 (s, $C_0^4$); 132,6 (sl, $C_1^4$); 138,9 (sl, CH=N et $C_i$); 150,4 (d,$^2J_{CP}$ = 5,9 Hz, $C_1^1$); 151,3 (sl, $C_0^1$).

*Etape 2bis : le même composé peut être obtenu selon la procédure alternative décrite ci-dessous*

A une solution de dendrimère Gc1 (233 mg, 0,127 mmol) à terminaisons $S=PCl_2$ dans le THF ou l'acétonitrile sont ajoutés 12,2 équivalents (500 mg, 1,55 mmol) de phénol fonctionnalisé synthétisé à l'étape 1 de **l'Exemple 50** (solubilisé dans l'acétonitrile ou le THF) et 15 équivalents (623 mg, 1,91 mmol) de $Cs_2CO_3$. La suspension résultante est agitée jusqu'à substitution complète des chlores (suivi RMN $^{31}P$ et $^1H$). Le mélange est décanté, le surnageant est collecté et le solide résiduel est lavé au THF. Les surnageants sont réunis et centrifugés. La solution limpide obtenue est concentrée sous pression réduite. Le résidu est dissous dans un minimum de THF puis précipité au pentane et finalement purifié par lavage (THF / pentane et THF / $Et_2O$) pour donner un solide blanc avec un rendement de 92 %. Les caractéristiques de ce produit sont données ci-dessus, dans l'étape 2.

*Etape 3 : dendrimère phosphoré de première génération à surface mono acide phosphonique (sel de Na)*

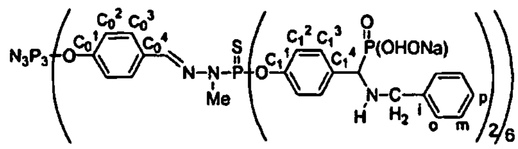

A une solution de dendrimères à terminaisons phosphonate de diméthyle obtenu dans l'étape 2 ou 2bis ci-dessus (220 mg, 41,9 µmol) dans l'acétonitrile est additionné à 0°C sous flux d'argon 13 équivalents d'HCl 1M dans l'éther (0,545 mL, 0,54 mmol). La solution hétérogène est agitée 2 heures à température ambiante puis concentrée sous pression réduite. Le résidu est mis en suspension dans l'acétonitrile distillé et 30 équivalents (82 µL, 0,628 mmol) de bromotriméthylsilane sont ajoutés sous argon à 0°C. La solution est agitée 20 heures à température ambiante puis concentrée sous pression réduite. 5 mL de méthanol sont ajoutés et le mélange est vigoureusement agité pendant 2 heures. Le méthanol est éliminé sous pression réduite et le résidu est lavé avec de l'éther distillé, puis de l'eau et du méthanol. Le solide est séché sous pression réduite jusqu'à obtention d'une poudre. Une solution de soude (0,1966 M, 2,55 mL, 12 éq.) est additionnée lentement sur le solide. La solution obtenue est filtrée puis lyophilisée.Le dendrimère à terminaisons acide amino phosphonique est obtenu sous forme d'une poudre blanche avec un rendement de 65 %. RMN $^{31}P\{^1H\}$ ($D_2O/CD_3CN$, 81,01 MHz) : 12,3 (s, $N_3P_3$), 14,6 (s, P=O), 66,6 (s, P=S). RMN $^1H$ ($D_2O/CD_3CN$, 200,13 MHz) : 3,39-3,65 (m, 30H, $CH_2Ph$ et $NCH_3$); 3,86 (m, 12H, $CH_2Ph$); 4,03 (d, 12H, $^2J_HP$ = 17 Hz, PCH); 6,91 (m, 12H, $C_0^2$-H); 7,25 (m, 24H, $C_1^2$-H); 7,38 (m, 60H, $C_6H_5$); 7,57 (m, 30H, $C_1^3$-H et CH=N), 7,97 (sl, 12H, $C_0^3$-H).

*Etape 3bis : le même composé peut être obtenu selon la procédure alternative décrite ci-dessous*

A une solution de dendrimère à terminaisons phosphonate de diméthyle obtenu à l'étape 2 ou 2bis ci-dessus (110 mg, 20,9 µmol) dans un mélange $CH_3CN/CH_2Cl_2$ (5/5 mL) est additionné à 0°C sous flux d'argon 36 équivalents de $N(Et)_3$ (106 µL, 0,755 mmol) puis 90 équivalents (250 µL, 1,89 mmol) de bromo-triméthylsilane. La solution est agitée 20 heures à température ambiante puis concentrée sous pression réduite. 3 mL de méthanol sont ajoutés et le mélange est vigoureusement agité pendant 2 heures. Le méthanol est éliminé sous pression réduite et le résidu est lavé avec de l'éther distillé, puis de l'eau et du méthanol. Le solide est séché sous pression réduite jusqu'à obtention d'une poudre. Une solution de soude (0,1966 M, 1,28 mL, 12 éq.) est additionnée lentement sur le solide. La solution obtenue est filtrée puis lyophilisée. Le dendrimère à terminaisons acide amino phosphonique est obtenu sous forme d'une poudre blanche. Ses caractéristiques sont données dans l'étape 3 ci-dessus.

*Etape 3ter : le même composé peut être obtenu selon une autre procédure alternative décrite ci-dessous*

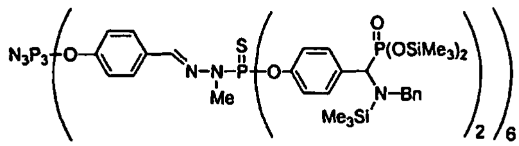

A une solution de dendrimère à terminaisons imine obtenu dans l'étape 1 ci-dessus (200 mg, 50,9 μmol) en solution dans 5 mL de dichlorométhane ou de chloroforme anhydre sont ajoutés 20 équivalents (360 μL, 1,019 mmol) de $P(OSiMe_3)_3$ et 12 équivalent de ClTMS (80 μL, 0,61 mmol). La solution est agitée à température ambiante ou chauffée à 50°C jusqu'à disparition complète de l'imine (24 à 96 heures, suivi en RMN $^{31}P$ et $^{1}H$). Le produit silylé n'est pas isolé mais caractérisé en RMN $^{31}P$
RMN $^{31}P\{^1H\}$ ($CDCl_3$, 81,01 MHz) : 9,2 (s, $O=P(OSiMe_3)_2$), 11,4 (s, $N_3P_3$), 66,2 (s, P=S).

Le mélange réactionnel est ensuite concentré sous pression réduite puis 5 mL de méthanol sont ajoutés et la suspension est agitée vigoureusement pendant 2 heures. Le méthanol est évaporé et le résidu est lavé à l'eau distillée (jusqu'à obtention d'eaux de lavage à pH = 6 environ). Le solide est lavé avec 3 fois 5 mL d'éther distillé puis séché sous vide. Au solide est additionnée une solution de soude (0,1966 M, 3,11 mL, 12 éq.), la solution obtenue est filtrée puis lyophilisée. Le dendrimère à terminaisons acide amino-phosphonique est obtenu sous forme de poudre blanche avec un rendement de 87 %. Ses caractéristiques sont données dans l'étape 3 ci-dessus.

**Exemple 26 : Synthèse d'un dendrimère à coeur cyclotriphosphazène de première génération à surface aza-mono-phosphonique dérivé de la méthylamine**

*Etape 1 : Méthylimine en surface d'un dendrimère phosphoré de première génération*

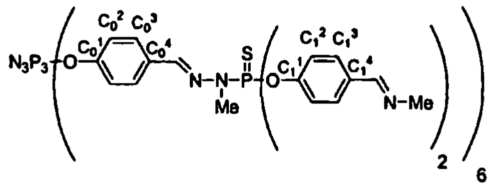

A une solution de Gc'1 (145 mg, 50,7 μmol) dans le THF (10 mL) sont ajoutés la méthylamine (8 M dans l'éthanol, 110 μL, 17,4 éq, 0,88 mmol) et 2 grammes de $MgSO_4$. La suspension est agitée 24 heures à température ambiante puis filtrée. Le solide est rincé avec 10 mL de THF et la solution est évaporée sous pression réduite. Le résidu visqueux est lavé à l'éther puis avec un mélange THF/pentane. Le solide obtenu est solubilisé dans le $CH_2Cl_2$, la solution résultante est ensuite filtrée et concentrée sous pression réduite pour donner un solide blanc avec un rendement de 100 %.
RMN $^{31}P\{^1H\}$ ($CDCl_3$, 81,01 MHz) : $\delta$ = 11,7 (s, $N_3P_3$); 65,2 (s, P=S).
RMN $^1H$ ($CDCl_3$, 200,13 MHz) : $\delta$ = 3,24 (d, 18H, $^3J_{HP}$ = 10,4 Hz, $NNCH_3$); 3,44 (s1, 36H, NMe); 6,97 (d, 12H, $^3J_{HH}$ = 8,5 Hz, $C_0^2$-H); 7,21 (d, 24H, $^3J_{HH}$ = 8,5 Hz, $C_1^2$-H); 7,58 (m, 42H, $C_0^3$-H, $C_1^3$-H et CH=NN); 8,16 (sl, 12H, CH=NMe).
RMN $^{13}C\{^1H\}$ ($CDCl_3$, 62,89 MHz) : $\delta$ = 32,9 (d, $^2J_{CP}$ = 13,0 Hz, $NNCH_3$); 48,2 (s, $NCH_3$); 121,5 (s, $C_0^2$); 121,6 (d, $^4J_{CP}$ = 3,8 Hz, $C_1^2$); 128,3 (s, $C_0^3$); 129,2 (s, $C_1^3$); 131,9 (s, $C_0^4$); 133,6 (s, $C_1^4$); 139,0 (m, CH=NN); 151,3 (sl, $C_0^1$); 152,1 (sl, $C_1^1$); 161,0 (s, CH=NMe).

*Etape 2 : dendrimère phosphoré de première génération à extrémités silylphosphonate*

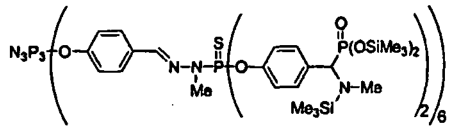

A une solution de dendrimère à terminaisons imine (217 mg, 72,0 µmol) en solution dans 5 mL de dichlorométhane ou de chloroforme anhydre sont ajoutés 20 équivalents (481 µL, 1,44 mmol) de $P(OSiMe_3)_3$. La solution est agitée à température ambiante ou chauffée à 50°C jusqu'à disparition complète de l'imine (24 à 96 heures, suivi en RMN $^{31}P$ et $^{1}H$). Ce produit n'est pas isolé, mais utilisé directement dans l'étape suivante
RMN $^{31}P\{^{1}H\}$ ($C_6D_6$, 81,01 MHz) : 9,0 (s, $O=P(OSiMe_3)_2$), 11,8 (s, $N_3P_3$), 66,4 (s, P=S).

*Etape 3 : Dendrimère phosphoré de première génération à extrémités acide monophosphonique (sel de Na)*

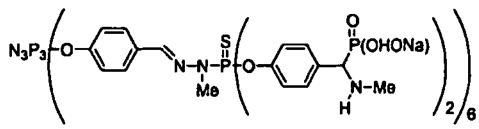

Le mélange réactionnel obtenu dans l'étape 2 ci-dessus est concentré sous pression réduite puis 5 mL de méthanol sont ajoutés et la suspension est agitée vigoureusement pendant 2 heures. Le méthanol est évaporé et le résidu est lavé à l'eau distillée (jusqu'à obtention d'eaux de lavage à pH 6 environ). Le solide est lavé avec 3 fois 5 mL d'éther distillé puis séché sous vide.

Au solide est additionnée une solution de soude (0,1966 M, 4,39 mL, 12 éq), la solution obtenue est filtrée puis lyophilisée. Le dendrimère à terminaisons acide amino phosphonique est obtenu sous forme de poudre blanche avec un rendement de 92 %.
RMN $^{31}P\{^{1}H\}$($D_2O/CD_3CN$, 81,01 MHz) : $\delta$ = 8,9 (s, P=O); 10,5 (s, $N_3P_3$); 65,1 (s, P=S).

Le même produit peut être obtenu par la méthode alternative suivante :

*Etape 2bis : Dendrimère phosphoré de première génération à extrémités méthylphosphonate*

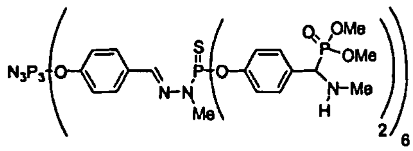

Le dendrimère à terminaisons imine obtenu dans l'étape 1 est solubilisé dans un large excès de diméthyl phosphite. La solution homogène est agitée 72 heures à TA puis 10 mL d'éther distillé sont ajoutés. Après décantation, le solvant est éliminé et le solide est lavé avec 3 fois 10 mL d'éther. Le résidu est solubilisé dans un minimum de THF puis précipité par ajout de pentane. Le solide obtenu est séché sous pression réduite et le dendrimère à terminaison N-(méthyl)-méthylphosphonate de diméthyle est obtenu pur avec un rendement de 80 % (poudre blanche).
RMN $^{31}P$-$\{^{1}H\}$ ($CDCl_3$) : $\delta$ = 11,5 (s); 29,0 (s); 65,2 (s).

*Etape 3bis : Dendrimère phosphoré de première génération à extrémités acide monophosphonique (sel de Na)*

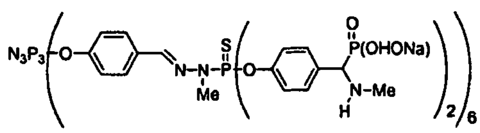

A une solution de 160 mg (37 μmol) de dendrimère obtenu dans l'étape 2bis dans l'acétonitrile (5 mL) sont ajoutés lentement à 0°C 46 équivalents de BrTMS (230 μL). La solution est alors agitée 20 heures à température ambiante. Le solvant est évaporé sous pression réduite et le résidu est traité par 10 mL de méthanol. Après 1 heure de vigoureuse agitation dans le méthanol, le solide est séché sous pression réduite. L'acide phosphonique est lavé avec 2 fois 20 mL d'éther distillé. Le solvant est éliminé et le dendrimère à extrémités acide phosphonique pur est traité lentement par une solution aqueuse de soude 0,1955 M (2,3 mL). La solution homogène est lyophilisée et le dendrimère à terminaison N-(méthyl)-acide méthylphosphonique (mono sel de sodium) est isolé avec un rendement quantitatif sous forme d'une poudre blanche.
RMN $^{31}P$-{$^1H$} ($CD_3CN/D_2O$): δ = 8.9 (s); 10,5 (s); 65,1 (sl).

**Exemple 27 : Synthèse d'un dendrimère à coeur cyclotriphosphazène de génération 0 à surface mono-phosphonique dérivé de la benzylamine**

*Etape 1 : dendrimère de génération 0 à surface monophosphonate*

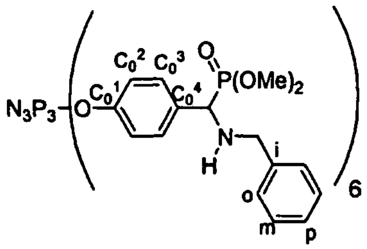

A une solution d'hexachloro-cyclotriphosphazène ($N_3P_3Cl_6$, 175 mg, 0.502 mmol) dans l'acétonitrile distillé (10 mL) sont ajoutés le carbonate de césium (1,3 g, 3,99 mmol) et le phénol amino-phosphonate de diméthyle (1 g, 3.11 mmol). La suspension est agitée à température ambiante durant 24 heures. Après décantation le surnageant est canulé, les sels sont lavés par 10 mL de THF et les phases organiques réunies sont centrifugées. Les surnageant sont collectés et concentrés sous pression réduite. Le résidu est précipité plusieurs fois dans un mélange THF / pentane puis le solide est lavé à l'éther et séché sous pression réduite. Le produit est obtenu sous forme d'une poudre blanche avec un rendement de 87 %. RMN $^{31}P\{^1H\}$ ($CDCl_3$, 81,01 MHz) : δ = 11,3 (s, $N_3P_3$); 28,9 (s, P=O).
RMN $^1H$ ($CDCl_3$, 200,13 MHz) : δ = 2,28 (s1, 6H, NH); 3,43 (d, 6H, $^2J_{HH}$ = 13 Hz, CHHPh); 3,48 (d, 18H, $^3J_{HP}$ = 10,2 Hz, POMe); 3,67 (d, 18H, $^3J_{HP}$ = 9,2 Hz, POMe); 3,73 (d, 6H, $^2J_{HH}$ = 13 Hz, CHHPh); 3,98 (d, 6H, $^2J_{HP}$ = 19,9 Hz, PCH); 7,2-7,50 (m, 54H, $H_{arom}$).
RMN $^{13}C\{^1H\}$ ($CDCl_3$, 50,3 MHz) : δ = 50,9 (d, $^3J_{CP}$ = 17,2 Hz, $CH_2Ph$); 53,3 (d, $^2J_{CP}$ = 6,5 Hz, POMe); 53,7(d, $^2J_{CP}$ = 7,0 Hz, POMe); 56,4 (d, $^1J_{CP}$ = 154,5 Hz, PCH); 120,9 (s, $C_0^2$); 127,2 (s, $C_p$); 128,2 (s, $C_m$); 128,4 (s, $C_o$); 129,8 (d, $^3J_{CP}$ = 5,9 Hz, $C_0^3$); 132,5 (d, $^2J_{CP}$ = 3,9 Hz, $C_0^4$); 138,9 (s, $C_i$); 150,3 (sl, $C_0^1$).

*Etape 2 : dendrimère de génération 0 à surface acide phosphonique (sel de Na)*

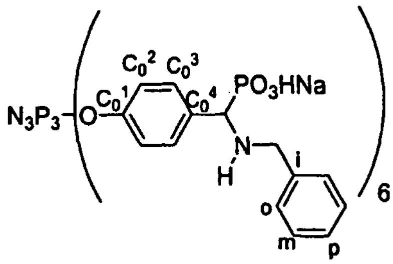

A une solution de dendrimères à terminaisons phosphonate de diméthyle obtenu à l'étape précédente (200 mg, 1 éq) dans un mélange $CH_3CN/CH_2Cl_2$ (5/5 mL) est additionné à 0°C sous flux d'argon 18 équivalents de $N(Et)_3$ puis 45 équivalents de bromo-triméthylsilane. La solution est agitée 20 heures à température ambiante puis concentrée sous pression réduite. 3 mL de méthanol sont ajoutés et le mélange est vigoureusement agité pendant 2 heures. Le

méthanol est éliminé sous pression réduite et le résidu est lavé avec de l'éther distillé, puis de l'eau et du méthanol. Le solide est séché sous pression réduite jusqu'à obtention d'une poudre. Une solution de soude (0,1966 M, 6 éq.) est additionnée lentement sur le solide. La solution obtenue est filtrée puis lyophilisée. Le dendrimère à terminaisons acide amino phosphonique est obtenu sous forme d'une poudre blanche.
RMN $^{31}P\{^1H\}$ ($D_2O/CD_3CN$, 81,01 MHz) : 12,1 (s, $N_3P_3$), 14,3 (s, P=O).

*Etape 2 bis : le même composé peut être obtenu selon la procédure alternative suivante :*

A une solution de dendrimères à terminaisons phosphonate de diméthyle synthétisé à l'étape 1 (200 mg, 1 eq) dans l'acétonitrile (5 mL) sont additionnés à 0°C sous flux d'argon 7 équivalents d'HCl 1M dans l'éther. La solution hétérogène est agitée 2 heures à température ambiante puis concentrée sous pression réduite. Le résidu est mis en suspension dans l'acétonitrile distillé et 15 équivalents de bromotriméthylsilane sont ajoutés sous argon à 0°C. La solution est agitée 20 heures à température ambiante puis concentrée sous pression réduite. 5 mL de méthanol sont ajoutés et le mélange est vigoureusement agité pendant 2 heures. Le méthanol est éliminé sous pression réduite et le résidu est lavé avec de l'éther distillé, puis de l'eau et du méthanol. Le solide est séché sous pression réduite jusqu'à obtention d'une poudre. Une solution de soude (0,1966 M, 6 éq.) est additionnée lentement sur le solide. La solution obtenue est filtrée puis lyophilisée. Le dendrimère à terminaisons acide amino phosphonique est obtenu sous forme d'une poudre blanche avec un rendement de 63 %.

**Exemple 28 : Synthèse du dendrimère de première génération à surface tétraisopropyl-gem-diphosphonate**

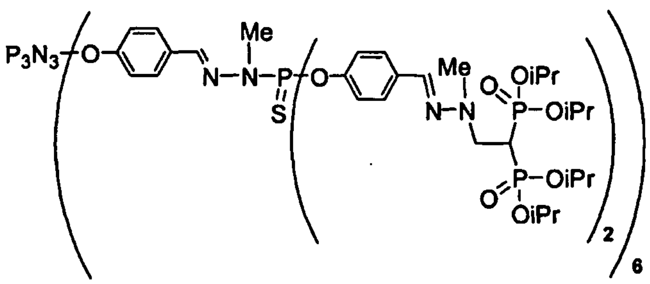

Le dendrimère Gc'$_1$ (7,0.10$^{-2}$ mmol, 200 mg) est mis en solution dans $CH_2Cl_2$ (10 mL) puis la monométhylhydrazine est additionnée à 0°C (1,3 mmol, 66 µL) ainsi que le tétraisopropyl-vinyl-gem-diphosphonate (0,7 g sachant qu'il est pur seulement à 65 %) ; cette addition doit être simultanée et lente pour éviter la formation d'un agrégat insoluble. L'addition terminée, le mélange est agité à température ambiante pendant 24 heures. L'évaporation du solvant sous pression réduite, suivie de 3 lavages avec 50 mL de pentane pur, permettent d'éliminer tous les sous produits de la réaction ainsi que les impuretés contenues dans le tétraisopropyl-vinyl-gem-diphosphonate de départ. Le produit final est isolé avec un rendement de 70%.
RMN $^{31}P$-$\{^1H\}$ ($CDCl_3$) : $\delta$ = 66,5 (s, $P_1$); 23,5 (s, $P(O)(OiPr)_2$); 11,9 (s, $P_0$) ppm.
RMN $^1H$ ($CDCl_3$) : $\delta$ = 1,29 (d, $^3J_{HH}$ = 6,1 Hz, 288H, -O-CH-($\underline{CH_3}$)$_2$); 2,91 (s, 36H, N-N($\underline{CH_3}$)-$CH_2$-); 3,00 (tt, $^2J_{HP}$ = 23,6 Hz, $^3J_{HH}$ = 6,0 Hz, 12H, -$\underline{CH}$-(P(O)(OiPr)$_2$)$_2$); 3,23 (d, $^3J_{HP}$ = 8,8 Hz, 18H, $CH_3$-N-$P_1$); 3,78 (td, $^3J_{HP}$ = 14,7 Hz, $^3J_{HH}$ = 6,2 Hz, 24H, -$\underline{CH_2}$-CH-(P(O)(OiPr)$_2$)$_2$); 4,74 (hept, $^3J_{HH}$ = 6,1 Hz, 48H, -O-$\underline{CH}$-($CH_3$)$_2$); 6,8-7,8 (m, 90H, $CH_{arom}$ et CH=N) ppm.
RMN $^{13}C$-$\{^1H\}$ ($CDCl_3$) : $\delta$ = 23,7 (d, $^3J_{CP}$= 3,1 Hz, -O-CH-($\underline{CH_3}$)$_2$); 23,8 (d, $^3J_{CP}$= 2,5 Hz, - O-CH-($\underline{CH_3}$)$_2$); 24,0 (d, $^3J_{CP}$ = 3,0 Hz, -O-CH-($\underline{CH_3}$)$_2$); 24,2 (d, $^3J_{CP}$= 2,9 Hz, -O-CH-($\underline{CH_3}$)$_2$); 32,9 (d, $^2J_{CP}$ = 11,7 Hz, $CH_3$-N-$P_1$), 37,7 (t, $^1J_{CP}$ = 132,2 Hz, -$\underline{CH}$-(P(O)(OiPr)$_2$)$_2$); 38,8 (s, N-N($\underline{CH_3}$)-$CH_2$-); 55,1 (s, -$\underline{CH_2}$-CH-(P(O)(OiPr)$_2$)$_2$); 70,9 (d, $^2J_{CP}$ = 6,6 Hz, -O-$\underline{CH}$-($CH_3$)$_2$); 71,3 (d, $^2J_{CP}$ = 6,8 Hz, -O-$\underline{CH}$-($CH_3$)$_2$); 121,2 (s large, $C_0^2$, $C_1^2$); 126,3 (s, $C_1^3$); 128,2 (s, $C_0^3$); 129,4 (s, $\underline{CH}$=N-N(Me)-$CH_2$); 132,1 (s, $C_0^4$), 134,5 (s, $C_1^4$), 138,8 (s large, $\underline{CH}$=N-N(Me)-$P_1$), 149,4 (d, $^2J_{CP}$= 7,5 Hz, $C_1^1$), 151,1 (s, $C_0^1$) ppm.

**Exemple 29 : Synthèse du dendrimère de seconde génération à surface tétraisopropyl-gem-diphosphonate**

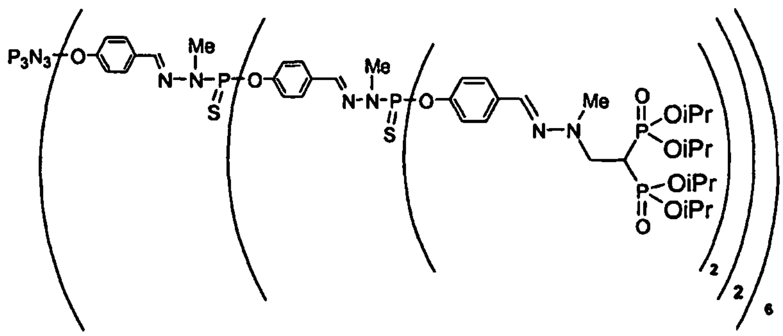

Le dendrimère Gc'$_2$ (2,9.10$^{-2}$ mmol, 200 mg) est mis en solution dans $CH_2Cl_2$ (10 mL) puis la monométhylhydrazine est additionnée à 0°C (1,05 mmol, 56 µL) ainsi que le tétraisopropyl-vinyl-gem-diphosphonate (0,575 g sachant qu'il est pur seulement à 65 %) ; cette addition doit être simultanée et lente pour éviter la formation d'un agrégat insoluble. L'addition terminée, le mélange est agité à température ambiante pendant 24 heures. L'évaporation du solvant sous pression réduite, suivie de 3 lavages avec 100 mL de pentane / éther 1/1, permettent d'éliminer tous les sous produits de la réaction ainsi que les impuretés contenues dans le tétraisopropyl-vinyl-gem-diphosphonate de départ. Le produit final est isolé avec un rendement de 79%.

RMN $^{31}P-\{^1H\}$ ($CDCl_3$) : δ = 66,4 (s, $P_2$); 66,1 (s, $P_1$); 23,5 (s, P(O)(OiPr)$_2$); 11,8 (s, $P_0$) ppm. RMN $^1H$ ($CDCl_3$) : δ = 1,25 (d, $^3J_{HH}$ = 6,0 Hz, 576H, -O-CH-(CH$_3$)$_2$); 2,87 (s, 72H, N-N(CH$_3$)-CH$_2$-); 2,97 (tt, $^2J_{HP}$ = 23,6 Hz, $^3J_{HH}$ = 6,6 Hz, 24H, -CH-(P(O)(OiPr)$_2$)$_2$); 3,24 (d large, $^3J_{HP}$ = 9,5 Hz, 54H, CH$_3$-N-P$_{1,2}$); 3,74 (td, $^3J_{HP}$ = 13,9 Hz, $^3J_{HH}$ = 6,2 Hz, 48H, -CH$_2$-CH-(P(O)(OiPr)$_2$)$_2$); 4,71 (hept, $^3J_{HH}$ = 5,9 Hz, 96H, -O-CH-(CH$_3$)$_2$); 6,8-7,8 (m, 210H, CH$_{arom}$ et CH=N) ppm. RMN $^{13}C-\{^1H\}$ ($CDCl_3$) : δ = 23,8 (d, $^3J_{CP}$ = 3,1 Hz, -O-CH-(CH$_3$)$_2$); 24,2 (d, $^3J_{CP}$ = 3,0 Hz, - O-CH-(CH$_3$)$_2$); 32,9 (d, $^2J_{CP}$ = 11,8 Hz, CH$_3$-N-P$_{1,2}$), 38,3 (t, $^1J_{CP}$ = 132,1 Hz, -CH-(P(O)(OiPr)$_2$)$_2$); 38,8 (s, N-N(CH$_3$)-CH$_2$-); 55,2 (s, -CH$_2$-CH-(P(O)(OiPr)$_2$)$_2$); 70,9 (d, $^2J_{CP}$ = 6,8 Hz, -O-CH-(CH$_3$)$_2$); 71,3 (d, $^2J_{CP}$ = 6,8 Hz, -O-CH-(CH$_3$)$_2$); 121,4 (s large, $C_0^2$, $C_1^2$, $C_2^2$); 126,4 (s, $C_2^3$); 128,3 (s large, $C_0^3$, $C_1^3$); 129,5 (s, CH=N-N(Me)-CH$_2$); 132,1 (s, $C_0^4$), 132,4 (s, $C_1^4$), 134,5 (s, $C_2^4$), 138,7 (s large, CH=N-N(Me)-P$_{1,2}$), 149,5 (d, $^2J_{CP}$ = 7,4 Hz, $C_2^1$), 151,2 (s, $C_0^1$,$C_1^1$)ppm.

**Exemple 30 : Synthèse du dendrimère de troisième génération à surface tétraisopropyl-gem-diphosphonate**

Le dendrimère Gc'$_3$ (1,35.10$^{-2}$ mmol, 200 mg) est mis en solution dans $CH_2Cl_2$ (10 mL) puis la monométhylhydrazine est additionnée à 0°C (0,97 mmol, 52 µL) ainsi que le tétraisopropyl-vinyl-gem-diphosphonate (0,532 g sachant qu'il est pur seulement à 65 %) ; cette addition doit être simultanée et lente pour éviter la formation d'un agrégat insoluble. L'addition terminée, le mélange est agité à température ambiante pendant 24 heures. L'évaporation du solvant sous pression réduite, suivie de 3 lavages avec 100 mL de pentane / éther 1/1, permettent d'éliminer tous les sous produits de la réaction ainsi que les impuretés contenues dans le tétraisopropyl-vinyl-gem-diphosphonate de départ. Le produit final est isolé avec un rendement de 80%.

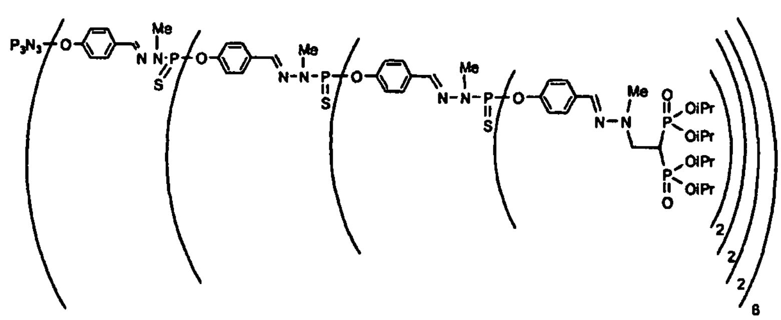

RMN $^{31}P-\{^1H\}$ ($CDCl_3$) : δ = 66,3 (s, $P_3$); 66,0 (s, $P_{1,2}$); 23,5 (s, P(O)(OiPr)$_2$); 11,4 (s, $P_0$) ppm.
RMN $^1H$ ($CDCl_3$) : δ = 1,26 (d, $^3J_{HH}$ = 6,0 Hz, 1152H, -O-CH-(CH$_3$)$_2$); 2,88 (s, 144H, N-N(CH$_3$)-CH$_2$-); 2,98 (tt, $^2J_{HP}$ = 23,9 Hz, $^3J_{HH}$ = 6,6 Hz, 48H, -CH-(P(O)(OiPr)$_2$)$_2$); 3,26 (d large, $^3J_{HP}$ = 9,5 Hz, 126H, CH$_3$-N-P$_{1,2,3}$); 3,74 (m, 96H, -CH$_2$-CH-(P(O)(OiPr)$_2$)$_2$); 4,70 (hept, $^3J_{HH}$ = 5,9 Hz, 192H, -O-CH-(CH$_3$)$_2$); 6,8-7,8 (m, 450H, CH$_{arom}$ et CH=N) ppm. RMN $^{13}C-\{^1H\}$ ($CDCl_3$) : δ = 23,9 (d, $^3J_{CP}$ = 2,9 Hz, -O-CH-(CH$_3$)$_2$); 24,2 (d, $^3J_{CP}$= 2,5 Hz, - O-CH-(CH$_3$)$_2$); 32,9 (d, $^2J_{CP}$ = 12,3 Hz, CH$_3$-N-P$_{1,2,3}$), 38,4 (t, $^1J_{CP}$ = 132,3 Hz, -CH-(P(O)(OiPr)$_2$)$_2$); 38,9 (s, N-N(CH$_3$)-CH$_2$-); 55,2 (s, -CH$_2$-CH-(P(O)(OiPr)$_2$)$_2$); 70,9 (d, $^2J_{CP}$ = 6,9 Hz, -O-CH-(CH$_3$)$_2$); 71,3 (d, $^2J_{CP}$ = 6,9 Hz, -O-CH-(CH$_3$)$_2$); 121,4 (s, $C_3^2$); 121,8 (s large, $C_0^2$, $C_1^2$, $C_2^2$); 126,4 (s, $C_3^3$); 128,3 (s large, $C_0^3$, $C_1^3$, $C_2^3$); 129,6 (s, CH=N-N(Me)-CH$_2$); 131,3 (s, $C_0^4$); 132,4

(s large, $C_1^4$, $C_2^4$); 134,5 (s, $C_3^4$); 138,7 (s large, CH=N-N(Me)-$P_{1,2,3}$); 149,5 (d, $^2J_{CP}$= 8,1 Hz, $C_3^1$); 151,2 (s large, $C_0^1$, $C_1^1$, $C_2^1$) ppm.

**Exemple 31 : Synthèse du phénol aza-bis-diméthyl-phosphonate dérivé de la tyramine**

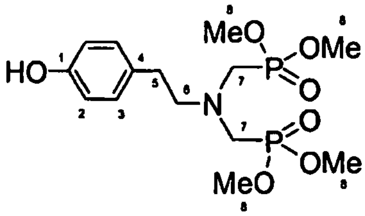

On mélange à 0°C la tyramine (6 g, 43,7 mmol) et le diméthyl-phosphite (10,32 ml, 112,5 mmol), puis on additionne lentement toujours à 0°C une solution de formaldéhyde à 37% dans l'eau (12,6 ml). L'ensemble est mis à température ambiante pendant 30 minutes et à reflux 1 heure avec une agitation magnétique. Enfin le brut réactionnel est mis sous pression réduite de manière à évaporer l'excès de formaldéhyde. Le produit est extrait avec un mélange chloroforme/eau (4/1) (3x100 ml de chloroforme). La phase organique est récupérée puis chromatographiée sur silice en utilisant l'acétone comme éluant. Le produit final est isolé avec un rendement de 65%.
RMN $^{31}P$-{$^1H$} ($CDCl_3$) : δ = 30,2 (s, $P(O)(OMe)_2$) ppm.
RMN $^1H$ ($CDCl_3$) : δ = 2,68 (t déformé, $^3J_{HH}$ = 7,2 Hz, 2H, -$CH_2$-$CH_2$-N); 3,05 (t déformé, $^3J_{HH}$ = 7,2 Hz, 2H, -$CH_2$-$CH_2$-N-); 3,20 (d, $^2J_{HP}$ = 8,9 Hz, 4H, N-$CH_2$-P); 3,75 (d, $^3J_{HP}$ = 10,7 Hz, 12H, -OMe); 6,6-7,1 (m, 4H, $CH_{arom}$); 8,16 (s large, 1H, -OH) ppm.
RMN $^{13}C$-{$^1H$} ($CDCl_3$) : δ = 32,7 (s, $C_5$); 49,4 (dd, $^3J_{CP}$ = 6,8 Hz, $^1J_{CP}$ = 158,5 Hz, $C_7$); 52,8 (d, $^2J_{CP}$ = 3 Hz, $C_8$); 58,8 (t, $^3J_{CP}$ = 7,5 Hz, $C_6$); 115,4 (s, $C_3$); 129,8 (s, $C_2$); 129,8 (s, $C_4$); 155,9 (s, $C_1$) ppm.

**Exemple 32: Synthèse du phénol aza-bis-diméthyl-phosphonate dérivé de la 4-hydroxyaniline**

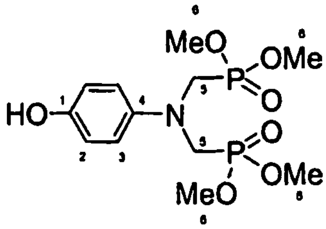

On mélange à 0°C la 4-hydroxyaniline (5g, 46 mmol) et le diméthyl-phosphite (10,5 ml, 115 mmol), puis on additionne lentement toujours à 0°C une solution de formaldéhyde à 37% dans l'eau (10,6 ml). L'ensemble est mis à température ambiante pendant 30 minutes et à reflux 1 heure avec une agitation magnétique. Enfin le brut réactionnel est mis sous pression réduite de manière à évaporer l'excès de formaldéhyde. Le produit est extrait avec un mélange chloroforme/eau (4/1) (3x100 ml de chloroforme). La phase organique est récupérée puis chromatographiée sur silice en utilisant l'acétone comme éluant. Le produit final est isolé avec un rendement de 30%.
RMN $^{31}P$-{$^1H$} ($CDCl_3$) : δ = 29,8 (s, $P(O)(OMe)_2$) ppm.
RMN $^1H$ ($CDCl_3$) : δ = 3,67 (d, $^3J_{HP}$ = 10,6 Hz, 12H, -OMe); 3,84 (d, $^2J_{HP}$ = 5,7 Hz, 4H, N-$CH_2$-P); 6,6-6,9 (m, $CH_{arom}$, 4H); 8,05 (s large, 1H, -OH) ppm.
RMN $^{13}C$-{$^1H$} ($CDCl_3$) : δ = 47,6 (d, $^1J_{CP}$ = 157,1 Hz, $C_5$); 52,6 (d, $^2J_{CP}$ = 3,8 Hz, $C_6$); 52,7 (d, $^2J_{CP}$ = 3,3 Hz, $C_6$); 115,8 (s, $C_3$); 117,3 (s, $C_2$); 141,0 (s, $C_4$); 150,9 (s, $C_1$) ppm.

**Exemple 33 : Synthèse du dendrimère de première génération à surface aza-bis-diméthyl-phosphonate dérivé de la tyramine:**

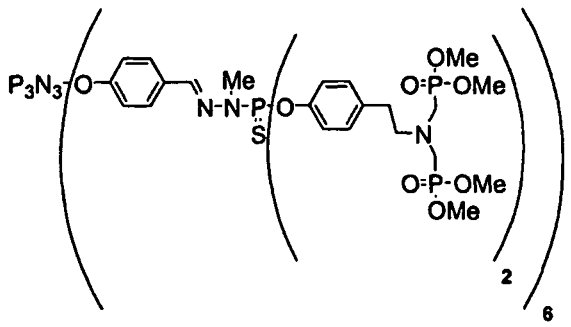

A une solution de dendrimère de $Gc_1$ (0,273 mmol, 500 mg) en solution dans du THF anhydre (10 mL) est additionné du carbonate de césium (6, 898 mmol, 2,25 g) puis le phénol aza-bis-diméthyl-phosphonate dérivé de la tyramine obtenu dans **l'Exemple 31** est ajouté (3,449 mmol, 1,31 g). On laisse le mélange sous agitation pendant 24 heures à température ambiante puis on filtre sur célite le mélange final de manière à séparer les sels. Enfin le produit final est lavé par précipitation dans du pentane et isolé avec un rendement de 70%.
RMN $^{31}P-\{^1H\}$ ($CDCl_3$) : $\delta$ = 68,7 (s, $P_1$); 31,9 (s, $P(O)(OMe)_2$); 13,7 (s, $P_0$) ppm.
RMN $^1H$ ($CDCl_3$) : $\delta$ = 2,69 (t, $^3J_{HH}$ = 6,8 Hz, 24H, $\underline{CH_2}$-$CH_2$-N); 2,99 (t, $^3J_{HH}$ = 6,8 Hz, 24H, $CH_2$-$\underline{CH_2}$-N); 3,13 (d, $^2J_{HP}$ = 9,17 Hz, 48H, -$\underline{CH_2}$-$P(O)(OCH_3)_2$); 3,2 (d, $^3J_{HP}$= 11,8 Hz, 18H, $CH_3$-N-$P_1$); 3,67 (d, $^3J_{HP}$ = 10,2 Hz, 144H, -P(O)(O-$\underline{CH_3}$)$_2$); 6,8-7,8 (m, 78H, $CH_{arom}$, CH=N) ppm.
RMN $^{13}C-\{^1H\}$ ($CDCl_3$) : $\delta$ = 32,9 (d, $^2J_{CP}$ = 11 Hz, $CH_3$-N-$P_1$); 32,95 (s, $\underline{CH_2}$-$CH_2$-N) 49,5 (dd, $^1J_{CP}$ = 157,5 Hz, $^3J_{CP}$ = 6,8 Hz -$\underline{CH_2}$-$P(O)(OCH_3)_2$); 52,6 (d, $^2J_{CP}$ = 4,0 Hz, -P(O)(O-$\underline{CH_3}$)$_2$); 57,8 (t, $^3J_{CP}$ = 7,2 Hz, $CH_2$-$\underline{CH_2}$-N); 120,8 (s, $C_0^2$); 120,8 (d, $^3J_{CP}$ = 4,1 Hz, $C_1^2$); 128,3 (s, $C_0^3$); 129,6 (s, $C_1^3$); 131,9 (s, $C_0^4$); 136,3 (s, $C_1^4$); 138,4 (d, $^3J_{CP}$= 14,1 Hz, CH=N); 148,5 (d, $^2J_{CP}$ = 7,0 Hz, $C_1^1$); 150,8 (d, $^2J_{CP}$ = 3,0 Hz, $C_0^1$) ppm.

**Exemple 34: Synthèse du dendrimère de deuxième génération à surface aza-bis-diméthyl-phosphonate dérivé de la tyramine**

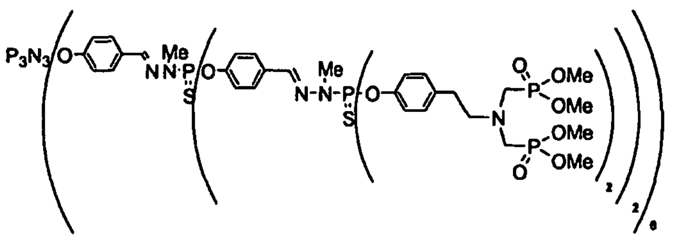

A une solution de dendrimère $Gc_2$ (0,104 mmol, 500 mg) en solution dans du THF anhydre (10 mL) est additionné du carbonate de césium (5,28 mmol, 1,72 g) puis le phénol aza-bis-diméthyl-phosphonate dérivé de la tyramine obtenu dans l'**Exemple 31** est ajouté (2,6 mmol, 1,00 g). On laisse le mélange sous agitation pendant 24 heures à température ambiante puis on filtre le mélange final sur célite de manière à séparer les sels. Enfin le produit final est lavé par précipitation dans du pentane et isolé avec un rendement de 78%.
RMN $^{31}P-\{^1H\}$ ($CDCl_3$) : $\delta$ = 66,5 (s, $P_2$); 66,2 (s, $P_1$); 30,1 (s, $P(O)(OMe)_2$), 12,1 (s, $P_0$) ppm. RMN $^1H$ ($CDCl_3$) : $\delta$ = 2,69 (s large, 48H, $\underline{CH_2}$-$CH_2$-N); 2,99 (s large, 48H, $CH_2$-$\underline{CH_2}$-N); 3,12 (d, $^2J_{HP}$ = 9,51 Hz, 96H, -$\underline{CH_2}$-$P(O)(OCH_3)_2$); 3,24 (d, $^3J_{HP}$ = 8,5 Hz, 54H, $CH_3$-N-P); 3,66 (d, $^3J_{HP}$ = 10,4 Hz, 288H, -P(O)(O-$\underline{CH_3}$)$_2$); 6,6-7,7 (m, 186H, $CH_{arom}$, CH=N) ppm. RMN $^{13}C-\{^1H\}$ ($CDCl_3$) : $\delta$ = 32,95 (s, $\underline{CH_2}$-$CH_2$-N); 33,0 (d, $^2J_{CP}$ =11,2 Hz, $CH_3$-N-P); 49,4 (dd, $^1J_{CP}$ = 157,5 Hz, $^3J_{CP}$ = 6,6 Hz -$\underline{CH_2}$-$P(O)(OCH_3)_2$); 52,7 (d, $^2J_{CP}$ = 4,2 Hz, -P(O)(O-$\underline{CH_3}$)$_2$); 58,0 (t, $^3J_{CP}$= 7,1 Hz, $CH_2$-$\underline{CH_2}$-N); 121,2 (s, $C_0^2$); 121,7 (s, $C_1^2$); 121,2 (d, $^3J_{CP}$ = 3,9 Hz, $C_2^2$) 128,3 (s, $C_1^3$); 129,65 (s, $C_0^3$); 129,9 (s, $C_2^3$); 132,1 (s, $C_0^4$); 132,4 (s, $C_1^4$); 136,5 (s, $C_2^4$); 138,6 (d, $^3J_{CP}$ = 13,3 Hz, CH=N); 148,8 (s, $C_0^1$); 148,9 (d, $^2J_{CP}$ = 7,5 Hz, $C_2^1$); 151,2 (d, $^2J_{CP}$ = 7,4 Hz, $C_1^1$) ppm.

**Exemple 35 : Synthèse du dendrimère de troisième génération à surface aza-bis-diméthyl-phosphonate dérivé de la tyramine**

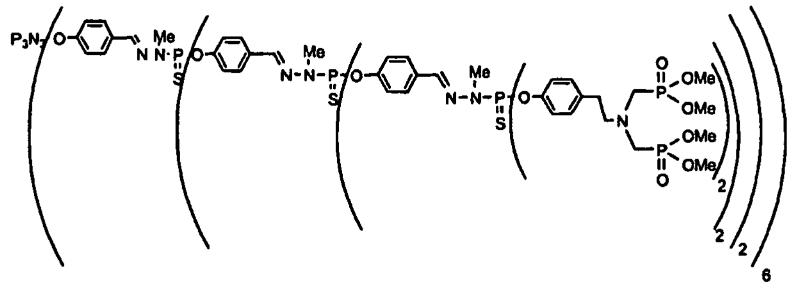

A une solution de dendrimère $Gc_3$ ($9,3.10^{-3}$ mmol, 100 mg) en solution dans du THF anhydre (2 mL) est additionné du carbonate de césium (0,941 mmol, 0,306 g) puis le phénol aza-bis-diméthyl-phosphonate dérivé de la tyramine obtenu dans **l'Exemple 31** est ajouté (0,471 mmol, 180 mg). On laisse le mélange sous agitation pendant 24 heures à température ambiante puis on filtre le mélange final sur célite de manière à séparer les sels. Enfin le produit final est lavé par précipitation dans du pentane et isolé avec un rendement de 80%. RMN $^{31}P-\{^1H\}$ ($CDCl_3$) : $\delta$ = 66,6 (s, $P_3$); 66,3 (s, $P_2$); 65,8 (s, $P_1$); 30,2 (s, $P(O)(OMe)_2$); 12,0 (s, $P_0$) ppm.
RMN $^1H$ ($CDCl_3$) : $\delta$ = 2,67 (s large, 96H, $\underline{CH_2}$-$CH_2$-N); 2,97 (s large, 96H, $CH_2$-$\underline{CH_2}$-N); 3,10 (d, $^2J_{HP}$ = 9,60 Hz, 192H, -$\underline{CH_2}$-P(O)($OCH_3)_2$); 3,25 (s large, 126H, $\underline{CH_3}$-N-P); 3,63 (d, $^3J_{HP}$ = 10,25 Hz, 576H, -P(O)(O-$\underline{CH_3})_2$); 6,5-7,7 (m, 402H, $\underline{CH}_{arom}$, CH=N) ppm.
RMN $^{13}C-\{^1H\}$ ($CDCl_3$) : $\delta$ = 32,9 (s, $\underline{CH_2}$-$CH_2$-N); 32,9 (s, $CH_3$-N-P); 49,3 (dd, $^1J_{CP}$= 157,5 Hz, $^3J_{CP}$ = 6,5 Hz -$\underline{CH_2}$-P(O)($OCH_3)_2$); 52,6 (d, $^2J_{CP}$ = 3,6 Hz, -P(O)(O-$\underline{CH_3})_2$); 58,0 (t, $^3J_{CP}$ = 6,9 Hz, $CH_2$-$\underline{CH_2}$-N); 120,5 (s, $C_0^2$); 121,2 (d, $^3J_{CP}$ = 3,1 Hz, $C_3^2$); 121,5 (s, $C_1^2$); 121,8 (s, $C_2^2$); 128,2 (s, $C_0^3$); 128,2 (s, $C_1^3$); 129,6 (s, $C_2^3$); 129,9 (s, $C_3^3$); 132,3 (s, $C_0^4$); 132,3 (s, $C_1^4$); 132,3 (s, $C_2^4$); 136,5 (s, $C_3^4$); 138,6 (d, $^3J_{CP}$ = 13,0 Hz, CH=N); 148,9 (d large, $^2J_{CP}$ = 6,3 Hz, $C_0^1$, $C_1^1$, $C_3^1$); 151,2 (d, $^2J_{CP}$= 6,1 Hz, $C_2^1$) ppm.

**Exemple 36 : Synthèse du dendrimère de première génération à surface aza-bis-phosphonique dérivé de la tyramine (GC1)**

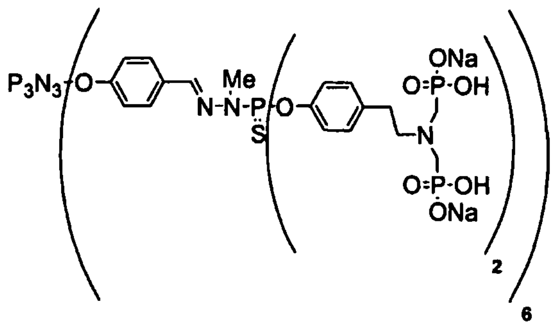

A une solution de dendrimère de première génération à extrémités aza-bis-diméthyl-phosphonate dérivé de la tyramine obtenu dans **l'Exemple 33** ($1,68.10^{-2}$ mmol, 100 mg) à 0°C dans de l'acétonitrile (5 mL) on additionne lentement du bromotriméthylsilane (1,04 mmol; 138 $\mu$l). Une fois l'addition terminée on laisse le mélange revenir à température ambiante pendant 12 heures. Le mélange est alors évaporé à sec puis on additionne 1 ml de méthanol anhydre à température ambiante et on laisse le mélange une heure sous agitation. Après évaporation à sec, le résidu est lavé plusieurs fois à l'éther pur. Le produit étant totalement insoluble dans les solvants organiques il est transformé en son mono sel de sodium en présence de soude (36,3 mg de soude, pour 100 mg de dendrimère). La solution résultante est lyophilisée pour donner le dendrimère sous forme d'une poudre blanche. Le produit final est isolé avec un rendement de 58%.
RMN $^{31}P-\{^1H\}$ ($CD_3CN/D_2O$) : $\delta$ = 67,9 (s, $P_1$); 14,4 (s, $P(O)(ONa)_2$); 12,9 (s, $P_0$) ppm.
RMN $^{13}C-\{^1H\}$ ($CD_3CN/D_2O$) : $\delta$ = 31,95 (s, $\underline{CH_2}$-$CH_2$-N); 35,5 (d, $^2J_{CP}$ = 10,9 Hz, $CH_3$-N-$P_1$); 57,0 (d, $^1J_{CP}$ = 136,8 Hz, -$\underline{CH_2}$-P(O)$(OH)_2$); 60,7 (s, $CH_2$-$\underline{CH_2}$-N); 124,1 (s, $C_0^2$); 124,1 (s, $C_1^2$); 131,3 (s, $C_0^3$); 133,5 (s, $C_1^3$); 135,3 (s, $C_0^4$); 139,0 (s, $C_1^4$); 143,2 (s large, CH=N); 151,7 (d, $^2J_{CP}$ = 7,0 Hz, $C_1^1$); 153,3 (s, $C_0^1$) ppm.

**Exemple 37 : Synthèse du dendrimère de deuxième génération à surface aza-bis-phosphonique dérivé de la tyramine (GC2)**

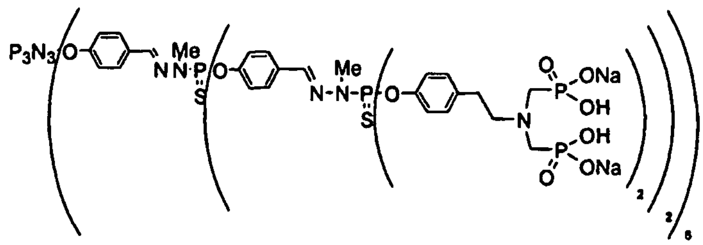

A une solution de dendrimère de deuxième génération à extrémités aza-bis-diméthyl-phosphonate dérivé de la tyramine obtenu dans **l'Exemple 34** (8,27.$10^{-2}$ mmol, 1,08 g) à 0°C dans de l'acétonitrile (10 mL) on additionne lentement du bromotriméthylsilane (10 mmol, 1,34 ml). Une fois l'addition terminée on laisse le mélange revenir à température ambiante pendant 12 heures. Le mélange est alors évaporé à sec puis on additionne 3 ml de méthanol anhydre à température ambiante et on laisse le mélange une heure sous agitation. Après évaporation à sec, le résidu est lavé plusieurs fois à l'éther pur. Le produit étant totalement insoluble dans les solvants organiques il est transformé en son mono sel de sodium en présence de soude (8,2 mg de soude, pour 50 mg de dendrimère). La solution résultante est lyophilisée pour donner le dendrimère sous forme d'une poudre blanche. Le produit final est isolé avec un rendement de 62%.
RMN $^{31}P\{^1H\}$ ($CD_3CN/D_2O$) : δ = 67,8 (s, $P_2$); 67,6 (s, $P_1$); 10,5 (s, P(O)(ONa)(OH)); 10,0 (s, $P_0$) ppm.
RMN $^{13}C$-$\{^1H\}$ ($CD_3CN/D_2O$): δ = 31,6 (s, $CH_3$-N-$P_1$); 35,3 (s, $\underline{CH_2}$-$CH_2$-N); 55,2 (d, $^1J_{CP}$ = 128,2 Hz, -$\underline{CH_2}$-P(O)$(OH)_2$); 60,4 (s, $CH_2$-$\underline{CH_2}$-N); 124,3 (s, $C_0^2$); 124,3 (s, $C_1^2$); 124,3 (s, $C_2^2$); 131,3 (s, $C_0^3$); 131,3 (s, $C_1^3$); 133,3 (s, $C_2^3$); 135,0 (s, $C_0^4$); 135,0 (s, $C_1^4$); 136,0 (s, $C_2^4$); 142,5 (s large, CH=N); 151,8 (s large, $C_2^1$); 153,3 (s large, $C_1^1$); 153,3 (s, $C_0^1$) ppm.

Les dendrimères à surface aza-bis-phosphonique ne peuvent pas être préparés par application ou adaptation de la méthode ci-dessus à partir des dendrimères à surface tétraisopropyl-gem-diphosphonate des exemples 28 à 30.

Les dendrimères à surface aza-bis-phosphonique peuvent être préparés par application ou adaptation de la méthode ci-dessus à partir des dendrimères à surface aza-bis-diméthyl-phosphonate dérivé de la 4-hydroxyaniline des exemples 38 et 39 suivants :

**Exemple 38 : Synthèse du dendrimère de première génération à surface aza-bis-diméthyl-phosphonate dérivé de la 4-hydroxyaniline**

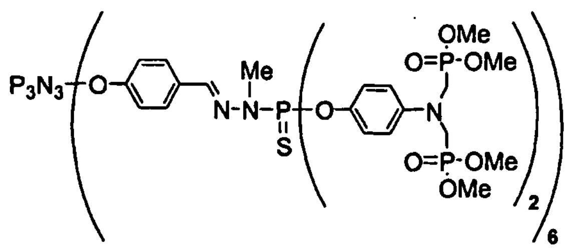

A une solution de dendrimère $Gc_1$ (0,116 mmol, 214 mg) en solution dans du THF anhydre (10 mL) est additionné du carbonate de césium (2,94 mmol, 955 mg) puis le phénol obtenu dans **l'Exemple 32** est ajouté (1,47 mmol, 520 mg). On laisse le mélange sous agitation pendant 24 heures à température ambiante puis on filtre le mélange final sur célite de manière à séparer les sels. Enfin le produit final est précipité dans du pentane, puis lavé à l'éther. Le produit final est isolé avec un rendement de 76%.
RMN $^{31}P$-$\{^1H\}$ ($C_6D_6$/THF) : δ = 67,9 (s, $P_1$); 29,3 (s, P(O)$(OMe)_2$); 12,3 (s, $P_0$) ppm.
RMN $^1H$ ($CDCl_3$) : δ = 3,20 (d, $^3J_{HP}$ = 10,4 Hz, 18H, $CH_3$-N-$P_1$); 3,70 (d, $^3J_{HP}$ = 10,5 Hz, 144H, -P(O)(O-$\underline{CH_3}$)$_2$); 3,9 (d, $^2J_{HP}$ = 4,8 Hz, 48H, -$\underline{CH_2}$-P(O)$(OCH_3)_2$); 6,7-7,8 (m, 78H, $CH_{arom}$, CH=N) ppm.
RMN $^{13}C$-$\{^1H\}$ ($CDCl_3$): δ = 33,0 (d, $^2J_{CP}$ = 11,8 Hz, $CH_3$-N-$P_1$); 46,4 (d, $^1J_{CP}$ = 158,3 Hz, - $\underline{CH_2}$-P(O)$(OCH_3)_2$); 52,6 (d, $^2J_{CP}$ = 3,7 Hz, -P(O)(O-$\underline{CH_3}$)$_2$); 52,7 (d, $^2J_{CP}$ = 3,9 Hz, -P(O)(O-$\underline{CH_3}$)$_2$); 114,4 (s, $C_1^2$); 121,2 (s, $C_0^2$); 122,0 (s, $C_1^3$); 128,3 (s, $C_0^3$); 132,2 (s, $C_0^4$); 138,5 (d, $^3J_{CP}$ = 14,1 Hz, CH=N); 142,9 (d, $^3J_{CP}$ = 6,5 Hz, $C_1^4$); 145,1 (s, $C_1^1$); 151,1 (s

large, $C_0^1$) ppm.

**Exemple 39 : Synthèse du dendrimère de deuxième génération à surface aza-bis-diméthyl-phosphonate dérivé de la 4-hydroxyaniline**

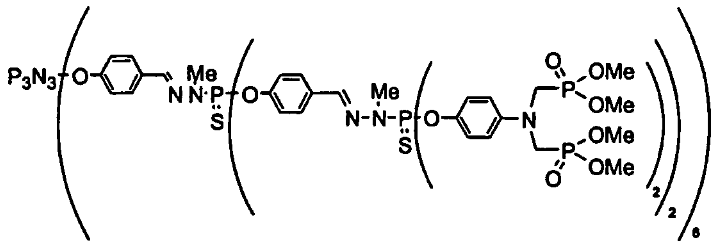

A une solution de dendrimère $Gc_2$ (4,2.10$^{-2}$ mmol, 200 mg) en solution dans du THF anhydre (5 mL) est additionné du carbonate de césium (2,00 mmol, 652 mg) puis le phénol obtenu dans **l'<u>Exemple 32</u>** est ajouté (1,05 mmol, 372 mg). On laisse le mélange sous agitation pendant 24 heures à température ambiante puis on filtre le mélange final sur célite de manière à séparer les sels. Enfin le produit final est précipité dans du pentane, puis lavé à l'éther. Le produit final est isolé avec un rendement de 81 %.
RMN $^{31}P-\{^1H\}$ ($CDCl_3$) : δ = 68,1 (s, $P_1$); 66,2 (s, $P_1$); 29,2 (s, $P(O)(OMe)_2$); 11,7 (s, $P_0$) ppm. RMN $^1H$ ($CDCl_3$) : 3,25 (d, $^3J_{HP}$ = 10,2 Hz, 54H, $CH_3$-N-$P_1$, $CH_3$-N-$P_2$); 3,65 (d, $^3J_{HP}$ = 10,3 Hz, 288H, -P(O)(O-$\underline{CH_3}$)$_2$); 3,88 (d, $^2J_{HP}$ = 4,7 Hz, 96H, -$\underline{CH_2}$-P(O)($OCH_3$)$_2$); 6,7-7,8 (m, 186H, $CH_{arom}$, CH=N) ppm.
RMN $^{13}C-\{^1H\}$ ($CDCl_3$) : δ = 32,9 (d large, $^2J_{CP}$ = 11,7 Hz, $\underline{CH_3}$-N-$P_1$, $\underline{CH_3}$-N-$P_2$); 46,3 (d, $^1J_{CP}$ = 158,5 Hz, -$\underline{CH_2}$-P(O)($OCH_3$)$_2$); 52,6 (s large, -P(O)(O-$\underline{CH_3}$)$_2$); 114,3 (s large, $C_0^2$, $C_1^2$, $C_2^2$); 121,8 (s, $C_2^3$); 128,1 (s, $C_1^3$); 131,3 (s, $C_0^3$); 131,7 (s, $C_0^4$); 132,1 (s, $C_1^4$); 138,4 (s large, CH=N); 142,6 (d, $^3J_{CP}$= 6,8 Hz, $C_2^4$); 145,0 (s, $C_2^1$); 151,0 (s large, $C_0^1$, $C_1^1$) ppm.

**Exemple 40 : Synthèse du dendrimère de génération zéro à surface aza-bis-diméthyl-phosphonate dérivé de la tyramine**

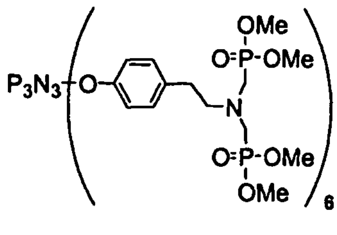

A une solution d'hexachlorocyclotriphosphazène (2,4 mmol, 834 mg) en solution dans du THF anhydre (5 mL) est additionné du carbonate de césium (31,2 mmol, 10,16 g) puis le phénol aza-bis-diméthyl-phosphonate dérivé de la tyramine obtenu dans **l'<u>Exemple 31</u>** est ajouté (15,6 mmol, 5,96 g). On laisse le mélange sous argon et avec une agitation magnétique pendant 3 jours à température ambiante. Après filtration sur célite, le produit est précipité dans du pentane. Le produit isolé peut contenir [0-5%] de phénol aza-bis-diméthyl-phosphonate en excès. Le produit final est isolé avec un rendement de 85%.
RMN $^{31}P-\{^1H\}$ ($CDCl_3$) : δ = 30,2 (s, $P(O)(OMe)_2$); 12,9 (s, $P_0$) ppm.
RMN $^1H$ ($CDCl_3$) : δ = 2,72 (t déformé, $^3J_{HH}$ = 8,4 Hz, 2H, -$\underline{CH_2}$-$CH_2$-N); 3,00 (t déformé, $^3J_{HH}$ = 8,2 Hz, 2H, -$CH_2$-$\underline{CH_2}$-N-); 3,18 (d, $^2J_{HP}$ = 8,9 Hz, 4H, N-$\underline{CH_2}$-P); 3,70 (d, $^3J_{HP}$ = 7,8 Hz, 12H, -OMe); 6,7-7,2 (m, 4H, $CH_{arom}$) ppm.
RMN $^{13}C-\{^1H\}$ ($CDCl_3$) : δ = 32,90 (s, $\underline{CH_2}$-$CH_2$-N); 49,4 (dd, $^1J_{CP}$= 157,3 Hz, $^3J_{CP}$ = 6,6 Hz -$\underline{CH_2}$-P(O)($OCH_3$)$_2$); 52,6 (d, $^2J_{CP}$ = 3,0 Hz, -P(O)(O-$\underline{CH_3}$)$_2$); 58,3 (t, $^3J_{CP}$= 7,8 Hz, $CH_2$-$\underline{CH_2}$-N); 120,7 (s, $C_0^2$); 129,7 (s, $C_0^3$); 135,9 (s, $C_0^4$); 149,0 (d, $^2J_{CP}$ = 3,9 Hz, $C_0^1$) ppm.

**Exemple 41 : Synthèse du dendrimère de génération zéro à surface aza-bis-diméthyl-phosphonique dérivé de la tyramine (GC0)**

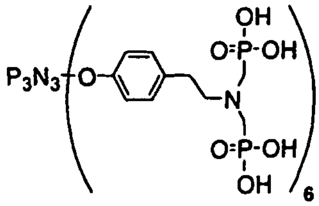

A une solution de dendrimère de génération zéro à extrémités aza-bis-diméthyl-phosphonate obtenu dans l'<u>**Exemple 40**</u> (4,9 mmol, 11,84 g) à 0°C dans de l'acétonitrile (5 mL) on additionne lentement du bromotriméthylsilane (165 mmol, 22 mL). Une fois l'addition terminée on laisse le mélange revenir à température ambiante pendant 24 heures. Le mélange est alors évaporé à sec puis on additionne lentement 5 ml d'eau à température ambiante et on laisse le mélange une heure sous agitation. Après filtration, le résidu est lavé plusieurs fois à l'éther pur. Le produit final est isolé avec un rendement de 50%.
RMN $^{31}P-\{^{1}H\}$ ($D_20$) : $\delta$ = 12,9 (s, $P_0$); 11,4 (s, $P(O)(OH)_2$) ppm.
RMN $^{13}C-\{^{1}H\}$ ($D_20$): $\delta$= 31,40 (s, $\underline{CH_2}-CH_2-N$); 53,9 (d, $^{1}J_{CP}$ = 140,07 Hz, $-\underline{CH_2}-P(O)(OH)_2$); 59,7 (s, $CH_2-\underline{CH_2}-N$); 123,9 (s, $C_0^{2}$); 132,9 (s, $C_0^{3}$); 135,7 (s, $C_0^{4}$); 151,4 (s large, $C_0^{1}$) ppm.

**Exemple 42 : Synthèse de dendrimère à surface aza-bis-phosphonique de structure DAB**

Le dendrimère DAB $G_3$ (180 mg) (Aldrich) (E.M.M. de Brabander-van den Berg, E.W. Meijer Angew. Chem. Int. Ed. Engl. 1993, 32, 1308), le diméthyl phosphite (0,4 mL) et le formaldéhyde en solution à 37% dans l'eau (0,5 mL) sont agités à 0°C pendant 30 minutes. Le mélange réactionnel est ensuite agité à une température comprise entre 0°C et 80°C et sous pression autogène jusqu'à ce que la réaction soit complète. Après refroidissement, le brut réactionnel est concentré sous pression réduite et le résidu brut lavé avec un solvant adapté, préférentiellement l'éther pour donner le produit attendu sous forme d'une poudre blanche.

**Exemple 43: Synthèse de dendrimère à surface aza-bis-phosphonique de structure PAMAM**

Le dendrimère PAMAM $G_3$ (400 mg) (Aldrich) (D.A. Tomalia, H. Baker, J. Dewald, M. Hall, G. Kallos, S. Martin, J. Roeck, J. Ryder, P.S. Smith, Polym. J. (Tokyo) 1985, 17, 117; D.A. Tomalia, H. Baker, J. Dewald, M. Hall, G. Kallos, S. Martin, J. Roeck, J. Ryder, P.S. Smith, Macromolecules, 1986, 19, 2466), le diméthyl phosphite (0,43 mL) et le formaldéhyde en solution à 37% dans l'eau (0,55 mL) sont agités à 0°C pendant 30 minutes. Le mélange réactionnel est ensuite agité à une température comprise entre 0°C et 80°C et sous pression autogène jusqu'à ce que la réaction soit complète. Après refroidissement, le brut réactionnel est concentré sous pression réduite et le résidu brut lavé avec un solvant adapté, préférentiellement l'éther pour donner le produit attendu sous forme d'une poudre blanche.

**Exemple 44 : synthèse du dendrimère de première génération à coeur cyclotriphosphazène dérivant de l'amino-méthyl bis-phosphonate**

*Etape 1: synthèse de l'imine dérivée de la méthylamine*

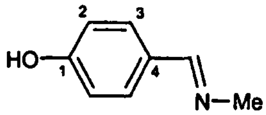

La méthylamine (25 mmol, 3mL) en solution à 33% dans l'éthanol absolu (8 $mol.L^{-1}$) ainsi que le 4-hydroxy-benzaldéhyde (20 mmol, 2,5 g) sont mélangés sans solvant à température ambiante. Le mélange est laissé sous agitation magnétique pendant 24 heures à température ambiante. L'éthanol est évaporé sous pression réduite pour obtenir une huile qui est dissoute dans un minimum d'éther puis précipitée dans du pentane. Cette imine n'a pas été isolée car elle

est directement utilisée dans l'étape suivante.

*Etape 2 : synthèse de l'amino-méthyl mono-phosphonate*

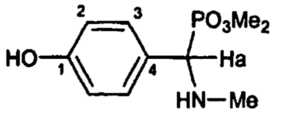

Le phénol porteur de la fonction imine de l'étape 1 ci-dessus (17,0 mmol, 2,3g) est mélangé sans solvant et à température ambiante avec quelques gouttes de triéthylamine ainsi que du diméthylphosphite (18,7 mmol, 1,7 ml). Le mélange est laissé pendant 12 heures à température ambiante puis il est évaporé à sec. La poudre obtenue est mise en solution dans l'acétone puis passée sur un « patch » de silice. Enfin l'éluant est évaporé pour obtenir le produit final avec un rendement de 68%.
RMN $^{31}P$-{$^{1}H$} ($CDCl_3$) : $\delta$ = 29,6 (s, P(O)($OMe)_2$) ppm.
RMN$^{1}H$ ($CDCl_3$) : $\delta$ = 2,29 (s, 3H, N-<u>$CH_3$</u>); 3,54 (d, $^{3}J_{HP}$ = Hz, 3H, -OMe); 3,72 (d, 3H, $^{3}J_{HP}$ = Hz, 3H, -OMe); 3,84 (d, $^{2}J_{HP}$= 23,9 Hz, 1H, H); 6,73 (d, $^{3}J_{HH}$ = Hz, $CH_{arom}$, 2H); 7,14 (dd, $CH_{arom}$, 2H) ppm.
RMN $^{13}C$-{$^{1}H$} ($CDCl_3$) : $\delta$ = 43,3 (t, $^{3}J_{CP}$ = 6,8 Hz, N-<u>Me</u>); 53,6 (d, $^{2}J_{CP}$ = 7,7 Hz, OMe); 54,1 (d, $^{2}J_{CP}$ = 6,4 Hz, OMe); 63,2 (dd, $^{1}J_{CP}$ = 159,6 Hz, $^{3}J_{CP}$ = 14,5 Hz, CH); 115,6 (s, $C_2$); 121,1 (d, $^{2}J_{CP}$ = 3,8 Hz, $C_4$); 132,0 (d, $^{3}J_{CP}$ = 8,9 Hz, $C_3$); 157,1 (s, $C_1$) ppm.

*Etape 3 : synthèse de l'amino-méthyl bis-phosphonate*

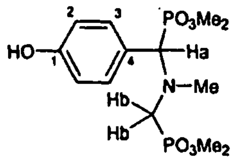

L'amine secondaire de l'étape 2 ci-dessus (6,1 mmol, 1,5 g) est mise en solution à température ambiante dans le formaldéhyde en solution aqueuse 37 % (12,2 mmol, 1 ml) et le diméthylphosphite (24,4 mmol, 2,24 mL) sans solvant. Le mélange est laissé sous agitation magnétique et à température ambiante pendant 12 heures. Puis le mélange final est lavé plusieurs fois avec un mélange éther/pentane 1/1. Enfin le produit est purifié par chromatographie sur gel de silice en utilisant l'acétate d'éthyle comme solvant (Rf=0,35), le produit final est isolé avec un rendement de 65%.
RMN $^{31}P$-{$^{1}H$} ($CDCl_3$) : $\delta$ = 28,1 (s, P(O)($OMe)_2$); 30.9 (s, P(O)($OMe)_2$) ppm.
RMN $^{1}H$ ($CDCl_3$) : $\delta$ = 2,41 (s, 3H, N-<u>$CH_3$</u>); 2,61 (dd, $^{2}J_{HP}$= 6,3 Hz, $^{2}J_{HH}$ = 15,3 Hz, 1H, Hb); 3,12 (dd, $^{2}J_{HP}$= 15,6 Hz, $^{2}J_{HH}$ = 15,6 Hz, 1H, Hb); 3,30-3.80 (m, 12H, -OMe); 4.05 (d, $^{2}J_{HP}$= 23.9 Hz, 1H, Ha); 6.74 (d, $^{3}J_{HH}$= 7,84 Hz, $CH_{arom}$, 2H); 7,17 (d, $^{3}J_{HH}$= 7,85 Hz, $CH_{arom}$, 2H); 9,08 (s large, 1H, -OH) ppm.
RMN $^{13}C$-{$^{1}H$} ($CDCl_3$) : $\delta$ = 42,3 (t, $^{3}J_{CP}$ = 6,3 Hz, N-<u>Me</u>); 49,2 (dd, $^{1}J_{CP}$ = 164,1 Hz, $^{3}J_{CP}$ = 10,1 Hz, $CH_2$); 53,0 (m, OMe); 65,2 (dd, $^{1}J_{CP}$ = 161,7 Hz, $^{3}J_{CP}$ = 13,5 Hz, CH); 115,4 (s, $C_2$); 120,9 (d, $^{2}J_{CP}$ = 3,5 Hz, $C_4$); 131,8 (d, $^{3}J_{CP}$ = 9,1 Hz, $C_3$); 157,8 (s, $C_1$) ppm.

*Etape 4: synthèse du dendrimère de première génération dérivant de l'amino-méthyl bis-phosphonate*

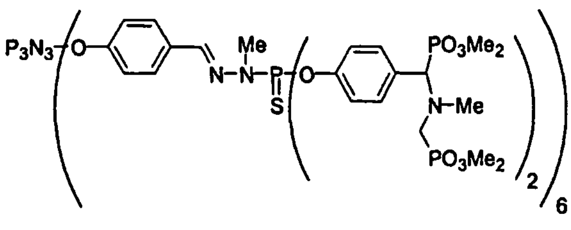

A une solution de dendrimère $Gc_1$ (0,047 mmol, 87 mg) dans du THF anhydre (2 mL) est additionné du carbonate de césium (1,2 mmol, 390 mg) puis le phénol aza-bis-diméthyl-phosphonate dérivé de la méthylamine de l'étape 3 ci-dessus est ajouté (0,6 mmol, 220 mg). On laisse le mélange sous agitation pendant 24 heures à température ambiante puis on filtre sur célite et on centrifuge le mélange final de manière à séparer les sels. Enfin le produit final est lavé par précipitation dans du pentane et isolé avec un rendement de 75%.
RMN $^{31}P-\{^1H\}$ ($CDCl_3$) : $\delta$ = 65,4 (s, $P_1$); 30,4 (s, P(O)(OMe)$_2$); 27,5 (s, P(O)(OMe)$_2$); 11,4 (s, $P_0$) ppm.
RMN $^1H$ ($CDCl_3$) : $\delta$ = 2,46 (s, 36H, N-$\underline{CH_3}$); 2,65 (dd, $^2J_{HP}$ = 7,4 Hz, $^2J_{HH}$ = 15,3 Hz, 12H, $CH_2$); 3,12 (dd, $^2J_{Hp}$ = 1 5,5 Hz, $^2J_{HH}$ =15,5 Hz, 12H, $CH_2$); 3,25 (d, $^3J_{HP}$ = 10,1 Hz, 18H, $CH_3$-N-$P_1$); 3,30-3.90 (m, 144H, -OMe); 4.2 (d, $^2J_{HP}$ = 23.4 Hz, 12H, CH); 6,7-7,6 (m, 78H, $CH_{arom}$, CH=N) ppm.
RMN $^{13}C-\{^1H\}$ ($CDCl_3$) : $\delta$ = 32,8 (d, $^2J_{CP}$= 12,3 Hz, $CH_3$-N-$P_1$); 42,2 (t, $^3J_{CP}$ = 6,8 Hz, N-$\underline{Me}$); 49,3 (dd, $^1J_{CP}$ = 164,0 Hz, $^3J_{CP}$ = 9,9 Hz, $CH_2$); 52,3-53,7 (m, OMe); 64,9 (dd, $^1J_{CP}$ = 138,1 Hz, $^3J_{CP}$ = 11,9 Hz, $\underline{CH}$); 121,1 (s large, $C_0^2$, $C_1^2$); 128,2 (s, $C_0^3$); 128,4 (d, $^2J_{CP}$ = 3,1 Hz, $C_1^4$); 131,8 (s, $C_0^4$); 131,8 (d, $^3J_{CP}$ = 8,2 Hz, $C_1^3$); 139,0 (d, $^3J_{CP}$ = 14,5 Hz, CH=N); 150,6 (d, $^2J_{CP}$ = 6,9 Hz, $C_1^1$); 151,2 (s, $C_0^1$) ppm.

*Etape 5 : synthèse du dendrimère de première génération dérivant de l'acide amino-méthyl bis-phosphonique*

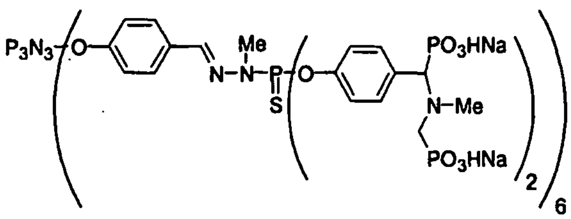

A une solution du dendrimère de première génération à extrémités aza-bis-diméthyl-phosphonate dérivé de la méthylamine de l'étape 4 (3,97.10$^{-2}$ mmol, 230 mg) à 0°C dans de l'acétonitrile (5 mL) on additionne lentement du bromotriméthylsilane (2,1 mmol; 280 $\mu$l). Une fois l'addition terminée on laisse le mélange revenir à température ambiante pendant 12 heures. Le mélange est alors évaporé à sec puis on additionne 1 ml de méthanol anhydre à température ambiante et on laisse le mélange une heure sous agitation. Après évaporation à sec, le résidu est lavé plusieurs fois à l'éther pur. Le produit étant totalement insoluble dans les solvants organiques il est transformé en son mono sel de sodium en présence de soude (3,1 ml d'une solution de soude, à 0,1955 mol.L$^{-1}$ pour 130 mg de dendrimère acide phosphonique). La solution résultante est lyophilisée pour donner le dendrimère sous forme d'une poudre blanche. Le produit final est isolé avec un rendement de 58%.
RMN $^{31}P-\{^1H\}$ ($CD_3CN/D_2O$) : $\delta$ = 66,09 (s, $P_1$ et $P_2$); 14,1 (s, $P_0$); 11,2 (s, $PO_3HNa$) ppm. RMN $^1H$ ($CD_3CN/D_2O$) : $\delta$ = 2,5-3,8 (m, 90H, $CH_3$-N-P, N-Me, $CH_2$, CH); 6,5-8,0 (m, 78H, $CH_{arom}$, CH=N).
RMN $^{13}C-\{^1H\}$ ($CD_3CN/D_2O$) : à =35,5 (s large, $CH_3$-N-$P_1$); 44,8 (s large, N-$\underline{Me}$); 54,5 (d, $^1J_{CP}$ =132,5 Hz, $CH_2$); 70,5 (d, $^1J_{CP}$ = 129,4 Hz, CH); 124,4 (s large, $C_0^2$, $C_1^2$); 130,4 (s large, $C_0^3$, $C_1^3$); 136,3 (s large, $C_0^4$, $C_1^4$); 142,9 (s large, CH=N); 153,9 (s large, $C_0^1$, $C_1^1$) ppm.

**Exemple 45: synthèse du dendrimère de deuxième génération à coeur cyclotriphosphazène dérivant de l'amino-méthyl bis-phosphonate**

*Etape 1 : synthèse du dendrimère de deuxième génération dérivant de l'amino-méthyl bis-phosphonate*

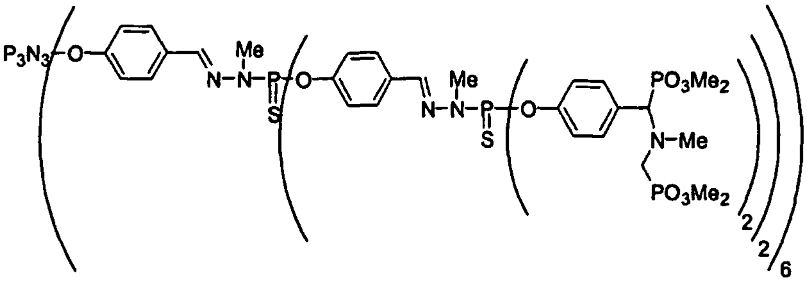

A une solution de dendrimère $Gc_2$ (0,024 mmol, 119 mg) dans du THF anhydre (2 mL) est additionné du carbonate de césium (1,3 mmol, 407 mg) puis le phénol aza-bis-diméthyl-phosphonate dérivé de la méthylamine de l'étape 3 de l'**Exemple 44** est ajouté (0,67 mmol, 230 mg). On laisse le mélange sous agitation pendant 24 heures à température ambiante puis on filtre sur célite et on centrifuge le mélange final de manière à séparer les sels. Enfin le produit final est lavé par précipitation dans du pentane et isolé avec un rendement de 80%. RMN $^{31}P-\{^1H\}$ ($CDCl_3$) : $\delta$ = 66,1 (s, $P_1$); 65,4 (s, $P_2$); 30,4 (s, $P(O)(OMe)_2$); 27,5 (s, $P(O)(OMe)_2$); 11,6 (s, $P_0$) ppm.
RMN $^1H$ ($CDCl_3$) : $\delta$ = 2,48 (s, 72H, N-$\underline{CH_3}$); 2,67 (dd, $^2J_{HP}$ = 7,1 Hz, $^2J_{HH}$ = 15,3 Hz, 24H, $CH_2$); 3,14 (dd, $^2J_{HP}$= 15,4 Hz, $^2J_{HH}$= 15,4 Hz, 24H, $CH_2$); 3,31 (d, $^3J_{HP}$ = 10,9 Hz, 54H, $CH_3$-N-$P_1$, $CH_3$-N-$P_2$); 3,30-3.90 (m, 288H, -OMe); 4.2 (d, $^2J_{HP}$ = 23.2 Hz, 24H, CH); 7,0-7,7 (m, 186H, $CH_{arom}$, CH=N) ppm.
RMN $^{13}C-\{^1H\}$ ($CDCl_3$) : $\delta$ = 32,9 (d, $^2J_{CP}$= 12,4 Hz, $CH_3$-N-$P_1$, $CH_3$-N-$P_2$); 42,3 (t, $^3J_{CP}$ = 7,0 Hz, N-$\underline{Me}$) ; 49,5 (dd, $^1J_{CP}$ = 163,7 Hz, $^3J_{CP}$ = 9,8 Hz, $CH_2$); 52,4-53,6 (m, OMe); 64,9 (dd, $^1J_{CP}$ = 160,2 Hz, $^3J_{CP}$ = 12,1 Hz, CH); 121,17 (s, $C_2^2$); 121,24 (s, $C_1^2$); 121,8 (s, $C_0^2$); 128,3 (s large, $C_2^4$); 128,5 (s large, $C_0^3$, $C_1^3$); 131,8 (d large, $^3J_{CP}$ = 8,2 Hz, $C_1^4$, $C_2^3$); 132,3 (s large, $C_0^4$); 138,9 (d, $^3J_{CP}$ = 13,8 Hz, CH=N); 150,7 (d large, $^2J_{CP}$ = 7,2 Hz, $C_2^1$); 151,2 (s large, $C_0^1$, $C_1^1$) ppm.

*Etape 2 : synthèse du dendrimère de deuxième génération dérivant de l'acide amino-méthyl bis-phosphonique*

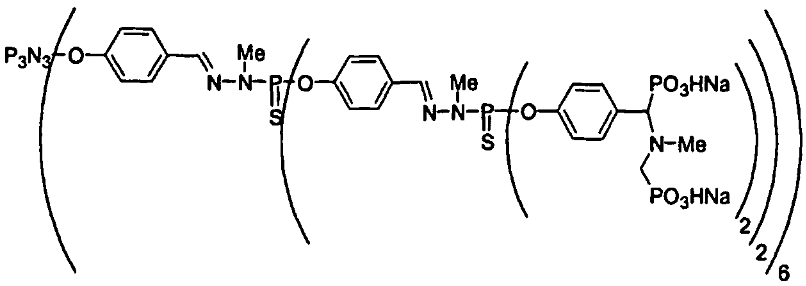

A une solution du dendrimère de deuxième génération à extrémités aza-bis-diméthyl-phosphonate dérivé de la méthylamine de l'étape 1 (1,49.10$^{-2}$ mmol, 190 mg) à 0°C dans de l'acétonitrile (5 mL) on additionne lentement du bromotriméthylsilane (1,6 mmol; 210 µl). Une fois l'addition terminée on laisse le mélange revenir à température ambiante pendant 12 heures. Le mélange est alors évaporé à sec puis on additionne 1 ml de méthanol anhydre à température ambiante et on laisse le mélange une heure sous agitation. Après évaporation à sec, le résidu est lavé plusieurs fois à l'éther pur. Le produit étant totalement insoluble dans les solvants organiques il est transformé en son mono sel de sodium en présence de soude (3,01 ml d'une solution de soude, à 0,1955 mol.L$^{-1}$ pour 140 mg de dendrimère acide phosphonique). La solution résultante est lyophilisée pour donner le dendrimère sous forme d'une poudre blanche. Le produit final est isolé avec un rendement de 54%.
RMN $^{31}P-\{^1H\}$ ($CD_3CN/D_2O$) : $\delta$ = 66,7 (s, $P_1$); 14,4 (s, $P_0$) 10,8 (s, $PO_3HNa$) ppm.
RMN $^1H$ ($CD_3CN/D_2O$) : $\delta$ = 2,5-3,8 (m, 198H, $CH_3$-N-$P_1$, $CH_3$-N-$P_2$, N-Me, $CH_2$, CH); 6,5-8,0 (m, 186H, $CH_{arom}$, CH=N).
RMN $^{13}C-\{^1H\}$ ($CD_3CN/D_2O$) : $\delta$ = 35,5 (s large, $CH_3$-N-$P_1$, $CH_3$-N-$P_2$); 44,6 (s large, N-$\underline{Me}$); 55,6 (d, $^1J_{CP}$ = 102,7 Hz, $CH_2$); 71,0 (d, $^1J_{CP}$ = 128,2 Hz, CH); 124,4 (s large, $C_0^2$, $C_1^2$, $C_2^2$); 130,4 (s large, $C_0^3$, $C_1^3$, $C_2^3$); 136,3 (s large, $C_0^4$, $C_1^4$, $C_2^4$); 142,9 (s large, CH=N); 153,9 (s, $C_0^1$, $C_1^1$, $C_2^1$) ppm.

**Exemple 46: synthèse du dendrimère de quatrième génération à coeur cyclotriphosphazène dérivant de l'amino-méthyl bis-phosphonate**

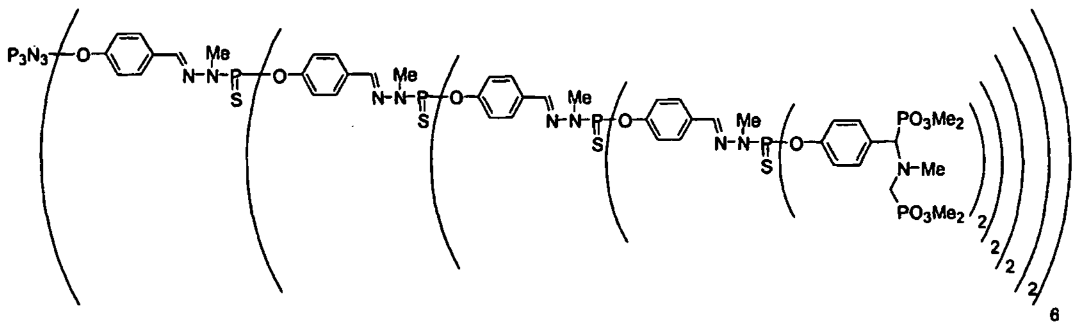

A une solution de dendrimère $Gc_4$ ($3{,}5.10^{-3}$ mmol, 79,2 mg) dans du THF anhydre (2 mL) est additionné du carbonate de césium (0,71 mmol, 230 mg) puis le phénol aza-bis-diméthyl-phosphonate dérivé de la méthylamine de l'étape 3 de l'**Exemple 44** est ajouté (0,35 mmol, 130 mg). On laisse le mélange sous agitation pendant 48 heures à température ambiante puis on filtre sur célite et on centrifuge le mélange final de manière à séparer les sels. Enfin le produit final est lavé par précipitation dans du pentane et isolé avec un rendement de 84%.
RMN $^{31}P$-{$^1H$} ($CDCl_3$) : δ = 66,1 (s large, $P_1$, $P_2$, $P_3$); 65,4 (s, $P_4$); 30,1 (s, $P(O)(OMe)_2$); 27,6 (s, $P(O)(OMe)_2$); 11,6 (s, $P_0$) ppm.
RMN $^1H$ ($CDCl_3$) : δ = 2,48 (s, 288H, N-$\underline{CH_3}$); 2,65 (dd, $^2J_{HP}$= 7,2 Hz, $^2J_{HH}$= 15,3 Hz, 96H, $CH_2$); 3,13 (dd, $^2J_{HP}$ = 15,2 Hz, $^2J_{HH}$ = 15,2 Hz, 96H, $CH_2$); 3,27 (s large, 270H, $CH_3$-N-$P_1$, $CH_3$-N-$P_2$, $CH_3$-N-$P_3$, $CH_3$-N-$P_4$); 3,30-3.90 (m, 1152H, -OMe); 4.2 (d, $^2J_{HP}$ = 23.3 Hz, 96H, CH); 7,0-7,7 (m, 834H, $CH_{arom}$, CH=N) ppm.
RMN $^{13}C$-{$^1H$} ($CDCl_3$) : δ = 32,9 (d, $^2J_{CP}$ = 12,4 Hz, $CH_3$-N-$P_1$, $CH_3$-N-$P_2$, $CH_3$-N-$P_3$, $CH_3$-N-$P_4$); 42,3 (t, $^3J_{CP}$ = 7,0 Hz, N-$\underline{Me}$); 49,5 (dd, $^1J_{CP}$ = 163,8 Hz, $^3J_{CP}$ = 9,9 Hz, $CH_2$); 52,4-53,9 (m, OMe); 65,1 (dd, $^1J_{CP}$ = 161,2 Hz, $^3J_{CP}$ = 12,3 Hz, CH); 121,45 (s, $C_3^2$); 122,1 (s large, $C_2^2$, $C_1^2$, $C_0^2$); 128,5 (s large, $C_0^3$ $C_1^3$ $C_2^3$); 128,7 (s large, $C_3^3$); 132,1 (d large, $^3J_{CP}$= 8,2 Hz, $C_3^4$, $C_2^4$); 132,4 (s large, $C_0^4$, $C_1^4$); 139,2 (d, $^3J_{CP}$=13,4 Hz, CH=N); 151,0 (d large, $^2J_{CP}$= 7,2 Hz, $C_3^1$); 151,6 (s large, $C_2^1$, $C_1^1$); 151,7 (s large, $C_0^1$) ppm.

**Exemple 47 : synthèse du dendrimère de première génération à coeur cyclotriphosphazène à surface dérivant de l'amino-butyl bis-phosphonate**

*Etape 1 : synthèse de l'imine dérivant de la butylamine*

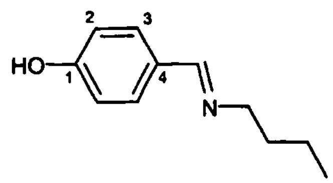

De la n-butylamine (43 mmol, 4,3mL) et du 4-hydroxybenzaldéhyde (41 mmol, 5 g) sont mélangés sans solvant et à température ambiante avec du tamis moléculaire 4Å. Le mélange est laissé sous agitation magnétique pendant 24 heures puis il est repris avec du THF pour être filtré sur célite. Le THF est évaporé sous pression réduite pour obtenir une huile épaisse et sombre. Cette huile peut être dissoute dans un minimum d'éther et est précipité avec du pentane. On obtient ainsi une poudre légèrement rosée avec un rendement de 80%. RMN $^1H$ ($CDCl_3$) : δ = 0,90 (t, $^3J_{HH}$= 7,6 Hz, 3H, -$CH_3$); 1,33 (m, 2H, $CH_2$-$\underline{CH_2}$-$CH_3$); 1,66 (m, 2H, $\underline{CH_2}$-$CH_2$-$CH_3$); 3,59 (t, $^3J_{HH}$ = 7,4 Hz, 2H, N-$\underline{CH_2}$-); 6.7 (d, $^3J_{HH}$ = 8,4 Hz, $CH_{arom}$, 2H); 7,5 (d, $^3J_{HH}$= 8,4 Hz, 2H, $CH_{arom}$); 8,14 (s, 1H, CH=N); 8,81 (s large, 1H, -OH) ppm. RMN $^{13}C$-{$^1H$} ($CDCl_3$) : δ = 13,9 (s, $\underline{CH_3}$); 20,3 (s, $\underline{CH_2}$-$CH_3$); 32,7 (s, $\underline{CH_2}$-$CH_2$-$CH_3$); 60,1 (s, N-$\underline{CH_2}$-$CH_2$- $CH_2$-$CH_3$); 116,3 (s, $C_2$); 125,4 (s, $C_4$); 130,6 (s, $C_3$); 161,5 (s, $C_1$); 162,9 (s, CH=N) ppm.

*Etape 2 : synthèse de l'amino-butyl mono-phosphonate*

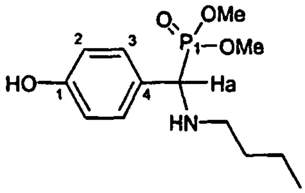

Le phénol porteur de la fonction imine de l'étape 1 (16,9 mmol, 3g) est mélangé sans solvant et à température ambiante avec de la triéthylamine (16,9 mmol, 2,35 ml) ainsi que du diméthylphosphite (16,9 mmol, 1,55 ml). Le mélange est laissé pendant 12 heures à température ambiante puis il est évaporé à sec. La poudre obtenue est mise en solution dans l'acétone puis passée sur un « patch » de silice. Enfin l'éluant est évaporé pour obtenir le produit final avec un rendement de 65%.

RMN $^{31}$P-{$^1$H} ($CDCl_3$) : $\delta$ = 29,8 (s, $P_1$) ppm.

RMN $^1$H ($CDCl_3$) : $\delta$ = 0,80 (t, $^3J_{HH}$ = 7,6 Hz, 3H, -$CH_3$); 1,20-1,55 (m, 4H, $CH_2$-$CH_2$); 2,41 (m, 2H, N-$CH_2$-); 3,6 (d, $^3J_{HP}$ = 10,4 Hz, 3H, -$P_1$OMe); 3,8 (d, $^3J_{HP}$ = 10,8 Hz, 3H, -$P_2$OMe); 4,0 (d, $^2J_{HP}$ = 26.0 Hz, 1H, Ha); 6.7 (d, $^3J_{HH}$ = 8,4 Hz, $CH_{arom}$, 2H); 7,2 (d, $^3J_{HH}$ = 8,4 Hz, $CH_{arom}$, 2H) ppm.

RMN $^{13}$C-{$^1$H} ($CDCl_3$) : $\delta$ = 13,9 (s, $CH_3$); 20,3 (s, $CH_2$-$CH_3$); 31,8 (s, $CH_2$-$CH_2$-$CH_3$); 47,4 (d, $^3J_{CP}$ = 17,6 Hz, N-$CH_2$-$CH_2$- $CH_2$-$CH_3$); 53,6 (d, $^2J_{CP}$ = 7,9 Hz, OMe); 53,9 (d, $^2J_{CP}$ = 6,2 Hz, OMe); 59,8 (d, $^1J_{CP}$ = 157,1 Hz, CH); 115,9 (s, $C_2$); 125,4 (s, $C_4$); 129,5 (s, $C_3$); 157,0 (s, $C_1$) ppm.

*Etape 3 : synthèse de l'amino-butyl bis-phosphonate*

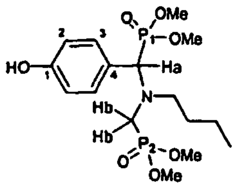

L'amine secondaire de l'étape 2 (5,8 mmol, 1,67 g) est mise en solution à température ambiante dans le formaldéhyde en solution aqueuse 37 % (8,7 mmol, 657$\mu$l) et le diméthylphosphite (5,8 mmol, 530 $\mu$l). Le mélange est laissé sous agitation magnétique et à température ambiante pendant 12 heures. Enfin l'excès de formaldéhyde est éliminé sous pression réduite et le produit est purifié par chromatographie sur gel de silice en utilisant l'acétate d'éthyle comme solvant, le produit final est isolé avec un rendement de 60%.

RMN $^{31}$P-{$^1$H} ($CDCl_3$) : $\delta$ = 28,3 (s, $P_1$); 30.9 (s, $P_2$) ppm.

RMN $^1$H ($CDCl_3$) : $\delta$ = 0,86 (t, $^3J_{HH}$ = 7,6 Hz, 3H, $CH_3$); 1,25-1,55 (m, 4H, $CH_2$-$CH_2$); 2,31 (m, 1H, N-$CH_2$-); 2,64 (dd, $^2J_{HP}$= 3,2 Hz, $^2J_{HH}$= 15,6 Hz, 1H, $CH_2$-$P_2$); 3,11 (m, 1H, N-$CH_2$-$CH_2$); 3,35 (ddd, $^2J_{HP}$= 17,2 Hz, $^2J_{HH}$ = 17,0 Hz, $^4J_{HP}$ = 1,6 Hz, 1H, $CH_2$-$P_2$); 3,5 (d, $^3J_{HP}$ = 10,4 Hz, 3H, -$P_1$OMe); 3,7 (d, $^3J_{HP}$ = 10,8 Hz, 3H, -$P_2$OMe); 3,8 (d, $^3J_{HP}$ = 10,8 Hz, 3H, - $P_2$OMe); 3,9 (d, $^3J_{HP}$ = 10,8 Hz, 3H, -$P_1$OMe); 4,4 (d, $^2J_{HP}$= 26.0 Hz, 1H, Ha); 6.84 (d, $^3J_{HH}$= 8,4 Hz, $CH_{arom}$, 2H); 7,26 (d, $^3J_{HH}$= 8,4 Hz, $CH_{arom}$, 2H); 9,1 (s large, 1H, -OH) ppm.

RMN $^{13}$C-{$^1$H} ($CDCl_3$) : $\delta$ = 14,4 (s, $CH_3$); 20,4 (s, $CH_2$-$CH_3$); 30,6 (s, $CH_2$-$CH_2$-$CH_3$); 46,0 (dd, $^1J_{CP}$ = 166,8 Hz, $^3J_{CP}$ = 8,8 Hz, $CH_2$-$P_2$); 53,0 (d, $^2J_{CP}$ = 6,8 Hz, $P_2$OMe); 53,4 (d, $^2J_{CP}$ = 7,0 Hz, $P_1$OMe); 53,7 (d, $^2J_{CP}$ = 7,0 Hz, $P_2$OMe); 54,1 (d, $^2J_{CP}$ = 7,1 Hz, $P_1$OMe); 53,1 (t, $^3J_{CP}$ = 7,8 Hz, N-$CH_2$-$CH_2$- $CH_2$-$CH_3$); 61,3 (dd, $^1J_{CP}$ = 162,9 Hz, $^3J_{CP}$ = 10,0 Hz, CH); 115,9 (s, $C_2$); 121,5 (d, $^2J_{CP}$ = 6,0 Hz, $C_4$); 132,5 (d, $^3J_{CP}$ = 9,1 Hz, $C_3$); 158,1 (s, $C_1$) ppm.

*Etape 4 : synthèse du dendrimère de première génération dérivant de l'amino-butyl bis-phosphonate*

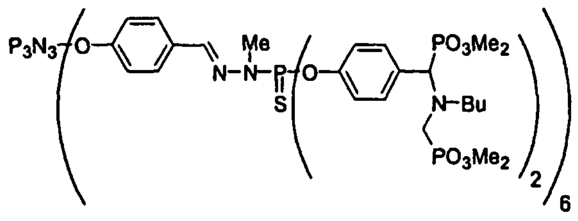

A une solution de dendrimère $Gc_1$ (0,058 mmol, 106 mg) dans du THF anhydre (3 mL) est additionné du carbonate de césium (1,4 mmol, 453 mg) puis le phénol aza-bis-diméthyl-phosphonate dérivé de la butylamine de l'étape 3 est ajouté (0,73 mmol, 300 mg). On laisse le mélange sous agitation pendant 24 heures à température ambiante puis on filtre sur célite et on centrifuge le mélange final de manière à séparer les sels. Enfin le produit final est lavé par précipitation dans du pentane et isolé avec un rendement de 65%.
RMN $^{31}P$-{$^1H$} ($CDCl_3$) : δ = 65,4 (s, $P_1$); 30,8 (s, $P(O)(OMe)_2$); 28,3 (s, $P(O)(OMe)_2$); 11,4 (s, $P_0$) ppm.
RMN $^1H$ ($CDCl_3$) : δ = 0,82 (t, $^3J_{HH}$= 7,6 Hz, 36H, $CH_3$); 1,20-1,50 (m, 48H, $CH_2$-$CH_2$); 2,27 (m, 12H, N-$\underline{CH_2}$-); 2,57 (dd, $^2J_{HP}$ = 3,4 Hz, $^2J_{HH}$ = 15,2 Hz, 12H, $\underline{CH_2}$-$P_2$); 3,11 (m, 12H, N-$\underline{CH_2}$-$CH_2$); 3,26 (d, $^3J_{HP}$ = 10,6 Hz, 18H, $CH_3$-N-P); 3,4 (d, $^3J_{HP}$ =10,6 Hz, 36H, -$P_1$OMe); 3,6 (d, $^3J_{HP}$ = 10,7 Hz, 36H, -$P_2$OMe); 3,7 (d, $^3J_{HP}$ = 10,8 Hz, 36H, -$P_2$OMe); 3,8 (d, $^3J_{HP}$ = 10,6 Hz, 36H, -$P_1$OMe); 4,4 (d, $^2J_{HP}$ = 25.0 Hz, 12H, Ha); 6,9-7,8 (m, 78H, $CH_{arom}$, CH=N) ppm. RMN $^{13}C$-{$^1H$} ($CDCl_3$): δ = 14,0 (s, $\underline{CH_3}$); 19,9 (s, $\underline{CH_2}$-$CH_3$); 30,2 (s, $\underline{CH_2}$-$CH_2$-$CH_3$); 32,8 (d, $^2J_{CP}$ = 11,5 Hz, $CH_3$-N-$P_1$); 46,0 (dd, $^1J_{CP}$ = 166,7 Hz, $^3J_{CP}$ = 8,6 Hz, $CH_2$-$P_2$); 52-54 (m, $PO_3Me_2$); 60,3 (dd, $^1J_{CP}$ = 163,9 Hz, $^3J_{CP}$ = 10,4 Hz, CH); 121,2 (s large, $C_0^2$, $C_1^2$); 128,2 (s, $C_0^3$); 128,8 (d, $^3J_{CP}$ = 4,5 Hz, $C_1^3$); 131,9 (s, $C_1^4$); 132,1 (s, $C_0^4$); 139,1 (d, $^3J_{CP}$ = 13,9 Hz, CH=N); 150,6 (d, $^2J_{CP}$= 6,9 Hz, $C_1^1$); 151,3 (s, $C_0^1$) ppm.

*Etape 5 : synthèse du dendrimère de première génération dérivant de l'acide amino-butyl bis-phosphonique*

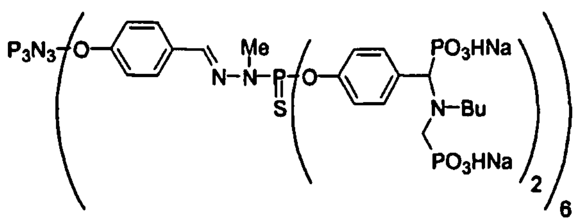

A une solution de dendrimère de première génération à extrémités aza-bis-diméthyl-phosphonate dérivé de la butylamine de l'étape 4 ($1,75.10^{-5}$ mmol, 110 mg) à 0°C dans de l'acétonitrile (4 mL) on additionne lentement du bromotriméthylsilane (0,92 mmol, 123 μl). Une fois l'addition terminée on laisse le mélange revenir à température ambiante pendant 12 heures. Le mélange est alors évaporé à sec puis on additionne 1 ml de méthanol anhydre à température ambiante et on laisse le mélange une heure sous agitation. Après évaporation à sec, le résidu est lavé plusieurs fois à l'éther pur. Le produit étant totalement insoluble dans les solvants organiques il est transformé en son mono sel de sodium en présence de soude (0,98 ml d'une solution de soude, à 0,1955 $mol.L^{-1}$ pour 45 mg de dendrimère acide phosphonique). La solution résultante est lyophilisée pour donner le dendrimère sous forme d'une poudre blanche. Le produit final est isolé avec un rendement de 55%.
RMN $^{31}P$-{$^1H$} ($CD_3CN/D_2O$) : δ = 69,3 (s, $P_1$); 15,3 (s, $P_0$); 13,5 (s, $P(O)(OHNa)_2$) ppm. RMN $^1H$ ($CD_3CN/D_2O$) : δ = 0,9 (s large, 36H, $CH_3$); 1,20-1,50 (m, 48H, $CH_2$-$CH_2$); 2,5-3,8 (m, 78H, $CH_3$-N-P, N-$CH_2$, $CH_2$, CH); 6,5-8,0 (m, 78H, $CH_{arom}$, CH=N).
RMN $^{13}C$-{$^1H$} ($CD_3CN/D_2O$): δ = 13,4 (s, $\underline{CH_3}$); 19,6 (s, $\underline{CH_2}$-$CH_3$); 26,2 (s, $\underline{CH_2}$-$CH_2$-$CH_3$); 33,4 (s large, $CH_3$-N-$P_2$, $CH_3$-N-$P_1$); 50,5 (d, $^1J_{CP}$ = 123,0 Hz, $CH_2$-$P_2$); 54,1 (s large, N-$CH_2$-$\underline{CH_2}$- $CH_2$-$CH_3$); 66,3 (dd, $^1J_{CP}$ = 126,9 Hz, CH); 121,0 (s, $C_0^2$); 121,9 (s, $C_1^2$); 128,6 (s, $C_0^3$); 129,0 (s, $C_1^3$); 132,8 (s, $C_0^4$); 134,1 (s, $C_1^4$); 141,5 (s large, CH=N); 150,8 (s, $C_0^1$); 151,5 (d, $^2J_{CP}$ = 6,9 Hz, $C_1^1$) ppm.

**Exemple 48 : synthèse du dendrimère de deuxième génération à coeur cyclotriphosphazène à surface dérivant de l'amino-butyl bis-phosphonate**

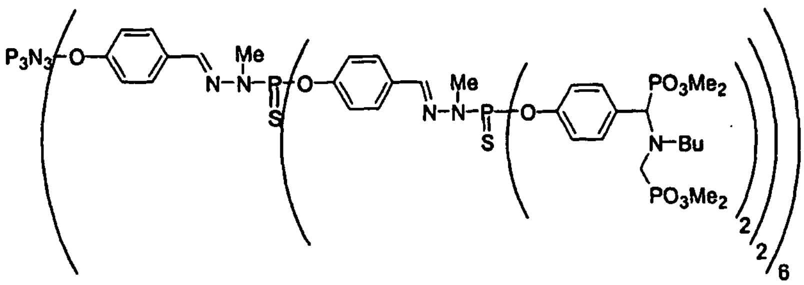

A une solution de dendrimère $Gc_2$ (0,0106 mmol, 51 mg) dans du THF anhydre (2 mL) est additionné du carbonate de césium (0,54 mmol, 176 mg) puis le phénol aza-bis-diméthyl-phosphonate dérivé de la butylamine de l'étape 3 de l'**Exemple 47** est ajouté (0,27 mmol, 110 mg). On laisse le mélange sous agitation pendant 36 heures à température ambiante puis on filtre sur célite et on centrifuge le mélange final de manière à séparer les sels. Enfin le produit final est lavé par précipitation dans du pentane et isolé avec un rendement de 75%.
RMN $^{31}P-\{^1H\}$ ($CDCl_3$) : δ = 66,1 (s, $P_1$); 65,3 (s, $P_2$); 30,8 (s, $P(O)(OMe)_2$); 28,3 (s, $P(O)(OMe)_2$); 11,4 (s, $P_0$) ppm.
RMN $^1H$ ($CDCl_3$) : δ = 0,88 (t, $^3J_{HH}$ = 7,6 Hz, 72H, $CH_3$); 1,20-1,45 (m, 96H, $CH_2$-$CH_2$); 2,33 (m, 24H, N-$\underline{CH_2}$-); 2,63 (dd, $^2J_{HP}$ = 3,4 Hz, $^2J_{HH}$ = 15,2 Hz, 24H, $\underline{CH_2}$-$P_2$); 3,16 (m, 24H, N-$\underline{CH_2}$-$CH_2$); 3,33 (m, 54H, $CH_3$-N-P); 3,44 (d, $^3J_{HP}$ = 12,0 Hz, 72H, -$P_1$OMe); 3,7 (d, $^3J_{HP}$ = 10,7 Hz, 72H, -$P_2$OMe); 3,8 (d, $^3J_{HP}$ = 10,6 Hz, 72H, -$P_2$OMe); 3,9 (d, $^3J_{HP}$ = 10,7 Hz, 72H, - $P_1$OMe); 4,5 (d, $^2J_{HP}$= 25.5 Hz, 24H, Ha); 6,9-7,8 (m, 186H, $CH_{arom}$, CH=N) ppm.
RMN $^{13}C-\{^1H\}$ ($CDCl_3$) : δ = 14,4 (s, $\underline{CH_3}$); 20,3 (s, $\underline{CH_2}$-$CH_3$); 30,6 (s, $\underline{CH_2}$-$CH_2$-$CH_3$); 33,3 (d, $^2J_{CP}$ = 11,7 Hz, $CH_3$-N-$P_2$, $CH_3$-N-$P_1$); 46,5 (dd, $^1J_{CP}$ = 166,7 Hz, $^3J_{CP}$ = 8,6 Hz, $CH_2$-$P_2$); 52,5-54,1 (m, $PO_3Me_2$); "53,1 (t, $^3J_{CP}$ = 7,8 Hz, N-$\underline{CH_2}$-$CH_2$-$CH_2$-$CH_3$) PB "; 61,3 (dd, $^1J_{CP}$ = 164,9 Hz, $^3J_{CP}$ = 10,9 Hz, CH); 121,6 (s large, $C_1^2$, $C_2^2$); 122,2 (s, $C_0^2$); 128,7 (s large, $C_2^3$); 129,3 (s large, $C_0^3$, $C_1^3$); 132,5 (d large, $^2J_{CP}$ = 7,5 Hz, $C_2^4$, $C_1^4$, $C_0^4$); 139,1 (s large, CH=N); 151,0 (d, $^2J_{CP}$= 6,9 Hz, $C_2^1$); 151,8 (s large, $C_1^1$, $C_0^1$) ppm.

**Exemple 49 : synthèse de dendrimères à surface dérivant du N-allyl-N-(4-hydroxy)-benzyl-α-amino-phosphonate de diméthyle**

*Etape 1 : synthèse d'un phénol monophosphonate*

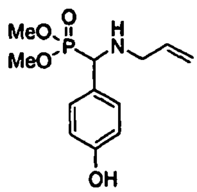

A une solution de 4-hydroxybenzaldéhyde (11 g, 0,1 mol) dans le $CH_2Cl_2$ (25 mL) sont ajoutés 10 à 15 grammes de $MgSO_4$ puis l'allylamine (7,5 mL, 0,1 mol) à 0°C. La réaction (exothermique) est maintenue à température ambiante et sous vigoureuse agitation pendant 1 nuit et le phosphite de diméthyle (9 mL, 0,1 mole) est additionné et la réaction est agitée à température ambiante pendant 3 jours. L'avancement de la réaction est suivi en RMN $^1H$ et $^{31}P$. Le mélange réactionnel est versé dans 100 mL d'eau puis extrait par 3 fois 100 mL de $CH_2Cl_2$. La phase organique est séchée sur $MgSO_4$ et le solvant est éliminé sous pression réduite. Le résidu est lavé 2 fois à l'éther jusqu'à obtention d'une huile très visqueuse jaune pâle avec un rendement 90 %.
RMN $^{31}P\{^1H\}$ ($CDCl_3$, 81,01 MHz) : δ = 29,3 (s, P=O).
RMN $^1H$ ($CDCl_3$, 200,13 MHz) : δ = 2,96 (dd, 1H, $^2J_{HH}$ = 14,0 Hz, $^3J_{HH}$ = 6,9 Hz, C**H**HCH$_2$=CH); 3,18 (dd, 1H, $^2J_{HH}$ = 14,0 Hz, $^3J_{HH}$ = 5,0 Hz, C**H**HCH$_2$=CH); 3,44 (d, 3H, $^3J_{HP}$ = 10,4 Hz, POMe); 3,65 (d, 3H, $^3J_{HP}$ = 10,5 Hz, POMe); 3,95 (d, 1H, $^2J_{HP}$ = 19,8 Hz, PCH); 5,01 (m, 2H, C**H**$_2$=CH); 5,72 (m, 1H, CH=); 6,74 (d, 2H, $^3J_{HH}$ = 8,1 Hz, $C^2$-H); 7,10 (d, 2H, $^3J_{HH}$ = 8,1 Hz, $C^3$-H).

*Etape 2 : synthèse d'un phénol bisphosphonate*

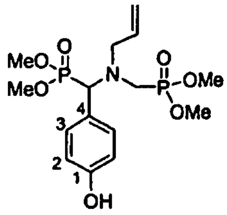

A une solution de d'α-amino-phosphonate synthétisé à l'étape précédente (970 mg, 3,58 mmol) dans le THF (5mL) est ajoutée une solution aqueuse à 37% de formaldéhyde (1,06 mL, 14,35 mmol, 4 éq.). Après environ 30 minutes le diméthylphosphite (492 µL, 5,37 mmol, 1,5 éq.) est additionné. La solution est agitée pendant 72 heures. L'avancement de la réaction est suivi en RMN $^{31}P$. Le mélange réactionnel est versé dans 30 mL d'eau puis extrait au $CH_2Cl_2$. La phase organique est séchée sur $MgSO_4$ et le solvant est éliminé sous pression réduite. Le résidu est lavé 2 fois à l'éther, séché sous vide et l'huile visqueuse est purifiée par chromatographie sur gel de silice (éluant : AcOEt / MeOH, 95:5) avec un rendement de 28 %. RMN $^{31}P\{^2H\}$ ($CDCl_3$, 81,01 MHz) : δ = 28,7 (s, P=O); 31,4 (s, P=O).
RMN $^1H$ ($CDCl_3$, 200,13 MHz) : δ = 2,57 (dd, 1H, $^2J_{HH}$ = 15,5 Hz et $^2J_{HP}$ = 2,9 Hz, PCH**H**); 2,80 (dd, 1H, $^2J_{HH}$ = 13,8Hz et $^3J_{HH}$ = 8,6 Hz, =CH-C**H**H); 3,43 (d, 3H, $^3J_{HP}$ = 10,5 Hz, POMe); 3,45 (dl, 1H, $^2J_{HH}$ = 16,0 Hz, PC**H**H); 3,69 (d, 3H, $^3J_{HP}$ = 10,7 Hz, POMe); 3,79 (d, 3H, $^3J_{HP}$ = 10,7 Hz, POMe); 3,86 (d, 3H, $^3J_{HP}$ = 10,6 Hz, POMe); 3,88 (dl, 1H, $^2J_{HH}$ = 14,0 Hz, =CH-CH**H**); 4,47 (d, 1H, $^2J_{HP}$ = 25,9 Hz, PCH); 5,20 (m, 2H, C**H**$_2$=CH); 5,79 (m, 1H, C**H**=$CH_2$); 6,82 (d, 2H, $^3J_{HH}$ = 8,4 Hz, $C^2$-H); 7,26 (d, 2H, $^3J_{HH}$ = 8,3 Hz, $C^3$-H); 9,02 (s1, OH).
RMN $^{13}C\{^1H\}$ ($CDCl_3$, 50,32 MHz) : δ = 45,2 (dd, $^1J_{CP}$ = 165,8 et $^3J_{CP}$ = 8,0 Hz, $PCH_2$); 52,62 (d, $^2J_{CP}$ = 6,9 Hz, POMe); 53,0 (d, $^2J_{CP}$ = 7,1 Hz, POMe); 53,2 (d, $^2J_{CP}$ = 7,2 Hz, POMe); 53,9 (d, $^2J_{CP}$ = 7,0 Hz, POMe); 56,1 (t, $^3J_{CP}$ = 8,2 Hz, $\underline{CH_2}$-CH=); 60,1 (dd, $^1J_{CP}$ = 163,0 Hz et $^3J_{CP}$ = 10,0 Hz, PCH); 115,6 (s, $C^2$); 118,7 (s, $H_2C$=); 120,7 (d, $^2J_{CP}$ = 5,4 Hz, $C^4$); 132,1 (d, $^3J_{CP}$ = 9,3 Hz, $C^3$); 135,6 (s, HC=); 157,6 (s, $C^1$).

*Etape 3 : Greffage sur dendrimère phosphoré de première génération*

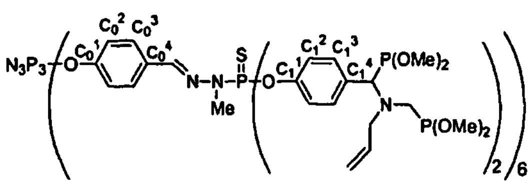

A une solution de dendrimère Gcl (223 mg, 0,122 mmol) à terminaisons $S=PCl_2$ dans le THF ou l'acétonitrile sont ajoutés 12,5 équivalents (600 mg, 1,53 mmol) de phénol fonctionnalisé (solubilisé dans l'acétonitrile ou le THF). 15 équivalents (596 mg, 1,83 mmol) de $Cs_2CO_3$ sont ensuite additionnés dans la solution et la suspension résultante est agitée jusqu'à substitution complète des chlores (suivi RMN $^{31}P$). Le mélange est décanté, le surnageant est collecté et le solide résiduel est lavé au THF. Les surnageants sont réunis et centrifugés. La solution limpide obtenue est concentrée sous pression réduite. Le résidu est dissous dans un minimum de THF puis précipité au pentane. Le solide obtenu est purifié par lavage (THF/pentane et THF/$Et_2O$) avec un rendement de 76 %.
RMN $^{31}P\{^1H\}$ ($CDCl_3$, 81,01 MHz) : δ =11,3 (s, $N_3P_3$); 27,9 (s, P=O); 30,7 (s, P=O); 65,3 (s, P=S).
RMN $^1H$ ($CDCl_3$, 250,13 MHz): δ = 2,54 (dd, 12H, $^2J_{HH}$ =15,7 Hz et $^2J_{HP}$ = 4,6 Hz, PCHH); 2,80 (dd, 12H, $^2J_{HH}$ = 13,4 Hz et $^3J_{HH}$ = 8,2 Hz, CHH-CH=); 3,29 (d, 18H, $^3J_{HP}$ = 9,9 Hz, $NCH_3$); 3,41 (d, 36H, $^3J_{HP}$ = 10,6 Hz, POMe); 3,42 (m, 12H, PCHH); 3,66 (d, 36H, $^3J_{HP}$ = 10,6 Hz, POMe); 3,76 (d, 36H, $^3J_{HP}$ = 10,7 Hz, POMe); 3,85 (d, 36H, $3J_{HP}$ = 10,6 Hz, POMe); 3,87 (dl, 12H, $^2J_{HH}$ = 13,4 Hz, CHH-CH=); 4,57 (d, 12H, $^2J_{HP}$ = 25,2 Hz, PCH); 5,17 (m, 24H, $CH_2$=); 5,74 (m, 12H, CH=); 7,02 (d, 12H, $^3J_{HH}$ = 8,2 Hz, $C_0{}^2$-H); 7,18 (d, 24H, $^3J_{HH}$ = 8,4 Hz, $C_1{}^2$-H); 7,46 (d, 24H, $^3J_{HH}$ = 8,4 Hz, $C_1{}^3$-H); 7,61 (d, 12H, $^3J_{HH}$ = 8,2 Hz, $C_0{}^3$-H); 7,62 (s, 6H, CH=N).
RMN $^{13}C\{^1H\}$ ($CDCl_3$, 62,89 MHz) : δ = 32,8 (d, $^2J_{CP}$ = 12,3 Hz, $NCH_3$); 45,1 (dd, $^1J_{CP}$ = 165,6 Hz et $^3J_{CP}$ = 8,2 Hz, $PCH_2$); 52,4 (d, $^2J_{CP}$ = 7,5 Hz, POMe); 52,7 (d, $^2J_{CP}$ = 6,1 Hz, POMe); 53,1(d, $^2J_{CP}$ = 7,4 Hz, POMe); 53,9 (d, $^2J_{CP}$ = 7,5 Hz, POMe); 56,0 (t, $^3J_{CP}$ = 8,2 Hz, $\underline{CH_2\text{-}CH=}$); 60,1 (dd, $^1J_{CP}$ = 161,7 et $^3J_{CP}$ = 9,2 Hz, PCH); 118,8 (s, $H_{2C}$=); 121,2 (dl, $^3J_{CP}$ = 4,6 Hz, $C_0{}^2$ et $C_1{}^2$); 128,3 (s, $C_0{}^3$); 128,4 (s, $C_1{}^4$); 132,0 (d, $^3J_{CP}$ = 8,6 Hz, $C_0{}^4$ et $C_1{}^3$); 135,5 (s, HC=); 139,2 (d, $^3J_{CP}$ =14,6 Hz, CH=N); 150,7 (d, $^2J_{CP}$ = 7,4 Hz, $C_1{}^1$); 151,3 (sl, $C_0{}^1$).

*Etape 4 : dendrimère phosphoré de première génération à extrémités acide bisphosphonique (sel de Na)*

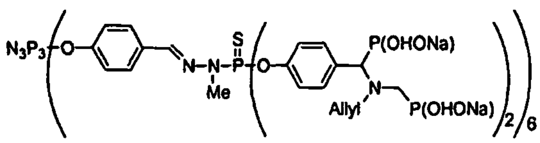

A une solution de dendrimère à terminaisons phosphonate de diméthyle synthétisé à l'étape précédente (200 mg, 32,7 μmol) dans l'acétonitrile est additionné à 0°C sous flux d'argon 60 équivalents de bromo-triméthylsilane (260 μL, 1,964 mmol). La solution est agitée 18 heures à température ambiante puis concentrée sous pression réduite. 5 mL de méthanol sont ajoutés et le mélange est vigoureusement agité pendant 2 heures. Le méthanol est éliminé sous pression réduite et le résidu est lavé avec de l'éther distillé, puis de l'eau et du méthanol. Le solide est séché sous pression réduite jusqu'à obtention d'une poudre. Une solution de soude (0,1966 M, 3,99 mL, 0,784 mmol, 24 éq.) est additionnée lentement sur le solide. La solution obtenue est filtrée puis lyophilisée. Le dendrimère à terminaisons acide amino bis-phosphonique est obtenu sous forme d'une poudre blanche avec un rendement de 79%. RMN $^{31}P\{^1H\}$ ($D_2O/CD_3CN$, 81,01 MHz) : δ = 11,0 (s, P=O); 11,3 (s, P=O); 12,7 (s, $N_3P_3$); 66,7 (s, P=S).

**Exemple 50 : synthèse de dendrimères à surface dérivant du N-benzyl-N-(4-hydroxy)-benzyl-α-amino-bis-phosphonate de diméthyle.**

*Etape 1 : synthèse d'un phénol monophosphonate*

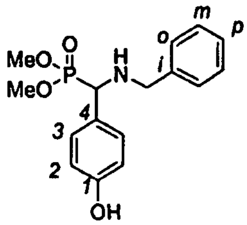

A une solution de 4-hydroxybenzaldéhyde (4,4 g; 40 mmol) dans le THF (30 mL) sont ajoutés plusieurs grammes de $MgSO_4$ puis la benzylamine (4,36 mL; 40 mmol). La réaction (exothermique) est maintenue à température ambiante et sous vigoureuse agitation pendant 2 heures. Après décantation, le surnageant est canulé puis concentré (à environ 50% de son volume initial) sous pression réduite. Le phosphite de diméthyle (3,66 mL; 40 mmol) est additionné et la réaction est chauffée sous argon à 50°C pendant 48 heures (ou agitée à température ambiante pendant 5 jours). L'avancement de la réaction est suivi en RMN $^1H$ et $^{31}P$. Le mélange réactionnel est versé dans une solution saturée de $NaHCO_3$ puis extrait par 3 fois 50 mL de $CH_2Cl_2$. La phase organique est séchée sur $MgSO_4$ et le solvant est éliminé sous pression réduite. Le résidu est lavé 2 fois à l'éther jusqu'à obtention d'un solide jaune pâle avec un rendement de 77 %.
RMN $^{31}P\{^1H\}$ ($CDCl_3$, 121,4 MHz) : δ = 29,7 (s, P=O).
RMN $^1H$ ($CDCl_3$, 200,13 MHz) : δ = 3,54 (d, 1H, $^2J_{HH}$ = 13,2Hz, CHHPh); 3,58 (d, 3H, $^3J_{HP}$ = 10,5 Hz, POMe); 3,80 (d, 3H, $^3J_{HP}$ = 10,5 Hz, POMe); 3,81 (d, 1H, $^2J_{HH}$ = 13,2 Hz, CHHPh); 3,99 (d, 1H, $^2J_{HP}$ = 19,8 Hz, PCH); 6,77 (d, 2H, $^3J_{HH}$ = 8,4 Hz, $C^2$-H); 7,19 (d, 2H, $^3J_{HH}$ = 8,4 Hz, $C^3$-H); 7,28 (m, 5H, $C_6H_5$); 8,50 (sl, 1H, OH).
RMN $^{13}C\{^1H\}$ ($CDCl_3$, 62,89 MHz) : δ = 50,8 (d, $^3J_{CP}$ = 17,5 Hz, $CH_2N$); 53,6 (d, $^2J_{CP}$ = 7,6 Hz, POMe); 54,1 (d, $^2J_{CP}$ = 7,8 Hz, POMe); 58,3 (d, $^1J_{CP}$ = 157,3 Hz, PCH); 116,1 (s, $C^2$); 125,1 (sl, $C^4$); 127,2 (s, $C_p$); 128,4 (s, $C_m$, $C_o$); 129,6 (d, $^2J_{CP}$ = 5,9 Hz, $C^3$); 139,1(s, $C_i$); 157,1 (s, $C^1$).

*Etape 2 : synthèse d'un phénol bisphosphonate*

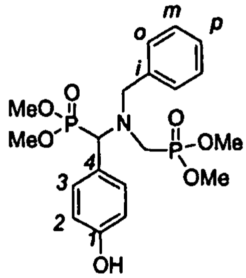

A une solution d'α-aminophosphonate synthétisé à l'étape précédente (2,5 g; 7,79 mmol) dans le THF (50 mL) est ajoutée une solution aqueuse à 37% de formaldéhyde (870 μL; 11,7 mmol; 1,5 éq.). Après 30 minutes le diméthylphosphite est additionné (785 μL; 8,56 mmol; 1,1 éq). La solution est agitée pendant 24 heures et 600 μL de formaldéhyde (solution aqueuse à 37%) sont ajoutés. L'avancement de la réaction est suivi en RMN $^{31}P$. Après 96 heures de réaction, le mélange réactionnel est versé dans une solution saturée de $NaHCO_3$ puis extrait au $CH_2Cl_2$. La phase organique est séchée sur $MgSO_4$ et le solvant est éliminé sous pression réduite. Le résidu est lavé 2 fois à l'éther, deux fois avec un mélange THF/pentane, séché sous vide et il est purifié par chromatographie sur gel de silice (éluant : AcOEt / MeOH; 95:5) pour donner une huile visqueuse avec un rendement de 65 %.
RMN $^{31}P\{^1H\}$($CDCl_3$, 81,01 MHz) : δ = 31,5 (s); 28,9 (s).
RMN $^1H$ ($CDCl_3$, 200,13 MHz) : δ = 2,70 (dd, 1H, $^2J_{HH}$ = 15,5 Hz et $^2J_{HP}$ = 3,4 Hz, CHHP); 3,29 (d, 1H, $^2J_{HH}$ =13,4 Hz, CHHPh); 3,44 (d, 3H, $^3J_{HP}$ =10,5 Hz, POMe); 3,49 (dd, 1H, $^2J_{HH}$ = 15,5 Hz et $^2J_{HP}$ = 16 Hz, CHHP); 3,65 (d, 3H, $^3J_{HP}$ = 10,6 Hz, POMe); 3,74 (d, 3H, $^3J_{HP}$ = 10,7 Hz, POMe); 3,85 (d, 3H, $^3J_{HP}$ = 10,8 Hz, POMe); 4,42 (d, 1H, $^2J_{HP}$ = 25,6 Hz, PCH); 4,44 (d, 1H, $^2J_{HH}$ = 13,4 Hz, CHHPh); 6,85 (d, 2H, $^3J_{HH}$ = 8,4 Hz, $C^2$-H); 7,35 (m, 7H, $H_{arom}$); 8,80 (sl, 1H, OH).
RMN $^{13}C\{^1H)$ ($CDCl_3$, 62,89 MHz) : δ = 45,2 (dd, $^1J_{CP}$ = 165,3 Hz et $^3J_{CP}$ = 7,5 Hz, $PCH_2$); 52,3 (d, $^2J_{CP}$ = 6,2 Hz, POMe); 52,9 (d, $^2J_{CP}$ = 9,2 Hz, POMe); 53,1 (d, $^2J_{CP}$ = 8,2 Hz, POMe); 53,5 (d, $^2J_{CP}$ = 6,3 Hz, POMe); 57,1 (sl, $\underline{CH_2}$Ph); 59,5 (dd, $^1J_{CP}$ = 163,4 Hz et $^3J_{CP}$ = 10,3 Hz, PCH); 115,5 (s, $C^2$); 120,4 (d, $^2J_{CP}$ = 4,3 Hz, $C^4$); 127,4 (s, $C_p$); 128,3 (s, $C_m$); 129,2 (s, $C_o$); 132,1 (d, $^3J_{CP}$ = 8,9 Hz, $C^3$); 138,2 (s, $C_i$); 157,9 (s, $C^1$).

*Etape 3 : greffage sur un dendrimère phosphoré de première génération*

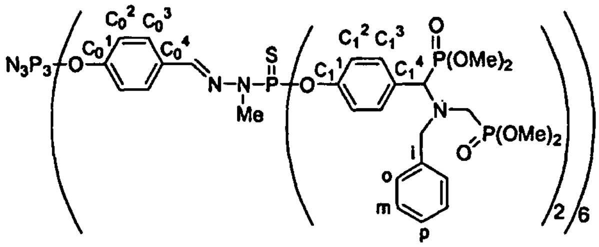

A une solution de dendrimère $Gc_1$ à terminaison S=$PCl_2$ (181 mg; 99 μmol) dans le THF ou l'acétonitrile (10 mL) sont ajoutés 12,6 équivalents (560 mg; 1,26 mmol) du phénol fonctionnalisé obtenu à l'étape précédente (solubilisé dans l'acétonitrile ou le THF). 15 équivalents (490 mg; 1,50 mmol) de $Cs_2CO_3$ sont ensuite additionnés dans la solution et la suspension résultante est agitée jusqu'à substitution complète des chlores (72 heures, suivi en RMN $^{31}P$). Le mélange est décanté, le surnageant est collecté et le solide résiduel est lavé au THF. Les surnageants sont réunis et centrifugés. La solution limpide obtenue est concentrée sous pression réduite. Le résidu est dissous dans un minimum de THF puis précipité au pentane et finalement purifié par lavage (THF / pentane; THF / $Et_2O$; $Et_2O$) pour donner un solide blanc avec un rendement de 90 %.
RMN $^{31}P\{^1H\}$ ($CDCl_3$, 101,2 MHz) : δ =11,3 (s, $N_3P_3$); 28,2 (s, P=O); 30,6 (s, P=O); 65,3 (s, P=S).
RMN $^1H$ ($CDCl_3$, 250,13 MHz) : δ = 2,64 (dd, 12H, $^2J_{HH}$ = 15,8 Hz et $^2J_{HP}$ = 3,4 Hz, CHHP); 3,27 (d, 12H, $^2J_{HH}$ = 12,5 Hz, CHHPh); 3,32 (d, 18H, $^3J_{HP}$ = 9,7 Hz, NMe); 3,39 (d, 36H, $^3J_{HP}$ = 10,6 Hz, POMe); 3,46 (m, 12H, PCHH); 3,58 (d, 36H, $^3J_{HP}$ = 10,7 Hz, POMe); 3,67 (d, 36H, $^3J_{HP}$ = 10,7 Hz, POMe); 3,83 (d, 36H, $^3J_{HP}$ = 10,7 Hz, POMe); 4,44 (d, 12H, $^2J_{HH}$ = 12,5 Hz, CHHPh); 4,52 (d, 12H, $^2J_{HP}$ = 24,9Hz, CHP); 7,04 (d, 12H, $^3J_{HP}$ = 8,4 Hz, $C_0^2$-H); 7,28 (m, 84H, $C_6H_5$, $C_1^2$-H); 7,49 (d, 24H, $^3J_{HP}$ = 8,2 Hz, $C_1^3$-H); 7,63 (m, 18H, $C_0^3$-H et CH=N).
RMN $^{13}C\{^1H\}$ ($CDCl_3$, 62,89 MHz) : δ = 32,8 (d, $^2J_{CP}$ = 11,4 Hz, $NCH_3$); 45,2 (dd, $^1J_{CP}$ = 164,6 et $^3J_{CP}$ = 8,4 Hz, $PCH_2$); 52,3 (d, $^2J_{CP}$ = 6,4 Hz, POMe); 52,7 (d, $^2J_{CP}$ = 5,8 Hz, POMe); 52,9 (d, $^2J_{CP}$ = 6,4 Hz, POMe); 53,6 (d, $^2J_{CP}$ = 7,6 Hz, POMe); 57,8 (t, $^3J_{CP}$ = 8,4 Hz, $\underline{CH_2}$-Ph); 59,7 (dd, $J_{CP}$ = 160,8 et $^3J_{CP}$ = 9,4 Hz, PCH); 121,3 (dl, $^3J_{CP}$ = 3,3 Hz, $C_0^2$ et

$C_1^2$); 127,5 (s, $C_p$); 128,4 (sl, $C_0^3$, $C_1^4$, $C_m$); 129,3 (s, $C_o$); 132,0 (s, $C_0^4$); 132,2 (d, $^3J_{CP}$ = 8,4 Hz, $C_1^3$); 138,2 (s, $C_i$); 139,1 (sl, CH=N); 150,8 (d, $^2J_{CP}$ = 7,2 Hz, $C_1^1$); 151,1 (sl, $C_0^1$).

*Etape 4 : dendrimère phosphoré de première génération à extrémités acide amino bis-phosphonique (sel de sodium)*

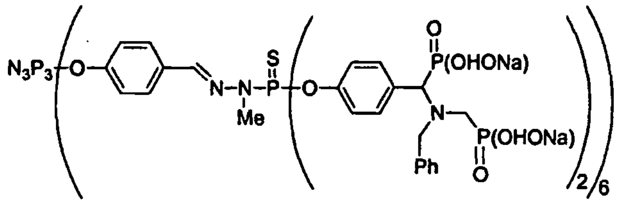

A une solution de dendrimère à terminaisons phosphonate de diméthyle obtenu à l'étape précédente (160 mg; 23,8 µmol) dans l'acétonitrile (5 mL) est additionné à 0°C sous flux d'argon 60 équivalents (190 µL; 1,430 mmol) de bromotriméthylsilane. La solution est agitée 16 heures à température ambiante puis concentrée sous pression réduite. 5 mL de méthanol sont ajoutés et le mélange est vigoureusement agité pendant 2 heures. Le méthanol est éliminé sous pression réduite et le résidu est lavé avec de l'éther distillé, puis de l'eau et du méthanol. Le solide est séché sous pression réduite jusqu'à obtention d'une poudre. Une solution de soude (0,1966 M, 2,07 mL, 24 éq.) est additionnée lentement sur le solide. La solution obtenue est filtrée puis lyophilisée. L'acide amino bis-phosphonique est obtenu sous forme d'une poudre blanche avec un rendement de 71%.
RMN $^{31}P\{^1H\}$ ($D_2O/CD_3CN$, 81,01 MHz) : δ = 11,8 (si, $N_3P_3$ et P=O), 67,1 (sl, P=S).

**Exemple 51 : synthèse d'acides carboxyliques aza-bis-phosphonate**

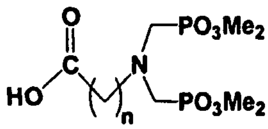

n =1 ou 3

(a) n = 1

*Synthèse de l'acide acétique amino [bis-{diméthoxy-phosphorylméthyl)}*

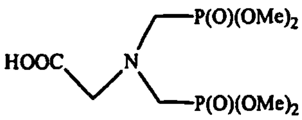

5g d'acide aminoacétique (66,6 mmol) sont introduits dans un ballon et dissous dans 20 mL de THF. 3 équivalents de formaldéhyde à 37% en solution aqueuse sont ajoutés à température ambiante et agités pendant 30 minutes. On additionne alors 4 équivalents de diméthylphosphite. Le mélange est maintenu sous agitation magnétique à température ambiante pendant 12 h, 40 mL d'eau distillée sont ajoutés au milieu réactionnel, le THF est éliminé sous pression réduite et le produit est extrait avec 3 x 100 mL de chloroforme. La phase organique est séchée sur sulfate de magnésium puis évaporée. L'acide acétique amino bisphosphonate est alors purifié par chromatographie sur colonne de silice en éluant avec un mélange $CH_2Cl_2$ / MeOH (95 / 5), et isolé sous forme de poudre blanc cassé avec un rendement de 37 %.
Rf ($CH_2Cl_2$ / MeOH : 95 / 5) = 0,32
RMN $^{31}P$-{$^1H$} ($CDCl_3$) δ = 30,0 ppm.
RMN $^1H$ ($CDCl_3$) δ = 3,22 (d, $^2J_{HP}$ = 10,1 Hz, 4H, $CH_2$-P), 3,61 (s, 2H, $CH_2$-CO), 3,68 (d, $^3J_{HP}$ = 10,6 Hz, 12H, O-$CH_3$), 10,8 (s, 1H, COOH) ppm.
RMN$^{13}C$-{$^1H$} ($CDCl_3$) δ = 49,8 (dd, $^1J_{CP}$ = 162,1 Hz, $^3J_{CP}$ = 9,9 Hz, $CH_2$-P), 53,0 (d, $^2J_{CP}$ = 5,9 Hz, $CH_3$-O), 55,7 (t, $^3J_{CP}$

= 5,8 Hz, N-$CH_2$-CO), 171,9 (s, COOH) ppm.

(b) n = 3

*Synthèse de l'acide butyrique amino [bis-(diméthoxy-phosphorylméthyl)]*

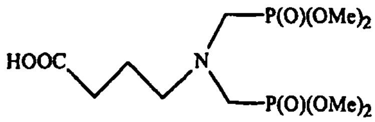

5g d'acide aminé (48,5 mmol) sont introduits dans un ballon et dissous dans 20 mL de THF, 3 équivalents de formaldéhyde à 37% en solution aqueuse sont ajoutés à température ambiante et agités pendant 30 minutes. On additionne alors 4 équivalents de diméthylphosphite. Le mélange est maintenu sous agitation magnétique à température ambiante pendant 12 h, 40 mL d'eau distillée sont ajoutés au milieu réactionnel, le THF est éliminé sous pression réduite et le produit est extrait avec 3 x 100 mL de chloroforme. La phase organique est séchée sur sulfate de magnésium puis évaporée. Le produit est alors purifié par chromatographie sur colonne de silice en éluant avec un mélange $CH_2Cl_2$ / MeOH (95 / 5), et isolé sous forme de poudre blanc cassé avec un rendement de 53%.
Rf ($CH_2Cl_2$ / MeOH : 95 / 5) = 0,35
RMN $^{31}P$-{$^1H$} ($CDCl_3$) $\delta$ = 30,7 ppm.
RMN $^1H$ ($CDCl_3$) $\delta$ = 1,74 quint, $^3J_{HH}$ = 7,1 Hz, 2H, $CH_2$-C$\underline{H_2}$-$CH_2$), 2,36 (t, $^3J_{HH}$ = 7,1 Hz, 2H, HOOC-C$\underline{H_2}$), 2,78 (t, $^3J_{HH}$ = 7,1 Hz, 2H, $CH_2$-C$\underline{H_2}$-N), 3,10 (d, $^2J_{HP}$ = 8,8 Hz, 4H, $CH_2$-P), 3,74 (d, $^3J_{HP}$ = 10,7 Hz, 12H, O-$CH_3$) ppm. Proton COOH non observé.
RMN $^{13}C$-{$^1H$} ($CDCl_3$) $\delta$= 22,6 (s, $\underline{C}H_2$-$CH_2$-$CH_2$), 31,1 (s, HOOC-$\underline{C}H_2$), 49,3 (dd, $^1J_{CP}$ = 158,0 Hz, $^3J_{CP}$ = 7,3 Hz, $CH_2$-P), 52,8 (d, $^2J_{CP}$ = 7,2 Hz, $CH_3$-O), 56,0 (t, $^3J_{CP}$ = 7,5 Hz, N-$\underline{C}H_2$- $CH_2$), 176,1 (s, COOH) ppm.

**Exemple 52 : clivage des esters diméthylphosphoniques de l'Exemple 51 en acides phosphoniques**

(a) n = 1 :

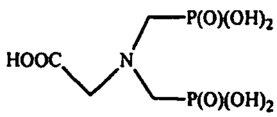

0,39 mmol de composé aza-bis-phosphonate sont dissous dans 4 mL d'acétonitrile et 2,33 mmol de BrTMS (5,5 équivalents) sont ajoutés goutte à goutte à 0°C sous atmosphère inerte. Le mélange est agité 30 minutes à 0°C puis 15h à température ambiante. L'acétonitrile est éliminé sous pression réduite puis 3 mL de méthanol sont additionnés. Le mélange est agité 30 minutes puis le solvant est évaporé. On ajoute 5 mL d'eau distillée et on maintient l'agitation pendant 1 heure à température ambiante puis la solution est lyophilisée. Le résidu sec est lavé 3 fois à l'éther. Le produit est obtenu sous la forme d'une poudre jaune avec un rendement de 83%.
RMN $^{31}P$-{$^1H$} ($D_2O$) $\delta$ = 10,6 ppm.
RMN $^{13}C$-{$^1H$) ($CD_3OD$) $\delta$ = 51,9 (dd, $^1J_{CP}$ = 148,5 Hz, $^3J_{CP}$ = 13,2 Hz, $CH_2$-P), 55,9 (s, N-$\underline{C}H_2$-CO), 168,1 (s, COOH) ppm.

(b) n = 3 :

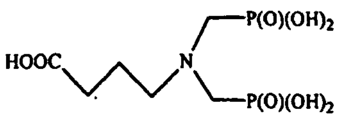

0,39 mmol de composé aza-bis-phosphonate sont dissous dans 4 mL d'acétonitrile et 2,33 mmol de BrTMS (5,5 équivalents) sont ajoutés goutte à goutte à 0°C sous atmosphère inerte. Le mélange est agité 30 minutes à 0°C puis 15h à température ambiante. L'acétonitrile est éliminé sous pression réduite puis 3 mL de méthanol sont additionnés. Le mélange est agité 30 minutes puis le solvant est évaporé. On ajoute 5 mL d'eau distillée et on maintient l'agitation pendant 1 heure à température ambiante puis la solution est lyophilisée. Le résidu sec est lavé 3 fois à l'éther. Le produit

est obtenu sous la forme d'une poudre jaune avec un rendement de 78%.
RMN $^{31}P-\{^{1}H\}$ ($D_2O$) $\delta$ = 11,3 ppm.
RMN $^{13}C-\{^{1}H\}$ ($D_2O$) $\delta$ = 21,4 (s, $CH_2$-$CH_2$-$CH_2$), 33,1 (s, CO-$CH_2$-), 53,8 (dd, $^{1}J_{CP}$ = 130,0 Hz, $^{3}J_{CP}$ = 4,1 Hz, $CH_2$-P), 58,8 (s, N-$CH_2$-$CH_2$), 179,4 (s, COOH) ppm.

**Exemple 53 : synthèse de composés amido-tyramine-azabis-phosphonate**

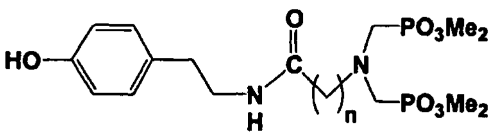

n =1 ou 3

(a) n = 1

*Couplage de l'acide acétique amino [bis-(diméthoxy phosphorylméthyl)] avec la tyramine*

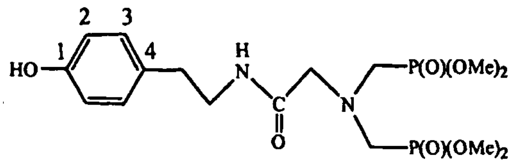

300 mg d'acide carboxylique (0,94 mmol) obtenu dans **l'Exemple 51** (n = 1), sont introduits dans un ballon sous argon et dissous dans 5 mL de DMF sec. La solution est placée à 0°C, on lui ajoute alors 1,3 équivalents de HOBt, l'agitation est maintenue pendant 15 minutes à 0°C puis on additionne 1,3 équivalents de DCC. Le mélange est agité pendant 30 minutes à 0°C puis pendant 1h à température ambiante. On observe la formation d'un précipité. Le mélange est replacé à 0°C puis on ajoute la tyramine (1,1 équivalents) et on agite 30 minutes à 0°C puis 15h à température ambiante. Le précipité est éliminé sur filtre millipore 5$\mu$ et la solution est lyophilisée. L'huile résiduelle est purifiée par chromatographie sur colonne de silice en éluant par un mélange $CH_2Cl_2$ / MeOH (90 / 10), Rf = 0,47, le produit est obtenu avec un rendement de 42% sous forme d'une poudre blanc cassé.
RMN, $^{31}P-\{^{1}H\}$ ($CDCl_3$) $\delta$ = 30,2 ppm.
RMN $^{1}H$ ($CDCl_3$) $\delta$ = 2,73 (t, $^{3}J_{HH}$ = 7,4 Hz, 2H, $C_6H_4$-C$\underline{H_2}$), 3,13 (d, $^{2}J_{HP}$ = 9,0 Hz, 4H, $CH_2$-P), 3,33-3,52 (m, 4H, CO-$CH_2$-N, C$\underline{H_2}$-NH), 3,74 (d, $^{3}J_{HP}$ = 10,7 Hz, 12H, $CH_3$-O), 6,75 (d, $^{3}J_{HH}$ = 8,4 Hz, 2H, $HC^2$) , 7,00 (d, $^{3}J_{HH}$ = 8,4 Hz, 2H, $HC^3$), 7,46 (t, $^{3}J_{HH}$ = 5,8 Hz, 1H, NH) ppm.
RMN $^{13}C-\{^{1}H\}$ ($CDCl_3$) $\delta$ = 34,7 (s, $C_6H_4$-$CH_2$),40,7 (s, $CH_2$-NH), 49,9 (dd, $^{1}J_{CP}$ = 158,9 Hz, $^{3}J_{CP}$ = 3,7 Hz, $CH_2$-P), 52,8 (d, $^{2}J_{CP}$ = 3,7 Hz, $CH_3$-O), 60,7 (t, $^{3}J_{CP}$ = 6,4 Hz, CO-$CH_2$-N), 115,4 (s, $C^2$), 129,7 (s, $C^3$, $C^4$), 155,4 (s, C'), 169,7 (s, CONH) ppm.

(b) n = 3

*Couplage de l'acide butyrique amino [bis-(diméthoxy-phosphorylméthyl)] avec la tyramine*

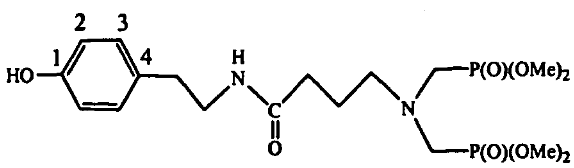

300 mg d'acide carboxylique (0,86 mmol) obtenu dans **l'Exemple 51** (n = 3), sont introduits dans un ballon

sous argon et dissous dans 5 mL de DMF sec. La solution est placée à 0°C, on lui ajoute alors 1,3 équivalents de HOBt, l'agitation est maintenue pendant 15 minutes à 0°C puis on additionne 1,3 équivalents de DCC. Le mélange est agité pendant 30 minutes à 0°C puis pendant 1h à température ambiante. On observe la formation d'un précipité. Le mélange est replacé à 0°C puis on ajoute la tyramine (1,1 équivalents) et on agite 30 minutes à 0°C puis 15h à température ambiante. Le précipité est éliminé sur filtre millipore 5µ et la solution est lyophilisée. L'huile résiduelle est purifiée par chromatographie sur colonne de silice en éluant par un mélange $CH_2Cl_2$ / MeOH (95 / 5), Rf = 0,52. Le produit est obtenu avec un rendement de 51%.
RMN $^{31}P-\{^1H\}$ ($CDCl_3$) $\delta$ = 30,6 ppm.
RMN $^1H$ ($CDCl_3$) $\delta$ = 1,71 (quint, $^3J_{HH}$ = 6,8 Hz, 2H, $CH_2-C\underline{H_2}-CH_2$), 2,20 (t, $^3J_{HH}$ = 6,8 Hz, 2H, $CO-C\underline{H_2}-CH_2-CH_2$), 2,69 (m, 4H, $CO-CH_2-CH_2-C-\underline{H_2}$, $C_6H_4-C\underline{H_2}$), 3,08 (d, $^2J_{HP}$ = 8,7 Hz, 4H, $CH_2-P$), 3,42 (td, $^3J_{HH}$ = 7,1 Hz, 2H, $C\underline{H_2}-NH$), 3,75 (d, $^3J_{HP}$ = 10,5 Hz, 12H, $CH_3-O$), 6,67 (t, $^3J_{HH}$ = 7,1 Hz, 1H, NH), 6,76 (d, $^3J_{HH}$ = 8,4 Hz, 2H, $H_{Ar}$), 6,98 (d, $^3J_{HH}$ = 8,4 Hz, 2H, $H_{Ar}$), 8,34 (s, OH) ppm.

RMN $^{13}C-\{^1H\}$ ($CDCl_3$) $\delta$ = 23,9 (s, $CH_2-\underline{C}H_2-CH_2$), 33,4 (s, $CO-\underline{C}H_2-CH_2-CH_2$), 34,6 (s, $C_6H_4-\underline{C}H_2$), 40,8 (s, $CH_2-NH$), 49,5 (dd, $^1J_{CP}$ = 159,4 Hz, $^3J_{CP}$ = 7,0 Hz, $CH_2-P$), 52,8 (d, $^2J_{CP}$ = 6,2 Hz, $CH_3-O$), 56,0 (t, $^3J_{CP}$ = 7,8 Hz, $CO-CH_2-CH_2-\underline{C}H_2$), 115,5 (s, $C^2$), 129,7 (s, $C^3$), 129,7 (s, $C^4$), 155,4 (s, $C^1$), 173,6 (s, CONH) ppm.

**Exemple 54: tyramines à extrémité [(carbamoylméthyl-amino)-méthyl]-acide phosphonique (sel de Na) obtenues à partir des produits de l'Exemple 53**

(a) n = 1:

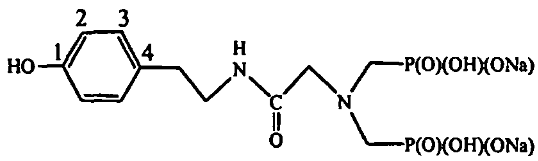

0,2 mmol de produit de couplage obtenu dans **l'Exemple 53** partie (a) sont mis en solution à 0°C dans 3 mL d'acétonitrile distillé. On additionne alors à la seringue, 5,5 équivalents de BrTMS. L'agitation est maintenue pendant 15 minutes à 0°C puis une nuit à température ambiante. Le mélange est alors tiré sous vide et méthanolysé puis hydrolysé à température ambiante. Après lyophilisation le résidu sec est lavé trois fois à l'éther pour donner l'acide phosphonique correspondants. Le sel de sodium est préparé par addition de 2 équivalents d'une solution aqueuse de soude 0,1955 N et isolé avec un rendement quantitatif sous forme d'une poudre blanc cassé.
RMN $^{13}P-\{^1H\}$ ($D_2O$/THFd8) $\delta$ = 19,9 ppm.
RMN $^{13}C-\{^1H\}$ ($D_2O$/THFd8) $\delta$ = 36,7 (s, $C_6H_4-\underline{C}H_2$), 44,1 (s, $CH_2-NH$), 58,1 (dd, $^1J_{CP}$ = 147,2 Hz, $^3J_{CP}$ = 14,5 Hz, $CH_2-P$), 63,3 (t, $^3J_{CP}$ = 8,7 Hz, $CO-\underline{C}H_2-N$), 121,4 (s, $C^2$), 127,6 (s, $C^4$), 132,5 (s, $C^3$), 167,0 (s, C'), 177,4 (s, CONH) ppm.

(b) n = 3:

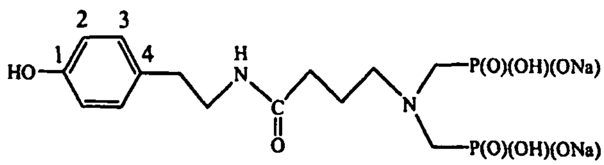

0,2 mmol de produit de couplage obtenu dans **l'Exemple 53** partie (b) sont mis en solution à 0°C dans 3 mL d'acétonitrile distillé. On additionne alors à la seringue, 5,5 équivalents de BrTMS. L'agitation est maintenue pendant 15 minutes à 0°C puis une nuit à température ambiante. Le mélange est alors tiré sous vide et méthanolysé puis hydrolysé à température ambiante. Après lyophilisation le résidu sec est lavé trois fois à l'éther pour donner les acides phosphoniques correspondants. Le sel de sodium est préparé par addition de 2 équivalents d'une solution aqueuse de soude 0,1955 N et isolé avec un rendement quantitatif sous forme d'une poudre blanc cassé.
RMN, $^{31}P-\{^1H\}$ ($D_2O$) $\delta$ = 10,9 ppm.

RMN $^{13}C$-{$^1H$} ($D_2O$) $\delta$ = 22,6 (s, $CH_2$-$\underline{C}H_2$-$CH_2$), 35,1 (s, CO-$\underline{C}H_2$-$CH_2$-$CH_2$), 36,2 (s, $C_6H_4$-$\underline{C}H_2$), 43,3 (s, $CH_2$-NH), 54,1 (d, $^1J_{CP}$ = 136,5 Hz, $CH_2$-P), 59,0 (s, CO-$CH_2$-$CH_2$-$\underline{C}H_2$), 118,1 (s, $C^2$), 133,0 (s, $C^3$), 133,8 (s, $C^4$), 156,6 (s, $C^1$), 177,0 (s, CONH) ppm.

**Exemple 55: synthèse de modèles de dendrimères DAB à extrémités aza-bis-phosphonique dérivé de la glycine**

*Etape 1 : Couplage de l'acide acétique amino [bis-(diméthoxy-phosphorylméthyl)] avec la tris-(2-aminoéthylamine) (modèle de dendrimère DAB)*

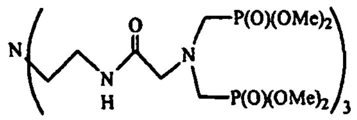

1,57 mmol d'acide carboxylique aza-bis-phosphonate obtenu dans **<u>l'Exemple 51</u>** (n = 1) sont introduits dans un ballon sous argon et dissous dans 5 mL de DMF sec. La solution est placée à 0°C, on lui ajoute alors 1,1 équivalents de 1-hydroxybenzotriazole (HOBt), l'agitation est maintenue pendant 15 minutes à 0°C puis on additionne 1,1 équivalents de 1,3-dicyclohexylcarbodiimide (DCC). Le mélange est agité pendant 30 minutes à 0°C puis pendant 1h à température ambiante. On observe la formation d'un précipité. On ajoute alors 0,4 mmol de tris-(2-aminoethylamine) en solution dans 1 mL de DMF sec à 0°C, l'agitation est maintenue 15 minutes à 0°C puis une nuit à température ambiante. Le précipité est éliminé par filtration sur filtre seringue millipore 5µ puis la solution est lyophilisée. Le produit est purifié par dissolution dans un volume minimum de $CH_2Cl_2$ et précipitation dans un grand volume de diéthyléther. Ces précipitations sont répétées trois fois pour éliminer les traces de HOBt. Le produit est obtenu avec un rendement de 55% sous forme de poudre blanc cassé.
RMN $^{3l}P$-{$^1H$} ($CDCl_3$) $\delta$ = 30,3 ppm.
RMN $^1H$ ($CDCl_3$) $\delta$ = 2,67 (sl, 6H, N-$CH_2$), 3,20 (d, $^2J_{HP}$ = 9,3 Hz, 12H, P-$CH_2$), 3,28 (sl, 6H, $C\underline{H}_2$-NHCO), 3,47 (s, 6H, N-$CH_2$-CO), 3,73 (d, $^3J_{HP}$ = 10,6 Hz, 36H, O-$CH_3$), 7,65 (sl, 3H, CONH) ppm.
RMN $^{13}C$-{$^1H$} ($CDCl_3$) $\delta$ = 36,9 (s, N-$\underline{C}H_2$-$CH_2$), 49,8 (dd, $^1J_{CP}$ = 158,2 Hz, $^3J_{CP}$ = 6,3 Hz, $CH_2$-P), 52,7 (d, $^2J_{CP}$ = 3,4 Hz, $CH_3$-O), 53,7 (s, N-$CH_2$-$\underline{C}H_2$), 59,9 (t, $^3J_{CP}$ = 6,4 Hz, N-$\underline{C}H_2$-CO), 170,1 (s, CONH) ppm.

*Etape 2 :* synthèse du modèle de dendrimère DAB à extrémités acide aza-bis-phosphonique dérivé de la glycine

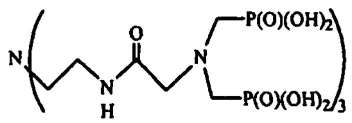

0,25 mmol de composé modèle de DAB azabis-phosphonate de l'étape 1 sont mis en solution dans 3 mL d'acétonitrile et placés à 0°C puis on additionne goutte à goutte 3,75 mmol de BrTMS sous atmosphère inerte. Après 30 minutes d'agitation à 0°C le bain de glace est enlevé et l'agitation est maintenue pendant 15h à température ambiante. Le solvant est éliminé sous pression réduite et 3 mL de MeOH sont ajoutés sur le résidu sec. On agite 30 minutes puis on élimine le solvant sous vide et on additionne 3 mL d'eau distillée. Après 1 heure d'agitation le mélange est lyophilisé. Le résidu sec est lavé 3 fois à l'éther sec. Le produit est obtenu sous la forme d'une poudre beige.
RMN $^{31}P$-{$^1H$} ($D_2O$, THFd8) $\delta$ = 11,2 ppm.

*Etape 3 : Modèle de dendrimère DAB à extrémités [(carbamoylméthyl-amino)-méthyl]-acide phosphonique (sel de Na)*

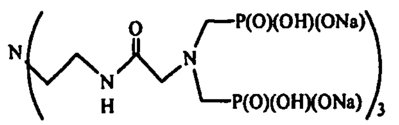

Le sel de sodium est obtenu par addition de 1 équivalent d'une solution aqueuse de soude 0,1955 N par groupement $-PO_3H_2$ présent dans la molécule obtenue dans l'étape 2. Le produit est isolé sous forme d'une poudre blanc cassé après lyophilisation avec un rendement de 67%.
RMN $^{31}P-\{^1H\}$ ($D_2O$/THFd8) $\delta$ = 17,8 ppm.
RMN $^{13}C-\{^1H\}$ ($D_2O$/THFd8) $\delta$ = 36,3 (s, N-$\underline{C}H_2$-$CH_2$), 54,2 (s, N-$CH_2$-$\underline{C}H_2$), 57,2 (dd, $^1J_{CP}$ = 149,0 Hz, $^3J_{CP}$ = 9,8 Hz, $CH_2$-P), 62,6 (s1, N-$\underline{C}H_2$-CO), 174,8 (s, CONH) ppm.

**Exemple 56: synthèse de modèles de dendrimères DAB à extrémités aza-bis-phosphonique dérivé de l'acide aminobutyrique**

*Etape 1 : Couplage de l'acide butyrique amino [bis-(diméthoxy-phosphorylméthyl)] avec la tris-(2-aminoéthylamine) (modèle de dendrimère DAB)*

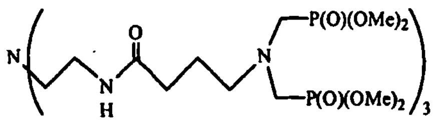

1,57 mmol d'acide carboxylique aza-bis-phosphonate obtenu dans **l'Exemple 51** (n = 3) sont introduits dans un ballon sous argon et dissous dans 5 mL de DMF sec. La solution est placée à 0°C, on lui ajoute alors 1,1 équivalents de 1-hydroxybenzotriazole (HOBt), l'agitation est maintenue pendant 15 minutes à 0°C puis on additionne 1,1 équivalents de 1,3-dicyclohexylcarbodiimide (DCC). Le mélange est agité pendant 30 minutes à 0°C puis pendant 1h à température ambiante. On observe la formation d'un précipité. On ajoute alors 0,4 mmol de tris-(2-aminoethylamine) en solution dans 1 mL de DMF sec à 0°C, l'agitation est maintenue 15 minutes à 0°C puis une nuit à température ambiante. Le précipité est éliminé par filtration sur filtre seringue millipore 5$\mu$ puis la solution est lyophilisée. Le produit est purifié par dissolution dans un volume minimum de $CH_2Cl_2$ et précipitation dans un grand volume de diéthyléther. Ces précipitations sont répétées trois fois pour éliminer les traces de HOBt. Le produit est obtenu sous forme d'une poudre blanc cassé avec un rendement de 66%. RMN $^{31}P-\{^1H\}$ ($CDCl_3$) $\delta$ = 30,5 ppm.
RMN $^1H$ ($CDCl_3$) $\delta$ = 1,16 (quint, $^3J_{HH}$ = 7,0 Hz, 6H, $CH_2$-$C\underline{H_2}$-$CH_2$), 2,24 (t, $^3J_{HH}$ = 7,0 Hz, 6H, CO-$CH_2$), 2,72 (t, $^3J_{HH}$ = 7,0 Hz, 6H, $C\underline{H_2}$-N-$CH_2$-P), 2,86 (sl, 6H, N-$C\underline{H_2}$-$CH_2$-NH), 3,08 (d, $^2J_{HP}$ = 8,9 Hz, 12H, P-$CH_2$), 3,36 (sl, 6H, $C\underline{H_2}$-NHCO), 3,71 (d, $^3J_{HP}$ = 10,5 Hz, 36H, O-$CH_3$), 7,65 (sl, 3H, CONH) ppm.
RMN $^{13}C-\{^1H\}$ ($CDCl_3$) $\delta$ = 23,6 (s, $CH_2$-$\underline{C}H_2$-$CH_2$), 33,1 (s, NHCO-$\underline{C}H_2$), 36,4 (s, NH-$CH_2$), 49,4 (dd, $^1J_{CP}$ = 157,8 Hz, $^3J_{CP}$ = 7,7 Hz, $CH_2$-P), 52,7 (d, $^2J_{CP}$ = 6,0 Hz, $CH_3$-O), 54,0 (s, N-$\underline{C}H_2$-$CH_2$-NH), 56,2 (t, $^3J_{CP}$ = 6,7 Hz, P-$CH_2$-N-$\underline{C}H_2$), 174,1 (s, CONH) ppm.

*Etape 2 : synthèse du modèle de dendrimère DAB à extrémités acide aza-bis-phosphonique dérivé de l'acide amino-butyrique*

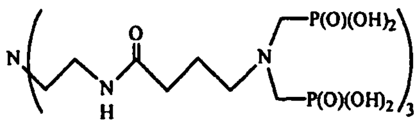

0,25 mmol de composé modèle de DAB azabis-phosphonate de l'étape 1 sont mis en solution dans 3 mL d'acétonitrile et placés à 0°C puis on additionne goutte à goutte 3,75 mmol de BrTMS sous atmosphère inerte. Après 30 minutes d'agitation à 0°C le bain de glace est enlevé et l'agitation est maintenue pendant 15h à température ambiante. Le solvant est éliminé sous pression réduite et 3 mL de MeOH sont ajoutés sur le résidu sec. On agite 30 minutes puis on élimine le solvant sous vide et on additionne 3 mL d'eau distillée. Après 1 heure d'agitation le mélange est lyophilisé. Le résidu sec est lavé 3 fois à l'éther sec. Le produit est obtenu sous la forme d'une poudre beige.
RMN $^{31}P-\{^1H\}$ ($D_2O$, THFd8) $\delta$ = 11,0 ppm.

*Etape 3 : Modèle de dendrimère DAB à extrémités [(carbamoylpropyl-amino)-méthyl]-acide phosphonique (sel de Na)*

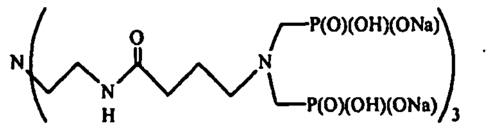

Le sel de sodium est obtenu par addition de 1 équivalent d'une solution aqueuse de soude 0,1955 N par groupement -$PO_3H_2$ présent dans la molécule obtenue dans l'étape 2. Le produit est isolé sous forme d'une poudre blanc cassé après lyophilisation avec un rendement de 70%.
RMN $^{31}P-\{^1H\}$ ($D_2O$/THFd8) δ = 11,4 ppm.
RMN $^{13}C-\{^1H\}$ ($D_2O$/THFd8) δ = 22,1 (s, $CH_2-\underline{C}H_2-CH_2$), 35,0 (s, NHCO-$\underline{C}H_2$), 37,0 (s, NH-$CH_2$), 53,8 (dl, $^1J_{CP}$ = 136,8 Hz, $CH_2$-P), 55,1 (s, N-$\underline{C}H_2-CH_2$-NH), 59,2 (s1, P-$CH_2$-N-$\underline{C}H_2$), 178,4 (s, CONH) ppm.

**Exemple 57: Synthèse de dendrimères de type DAB de génération 1 ayant 4 groupements azabis-phosphonique dérivé de la glycine**

*Etape 1 : Couplage de l'acide acétique amino [bis-(diméthoxy-phosphorylméthyl)] avec la première génération de dendrimère DAB*

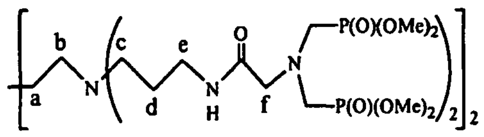

A 2 mmol d'acide carboxylique aza-bis-phosphonate obtenu dans **<u>l'Exemple 51</u>** (n = 1), on additionne sous atmosphère inerte 4 mL de DMF sec. La solution est placée à 0°C puis 2,6 mmol de HOBt sont ajoutés et on maintient l'agitation à 0°C pendant 30 minutes, 2,6 mmol de DCC sont additionnés. Après 30 minutes à 0°C on laisse le mélange remonter à température ambiante et on maintient l'agitation pendant 1 heure supplémentaire, on observe la formation progressive d'un précipité. La suspension est à nouveau placée à 0°C puis on ajoute 0,33 mmol de dendrimère DAB de première génération. Après 30 minutes à 0°C l'agitation est maintenue à température ambiante pendant 20h. Le précipité est éliminé sur filtres millipores 5 μ puis le DMF est lyophilisé. Le produit est traité trois fois par dissolution dans un volume minimum de dichlorométhane et précipitation dans un grand volume de diéthyléther de façon à éliminer l'excès de réactifs. Le dendrimère est obtenu sous forme d'une poudre blanc cassé avec un rendement de 73%.
RMN $^{31}P-\{^1H\}$ ($CDCl_3$) δ = 30,2 ppm.
RMN $^1H$ ($CDCl_3$) δ = 1,67 (sl, 4H, Ha), 1,86 (sl, 8H, Hd), 2,97 (sl, 12H, Hb et Hc), 3,20 (d, $^2J_{HP}$ = 9,2 Hz, 16H, $CH_2P$), 3,28 (sl, 8H, He), 3,47 (sl, 8H, Hf), 3,73 (d, $^3J_{HP}$ = 10,6 Hz, 48H, OMe), 7,98 (sl, 4H, CONH) ppm.
RMN $^{13}C-\{^1H\}$ ($CDCl_3$) δ = 21,9 (s, Ca), 24,4 (s, Cd), 36,5 (s, Ce), 49,8 (dd, $^1J_{CP}$ = 157,6 Hz, $^3J_{CP}$ = 6,3 Hz, $CH_2P$), 50,5 (s, Cc), 52,4 (s, Cb), 52,7 (d, $^2J_{CP}$ = 5,9 Hz, OMe), 60,3 (t, $^3J_{CP}$ = 6,5 Hz, Cf), 170,3 (s, CONH) ppm.

*Etape 2 : Synthèse du dendrimère de type DAB de génération 1 ayant 4 groupements acide azabis-phosphonique dérivé de la glycine en surface*

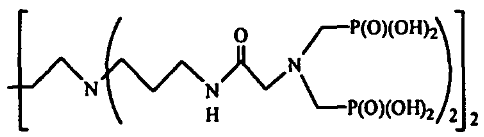

A 0,2 mmol de dendrimère de type DAB à extrémités azabis-phosphonate de génération 1 de l'étape 1, on ajoute sous atmosphère inerte 4 mL d'acétonitrile fraîchement distillé et le mélange est refroidi à 0°C. On ajoute alors goutte à goutte 6,4 mmol de BrTMS (soit 32 équivalents). Le mélange est maintenu à 0°C pendant 30 minutes puis sous agitation à température ambiante pendant 15 heures supplémentaires. L'acétonitrile est éliminé sous pression réduite puis le mélange est méthanolysé et hydrolysé comme dans les cas précédents. Le résidu sec est alors lavé deux fois

avec un mélange THF / diéthyléther (1 / 9). La poudre est alors séchée sous vide pour conduire au produit pur avec un rendement de 79%.
RMN $^{31}P$-{1H} ($D_2O$, THFd8) δ =11,5 ppm.

*Etape 3 : dendrimère DAB de première génération à extrémités (carbamoylméthyl-amino)-méthyl]-acide phosphonique (sel de Na)*

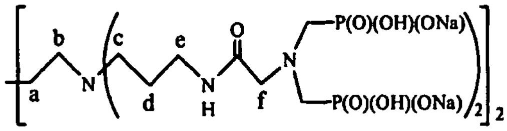

Le sel de sodium est obtenu par addition de 1 équivalent d'une solution aqueuse de soude 0,1955 N par groupement $-PO_3H_2$ présent dans la molécule obtenue dans l'étape 2. Le dendrimère est isolé sous forme d'une poudre beige avec un rendement de 68% après lyophilisation.
RMN $^{31}P$-{1H} ($D_2O/CD_3COCD_3$) δ = 19,9 ppm.
RMN $^{13}C$-{$^1H$} ($CDCl_3$) δ = 23,7 (s, Ca), 26,0 (s, Cd), 38,8 (s, Ce), 54,4 (d, $^1J_{CP}$ = 159,6 Hz, $CH_2P$), 54,6 (s, Cc), 58,1 (s, Cb), 63,1 (sl, Cf), 175,8 (s, CONH) ppm.

**Exemple 58: Synthèse de dendrimères de type DAB de génération 1 ayant 4 groupements azabis-phosphonique dérivé de l'acide aminobutyrique**

*Etape 1 : Couplage de l'acide butyrique amino [bis-(diméthoxy-phosphorylméthyl)] avec la première génération de dendrimère DAB*

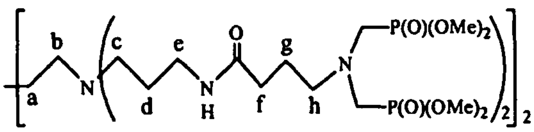

A 2 mmol d'acide carboxylique aza-bis-phosphonate obtenu dans l'**Exemple 51** (n = 3) (ex. A3), on additionne sous atmosphère inerte 4 mL de DMF sec. La solution est placée à 0°C puis 2,6 mmol de HOBt sont ajoutés et on maintient l'agitation à 0°C pendant 30 minutes, 2,6 mmol de DCC sont additionnés. Après 30 minutes à 0°C on laisse le mélange remonter à température ambiante et on maintient l'agitation pendant 1 heure supplémentaire, on observe la formation progressive d'un précipité. La suspension est à nouveau placée à 0°C puis on ajoute 0,33 mmol de dendrimère DAB de première génération. Après 30 minutes à 0°C l'agitation est maintenue à température ambiante pendant 20h. Le précipité est éliminé sur filtres millipores 5 μ puis le DMF est lyophilisé. Le produit est traité trois fois par dissolution dans un volume minimum de dichlorométhane et précipitation dans un grand volume de diéthyléther de façon à éliminer l'excès de réactifs. Le dendrimère est obtenu sous forme d'une poudre blanc cassé avec un rendement de 69%.
RMN $^{31}P$-{$^1H$} ($CDCl_3$) δ = 30,4 ppm.
RMN $^1H$ ($CDCl_3$) δ = 1,70-1,83 (m, 20H, Ha, Hd et Hg), 2, 23 (t, $^3J_{HH}$ = 6,9 Hz, 8H, Hf), 2,72 (m, 8H, Hh), 2,97 (m, 12H, Hb et Hc), 3,09 (d, $^3J_{HH}$ = 6,9 Hz, 16H, $CH_2P$), 3,24 (m, 8H, He), 3,72 (d, $^3J_{HP}$ = 10,5 Hz, 48H, OMe), 7,83 (m, 4H, CONH) ppm.
RMN $^{13}C$-{$^1H$} ($CDCl_3$) δ = 21,5 (s, Ca), 23,6 (s, Cg), 23,9 (s, Cd), 33,2 (s, Cf), 36,4 (s, Ce), 49,4 (dd, $^1J_{CP}$ = 151,7 Hz, $^3J_{CP}$ = 6,5 Hz, $CH_2P$), 50,5 (s, Cc), 52,2 (s, Cb), 52,7 (d, $^2J_{CP}$ = 5,5 Hz, OMe), 56,2 (t, $^3J_{CP}$ = 6,5 Hz, Ch), 173,9 (s, CONH) ppm.

*Etape 2 : Synthèse du dendrimère de type DAB de génération 1 ayant 4 groupements acide azabis-phosphonique dérivé de l'acide aminobutyrique*

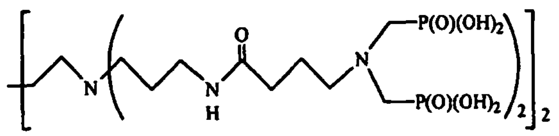

A 0,2 mmol de dendrimère de type DAB à extrémités azabis-phosphonate de génération 1 de l'étape 1, on ajoute sous atmosphère inerte 4 mL d'acétonitrile fraîchement distillé et le mélange est refroidi à 0°C. On ajoute alors goutte à goutte 6,4 mmol de BrTMS (soit 32 équivalents). Le mélange est maintenu à 0°C pendant 30 minutes puis sous agitation à température ambiante pendant 15 heures supplémentaires. L'acétonitrile est éliminé sous pression réduite puis le mélange est méthanolysé et hydrolysé comme dans les cas précédents. Le résidu sec est alors lavé deux fois avec un mélange THF / diéthyléther (1 / 9). La poudre est alors séchée sous vide pour conduire au produit pur avec un rendement de 68%
RMN $^{31}P-\{^1H\}$ ($D_2O$, $CD_3COCD_3$) $\delta$ = 11,0 ppm.

*Etape 3 : Dendrimère DAB de première génération à extrémités [(carbamoylpropyl-amino)-méthyl]-acidephosphonique (sel de Na)*

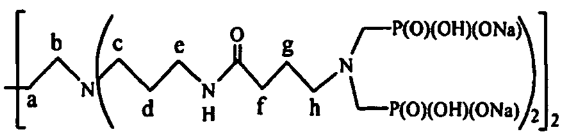

Le sel de sodium est obtenu par addition de 1 équivalent d'une solution aqueuse de soude 0,1955 N par groupement $-PO_3H_2$ présent dans la molécule obtenue dans l'étape 2. Le dendrimère est isolé sous forme d'une poudre blanc cassé avec un rendement de 69% après lyophilisation.
RMN $^{31}P-\{^1H\}$ ($D_2O/CD_3COCD_3$) $\delta$ = 10,2 ppm.
RMN $^{13}C-\{^1H\}$ ($D_2O/CD_3COCD_3$) $\delta$ = 22,7 (s, Cg), 23,3 (s, Ca), 26,0 (s, Cd), 35,2 (s, Cf), 39,0 (s, Ce), 52,9 (s, Cc), 55,0 (d, $^1J_{CP}$ = 130,7 Hz, $CH_2P$), 54,4 (s, Cb), 58,8 (s, Ch), 177,6 (s, CONH) ppm.

**Exemple 59: Synthèse de dendrimères de type DAB de génération 2 ayant 8 groupements azabis-phosphonique dérivé de la glycine**

*Etape 1 : Couplage de l'acide acétique amino [bis-(diméthoxy-phosphorylméthyl)] avec la deuxième génération de dendrimère DAB*

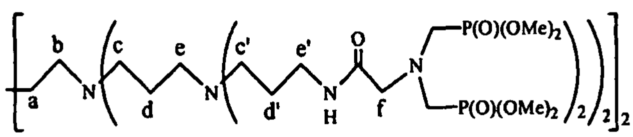

A 2 mmol d'acide carboxylique aza-bis-phosphonate obtenu dans l'**Exemple 51** (n = 1), on additionne sous atmosphère inerte 4 mL de DMF sec. La solution est placée à 0°C puis 2,6 mmol de HOBt sont ajoutés et on maintient l'agitation à 0°C pendant 30 minutes, 2,6 mmol de DCC sont additionnés. Après 30 minutes à 0°C on laisse le mélange remonter à température ambiante et on maintient l'agitation pendant 1 heure supplémentaire, on observe la formation progressive d'un précipité. La suspension est à nouveau placée à 0°C puis on ajoute 0,17 mmol de dendrimère DAB de deuxième génération. Après 30 minutes à 0°C l'agitation est maintenue à température ambiante pendant 20h. Le précipité est éliminé sur filtres millipores 5 $\mu$ puis le DMF est lyophilisé. Le produit est traité trois fois par dissolution dans un volume minimum de dichlorométhane et précipitation dans un grand volume de diéthyléther de façon à éliminer l'excès de réactifs. Le dendrimère est obtenu sous forme d'une poudre blanc cassé avec un rendement de 64%.
RMN $^{31}P-\{1H\}$ ($CDCl_3$) $\delta$ = 30,3 ppm.

*Etape 2 : Synthèse du dendrimère de type DAB de génération 2 ayant 8 groupements acide azabis-phosphonique dérivé de la glycine en surface*

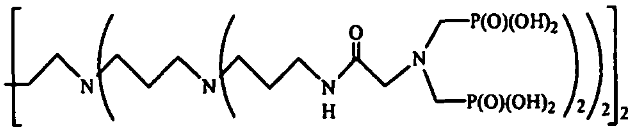

A 0,2 mmol de dendrimère de type DAB à extrémités azabis-phosphonate de génération 2 de l'étape 1, on ajoute sous atmosphère inerte 4 mL d'acétonitrile fraîchement distillé et le mélange est refroidi à 0°C. On ajoute alors goutte à goutte 12,8 mmol de BrTMS (soit 64 équivalents). Le mélange est maintenu à 0°C pendant 30 minutes puis sous agitation à température ambiante pendant 15 heures supplémentaires. L'acétonitrile est éliminé sous pression réduite puis le mélange est méthanolysé et hydrolysé comme dans les cas précédents. Le résidu sec est alors lavé deux fois avec un mélange THF / diéthyléther (1 / 9). La poudre est alors séchée sous vide pour conduire au produit pur avec un rendement de 68%.
RMN $^{31}P-\{^1H\}$ ($D_2O$, $CD_3COCD_3$) $\delta$ =10,6 ppm.

*Etape 3 : dendrimère DAB de deuxième génération à extrémités (carbamoylméthyl-amino)-méthyl]-acide phosphonique (sel de Na)*

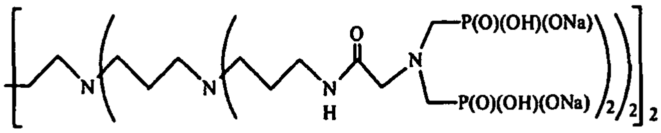

Le sel de sodium est obtenu par addition de 1 équivalent d'une solution aqueuse de soude 0,1955 N par groupement $-PO_3H_2$ présent dans la molécule obtenue dans l'étape 2. Le dendrimère est isolé sous forme d'une poudre beige avec un rendement de 68% après lyophilisation.
RMN $^{31}P-\{^1H\}$ ($D_2O$, $CD_3COCD_3$) $\delta$ =18,6 ppm.

**Exemple 60: Synthèse de dendrimères de type DAB de génération 2 ayant 8 groupements azabis-phosphonique dérivé de l'acide aminobutyrique**

*Etape 1 : Couplage de l'acide butyrique amino [bis-(diméthoxy-phosphorylméthyl)] avec la deuxième génération de dendrimère DAB*

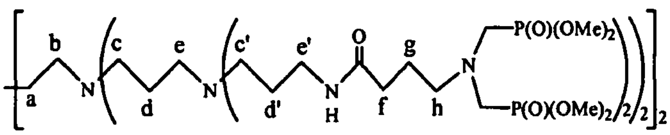

A 2 mmol d'acide carboxylique aza-bis-phosphonate obtenu dans l'**Exemple 51** (n = 3), on additionne sous atmosphère inerte 4 mL de DMF sec. La solution est placée à 0°C puis 2,6 mmol de HOBt sont ajoutés et on maintient l'agitation à 0°C pendant 30 minutes, 2,6 mmol de DCC sont additionnés. Après 30 minutes à 0°C on laisse le mélange remonter à température ambiante et on maintient l'agitation pendant 1 heure supplémentaire, on observe la formation progressive d'un précipité. La suspension est à nouveau placée à 0°C puis on ajoute 0,17 mmol de dendrimère de deuxième génération DAB. Après 30 minutes à 0°C l'agitation est maintenue à température ambiante pendant 20h. Le précipité est éliminé sur filtres millipores 5 $\mu$ puis le DMF est lyophilisé. Le produit est traité trois fois par dissolution dans un volume minimum de dichlorométhane et précipitation dans un grand volume de diéthyléther de façon à éliminer

l'excès de réactifs. Le dendrimère est obtenu sous forme d'une poudre blanc cassé avec un rendement de 75%.
RMN $^{31}P-\{^1H\}$ ($CDCl_3$) $\delta$ = 30,4 ppm.
RMN $^1H$ ($CDCl_3$) $\delta$ = 1,62-1,91 (m, 44H, Ha, Hd, Hd' et Hg), 2,16 (s1, 24H, Hc et Hc'), 2,72 (s1, 24H, He et He'), 2,88-3,25 (m, 68H, Hb, Hf, Hh et $CH_2P$), 3,69 (d, $^3J_{HP}$ = 10,5 Hz, 96H, OMe), 7,80 (m, 8H, CONH) ppm.
RMN $^{13}C-\{^1H\}$ ($CDCl_3$) $\delta$ = 20,8 (s, Ca), 23,6 (s, Cg), 24,4 (s, Cd et Cd'), 33,2 (s, Cf), 36,6 (s, Ce'), 49,3 (dd, $^1J_{CP}$ = 158,1 Hz, $^3J_{CP}$ = 6,8 Hz, $CH_2P$), 50,5 (s, Cc et Cc'), 52,4 (s, Cb et Ce), 52,7 (d, $^2J_{CP}$ = 5,8 Hz, OMe), 56,2 (t, $^3J_{CP}$ = 6,5 Hz, Ch), 173,7 (s, CONH) ppm.

*Etape 2 : Synthèse du dendrimère de type DAB de génération 2 ayant 8 groupements acide azabis-phosphonique dérivé de l'acide aminobutyrique en surface*

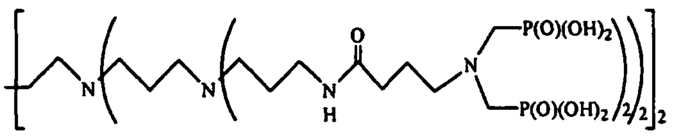

A 0,2 mmol de dendrimère de type DAB à extrémités azabis-phosphonate de génération 1 ou 2 de l'étape 1, on ajoute sous atmosphère inerte 4 mL d'acétonitrile fraîchement distillé et le mélange est refroidi à 0°C. On ajoute alors goutte à goutte 12,8 mmol de BrTMS (soit 64 équivalents). Le mélange est maintenu à 0°C pendant 30 minutes puis sous agitation à température ambiante pendant 15 heures supplémentaires. L'acétonitrile est éliminé sous pression réduite puis le mélange est méthanolysé et hydrolysé comme dans les cas précédents. Le résidu sec est alors lavé deux fois avec un mélange THF / diéthyléther (1 / 9). La poudre est alors séchée sous vide pour conduire au produit pur avec un rendement de 74%.
RMN $^{31}P-\{^1H\}$ ($D_2O$, $CD_3COCD_3$) $\delta$ = 10,9 ppm.

*Etape 3 : dendrimère DAB de deuxième génération à extrémités [(carbamoylpropyl-amino)-méthyl]-acide phosphonique (sel de Na)*

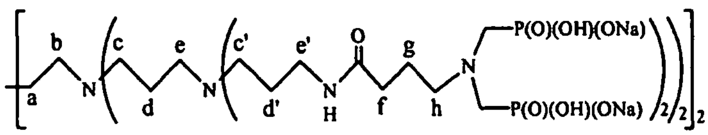

Le sel de sodium est obtenu par addition de 1 équivalent d'une solution aqueuse de soude 0,1955 N par groupement $-PO_3H_2$ présent dans la molécule obtenue dans l'étape 2. Le dendrimère est isolé sous forme d'une poudre blanc cassé avec un rendement de 72% après lyophilisation.
RMN $^{31}P-\{^1H\}$ ($D_2O/CD_3COCD_3$) $\delta$ =10,1 ppm.
RMN $^{13}C-\{^1H\}$ ($D_2O/CD_3COCD_3$) $\delta$ = 21,7 (s, Ca), 22,8 (s, Cg), 25,8 (s, Cd et Cd'), 35,2 (s, Cf), 39,1 (s, Ce'), 55,2 (d, $^1J_{CP}$ = 129,4 Hz, $CH_2P$), 52,4 (s, Cc et Cc'), 52,9 (s, Cb et Ce), 58,8 (s, Ch), 177,7 (s, CONH) ppm.

**Exemple 61 : modèle de dendrimère PAMAM à groupement azabis-phosphonique dérivé de la glycine**

*Etape 1 : couplage de l'acide acétique amino [bis-(diméthoxy-phosphorylméthyl)] avec le N-(2-aminoéthyl)-acétamide*

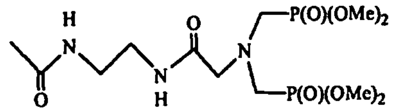

1,58 mmol d'acide carboxylique aza-bis-phosphonate obtenu dans l'**Exemple 51** (n = 1), sont mis en solution

dans 5 mL de DMF sec à 0°C. On additionne alors 1,74 mmol de HOBt et on agite 15 minutes à 0°C puis on ajoute 1,74 mmol de DCC et on maintient l'agitation 30 minutes à 0°C et 1 heure à température ambiante. Un précipité se forme, le mélange est porté à 0°C et 1,58 mmol de N-(2-aminoéthyl)-acétamide sont ajoutés à 0°C. On agite 15 minutes à 0°C puis une nuit à température ambiante. Le produit de couplage est purifié sur colonne chromatographique de silice en utilisant comme éluant du $CH_2Cl_2$ pur puis en faisant un gradient jusqu'à 10% de MeOH. Il est obtenu avec un rendement de 43% sous forme d'une poudre blanc cassé.
Rf = 0,19 ($CH_2Cl_2$ / MeOH : 95 / 5).
RMN $^{31}P$-{$^1H$} ($CDCl_3$) $\delta$ = 30,0 ppm.
RMN $^1H$ ($CDCl_3$) $\delta$ = 1,93 (s, 3H, $COCH_3$), 3,15 (d, $^2J_{HP}$ = 9,3 Hz, 4H, P-$CH_2$), 3,36-3,42 (m, 6H, N-C$\underline{H_2}$-CO, NH-C$\underline{H_2}$-C$\underline{H_2}$ -NH), 3,76 (d, $^3J_{HP}$ = 10,7 Hz, 12H, O-$CH_3$), 7,55 (s1, 1H, CONH), 7,78 (s1, 1H, CONH) ppm.
RMN $^{13}C$-{$^1H$} ($CDCl_3$) $\delta$ = 23,0 (s, $COCH_3$), 39,3 (s, NH-$CH_2$), 39,4 (s, NH-$CH_2$), 50,7 (dd, $^1J_{CP}$ = 160,1 Hz, $^3J_{CP}$ = 6,5 Hz, $CH_2$-P), 52,8 (d, $^2J_{CP}$ = 4,4 Hz, $CH_3$-O), 61,5 (t, $^3J_{CP}$ = 7,0 Hz, N-$\underline{C}H_2$-CO), 170,1 (s, CONH), 170,7 (s, CONH) ppm.

*Etape 2: modèle de dendrimère PAMAM à extrémités [(carbamoylméthyl-amino)-méthyl]-acide phosphonique (sel de Na)*

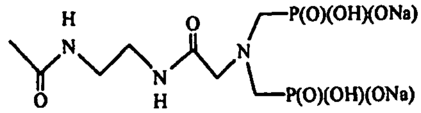

0,5 mmol du modèle de PAMAM aza-bis-phosphonate de l'étape 1 sont mis en solution dans 5 mL d'acétonitrile distillé, puis on additionne, à 0°C, goutte à goutte à la seringue, 1,1 équivalent de BrTMS par équivalent de liaison P-OMe présente dans la molécule de départ. L'agitation est maintenue 30 minutes à 0°C puis une nuit à température ambiante. Le mélange est tiré sous vide et 5 mL de méthanol sont additionnés sur le résidu sec. Après 1 heure d'agitation à température ambiante, la solution est tirée sous vide et on additionne 3 mL d'eau distillée. Après 1 heure à température ambiante, la solution est lyophilisée. Le résidu sec est lavé à l'éther distillé à trois reprises. Le sel de sodium est obtenu par addition de 1 équivalent d'une solution aqueuse de soude 0,1955 N par groupement - $PO_3H_2$ présent dans la molécule. Le modèle est obtenu après lyophilisation sous forme d'une poudre blanc cassé et avec un rendement de 68%.
RMN $^{31}P$-{$^1H$} ($D_2O$/THFd8) $\delta$ = 18,0 ppm.

**Exemple 62: modèle de dendrimère PAMAM à groupement azabis-phosphonique dérivé de l'acide butyrique**

*Etape 1 : couplage de l'acide butyrique amino [bis-(diméthoxy-phosphorylméthyl)] avec le N-(2-aminoéthyl)-acétamide*

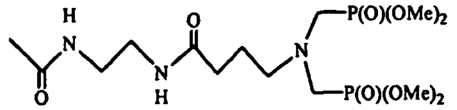

1,58 mmol d'acide carboxylique aza-bis-phosphonate obtenu dans l'**<u>Exemple 51</u>** (n = 3), sont mis en solution dans 5 mL de DMF sec à 0°C. On additionne alors 1,74 mmol de HOBt et on agite 15 minutes à 0°C puis on ajoute 1,74 mmol de DCC et on maintient l'agitation 30 minutes à 0°C et 1 heure à température ambiante. Un précipité se forme, le mélange est porté à 0°C et 1,58 mmol de N-(2-aminoéthyl)-acétamide sont ajoutés à 0°C. On agite 15 minutes à 0°C puis une nuit à température ambiante. On obtient ainsi le produit de couplage, qui est purifié sur colonne chromatographique de silice en utilisant comme éluant du $CH_2Cl_2$ pur puis en faisant un gradient jusqu'à 10% de MeOH. Il est obtenu avec un rendement de 52%, sous forme d'une poudre blanc cassé.
Rf= 0,43 ($CH_2Cl_2$ / MeOH : 90 /10).
RMN $^{31}P$-{$^1H$} ($CDCl_3$) $\delta$ = 30,0 ppm.
RMN $^1H$ ($CDCl_3$) $\delta$ = 1,68 (quint, $^3J_{HH}$ = 6,6 Hz, 2H, $CH_2$-C$\underline{H_2}$-$CH_2$), 1,87 (s, 3H, $COCH_3$), 2,20 (t, $^3J_{HH}$ = 6,6 Hz, 2H, N-C$\underline{H}$2-CO), 2,69 (t, $^3J_{HH}$ = 6,6 Hz, 2H, $CH_2$-N), 3,02 (d, $^2J_{HP}$ = 8,7 Hz, 4H, P-$CH_2$), 3,23-3,29 (m, 4H, NH-C$\underline{H_2}$-C$\underline{H_2}$-NH), 3,69 (d, $^3J_{HP}$ =10,6 Hz, 12H, O-$CH_3$), 7,26 (sl, 1H, CONH), 7,37 (sl, 1H, CONH) ppm.
RMN $^{13}C$-{$^1H$} ($CDCl_3$) $\delta$ = 22,9 (s, $COCH_3$), 23,8 (s, **$CH_2$-$CH_2$-$CH_2$),** 33,2 (s, NHCO-$\underline{C}H_2$), 39,3 (s, NH-$CH_2$), 40,0 (s, NH-$CH_2$), 49,5 (dd, $^1J_{CP}$ = 159,4 Hz, $^3J_{CP}$ = 7,2 Hz, $CH_2$-P), 52,6 (d, $^2J_{CP}$ = 5,4 Hz, $CH_3$-O), 55,9 (t, $^3J_{CP}$ = 7,1 Hz,

N-$\underline{C}H_2$-$CH_2$), 170,9 (s, CONH), 174,3 (s, CONH) ppm.

*Etape 2 : modèle de dendrimère PAMAM à extrémités [(carbamoylpropyl-amino)-méthyl]-acide phosphonique (sel de Na)*

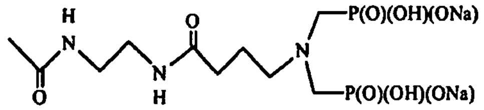

0,5 mmol du composé aza-bis-phosphonate de l'étape 1 est mis en solution dans 5 mL d'acétonitrile distillé, puis on additionne, à 0°C, goutte à goutte à la seringue, 1,1 équivalent de BrTMS par équivalent de liaison P-OMe présente dans la molécule de départ. L'agitation est maintenue 30 minutes à 0°C puis une nuit à température ambiante. Le mélange est tiré sous vide et 5 mL de méthanol sont additionnés sur le résidu sec. Après 1 heure d'agitation à température ambiante, la solution est tirée sous vide et on additionne 3 mL d'eau distillée. Après 1 heure à température ambiante, la solution est lyophilisée. Le résidu sec est lavé à l'éther distillé à trois reprises. Le sel de sodium est obtenu par addition de 1 équivalent d'une solution aqueuse de soude 0,1955 N par groupement -$PO_3H_2$ présent dans la molécule. Le produit est obtenu sous forme de poudre blanc cassé avec un rendement de 71% après lyophilisation.
RMN $^{31}$P-{$^1$H} ($D_2O$) δ = 10,7 ppm.
RMN $^{13}$C-{$^1$H} ($D_2O$) δ = 22,5 (s, $COCH_3$), 24,7 (s, $CH_2$-$\underline{C}H_2$-$CH_2$), 35,5 (s, NHCO-$\underline{C}H_2$), 41,3 (s, NH-$CH_2$), 41,4 (s, NH-$CH_2$), 54,2 (dl, $^1J_{CP}$ = 134,1 Hz, $CH_2$-P), 58,9 (s1, N-$\underline{C}H_2$-$CH_2$), 177,1 (s, CONH), 177,6 (s, CONH) ppm.

**Exemple 63 : synthèse du dendrimère de type PAMAM de génération 1 ayant 4 groupements azabis-phosphonate dérivé de la glycine en surface**

Etape 1 : *couplage de l'acide acétique amino [bis-(diméthoxy-phosphorylméthyl)] avec la première génération de dendrimère PAMAM*

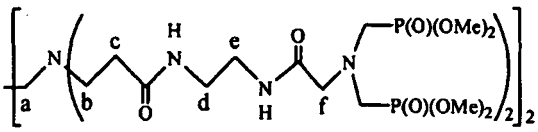

A 2 mmol d'acide carboxylique aza-bis-phosphonate obtenu dans l'**Exemple 51** (n = 1), on additionne sous atmosphère inerte 4 mL de DMF sec. La solution est placée à 0°C puis 2,6 mmol de HOBt sont ajoutés et on maintient l'agitation à 0°C pendant 30 minutes, 2,6 mmol de DCC sont additionnés. Après 30 minutes à 0°C on laisse le mélange remonter à température ambiante et on maintient l'agitation pendant 1 heure supplémentaire, on observe la formation progressive d'un précipité. La suspension est à nouveau placée à 0°C puis on ajoute 0,33 mmol de dendrimère PAMAM de première génération. Après 30 minutes à 0°C l'agitation est maintenue à température ambiante pendant 20h. Le précipité est éliminé sur filtres millipores 5 μ puis le DMF est lyophilisé. Le produit est traité trois fois par dissolution dans un volume minimum de dichlorométhane et précipitation dans un grand volume de diéthyléther de façon à éliminer l'excès de réactifs. Le dendrimère est isolé sous forme de poudre blanc cassé avec un rendement de 67 %.
RMN $^{31}$P-{$^1$H} ($CDCl_3$) δ = 30,1 ppm.
RMN $^1$H ($CDCl_3$) δ = 2,48 (sl, 12H, Ha et Hc), 2,98 (m, 8H, Hb), 3,18 (d, $^2J_{HP}$ = 9,4 Hz, 16H, $CH_2$P), 3,29 (sl, 16H, Hd et He), 3,41 (sl, 8H, Hf), 3,72 (d, $^3J_{HP}$ = 10,6 Hz, 48H, OMe), 7,77 (sl, 4H, CONH), 8,17 (sl, 4H, CONH) ppm.
RMN $^{13}$C-{$^1$H} ($CDCl_3$) δ = 31,6 (s, Cc), 38,9 (s, Ce), 39,2 (s, Cd), 48,8 (s, Cb), 49,7 (s, Ca), 50,2 (dd, $^1J_{CP}$ = 159,2 Hz, $^3J_{CP}$ = 6,5 Hz, $CH_2$P), 52,8 (d, $^2J_{CP}$ = 5,0 Hz, OMe), 60,7 (t, $^3J_{CP}$ = 6,6 Hz, Cf), 170,3 (s, CONH), 171,4 (s, CONH) ppm.

*Etape 2 : Synthèse du dendrimère PAMAM de génération 1 ayant 4 groupements acide azabis-phosphonique dérivés de la glycine en surface*

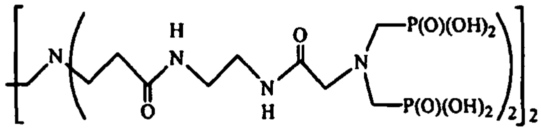

A 0,2 mmol de dendrimère de type PAMAM à extrémités azabis-phosphonate de génération 1 de l'étape 1, on ajoute sous atmosphère inerte 4 mL d'acétonitrile fraîchement distillé et le mélange est refroidi à 0°C. On ajoute alors goutte à goutte 6,4 mmol de BrTMS (soit 32 équivalents). Le mélange est maintenu à 0°C pendant 30 minutes puis sous agitation à température ambiante pendant 15 heures supplémentaires. L'acétonitrile est éliminé sous pression réduite puis le mélange est méthanolysé et hydrolysé comme dans les cas précédents. Le résidu sec est alors lavé deux fois avec un mélange THF / diéthyléther (1 / 9). La poudre est alors séchée sous vide pour conduire au produit pur avec un rendement de 65%
RMN $^{31}P$-{$^1H$} ($D_2O$, THFd8) $\delta$ = 10,9 ppm.

*Etape 3 : dendrimère PAMAM de première génération à extrémités [(carbamoylméthyl-amino)-méthyl]-acide phosphonique (sel de Na)*

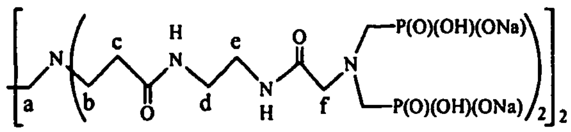

Le sel de sodium est obtenu par addition de 1 équivalent d'une solution aqueuse de soude 0,1955 N par groupement $-PO_3H_2$ présent dans la molécule obtenue à étape 2. Le produit est isolé avec un rendement de 72% après lyophilisation sous forme d'une poudre blanc cassé.
RMN $^{31}P$-{$^1H$} (D2O/$CD_3COCD_3$) $\delta$ = 19,6 ppm.

**Exemple 64 : synthèse du dendrimère de type PAMAM de génération 1 ayant 4 groupements azabis-phosphonate dérivé de l'acide aminobutyrique en surface**

*Etape 1 : couplage de l'acide butyrique amino [bis-(diméthoxy-phosphorylméthyl)] avec la première génération de dendrimère PAMAM*

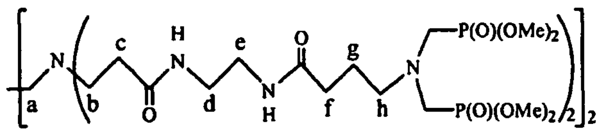

A 2 mmol d'acide carboxylique aza-bis-phosphonate obtenu dans l'**Exemple 51** (n = 3), on additionne sous atmosphère inerte 4 mL de DMF sec. La solution est placée à 0°C puis 2,6 mmol de HOBt sont ajoutés et on maintient l'agitation à 0°C pendant 30 minutes, 2,6 mmol de DCC sont additionnés. Après 30 minutes à 0°C on laisse le mélange remonter à température ambiante et on maintient l'agitation pendant 1 heure supplémentaire, on observe la formation progressive d'un précipité. La suspension est à nouveau placée à 0°C puis on ajoute 0,33 mmol de dendrimère PAMAM de première génération. Après 30 minutes à 0°C l'agitation est maintenue à température ambiante pendant 20h. Le précipité est éliminé sur filtres millipores 5$\mu$ puis le DMF est lyophilisé. Le produit est traité trois fois par dissolution dans un volume minimum de dichlorométhane et précipitation dans un grand volume de diéthyléther de façon à éliminer l'excès de réactifs. Il est isolé sous forme d'une poudre blanc cassé avec un rendement de 61 %.
RMN $^{31}P$-{$^1H$} ($CDCl_3$) $\delta$ = 30,4 ppm.
RMN $^1H$ ($CDCl_3$) $\delta$ = 1,72 (quint, $^3J_{HH}$ = 6,3 Hz, 8H, Hg), 2,21 (t, $^3J_{HH}$ = 6,3 Hz, 8H, Hf), 2,55 (m, 12H, Ha et Hc), 2,73 (t, $^3J_{HH}$ = 6,3 Hz, 8H, Hh), 2,93 (m, 8H, Hb), 3,09 (d, $^2J_{HP}$ = 9,1 Hz, 16H, $CH_2P$), 3,28 (sl, 16H, Hd et He), 3,73 (d, $^3J_{HP}$ = 10,6 Hz, 48H, OMe), 7,65 (sl, 4H, CONH), 8,21 (sl, 4H, CONH) ppm.

RMN $^{13}C-\{^1H\}$ ($CDCl_3$) δ = 23,5 (s, Cg), 32,1 (s, Cc), 33,3 (s, Cf), 39,2 (s, Ce), 39,6 (s, Cd), 49,2 (s, Cb), 49,5 (dd, $^1J_{CP}$ = 157,9 Hz, $^3J_{CP}$ = 7,2 Hz, $CH_2P$), 50,1 (s, Ca), 52,6 (d, $^2J_{CP}$ = 4,6 Hz, OMe), 56,1 (t, $^3J_{CP}$ = 6,7 Hz, Ch), 171,7 (s, CONH), 173,7 (s, CONH) ppm.

*Etape 2 : Synthèse du dendrimère PAMAM de génération 1 ayant 4 groupements acide azabis-phosphonique dérivés de l'acide butyrique en surface*

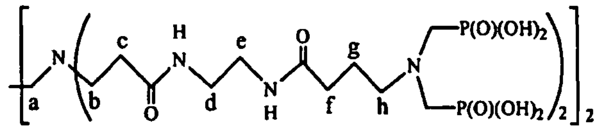

A 0,2 mmol de dendrimère de type PAMAM à extrémités azabis-phosphonate de génération 0 ou 1 de l'étape 1, on ajoute sous atmosphère inerte 4 mL d'acétonitrile fraîchement distillé et le mélange est refroidi à 0°C. On ajoute alors goutte à goutte 6,4 mmol de BrTMS (soit 32 équivalents). Le mélange est maintenu à 0°C pendant 30 minutes puis sous agitation à température ambiante pendant 15 heures supplémentaires. L'acétonitrile est éliminé sous pression réduite puis le mélange est méthanolysé et hydrolysé comme dans les cas précédents. Le résidu sec est alors lavé deux fois avec un mélange THF / diéthyléther (1 / 9). La poudre est alors séchée sous vide pour conduire au produit pur avec un rendement de 71%
RMN $^{31}P-\{^1H\}$ ($D_2O$, $CD_3COCD3$) δ = 11,1 ppm.

*Etape 3 : dendrimère PAMAM de première génération à extrémités [(carbamoylpropyl-amino)-méthyl]-acide phosphonique (sel de Na)*

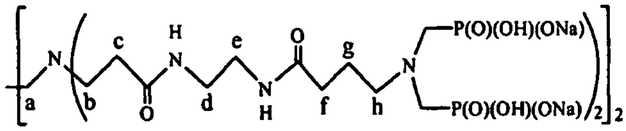

Le sel de sodium est obtenu par addition de 1 équivalent d'une solution aqueuse de soude 0,1955 N par groupement -PO3H$_2$ présent dans la molécule obtenue à l'étape 2. Le produit est isolé avec un rendement de 73% après lyophilisation sous forme d'une poudre blanc cassé.
RMN $^{31}P-\{^1H\}$ ($D_2O/CD_3COCD_3$) δ = 10,2 ppm.
RMN $^{13}C-\{^1H\}$ ($D_2O/CD_3COCD_3$) δ = 22,8 (s, Cg), 33,4 (s, Cc), 35,2 (s, Cf), 41,3 (s, Ce), 41,4 (s, Cd), 51,3 (s, Cb), 51,7 (s, Ca), 55,1 (d, $^1J_{CP}$ = 130,0 Hz, $CH_2P$), 58,9 (sl, Ch), 175,9 (s, CONH), 177,5 (s, CONH) ppm.

**Exemple 65: synthèse du dendrimère de type PAMAM de génération 2 ayant 8 groupements azabis-phosphonate dérivé de la glycine en surface**

*Etape 1 : couplage de l'acide acétique amino [bis-(diméthoxy-phosphorylméthyl)] avec la deuxième génération de dendrimère PAMAM*

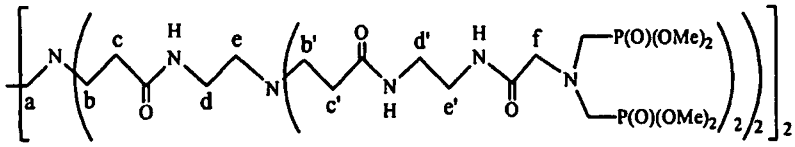

A 2 mmol d'acide carboxylique aza-bis-phosphonate obtenu dans l'**Exemple 51** (n = 1), on additionne sous atmosphère inerte 4 mL de DMF sec. La solution est placée à 0°C puis 2,6 mmol de HOBt sont ajoutés et on maintient

l'agitation à 0°C pendant 30 minutes, 2,6 mmol de DCC sont additionnés. Après 30 minutes à 0°C on laisse le mélange remonter à température ambiante et on maintient l'agitation pendant 1 heure supplémentaire, on observe la formation progressive d'un précipité. La suspension est à nouveau placée à 0°C puis on ajoute 0,17 mmol de dendrimère PAMAM de deuxième génération. Après 30 minutes à 0°C l'agitation est maintenue à température ambiante pendant 20h. Le précipité est éliminé sur filtres millipores 5 μ puis le DMF est lyophilisé. Le produit est traité trois fois par dissolution dans un volume minimum de dichlorométhane et précipitation dans un grand volume de diéthyléther de façon à éliminer l'excès de réactifs. Le dendrimère est isolé sous forme de poudre blanc cassé avec un rendement de 63 %.
RMN $^{31}P-\{^1H\}$ ($CDCl_3$) $\delta$ = 30,2 ppm.

*Etape 2 : Synthèse du dendrimère PAMAM de génération 2 ayant 8 groupements acide azabis-phosphonique dérivés de la glycine en surface*

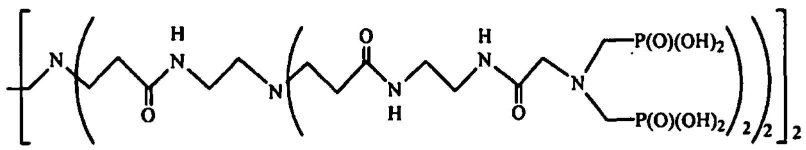

A 0,2 mmol de dendrimère de type PAMAM à extrémités azabis-phosphonate de génération 0 ou 1 de l'étape 1, on ajoute sous atmosphère inerte 4 mL d'acétonitrile fraîchement distillé et le mélange est refroidi à 0°C. On ajoute alors goutte à goutte 12,8 mmol de BrTMS (soit 64 équivalents). Le mélange est maintenu à 0°C pendant 30 minutes puis sous agitation à température ambiante pendant 15 heures supplémentaires. L'acétonitrile est éliminé sous pression réduite puis le mélange est méthanolysé et hydrolysé comme dans les cas précédents. Le résidu sec est alors lavé deux fois avec un mélange THF / diéthyléther (1 / 9). La poudre est alors séchée sous vide pour conduire au produit pur avec un rendement de 73%
RMN $^{31}P-\{^1H\}$ ($D_2O$, $CD_3COCD_3$) $\delta$ = 11,0 ppm.

*Etape 3 : dendrimère PAMAM de deuxième génération à extrémités [(carbamoylméthyl-amino)-méthyl]-acide phosphonique (sel de Na)*

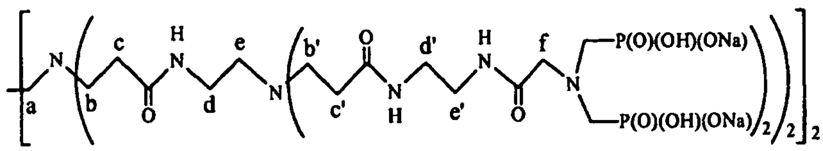

Le sel de sodium est obtenu par addition de 1 équivalent d'une solution aqueuse de soude 0,1955 N par groupement $-PO_3H_2$ présent dans la molécule obtenue dans l'étape 2. Le produit est isolé avec un rendement de 69% après lyophilisation sous forme d'une poudre blanc cassé.
RMN $^{31}P-\{^1H\}$ ($D_2O$, $CD_3COCD3$) $\delta$ = 18,5 ppm.

**Exemple 66: synthèse du dendrimère de type PAMAM de génération 2 ayant 8 groupements azabis-phosphonate dérivé de l'acide aminobutyrique en surface**

*Etape 1 : Couplage de l'acide butyrique amino [bis-(diméthoxy-phosphorylméthyl)] avec la deuxième génération de dendrimère PAMAM*

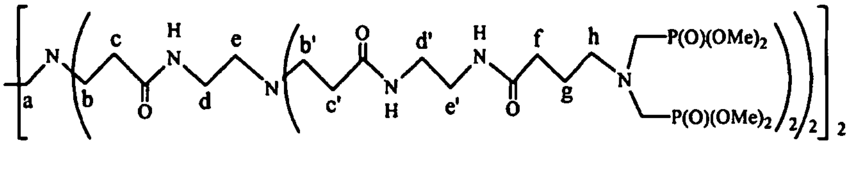

A 2 mmol d'acide carboxylique aza-bis-phosphonate obtenu dans l'**Exemple 51** (n = 3), on additionne sous atmosphère inerte 4 mL de DMF sec. La solution est placée à 0°C puis 2,6 mmol de HOBt sont ajoutés et on maintient l'agitation à 0°C pendant 30 minutes, 2,6 mmol de DCC sont additionnés. Après 30 minutes à 0°C on laisse le mélange remonter à température ambiante et on maintient l'agitation pendant 1 heure supplémentaire, on observe la formation progressive d'un précipité. La suspension est à nouveau placée à 0°C puis on ajoute 0,17 mmol du dendrimère PAMAM de deuxième génération. Après 30 minutes à 0°C l'agitation est maintenue à température ambiante pendant 20h. Le précipité est éliminé sur filtres millipores 5 μ puis le DMF est lyophilisé. Le produit est traité trois fois par dissolution dans un volume minimum de dichlorométhane et précipitation dans un grand volume de diéthyléther de façon à éliminer l'excès de réactifs. Le dendrimère est obtenu sous forme d'une poudre blanc cassé avec un rendement de 63 %.
RMN $^{31}P-\{^1H\}$ ($CDCl_3$) δ = 30,4 ppm.
RMN $^1H$ ($CDCl_3$) δ = 1,69 (quintl, $^3J_{HH}$ = 5,9 Hz, 16H, Hg), 2,17 (tl, $^3J_{HH}$ = 5,9 Hz, 16H, Hf), 2,21-2,93 (m, 76H, Ha, Hb, Hc, He, Hh, Hb', Hc'), 3,06 (d, $^2J_{HP}$ = 9,1 Hz, 32H, $CH_2P$), 3,25 (sl, 40H, Hd, Hd' et He'), 3,69 (d, $^3J_{HP}$ = 10,5 Hz, 96H, OMe), 7,70 (sl, 8H, CONH), 8,07 (sl, 8H, CONH), 8,28 (sl, 4H, CONH) ppm.
RMN $^{13}C-\{^1H\}$ ($CDCl_3$) δ = 23,7 (s, Cg), 33,5 (s, Cc et Cc'), 39,7 (s, Cd, Ce' et Cf), 48,8 (s, Cd'), 49,6 (dd, $^1J_{CP}$ = 158,1 Hz, $^3J_{CP}$ = 7,0 Hz, $CH_2P$), 50,4 (s, Ca, Cb et Cb'), 52,4 (s, Ce), 53,0 (sl, OMe), 56,3 (t, $^3J_{CP}$ = 8,2 Hz, Ch), 171,6 (s, CONH), 172,7 (s, CONH), 174,1 (s, CONH) ppm.

*Etape 2 : Synthèse du dendrimère PAMAM de génération 2 ayant 8 groupements acide azabis-phosphonique dérivés de l'acide butyrique en surface*

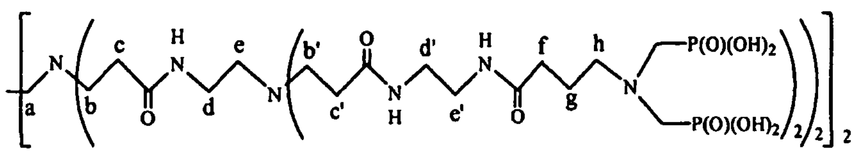

A 0,2 mmol de dendrimère de type PAMAM à extrémités azabis-phosphonate de génération 0 ou 1 de l'étape 1, on ajoute sous atmosphère inerte 4 mL d'acétonitrile fraîchement distillé et le mélange est refroidi à 0°C. On ajoute alors goutte à goutte 12,8 mmol de BrTMS (soit 64 équivalents). Le mélange est maintenu à 0°C pendant 30 minutes puis sous agitation à température ambiante pendant 15 heures supplémentaires. L'acétonitrile est éliminé sous pression réduite puis le mélange est méthanolysé et hydrolysé comme dans les cas précédents. Le résidu sec est alors lavé deux fois avec un mélange THF / diéthyléther (1 / 9). La poudre est alors séchée sous vide pour conduire au produit pur avec un rendement de 67%.
RMN $^{31}P-\{^1H\}$ ($D_2O$, $CD_3COCD_3$) δ = 11,3 ppm.

*Etape 3 : dendrimère PAMAM de deuxième génération à extrémités [(carbamoylpropyl-amino)-méthyl]-acide phosphonique (sel de Na)*

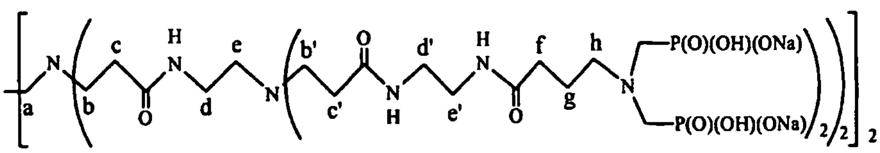

Le sel de sodium est obtenu par addition de 1 équivalent d'une solution aqueuse de soude 0,1955 N par groupement $-PO_3H_2$ présent dans la molécule obtenue dans l'étape 2. Le produit est isolé avec un rendement de 71% après lyophilisation sous forme d'une poudre blanc cassé.
RMN $^{31}P-\{^1H\}$ ($D_2O/CD_3COCD_3$) δ =10,1 ppm.
RMN $^{13}C-\{^1H\}$ ($D_2O/CD_3COCD_3$) δ = 22,8 (s, Cg), 32,0 (s, Cc et Cc'), 35,2 (s, Cd), 41,3 (s, Ce' ou Cf), 41,6 (s, Ce' ou Cf), 51,1 (s, Cd'), 52,4 (s, Cb et Cb'), 54,4 (s, Ce), 55,3 (d, $^1J_{CP}$ = 132,6 Hz, $CH_2P$), 58,9 (s, Ch), 174,7 (s, CONH), 176,5 (s, CONH), 177,7 (s, CONH) ppm.

**Exemple 67 : Synthèse de dendrimères phosphorés de type Gc présentant 12 extrémités amido-azabis-phosphonique dérivé de la glycine en surface**

*Etape 1 : couplage du phénol à extrémité [carbamoyl-méthyl-amino-méthyl] diméthylesterphoshonique avec le dendrimère phosphoré de première génération*

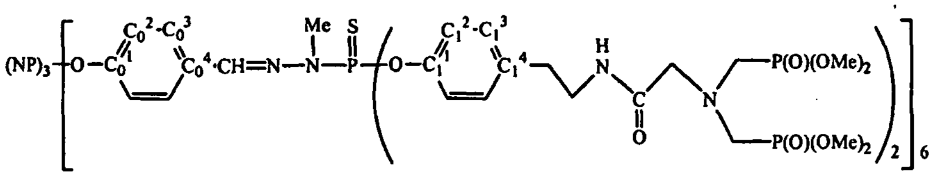

0,017 mmol de dendrimère phosphoré de génération 1 (12 extrémités C1) sont mis en solution dans 3 mL de THF sec. Sur cette solution on ajoute successivement 5,04 mmol de carbonate de césium puis 0,23 mmol de composé tyramine-amido-azabis-phosphonate obtenu dans l'**Exemple 53** (n = 1) en solution dans 3 mL de THF sec. Le mélange est agité une nuit à température ambiante puis filtré sur célite. Le milieu réactionnel est évaporé sous pression réduite puis le résidu sec est dissous dans un volume minimum de dichlorométhane. Le produit est alors précipité dans un grand volume d'éther. Cette opération est répétée trois fois pour éliminer le léger excès de phénol de départ. Le produit est obtenu sous forme d'une poudre blanc cassé avec un rendement de 88%.
RMN $^{31}P-\{^1H\}$ ($CDCl_3$) $\delta$ = 11,7 (s, $N_3P_3$), 30,1 (s, $PO_3Me_2$), 66,6 (s, P=S) ppm.
RMN $^1H$ ($CDCl_3$) $\delta$ = 2,77 (t, $^3J_{HH}$ = 6,8 Hz, 24H, C$\underline{H_2}$-$CH_2$-N), 3,13 (d, $^3J_{HP}$ = 9,4 Hz, 48H, P-$CH_2$), 3,23 (d, $^3J_{HP}$ = 10,1 Hz, 18H, N-$CH_3$), 3,41-3,48 (m, 48H, C$\underline{H_2}$-NH, CO-$CH_2$-N), 3,72 (d, $^2J_{HP}$ = 10,7 Hz, 144H, OMe), 6,97-7,15, 7,50-7,64 (m, 90H, $H_{Ar}$, CH=N, NH) ppm.
RMN $^{13}C-\{^1H\}$ ($CDCl_3$) $\delta$ = 33,0 (d, $^2J_{CP}$ = 12,0 Hz, $CH_3$-N), 35,0 (s, $C_6H_4$-$\underline{C}H_2$), 40,4 (s, $CH_2$-NH), 49,9 (dd, $^1J_{CP}$ =158,4 Hz, $^3J_{CP}$ = 6,0 Hz, $CH_2P$), 52,8 (s, OMe), 60,8 (sl, CO-$\underline{C}H_2$-N), 121,3 (s, $C_0^2$, $C_1^2$), 128,3 (s, $C_0^3$), 129,8 (s, $C_1^3$), 132,2 (s, $C_0^4$), 136,2 (s, $C_1^4$), 138,8 (d, $^3J_{CP}$ = 11,1 Hz, CH=N), 149,9 (d, $^2J_{CP}$ = 6,0 Hz, $C_1^1$), 151,2 (sl, $C_0^1$), 169,6 (s, CONH) ppm.

*Etape 2 : Synthèse du dendrimère phosphoré de type Gc présentant 12 extrémités acide amido-azabis-phosphonique dérivé de la glycine en surface*

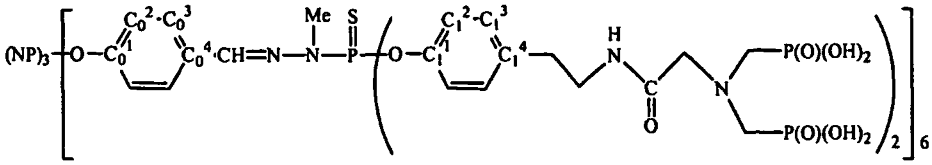

0,015 mmol de dendrimère à extrémité amido-azabis-phosphonate précédemment décrits dans l'étape 1 sont mis en solution sous atmosphère inerte dans 3 mL d'acétonitrile distillé. La solution est placée à 0°C puis 48 équivalents de BrTMS (0,73 mmol) sont ajoutés goutte à goutte sous argon. Le mélange est agité pendant 30 minutes à 0°C puis une nuit à température ambiante. Après méthanolyse et hydrolyse comme décrit dans le protocole habituel (ie DAB et PAMAM), le résidu sec est lavé avec de l'éther sec pour conduire au produit pur avec un rendement de 63%.
RMN $^{31}P-\{^1H\}$ ($D_2O$, THFd8) $\delta$ =11,9 (s, $PO_3H_2$), 12,8 (s, $N_3P_3$), 66,5 (s, P=S) ppm.

*Etape 3 : dendrimère phosphoré de première génération à extrémités [(carbamoylméthyl-amino)-méthyl]-acide phosphonique (sel de Na)*

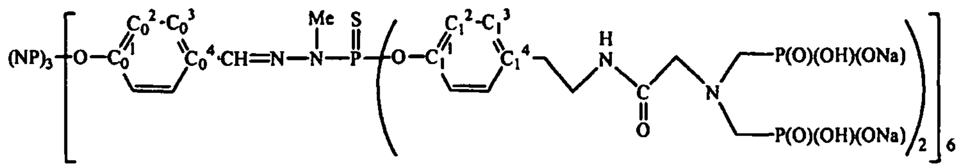

Le sel de sodium est obtenu par réaction de 24 équivalents de solution de soude à 0,1955N sur un équivalent de dendrimère obtenu dans l'étape 2, pour donner après une heure d'agitation à température ambiante et lyophilisation le produit attendu avec un rendement de 70%.
RMN $^{31}P-\{^1H\}$ ($D_2O$/THFd8) δ =12,8 (s, $N_3P_3$), 16,5 (s, $PO_3HNa$), 66,8 (s, P=S) ppm.
RMN $^{13}C-\{^1H\}$ ($D_2O$/THFd8) δ = 33,2 (sl, $CH_3$-N), 37,0 (s, $C_6H_4$-$\underline{C}H_2$), 43,6 (s, $CH_2$-NH), 58,1 (d, $^1J_{CP}$ = 140,7 Hz, $CH_2$-P=O), 62,0 (sl, CO-$\underline{C}H_2$-N), 121,7 (s, $C_0^2$, $C_1^2$), 123,6 (s, $C_1^3$), 130,9 (s, $C_0^3$), 132,8 (s, $C_1^4$), 136,5 (s, $C_0^4$), 139,6 (sl, CH=N), 151,5 (sl. $C_1^1$), 153,4 (sl, $C_0^1$), 172,8 (s, CONH) ppm.

**Exemple 68 : Synthèse de dendrimères phosphorés de type Gc présentant 12 extrémités amido-azabis-phosphonique dérivé de l'acide butyrique en surface**

*Etape 1 : Couplage du phénol à extrémité [carbamoyl-propyl-amino-méthyl] diméthylesterphoshonique avec le dendrimère phosphoré de première génération*

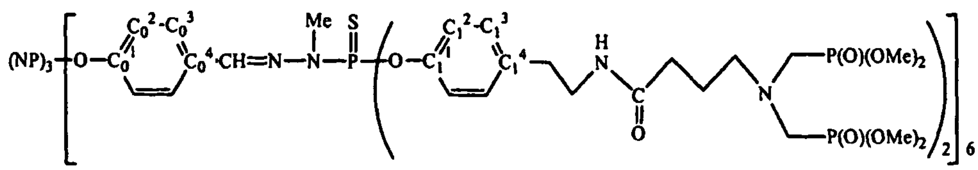

0,017 mmol de dendrimère phosphoré de génération 1 (12 extrémités Cl) sont mis en solution dans 3 mL de THF sec. Sur cette solution on ajoute successivement 5,04 mmol de carbonate de césium puis 0,23 mmol de composé tyramine-amido-azabis-phosphonate obtenu dans l'**Exemple 53** (n = 3) en solution dans 3 mL de THF sec. Le mélange est agité une nuit à température ambiante puis filtré sur célite. Le milieu réactionnel est évaporé sous pression réduite puis le résidu sec est dissous dans un volume minimum de dichlorométhane. Le produit est alors précipité dans un grand volume d'éther. Cette opération est répétée trois fois pour éliminer le léger excès de phénol de départ. Le produit est obtenu sous forme d'une poudre blanc cassé avec un rendement de 85 %.
RMN $^{31}P-\{^1H\}$ ($CDCl_3$) δ = 11,9 (s, $N_3P_3$), 30,5 (s, $PO_3Me_2$), 66,5 (s, P=S) ppm.
RMN $^1H$ ($CDCl_3$) δ = 1,67 (quint, $^3J_{HH}$ = 6,2 Hz, 24H, $CH_2$-C-$\underline{H_2}$-$CH_2$), 2,19 (t, $^3J_{HH}$ = 6,2 Hz, 24H, CO-C$\underline{H_2}$-$CH_2$-$CH_2$), 2,67-2,70 (m, 48H, CO-$CH_2$-$CH_2$-C$\underline{H_2}$, $C_6H_4$-C$\underline{H_2}$), 3,04 (d, $^2J_{HP}$ = 8,9 Hz, 48H, P-$CH_2$), 3,18 (d, $^3J_{HP}$ = 10,2 Hz, 18H, N-$CH_3$), 3,33-3,42 (m, 24H, C$\underline{H_2}$-NH), 3,70 (d, $^3J_{HP}$ = 10,5 Hz, 144H, $CH_3$-O), 6,91-7,04 (m, 72H, NHCO, $H_{Ar}$), 7,56-7,61 (m, 18H, $H_{Ar}$, CH=N) ppm.
RMN $^{13}C-\{^1H\}$ ($CDCl_3$) δ = 24,1 (s, $CH_2$-$\underline{C}H_2$-$CH_2$), 33,0 (d, $^2J_{CP}$ = 11,8 Hz, $CH_3$-N), 33,4 (s, CO-$\underline{C}H_2$), 34,9 (s, $C_6H_4$-$\underline{C}H_2$), 40,5 (s, $CH_2$-NH), 49,5 (dd, $^1J_{CP}$ = 159,3 Hz, $^3J_{CP}$ = 6,9 Hz, $CH_2P$), 52,7 (d, $^2J_{CP}$ = 4,9 Hz, $OCH_3$), 56,1 (t, $^3J_{CP}$ = 6,9 Hz, CO-$\underline{C}H_2$-N), 121,1 (s, $C_0^2$), 121,2 (s, $C_1^2$), 128,3 (s, $C0^3$), 129,8 (s, $C_1^3$), 132,2 (s, $C_0^4$), 136,5 (s, $C_1^4$), 138,7 (d, $^3J_{CP}$ = 13,9 Hz, CH=N), 148,9 (d, $^2J_{CP}$ = 6,9 Hz, $C_1^1$), 151,2 (sl, $C_0^1$), 173,4 (s, CONH) ppm.

*Etape 2: Synthèse du dendrimère phosphoré de type Gc présentant 12 extrémités acide amido-azabis-phosphonique dérivé de l'acide butyrique en surface*

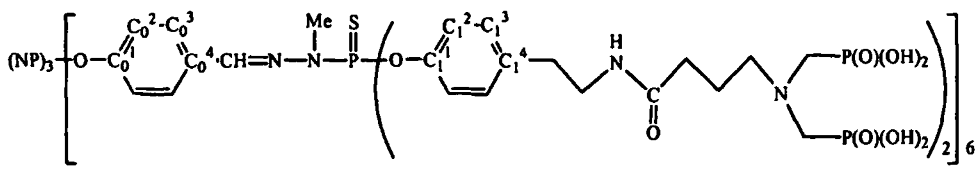

0,015 mmol de dendrimères à extrémité amido-azabis-phosphonate précédemment décrits dans l'étape 1 sont mis en solution sous atmosphère inerte dans 3 mL d'acétonitrile distillé. La solution est placée à 0°C puis 48 équivalents de BrTMS (0,73 mmol) sont ajoutés goutte à goutte sous argon. Le mélange est agité pendant 30 minutes à 0°C puis une nuit à température ambiante. Après méthanolyse et hydrolyse comme décrit dans le protocole habituel (ie DAB et PAMAM), le résidu sec est lavé avec de l'éther sec pour conduire au produit pur avec un rendement de 58%.
RMN $^{31}P\{^1H\}$ ($D_2O$, THFd8) δ =12,1 (s, $PO_3H_2$), 12,8 (s, $N_3P_3$), 66,5 (s, P=S) ppm.

Etape 3 : *dendrimère phosphoré de première génération à extrémités [(carbamoylpropyl-amino)-méthyl]-acide phosphonique (sel de Nα)*

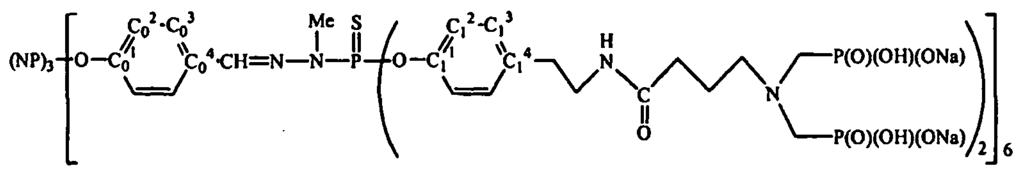

Le produit à extrémités aza-bis-phosphonique ainsi obtenus dans l'étape 2 est mis en présence de 24 équivalents de solution de soude à 0,1955N pour obtenir le sel de sodium correspondant avec un rendement de 72% après une heure d'agitation à température ambiante et lyophilisation.
RMN $^{31}P$-$\{^1H\}$ ($D_2O$/THFd8) δ =10,3 (s, $PO_3HNa$), 66,8 (s, P=S) ($N_3P_3$ non observé) ppm
RMN $^{13}C$-$\{^1H\}$ ($D_2O$/THFd8) δ= 22,8 (s, $CH_2$-$\underline{C}H_2$-$CH_2$), 35,0 (sl, $CH_3$-N, CO-$\underline{C}H_2$), 36,8 (s, $C_6H_4$-$\underline{C}H_2$), 43,4 (s, $CH_2$-NH), 53,4 (s, CO-$\underline{C}H_2$-N), 57,4 (d, $^1J_{CP}$ = 146,6 Hz, $CH_2P$), 121,9 (sl, $C_0^2$, $C_1^2$), 123,7 (s, $C_1^3$), 131,0 (s, $C_0^3$), 132,6 (s, $C_1^4$), 136,1 (s, $C_0^4$), 139,3 (s, CH=N), 151,5 (s1, $C_1^1$), 153,6 (s, $C_0^1$), 176,6 (s, CONH) ppm.

**Exemple 69: Dendrimères de type polyaryléther à extrémités acide phosphonique dérivé de la glycine**

*Etape 1 : couplage de l'acide acétique amino (bis-(diméthoxy-phosphorylméthyl)] avec un dendrimère de deuxième génération de type polyaryléther*

Les dendrimères de type polyaryléther (PAE) à terminaisons hydrazine ont été préparés selon la procédure décrite dans la littérature [K. Kono, M. Liu, J. M. J. Fréchet, *Bioconjugate Chem.* **1999**, *10,* 1115-1121].

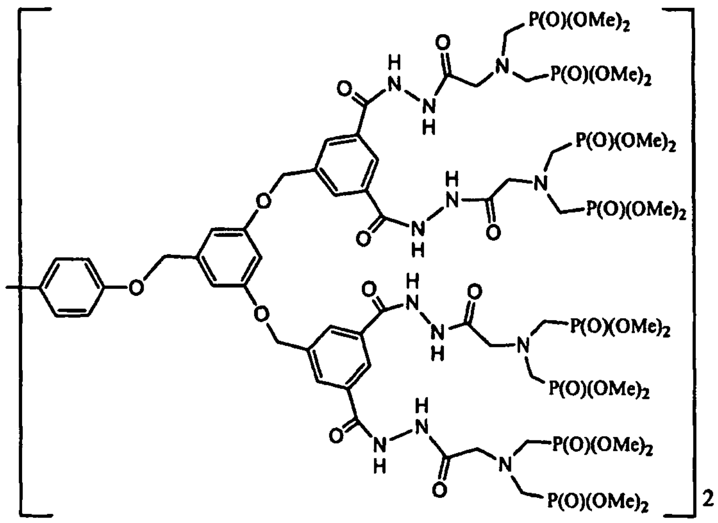

2 mmol d'acide carboxylique azabis-phosphonate obtenu dans l'**Exemple 51** (n = 1) sont mis en solution dans 4 mL de DMF sec à 0°C. On additionne alors 1,2 équivalents de HOBt et on maintient l'agitation à 0°C pendant 30 minutes. 1,2 équivalents de DCC sont ajoutés et le mélange est agité pendant 30 minutes à 0°C puis pendant 1 heure à température ambiante. Le mélange est à nouveau porté à 0°C et 225 mg (0,18 mmol) de dendrimère PAE possédant 8 extrémités hydrazide en solution dans 2 mL de DMF sec sont additionnés. L'agitation est maintenue pendant 30

minutes à 0°C puis pendant 24 heures à température ambiante. Le précipité est éliminé sur filtre millipore (5 μ) puis le DMF est lyophilisé. Le produit est traité trois fois par le procédé suivant : dissolution dans un volume minimum de dichlorométhane puis précipitation dans un grand volume de diéthyléther. Le dendrimère est obtenu avec un rendement de 63 %.
RMN $^{31}P-\{^1H\}$ ($CDCl_3$) $\delta$ = 29,9 ppm.

*Etape 2 : dendrimère PAE de deuxième génération à extrémités [(carbamoylméthyl-amino)-méthyl]-acide phosphonique (sel de Nα)*

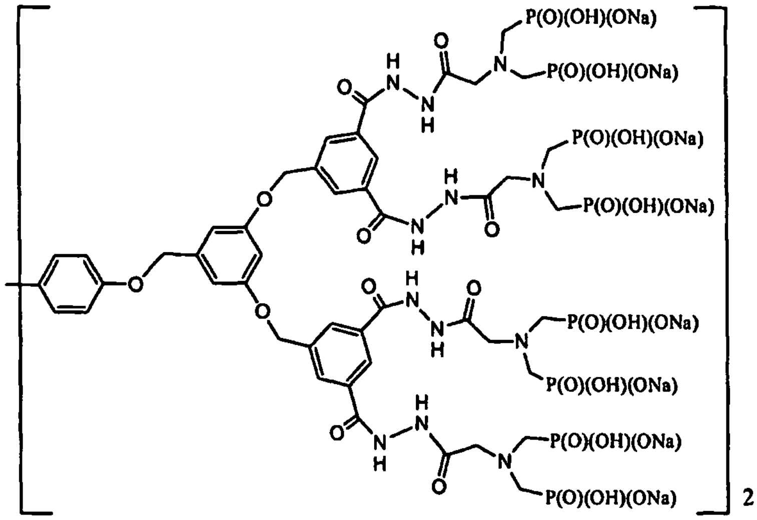

A 0,11 mmol de dendrimère PAE à extrémités aza-bis-phosphonate de l'étape 1 on ajoute sous atmosphère inerte 4 mL d'acétonitrile fraîchement distillé et le mélange est refroidi à 0°C. On ajoute alors goutte à goutte 3,87 mmol de BrTMS (soit 35,2 équivalents). Le mélange est maintenu à 0°C pendant 30 minutes puis sous agitation à température ambiante pendant 15 heures supplémentaires. L'acétonitrile est éliminé sous pression réduite puis le mélange est méthanolysé et hydrolysé. Après lyophilisation, le résidu sec est lavé avec un mélange THF / éther (1 / 9). L'addition de 1 équivalent de NaOH (0,1955N en solution aqueuse) par fonction $PO_3H_2$ de surface conduit au sel de sodium correspondant. Après lyophilisation, le produit est obtenu avec un rendement de 75%.
RMN $^{31}P-\{^1H\}$ ($D_2O$ / $CD_3COCD_3$) $\delta$ =19,1 ppm.

**Exemple 70: Dendrimères de type polyaryléther à extrémités acide phosphonique dérivé de l'acide butyrique**

*Etape 1 : couplage de l'acide butyrique amino [bis-(diméthoxy-phosphorylméthyl)] avec un dendrimère de deuxième génération de type polyaryléther*

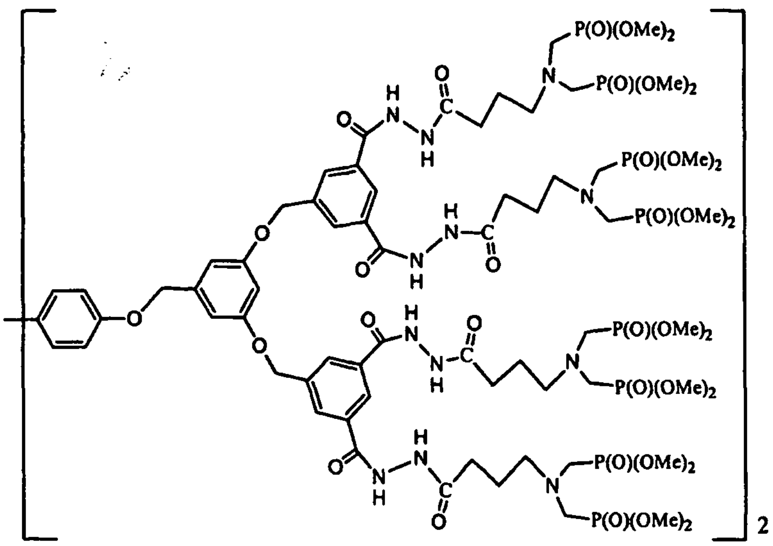

2 mmol d'acide carboxylique aza-bis-phosphonate obtenu dans l'**Exemple 51** (n = 3) sont mis en solution dans 4 mL de DMF sec à 0°C. On additionne alors 1,2 équivalents de HOBt et on maintient l'agitation à 0°C pendant 30 minutes. 1,2 équivalents de DCC sont ajoutés et le mélange est agité pendant 30 minutes à 0°C puis pendant 1 heure à température ambiante. Le mélange est à nouveau porté à 0°C et 225 mg (0,18 mmol) de dendrimère PAE possédant 8 extrémités hydrazide en solution dans 2 mL de DMF sec sont additionnés. L'agitation est maintenue pendant 30 minutes à 0°C puis pendant 24 heures à température ambiante. Le précipité est éliminé sur filtre millipore (5 $\mu$) puis le DMF est lyophilisé. Le produit est traité trois fois par le procédé suivant : dissolution dans un volume minimum de dichlorométhane puis précipitation dans un grand volume de diéthyléther. Le dendrimère est isolé avec un rendement de 68 %.
RMN $^{31}P-\{^1H\}$ ($CDCl_3$) $\delta$ = 30,2 ppm.

*Etape 2 : dendrimère PAE de deuxième génération à extrémités [(carbamoylpropyl-amino)-méthyl]-acide phosphonique (sel de N$\alpha$)*

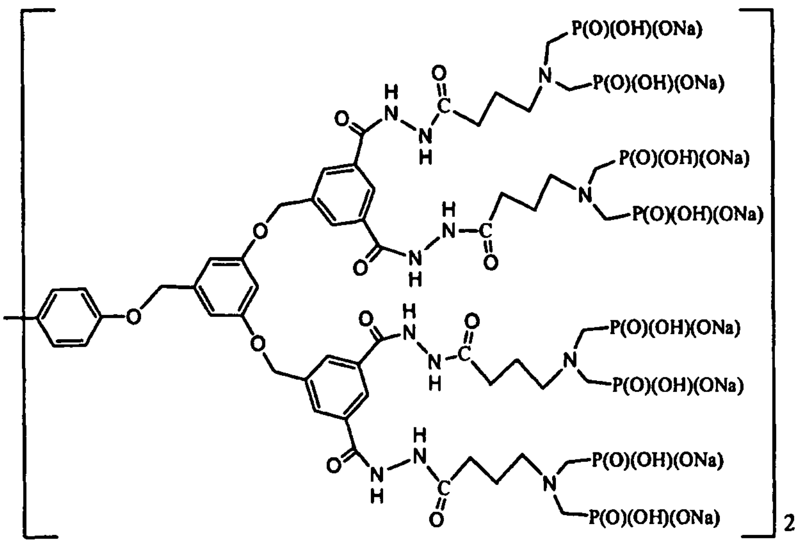

A 0,11 mmol de dendrimère à extrémités aza-bis-phosphonate de l'étape 1 on ajoute sous atmosphère inerte 4 mL d'acétonitrile fraîchement distillé et le mélange est refroidi à 0°C. On ajoute alors goutte à goutte 3,87 mmol de BrTMS (soit 35,2 équivalents). Le mélange est maintenu à 0°C pendant 30 minutes puis sous agitation à température ambiante pendant 15 heures supplémentaires. L'acétonitrile est éliminé sous pression réduite puis le mélange est

méthanolysé et hydrolysé. Après lyophilisation, le résidu sec est lavé avec un mélange THF / éther (1 / 9). L'addition de 1 équivalent de NaOH (0,1955N en solution aqueuse) par fonction $PO_3H_2$ de surface conduit au sel de sodium correspondant. Après lyophilisation, le produit est obtenu avec un rendement de 71 %.
RMN $^{31}P$-{$^1H$} ($D_2O$ / $CD_3COCD_3$) $\delta$ =10,4 ppm.

**Exemple 71 : synthèse d'un dendrimère à surface tyrosine aza-bis-phosphonate**

*Etape 1 : dérivé bis phosphonate de la tyrosine*

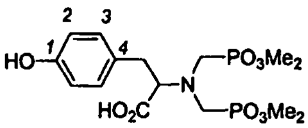

A une suspension de (D-L) tyrosine (2g, 11,05 mmol) dans 4 mL de THF sont ajoutés du formaldéhyde aqueux (2,5 mL, 30,8 mmol, 2,8 éq.) et du phosphite de diméthyle (3 mL, 32,7 mmol, 2,96 éq.). La suspension est agitée une nuit à température ambiante et la solution homogène résultante est concentrée sous pression réduite. Le résidu est lavé avec 2 fois 15 mL d'AcOEt et 2 fois 15 mL de $CH_2Cl_2$. Le solide est ensuite séché sous pression réduite. Le phénol bis phosphonate est obtenu sous forme d'une poudre blanche avec un rendement de 85 %.
RMN $^{31}P\{^1H\}$ ($CD_3OD$, 81,01 MHz) : $\delta$ = 31,0 (s, P=O).
RMN $^1H$ ($CD_3OD$, 200,13 MHz) : $\delta$ = 2,81 (dd, 2H, $^2J_{HH}$ = 14,1 Hz, $^3J_{HH}$ = 7,4 Hz, CHHCH); 3,04 (dd, 2H, $^2J_{HH}$ = 14,1 Hz, $^3J_{HH}$ = 7,2 Hz, CHHCH); 3,22-3,51 (m, 4H, $PCH_2$); 3,70 (d, 6H, $^3J_{HP}$ = 8,1 Hz, POMe); 3,76 (d, 6H, $^3J_{HP}$ = 8,1 Hz, POMe); 4,21 (t, $^3J_{HP}$ = 7,3 Hz, CH); 6,70 (d, 2H, $^3J_{HH}$ = 8,5 Hz, $C^2$-H); 7,13 (d, 2H, $^3J_{HP}$ = 8,5 Hz, $C^3$-H).
RMN $^{13}C\{^1H\}$ ($CD_3OD$, 62,89 MHz) : $\delta$ = 35,0 (s, $CH_2$); 47,3 (dd, $^1J_{CP}$ = 167,3 et $^3J_{CP}$ = 9,3 Hz, $PCH_2$); 52,9 (d, $^2J_{CP}$ = 7,0 Hz, POMe); 53,3 (d, $^2J_{CP}$ = 7,0 Hz, POMe); 66,6 (t, $^3J_{CP}$ = 6,9 Hz, CH); 115,4 (s, $C^2$); 129,2 (s, $C^4$); 130,9 (s, $C^3$); 156,4 (s, $C^1$); 173,8 (COOH).

*Etape 2 : méthylation de l'acide*

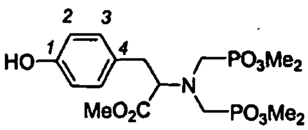

Une solution de tyrosine bis-phosphonate synthétisée à l'étape précédente (760 mg, 1,79 mmol) dans 12 mL de méthanol est chauffée à reflux pendant 36 heures en présence d'une quantité catalytique d'acide para-toluène sulfonique. Après refroidissement la solution est filtrée puis concentrée sous pression réduite. L'huile résiduelle est lavé avec un mélange éther/ pentane puis précipité dans un mélange THF / $Et_2O$ / pentane avec un rendement de 93 %.
RMN $^{31}P\{^1H\}$ ($CDCl_3$, 121,5 MHz) : $\delta$ = 29,6 (s, P=O).
RMN $^1H$ ($CDCl_3$, 200,13 MHz) : $\delta$ = 2,87 (dd, 2H, $^2J_{HH}$ = 13,7 Hz et $^3J_{HH}$ = 8,0 Hz, CHHPh); 3,17 (dd, 2H, $^2J_{HH}$ = 16,0 Hz et $^2J_{HP}$ = 5,7 Hz, CHHP); 3,29 (t, 2H, $^2J_{HH}$ = $^2J_{HP}$ = 16,0 Hz, CHHP); 3,59 (s, 3H, COOMe); 3,69 (d, 6H, $^3J_{HP}$ = 11,8 Hz, POMe); 3,70 (d, 6H, $^3J_{HP}$ = 10,6 Hz, POMe); 4,38 (t, 1H, $^3J_{HH}$ = 7,0 Hz, CH); 6,76 (d, 2H, $^3J_{HH}$ = 8,3 Hz, $C^2$-H); 7,08 (d, 2H, $^3J_{HH}$ = 8,3 Hz, $C^3$-H); 8,50 (sl, OH).
RMN $^{13}C\{^1H\}$ ($CDCl_3$, 75,5 MHz) : $\delta$ = 35,6 (s, $CH_2Ph$); 47,6 (dd, $^1J_{CP}$ = 166,8 Hz et $^3J_{CP}$ = 9,8 Hz, $PCH_2$); 51,8 (s, OMe); 53,9 (d, $^3J_{CP}$ = 6,8 Hz, POMe); 53,8 (d, $^3J_{CP}$ = 6,8 Hz, POMe); 66,5 (t, $^3J_{CP}$ = 6,8 Hz, CH); 115,7 (s, $C^2$); 128,2 (s, $C^4$); 130,7 (s, $C^3$); 156,2 (s, $C^1$); 172,6 (s, $CO_2Me$).

*Etape 3 : greffage sur dendrimère*

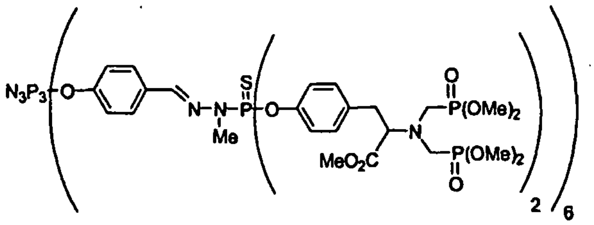

A une solution de dendrimère Gc1 (143 mg, 78,3 μmol) à terminaison $S=PCl_2$ dans le THF sont ajoutés 12,2 équivalents de phénol synthétisé à l'étape précédente (420 mg, 0,956 mmol) et 15 équivalents de $Cs_2CO_3$ (383 mg, 1,175 mmol). La suspension résultante est agitée (26 heures) jusqu'à substitution complète des chlores (suivi par RMN $^{31}P$). Le mélange est décanté, le surnageant est collecté et le solide résiduel est lavé au THF. Les surnageants sont réunis et centrifugés. La solution limpide obtenue est concentrée sous pression réduite. Le résidu est dissous dans un minimum de THF puis précipité au pentane. Le solide obtenu est purifié par lavage (THF / $Et_2O$ et $CH_2Cl_2$ / pentane) avec un rendement de 73 %.
RMN $^{31}P\{^1H\}$ ($CDCl_3$, 121,5 MHz) : δ =11,5 (s, $N_3P_3$); 29,6 (s, P=O); 66,4 (s, P=S).
RMN $^1H$ ($CDCl_3$, 400,13 MHz) : δ = 2,82-3,10 (m, 24H, $CH_2Ar$); 3,10-3,50 (m, 66H, $PCH_2$ et $NCH_3$); 3,62 (d, 72H, $^3J_{HP}$ = 10,6 Hz, POMe); 3,68 (d, 72H, $^3J_{HP}$ = 10,6 Hz, POMe); 3,75 (s, 36H, COOMe); 4,41 (t, 12H, $^3J_{HH}$ = 6,9 Hz, CH); 7,05 (m, 36H, $C_0^2$-H et $C_1^2$-H); 7,24 (m, 24H, $C_1^3$-H); 7,61 (m, 18H, $C_1^3$-H et CH=N).
RMN $^{13}C\{^1H)$ ($CDCl_3$, 62,89 Hz) : δ = 32,8 (d, $^2J_{CP}$ = 11,9 Hz, $NCH_3$); 35,2 (s, $CH_2Ph$); 46,9 (dd, $^1J_{CP}$ = 165,5 et $^3J_{CP}$ = 9,18 Hz, $PCH_2$); 51,5 (s, OMe); 52,4 (d, $^2J_{CP}$ = 7,4 Hz, POMe); 53,1(d, $^2J_{CP}$ = 6,3 Hz, POMe); 65,2 (s, CH); 121,1 (s, $C_0^2$ et $C_1^2$); 128,2 (s, $C_0^3$); 130,5 (s, $C_1^3$); 132,0 (s, $C_0^4$); 134,6 (s, $C_1^4$); 138,8 (d, $^3J_{CP}$ = 14,0 Hz, CH=N); 149,1 (d, $^2J_{CP}$ = 6,5 Hz, $C_1^1$); 151,2 (sl, $C_0^1$); 171,8 (s, $CO_2Me$).

*Etape 4 : dendrimère phosphoré de première génération à extrémités acide bisphosphonique (sel de Nα)*

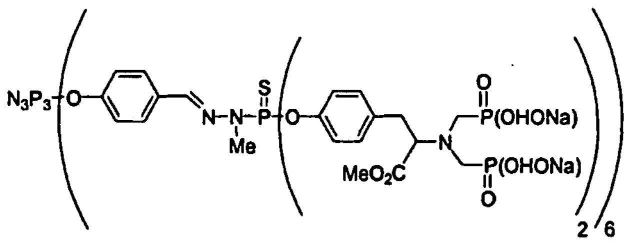

A une solution de dendrimère à terminaisons phosphonate de diméthyle synthétisé à l'étape précédente (150 mg, 22,5 μmol) dans l'acétonitrile sont additionnés à 0°C sous flux d'argon 75 équivalents (222 μL, 1,68 mmol) de bromotriméthylsilane. La solution est agitée une nuit à température ambiante puis concentrée sous pression réduite. 5 mL de méthanol sont ajoutés et le mélange est vigoureusement agité pendant 2 heures. Le méthanol est éliminé sous pression réduite et le résidu est lavé avec de l'éther distillé, puis de l'eau et du méthanol. Le solide est séché sous pression réduite jusqu'à obtention d'une poudre. Une solution de soude (0,1966 M, 2,72 mL, 24 éq.) est additionnée lentement sur le solide. La solution obtenue est filtrée puis lyophilisée. Le dendrimère à terminaisons acide amino bis-phosphonique est obtenu sous forme d'une poudre blanche avec un rendement de 91 %.
RMN $^{31}P\{^1H\}$ ($D_2O/CD_3CN$ 81,01 MHz) : δ =12,6 (s, $N_3P_3$); 20,9 (s, P=O); 67,5 (sl, P=S).

**Exemple 72 : synthèse d'un dendrimère à surface benzyl-aza-bis-phosphonate**

*Etape 1: phénol azabisphosphonate*

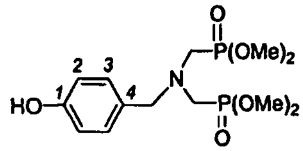

A une solution de 4-hydroxybenzylamine (500 mg, 4,06 mmol) dans le THF (7 mL) est additionnée une solution aqueuse de formaldéhyde (37 % dans l'eau, 990 µL, 12,19 mmol, 3 éq.). Après 30 minutes d'agitation, 3 équivalents (1,12 mL, 12,2 mmol) de phosphite de diméthyle sont additionnés. La solution est agitée à température ambiante pendant 48 heures puis versée dans 150 mL d'AcOEt. La phase organique est lavée avec une solution de $NaHCO_3$ et de saumure puis séchée sur $MgSO_4$ et concentrée sous pression réduite. Le résidu est chromatographié sur colonne à gel de silice (éluant : acétone / MeOH; 95:5) avec un rendement de 72 %.
RMN $^{31}P\{^1H\}$ ($CDCl_3$, 81,01 MHz) : $\delta$ = 31,2 (s, P=O).

*Etape 2 : greffage sur dendrimère phosphoré de première génération*

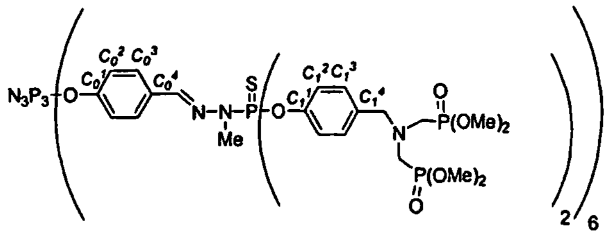

A une solution de dendrimère Gc1 (105 mg, 57,6 µmol) à terminaison $S=PCl_2$ dans le THF sont ajoutés 13 équivalents (275 mg, 0,75 mmol) de phénol synthétisé à l'étape précédente (solubilisé dans le THF) et 15 équivalents (281 mg, 0,864 mmol) de $Cs_2CO_3$. La suspension résultante est agitée jusqu'à substitution complète des chlores (suivi par RMN $^{31}P$). Le mélange est décanté, le surnageant est collecté et le solide résiduel est lavé au THF. Les surnageants sont réunis et centrifugés. La solution limpide obtenue est concentrée sous pression réduite. Le résidu est dissous dans un minimum de THF puis précipité au pentane. Le solide obtenu est purifié par lavage (THF / pentane et THF /$Et_2O$) avec un rendement de 90 %.
RMN $^{31}P\{^1H\}$ ($CDCl_3$, 81,01 MHz) : $\delta$ = 11,3 (s, $N_3P_3$); 31,0 (s, P=O); 65,6 (s, P=S).

*Etape 3 : dendrimère phosphoré de première génération à extrémités acide bisphosphonique (sel de Nα)*

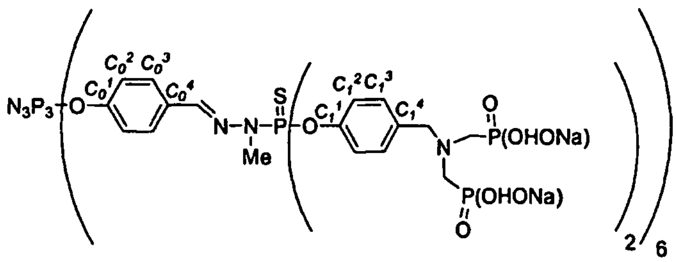

A une solution de dendrimère à terminaisons phosphonate de diméthyle synthétisé à l'étape précédente (180 mg, 31 µmol) dans l'acétonitrile sont additionnés à 0°C sous flux d'argon 60 équivalents (245 µL, 1,86 mmol) de bromotriméthylsilane. La solution est agitée une nuit à température ambiante puis concentrée sous pression réduite. 5 mL de méthanol sont ajoutés et le mélange est vigoureusement agité pendant 2 heures. Le méthanol est éliminé sous pression réduite et le résidu est lavé avec de l'éther distillé, puis de l'eau et du méthanol. Le solide est séché sous pression réduite jusqu'à obtention d'une poudre. Une solution de soude (0,1966 M, 3,78 mL, 24 éq.) est additionnée lentement sur le solide. La solution obtenue est filtrée puis lyophilisée. L'acide amino bis-phosphonique est obtenu sous forme d'une poudre blanche avec un rendement de 92 %.

RMN $^{31}P\{^1H\}$ ($D_2O/CD_3CN$, 81,01 MHz) : δ = 10,8 (sl, P=O); 11,1 (sl, $N_3P_3$); 65,2 (s, P=S).

**Exemple 73 : Synthèse du coeur cyclotétraphosphazène octaaldéhyde**

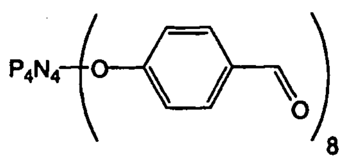

Dans 100 mL de tétrahydrofuranne sont ajoutés l'octachlorocyclotétraphosphazène (2,15 mmol, 1g) et de l'hydrure de sodium (18,9 mmol, 454 mg), l'ensemble est mis à -20°C avec une agitation magnétique. Puis on additionne goutte à goutte le 4-hydroxybenzaldéhyde (18,9 mmol, 2,31 g) en solution dans le THF (20 mL). Après addition le mélange remonte lentement à température ambiante puis est laissé sous agitation pendant 48 heures. Après filtration sur célite, le produit est lavé plusieurs fois avec du méthanol froid pour enlever l'excès de sel de sodium du 4-hydroxybenzaldéhyde. Le produit est obtenu sous forme de poudre blanche avec un rendement de 90%.
RMN $^{31}P$-$\{^1H\}$ ($CDCl_3$) : δ =-10,5 (s, P) ppm.
RMN $^1H$ ($CDCl_3$) : δ = 7,04 (d, $^3J_{HH}$ = 8,4 Hz, 16H, $CH_{arom}$); 7,56 (d, $^3J_{HH}$ = 8,6 Hz, 16H, $CH_{arom}$); 9,68 (s, 8H, CH=O) ppm.
RMN $^{13}C$-$\{^1H\}$ ($CDCl_3$) : δ = 121,9 (s, $C_0^2$); 132,1 (s, $C_0^3$); 134,6 (s, $C_0^4$); 155,7 (s, $C_0^1$); 191,5 (s, CHO) ppm.

**Exemple 74 : Synthèse d'un dendrimère à coeur cyclotétraphosphazène et à surface dichloro-thio-phosphorhydrazide**

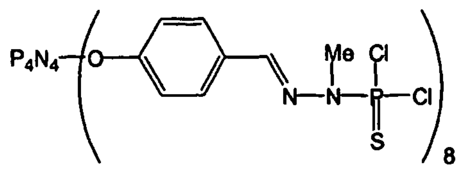

Le coeur cyclotétraphosphazène octaaldéhyde obtenu dans l'**Exemple 73** (0,87 mmol, 1g) est placé sans solvant dans un ballon. On additionne sur cette poudre rapidement et à - 20°C le dichloro-thio-phosphorhydrazide (7,66 mmol, 33,3 mL) en solution dans le chloroforme à 0,23 $mol.L^{-1}$. Une fois l'addition terminée le mélange est maintenu sous agitation magnétique et à température ambiante pendant 12 heures. On évapore le solvant du mélange réactionnel puis on reprend trois fois le produit dans un minimum de tétrahydrofuranne pour le précipiter dans du pentane. Le produit est isolé sous forme d'une poudre blanche avec un rendement de 86%.
RMN $^{31}P$-$\{^1H\}$ ($CDCl_3$) : δ = 65,7 (s, $P_1$); -10,0 (s, $P_0$) ppm.
RMN $^1H$ ($CDCl_3$) : δ = 3,47 (d, $^3J_{HP}$= 14,0 Hz, 24H, $CH_3$-N-$P_1$); 7,04 (d, $^3J_{HH}$= 8,4 Hz, 16H, $CH_{arom}$); 7,56 (d large, $^4J_{HP}$ = $^3J_{HH}$ = 8,6 Hz, 24H, $CH_{arom}$, CH=N) ppm.
RMN $^{13}C$-$\{^1H\}$ ($CDCl_3$) : δ = 31,9 (d, $^2J_{CP}$ = 12,9 Hz, $CH_3$-N-$P_1$); 121,2 (s, $C_0^2$); 128,5 (s, $C_0^3$); 130,8 (s, $C_0^4$); 140,7 (d, $^3J_{CP}$ = 1 8,8 Hz, CH=N); 152,3 (s, $C_0^1$) ppm.

**Exemple 75 : Synthèse d'un dendrimère à coeur cyclotétraphosphazène et à surface aza-bis-diméthyl-phosphonate dérivé de la tyramine**

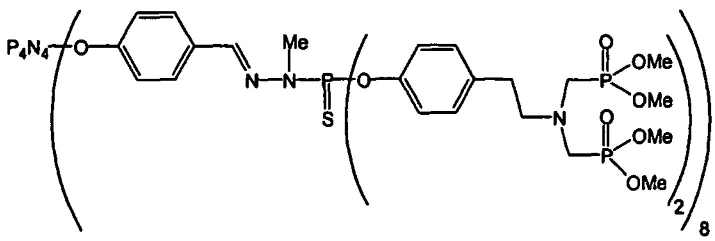

A une solution de dendrimère à coeur cyclotétraphosphazène et à surface dichloro-thio-phosphorhydrazide obtenu dans l'**Exemple 74** (0,41 mmol, 1 g) dans du THF anhydre (10 mL) sont additionnés du carbonate de césium (14,4 mmol, 4,68 g) et le phénol aza-bis-diméthyl-phosphonate dérivé de la tyramine obtenue dans l'**Exemple 31** (7,23 mmol, 2,8 g). On laisse le mélange sous agitation pendant 24 heures à température ambiante puis on filtre le mélange final sur célite avec du THF de manière à séparer les sels. Enfin le produit final est lavé par précipitation dans un mélange pentane/éther 1/1, le produit final est isolé sous forme de poudre blanche avec un rendement de 80%.
RMN $^{31}P\{^1H\}$ ($CDCl_3$) : δ = 66,8 (s, $P_1$); 30,2 (s, $P(O)(OMe)_2$); -9,4 (s, $P_0$) ppm.
RMN$^1H$ ($CDCl_3$) : δ = 2,67 (t large, $^3J_{HH}$ = 6,5 Hz, 32H, $\underline{CH_2}$-$CH_2$-N); 2,96 (t large, $^3J_{HH}$ = 6,5 Hz, 32H, $CH_2$-$\underline{CH_2}$-N); 3,10 (d, $^2J_{HP}$ = 9,57 Hz, 64H, -$\underline{CH_2}$-$P(O)(OCH_3)_2$); 3,14 (d, $^3J_{HP}$= 11,8 Hz, 24H, $CH_3$-N-$P_1$); 3,64 (d, $^3J_{HP}$ = 10,5 Hz, 192H, -P(O)(O-$\underline{CH_3})_2$); 6,8-7,8 (m; 104H, $CH_{arom}$, CH=N) ppm.
RMN $^{13}C$-{$^1H$) ($CDCl_3$) : δ = 32,9 (s large, $CH_3$-N-$P_1$); 32,9 (s large, $\underline{CH_2}$-$CH_2$-N) 49,3 (dd, $^1J_{CP}$ = 157,6 Hz, $^3J_{CP}$ = 6,7 Hz -$\underline{CH_2}$-$P(O)(OCH_3)_2$); 52,6 (d, $^2J_{CP}$ = 4,1 Hz, -P(O)(O-$\underline{CH_3})_2$); 58,1 (t, $^3J_{CP}$ = 7,8 Hz, $CH_2$-$\underline{CH_2}$-N); 121,2 (s, $C_0^2$); 121,2 (d, $^3J_{CP}$ = 3,6 Hz, $C_1^2$); 128,2 (s, $C_0^3$); 129,9 (s, $C_1^3$); 131,7 (s, $C_0^4$); 136,6 (s, $C_1^4$); 138,9 (d, $^3J_{CP}$ = 16,0 Hz, CH=N); 148,9 (d, $^2J_{CP}$= 6,1 Hz, $C_1^1$); 151,8 (s large, $C_0^1$) ppm.

**Exemple 76 : Synthèse d'un dendrimère à coeur cyclotétraphosphazène et à surface aza-bis-phosphonique dérivé de la tyramine**

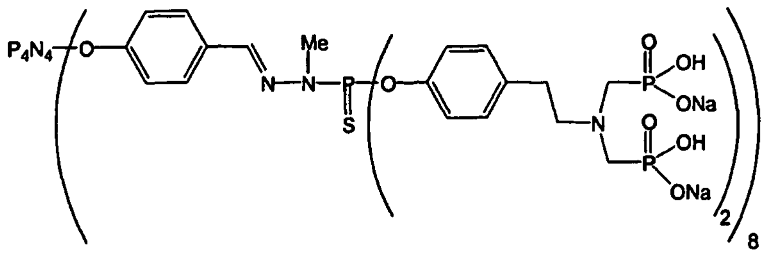

A une solution de dendrimère de première génération à extrémités aza-bis-diméthyl-phosphonate dérivé de la tyramine et à coeur cyclotétraphosphazène obtenu dans l'**Exemple 75** (8,27. $10^{-2}$ mmol, 570 mg) à 0°C dans de l'acétonitrile (5 mL) on additionne lentement du bromotriméthylsilane (5,82 mmol, 777 μl). Une fois l'addition terminée on laisse le mélange revenir à température ambiante pendant 12 heures. Le mélange est alors évaporé à sec puis on additionne 1 ml de méthanol anhydre à température ambiante et on laisse le mélange une heure sous agitation. Le produit est ensuite évaporé à sec et la même opération est effectuée avec 1 mL d'eau. Après lyophilisation, le résidu est lavé plusieurs fois à l'éther. Le produit étant totalement insoluble dans les solvants organiques il est transformé en son mono sel de sodium en présence d'une solution d'hydroxyde de sodium titrée. La solution résultante est lyophilisée pour donner le dendrimère sous forme d'une poudre blanche. Le produit final est isolé avec un rendement de 52%.
RMN $^{31}P$-{$^1H$} ($CD_3CN/D_2O$) : δ = 67,7 (s, $P_1$); 10,1 (s large, P(O)(OH)(ONa)); 8,7 (s large, P(O)(OH)(ONa)); -9,5 (s, $P_0$) ppm.
RMN $^1H$ ($CD_3CN/D_2O$) : δ = 2,5-4,2 (m, 152H, $CH_2$-$\underline{CH_2}$-N, $\underline{CH_2}$-$CH_2$-N, -$\underline{CH_2}$-P(O)(OH)(ONa), $CH_3$-N-$P_1$)); 4,84 (s large, 32H, -P(O)(OH)(ONa)); 6,7-8,1 (m, 104H, $CH_{arom}$, CH=N) ppm.
RMN $^{13}C$-{$^1H$} ($CD_3CN/D_2O$): δ = 31,4 (s large, $\underline{CH_2}$-$CH_2$-N); 35,1 (s large, $CH_3$-N-$P_1$); 55,5 (d, $^1J_{CP}$ = 169,2 Hz, -$\underline{CH_2}$-P (O)(OH)(ONa)); 59,9 (s large, $CH_2$-$\underline{CH_2}$-N); 121,6 (s large, $C_0^2$, $C_1^2$,); 123,9 (s large, $C_1^3$); 130,8 (s large, $C_0^3$); 133,2 (s large, $C_1^4$); 136,8 (s large, $C_0^4$); 142,7 (s large, CH=N); 151,7 (s large, $C_1^1$); 153,8 (s large, $C_0^1$) ppm.

**Exemple 77 : Synthèse du dendrimère de type 6-2-5-2 à surface dichlorothiophosphorhydrazide**

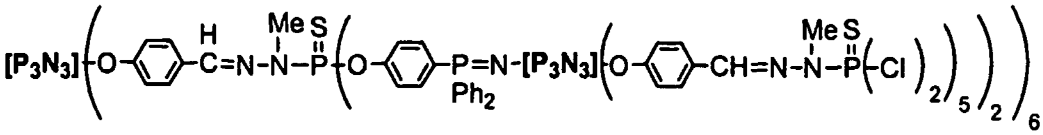

Les dendrimères hyperdenses de type 6-2-5, 6-5-2, 6-5-5 utilisés et définis ci-dessous ont été décrits dans V. Maraval et al. Angew. Chem. Int. Ed. (2003) 42, 1822.

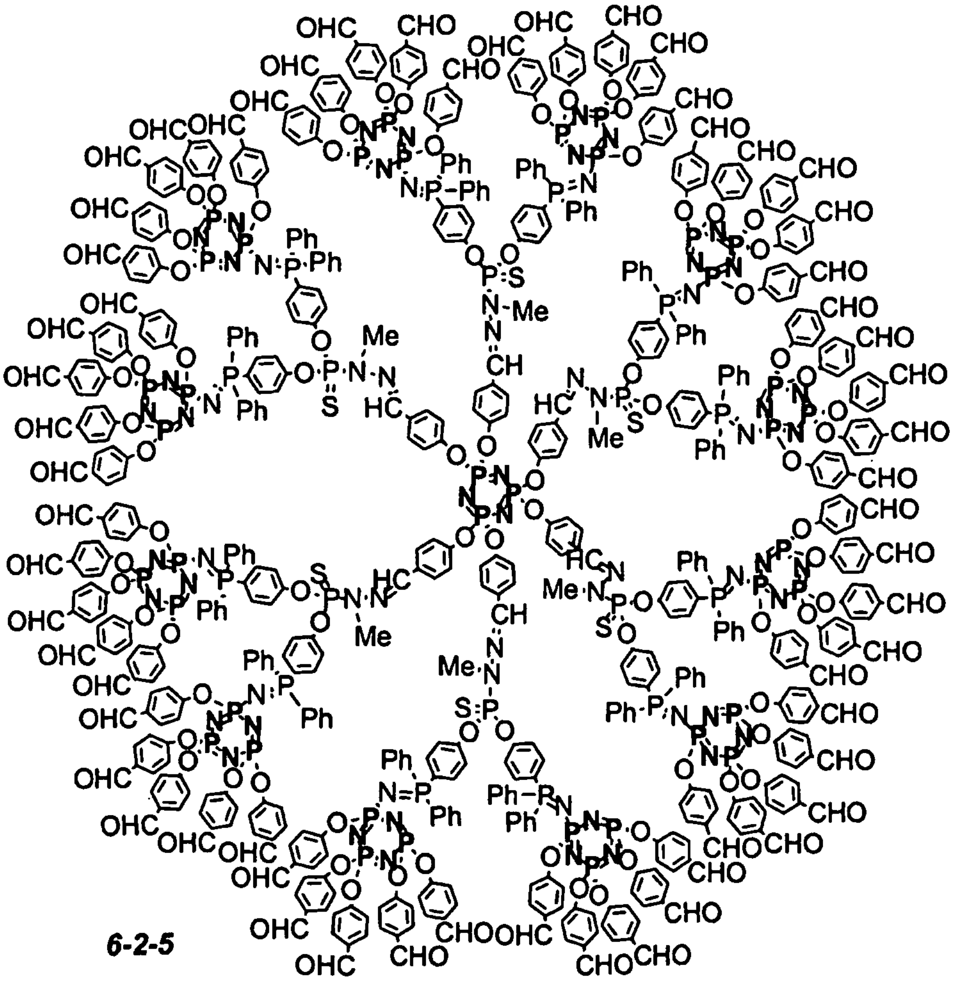

6-2-5

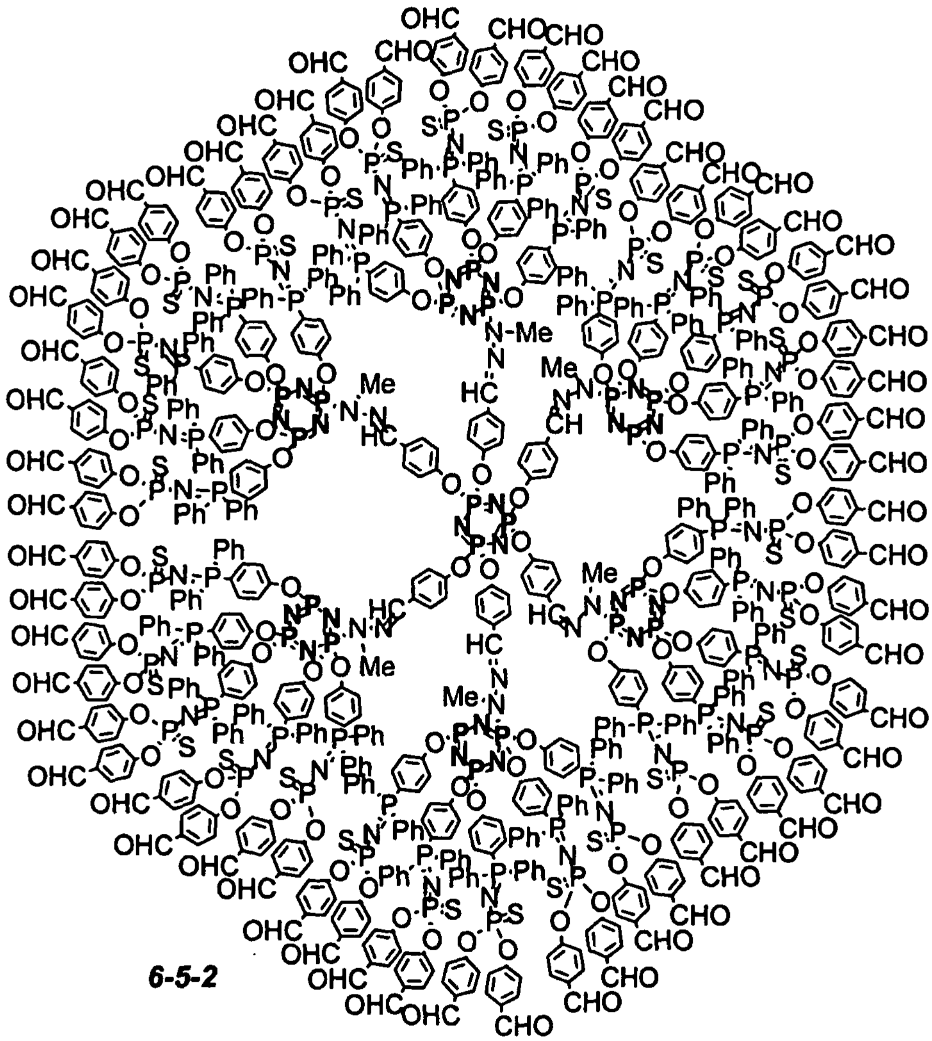

6-5-2

$[P_3N_3]$ = 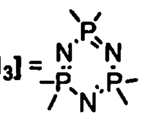

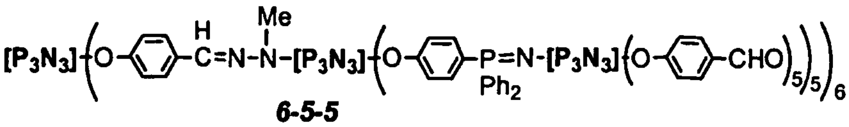

6-5-5

A une solution de dendrimère de type 6-2-5 à extrémités aldéhyde (250 mg, 18,1.10$^{-3}$ mmol) dans 2 mL de chloroforme est ajouté à -30°C un léger excès (1,1 équivalents par fonction aldéhyde) d'une solution de N-méthyl-dichlorothiophosphorhydrazide à 0,2 M dans le chloroforme. Après agitation pendant 5 à 60 minutes à une température comprise entre -30 et 0°C le mélange est filtré sur célite puis précipité par ajout de pentane. La poudre obtenue est dissoute dans le minimum de THF puis précipitée au pentane et enfin séchée pour être isolée avec un rendement final de 92%.

**Exemple 78 : Synthèse du dendrimère de type 6-2-5-2 à surface aza-bis-diméthyl-phosphonate dérivé de la tyramine**

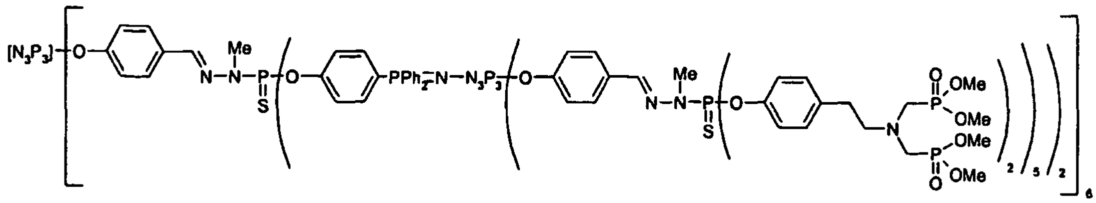

A une solution de dendrimère de type 6-2-5-2 (200 mg, 8,5.10$^{-3}$ mmol) à surface dichlorothiophosphorhydrazide obtenu dans l'**Exemple 31** dans du THF (2 mL) est additionné du carbonate de césium (1,019 mmol, 0,332 g) puis le phénol aza-bis-diméthyl-phosphonate dérivé de la tyramine est ajouté (0,510 mmol, 195 mg). On laisse le mélange sous agitation pendant 24 heures à température ambiante puis on filtre le mélange final sur célite de manière à séparer les sels. Enfin le produit final est lavé par précipitation dans du pentane et isolé avec un rendement de 85%.

**Exemple 79 : Synthèse du dendrimère de type 6-2-5-2 à surface aza-bis-diméthyl-phosphonique dérivé de la tyramine**

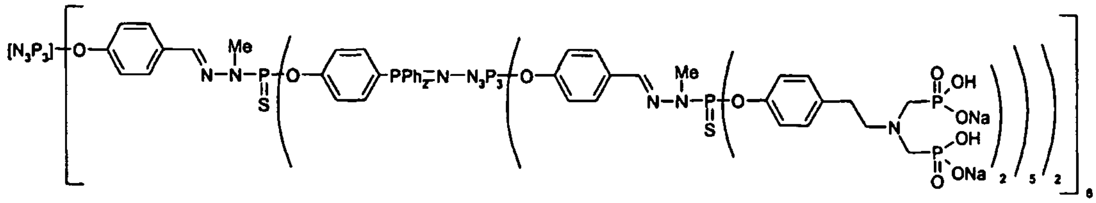

A une solution de dendrimère de type 6-2-5-2 (200 mg, 3,08.10$^{-3}$ mmol) à surface aza-bis-diméthyl-phosphonate dérivé de la tyramine obtenu dans l'**Exemple 78** à 0°C dans de l'acétonitrile (5 mL) on additionne lentement du bromo-triméthylsilane (1,1 équivalents par groupement méthoxy). Une fois l'addition terminée on laisse le mélange revenir à température ambiante pendant 12 heures. Le mélange est alors évaporé à sec puis on additionne 1 mL de méthanol anhydre à température ambiante et on laisse le mélange une heure sous agitation. Après évaporation à sec, le résidu est lavé plusieurs fois à l'éther pur. Le produit est ensuite transformé en son mono sel de sodium en présence de soude (1 équivalent de NaOH par terminaison acide phosphonique). La solution résultante est lyophilisée pour donner le dendrimère sous forme d'une poudre blanche. Le produit final est isolé avec un rendement de 52%.

**Exemple 80 : Synthèse du dendrimère de type 6-5-2-2 à surface dichlorothiophosphorhydrazide**

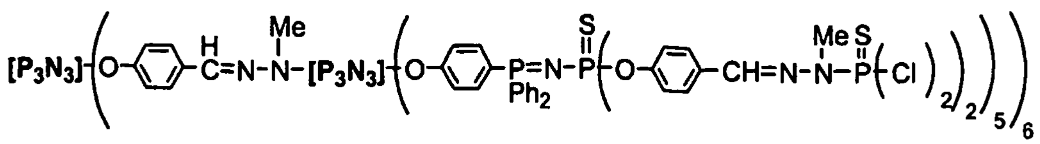

A une solution de dendrimère de type 6-5-2 (250 mg) dans 2 mL de chloroforme est ajouté à -30°C un léger excès (1,1 équivalents par fonction aldéhyde) d'une solution de N-méthyl-dichlorothiophosphorhydrazide à 0,2 M dans le chloroforme. Après agitation pendant 5 à 60 minutes à une température comprise entre -30 et 0°C le mélange est filtré sur célite puis précipité par ajout de pentane. La poudre obtenue est dissoute dans le minimum de THF puis précipitée au pentane et enfin séchée pour être isolée avec un rendement final de 90%.

**Exemple 81 : Synthèse du dendrimère de type 6-5-2-2 à surface aza-bis-diméthyl-phosphonate dérivé de la tyramine**

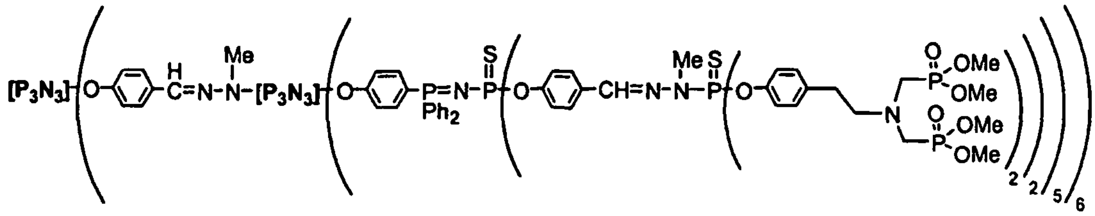

A une solution de dendrimère de type 6-5-2-2 (200 mg) à surface dichlorothiophosphorhydrazide dans du THF (2 mL) est additionné du carbonate de césium (2,05 équivalents par chlore terminal) puis le phénol aza-bis-diméthyl-phosphonate dérivé de la tyramine obtenu dans l'**Exemple 31** est ajouté (1,03 équivalents par chlore terminal). On laisse le mélange sous agitation pendant 24 heures à température ambiante puis on filtre le mélange final sur célite de manière à séparer les sels. Enfin le produit final est lavé par précipitation dans du pentane et isolé avec un rendement de 88%.

**Exemple 82 : Synthèse du dendrimère de type 6-5-2-2 à surface aza-bis-diméthyl-phosphonique dérivé de la tyramine**

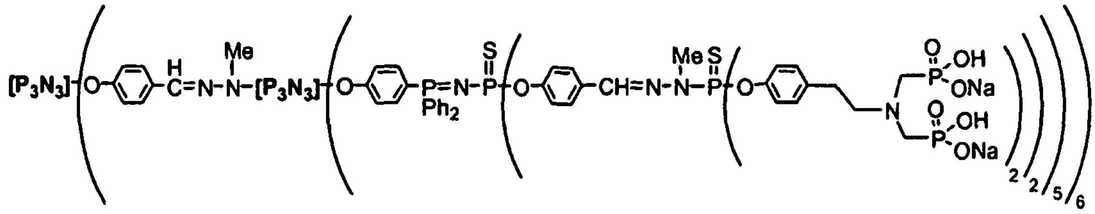

A une solution de dendrimère de type 6-5-2-2 (200 mg) à surface aza-bis-diméthyl-phosphonate dérivé de la tyramine obtenu dans l'**Exemple 81** à 0°C dans de l'acétonitrile (5 mL) on additionne lentement du bromotriméthylsilane (1,1 équivalents par groupement méthoxy). Une fois l'addition terminée on laisse le mélange revenir à température ambiante pendant 12 heures. Le mélange est alors évaporé à sec puis on additionne 1 mL de méthanol anhydre à température ambiante et on laisse le mélange une heure sous agitation. Après évaporation à sec, le résidu est lavé plusieurs fois à l'éther pur. Le produit est ensuite transformé en son mono sel de sodium en présence de soude (1 équivalent de NaOH par terminaison acide phosphonique). La solution résultante est lyophilisée pour donner le dendrimère sous forme d'une poudre blanche. Le produit final est isolé avec un rendement de 61%.

**Exemple 83 : Synthèse du dendrimère de type 6-5-5-2 à surface dichlorothiophosphorhydrazide**

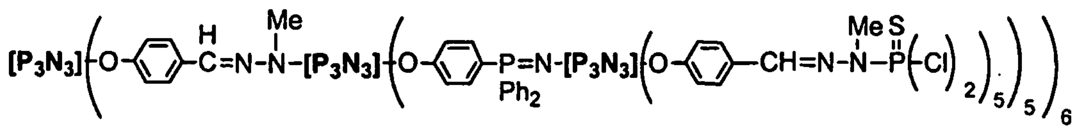

A une solution de dendrimère de type 6-5-5 (250 mg) dans 2 mL de chloroforme est ajouté à -30°C un léger excès (1,1 équivalents par fonction aldéhyde) d'une solution de N-méthyl-dichlorothiophosphorhydrazide à 0,2 M dans le chloroforme. Après agitation pendant 5 à 60 minutes à une température comprise entre -30 et 0°C le mélange est filtré sur célite puis précipité par ajout de pentane. La poudre obtenue est dissoute dans le minimum de THF puis précipitée au pentane et enfin séchée pour être isolée avec un rendement final de 93%.

**Exemple 84 : Synthèse du dendrimère de type 6-5-5-2 à surface aza-bis-diméthyl-phosphonate dérivé de la tyramine**

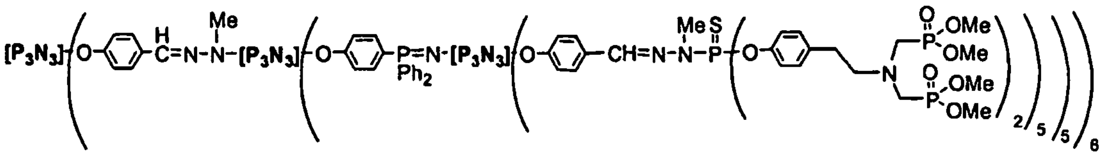

A une solution de dendrimère de type 6-5-5-2 (250 mg) à surface dichlorothiophosphorhydrazide dans du THF (2 mL) est additionné du carbonate de césium (2,05 équivalents par chlore terminal) puis le phénol aza-bis-diméthyl-phosphonate dérivé de la tyramine obtenu dans l'**Exemple 31** est ajouté (1,03 équivalents par chlore terminal). On laisse le mélange sous agitation pendant 24 heures à température ambiante puis on filtre le mélange final sur célite de manière à séparer les sels. Enfin le produit final est lavé par précipitation dans du pentane et isolé avec un rendement de 83%.

**Exemple 85 : Synthèse du dendrimère de type 6-5-5-2 à surface aza-bis-diméthyl-phosphorique dérivé de la tyramine**

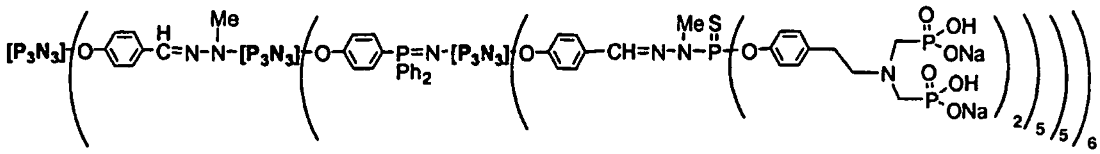

A une solution de dendrimère de type 6-5-5-2 (250 mg) à surface aza-bis-diméthyl-phosphonate dérivé de la tyramine obtenu dans l'**Exemple 84** à 0°C dans de l'acétonitrile (5 mL) on additionne lentement du bromotriméthylsilane (1,1 équivalents par groupement méthoxy). Une fois l'addition terminée on laisse le mélange revenir à température ambiante pendant 12 heures. Le mélange est alors évaporé à sec puis on additionne 1 mL de méthanol anhydre à température ambiante et on laisse le mélange une heure sous agitation. Après évaporation à sec, le résidu est lavé plusieurs fois à l'éther pur. Le produit est ensuite transformé en son mono sel de sodium en présence de soude (1 équivalent de NaOH par terminaison acide phosphonique). La solution résultante est lyophilisée pour donner le dendrimère sous forme d'une poudre blanche. Le produit final est isolé avec un rendement de 66%.

**Exemple 86 : synthèse d'un dendrimère hyperdense à coeur $N_3P_3$ et porteur de 30 extrémités acide aza-bis-phosphonique dérivé de la tyramine**

*Etape 1 : synthèse de l'hexa(N-methylhydrazino) cyclotriphosphazène*

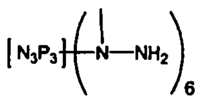

Cette molécule a été décrite par J.P. Majoral et al. dans Angew. Chem. Int. Ed. Engl. 1993, 32, 1477 et Inorg. Chem. 1994, 33, 6351.

*Etape 2 : synthèse du pentachloro-(4-formylphenoxy)cyclotriphosphazène*

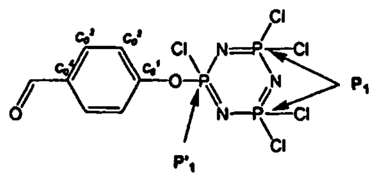

A une solution contenant 4,8 g d'hexachlorocyclotriphosphazène (13,8 mmol) dans le THF (200 mL), est ajoutée à 0°C et sous atmosphère inerte 500 mg de sel de sodium du 4-hydroxybenzaldéhyde (3,47 mmol). Le milieu réactionnel est agité pendant 12 heures tandis qu'on laisse la température remonter doucement à température ambiante. Le brut réactionnel est évaporé à sec puis purifié par chromatographie "flash" sur colonne de silice. Le produit est isolé sous forme d'une huile translucide avec un rendement de 75 %.
RMN $^{31}P\{^1H\}$ ($CDCl_3$, 81 MHz) : $\delta$ = 15,2 (t, $^2J_{PP}$ = 62,0 Hz, $P'_1$); 26,0 (d, $^2J_{PP}$ = 62,0 Hz, $P_1$) ppm.
RMN $^1H$ (CDCl3): $\delta$ = 7,43 (d, $^3J_{HH}$ = 7,5 Hz, 2H, $C_0^2$-H); 7,95 (d, $^3J_{HH}$ = 7,5 Hz, 2H, $C_0^3$-H); 10,00 (s, 1H, CHO) ppm.

*Etape 3 : synthèse du dendrimère porteur de 30 extrémités chlorées*

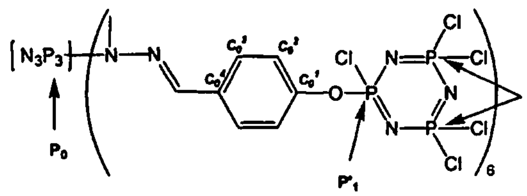

A une solution contenant 655 mg de pentachloro-(4-formylphenoxy)cyclotriphosphazène (1,51 mmol) dans le chloroforme (10 mL) est ajouté à température ambiante 61 mg d'hexa(N-methylhydrazino)cyclotriphosphazène (0,15 mmol). Le mélange réactionnel est agité pendant 2 heures et demie. Le brut réactionnel est évaporé à sec puis purifié par chromatographie "flash" sur colonne de silice. Le produit est isolé sous forme d'un solide blanc avec un rendement de 97 %.
RMN $^{31}P\{^1H\}$ ($CDCl_3$, 81 MHz) : $\delta$ =15,6 (t, $^2Jpp$ = 60,0 Hz, $P'_1$); 21,7 (s, $P_0$); 26,0 (d, $^2J_{PP}$ = 60,0 Hz, $P_1$) ppm.
RMN $^1H$ ($CDCl_3$, 200,13 MHz) : $\delta$ = 3,31 (s, 18H, $CH_3$-N); 7,17 (d, $^3J_{HH}$ = 7,8 Hz, 12H, $C_0^2$-H); 7,52 (s, 6H, CH=N); 7,62 (d, $^3J_{HH}$ = 8,3 Hz, 12H, $C_0^3$-H) ppm.
RMN $^{13}C\{^1H\}$ ($CDCl_3$, 50,32 MHz) : $\delta$ = 32,4 (s, $CH_3$-N); 121,4 (d, $^3J_{CP}$ = 5,2 Hz, $C_0^2$); 127,9 (d, $^4J_{CP}$ =1,2 Hz, $C_0^3$); 134,7 (dl, $^4J_{CP}$ = 2,3 Hz, $C_0^4$); 135,2 (sl, CH=N); 149,0 (d, $^2J_{CP}$ =10,2 Hz, $C_0^1$) ppm.

***Etape 4 : Synthèse du dendrimère à extrémités phénol aza-bis-diméthyl-phosphonate dérivé de la tyramine***

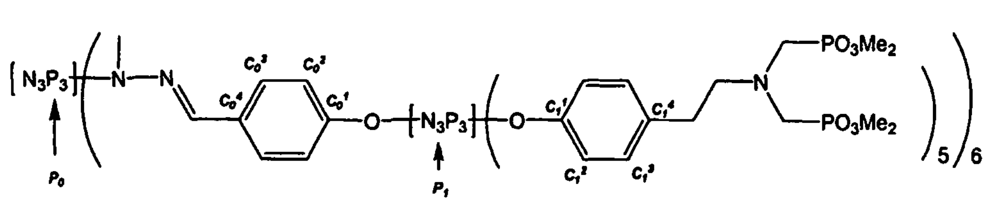

A une solution de 597 mg du phénol aza-bis-diméthyl-phosphonate dérivé de la tyramine obtenu dans l'**Exemple 31** (1,55 mmol) dans le THF (8 mL) est ajouté à température ambiante 150 mg du dendrimère porteur de 30 extrémités chlorées (0,05 mmol), puis 1,11 g de carbonate de césium (3,42 mmol). Le milieu réactionnel est agité pendant 12 heures. Les sels de césium sont retirés par dilution (20 mL de THF) et centrifugation du brut réactionnel. Des lavages THF / pentane permettent d'isoler le produit sous forme d'une huile avec un rendement de 70 %.
RMN $^{31}P\{^1H\}$ (acétone $d_6$, 81 MHz) : $\delta$ = 12,9 (sl, $P_1$); 20,7 (s, $P_0$); 30,2 (s, $PO_3Me_2$); 30,3 (s, $PO_3Me_2$) ppm.
RMN $^1H$ (acétone $d_6$, 500,33 MHz) : $\delta$ = 2,79 (t déformé, $^3J_{HH}$ = 6,5 Hz, 24H, $\underline{CH_2}$-$CH_2$-N); 2,87 (t déformé, $^3J_{HH}$ = 6,5 Hz, 36H, $\underline{CH_2}$-$CH_2$-N); 3,07 (t déformé, $^3J_{HH}$ = 6,5 Hz, 24H, $CH_2$-$\underline{CH_2}$-N); 3,10 (t déformé, $^3J_{HH}$ = 6,5 Hz, 36H, $CH_2$-$\underline{CH_2}$-N); 3,22 (d, $^2J_{HP}$ = 9,7 Hz, 48H, N-$CH_2$-P); 3,27 (d, $^2J_{HP}$ = 9,7 Hz, 72H, N-$CH_2$-P); 3,44 (sl, 18H, $CH_3$-N); 3,68

(d, $^3J_{HP}$ = 10,4 Hz, 144H, P(O)($OCH_3$)); 3,72 (d, $^3J_{HP}$ = 10,4 Hz, 72H, P(O)($OCH_3$)); 3,73 (d, $^3J_{HP}$ = 10,4 Hz, 144H, P(O)($OCH_3$)); 6,85 (d, $^3J_{HH}$ = 8,1 Hz, 24H, $C_1^2$-H); 6,89 (d, $^3J_{HH}$ = 8,1 Hz, 12H, $C_1^2$-H); 6,90 (d, $^3J_{HH}$ = 8,1 Hz, 24H, $C_1^2$-H); 7,01 (d, $^3J_{HH}$ = 8,1 Hz, 12H, $C_0^2$-H); 7,20 (d, $^3J_{HH}$ = 8,1 Hz, 24H, $C_1^3$-H); 7,24 (d, $^3J_{HH}$ = 8,1 Hz, 36H, $C_1^3$-H); 7,67 (d, $^3J_{HH}$ = 8,1 Hz, 12H, $C_0^3$-H); 7,84 (sl, 6H, CH=N) ppm.
RMN $^{13}C\{^1H\}$ (acétone $d_6$, 125,81 MHz) : δ = 32,3 (sl, $CH_3$-N, $\underline{CH_2}$-$CH_2$-N); 49,1 (dd,$^1J_{CP}$ = 156,0 Hz, $^3J_{CP}$ = 7,6 Hz, N-$CH_2$-P); 52,0 (s, P(O)($OCH_3$)); 58,2 (m, $CH_2$-$\underline{CH_2}$-N); 58,3 (m, $\underline{CH_2}$-$\underline{CH_2}$-N); 120,6 (s, $C_1^2$); 120,7 (s, $C_1^2$); 120,9 (sl, $C_0^2$); 127,6 (s, $C_0^3$); 130,0 (s, $C_1^3$); 133,7 (s, $C_0^4$); 136,1 (sl, CH=N); 136,9 (s, $C_1^4$); 137,0 (s, $C_1^4$); 149,0 (sl, $C_1^1$); 150,7 (d, $^2J_{CP}$ = 10,2 Hz, $C_0^1$) ppm.

*Etape 5 : Synthèse du dendrimère à extrémités sel de sodium de l'acide aza-bisphosphonique dérivé de la tyramine*

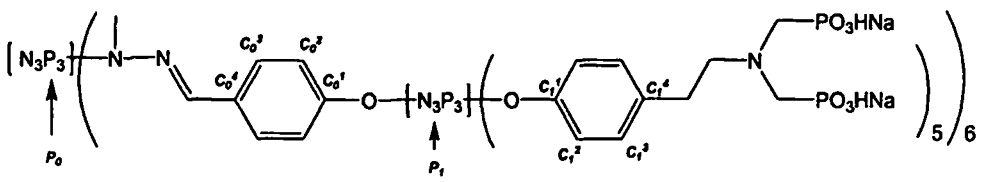

A une solution à 0°C sous atmosphère inerte contenant 100 mg du dendrimère porteur de 30 extrémités acide aza-bisphosphonique dérivé de la tyramine (0,008 mmol) dans 3 mL d'acétonitrile sont ajoutés lentement 132 μl de bromotriméthylsilane (0,997 mmol). A la fin de l'addition, le milieu réactionnel est agité à température ambiante pendant 12 heures. Le milieu réactionnel est ensuite évaporé à sec puis 2,5 mL de méthanol sont ajoutés à température ambiante. Le milieu réactionnel est agité pendant 1 heure puis évaporé à sec. Cette opération de méthanolyse est répétée une deuxième fois puis le produit est lavé plusieurs fois à l'éther diéthylique.
Le produit résultant, pour des raisons d'analyse RMN, est ensuite transformé en son sel de sodium. Le produit est tout d'abord mis en présence d'eau (1 mL) puis 2,30 mL de soude aqueuse sont ajoutés (0,1966 N). Après dissolution totale du dendrimère, la solution est lyophilisée, ce qui permet d'obtenir le dendrimère sous forme d'une poudre blanche avec un rendement de 70%.
RMN $^{31}P\{^1H\}$ ($D_2O/CD_3CN$, 81 MHz) : δ = 10,7 (s, P(O)(OH)(ONa)); 12,5 (sl, $P_1$); 20,9 (s, $P_0$) ppm.

**Exemple 87 : synthèse d'un dendrimère hyperdense à coeur $N_3P_3$ et porteur de 30 extrémités acide alpha-hydroxy-phosphonique**

*Etape 1 : Synthèse du dendrimère à extrémités aldéhyde*

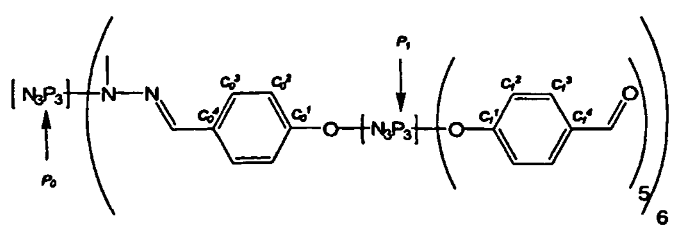

A une solution contenant 200 mg de dendrimère porteur de 30 extrémités chlorées obtenu dans l'étape 3 de l'**Exemple 86** (0,07 mmol) dans le THF (8 mL) sont ajoutés à température ambiante 300 mg de sel de sodium du 4-hydroxybenzaldéhyde (2,1 mmol). Le milieu réactionnel est agité pendant 12 heures. Les sels de sodium sont retirés par dilution et centrifugation du brut réactionnel. Des lavages acétone/pentane permettent d'isoler le produit sous forme d'un solide blanc cassé avec un rendement de 70 %.
RMN $^{31}P\{^1H\}$ (acétone $d_6$, 81 MHz) : δ =11,7 (s large, $P_1$); 21,3 (s, $P_0$) ppm.
RMN $^1H$ (acétone $d_6$, 500,33 MHz) : δ = 3,51 (s, 18H, $CH_3$-N); 6,91 (d, $^3J_{HH}$ = 8,4 Hz, 12H, $C_0^2$-H); 7,13 (d, $^3J_{HH}$ = 8,6 Hz, 24H, $C_1^2$-H); 7,21 (d, $^3J_{HH}$ = 8,6 Hz, 12H, $C_1^2$-H); 7,23 (d, $^3J_{HH}$ = 8,6 Hz, 24H, $C_1^2$-H); 7,53 (d, $^3J_{HH}$ = 8,4 Hz, 12H, $C_0^3$-H); 7,75 (s, 6H, CH=N); 7,76 (d, $^3J_{HH}$ = 8,6 Hz, 24H, $C_1^3$-H); 7,80 (d, $^3J_{HH}$ = 8,6 Hz, 12H, $C_1^3$-H); 7,81 (d, $^3J_{HH}$ = 8,6 Hz, 24H, $C_1^3$-H); 9,94 (s, 6H, CHO); 9,96 (s, 24H, CHO) ppm.

RMN $^{13}C\{^1H\}$ (acétone $d_6$, 125,81 MHz) : $\delta$ = 32,2 (m, $CH_3$-N); 120,9 (sl, $C_0^2$); 121,2 (s, $C_1^2$); 121,3 (s, $C_1^2$); 121,4 (s, $C_1^2$); 127,6 (s, $C_0^3$); 131,3 (s, $C_1^3$); 131,4 (s, $C_1^3$); 134,0 (s, $C_1^4$); 134,1 (s, $C_1^4$); 134,2 (s, $C_0^4$); 135,7 (m, CH=N); 149,6 (m, $C_0^1$); 154,3 (m, $C_1^1$); 154,4 (m, $C_1^1$); 154,5 (m, $C_1^1$); 190,6 (s, CHO); 190,9 (s, CHO) ppm.

*Etape 2 : Synthèse du dendrimère à extrémités α-hydroxyphosphonate de diméthyl*

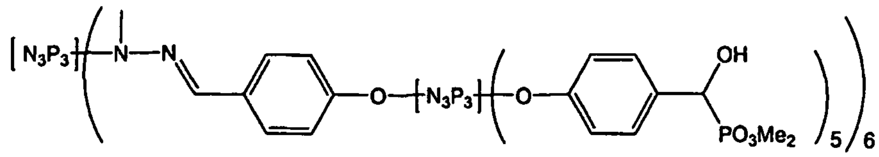

A une solution contenant 200 mg du dendrimère porteur de 30 extrémités benzaldéhyde (0,037 mmol) dans le THF (1 mL) sont ajoutés à température ambiante 111 µmL de diméthylphosphite (1,21 mmol) puis une goutte de triéthylamine. Le milieu réactionnel est agité pendant 12 heures. Le milieu réactionnel devenu visqueux est ensuite lavé à l'éther diéthylique, ce qui permet d'isoler le produit sous forme d'un solide blanc avec un rendement de 70 %.
RMN $^{31}P\{^1H\}$ (DMSO $d_6$, 81 MHz) : $\delta$ = 11,8 (s, $P_1$); 20,6 (s, $P_0$); 27,2 (s, $P(O)(OCH_3)_2$) ppm.
RMN $^1H$ (DMSO $d_6$, 500,33 MHz) : $\delta$ = 2,21 (m, 18H, $CH_3$-N); 3,45-3,68 (m, 180H, $P(O)(OCH_3)$); 5,03 (m, 30H, P-CH); 6,31 (d large, $^3J_{HP}$ = 14,6 Hz, 30H, CH-<u>OH</u>); 6,91 (m, 72H, $C_0^2$-H, $C_1^2$-H); 7,35 (m, 60H, $C_1^3$-H); 7,54 (m, 12H, $C_0^3$-H); 7,73 (s large, 6H, CH=N) ppm.

*Etape 3 : Synthèse du dendrimère à extrémités sel de sodium de l'acide α-hydroxyphosphonique*

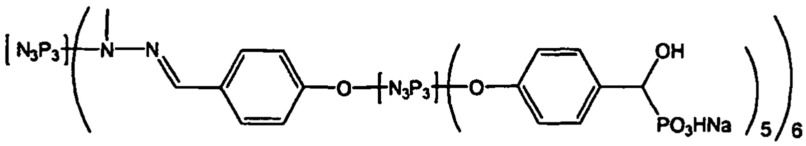

A une solution de 3 mL d'acétonitrile à 0°C sous atmosphère inerte contenant 100 mg du dendrimère porteur de 30 extrémités α-hydroxyphosphonate de diméthyle (0,011 mmol) sont ajoutés lentement 99 µl de bromotriméthylsilane (0,753 mmol). A la fin de l'addition, le milieu réactionnel est agité à température ambiante pendant 12 heures. Le milieu réactionnel est ensuite évaporé à sec puis 2,5 mL de méthanol sont ajoutés à température ambiante. Le milieu réactionnel est agité pendant 1 heure puis évaporé à sec. Cette opération de méthanolyse est répétée une deuxième fois puis le produit est lavé plusieurs fois à l'éther diéthylique.

Le produit résultant, pour des raisons d'analyse RMN, est ensuite transformé en son sel de sodium. Le produit est tout d'abord mis en présence d'eau (1 mL) puis 1,74 mL de soude aqueuse est ajoutée (0,1966 N). Après dissolution totale du dendrimère, la solution est lyophilisée, ce qui permet d'obtenir le dendrimère sous forme d'une poudre blanche avec un rendement de 70%.
RMN $^{31}P\{^1H\}$ ($D_2O/CD_3CN$, 81 MHz) : $\delta$ = 12,6 (sl, $P_1$); 19,7 (s, $P_0$); 20,1 (s, P(O)(OH)(ONa)) ppm.

**Exemple 88 : Synthèse du dendrimère de première génération à surface aza-bis-diméthyl-phosphonate dérivé de la tyramine et à coeur fluorescent :**

<u>a. Synthèse du phénol fluorescent dérivé de l'anhydride diphényl-maléique :</u>

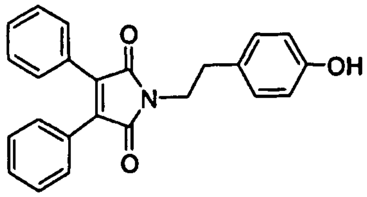

Dans un ballon de 250 mL sont mis à température ambiante : l'anhydride diphényl-maléique (20 mmol, 5 g), de la tyramine (40 mmol, 5,48g), de la N,N diisopropyléthylamine (14 mmol, 32 ml), 50 g de phénol utilisé comme solvant, et 50 g de tamis moléculaire 4Å. Le mélange est placé 1 heure et demie à 150°C puis refroidi à température ambiante. Le produit est alors dilué dans 1,2 1 de dichlorométhane, filtré sur célite et lavé avec 1,6 1 d'acide chlorhydrique aqueux à 4%. La phase organique est séchée avec du sulfate de magnésium et concentré. Le phénol utilisé comme solvant est éliminé par sublimation à 70°C. Enfin le produit est purifié par chromatographie sur gel de silice en utilisant comme éluant un gradient : chloroforme puis éther. Le produit final est isolé avec un rendement de 20%. RMN $^1H$ ($CDCl_3$) : $\delta$ = 2,93 (t, $^3J_{HH}$ = 7,8 Hz, 2H, $\underline{CH_2}$-$CH_2$-N); 3,86 (t, $^3J_{HH}$ = 7,8 Hz, 2H, $CH_2$-$\underline{CH_2}$-N); 5,38 (s large, 1H, OH); 6,7-7,5 (m, 14H, $CH_{arom}$) ppm.

b. Synthèse du cyclotriphosphazène monosubstitué par le phénol fluorescent dérivé de l'anhydride diphényl-maléique:

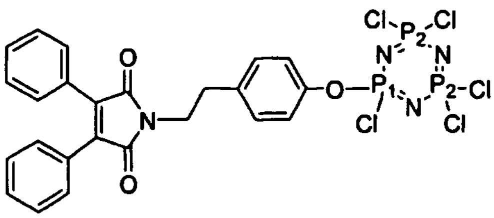

L'hexachloro cyclotriphosphazène (0,403 mmol, 140 mg) est mis en solution dans 20 mL de toluène à température ambiante avec de la triéthylamine (0,54 mmol, 100 µl) et le phénol fluorescent (0,277 mmol, 100 mg) obtenu à l'étape a. L'ensemble est laissé sous agitation à température ambiante pendant 72 heures puis filtré sur célite et concentré. Enfin le produit est purifié par chromatographie sur gel de silice. Pour cela on utilise un gradient de solvant : dans un premier temps hexane pur puis un mélange hexane/éther 1/1. Le produit final est isolé avec un rendement de 40%.
RMN $^{31}P$-{$^1H$} ($CDCl_3$) : $\delta$ = 15,6 (t, $P_1$); 25,8 (d, $P_2$) ppm.
RMN $^1H$ ($CDCl_3$): $\delta$ = 3,03 (t, $^3J_{HH}$ = 7,8 Hz, 2H, $\underline{CH_2}$-$CH_2$-N); 3,91 (t, $^3J_{HH}$ = 7,8 Hz, 2H, $CH_2$-$\underline{CH_2}$-N); 7,2-7,5 (m, 14H, $CH_{arom}$) ppm.

c. Synthèse du coeur penta aldéhyde monosubstitué par le phénol fluorescent dérivé de l'anhydride diphényl-maléique :

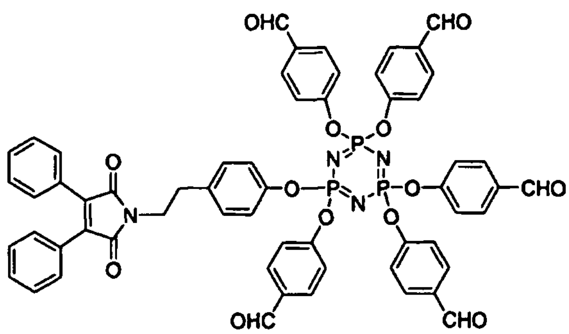

Le cyclotriphosphazène mono-substitué par le groupe fluorescent, dérivé de l'anhydride diphényl-maléique, (1,07 mmol, 730 mg) obtenu lors de l'étape b, est mis en solution dans du THF anhydre (5 mL) avec du carbonate de césium (11,8 mmol, 3,85 g) et le 4-hydroxy-benzaldéhyde (5,57 mmol, 680 mg). On laisse le mélange sous agitation

pendant 12 heures à température ambiante. Le produit final est mis en solution dans un minimum de THF et lavé par précipitation dans du pentane. Il est isolé avec un rendement de 80%.
RMN $^{31}P-\{^1H\}$ $(CDCl_3)$ : $\delta$ = 11,7 (s, P) ppm.
RMN $^1H$ $(CDCl_3)$ : $\delta$ = 2,96 (t, $^3J_{HH}$ = 7,8 Hz, 2H, $\underline{CH_2}$-$CH_2$-N); 3,83 (t, $^3J_{HH}$ = 7,8 Hz, 2H, $CH_2$-$\underline{CH_2}$-N); 6,9-7,8 (m, 34H, $CH_{arom}$); 9,92 (s, 5H, CHO) ppm.

d. Synthèse du dendrimère de première génération à surface dichlorothiophosphine et à coeur fluorescent :

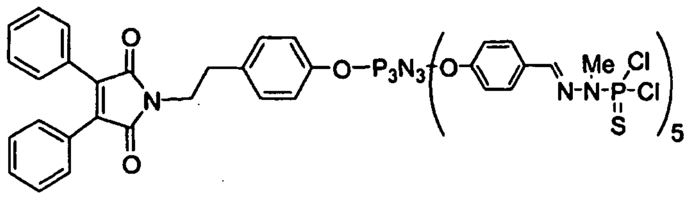

Sur un coeur penta-fonctionnel porteur du groupe fluorescent, dérivé de l'anhydride diphényl-maléique, (0,279 mmol, 310 mg), obtenu lors de l'étape c, en poudre, est additionnée à 0°C, l'hydrazino-dichloro-thiophosphine (7 mL, 0,24 $mol.L^{-1}$) en solution dans le chloroforme. Le mélange est laissé sous agitation magnétique et à température ambiante pendant 8 heures puis évaporé. Enfin la poudre obtenue est lavée trois fois par précipitation dans un mélange dichlorométhane pentane 1/5. Le produit final est isolé avec un rendement de 76%.
RMN $^{31}P-\{^1H\}$ $(CDCl_3)$ : $\delta$ = 66,00 (s, $P_1$); 65,91 (s, $P_1$); 11,9 (m, $P_0$) ppm.
RMN $^1H$ $(CDCl_3)$ : $\delta$ = 2,93 (t, $^3J_{HH}$ = 7,8 Hz, 2H, $\underline{CH_2}$-$CH_2$-N); 3,42 (d, $^3J_{HP}$ = 8,9 Hz, 9H, $CH_3$-N-$P_1$); 3,48 (d, $^3J_{HP}$ = 8,7 Hz, 6H, $CH_3$-N-$P_1$); 3,78 (t, $^3J_{HH}$ = 7,8 Hz, 2H, $CH_2$-$\underline{CH_2}$-N); 6,7-7,7 (m, 34H, $CH_{arom}$) ppm.

e. Synthèse du dendrimère de première génération à surface azabisphosphonate et à coeur fluorescent :

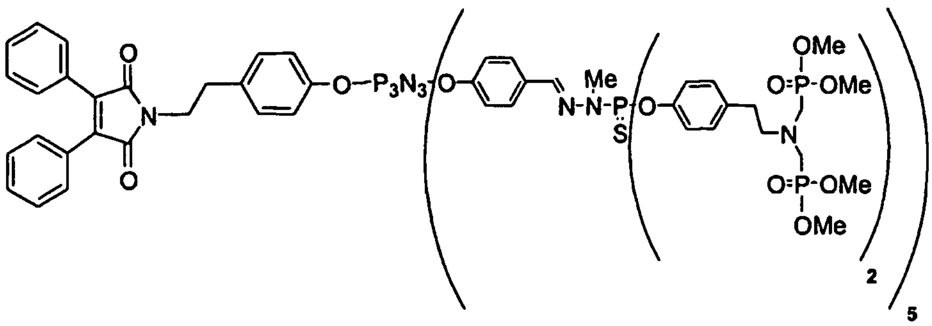

Finalement, à une solution de dendrimère de première génération porteur du groupe fluorescent, dérivé de l'anhydride diphényl-maléique, (0,198 mmol, 380 mg), obtenu lors de l'étape d, dans du THF anhydre (5 mL) est additionné du carbonate de césium (4,16 mmol, 1,35 g) et le phénol aza-bis-diméthyl-phosphonate dérivé de la tyramine (2,08 mmol, 736 mg). On laisse le mélange sous agitation pendant 24 heures à température ambiante puis on filtre sur célite le mélange final de manière à séparer les sels. Enfin le produit final est lavé par précipitation dans du pentane et isolé avec un rendement de 70%.
RMN $^{31}P-\{^1H\}$ $(CDCl_3)$ : $\delta$ = 66,58 (s, $P_1$); 30,19 (s, $PO_3Me_2$); 11,8 (s, $P_0$) ppm.
RMN $^1H$ $(CDCl_3)$ : $\delta$ = 2,70 (s large, 22H, $\underline{CH_2}$-$CH_2$-N); 3,00 (s large, 22H, $CH_2$-$\underline{CH_2}$-N); 3,13 (d, $^2J_{HP}$ = 9,2 Hz, 40H, -$\underline{CH_2}$-P(O)$(OCH_3)_2$); 3,20 (d, $^3J_{HP}$ = 11,8 Hz, 15H, $CH_3$-N-$P_1$); 3,68 (d, $^3J_{HP}$ = 10,4 Hz, 96H, -P(O)(O-$\underline{CH_3}$)$_2$); 6,6-7,7 (m, 79H, $CH_{arom}$, CH=N) ppm.

**Exemple 89: Synthèse du dendrimère de première génération à surface aza-bis-phosphonique dérivé de la tyramine et à coeur fluorescent :**

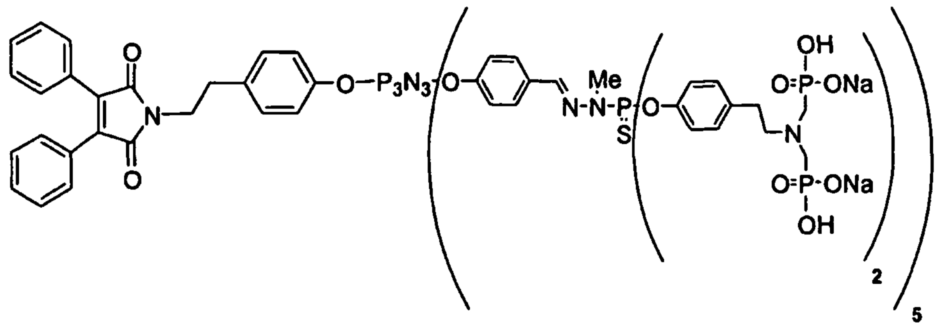

A une solution de dendrimère de première génération porteur du groupe fluorescent, dérivé de l'anhydride diphényl-maléique, et à surface aza-bis-diméthyl-phosphonate (0,098 mmol, 500 mg), obtenu dans l'**Exemple 88**, dans l'acétonitrile (10 ml) est additionné à 0°C et goutte à goutte du bromotriméthylsilane (4,3 mmol, 578 μl). Une fois l'addition terminée on laisse le mélange revenir à température ambiante pendant 12 heures. Le mélange est alors évaporé à sec puis on additionne 1 ml de méthanol anhydre à température ambiante et on laisse le mélange une heure sous agitation. Après évaporation à sec, le résidu est lavé plusieurs fois à l'éther pur. Le produit étant totalement insoluble dans les solvants organiques il est transformé en son mono sel de sodium en présence d'une solution aqueuse de soude (8,6 ml à 0,1955 mol.$L^{-1}$, pour 380 mg de dendrimère). La solution résultante est lyophilisée pour donner le dendrimère sous forme d'une poudre blanche-jaune. Le produit final est isolé avec un rendement de 51 %.
RMN $^{31}P-\{^1H\}$ ($CD_3CN/D_2O$) : $\delta$ = 66,58 (s, $P_1$); 14,2 (s, P(O)(ONa)(OH)); 11,8 (s, $P_0$) ppm.

**Exemple 90 : synthèse d'un dendrimère phosphoré de première génération porteur d'un marqueur fluorescent dérivé de la julolidine à extrémités acide aza-bis-phosphonique dérivé de la tyramine**

*Etape 1 : Synthèse de la 2,3,6,7-tetrahydro-1H,5H-3-formyl-benzo(ij)quinolizine (ou julolidine paraformylée)*

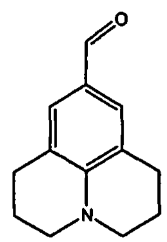

La synthèse de cette molécule a été réalisée suivant la procédure décrite par M.A. Haidekk et al. Chemistry and Biology 2001, 8, 123 -131.

*Etape 2 : Synthèse du 2-cyano-N-[2-(4-hydroxy-phényl)-éthyl]-acétamide*

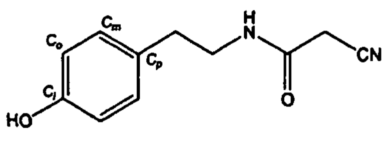

A une solution contenant 1,00 g (8,84 mmol) de cyanoacétate d'éthyle dans 13 mL de diméthylformamide sous atmosphère inerte sont ajoutés 1,28 g (9,33 mmol) de tyramine. L'ensemble est agité à 110 °C pendant 4 heures, puis à température ambiante pendant 12h. Le milieu réactionnel est ensuite dilué dans 100 mL d'acétate d'éthyle et lavé avec 50 mL d'une solution acide aqueuse à pH = 3. La phase aqueuse est à nouveau extraite avec 50 mL d'acétate d'éthyle. Les phases organiques sont réunies, séchées sur sulfate de magnésium, filtrées et le solvant est évaporé sous pression réduite. Le solide visqueux brun résultant est ensuite dissous plusieurs fois dans le toluène et l'azéotrope

toluène / DMF est évaporé. Enfin, le solide est lavé au dichlorométhane et à l'éther. Le produit est isolé sous forme d'un solide marron pâle avec un rendement de 65 %.

RMN $^1H$ (acétone $d_6$, 200,13 MHz) : δ = 2,71 (t, $^3J_{HH}$ = 7,0 Hz, 2H, $\underline{CH_2}$-$C_6H_4$); 3,41 (m, 2H, HN-$\underline{CH_2}$); 3,56 (s, 2H, $\underline{CH_2}$-CN); 6,76 (d, $^3J_{HH}$ = 8,3 Hz, 2H, $C_o$-H); 7,05 (d, $^3J_{HH}$ = 8,2 Hz, 2H, $C_m$-H); 7,52 (sl, 1H, OH); 8,21 (sl, 1H, NH) ppm.

RMN $^{13}C\{^1H\}$ (acétone $d_6$, 50,32 MHz) : δ = 26,1 (s, $\underline{CH_2}$-CN); 35,1 (s, $\underline{CH_2}$-$C_6H_4$); 42,3 (s, HN-$\underline{CH_2}$); 116,0 (s, $C_o$ et CN); 130,4 (s, $C_m$ et $C_p$); 156,6 (s, $C_i$); 162,7 (s, CO) ppm.

*Etape 3 : Synthèse du 2-cyano-N-[2-(4-hydroxy-phényl)-éthyl]-3-(2, 3, 6, 7-tetrahydro-1H,5H-3-formyl-benzo(ij)quinolizine)-acrylamide*

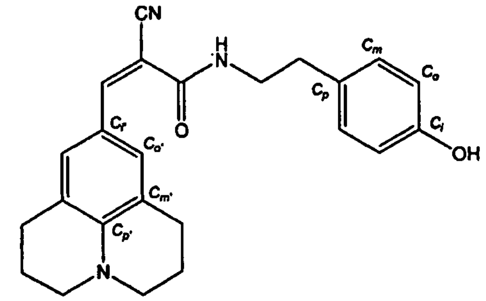

A une solution de 130 mg (0,646 mmol) de julolidine formylée dans 14 mL de THF sont ajoutés 198 mg (0,969 mmol) de 2-cyano-N-[2-(4-hydroxy-phényl)-éthyl]-acétamide et 360 μL (2,580 mmol) de triéthylamine. Le mélange réactionnel est porté à reflux pendant 18 heures. Le solvant est ensuite évaporé sous pression réduite et le résidu est purifié par chromatographie sur colonne de silice (éluant : dichlorométane contenant 2 % de méthanol). Le produit obtenu (Rf = 0,29) est isolé sous forme d'un solide orange avec un rendement de 67%.

RMN $^1H$ (DMSO $d_6$, 500,33 MHz) : δ = 1,86 (m, 4H, $\underline{CH_2}$-$CH_2$-N); 2,64 (m, 6H, $\underline{CH_2}$-$CH_2$-$CH_2$-N, HN-$CH_2$-$\underline{CH_2}$); 3,31 (m, 6H, $CH_2$-$CH_2$-$\underline{CH_2}$-N, HN-$\underline{CH_2}$-$CH_2$); 6,66 - 7,01 (m, 4H, $C_m$-H, $C_o$-H); 7,42 (s, 2H, $C_o$-H); 7,79 (s, 1H, $\underline{HC}$=C-CN); 7,97 (t, $^3J_{HH}$ = 7,5 Hz, 1H, NH); 9,18 (s, 1H, OH) ppm.

RMN $^{13}C\{^1H\}$ (DMSO $d_6$, 125,81 MHz) : δ = 21,1 (s, $\underline{CH_2}$-$CH_2$-N); 27,6 (s, $\underline{CH_2}$-$CH_2$-$CH_2$-N); 34,8 (s, $\underline{CH_2}$-$CH_2$-NH); 42,0 (s, $CH_2$-NH); 49,8 (s, $CH_2$-N); 95,2 (s, $\underline{C}$-CN); 115,6 (s, $C_o$); 118,1 (s, $C_{i'}$); 119,0 (s, CN); 120,9 (s, $C_{m'}$); 129,9 (s, $C_m$, $C_p$); 130,6 (s, $C_{o'}$); 147,1 (s, $C_{p'}$); 150,6 (s, $\underline{HC}$=C-CN); 156,1 (s, $C_i$); 162,7 (s, CO) ppm.

*Etape 4 : Synthèse du penta(4-formylphénoxy)-chlorocyclotriphosphazène*

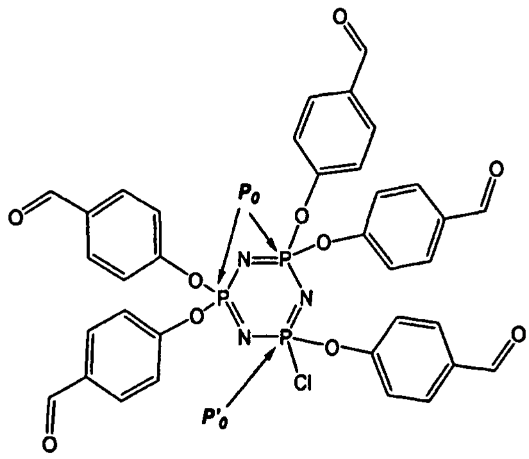

A une solution contenant 1,2 g d'hexachlorocyclotriphosphazène (3,45 mmol) dans le THF (300 mL), sont ajoutés à 0°C et sous atmosphère inerte 2591 mg de sel de sodium du 4-hydroxybenzaldéhyde (18 mmol). Le milieu réactionnel est agité pendant 12 heures tandis qu'on laisse la température remonter doucement à température ambiante.

Le brut réactionnel est évaporé à sec puis purifié par chromatographie "flash" sur colonne de silice. Le produit est isolé sous forme d'une huile translucide avec un rendement de 70 %.
RMN $^{31}P\{^1H\}$ ($CDCl_3$, 81 MHz) : $\delta$ = 9,2 (d, $^2J_{PP}$ = 86,6 Hz, $P_0$); 24,3 (t, $^2J_{PP}$ = 86,6 Hz, $P'_0$) ppm.

*Etape 5 : Synthèse d'un coeur dendritique de type AB5 portant un fluorophore dérivé de la julolidine et 5 fonctions aldéhyde*

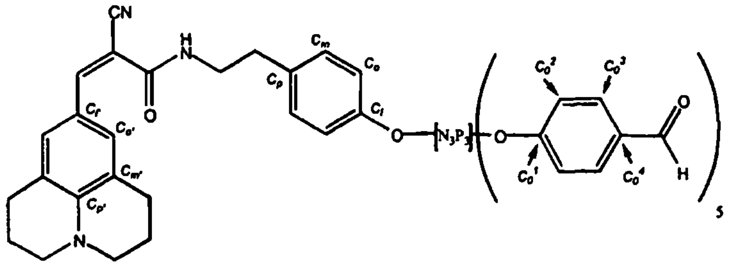

A une solution contenant 92 mg (237 mmol) de 2-cyano-N-[2-(4-hydroxyphényl)-éthyl]-3-(2,3,6,7-tetrahydro-1H,5H-3-formyl-benzo(ij)quinolizine)-acrylamide dans 10 mL de THF sont ajoutés ajouté 184 mg (237 mmol) de penta (4-formylphénoxy)-chlorocyclotri-phosphazène décrit à l'étape précédente puis 155 mg (475 mmol) de carbonate de césium. Le milieu réactionnel est agité à température ambiante pendant 12 heures. Les sels de césium sont éliminés par centrifugation, et après évaporation du solvant sous pression réduite le résidu brut est purifié par chromatographie sur colonne de silice (éluant : acétate d'éthyle / pentane, 1:1). On isole le produit (Rf= 0,26) sous forme d'une huile orange.
RMN $^{31}P\{^1H\}$ ($CDCl_3$, 81,02 MHz) : $\delta$ =10,9 (sl) ppm.
RMN $^1H$ (acétone $d_6$, 200,13 MHz) : $\delta$ = 1,90 (m, 4H, $\underline{CH_2}$-$CH_2$-N); 2,67 (t, $^3J_{HH}$ = 6,3 Hz, 4H, $\underline{CH_2}$-$CH_2$-$CH_2$-N); 2,84 (t déformé, $^3J_{HH}$ = 6,9 Hz, 2H, HN-$CH_2$-$\underline{CH_2}$); 3,25 (t, $^3J_{HH}$ = 6,0 Hz, 4H, $\underline{CH_2}$-N); 3,58 (t, $^3J_{HH}$ = 6,0 Hz, 2H, HN-$\underline{CH_2}$); 6,37 (t, $^3J_{HH}$ = 5,4 Hz, 1H, NH); 6,92 (d, $^3J_{HH}$ = 8,4 Hz, 2H, $C_o$-H); 7,10 (m, 12H, $C_m$-H et $C_0^2$-H); 7,36 (s, 2H, $C_{o'}$-H), 7,69 (d, $^3J_{HH}$ = 8,6 Hz, 10H, $C_o^3$-H); 7,95 (s, 1H, $\underline{HC}$=C-CN); 9,90 (s; 3H, CHO), 9,92 (s; 2H, CHO) ppm.

*Etape 6 : Synthèse du dendrimère fluorescent à terminaison $PSCl_2$*

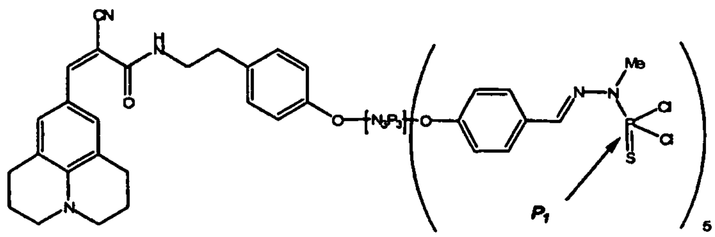

A une solution de dichlorothiophospho-(N-méthyl)-hydrazide (0,3 mmol) dans le chloroforme (1,5 mL) est ajouté, à 0°C, 100 mg du composé obtenu à l'étape 5 (0,05 mmol). Le mélange réactionnel est agité pendant 12 heures. Après évaporation du solvant de réaction, le produit est dilué dans un minimum de dichlorométhane et précipité par ajout d'un grand volume de pentane. Ce traitement est réalisé trois fois. Le produit est isolé ave un rendement de 90 %.
RMN $^{31}P\{^1H\}$ ($CDCl_3$, 81,02 MHz) : $\delta$ = 11,8 (sl, $N_3P_3$); 65,9 (s, $P_1$); 66,0 (s, $P_1$); 66,1 (s, $P_1$) ppm.

*Etape 7 : Synthèse du dendrimère fluo à extrémités aza-bis-diméthyl-phosphonate dérivé de la tyramine*

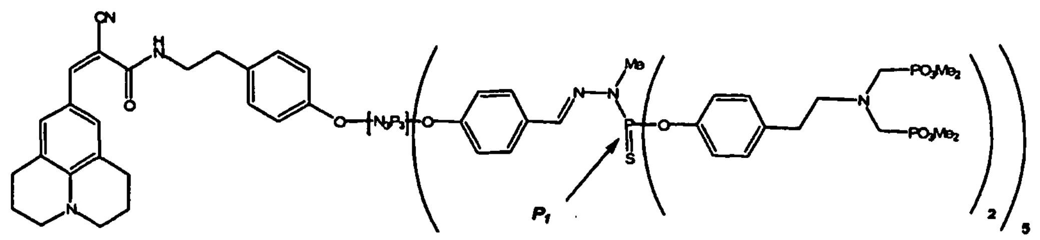

A une solution contenant 100 mg de dendrimère fluorescent à terminaison $PSCl_2$ (0,052 mmol) dans 5 mL de THF est additionné 339 mg de carbonate de césium (1,04 mmol) et 198 mg de phénol aza-bis-diméthyl-phosphonate dérivé de la tyramine obtenu dans l'**Exemple 31** (0,520 mmol). Le mélange est agité pendant 12 heures à température ambiante puis les sels formés sont éliminés par centrifugation. Après évaporation du solvant de réaction, le produit est dilué dans un minimum de THF et précipité par ajout d'un grand volume de pentane. Le produit est isolé avec un rendement de 70 %.
RMN $^{31}P\{^1H\}$ ($CDCl_3$, 81,02 MHz) : $\delta$ = 11,9 (s, $N_3P_3$); 30,3 (s, $PO_3Me_2$); 30,6 (s, $PO_3Me_2$); 66,7 (s,$P_1$); 66,8 (s,$P_1$) ppm.

*Etape 8 : Synthèse du dendrimère fluo à extrémités sel dë sodium de l'acide aza-bis-phosphonique dérivé de la tyramine*

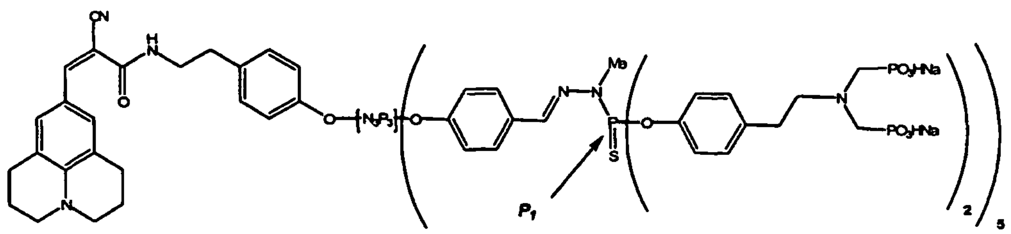

A une solution contenant 100 mg de dendrimère fluorescent à extrémités aza-bis-diméthyl-phosphonate dérivé de la tyramine obtenu à l'étape précédente (0,019 mmol) dans l'acétonitrile (5 mL) à 0°C sous atmosphère inerte sont ajoutés lentement 110 µL de bromotriméthylsilane (0,838 mmol). A la fin de l'addition, le milieu réactionnel est agité à température ambiante pendant 12 heures. Le milieu réactionnel est ensuite évaporé à sec puis sont ajoutés 2,5 mL de méthanol à température ambiante. Le milieu réactionnel est agité pendant 1 heure puis évaporé à sec. Cette opération de méthanolyse est répétée une deuxième fois puis le produit est lavé plusieurs fois à l'éther diéthylique. Le produit résultant, pour des raisons d'analyse RMN, est ensuite transformé en son sel de sodium. Le produit est tout d'abord mis en présence d'eau (1 mL) puis 4,8 mL de soude aqueuse (0,1966 N) sont ajoutés. Après dissolution totale du dendrimère, la solution est lyophilisée, ce qui permet d'obtenir le dendrimère sous forme d'une poudre blanche avec un rendement de 75%.
RMN $^{31}P\{^1H\}$ ($D_2O/CD_3CN$, 81,02 MHz) : $\delta$ = 10,3 (sl, $PO_3HNa$); 13,1 (s, $N_3P_3$); 64,5 (s, $P_1$) ppm.

**Exemple 91 : Synthèse de dendrimère à coeur cyclotriphosphazène et à surface aza-bis-phosphonique dérivé de la tyramine, ayant une branche manquante**

*Etape 1 : Réaction de monosubstitution sur le cyclotriphosphazène*

140 mg d'hexachlorocyclotriphosphazène, 0,1 mL de triéthylamine et 1 équivalent de phénol sont placés sous agitation à TA dans 15 mL, de toluène pendant 72 heures. Le mélange réactionnel est ensuite filtré puis concentré sous pression réduite. Le résidu brut est purifié par chromatographie sur gel de silice avec comme éluant un mélange éther/hexane 1: 1. Le produit final est obtenu sous forme d'une pâte avec un rendement de 65%.
Le produit obtenu correspond à la formule suivante :

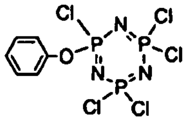

RMN $^{31}P\{^1H\}$ ($CDCl_3$, 81 MHz) : δ = 15,7 (d, $^2J_{CP}$ = 60 Hz, PCl(OPh)), 25,9 (d, $^2J_{CP}$ = 60 Hz, $PCl_2$).

Un dendrimère est ensuite construit en utilisant des méthodes bien connues de l'homme de l'art, notamment décrites dans « A general synthetic strategy for neutral phosphorus containing dendrimers » Launay N., Caminade A.M., Lahana R., Majoral J.P., Angew. Chem. 1994, 106, 1682. Angew. Chem. Int. Ed. Engl. 1994, 33, 1589 et dans «Synthesis of bowl-shaped dendrimers from generation 1 to generation 8 » Launay N., Caminade A.M., Majoral J.P., J. Organomet. Chem. 1997, 529, 51. Puis le phénol bisphosphonate est greffé en surface selon la méthode utilisée pour les dendrimères symétriques des Exemples précédents.

En particulier, on peut procéder comme suit :

*Etape 2 : greffage de l'hydroxybenzaldéhyde*

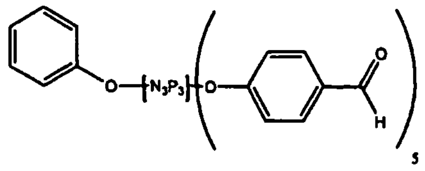

A une solution contenant 650 mg de coeur dendritique obtenu à l'étape précédente dans 20 mL de THF sont additionnés 1170 mg de 4-hydroxybenzaldéhyde et 6800 mg de carbonate de cesium. Le mélange est agité à TA pendant une nuit, et les sels sont éliminés par centrifugation. La solution résultante est ensuite concentrée sous pression réduite et précipitée avec un mélange Ether/Pentane (1/1) pour donner une poudre blanche avec un rendement de 82%. RMN $^{31}P\{^1H\}$ ($CDCl_3$, 81 MHz) : δ = 12,1 (sl, $N_3P_3$).

*Etape 3 : condensation de phosphorhydrazide: première génération du dendrimère*

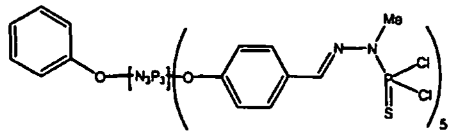

A une solution contenant 1000 mg de coeur dendritique obtenu à l'étape précédente dans 20 mL de $CHCl_3$ sont additionnés 30 mL de dichlorothiophosphorhydrazide fraîchement synthétisé à 0,24M dans le $CHCl_3$. La solution est agitée à TA pendant une nuit puis le brut réactionnel est concentré sous pression réduite et précipité par ajout de pentane. La poudre blanche obtenue est ensuite purifiée par chromatographie sur gel de silice (éluant $CH_2Cl_2$/pentane, 1/1) avec un rendement de 76%.
RMN $^{31}P\{^1H\}$ ($CDCl_3$, 81 MHz) : δ = 11,7 (sl, $N_3P_3$), 65,9 (s, P=S), 66,0 (s, P=S).

*Etape 4 : greffage du phénol aminobisphosphonate dérivé de la tyramine*

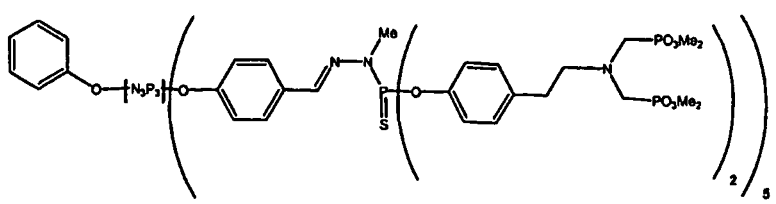

A une solution contenant 120 mg de dendrimère obtenu à l'étape précédente dans 20 mL de THF sont additionnés 279 mg de phénol aminobisphosphonate dérivé de la tyramine obtenu dans l'exemple 31 et 516 mg de carbonate de césium. La suspension est agitée à TA pendant une et les sels sont éliminés par centrifugation. La solution résultante

est ensuite concentrée sous pression réduite et précipitée avec un mélange Ether/Pentane (1/1) pour donner une poudre blanche avec un rendement de 86 %.
RMN $^{31}P\{^1H\}$ ($CDCl_3$, 81 MHz) : $\delta$ = 11,9 (s l, $N_3P_3$), 30,3 (s, P=0), 66,7 (s, P=S).

*Etape 5 : dendrimère de première generation à branche manquante et à extrémités sel de Na*

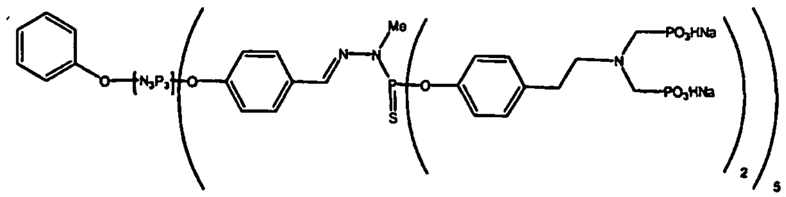

A une solution contenant 100 mg de dendrimère obtenu à l'étape précédente dans 5 mL d'acétonitrile à 0°C sous atmosphère inerte sont additionnés 52 équivalents de BrTMS. A la fin de l'addition, le milieu réactionnel est agité à température ambiante pendant 12 heures. Le milieu réactionnel est ensuite évaporé à sec puis sont ajoutés 2,5 mL de méthanol à température ambiante. Le milieu réactionnel est agité pendant 1 heure puis évaporé à sec. Cette opération de méthanolyse est répétée une deuxième fois puis le produit est lavé plusieurs fois à l'éther diéthylique. Le produit résultant, pour des raisons d'analyse RMN, est ensuite transformé en son sel de sodium. Le produit est tout d'abord mis en présence d'eau (1 mL) puis 10 équivalents de soude aqueuse (0,1966 N) sont ajoutés. Après dissolution totale du dendrimère, la solution est lyophilisée, ce qui permet d'obtenir le dendrimère sous forme d'une poudre blanche avec un rendement de 79%.
RMN $^{31}P\{^1H\}$ ($CDCl_3$, 81 MHz) : $\delta$ = 9,9 (s, P=0), 11,8 (sl, $N_3P_3$), 66,3 (s, P=S).

**Exemple 92 : synthèse d'un dendrimère de première génération ayant deux fonctions du noyau bloquées par un biphénol et à extrémités acide aza-bis-phosphonique dérivé de la tyramine**

*Etape 1 : synthèse du tétrachloro-(2,2'-dihydroxybiphényl)cyclotriphosphazène*

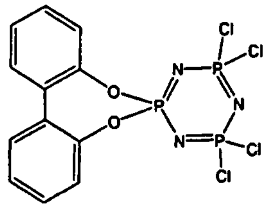

La synthèse de cette molécule a été réalisée suivant la procédure décrite par R. Pelc et al. Phosphorus, Sulfur and Silicon 1990, 47, 375 - 382.

*Etape 2 : synthèse du tétra(4-formylphénoxy)(2,2'-dihydroxybiphényl)cyclotriphosphazène*

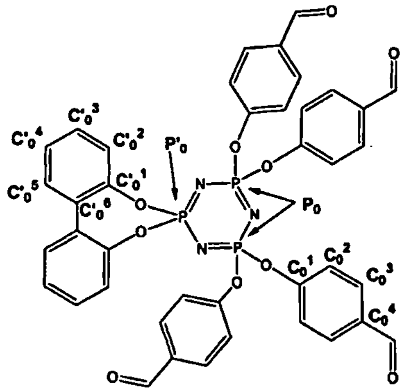

A une solution contenant 1,270 mg (2,76 mmol) de tétrachloro-(2,2'-dihydroxybiphényl) cyclotriphosphazène dans 4 mL de THF sont ajoutés 1,640 mg (11,39 mmol) de sel de sodium du 4-hydroxybenzaldéhyde. Le mélange réactionnel est agité pendant 12 heures. Après dilution du milieu réactionnel avec 20 mL de THF, les sels de sodium sont retirés par centrifugation. Après évaporation à sec, le produit est lavé deux fois au méthanol. Le produit est isolé avec un rendement de 90 %.

RMN $^{31}P\{^1H\}$ ($CDCl_3$, 81 MHz) : ô = 11,5 (d, $^2J_{PP}$ = 94,8 Hz, $P_0$); 27,5 (t, $^2J_{PP}$ = 94,8 Hz, $P'_0$) ppm.

RMN $^1H$ ($CDCl_3$, 200,13 MHz) : δ = 6,78 (m, 2H, $C'_0{}^2$-H); 7,37 (m, 12H, $C'_0{}^3$-H, $C'_0{}^5$-H, $C_0{}^2$-H); 7,55 (m, 2H, $C'_0{}^4$-H); 7.82 (m, 8H, $C_0{}^3$-H); 9,95 (s, 4H, CHO) ppm.

RMN $^{13}C\{^1H\}$ ($CDCl_3$, 50,32 MHz) : δ = 121,3 (d, $^3J_{CP}$ = 4,6 Hz, $C'_0{}^2$); 121,5 (d, $^3J_{CP}$ = 7,3 Hz, $C_0{}^2$); 126,5 (d, $^4J_{CP}$ = 1,6 Hz, $C'_0{}^5$); 128,4 (d, $^3J_{CP}$ = 1,4 Hz, $C'_0{}^6$); 129,9 (s, $C'_0{}^3$, $C'_0{}^4$); 131,4 (s, $C_0{}^3$); 133,7 (s, $C_0{}^4$); 147,5 (d, $^2J_{CP}$ = 9,3 Hz, $C'_0{}^1$); 154,8 (t, $^2J_{CP}$ = 3,7 Hz, $C_0{}^1$); 190,6 (s, CHO) ppm.

*Etape 3 : Synthèse du dendrimère à extrémités chlorées*

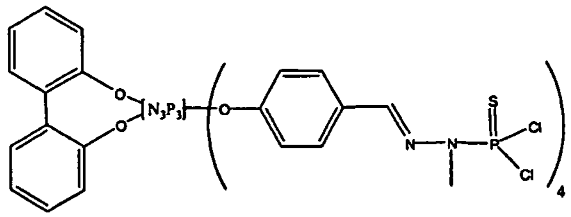

A une solution contenant 129 mg (0,16 mmol) de tétra(4-formylphénoxy)(2,2'-dihydroxy biphényl)cyclotriphosphazène dans 2 mL de THF sont ajoutés à 0°C 3 mL (0,66 mmol) d'une solution de dichlorothiophospho-N-méthylhydrazide à 0,22 mol.l$^{-1}$. Le mélange réactionnel est agité à 0°C pendant 2 heures. Le produit est purifié par lavages THF / pentane et isolé sous forme d'un solide blanc avec un rendement de 95 %.

RMN $^{31}P\{^1H\}$ ($CDCl_3$, 81 MHz) : δ = 11,5 (d, $^2J_{PP}$ = 96 Hz, $P_0$); 27,2 (t, $^2J_{PP}$ = 96 Hz, $P'_0$); 66,3 (s, $P_1$) ppm.

*Etape 4 : Synthèse du dendrimère à extrémités aza-bis-diméthyl-phosphonate dérivé de la tyramine*

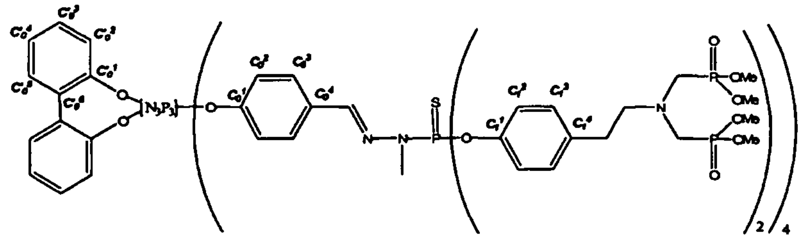

A une solution contenant 206 mg (0,14 mmol) du dendrimère à extrémités chlorées de l'étape précédente dans

4 mL de THF sont ajoutés à température ambiante 500 mg (1,31 mmol) de phénol aza-bis-diméthyl-phosphonate dérivé de la tyramine obtenu dans l'**Exemple 31**, puis 750 mg (2,3 mmol) de carbonate de césium. Le mélange réactionnel est agité pendant 12 heures, dilué avec 20 mL de THF et les sels de césium sont retirés par centrifugation. Après évaporation des volatiles, le résidu est dilué dans le minimum de THF et précipité avec un large excès de pentane. On obtient ainsi le produit attendu sous forme d'un solide visqueux avec un rendement de 90%.
RMN $^{31}P\{^1H\}$ ($CDCl_3$, 81 MHz) : $\delta$ = 11,5 (d, $^2J_{PP}$ = 96 Hz, $P_0$); 27,5 (t, $^2J_{PP}$ = 96 Hz, $P'_0$); 29,8 (s, $PO_3Me_2$); 65,9 (s, $P_1$) ppm.
RMN $^1H$ ($CDCl_3$, 200,13 MHz) : $\delta$ = 2,74 (t déformé, $^3J_{HH}$ = 7,4 Hz, 16H, $\underline{CH_2}$-$CH_2$-N); 3,04 (t déformé, $^3J_{HH}$ = 7,4 Hz, 16H, $CH_2$-$\underline{CH_2}$-N); 3,18 (d, $^2J_{HP}$ = 9,1 Hz, 32H, N-$CH_2$-P); 3,72 (d, $^3J_{HP}$ =10,5 Hz, 96H, P(O)(OMe)); 6,78-7,69 (m, 60H, $H_{arom}$ et CH=N) ppm.

*Etape 5: Synthèse du dendrimère à extrémités sel de sodium de l'acide aza-bisphosphonique dérivé de la tyramine*

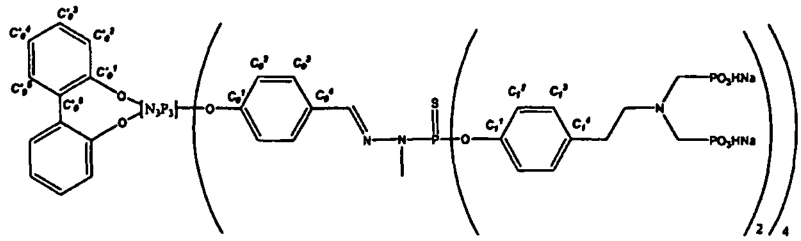

A une solution de 3 mL d'acétonitrile maintenue à 0°C sous atmosphère inerte contenant 200 mg du composé de l'étape précédente sont ajoutés lentement 35 équivalents de bromotriméthylsilane. A la fin de l'addition, le milieu réactionnel est agité à température ambiante pendant 12 heures. Le milieu réactionnel est ensuite évaporé à sec puis 1 mL de méthanol est ajouté à température ambiante. Le milieu réactionnel est agité pendant 1 heure puis évaporé à sec. Cette opération de méthanolyse est répétée une deuxième fois puis le résidu brut est lavé plusieurs fois à l'éther diéthylique.
Le produit résultant, pour des raisons d'analyse RMN, est ensuite transformé en son sel de sodium. Le produit est tout d'abord mis en présence d'eau (1 mL) puis 16 équivalents de soude aqueuse (0,1966 N) sont ajoutés. La solution résultante est lyophilisée, ce qui permet d'obtenir le composé sous forme d'une poudre blanche avec un rendement de 82%.
RMN $^{31}P\{^1H\}$ ($CDCl_3$, 81 MHz) : $\delta$ = 9,5 (sl, $PO_3HNa$); 11,0 (d, $^2J_{PP}$ = 95 Hz, $P_0$); 27,1 (t, $^2J_{PP}$ = 95 Hz, $P'_0$); 65,7 (s, $P_1$) ppm.

**Exemple 93 : synthèse d'un dendron de génération 1 à extrémités aza-bis-diméthyl-phosphonate dérivé de la tyramine**

On désigne par dendron un dendrimère ayant une ou plusieurs chaînes de liaison manquantes lesdites chaînes de liaison manquantes étant remplacées par un groupe réactif.

*Etape 1 : Synthèse de dichlorothiophospho-(N-méthyl)-(4-méthoxyphényl)-hydrazone*

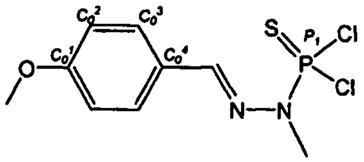

A une solution de dichlorothiophospho-N-méthylhydrazide (4,4 mmol) dans le chloroforme (22 mL) est ajouté, à 0°C, du para-anisaldehyde (2,71 mmol, 330 µL). Le mélange réactionnel est agité pendant 12 heures. Après évaporation du solvant de réaction, le produit est lavé à l'éther puis purifié par chromatographie. Le produit est isolé sous forme d'une poudre blanche avec un rendement de 90 %.

RMN $^{31}P\{^1H\}$ ($CDCl_3$, 81 MHz) : $\delta$ = 66,9 (s, $P_1$) ppm.
RMN $^1H$ ($CDCl_3$, 200,13 MHz) : $\delta$ = 3,50 (d, $^3J_{HP}$ = 14,1 Hz, 3H, $CH_3$-N); 3,87 (s, 3H, $CH_3$-O); 6,96 (d, $^3J_{HH}$ = 8,1 Hz, 2H, $C_0^2$-H); 7,66 (s, CH=N); 7,68 (d, $^3J_{HH}$ = 8,2 Hz, 2H, $C_0^3$-H) ppm.

RMN $^{13}\{^1H\}$ ($CDCl_3$, 50,32 MHz) : $\delta$ = 31,8 (d, $^2J_{CP}$ = 13,1 Hz, $CH_3$-N); 55,4 (s, $CH_3$-O); 114,2 (s, $C_0^2$); 126,9 (s, $C_0^4$); 128,9 (s, $C_0^3$); 141,3 (d, $^3J_{CP}$ = 18,7 Hz, CH=N); 161,2 (s, $C_0^1$) ppm.

*Etape 2 : Synthèse d'un dendron de génération 0 à extrémités aza-bis-diméthyl-phosphonate dérivé de la tyramine*

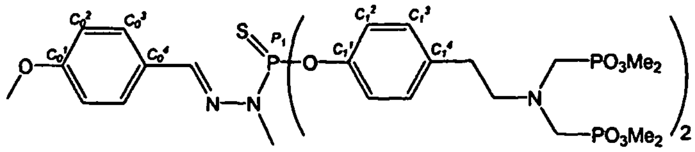

A une solution contenant 596 mg du phénol aza-bis-diméthyl-phosphonate dérivé de la tyramine obtenu dans **l'<u>Exemple 31</u>** (1,49 mmol) dans 5 mL l'acétone est ajouté à température ambiante 195 mg de dichlorothiophospho-(N-méthyl)-(4-méthoxyphényl)-hydrazone (0,66 mmol), puis 881 mg de carbonate de césium (2,70 mmol). Le mélange réactionnel est agité pendant 12 heures, dilué avec 20 mL de THF et les sels de césium sont retirés par centrifugation. Après évaporation des volatiles, le résidu est dilué dans le minimum de THF et précipité avec un large excès de pentane. On obtient ainsi le produit attendu sous forme d'un solide visqueux avec un rendement de 94 %.
RMN $^{31}P\{^1H\}$ ($CDCl_3$, 81 MHz) : $\delta$ = 30,3 (s, $PO_3Me_2$); 66,6 (s, $P_1$) ppm.
RMN $^1H$ ($CDCl_3$, 200,13 MHz) : $\delta$ = 2,73 (t déformé, $^3J_{HH}$ = 7,1 Hz, 4H, $\underline{CH_2}$-$CH_2$-N); 3,03 (t déformé, $^3J_{HH}$ = 7,1 Hz, 4H, $CH_2$-$\underline{CH_2}$-N); 3,16 (d, $^3J_{HP}$ = 9,0 Hz, 8H, N-$CH_2$-P); 3,30 (d, $^3J_{HP}$ = 11,1 Hz, 3H, $CH_3$-N), 3,71 (d, $^3J_{HP}$ = 10,4 Hz, 24H, P(O)($O\overline{C}H_3)_2$); 3,84 (s, 3H, $CH_3$-O); 6,92 (d, $^3J_{HH}$ = 8,8 Hz, 2H, $C_0^2$-H); 7,13 (m, 8H, $C_1^2$-H, $C_1^3$-H); 7,59 (s, CH=N); 7,66 (d, $^3J_{HH}$ = 8,7 Hz, 2H, $C_0^3$-H) ppm.
RMN $^{13}C\{^1H\}$ ($CDCl_3$, 50,32 MHz) : $\delta$ = 32,8 (s, $\underline{CH_2}$-$CH_2$-N); 33,0 (s, $CH_3$-N); 49,4 (dd, $^1J_{CP}$ = 157,4 Hz, $^3J_{CP}$ = 7,1 Hz, N-$CH_2$-P); 52,6 (d, $^2J_{CP}$ = 5,2 Hz, $PO_3Me_2$); 55,4 (s, $CH_3$-O); 58,1 (t, $^3J_{CP}$ = 7,4 Hz, $CH_2$-$\underline{CH_2}$-N); 114,1 (s, $C_0^2$); 121,3 (d, $^3J_{CP}$ = 4,4 Hz, $C_1^2$); 127,7 (s, $C_0^4$); 128,4 (s, $C_0^3$); 129,8 (s, $C_1^3$); 136,3 (s, $C_1^4$); 139,4 (d, $^3J_{CP}$ = 4,5 Hz, CH=N); 149,0 (d, $^2J_{CP}$ = 6,6 Hz, $C_1^1$); 160,6 (s, $C_0^1$) ppm.

*Etape 3 : Synthèse du dendron de génération 0 à extrémités sel de sodium de l'acide aza-bis-phosphonique dérivé de la tyramine*

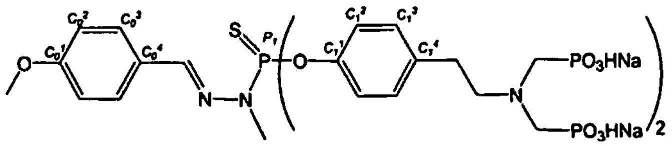

A une solution contenant 286 mg du composé de l'étape 2 (0,29 mmol) dans 3 mL d'acétonitrile maintenue à 0°C sous atmosphère inerte est ajouté lentement 0,396 mL de bromotriméthylsilane (3,0 mmol). A la fin de l'addition, le milieu réactionnel est agité à température ambiante pendant 12 heures. Le milieu réactionnel est ensuite évaporé à sec puis 1 mL de méthanol est ajouté à température ambiante. Le milieu réactionnel est agité pendant 1 heure puis évaporé à sec. Cette opération de méthanolyse est répétée une deuxième fois puis le résidu brut est lavé plusieurs fois à l'éther diéthylique.

Le produit résultant, pour des raisons d'analyse RMN, est ensuite transformé en son sel de sodium. Le produit est tout d'abord mis en présence d'eau (1 mL) puis 4,60 mL de soude aqueuse est ajoutée (0,1966 N). La solution résultante est lyophilisée, ce qui permet d'obtenir le dendron sous forme d'une poudre blanche avec un rendement de 80%.
RMN $^{31}P\{^1H\}$ ($CD_3CN/D_2O$, 81 MHz) : $\delta$ = 10,1 (s, $PO_3HNa$); 68,4 (s, $P_1$) ppm.
RMN $^1H$ ($CD_3CN/D_2O$, 500,33 MHz) : $\delta$ = 2,99 (t déformé, $^3J_{HH}$ = 7,5 Hz, 4H, $\underline{CH_2}$-$CH_2$-N); 3,19 (d, $^3J_{HP}$ =10,6 Hz, 3H, $CH_3$-N); 3,34 (d, $^2J_{HP}$ = 11,7 Hz, 8H, N-$CH_2$-P); 3,56 (t déformé, $^3J_{HH}$ = 7,5 Hz, 4H; $CH_2$-$\underline{CH_2}$-N); 3,73 (s, 3H, $CH_3$-O); 6,90 (d, $^3J_{HH}$ = 8,7 Hz, 2H, $C_0^2$-H); 7,09 (d, $^3J_{HH}$ = 8,1 Hz, 4H, $C_1^2$-H); 7,23 (d, $^3J_{HH}$ = 8,1 Hz, 4H, $C_1^3$-H); 7,55 (d, $^3J_{HH}$ = 8,5 Hz, 2H, $C_0^3$-H); 7,76 (s, 1H, CH=N) ppm.

RMN $^{13}C\{^1H\}$ ($CD_3CN/D_2O$, 125,86 Hz) : δ = 28,9 (s, $\underline{CH_2}$-$CH_2$-N); 32,6 (d, $^2J_{CP}$ = 11,3 Hz, $CH_3$-N); 52,3 (d, $^1J_{CP}$ = 130,8 Hz, N-$CH_2$-P); 55,4 (s, $CH_3$-O); 57,5 (s, $CH_2$-$\underline{CH_2}$-N); 114,3 (s, $C_0^2$); 121,5 (d, $^3J_{CP}$ = 5,0 Hz; $C_1^2$); 127,3 (s, $C_0^4$); 128,8 (s, $C_0^3$); 130,5 (s, $C_1^3$); 134,0 (s, $C_1^4$); 143,0 (d, $^3J_{CP}$ = 3,8 Hz, CH=N); 148,9 (d; $^2J_{CP}$ = 6,3 Hz, $C_1^1$); 160,2 (s, $C_0^1$) ppm.

**Exemple 94 : synthèse d'un dendrimère de génération 0 à coeur PS et à extrémités acide aza-bis-phosphonique dérivé de la tyramine**

*Etape 1 : synthèse du dendrimère de génération 0 à coeur PS et à extrémités aza-bis-diméthyl-phosphonate dérivé de la tyramine*

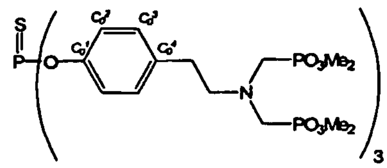

A une solution contenant 700 mg du phénol aza-bis-diméthyl-phosphonate dérivé de la tyramine obtenu dans l'**Exemple 31** (1,75 mmol) dans 8 mL d'acétonitrile est ajouté à 0°C et sous atmosphère inerte 0,058 mL de trichlorothiophosphore (0,57 mmol), puis 1,140 g de carbonate de césium (3,50 mmol). Le mélange est agité pendant 12 heures à température ambiante puis les sels formés sont éliminés par centrifugation. Après évaporation du solvant de réaction, le produit est dilué dans un minimum de THF et précipité par ajout d'un grand volume de pentane. Ces lavages par précipitation permettent d'isoler le produit sous forme d'une huile avec un rendement de 98 %.
RMN $^{31}P\{^1H\}$ ($CDCl_3$, 81 MHz) : δ = 30,3 (s, $P(O)(OCH_3)_2$); 57,0 (s, $P_0$) ppm.
RMN $^1H$ ($CDCl_3$, 300,13 MHz) : δ = 2,79 (t déformé, $^3J_{HH}$ = 7,3 Hz, 6H, $\underline{CH_2}$-$CH_2$-N); 3,05 (t déformé, $^3J_{HH}$ = 7,3 Hz, 6H, $CH_2$-$\underline{CH_2}$-N); 3,18 (d, $^2J_{HP}$ = 9,0 Hz, 12H, N-$CH_2$-P); 3,72 (d, $^3J_{HP}$ = 10,5 Hz, 36H, $P(O)(OCH_3)_2$); 7,11 (d, $^3J_{HH}$ = 8,0 Hz, 6H, $C_0^2$-H); 7,22 (d, $^3J_{HH}$ = 8,0 Hz, 6H, $C_0^3$-H) ppm.

*Etape 2 : Synthèse du dendrimère de génération 0 à coeur PS et à extrémités sel de sodium de l'acide aza-bis-phosphonique dérivé de la tyramine*

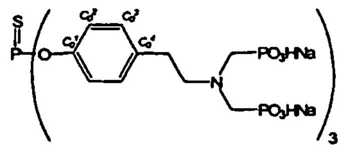

A une solution à 0°C sous atmosphère inerte contenant 325 mg du dendrimère de génération 0 à extrémités aza-bis-diméthyl-phosphonate dérivé de la tyramine (0,27 mmol) obtenu à l'étape précédente dans 3 mL d'acétonitrile sont ajoutés lentement 492 μl de bromotriméthylsilane (3,7 mmol). A la fin de l'addition, le milieu réactionnel est agité à température ambiante pendant 12 heures. Le milieu réactionnel est ensuite évaporé à sec puis 2,5 mL de méthanol sont ajoutés à température ambiante. Le milieu réactionnel est agité pendant 1 heure puis évaporé à sec. Cette opération de méthanolyse est répétée une deuxième fois puis le produit est lavé plusieurs fois à l'éther diéthylique.

Le produit résultant, pour des raisons d'analyse RMN, est ensuite transformé en son sel de sodium. Le produit est tout d'abord mis en présence d'eau (1 mL) puis 6,22 mL de soude aqueuse est ajoutée (0,1966 N). Après dissolution totale du dendrimère, la solution est lyophilisée, ce qui permet d'obtenir le dendrimère sous forme d'une poudre blanche avec un rendement de 75%.
RMN $^{31}P\{^1H\}$ ($D_2O/CD_3CN$, 81 MHz) : δ = 10,2 (s, $PO_3HNa$); 58,5 (s, $P_0$) ppm.
RMN $^1H$ ($D_2O/CD_3CN$, 200,13 MHz) : δ = 3,01-4,30 (m, 24H, $CH_2$); 7,51 - 7,63 (m, 12H, $C_0^2$-H, $C_0^3$-H) ppm.
RMN $^{13}C\{^1H\}$ ($D_2O/CD_3CN$, 50,32 Mz) : δ = 31,8 (s, $\underline{CH_2}$-$CH_2$-N); 55,5 (d, $^1J_{CP}$ = 128,3 Hz, N-$\underline{CH_2}$-P); 60,6 (s, $CH_2$-$\underline{CH_2}$-N); 124,2 (d, $^3J_{CP}$ = 4,48 Hz, $C_0^2$); 133,7 (s, $C_0^3$); 137,5 (s, $C_0^4$); 152,1 (d, $^2J_{CP}$ = 8,7 Hz, $C_0^1$) ppm.

**Exemple 95 : synthèse de dendrimères phosphorés de type Salamonczyk**

*Etape 1 : Synthèse d'une diphénoxyamino phosphine dérivée de la tyramine*

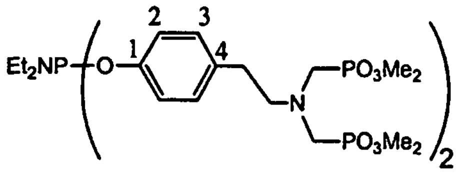

Le protocole expérimental utilisé pour préparer cette molécule a été inspiré de celui utilisé par Salamonczyk pour faire croître ses dendrimères (Tetrahedron Lett. 2000, 41, 1643). Le dérivé de la tyramine aza bis phosphonate obtenu dans l'**Exemple 31** est pesé dans un tube de Schlenk sous argon (2,3 g) et dissous dans 10 mL de THF distillé. La diethylaminodichlorophosphine est introduite dans un autre tube de Schlenk (0,5 mL) et mise en solution dans 5 mL de THF distillé. Les deux tubes de Schlenk sont placés à -70°C. 1,4 mL de triéthylamine sont alors ajoutés à la solution de dichlorophosphine puis la solution de tyramine aza bis phosphonate est canulée sur le mélange toujours à -70°C. L'agitation est maintenue une demi heure à froid puis 4 heures à température ambiante. Le mélange est alors filtré sur célite sous argon puis le solvant est éliminé sous pression réduite. Le résidu sec est conservé sous argon à froid et utilisé sans autre traitement dans la suite de la synthèse.
RMN $^{31}P\{^1H\}$ ($CDCl_3$) : δ = 30,4 (s, $PO_3Me_2$); 144,5 (s, $Et_2NP$) ppm.
RMN $^1H$ ($CDCl_3$) : δ = 1,00 (t, $^3J_{HH}$ = 7,2 Hz, 6H, $C\underline{H_3}CH_2$); 2,70 (m, 4H, N-$\underline{CH_2}CH_2$); 3,00 (m, 4H, $CH_2\underline{CH_2}P$); 3,11-3,23 (m, 12H, $CH_2P$, $CH_3\underline{CH_2}$); 3,69 (d, $^3J_{HP}$ = 6,9 Hz, 24H, $CH_3O$); 6,90 (d, $^3J_{HH}$ = 8,4 Hz, 4H, $C^2H$); 6,97 (d, $^3J_{HH}$ = 8,4 Hz, 4H, $C^3H$) ppm.

*Etape 2 : Couplage avec un dendrimère de génération 0 de type Salamonczyk*

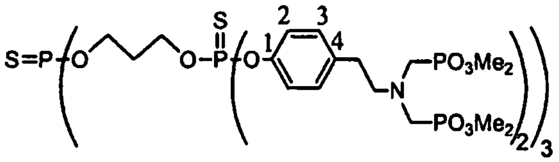

2,95 g de diéthylaminophosphine obtenus dans l'étape 1 sont mis en solution dans 10 mL de dichlorométhane sous argon. On lui ajoute à température ambiante une solution de 219 mg de $(S)P(O\text{-}(CH_2)_3OH)_3$ (synthétisé comme décrit dans G.M. Salamonczyk, Tetrahedron Lett. 2000, 41, 1643) dans 10 mL de dichlorométhane. Ce mélange est additionné sur une solution de 400 mg de tétrazole dans 5 mL d'acétonitrile. La solution ainsi obtenue est agitée 3 heures à température ambiante sous argon puis on lui additionne une pointe de spatule de soufre et on maintient l'agitation à TA jusqu'à ce que toutes les phosphines soient soufrées (contrôle par RMN $^{31}P$) soit environ 5 jours. Le mélange est filtré puis le solvant évaporé. Le résidu sec est ensuite chromatographié sur gel de silice avec un mélange d'éluant $CHCl_3$ / MeOH (90 / 10). Rf = 0,41.
RMN $^{31}P\{^1H\}$ ($CDCl_3$) : δ = 30,3 (s, $PO_3Me_2$); 62,3 (s, P1); 71,7 (s, P0) ppm.
RMN $^1H$ ($CDCl_3$): δ = 2,08 (q, $^3J_{HH}$ = 5,7 Hz, 6H, $CH_2\underline{CH_2}CH_2$); 2,75 (m, 12H, $\underline{CH_2}C_6H_4$); 3,04 (m, 12H, $NC\underline{H_2}$); 3,19 (d, $^2J_{HP}$ = 9,2 Hz, 24H, $CH_2P$); 3,73 (d, $^3J_{HP}$ = 10,5 Hz, 72H, $CH_3O$); 4,18 (dt, $^3J_{HH}$ = 5,7 Hz, $^3J_{HP}$ = 8,7 Hz, 6H, $CH_2OP(S)$); 4,33 (dt, $^3J_{HH}$ = 6,2 Hz, $^3J_{HP}$ = 9,2 Hz, 6H, $CH_2OP(S)$), 7,05 (d, $^3J_{HH}$ = 7,8 Hz, 12H, $C^2H$); 7,18 (d, $^3J_{HH}$ = 7,8 Hz, 12H, $C^3H$) ppm.
RMN $^{13}C\{^1H\}$ ($CDCl_3$) : δ = 30,6 (dd, $^3J_{CP}$ = $^3J_{CP}$ = 7,6 Hz, $CH_2\underline{C}H_2CH_2$); 33,0 (s, $\underline{C}H_2C_6H_4$); 49,4 (dd, $^1J_{CP}$ = 159,0 Hz, $^3J_{CP}$ = 7,7 Hz, $NCH_2P$); 52,8 (d, $^2J_{CP}$ = 7,4 Hz, $OCH_3$); 58,2 (t, $^3J_{CP}$ = 7,6 Hz, $CH_2\underline{C}H_2N$); 64,4 (d, $^2J_{CP}$ = 3,7 Hz, $P(S)O\underline{C}H_2CH_2CH_2$); 65,5 (d, $^2J_{CP}$ = 5,2 Hz, $\underline{C}H_2OP(S)OC_6H_4$); 120,8 (d, $^3J_{CP}$ = 5,0 Hz, $C^2$); 130,0 (s, $C^3$); 136,6 (s, $C^4$); 148,9 (d, $^2J_{CP}$ = 7,2 Hz, $C^1$) ppm.

*Etape 3 : Synthèse de la génération 0 de dendrimères de type Salamonczyk à extrémités sel de sodium de l'acide azabisphosphonique dérivé de la tyramine*

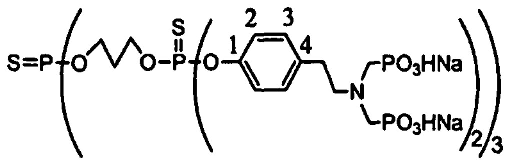

350 mg de dendrimère à extrémités azabisphosphonate sont mis en solution dans 5 mL d'acétonitrile sec; on lui additionne alors à 0°C goutte à goutte à la seringue 0,420 mL de BrTMS (soit 1.05 équivalent de BrTMS par liaison P-OMe). Après 30 minutes à 0°C le bain de glace est enlevé et l'agitation est maintenue une nuit à TA. Le mélange est tiré sous vide. On ajoute sur le résidu sec 5 mL de MeOH et on agite une heure. Après avoir tiré sous vide le résidu sec est hydrolysé pendant une heure à TA avec 5 mL d'eau distillée. Après lyophilisation la poudre est lavée deux fois avec 10 mL d'éther. Le produit est obtenu avec un rendement de 83 %. 6,4 mL d'une solution de NaOH 0,1966 M sont ajoutés pour former le mono sel de sodium et rendre le produit hydrosoluble. Après lyophilisation le produit est obtenu de façon quantitative et ne nécessite pas de purification supplémentaire.
RMN $^{31}P\{^1H\}$ ($D_2O$ / $CD_3CN$) : δ = 10,5 (s, $PO_3HNa$); 62,8 (s, P1); 70,8 (s, P0) ppm.
RMN $^{13}C\{^1H\}$ ($D_2O$/ $CD_3CN$) : δ = 31,9 (s, $\underline{C}H_2C_6H_4$); 33,0 (dl, $^3J_{CP}$ = 9,0 Hz, $CH_2\underline{C}H_2CH_2$); 54,9 (dl, $^1J_{CP}$ = 130,9 Hz, $NCH_2P$); 60,6 (sl, $CH_2\underline{C}H_2N$); 67,6 (sl, $P(S)O\underline{C}H_2CH_2CH_2$); 69,0 (sl, $\underline{C}H_2OP(S)OC_6H_4$); 124,3 (d, $^3J_{CP}$ = 3,4 Hz, $C^2$); 133,6 (s, $C^3$); 137,2 (s, $C^4$); 152,2 (d, $^2J_{CP}$ = 7,4 Hz, $C^1$) ppm.

**Exemple 96 : synthèse de dendrimères de type carbosilane à surface bis-phosphonate**

*Etape 1: Synthèse d'un allyl aminobisphosphonate*

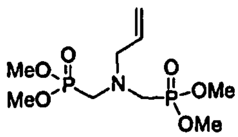

A une solution d'allylamine (1,5 mL, 20 mmol) dans le THF (6 mL) est additionné lentement le formaldéhyde aqueux (3,27 mL, 2,2 équivalents, 44 mmol). Après 15 minutes d'agitation, le phosphite de diméthyle (4,03 mL, 2,2 équivalents, 44 mmol) est ajouté et la solution est vigoureusement agitée 20 heures à température ambiante. 1,6 mL de formaldéhyde sont additionnés et après 15 heures d'agitation la solution est diluée avec 15 mL d'eau puis extraite par 100 mL de $CH_2Cl_2$. La phase organique est séchée sur $MgSO_4$, filtrée puis concentrée sous pression réduite. L'huile obtenue est lavée avec 2 fois 30 mL d'éther puis séchée. Le rendement est de 76 %.
RMN $^{31}P\{^1H\}$ ($CDCl_3$, 81,01 MHz) : δ = 30,5 (s, P=O).
RMN $^1H$ ($CDCl_3$); 200,13 MHz) : δ = 3,01 (d, 4H, $^2J_{HP}$ = 9,1 Hz, $PCH_2$); 3,26 (m, 2H, $\underline{CH_2}$-CH); 3,63 (d, 12H, $^3J_{HP}$ = 10,6, POMe); 5,10 (m, 2H, $CH_2$=); 5,67 (m, 1H, CH=).

*Etape 2 : Synthèse d'un dendrimère carbosilane à terminaisons azabisphosphonate*

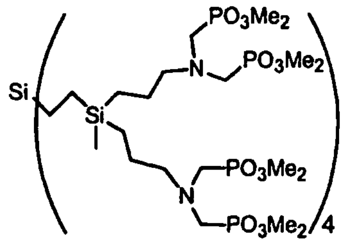

Le dendrimère de première génération présentant 8 liaisons Si-Cl en périphérie a été préparé selon la procédure décrite dans la littérature [L.-L. Zhou, J. Roovers Macromolecules 1993, 26, 963]. Le passage aux liaisons Si-H est réalisé avec $LiAlH_4$ / Ether selon la procédure décrite [ D. Seyferth, D. Y. Son Organometallics 1994, 13, 2682]. 500 mg (1,55 mmol) de dendrimère possédant 8 liaisons Si-H en périphérie sont mélangés avec 8,4 équivalents de N-allyl-aza-bis-phosphonate obtenu dans l'étape 1 (soit 13,64 mmol) et deux gouttes de catalyseur au platine en solution dans l'isopropanol ($H_2PtCl_6$,$nH_2O$). Le mélange est agité pendant 12h à 70°C puis une nuit à température ambiante. Le solvant est éliminé et le résidu sec est lavé à l'hexane. Le produit est alors purifié par chromatographie sur colonne de silice et obtenu avec un rendement de 65%.
RMN $^{31}P\{^1H\}$ ($CDCl_3$) $\delta$ =30,1 ppm.

*Etape 3 : Dendrimères carbosilanes à terminaisons acide aza-bis-phosphonique (sel de Na)*

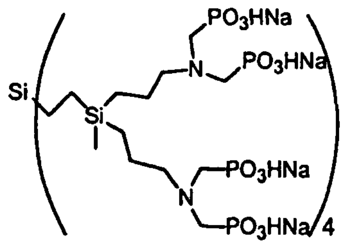

400 mg (0,15 mmol) de dendrimère carbosilane présentant 8 groupements aza-bis-phosphonate en périphérie sont dissous dans 4'mL d'acétonitrile fraîchement distillé et placés à 0°C. 8,4 équivalents de BrTMS (soit 1,28 mmol) sont alors additionnés goutte à goutte à la seringue. Après 30 minutes à 0°C et une nuit à température ambiante le mélange est tiré sous vide et le résidu sec est méthanolysé puis hydrolysé. Après lyophilisation le solide est lavé à l'éther. L'addition de 1 équivalent de NaOH (0,1955N en solution aqueuse) par fonction $PO_3H_2$ de surface conduit au sel de sodium correspondant avec un rendement de 68%.
RMN $^{31}P\{^1H\}$ ($D_2O$ / $CD_3COCD_3$) $\delta$ = 10,9 ppm.

Exemple 97 : dendrimère poly-L-Lysine à surface acide amino bisphosphonique dérivé de la glycine

*Etape 1 : déprotection et greffage de l'acide amino [bis-(dimethoxy-phosphorylméthyl)] :*

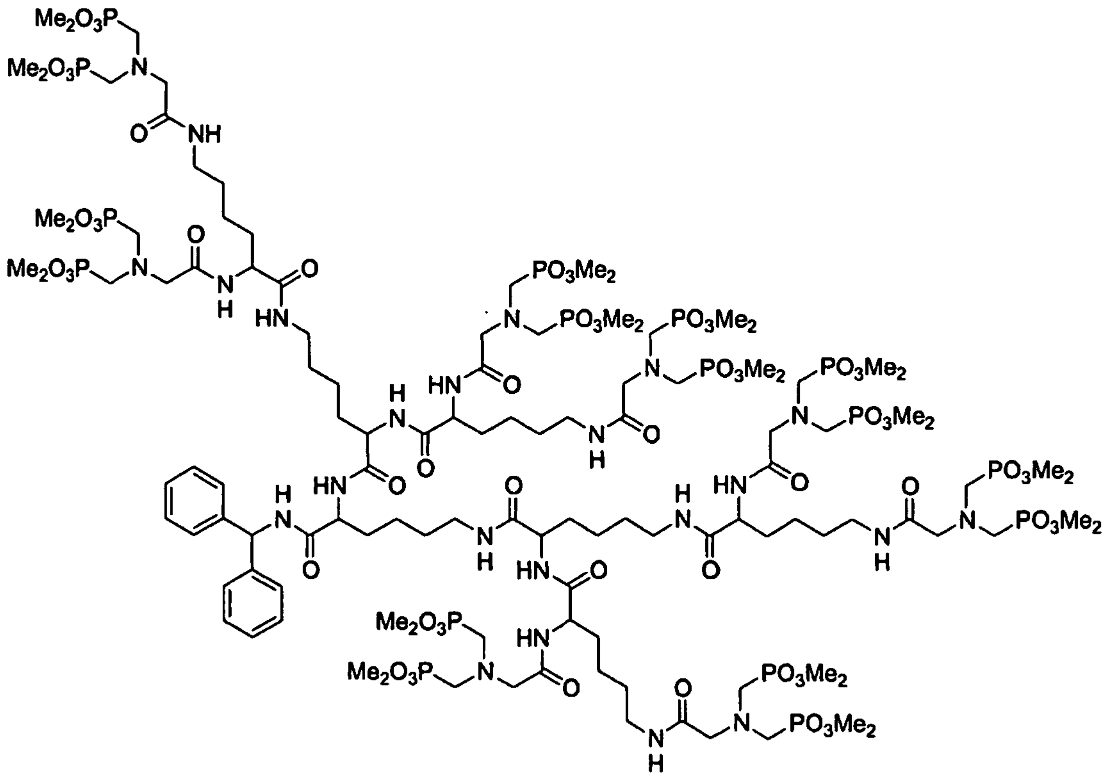

100 mg de dendrimère BHA-Lysine-100% BOC (Aldrich) de génération 1 sont mis en solution dans 20 mL de dichlorométhane contenant 30% d'acide trifluoroacétique pendant trois heures à 25°C. Après évaporation des volatiles, le produit brut est séché une nuit sous pression réduite, puis mis en solution dans 1 mL de DMF sec à 0°C avec 8 équivalents de triéthylamine. 8,8 équivalents d'acide carboxylique aza-bis-phosphonate obtenu dans **l'Exemple 51** (n = 1) sont introduits dans un autre ballon sous argon et dissous dans 5 mL de DMF sec à 0°C. On ajoute ensuite sous argon 8,8 équivalents de 1-hydroxybenzotriazole (HOBt), l'agitation est maintenue pendant 15 minutes à 0°C puis on additionne 8,8 équivalents de 1,3-dicyclohexylcarbodiimide (DCC). Le mélange est agité pendant 30 minutes à 0°C puis pendant 1h à température ambiante. On observe la formation d'un précipité. On ajoute alors la solution de dendrimère de première génération poly-L-lysine déprotégé dans 1 mL de DMF sec à 0°C, l'agitation est maintenue 15 minutes à 0°C puis une nuit à température ambiante. Le précipité est éliminé par filtration sur filtre seringue millipore 5μ puis la solution est lyophilisée. Le produit est purifié par dissolution dans un volume minimum de $CH_2Cl_2$ et précipitation dans un grand volume de diéthyléther. Ces précipitations sont répétées trois fois pour éliminer les traces de HOBt. Le produit est obtenu avec un rendement de 65% sous forme de poudre blanc cassé.

RMN $^{31}P-\{^1H\}$ ($CDCl_3$) $\delta$ = 30,2 ppm.

*Etape 2 : extrémités acide phosphonique*

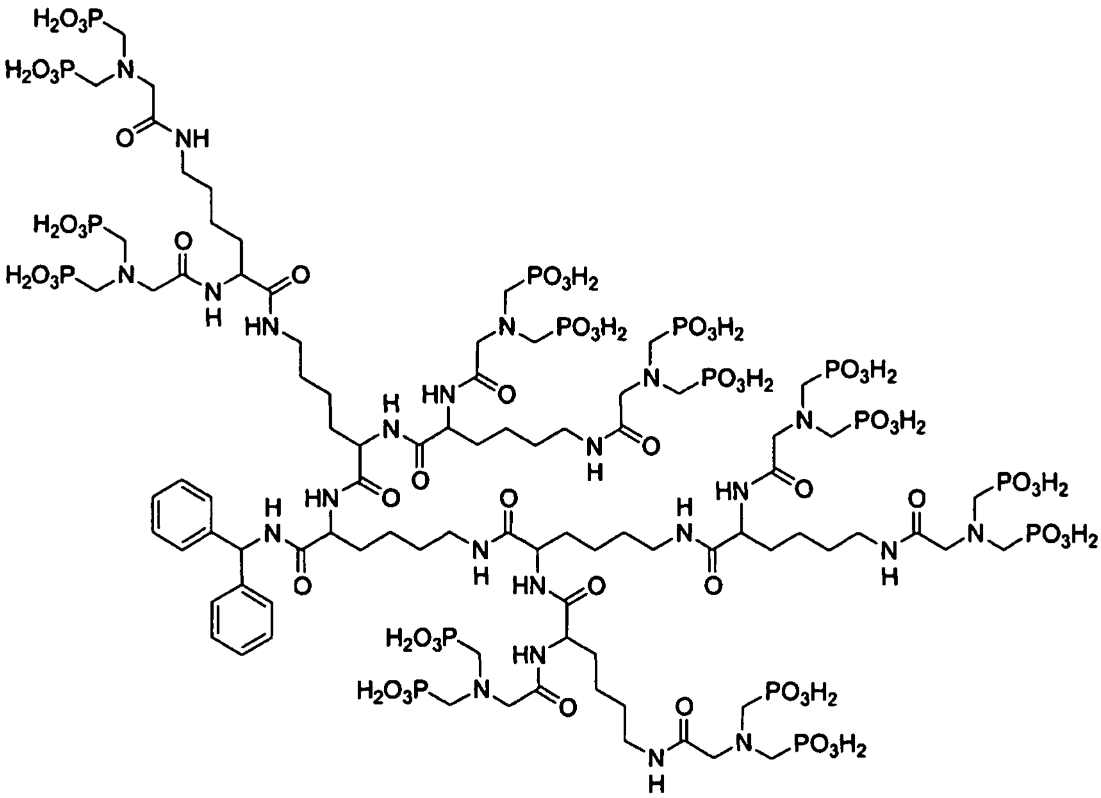

A 0,2 mmol de dendrimère de type poly-L-lysine à extrémités azabis-phosphonate de génération 1 de l'étape précédente, on ajoute sous atmosphère inerte 4 mL d'acétonitrile fraîchement distillé et le mélange est refroidi à 0°C. On ajoute alors goutte à goutte 12,8 mmol de BrTMS (soit 64 équivalents). Le mélange est maintenu à 0°C pendant 30 minutes puis sous agitation à température ambiante pendant 15 heures supplémentaires. L'acétonitrile est éliminé sous pression réduite puis le mélange est méthanolysé et hydrolysé comme dans les cas précédents. Le résidu sec est alors lavé deux fois avec un mélange THF / diéthyléther (1 / 9). La poudre est alors séchée sous vide pour conduire au produit pur avec un rendement de 68%.

RMN $^{31}P-\{^1H\}$ ($D_2O$, $CD_3COCD_3$) $\delta$ = 10,6 ppm.

*Etape 3 : sel de Na*

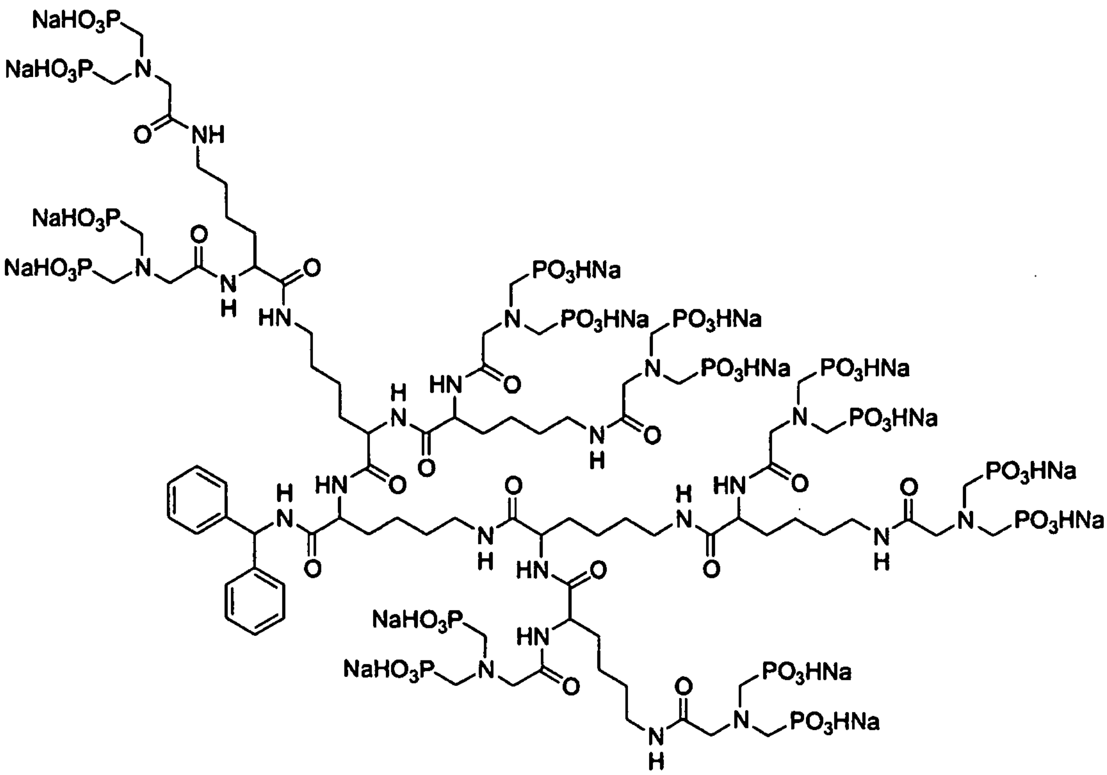

Le sel de sodium est obtenu par addition de 1 équivalent d'une solution aqueuse de soude 0,1955 N par groupement -$PO_3H_2$ présent dans la molécule obtenue dans l'étape précédente. Le dendrimère est isolé sous forme d'une poudre blanche avec un rendement de 86% après lyophilisation.
RMN $^{31}P-\{^1H\}$ ($D_2O$, $CD_3COCD_3$) $\delta$ = 18,5 ppm.

**Exemple 98 : Utilisation de dendrimères à surface aza-bis-phosphonique pour la culture de cellules**

Le composé dendrimérique à surface aza-bis-phosphonique GC1 (**Exemple 36**) sous sa forme de sel de sodium a été principalement utilisé pour la culture de cellules mononucléées du sang périphérique :

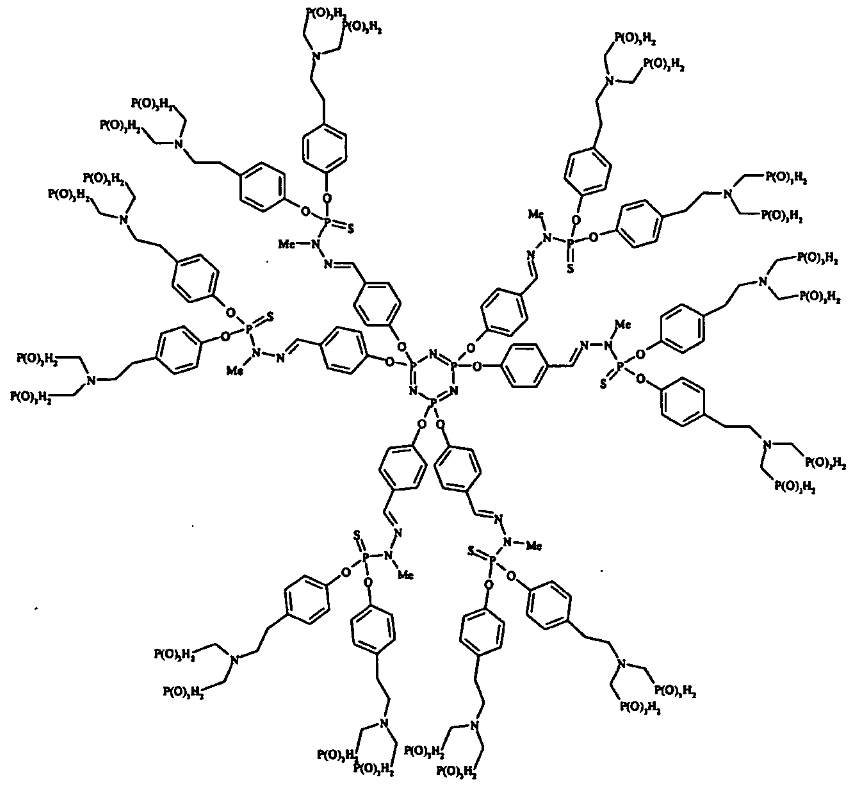

**GC1 (forme acide)**

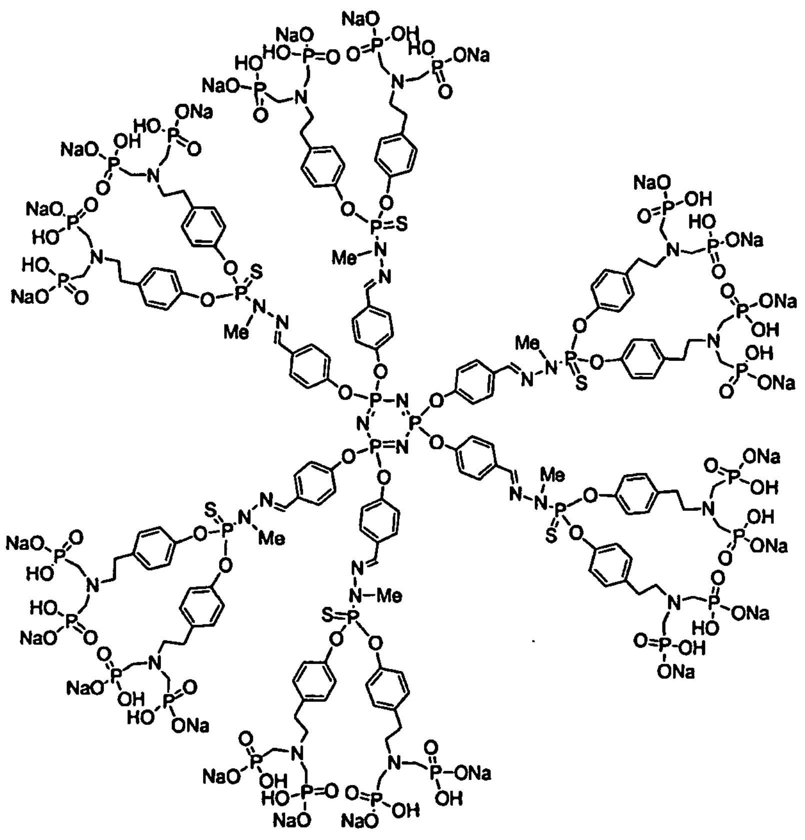

**GC1 (sel de sodium)**

Ce composé est soluble en solutions salines telles que tampons PBS à des concentrations inférieures ou égales à 1 mg/ml. On prépare des solutions stocks de GC1 en PBS stérile apyrogène (Cambrex Bio Science, Verviers, BELGIQUE), par exemple à une concentration de 2 mM (environ 1 mg/ml), soit 100X, que l'on stérilise ensuite par filtration sur micromembrane à 0,2 μm ou irradiation ; ces solutions sont conservées à 4°C.

**Procédé de culture cellulaire avec dendrimère, par exemple GC1**

Les cellules mononucléées du sang périphérique (PBMC) obtenues d'un donneur humain adulte sain par centrifugation sur gradient de densité (Ficoll-Hypaque, Amersham Pharmacia Biotech, Upsalla, SUÈDE) sont mises en culture dans des flacons de culture cellulaire stériles à une concentration de 1,5 millions de cellules par ml de milieu de culture de type RPMI 1640, 25 mM Hépès / Ultraglutamine 1 (Cambrex Bio Science, Verviers, BELGIQUE) supplémenté avec :

i) 1 mM pyruvate de sodium (Invitrogen Corporation, Paisley, ROYAUME-UNI)
ii) 100 μg/ml de streptomycine et 100 U/ml de pénicilline (Cambrex Bio Science, Verviers, BELGIQUE) ;
iii) 10% en volume de sérum de veau foetal (Invitrogen Corporation, Paisley, ROYAUME-UNI) décomplémenté 30 minutes à 56°C (SVF) ;
iv) 20 μM de dendrimère GC1 solubilisé à une concentration de 2 mM dans du PBS stérile apyrogène ;
v) le milieu de culture ainsi produit est additionné de 200 U/ml d'IL2 humaine recombinante (Sanofi-Synthélabo, Paris, FRANCE).

La culture se déroule pendant plusieurs jours en incubateur à 37°C, à humidité constante et dans une atmosphère de 5% de $CO_2$ dans l'air. Régulièrement pendant la durée de la culture, le milieu de culture est changé, par exemple par tiers ou moitié du volume, par exemple tous les 3 jours au début, puis tous les 2 jours et même tous les jours lorsque les cellules sont en phase exponentielle de multiplication. Au fur et à mesure de l'amplification du nombre de cellules, le volume de culture est augmenté pour maintenir la concentration cellulaire entre 0,7 et 1,5 million par ml. Le milieu de culture renouvelé est supplémenté par 10% de SVF, le dendrimère GC1 à 20 μM et de l'IL2 humaine recombinante à 200 U/ml sur le volume total de culture. Ces cultures *in vitro* durent de 15 à 25 jours.

**Activation et maintien en culture de monocytes, purifiés à partir de PBMC, sous l'effet du dendrimère GC1 (Figure 14**

Les monocytes purifiés à partir de PBMC (tri magnétique positif sur colonne des cellules CD14$^+$, systèmes Miltenyi Biotech ou StemCell Technologies Inc.) sont mis en culture dans les conditions décrites auparavant, mais sans IL2.

En 3 à 6 jours, la présence de GC1 (20 μM) dans le milieu de culture des monocytes entraîne des modifications morphologique (par exemple grossissement des monocytes, **Figure 14A**) et phénotypique des cellules (par exemple diminution de l'expression des marqueurs HLA-A,B et C ; CD14 ; HLA-DR mesurée par cytométrie de flux à l'aide des anticorps anti-HLA A, B et C-PC5 : clone G46-2.6, BD Biosciences ; anti-CD14-PE : clone RM052 et anti-HLA DR-FITC : clone Immu-357, Beckman-Coulter) (**Figure 14B**).

Ces modifications morphologique et phénotypique traduisent une activation des monocytes en culture en présence de GC1.

On observe, par exemple, induction de l'augmentation des capacités phagocytaires des monocytes, mise en évidence par exemple par internalisation de bactéries *(Mycobacterium bovis BCG)* rendues fluorescentes par transfection d'un plasmide codant la Green Fluorescent Protein (GFP) (infection des monocytes avec une Multiplicité d'Infection de 200 bactéries pour un monocytes). L'intemalisation est quantifiée par cytométrie de flux (détection de la GFP). L'activation des monocytes est également montrée par une augmentation de la translocation nucléaire du facteur de transcription NF-κB en présence du dendrimère GC1 dans le milieu de culture. Les extraits nucléaires sont préparés avec le kit « Nuclear Extract » (Active Motif), la concentration protéique des extraits est mesurée avec le kit « Micro BCA Protein Assay Reagent Kit » (Pierce Biotechnology), la quantification de NF-κB dans les extraits nucléaires protéiques se fait avec le kit « TransAM NF-κB p50 Chemi » (Active Motif) par luminométrie (Mithras LB940, Berthold Biotechnologies) (**Figure 14C**).

Cette activation des monocytes en culture avec le dendrimère GC1 a finalement pour conséquence un maintien des monocytes en culture : la numération des monocytes en culture avec le dendrimère GC1 est toujours supérieure à celle des monocytes en culture sans le dendrimère GC1. Ce maintien des monocytes en culture sous l'effet de GC1 corrèle avec la diminution du pourcentage de monocytes en apoptose dans la culture (mesurée par le pourcentage de cellules positives à l'annexine V en cytométrie de flux, «Annexin V-FITC Detection Kit I », BD Biosciences) (**Figure 14D**). Le dendrimère GC1 a un effet anti-apoptotique sur les monocytes humains en culture *in vitro.*

**Phénotype des cellules amplifiées par culture avec le dendrimère GC1 (Figures 1A-1G)**

Lors de la mise en culture (t0) puis après deux semaines de culture avec le dendrimère GC1, le phénotypage des cellules est réalisé par marquage immunologique et révélé par cytométrie en flux (cytomètre XL Epics, Beckman-Coulter-Immunotech, Marseille, FRANCE). Dans les cultures avec le dendrimère GC1 on observe avec tous les donneurs testés que les cellules en culture deviennent majoritairement des cellules NK, lesquelles ne représentent qu'une minorité au début de la culture. L'exemple présenté comporte 23% de cellules NK au départ et 76% de cellules NK après deux semaines de culture *in vitro* avec GC1. Ces cellules sont identifiées par leur phénotype $CD3\text{-}CD16^{+}CD56^{+}$ (anticorps anti-CD3-FITC/anti-CD56-PC5 : clones UCHT1/NKH-1, anti-CD16-PE : clone 3G8, Beckman-Coulter-Immunotech, Marseille, FRANCE). Leur identification est confirmée par la présence des NCR NKp30 et NKp44 et des NKR NKG2D et CD85j (anticorps anti-NKp30 : clone Z25, anti-NKp44-PE : clone Z231, anti-NKG2D : clone ON72, anti-CD85j (ILT2) : clone HPF1, Beckman-Coulter-Immunotech, Marseille, FRANCE), divers marqueurs spécifiques des cellules NK humaines. Par ailleurs, ces cellules NK amplifiées avec GC1 expriment de manière inattendue le Toll-like receptor - 2 (TLR2) détecté par cytométrie de flux avec un anticorps anti-TLR2-PE (clone TL2.1, BioLegend, San Diego, Californie, USA).

**Compositions lymphocytaires après quinze jours de culture *in vitro* de PBMC avec le dendrimère GC1 (Figures 2A-2D)**

Les PBMC obtenues d'un donneur humain adulte sain sont mises en culture 15 jours avec GC1 puis dénombrées par comptage total des cellules vivantes ($N_{tot}$). La population totale de chaque culture est composée de diverses sous-populations identifiées par cytométrie de flux : on détecte seulement des cellules NK, les lymphocytes T $\gamma\delta$ et $T\alpha\beta CD8^{+}$ (anticorps anti-TCR-V$\gamma$9-FITC: clone IMMU 360, anti-CD8-PE : clone B9.11, Beckman-Coulter-Immunotech, Marseille, FRANCE), en proportions variables selon les donneurs. Le nombre de cellules de chaque sous-type est obtenu par le calcul suivant :

[ nombre de cellules de chaque sous-population = $N_{tot}$ x % de la sous-population dans cette culture ]

Les comptages des sous-populations constituant les lignées cellulaires obtenues avec GC1 sont comparés aux mêmes comptages réalisés au départ de la culture (J0) et dans les cultures réalisées dans les mêmes conditions sans le dendrimère GC1. A partir des cellules provenant de tous les donneurs testés, la culture lymphocytaire avec GC1 favorise la prolifération des cellules NK vivantes.

Ces résultats démontrent que le dendrimère GC1 présente des propriétés immuno-stimulantes générales pour les lymphocytes humains.

**Amplification de cellules NK à partir de PBMC cultivés *in vitro* avec le dendrimère GC1 (Figure 3)**

Les cellules NK obtenues à partir de PBMC de donneurs sains mis en culture dans les conditions décrites auparavant sont comptées, au départ de l'expérience puis au bout de 3,5 semaines de culture. L'amplification du nombre de cellules NK est comparée à celle des cellules NK obtenues à partir de PBMC mis en culture dans les mêmes conditions mais sans le dendrimère GC1 (chaque point représente un donneur différent). Ces résultats démontrent que le dendrimère GC1 présente des propriétés immuno-stimulantes pour les cellules NK humaines.

**Amplification de cellules NK par culture *in vitro* avec le dendrimère GC1 à partir de PBMC de patients cancéreux (Figure 11)**

Par ailleurs, des résultats semblables ont été obtenus à partir de PBMC de patients cancéreux atteints de Myelome Multiple mis en culture dans les conditions décrites auparavant au bout de 2,5 semaines de culture. L'amplification du nombre de cellules NK en présence de GC1 (cercle plein) est comparée à celle obtenue à partir de PBMC mis en culture dans les mêmes conditions mais sans dendrimère (cercle vide) dans le cas de 14 donneurs sains et 14 patients cancéreux.

**Amplification de cellules NK à partir de PBMC cultivés *in vitro* avec le dendrimère GC1 en présence de différentes cytokines (Figures 12A, 12B)**

Les résultats obtenus ci-dessus ont été confirmés en combinant l'ajout de GC1 avec diverses cytokines. Ainsi, les cellules NK obtenues à partir de PBMC de donneurs sains ont été mises en culture avec GC1 en présence : d'IL2 (200 U/ml soit 8 ng/ml), d'IL15 (10 ng/ml), ou d'un mélange d'IL2 (200 U/ml soit 8 ng/ml) et d'IL15 : (10 ng/ml)).

Les résultats obtenus, représentatifs de 4 donneurs, démontrent que l'IL15 seule, comme l'IL2 seule, ou l'IL15 et l'IL2 associées permettent une amplification des NK en présence de GC1.

**Bioactivités de différents dendrimères sur cellules lymphoïdes, notamment NK humaines (Figures 4A-4B)**

La titration des concentrations actives de dendrimère GC1 testé sur l'amplification *in vitro* de cellules NK humaines dans une gamme de concentrations allant de 1 à 100 μM montre un optimum à 20 μM **(Figure 4A).** Les dendrimères portant les mêmes fonctions azabisphosphoniques en surface, mais de génération successives (GC0 < GC1 < GC2) présentent les mêmes propriétés immunostimulantes que GC1 **(Figure 4B),** notamment à la concentration 20 μM, sur l'amplification *in vitro* de cellules NK humaines (conditions de culture décrites auparavant). Le monomère phénol-azabisphosphonique dérivé de la tyramine est inactif dans les mêmes essais.

D'autres dendrimères, notamment ceux des **Exemples 5, 15, 26, 49, 71 et 76** ont été testés comparativement au dendrimère GC1 **(Exemple 36),** dans les conditions décrites auparavant, et au milieu de culture sans dendrimère (point 0 de la **Figure 13).** Le pourcentage et le nombre total de cellules NK obtenus après 2,5 semaines de culture ont été déterminés pour chacun des dendrimères testés **(Figure 13).**

**Fonctionnalité des cellules NK obtenues avec le dendrimère GC1 :**

**1. capacité intacte à établir des interactions entre cellules (trogocytose) (Figures 5A-5C)**

Des cellules lymphoïdes normales s'accrochent activement à la surface de leur cible (phénomène dénommé trogocytose) avant toute réponse effectrice. Les cellules NK obtenues *in vitro* grâce au dendrimère GC1 et marquées en intracellulaire au CMTMR (Molecular Probes, Eugene, Oregon, USA) (incuber 5 millions de cellules 30 minutes à 37°C dans 1 ml de milieu RPMI 1640 supplémenté additionné de 1 μl de CMTMR, puis rincer 5 fois avec 10 ml de milieu RPMI 1640 supplémenté) sont incubées avec des cellules cancéreuses cibles (lymphome B **(Figure 5A)** et carcinome colique **(Figure 5B))** ayant incorporé le marqueur membranaire fluorescent vert PKH 67 (Sigma-Aldrich, Saint-Louis, Missouri, USA) (incuber 5 millions de cellules 5 minutes à température ambiante dans 250 μl de diluant C Sigma-Aldrich additionné de PKH 67 au 1/500, puis ajouter 250 μl de SVF, laisser incuber 1 minute à température ambiante, puis rincer 3 fois avec 10 ml de milieu RPMI 1640 supplémenté). Au bout d'une heure d'incubation à 37°C en plaque multi-puits (96 puits à fond rond) (6 x $10^5$ cellules de chaque sorte dans 100 μl de RPMI 1640 supplémenté, avec 10% de SVF ; la plaque multi-puits est centrifugée 2 minutes à 800 tours/min) dans un incubateur à 37°C, à humidité constante et dans une atmosphère de 5% de $CO_2$ dans l'air, les cellules complexées sont séparées par lavage au PBS contenant 0,5 mM d'EDTA puis l'acquisition du marqueur PKH 67 (trogocytose ou transfert synaptique) par les cellules NK est mesurée par cytométrie de flux (FACSCalibur et CellQuest Software, BD Biosciences, Mountain View, Californie, USA) (mesure de la moyenne d'intensité de fluorescence verte de toutes les cellules NK au temps 0 puis au bout de 60 minutes de l'incubation). Les cellules NK obtenues avec le dendrimère GC1 ont une trogocytose fonctionnelle comme celle des cellules NK normales sur deux types distincts de cellules cancéreuses (lymphome B, carcinome).

Après le transfert synaptique, les cellules sont resuspendues délicatement, déposées sur une plaque de verre recouverte de poly-L-lysine (Sigma-Aldrich, Saint-Louis, Missouri, USA) pour une incubation de 5 minutes à 37°C. Après fixation par du PBS contenant 4% de para-formaldéhyde, les cellules sont lavées et recouverte de PBS contenant 90% de glycérol et 2% de 1-4-diazabicyclo (2.2.2) octane (DABCO, Sigma-Aldrich, Saint-Louis, Missouri, USA) et d'une lamelle. La photographie de microscopie confocale obtenue avec un appareil LSM410 (Zeiss, lena, ALLEMAGNE) (flèches, **Figure 5C)** montre la trogocytose de cellules cancéreuses vertes (cibles) par les cellules NK obtenues avec le dendrimère GC1 (cytoplasme en rouge) durant leur interaction.

Ces résultats démontrent que la culture avec le dendrimère GC1 n'affecte pas la capacité d'interaction des cellules NK obtenues.

**Fonctionnalité des cellules NK obtenues avec le dendrimère GC1 :**

**2. fonctionnalité des récepteurs activateurs NKp30 et NKG2D (lyse redirigée) (Figure 6A-6B)**

La fonctionnalité des principaux récepteurs activateurs de lyse exprimés à la surface des cellules NK produites avec le dendrimère GC1 est testée par le test classique de lyse redirigée. Brièvement, ce test consiste à déclencher l'activité tueuse des cellules NK au moyen d'anticorps spécifiques de ces récepteurs activateurs **(Figure 6A),** l'activité tueuse s'exerçant alors sur des cellules particulières : mastocytome murin P815 (cultivé en RPMI 1640, 25 mM Hépès / Ultraglutamine 1 supplémenté avec 1 mM pyruvate de sodium, 100 μg/ml streptomycine et 100 U/ml de pénicilline, 10% en volume de SVF) qui ne sont pas tuées si les cellules NK ne sont pas activées. Les cellules cibles P815 sont chargées en $^{51}Cr$ (sulfate de chrome-$^{51}Cr$ à 10 mCi/ml, ICN Biomedicals, Costa Mesa, Californie, USA) (2 millions de

cellules sont incubées avec 20 μl de sulfate de chrome, 1 h à 37°C, puis rincées 3 fois avec 1 ml de milieu de culture). Ensuite, 3000 cellules cibles chargées sont mises en présence, pendant 4 heures (dans un incubateur à 37°C, à humidité constante et dans une atmosphère de 5% de $CO_2$ dans l'air), des cellules NK humaines et des anticorps suivants : aucun anticorps, anticorps non spécifique : 4 μg/ml isotype contrôle (IgG1de souris : clone 679.1Mc7, Beckman-Coulter-Immunotech, Marseille, FRANCE), 4 μg/ml anti-NKG2D (clone ON72, Beckman-Coulter-Immunotech, Marseille, FRANCE), 4 μg/ml anti-NKp30 (clone AZ20, Innate Pharma, Marseille, FRANCE). L'incubation se fait en plaque multi-puits (96 puits à fond rond) dans un volume total de 200 μl, après avoir centrifugé 2 minutes à 800 tours/min.

Les rapports cellules effectrices versus cellules cibles (E:T) sont ici de 10 contre 1 (soit 30000 effectrices et 3000 cibles) et de 3 contre 1 (soit 9000 effectrices et 3000 cibles), respectivement.

On mesure dans chaque essai le relargage de $^{51}Cr$ provenant des cibles dans 100 μl de milieu de culture, en faisant la moyenne de 3 expériences identiques. Pour chaque condition expérimentale (anticorps utilisé) on mesure la radioactivité suivante (cpm) :

- le MR : quantité totale de $^{51}Cr$ incorporée par les cellules cibles seules ;
- le SR : relargage spontané de $^{51}Cr$ par les cibles seules ;
- X : relargage de $^{51}Cr$ mesuré dans le test.

On obtient le pourcentage de lyse spécifique dans chaque expérience à partir de la formule suivante :

$$[\text{ lyse spécifique } (\%) = (X - SR) / (MR - SR)\ ]$$

Les résultats présentés **(Figure 6B)** démontrent la fonctionnalité des récepteurs activateurs NKG2D et NKp30 exprimés par une lignée NK obtenue avec GC1.

**Fonctionnant des cellules NK obtenues avec le dendrimère GC1 :**

**3. contrôle inaltéré de leur activité cytolytique (Figures 7A-7C)**

Les cellules NK obtenues avec le dendrimère GC contrôlent normalement leur activité cytotoxique directe vis-à-vis de cellules cibles potentielles : elles ne lysent pas les lymphocytes autologues au repos (cellules d'haplotype CMH de classe 1 identique), mais lysent très efficacement les cibles classiques K562 et Daudi (cellules de leucémie myéloide chronique et lymphome de Burkitt, respectivement, toutes deux déficientes en molécules CMH de classe I). La lyse directe (sans rajout d'anticorps) des cellules cibles spécifiées (lymphome de Burkitt Daudi, LMC K562 ou PBMC autologues des NK) est mesurée comme dans le test précédent, avec des rapports E:T compris entre 30:1 et 0,2:1. Les résultats présentés démontrent que la culture avec le dendrimère GC1 n'affecte pas la capacité cytolytique des cellules NK obtenues.

**Fonctionnalité des cellules NK obtenues avec le dendrimère GC1 :**

**4. large spectre d'activité antitumorale (Figure 8)**

Les cellules NK obtenues avec GC1 exercent leur cytotoxicité vis-à-vis d'un large spectre de cellules cancéreuses cibles réparties en leucémies et carcinomes. La lyse des cellules cibles est mesurée comme décrit auparavant par relargage de $^{51}Cr$ avec des rapports E:T de 1:1 (barres blanches) et 10:1 (barres grises) et elle est exprimée en pourcentage de lyse spécifique. Ces résultats démontrent que la culture avec le dendrimère GC1 n'affecte pas la capacité cytolytique des cellules NK obtenues vis-à-vis d'un large spectre de cellules cancéreuses.

**Exemple 99 : Evaluation de la toxicité *in vivo* de dendrimères à surface aza-bis-phosphonique**

Une suspension de GC1 à 1 mg/ml a été réalisée en PBS apyrogène et la solution obtenue a été stérilisée par filtration sur une membrane de porosité 0,22 μm.

La suspension a été administrée par voie intraveineuse à raison de 0 (témoin), 10, 100 ou 1000 μg par animal tous les trois jours durant 120 jours respectivement à 4 groupes de 5 souris BALB/c.

Aucune toxicité n'a été observée.

**Exemple 100 : Utilisation de cellules NK amplifiées par des dendrimères à surface aza-bis-phosphonique pour le traitement des cancers**

Le modèle animal choisi est celui de souris immunodéficientes ayant reçu une xénogreffe de cellules tumorales humaines.

Des souris immunodéficientes, mâles et femelles, de plus de 3 mois, ont été élevées dans un environnement de stricte stérilité, en isolateur ventilé par de l'air filtré et stérilisé, à 22°C et 40 % d'humidité, sous un cycle jour-12h/nuit-12h. Les cages, biberons et eau ont été stérilisés en autoclave à 120 °C pendant 30 minutes et les aliments ainsi que les litières sont traitées par irradiation $\gamma$. Toutes les manipulations ont été réalisées aseptiquement sous une hotte à flux laminaire.

Les souris ont été soumises à une anesthésie générale par injection i.p. de 0,3 à 0,4 ml de hypnomidate à 2 mg/ml. 1 x $10^7$ cellules tumorales de leucémie K 562 en suspension dans 200 $\mu$l de PBS ont ensuite été injectées en sous-cutanée, dans le dos des souris.

Lorsque la tumeur atteint approximativement 1 cm de longueur (environ 2 à 3 semaines après l'injection), des cellules NK humaines obtenues à partir de PBMC humaines cultivées en présence du composé GC1 comme cela est décrit dans l'Exemple 49 ont été administrées par voie intraveineuse à proximité de la tumeur à raison de 0 (témoin), $10^4$, $10^6$ ou $10^8$ cellules par animal respectivement à 4 groupes de 5 souris. Un rappel a été effectué de 3 à 5 semaines après la première administration de cellules.

Le volume de la tumeur a été mesuré régulièrement.

Les premiers résultats obtenus indiquent que l'administration des cellules NK préparées à l'aide du composé GC1 est susceptible d'inhiber la croissance tumorale.

**Exemple 101 : Utilisation de dendrimères à surface aza-bis-phosphonique pour le traitement des cancers**

Le modèle animal choisi est celui de souris immunodéficientes ayant reçu une xénogreffe de cellules tumorales humaines.

Des souris immunodéficientes, mâles et femelles, de plus de 3 mois, ont été élevées dans un environnement de stricte stérilité, en isolateur ventilé par de l'air filtré et stérilisé, à 22°C et 40 % d'humidité, sous un cycle jour-12h/nuit-12h. Les cages, biberons et eau ont été stérilisés en autoclave à 120 °C pendant 30 minutes et les aliments ainsi que les litières sont traités par irradiation $\gamma$. Toutes les manipulations ont été réalisées aseptiquement sous une hotte à flux laminaire.

Les souris ont été soumises à une anesthésie générale par injection i.p. de 0,3 à 0,4 ml de hypnomidate à 2 mg/ml. 1x$10^7$cellules tumorales de leucémie K 562 en suspension dans 200 $\mu$l de PBS ont ensuite été injectées en sous-cutanée, dans le dos des souris. Au 10ème jour de greffe la longueur de la tumeur atteint environ 1 cm.

Une suspension de GC1 à 1 mg/ml a été réalisée en PBS apyrogène et la solution obtenue a été stérilisée par filtration sur une membrane de porosité 0,22 $\mu$m.

Au dixième jour suivant la greffe, la suspension a été administrée par voie intraveineuse à raison de 0 (témoin), 10, 100 ou 1000 $\mu$g par animal tous les trois jours durant 120 jours respectivement à 4 groupes de 5 souris.

Le volume de la tumeur a été mesuré régulièrement.

Les premiers résultats obtenus indiquent que l'administration de GC1 est susceptible d'inhiber la croissance tumorale.

**Exemple 102 : Utilisation de dendrimères à surface aza-bis-phosphonique fluorescents pour le marquage de lymphocytes**

Le dendrimère fluorescent de **l'Exemple 89** (GC1F) a été introduit dans une culture *in vitro* de lymphocytes totaux et l'ensemble a été incubé pendant 4 h, 24 h ou 15 jours. Les cellules ont ensuite été analysées par cytométrie en flux : les lymphocytes ont été sélectionnés sur la base de leur morphologie **(Figure 10A),** puis analysés pour leur fluorescence due au dendrimère fluorescent **(Figure 10B).**

La comparaison de la fluorescence de lymphocytes maintenus en présence d'IL2 seule, d'IL2 + GC1F, ou d'IL2 + GC1 (non fluorescent) **(Figure 10B)** indique une acquisition de la fluorescence liée aux dendrimères sur les lymphocytes après quinze jours de culture, alors qu'aucun signal n'était détecté après quatre heures de co-incubation, et qu'un très faible décalage était observable après vingt-quatre heures de co-incubation.

## Revendications

1. Utilisation de dendrimères à terminaisons monophosphoniques ou bisphosphoniques pour stimuler la croissance

de cultures cellulaires ou activer des cellules en culture.

**2.** Utilisation selon la revendication 1, dans laquelle les cellules sont des cellules hématopoïétiques.

**3.** Utilisation selon la revendication 1 ou 2, de dendrimères à terminaisons monophosphoniques ou bisphosphoniques pour la préparation de compositions cellulaires enrichies en cellules de la lignée lymphoïde exprimant le récepteur NKG2D, à partir d'échantillons biologiques.

**4.** Utilisation selon l'une des revendications 1 à 3, dans laquelle les dendrimères à terminaisons monophosphoniques ou bisphosphoniques sont de génération n et comprennent un noyau central § de valence m pouvant établir m-2 liaisons, sous réserve que m soit supérieur à 2, ou m-1 liaisons, sous réserve que m soit supérieur à 1, ou m liaisons avec des chaînes de liaison, de préférence identiques entre elles, m représentant un nombre entier de 1 à 20, en particulier de 1 à 10 et plus particulièrement de 1 à 8, et n représentant un nombre entier de 0 à 12, lesquelles chaînes de liaison sont constituées par :

• des chaînes de génération fixées en arborescence autour du noyau sur chacune des liaisons, lorsque n est supérieur ou égal à 1, une chaine de génération d'une génération donnée étant reliée à

- une chaîne de génération de la génération immédiatement inférieure à la génération donnée, ou au noyau lorsque la génération donnée vaut 1, et à
- au moins 2 chaînes de génération de la génération immédiatement supérieure à la génération donnée, ou éventuellement à au moins une chaîne intermédiaire lorsque la génération donnée vaut n,

un groupe terminal étant fixé à l'extrémité de chaque chaîne de génération de génération n, ou le cas échéant à l'extrémité de chaque chaîne intermédiaire, ou

• des chaînes intermédiaires fixées autour du noyau sur chacune des liaisons, lorsque n vaut 0, un groupe terminal étant fixé à l'extrémité de chaque chaîne intermédiaire ;
ledit groupe terminal étant représenté par la formule :

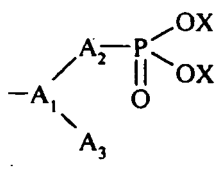

où

$A_1$ représente N ; un groupe P=Y, où Y représente O, S, ou aucun atome ; un groupe N-R ; ou un groupe C-R ; R représentant H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR'R'', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R' et R'' représentant indépendamment l'un de l'autre H ou un groupement alkyle de 1 à 16 atomes de carbone, aryle de 6 à 24 atomes de carbone ou aralkyle de 7 à 16 atomes de carbone ;

$A_2$ représente une liaison simple ou une chaîne hydrocarbonée de 1 à 6 chaînons, linéaire, ramifiée ou cyclique, chacun desdits chaînons contenant éventuellement un ou plusieurs hétéroatomes, lesdits hétéroatomes étant préférentiellement choisis parmi un atome de soufre, d'oxygène, de phosphore, ou d'azote, plus préférentiellement d'azote, et étant éventuellement substitués par au moins un substituant choisi parmi H, un groupement alkyle de 1 à 16 atomes de carbone, un halogène, un groupement $-NO_2$, un groupement -NRR', un groupement -CN, un groupement $-CF_3$, un groupement hydroxyle, un groupement alkoxy de 1 à 16 atomes de carbone, un groupement aryle ou hétéroaryle de 1 à 24 atomes de carbone, l'hétéroélément étant préférentiellement choisi parmi l'oxygène, l'azote ou le soufre, un groupement aralkyle de 7 à 16

atomes de carbone, R et R' représentant indépendamment l'un de l'autre H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone ;

$A_3$ représente H, ou une chaîne hydrocarbonée de 1 à 6 chaînons, linéaire, ramifiée ou cyclique, chacun desdits chaînons pouvant éventuellement être choisi parmi un hétéroatome, ledit hétéroatome étant préférentiellement choisi parmi le soufre, l'azote, le phosphore, ou le silicium, plus préférentiellement l'azote, chaque chaînon pouvant être éventuellement substitué par au moins un groupement choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone, ou aralkyle de 7 à 16 atomes de carbone ou

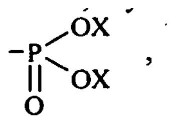

,

en particulier $A_3$ peut représenter

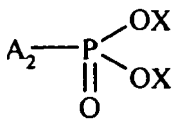

,

chacun des $A_2$ étant identiques ou différents ;

chaque OX, identique ou différent pour chaque groupement phosphonique, représente OH, OAlkyle, où le groupe alkyle comprend de 1 à 16 atomes de carbone, OAryle, où le groupe aryle comprend de 6 à 24 atomes de carbone, OAralkyle, où le groupe aralkyle comprend de 7 à 24 atome de carbone, OAlkylaryle, où le groupe alkylaryle comprend de 7 à 24 atomes de carbone, $OSiR'_1R'_2R'_3$, où $R'_1$, $R'_2$ et $R'_3$, identiques ou différents, représentent un groupement alkyle de 1 à 16 atomes de carbone, ou $O^-M^+$, où $M^+$ est un cation d'éléments du groupe IA, IB, IIA, IIB ou IIIA, IIIB de la classification périodique des éléments, de préférence $M^+$ est choisi parmi les cations des atomes de sodium, de potassium, de cuivre, de calcium, de baryum, de zinc, de magnésium, de lithium et d'aluminium, ou de groupes hydrocarbonés de 1 à 100 atomes de carbone, ou azotés de 0 à 100 atomes de carbone, tel que $NR_1R_2R_3R_4^+$, où, indépendamment les uns des autres $R_1$, $R_2$, $R_3$ et $R_4$ représentent H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone ;

ledit noyau central § représentant un groupe comprenant de 1 à 500 atomes, et contenant éventuellement un

ou plusieurs hétéroatomes, lesdits hétéroatomes étant préférentiellement choisis parmi l'oxygène, le soufre, l'azote, le phosphore ou le silicium.

**5.** Utilisation selon la revendication 4, dans laquelle le noyau établit m liaisons avec m chaînes de liaison identiques constituées :

• soit par des chaînes de génération fixées en arborescence autour du noyau sur chacune des liaisons, l'extrémité de chaque chaîne de la génération la plus éloignée du noyau central étant fixée soit à un groupe terminal soit à une chaîne intermédiaire, et l'extrémité de chaque chaîne intermédiaire étant fixée à un groupe terminal,
• soit par des chaînes intermédiaires fixées autour du noyau sur chacune des liaisons, l'extrémité de chaque chaîne intermédiaire étant fixée à un groupe terminal.

**6.** Utilisation selon la revendication 4, dans laquelle le noyau établit m-2 ou m-1 liaisons, m représentant un entier de 3 à 20, en particulier de 3 à 10 et plus particulièrement de 3 à 8, avec respectivement m-2 ou m-1 chaînes de liaison identiques constituées :

• soit par des chaînes de génération fixées en arborescence autour du noyau sur chacune des liaisons, l'extrémité de chaque chaîne de la génération la plus éloignée du noyau central étant fixée soit à un groupe terminal soit à une chaîne intermédiaire, et l'extrémité de chaque chaîne intermédiaire étant fixée à un groupe terminal,
• soit par des chaînes intermédiaires fixées autour du noyau sur chacune des liaisons,
l'extrémité de chaque chaîne intermédiaire étant fixée à un groupe terminal ;

les 1 ou 2 liaisons restantes étant fixées à des groupes de liaison, identiques ou différents, éventuellement liés ensemble, notamment par l'intermédiaire d'une liaison covalente, constitués :

- soit d'une partie des chaînes de liaison définies ci-dessus,
- soit d'un atome d'hydrogène,
- soit de groupes hydrocarbonés comprenant de 1 à 500 atomes de carbone, lesdits groupes hydrocarbonés étant notamment constitués de H ou d'une chaîne hydrocarbonée de 1 à 200 chaînons, linéaire, ramifiée ou cyclique, contenant éventuellement une ou plusieurs doubles ou triples liaisons, chacun desdits chaînons pouvant éventuellement être choisi parmi un hétéroatome, ledit hétéroatome étant préférentiellement choisi parmi un atome d'azote, d'oxygène, de phosphore, de silicium ou de soufre, un groupement aryle de 6 à 24 atomes de carbone, un groupement hétéroaryle de 1 à 24 atomes de carbone, un groupement carboxyle, un groupement >C=NR, chaque chaînon pouvant être éventuellement substitué par au moins un substituant choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement - $CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone, le premier chaînon de ladite chaîne hydrocarbonée étant de préférence un oxygène ou un azote.

**7.** Utilisation selon l'une des revendications 4 à 6, dans laquelle les chaînes de génération sont choisies parmi toute chaîne hydrocarbonée de 1 à 12 chaînons, linéaire, ramifiée ou cyclique, contenant éventuellement une ou plusieurs doubles ou triples liaisons, chacun desdits chaînons pouvant éventuellement être choisi parmi un hétéroatome, ledit hétéroatome étant préférentiellement choisi parmi l'azote, l'oxygène, le soufre, le phosphore ou le silicium, un groupement aryle de 6 à 24 atomes de carbone, un groupement hétéroaryle de 1 à 24 atomes de carbone, l'hétéroélément étant préférentiellement choisi parmi l'oxygène, l'azote ou le soufre, un groupement carboxyle, un groupement >C=NR, chaque chaînon pouvant éventuellement être substitué par au moins un substituant choisi parmi un groupement alkyle de 1 à 16 atomes de carbone, un atome d'halogène, un groupement $-NO_2$, un groupement - NRR', un groupement -CN, un groupement $-CF_3$, un groupement hydroxyle, un groupement alkoxy de 1 à 16 atomes de carbone, un groupement aryle de 6 à 24 atomes de carbone, ou un groupement aralkyle de 7 à 16 atomes de carbone, R et R' représentant indépendamment l'un de l'autre H ou un groupement alkyle de 1 à 16 atomes de carbone, aryle de 6 à 24 atomes de carbone, ou aralkyle de 7 à 16 atomes de carbone.

**8.** Utilisation selon l'une des revendications 4 à 7, dans laquelle les chaînes intermédiaires sont choisies parmi les groupes correspondant à la formule :

-J-K-L-

où

- J représente un atome d'oxygène, de soufre, ou un groupement -NR- ;
- K représente un groupement aryle de 6 à 24 atomes de carbone, hétéroaryle de 1 à 24 atomes de carbone, l'hétéroélément étant préférentiellement choisi parmi l'oxygène, l'azote ou le soufre, alkyle de 1 à 16 atomes de carbone, chacun pouvant être éventuellement substitué par un atome d'halogène ou un groupement $-NO_2$, -NRR', - CN, $-CF_3$, -OH, alkyle de 1 à 16 atomes de carbone, aryle de 6 à 24 atomes de carbone, aralkyle de 7 à 16 atomes de carbone ;
- L représente une chaîne hydrocarbonée de 0 à 10 chaînons, linéaire, ramifiée ou cyclique, contenant éventuellement une ou plusieurs doubles ou triples liaisons, chacun desdits chaînons pouvant éventuellement être un hétéroatome, ledit hétéroatome étant préférentiellement choisi parmi l'oxygène, le soufre, l'azote, le phosphore, le silicium chaque chaînon pouvant être éventuellement substitué par au moins un substituant choisi parmi un groupement alkyle de 1 à 16 atomes de carbone, un halogène, un atome d'oxygène, $-NO_2$, -NRR', -CN, $-CF_3$, -OH, un groupement alkoxy de 1 à 16 atomes de carbone, un groupement aryle de 6 à 24 atomes de carbone, un groupement aralkyle de 7 à 16 atomes de carbone ;

R et R' représentant indépendamment l'un de l'autre H ou un groupement alkyle de 1 à 16 atomes de carbone, aryle de 6 à 24 atomes de carbone, ou aralkyle de 7 à 16 atomes de carbone.

**9.** Utilisation selon l'une des revendications 4 à 8, dans laquelle le noyau est choisi parmi :

- un atome d'azote ou de silicium ;
- un groupement de formule

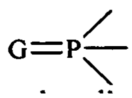

,

dans lequel G représente un atome d'oxygène, d'azote, de soufre, de sélénium, de tellure ou un groupement =NR, R représentant H ou un groupement alkyle de 1 à 16 atomes de carbone, aryle de 6 à 24 atomes de carbone, ou aralkyle de 7 à 16 atomes de carbone, tel que le groupement thiophosphoryle de formule

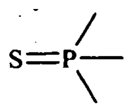

;

- un groupement bis-phényloxy de formule

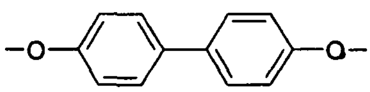

- un groupement 1,2-diamino-éthane de formule

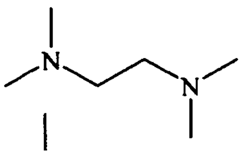

,

- un groupement 1,4-diamino-butane de formule

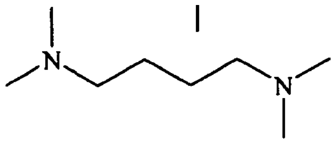,

- un groupement cyclotriphosphazène de formule

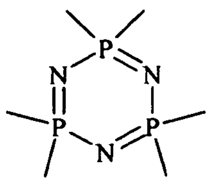,

également notée $N_3P_3$ ou $P_3N_3$,
- un groupement cyclotétraphosphazène de formule

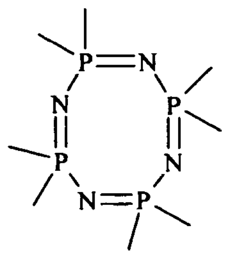,

également notée $N_4P_4$ ou $P_4N_4$,

**10.** Utilisation selon l'une des revendications 1 à 9, de dendrimères de structure PAMAM, DAB ou PMMH.

**11.** Utilisation selon l'une des revendications 4 à 5 et 7 à 9, de dendrimères à terminaisons monophosphoniques ou bisphosphoniques correspondant à la formule générale (1a) suivante :

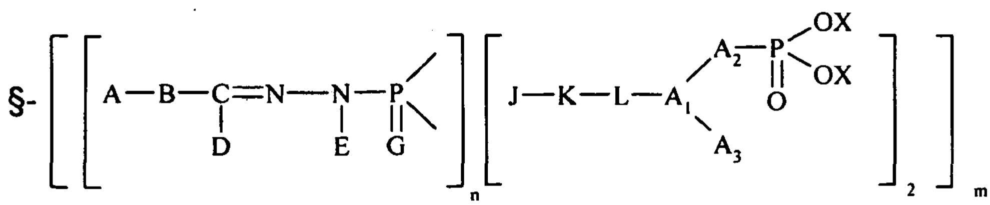

**(1a)**

où n représente un entier de 0 à 3, à savoir :

- lorsque n = 0, la formule (1a) correspond à la formule (2a) suivante,

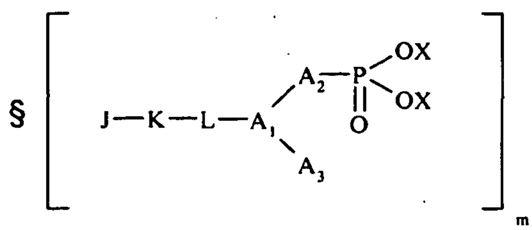

**(2a)**

- lorsque n = 1, la formule (1a) correspond à la formule (3a) suivante,

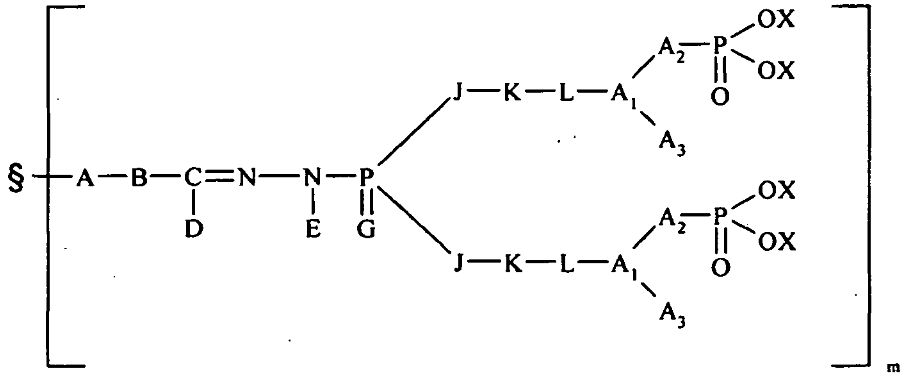

(3a)

- lorsque n = 2, la formule (1a) correspond à la formule (4a) suivante,

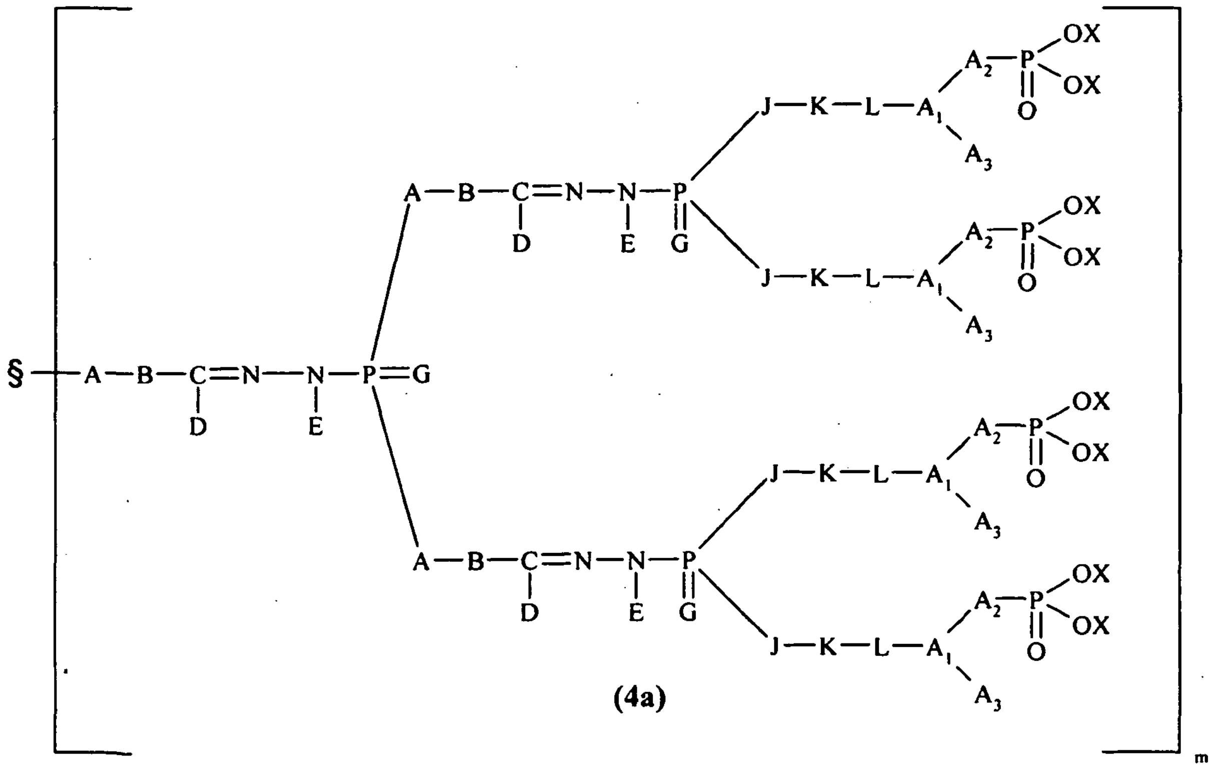

(4a)

- et lorsque n = 3, la formule (1a) correspond à la formule (5a) suivante,

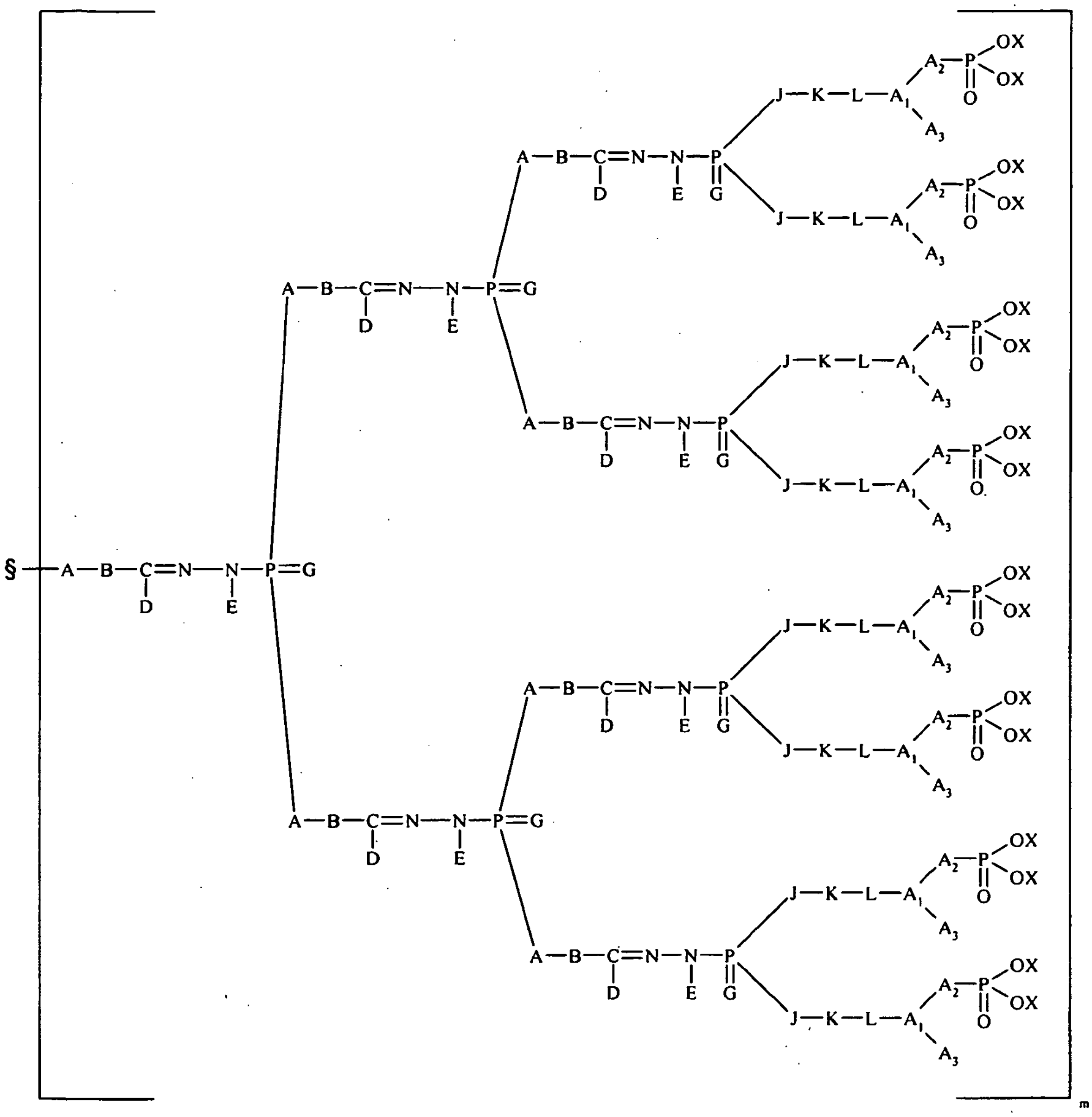

**(5a)**

et dans lesquelles formules :

- le noyau central § est choisi parmi les groupes suivants :

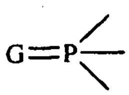 , 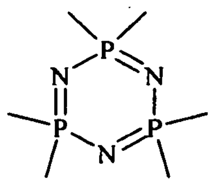 , 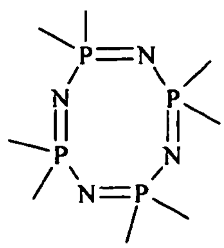 ;

• m représente 3, 6 ou 8 ;
• la chaîne de génération correspond à la formule :

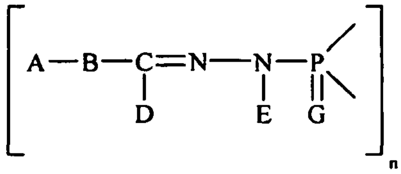

où
- A représente un atome d'oxygène, de soufre, de phosphore ou un groupement -NR-;
- B représente un groupement aryle de 6 à 24 atomes de carbone, hétéroaryle de 1 à 24 atomes de carbone, alkyle de 1 à 16 atomes de carbone, chacun pouvant être éventuellement substitué par un atome d'halogène ou un groupement $-NO_2$, -NRR', - CN, $-CF_3$, -OH, alkyle de 1 à 16 atomes de carbone, aryle de 6 à 24 atomes de carbone, aralkyle de 7 à 16 atomes de carbone ;
- D représente un atome d'hydrogène, un groupement alkyle de 1 à 16 atomes de carbone, alkoxy de 1 à 16 atomes de carbone, aryle de 6 à 24 atomes de carbone, aralkyle de 7 à 16 atomes de carbone, chacun pouvant être éventuellement substitué par un atome d'halogène ou un groupement $-NO_2$, -NRR', -CN, $-CF_3$, -OH, alkyle de 1 à 16 atomes de carbone, aryle de 6 à 24 atomes de carbone, aralkyle de 7 à 16 atomes de carbone ;
- E représente un atome d'hydrogène, un groupement alkyle de 1 à 16 atomes de carbone, alkoxy de 1 à 16 atomes de carbone, aryle de 6 à 24 atomes de carbone, aralkyle de 7 à 16 atomes de carbone, chacun pouvant être éventuellement substitué par un atome d'halogène ou un groupement $-NO_2$, -NRR', -CN, $-CF_3$, -OH, alkyle de 1 à 16 atomes de carbone, aryle de 6 à 24 atomes de carbone, aralkyle de 7 à 16 atomes de carbone ;
- G représente un atome d'oxygène, d'azote, de soufre, de sélénium, de tellure ou un groupement =NR ;
R et R' représentant indépendamment l'un de l'autre H ou un groupement alkyle de 1 à 16 atomes de carbone, aryle de 6 à 24 atomes de carbone, ou aralkyle de 7 à 16 atomes de carbone ;

• la chaîne intermédiaire correspond à la formule :

-J-K-L-

où

- J représente un atome d'oxygène, de soufre, ou un groupement -NR- ;
- K représente un groupement aryle de 6 à 24 atomes de carbone, hétéroaryle de 1 à 24 atomes de carbone, l'hétéroélément étant préférentiellement choisi parmi l'oxygène, l'azote ou le soufre, alkyle de 1 à 16 atomes de carbone, chacun pouvant être éventuellement substitué par un atome d'halogène ou un groupement $-NO_2$, -NRR', - CN, $-CF_3$, -OH, alkyle de 1 à 16 atomes de carbone, aryle de 6 à 24 atomes de carbone, aralkyle de 7 à 16 atomes de carbone ;
- L représente une chaîne hydrocarbonée de 0 à 10 chaînons, linéaire, ramifiée ou cyclique, contenant éventuellement une ou plusieurs doubles ou triples liaisons, chacun desdits chaînons pouvant éventuellement être un hétéroatome, ledit hétéroatome étant préférentiellement choisi parmi l'oxygène, le soufre, l'azote, le phosphore, le silicium chaque chaînon pouvant être éventuellement substitué par au moins un substituant choisi parmi un groupement alkyle de 1 à 16 atomes de carbone, un halogène, un atome d'oxygène, $-NO_2$, -NRR', -CN, $-CF_3$, -OH, un groupement alkoxy de 1 à 16 atomes de carbone, un groupement aryle de 6 à 24 atomes de carbone, un groupement aralkyle de 7 à 16 atomes de carbone ;
R et R' représentant indépendamment l'un de l'autre H ou un groupement alkyle de 1 à 16 atomes de carbone, aryle de 6 à 24 atomes de carbone, ou aralkyle de 7 à 16 atomes de carbone ;

• le groupe terminal correspond à la formule :

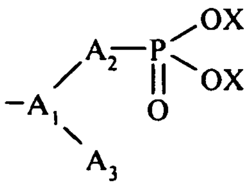

où $A_1$, $A_3$ et X ont été définis précédemment, chacun des X étant identique ou différent.

**12.** Utilisation selon la revendication 11, d'un dendrimère de formule générale (1a) dans laquelle $A_3$ représente :

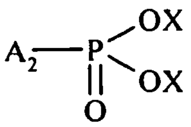

ladite formule générale (1a) correspondant alors à la formule générale (1) suivante :

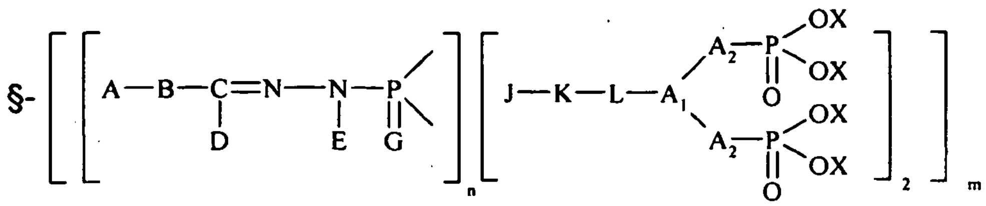

(1)

§, A, B, C, D, E, G, J, K, L, $A_1$, $A_2$ et X étant tels que définis dans la revendication 11.

**13.** Utilisation selon la revendication 12, d'un dendrimère de formule générale (1) de structure PMMH, dans laquelle

§ représente

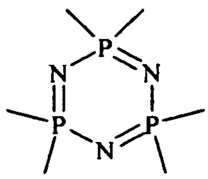

;

m représente 6 ;
n représente 0, 1, ou 2 ;
A représente un atome d'oxygène ;
B représente un groupement benzénique ;
D représente un hydrogène ;
E représente un groupement méthyle ;
G représente un atome de soufre ;
J représente un atome d'oxygène ;
K représente un groupement benzénique ;
L représente une chaîne hydrocarbonée saturée linéaire non substituée à deux atomes de carbone ;
$A_1$ représente un atome d'azote ;
$A_2$ représente un groupement $CH_2$ ;

X représente un groupement méthyle, ou un atome d'hydrogène ou de sodium ;

ledit dendrimère étant désigné GCn, n étant défini ci-dessus.

**14.** Utilisation selon l'une des revendications 4, 5 et 7 à 13, des composés de formules suivantes :

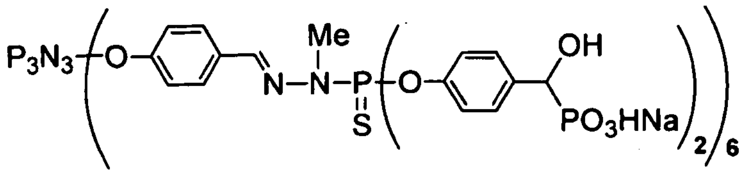

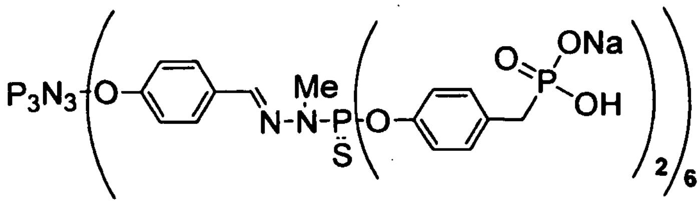

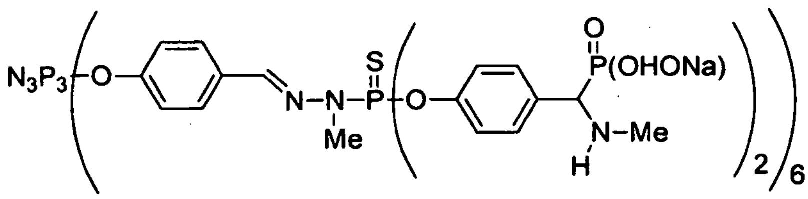

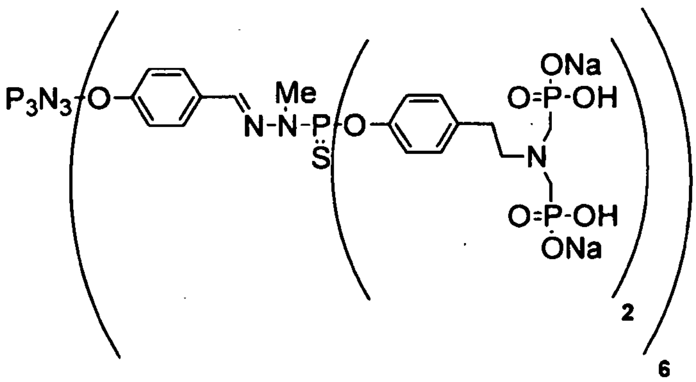

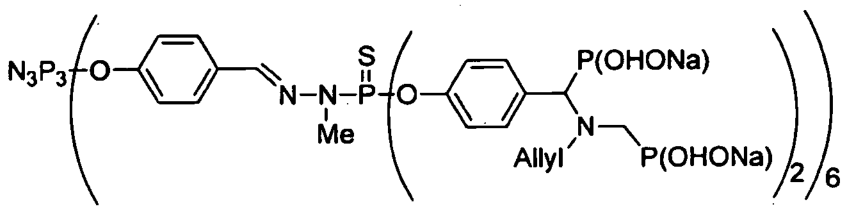

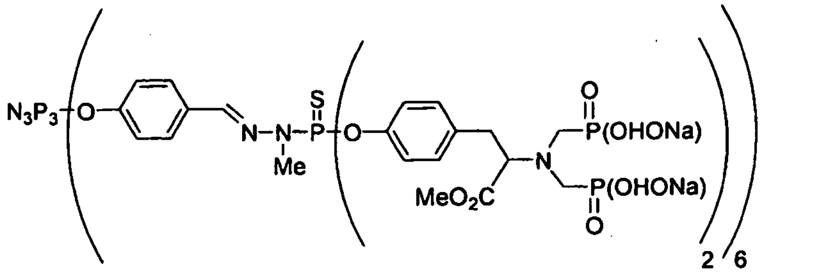

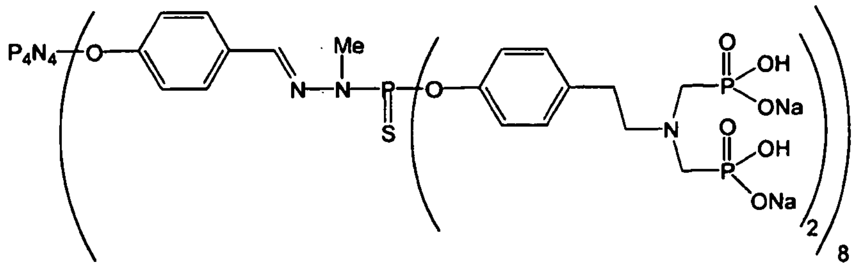

ou de formules GC 1 suivantes :

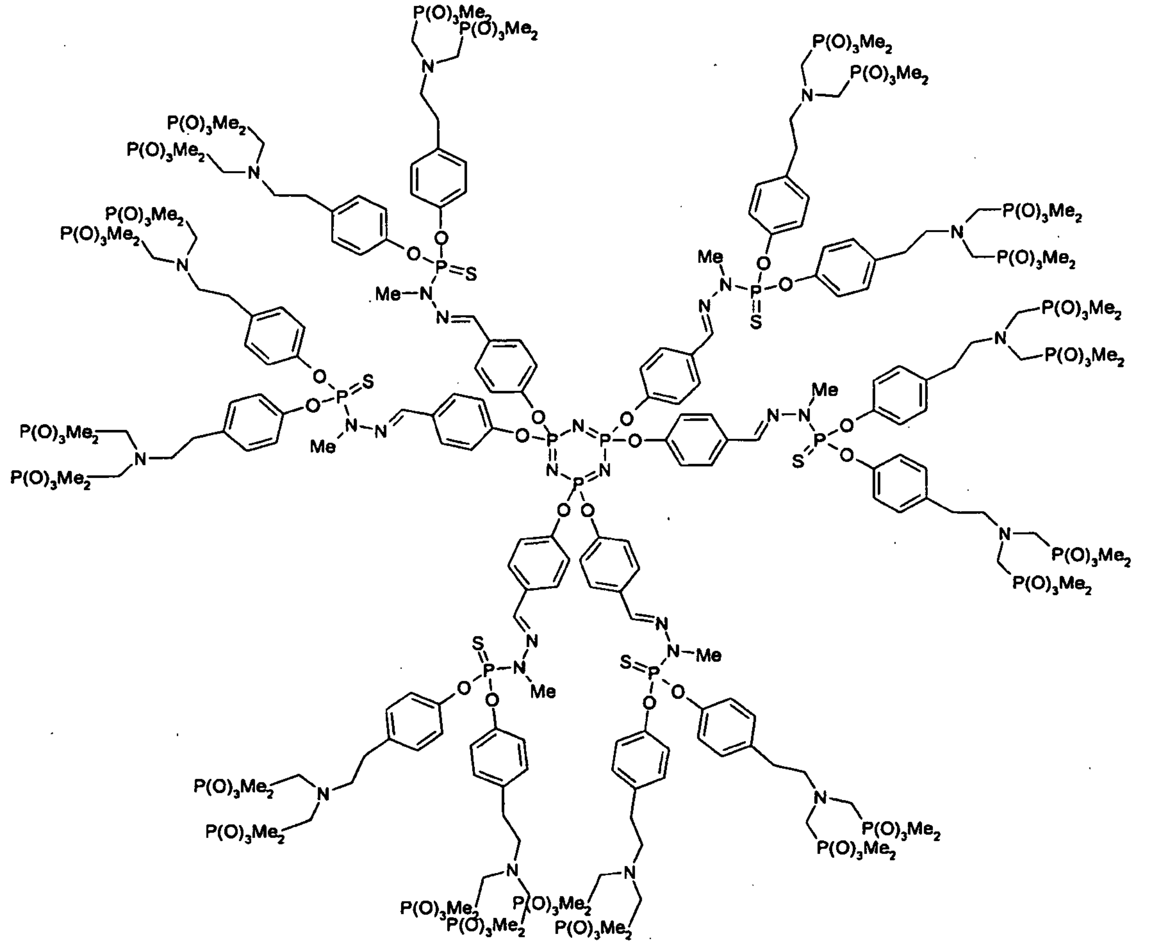

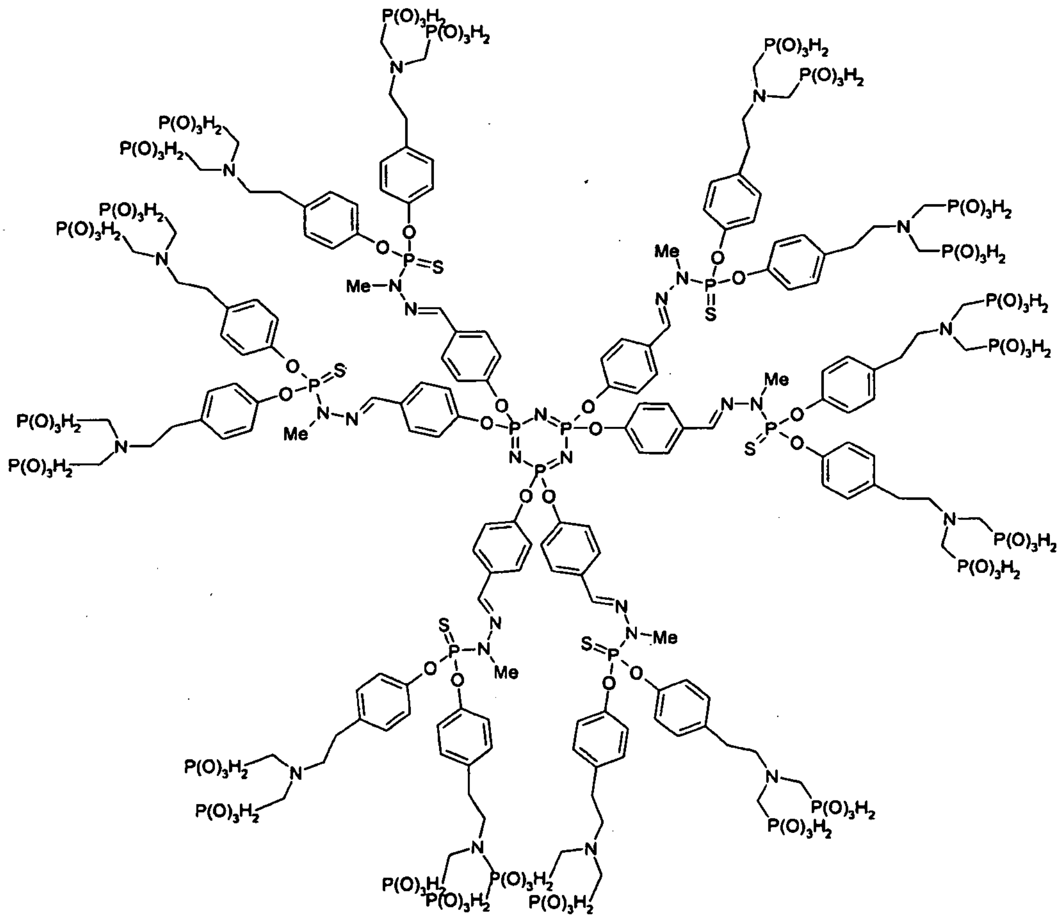

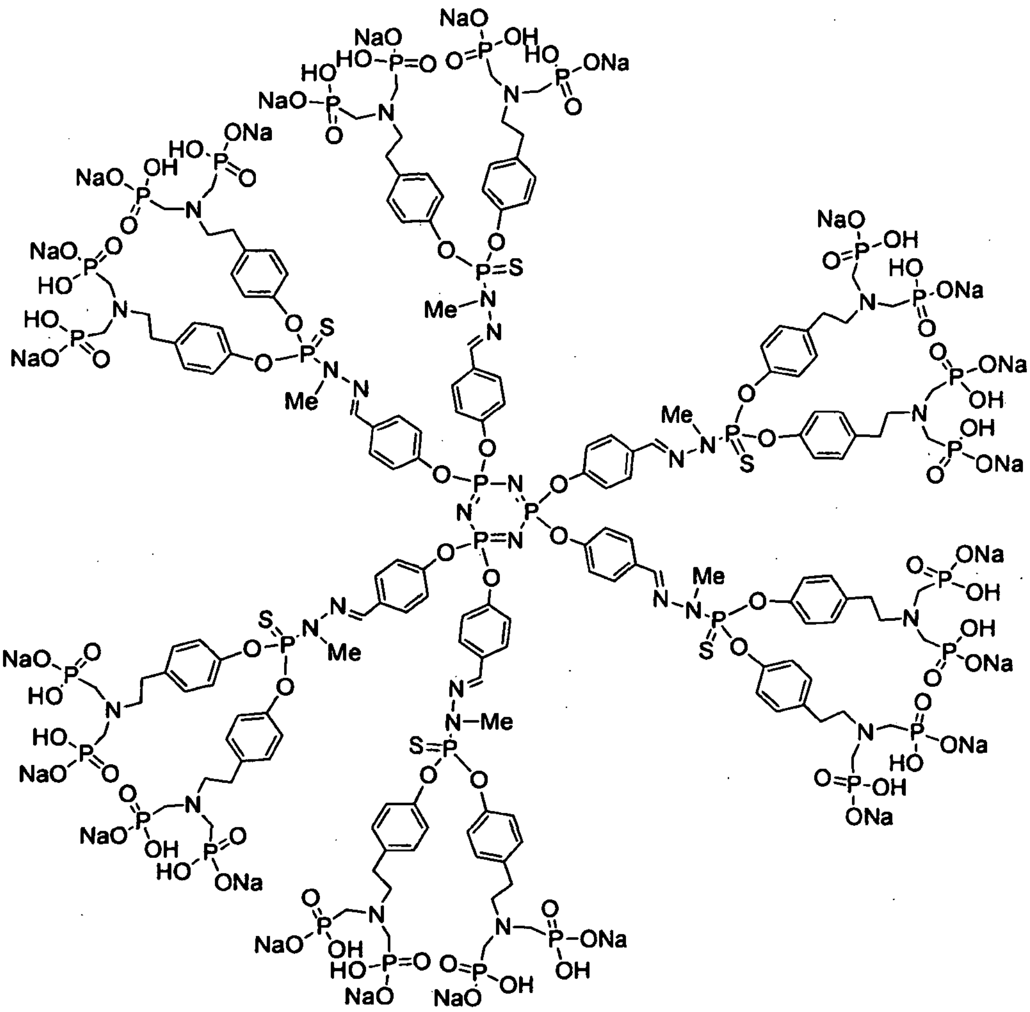

**15.** Utilisation selon l'une des revendications 4 et 6 à 9, de dendrimères à terminaisons bisphosphoniques correspondant à la formule générale (7) suivante :

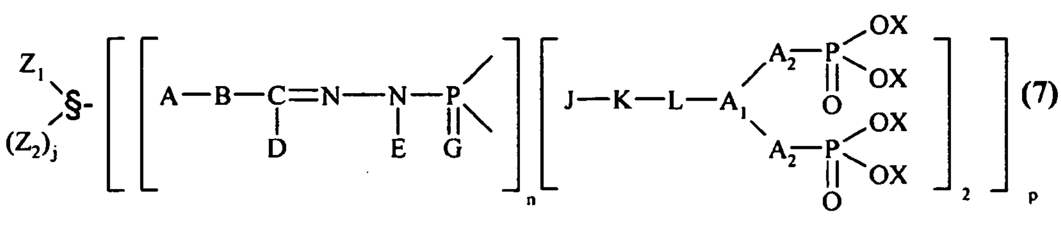

(7)

où n représente un entier de 0 à 3, m représente 3, 6 ou 8, p représente m - 1 ou m - 2, et j représente 0 lorsque p représente m - 1 et 1 lorsque p représente m - 2, à savoir :

- lorsque p = m - 1, la formule (7) correspond à la formule (8) suivante :

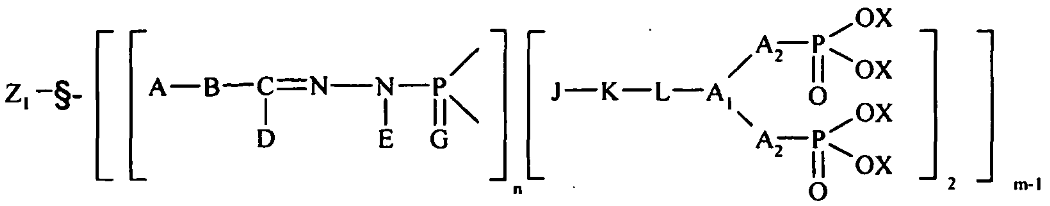

**(8)**

- lorsque p = m - 2, la formule (7) correspond à la formule (9) suivante

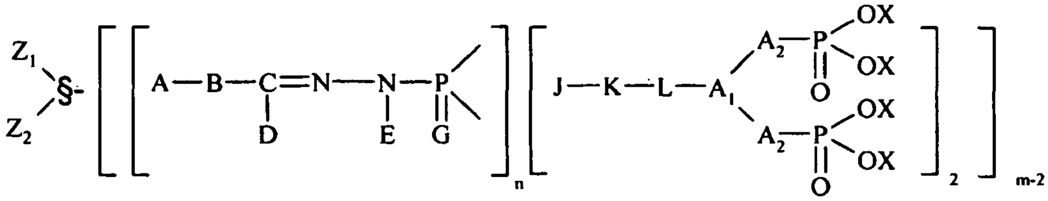

**(9)**

et dans lesquelles formules :

- le noyau central § est choisi parmi les groupes suivants :

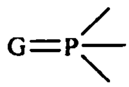

, 

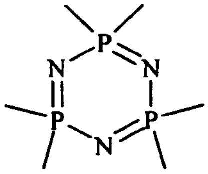

, 

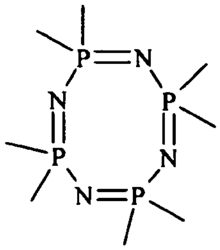

;

- la chaîne de génération correspond à la formule :

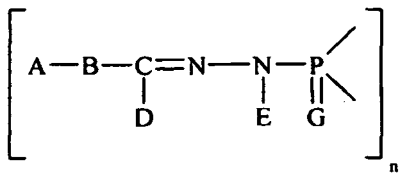

où
- A représente un atome d'oxygène, de soufre, de phosphore ou un groupement -NR-;
- B représente un groupement aryle de 6 à 24 atomes de carbone, hétéroaryle de 1 à 24 atomes de carbone, alkyle de 1 à 16 atomes de carbone, chacun pouvant être éventuellement substitué par un atome d'halogène ou un groupement $-NO_2$, -NRR', - CN, $-CF_3$, -OH, alkyle de 1 à 16 atomes de carbone, aryle de 6 à 24 atomes de carbone, aralkyle de 7 à 16 atomes de carbone ;
- D représente un atome d'hydrogène, un groupement alkyle de 1 à 16 atomes de carbone, alkoxy de 1 à 16 atomes de carbone, aryle de 6 à 24 atomes de carbone, aralkyle de 7 à 16 atomes de carbone, chacun pouvant être éventuellement substitué par un atome d'halogène ou un groupement $-NO_2$, -NRR', -CN, $-CF_3$, -OH, alkyle de 1 à 16 atomes de carbone, aryle de 6 à 24 atomes de carbone, aralkyle de 7 à 16 atomes de carbone ;
- E représente un atome d'hydrogène, un groupement alkyle de 1 à 16 atomes de carbone, alkoxy de 1 à 16 atomes de carbone, aryle de 6 à 24 atomes de carbone, aralkyle de 7 à 16 atomes de carbone, chacun pouvant être éventuellement substitué par un atome d'halogène ou un groupement $-NO_2$, -NRR', -CN, $-CF_3$, -OH, alkyle de 1 à 16 atomes de carbone, aryle de 6 à 24 atomes de carbone, aralkyle de 7 à 16 atomes de carbone ;

- G représente un atome d'oxygène, d'azote, de soufre, de sélénium, de tellure ou un groupement =NR ; R et R' représentant indépendamment l'un de l'autre H ou un groupement alkyle de 1 à 16 atomes de carbone, aryle de 6 à 24 atomes de carbone, ou aralkyle de 7 à 16 atomes de carbone ;

•la chaîne intermédiaire correspond à la formule :

-J-K-L-

où
- J représente un atome d'oxygène, de soufre, ou un groupement -NR- ;
- K représente un groupement aryle de 6 à 24 atomes de carbone, hétéroaryle de à 24 atomes de carbone, l'hétéroélément étant préférentiellement choisi parmi l'oxygène, l'azote ou le soufre, alkyle de 1 à 16 atomes de carbone, chacun pouvant être éventuellement substitué par un atome d'halogène ou un groupement $-NO_2$, -NRR', - CN, $-CF_3$, -OH, alkyle de 1 à 16 atomes de carbone, aryle de 6 à 24 atomes de carbone, aralkyle de 7 à 16 atomes de carbone ;
- L représente une chaîne hydrocarbonée de 0 à 10 chaînons, linéaire, ramifiée ou cyclique, contenant éventuellement une ou plusieurs doubles ou triples liaisons, chacun desdits chaînons pouvant éventuellement être un hétéroatome, ledit hétéroatome étant préférentiellement choisi parmi l'oxygène, le soufre, l'azote, le phosphore, le silicium, chaque chaînon pouvant être éventuellement substitué par au moins un substituant choisi parmi un groupement alkyle de 1 à 16 atomes de carbone, un halogène, un atome d'oxygène, $-NO_2$, -NRR', -CN, $-CF_3$, -OH, un groupement alkoxy de 1 à 16 atomes de carbone, un groupement aryle de 6 à 24 atomes de carbone, un groupement aralkyle de 7 à 16 atomes de carbone ;
R et R' représentant indépendamment l'un de l'autre H ou un groupement alkyle de 1 à 16 atomes de carbone, aryle de 6 à 24 atomes de carbone, ou aralkyle de 7 à 16 atomes de carbone ;

• le groupe terminal correspond à la formule :

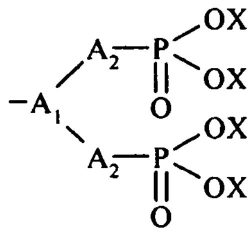

où $A_1$, $A_2$ et X ont été définis précédemment, chacun des X étant identique ou différent ;

$Z_1$ et $Z_2$ étant identiques ou différents, éventuellement liés ensemble, notamment par l'intermédiaire d'une liaison covalente, et représentant H ou une chaîne hydrocarbonée de 1 à 16 chaînons, linéaire, ramifiée ou cyclique, contenant éventuellement une ou plusieurs doubles ou triples liaisons, chacun desdits chaînons pouvant éventuellement être choisi parmi un hétéroatome, ledit hétéroatome étant préférentiellement choisi parmi un atome d'azote, d'oxygène, de phosphore, de silicium ou de soufre, un groupement aryle de 6 à 24 atomes de carbone, un groupement hétéroaryle de 1 à 24 atomes de carbone, un groupement carboxyle, un groupement >C=NR, chaque chaînon pouvant être éventuellement substitué par au moins un substituant choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement - $CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone, le premier chaînon de ladite chaîne hydrocarbonée étant de préférence un oxygène ou un azote.

**16.** Utilisation selon la revendication 15, d'un dendrimère de formule générale (8) de structure PMMH, dans laquelle

§ représente

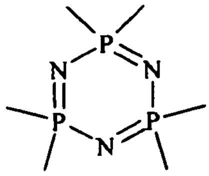 ;

m représente 6 ;
p représente 5 ;
n représente 0, 1, ou 2 ;
A représente un atome d'oxygène ;
B représente un groupement benzénique ;
D représente un hydrogène ;
E représente un groupement méthyle ;
G représente un atome de soufre ;
J représente un atome d'oxygène ;
K représente un groupement benzénique ;
L représente une chaîne hydrocarbonée saturée linéaire non substituée à deux atomes de carbone ;
$A_1$ représente un atome d'azote ;
$A_2$ représente un groupement $CH_2$ ;
X représente un groupement méthyle, ou un atome d'hydrogène ou de sodium ;
$Z_1$ représente un groupement phényloxy ;

ledit dendrimère étant désigné GCn', n étant défini ci-dessus.

**17.** Utilisation selon l'une des revendications 4, 6 à 9 ou 15 à 16,

- des composés de formules suivantes :

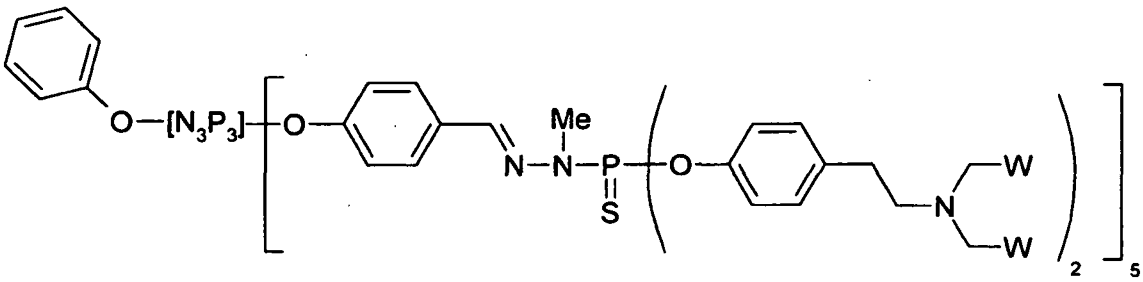

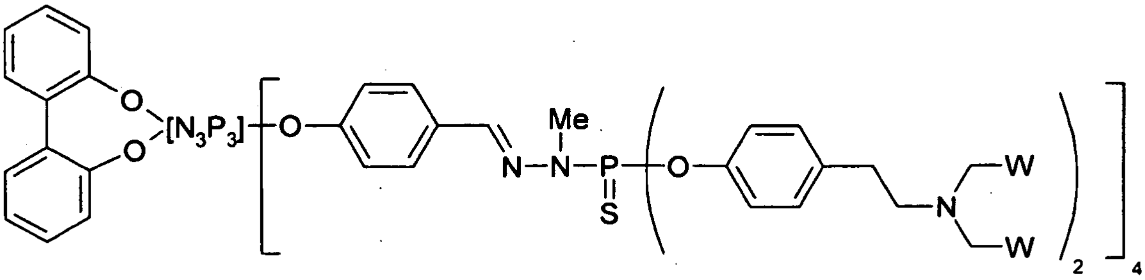

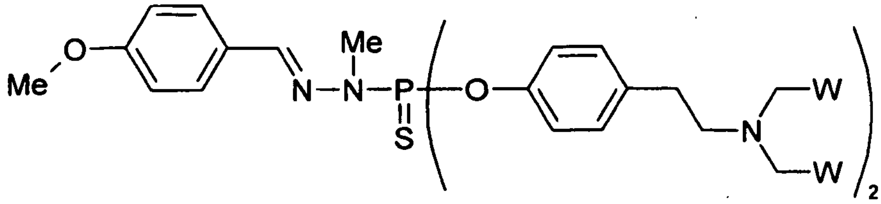

dans lesquelles W représente $PO_3Me_2$, $PO_3HNa$, $PO_3H_2$ les dits composés correspondant, notamment, au composé GC1' de formule (10) suivante :

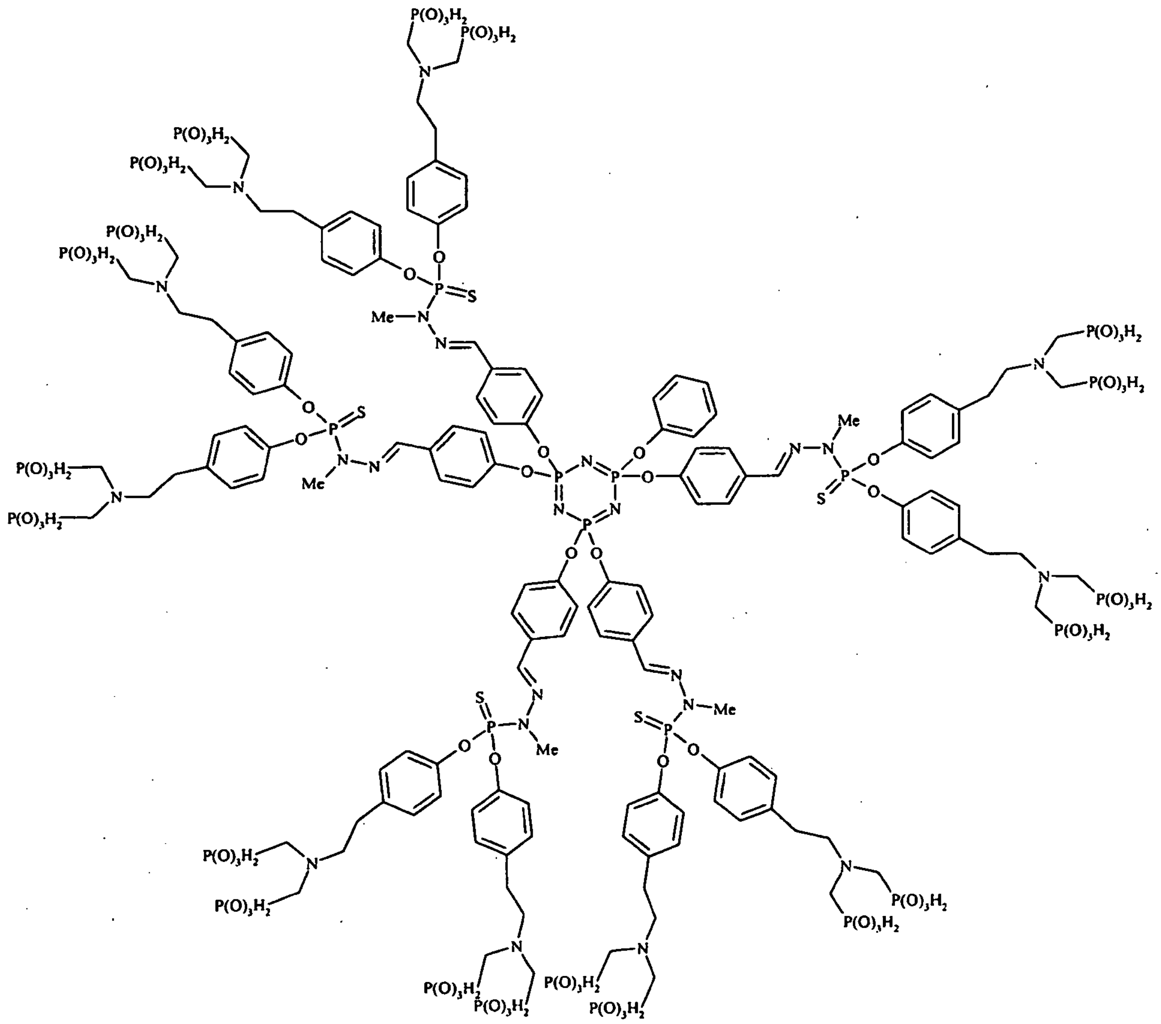

(10)

- ou des composés de formule suivante :

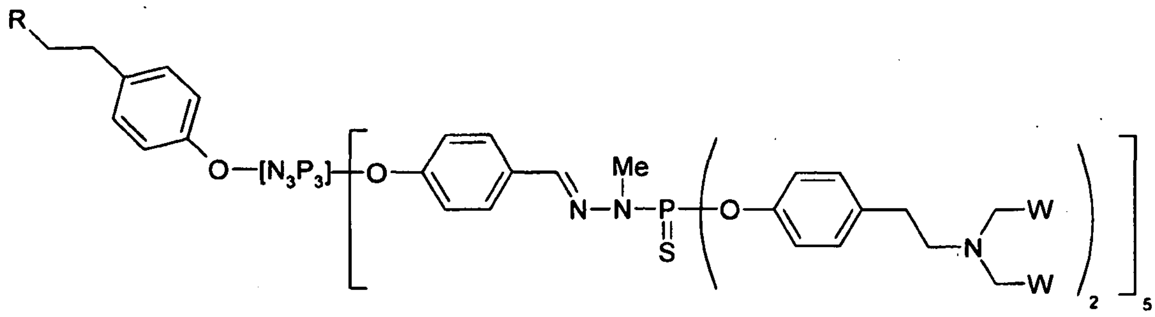

dans laquelle W représente $PO_3Me_2$, $PO_3HNa$, ou $PO_3H_2$ et R représente un groupe fluorescent choisi parmi :

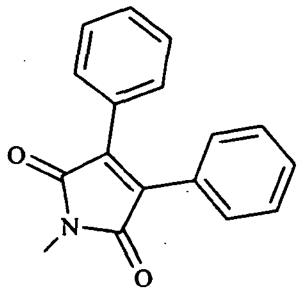 , 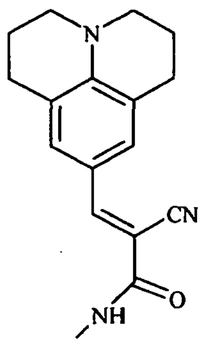 ,

lesdits composés correspondant notamment aux composés de formules suivantes :

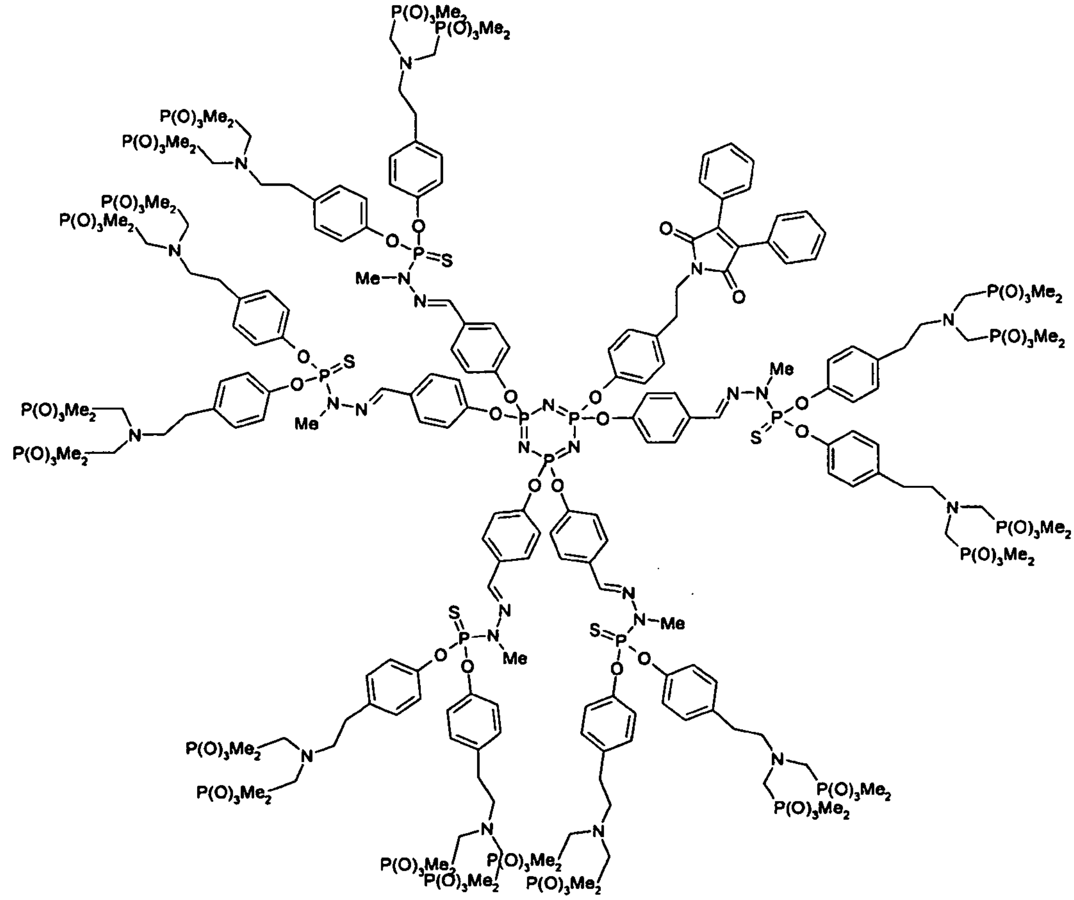

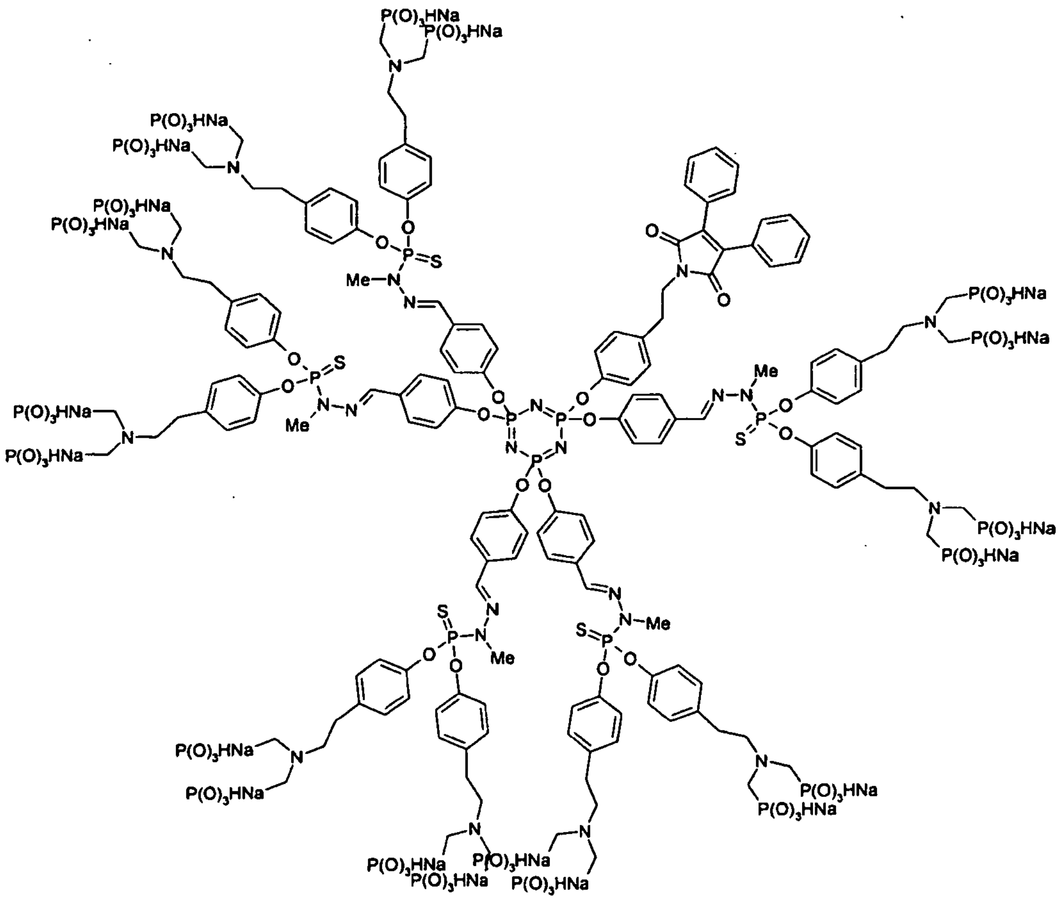

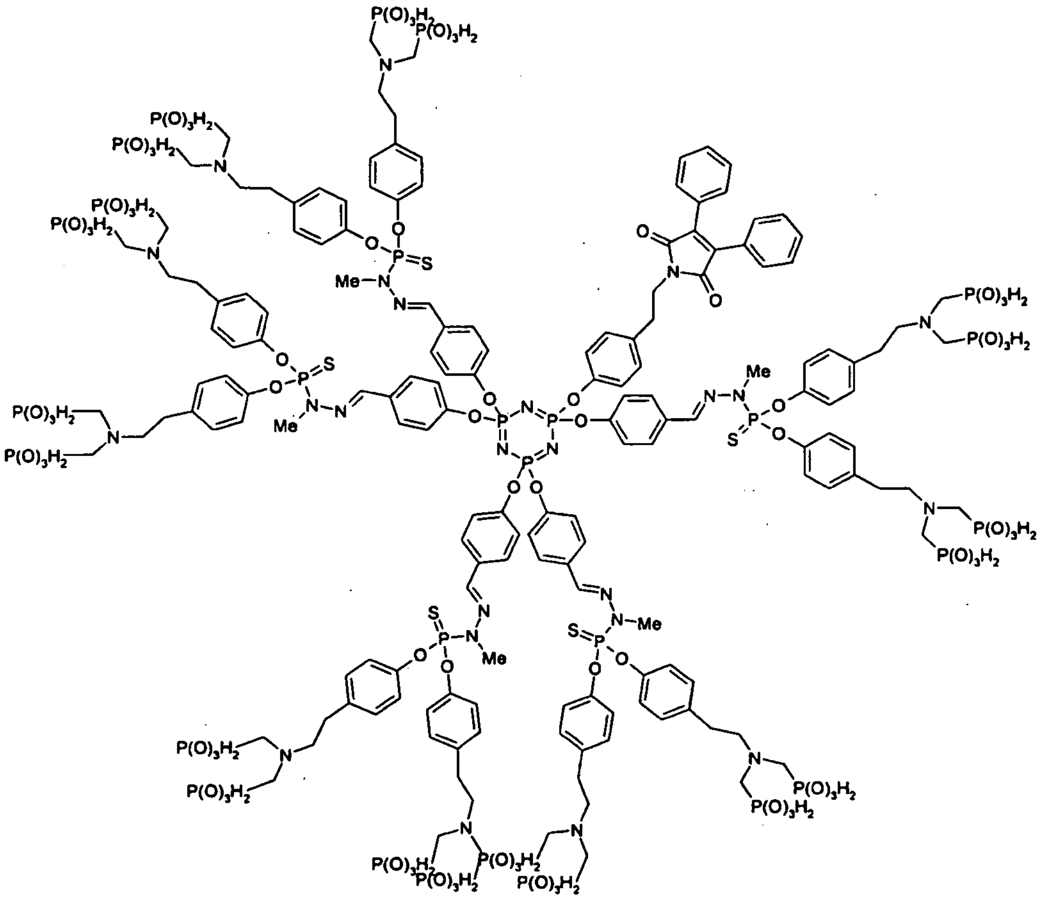

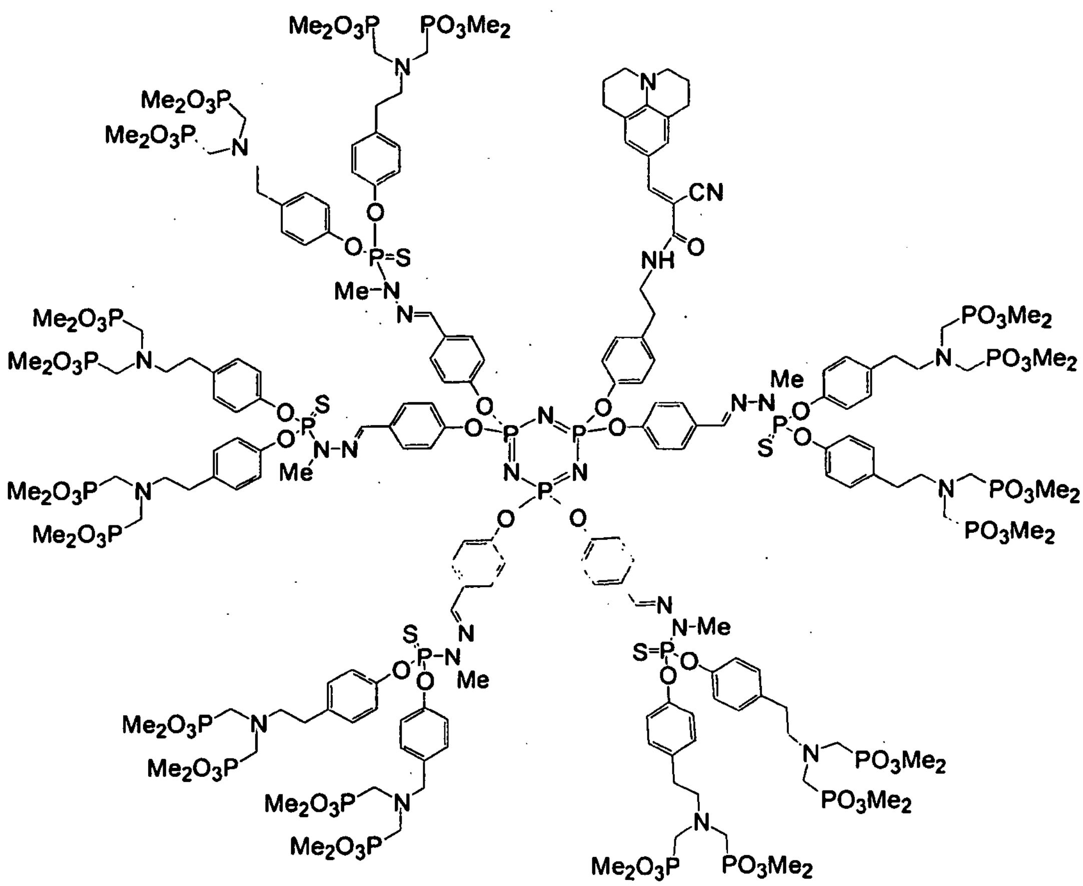

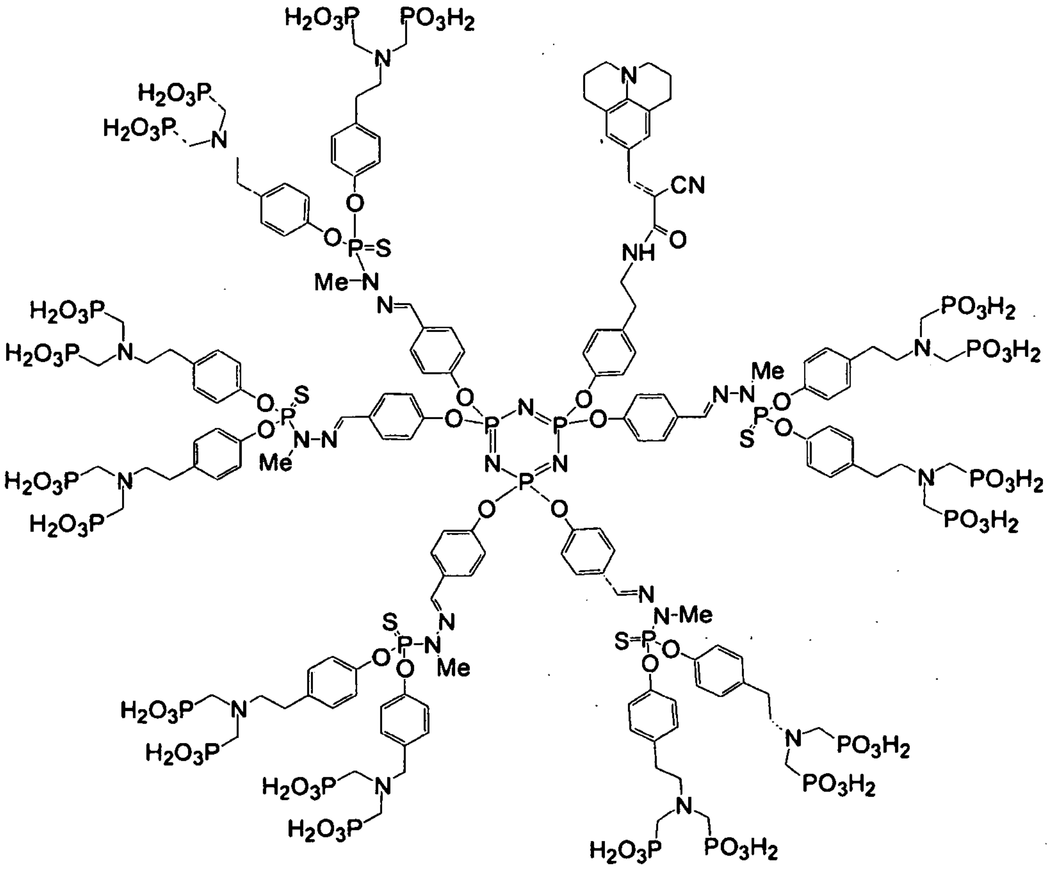

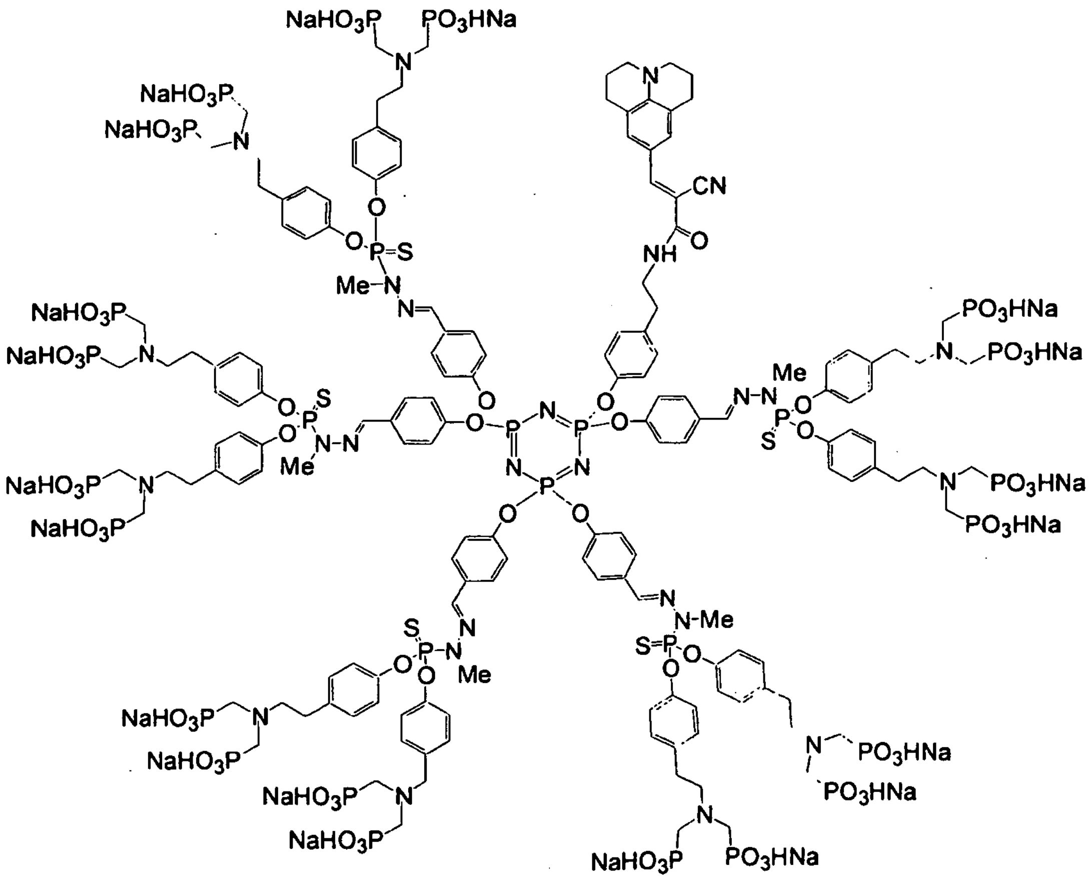

18. Utilisation selon l'une des revendications 1 à 17, dans laquelle les cellules de la lignée lymphoïde exprimant le récepteur NKG2D sont dérivées de cellules NK, de lymphocytes T $\alpha\beta$ $CD8^+$ ou de lymphocytes T $\gamma 8$, notamment de cellules NK.

19. Utilisation selon l'une des revendications 1 à 17, dans laquelle des cellules de la lignée monocytaire en culture sont activées par les dendrimères à terminaisons monophosphoniques ou bisphosphoniques, l'activation des cellules de la lignée monocytaire correspondant notamment :

- à une augmentation de la taille des cellules activées par rapport aux cellules non activées, et/ou
- à une diminution de l'expression des molécules de CMH de classe I et de classe II, ou de la molécule CD14 par rapport aux cellules non activées, et/ou
- à une augmentation de la translocation nucléaire du facteur NF$\kappa$B.

20. Utilisation selon la revendication 19, dans laquelle les cellules de la lignée monocytaire en culture présentent une apoptose réduite par rapport à des cellules de la lignée monocytaire cultivées en absence de dendrimère à terminaisons monophosphoniques ou bisphosphoniques.

21. Milieu de culture cellulaire, **caractérisé en ce qu'**il contient au moins un composé dendrimérique à terminaisons monophosphoniques ou bisphosphoniques.

22. Milieu de culture selon la revendication 21, **caractérisé en ce qu'**il comprend en outre au moins un facteur de croissance et/ou d'activation des cellules NK.

23. Milieu de culture selon la revendication 21 ou 22, **caractérisé en ce qu'**il comprend au moins une interleukine choisie parmi le groupe comprenant : IL-2, IL-7, IL-12, IL-15, IL-18, ou IL-21.

**24.** Milieu de culture selon l'une des revendications 21 à 23, **caractérisé en ce qu'**il comprend au moins un composé dendrimérique à terminaisons monophosphoniques ou bisphosphoniques en association avec de l'IL-2.

**25.** Milieu de culture selon l'une des revendications 21 à 24, **caractérisé en ce que** le composé dendrimérique correspond aux dendrimères tels que définis dans l'une des revendications 4 à 17.

**26.** Milieu de culture selon l'une des revendications 21 à 24, **caractérisé en ce que** le composé dendrimérique correspond aux dendrimères définis dans la revendications 14, ou à GC0 ou GC2, notamment à GC1.

**27.** Milieu de culture selon l'une des revendications 21 à 26, **caractérisé en ce qu'**il comprend le composé GC1 à la concentration d'environ 10 à environ 50 μM, notamment environ 20 μM, en association avec de l'IL-2 à la concentration d'environ 100 à environ 1000 unités par ml, correspondant à une concentration d'environ 4 à environ 40 ng par ml, notamment environ 400 unités par ml, correspondant à environ 16 ng/ml, et **en ce que** l'IL-2 correspond à de l'IL-2 recombinante humaine.

**28.** Procédé de préparation de compositions cellulaires enrichies en cellules de la lignée lymphoïde exprimant le récepteur NKG2D, notamment en cellules NK, **caractérisé en ce qu'**il comprend une étape de mise en présence d'un échantillon biologique avec un dendrimère à terminaisons monophosphoniques ou bisphosphoniques.

**29.** Procédé selon la revendication 28 **caractérisé en ce qu'**il comprend des cellules de la lignée lymphoïde et/ou des cellules de la lignée monocytaire.

**30.** Procédé selon la revendication 28 ou 29, **caractérisé en ce que** l'échantillon biologique est constitué de sang humain, notamment d'une fraction cellulaire mononucléée d'un échantillon de sang périphérique humain.

**31.** Procédé selon l'une des revendications 28 à 30, **caractérisé en ce que** le dendrimère à terminaisons monophosphoniques ou bisphosphoniques correspond aux dendrimères tels que définis dans l'une des revendications 4 à 17.

**32.** Procédé selon l'une des revendications 28 à 31, **caractérisé en ce que** le dendrimère à terminaisons monophosphoniques ou bisphosphoniques correspond aux dendrimères définis dans la revendications 14, ou à GC0 ou GC2, notamment à GC 1.

**33.** Compositions cellulaires enrichies en cellules de la lignée lymphoïde exprimant le récepteur NKG2D, notamment en cellules NK, telles qu'obtenues par le procédé selon l'une des revendications 28 à 32.

**34.** Compositions cellulaires selon la revendication 33, **caractérisées en ce qu'**elles comprennent également un dendrimère à terminaisons monophosphoniques ou bisphosphoniques, notamment GC 1.

**35.** Procédé de préparation de monocytes activés ou de compositions cellulaires comprenant des monocytes activés, **caractérisé en ce qu'**il comprend une étape de mise en présence d'un échantillon biologique comprenant des monocytes avec un dendrimère à terminaisons monophosphoniques ou bisphosphoniques.

**36.** Procédé selon la revendication 35, **caractérisé en ce que** l'échantillon biologique est constitué de sang humain, notamment d'une fraction cellulaire mononucléée d'un échantillon de sang périphérique humain.

**37.** Procédé selon la revendication 35 ou 36, **caractérisé en ce que** le dendrimère à terminaisons monophosphoniques ou bisphosphoniques correspond aux dendrimères tels que définis dans l'une des revendications 3 à 16.

**38.** Procédé selon l'une des revendications 35 à 37, **caractérisé en ce que** le dendrimère à terminaisons monophosphoniques ou bisphosphoniques correspond aux dendrimères définis dans la revendications 14, ou à GC0 ou GC2, notamment à GC1.

**39.** Monocytes activés ou compositions cellulaires comprenant des monocytes activés tels qu'obtenus par le procédé selon l'une des revendications 35 à 38.

**40.** Composition pharmaceutique **caractérisée en ce qu'**elle comprend à titre de substance active des cellules lymphoïdes exprimant le récepteur NKG2D, notamment des cellules NK, et au moins un dendrimère à terminaisons monophosphoniques ou bisphosphoniques, en association avec un véhicule pharmaceutiquement acceptable.

**41.** Composition pharmaceutique selon la revendication 40, **caractérisée en ce que** le dendrimère à terminaisons monophosphoniques ou bisphosphoniques correspond aux dendrimères tels que définis dans l'une des revendications 4 à 17.

**42.** Composition pharmaceutique selon la revendication 40 ou 41, **caractérisée en ce que** le dendrimère à terminaisons monophosphoniques ou bisphosphoniques correspond aux dendrimères définis dans la revendications 14, ou à GC0 ou GC2, notamment à GC1.

**43.** Composition pharmaceutique, **caractérisée en ce qu'**elle contient à titre de substance active une composition cellulaire telle que définie dans la revendication 33 et/ou des monocytes activés ou une composition cellulaire comprenant des monocytes activés tels que définis dans la revendication 39, en association avec un véhicule pharmaceutiquement acceptable.

**44.** Composition pharmaceutique selon l'une des revendications 40 à 43, **caractérisée en ce qu'**elle convient pour l'administration à un individu d'une dose unitaire d'environ $10^5$ à environ $5.10^9$ cellules lymphoïdes exprimant le récepteur NKG2D, notamment de cellules NK.

**45.** Utilisation de cellules lymphoïdes exprimant le récepteur NKG2D, notamment de cellules NK, et de GC1 pour la préparation de médicaments destinés à traiter et/ou à prévenir les cancers, dont les tumeurs de tissus hématopoïétiques, telles que les leucémies myéloïdes ou les lymphomes anaplasiques, et les mélanomes.

**46.** Utilisation d'une composition cellulaire telle que définie dans la revendication 33 et/ou de monocytes activés ou d'une composition cellulaire comprenant des monocytes activés tels que définis dans la revendication 39, pour la préparation de médicaments destinés à traiter et/ou à prévenir les cancers, dont les tumeurs de tissus hématopoïétiques, telles que les leucémies myéloïdes ou les lymphomes anaplasiques, et les mélanomes.

**47.** Composition pharmaceutique contenant à titre de substance active au moins un dendrimère à terminaisons monophosphoniques ou bisphosphoniques en association avec un véhicule pharmaceutiquement acceptable.

**48.** Composition pharmaceutique selon la revendication 47, **caractérisée en ce que** le dendrimère à terminaisons monophosphoniques ou bisphosphoniques correspond aux dendrimères tels que définis dans l'une des revendications 4 à 14.

**49.** Composition pharmaceutique selon la revendication 47 ou 48, **caractérisée en ce que** le dendrimère à terminaisons monophosphoniques ou bisphosphoniques correspond aux dendrimères définis dans la revendications 14, ou à GC0 ou GC2, notamment à GC1.

**50.** Dendrimères à terminaisons monophosphoniques ou bisphosphoniques de génération n, n représentant un nombre entier de 0 à 12, comprenant un noyau central § de valence m, m représentant un entier de 3 à 20, en particulier de 3 à 10 et plus particulièrement de 3 à 8, le noyau établissant m-2 ou m-1 liaisons avec respectivement m-2 ou m-1 chaînes de liaison identiques constituées :

- de chaînes de génération fixées en arborescence autour du noyau sur chacune des liaisons, lorsque n est supérieur ou égal à 1, une chaîne de génération d'une génération donnée étant reliée à
  - une chaîne de génération de la génération immédiatement inférieure à la génération donnée, ou au noyau lorsque la génération donnée vaut 1, et à
  - au moins 2 chaînes de génération de la génération immédiatement supérieure à la génération donnée, ou éventuellement à au moins une chaîne intermédiaire lorsque la génération donnée vaut n,

  un groupe terminal étant fixé à l'extrémité de chaque chaîne de génération, ou le cas échéant à l'extrémité de chaque chaîne intermédiaire, ou
- de chaînes intermédiaires fixées autour du noyau sur chacune des liaisons, lorsque n vaut 0, un groupe terminal étant fixé à l'extrémité de chaque chaîne intermédiaire ;

ledit groupe terminal étant représenté par la formule :

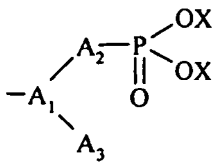

où

$A_1$ représente N ; un groupe P=Y, où Y représente O, S, ou aucun atome ; un groupe N-R ; ou un groupe C-R ; R représentant H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR'R", un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R' et R" représentant indépendamment l'un de l'autre H ou un groupement alkyle de 1 à 16 atomes de carbone, aryle de 6 à 24 atomes de carbone ou aralkyle de 7 à 16 atomes de carbone ;

$A_2$ représente une liaison simple ou une chaîne hydrocarbonée de 1 à 6 chaînons, linéaire, ramifiée ou cyclique, chacun desdits chaînons contenant éventuellement un ou plusieurs hétéroatomes, lesdits hétéroatomes étant préférentiellement choisis parmi un atome de soufre, d'oxygène, de phosphore, ou d'azote, plus préférentiellement d'azote, et étant éventuellement substitués par au moins un substituant choisi parmi H, un groupement alkyle de 1 à 16 atomes de carbone, un halogène, un groupement $-NO_2$, un groupement -NRR', un groupement -CN, un groupement $-CF_3$, un groupement hydroxyle, un groupement alkoxy de 1 à 16 atomes de carbone, un groupement aryle ou hétéroaryle de 1 à 24 atomes de carbone, l'hétéroélément étant préférentiellement choisi parmi l'oxygène, l'azote ou le soufre, un groupement aralkyle de 7 à 16 atomes de carbone, R et R' représentant indépendamment l'un de l'autre H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone ;

$A_3$ représente H, ou une chaîne hydrocarbonée de 1 à 6 chaînons, linéaire, ramifiée ou cyclique, chacun desdits chaînons pouvant éventuellement être choisi parmi un hétéroatome, ledit hétéroatome étant préférentiellement choisi parmi le soufre, l'azote, le phosphore, ou le silicium, plus préférentiellement l'azote, chaque chaînon pouvant être éventuellement substitué par au moins un groupement choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone, ou aralkyle de 7 à 16 atomes de carbone ou

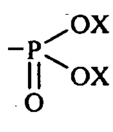,

en particulier $A_3$ peut représenter

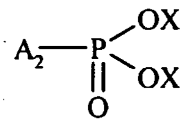

chacun des $A_2$ étant identiques ou différents ;

chaque OX, identique ou différent pour chaque groupement phosphonique, représente OH, OAlkyle, où le groupe alkyle comprend de 1 à 16 atomes de carbone, OAryle, où le groupe aryle comprend de 6 à 24 atomes de carbone, OAralkyle, où le groupe aralkyle comprend de 7 à 24 atome de carbone, OAlkylaryle, où le groupe alkylaryle comprend de 7 à 24 atomes de carbone, $OSiR'_1R'_2R'_3$, où $R'_1$, $R'_2$ et $R'_3$, identiques ou différents, représentent un groupement alkyle de 1 à 16 atomes de carbone, ou $O^-M^+$, où $M^+$ est un cation d'éléments du groupe IA, IB, IIA, IIB ou IIIA, IIB de la classification périodique des éléments, de préférence $M^+$ est choisi parmi les cations des atomes de sodium, de potassium, de cuivre, de calcium, de baryum, de zinc, de magnésium, de lithium et d'aluminium, ou de groupes hydrocarbonés de 1 à 100 atomes de carbone, ou azotés de 0 à 100 atomes de carbone, tel que $NR_1R_2R_3R_4^+$, où, indépendamment les uns des autres $R_1$, $R_2$, $R_3$ et $R_4$ représentent H ou une chaîne hydrocarbonée de 1 à 16 chaînons linéaire, ramifiée ou cyclique, contenant éventuellement un ou plusieurs hétéroatomes, lesdits hétéroatomes étant préférentiellement choisis parmi l'oxygène, le soufre, l'azote, le phosphore, le silicium et/ou une ou plusieurs doubles ou triples liaisons, chacun desdits chaînons étant éventuellement substitué par au moins un substituant choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone ;

les 1 ou 2 liaisons restantes étant fixées à des groupes de liaison, identiques ou différents, éventuellement liés ensemble, notamment par l'intermédiaire d'une liaison covalente, constitués
soit d'une partie des chaînes de liaison définies ci-dessus,
soit d'un atome d'hydrogène,
soit de groupes hydrocarbonés comprenant de 1 à 500 atomes de carbone,
lesdits groupes hydrocarbonés étant notamment constitués de H ou d'une chaîne hydrocarbonée de 1 à 16 chaînons, linéaire, ramifiée ou cyclique, contenant éventuellement une ou plusieurs doubles ou triples liaisons, chacun desdits chaînons pouvant éventuellement être choisi parmi un hétéroatome, ledit hétéroatome étant préférentiellement choisi parmi un atome d'azote, d'oxygène, de phosphore, de silicium ou de soufre, un groupement aryle de 6 à 24 atomes de carbone, un groupement hétéroaryle de 1 à 24 atomes de carbone, un groupement carboxyle, un groupement >C=NR, chaque chaînon pouvant être éventuellement substitué par au moins un substituant choisi parmi un groupement hydroxyle, un groupement - NR"R"', un groupement alkoxy de 1 à 16 atomes de carbone, un groupement alkyle de 1 à 16 atomes de carbone, un atome d'halogène, un groupement $-NO_2$, un groupement -CN, un groupement $-CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone, le premier chaînon de ladite chaîne hydrocarbonée étant de préférence un oxygène ou un azote ;
ledit noyau central §
étant choisi parmi :

- un atome d'azote ou un atome de silicium,
- un groupement de formule

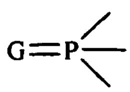

dans lequel G représente un atome d'oxygène, d'azote, de soufre, de sélénium, de tellure ou un groupement =NR, R représentant H ou un groupement alkyle de 1 à 16 atomes de carbone, aryle de 6 à 24 atomes de carbone, ou aralkyle de 7 à 16 atomes de carbone, tel que le groupement thiophosphoryle de formule

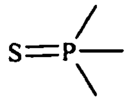 ;

- un groupement bis-phényloxy de formule

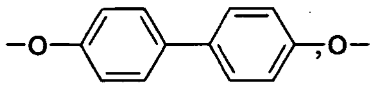 ,

- un groupement 1,2-diamino-éthane de formule

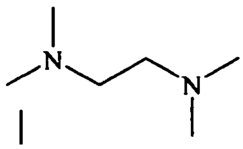 ,

- un groupement 1,4-diamino-butane de formule

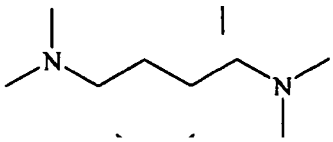 ,

- un groupement cyclotriphosphazène de formule

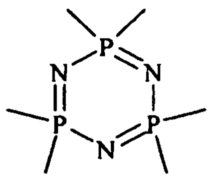 ,

- un groupement cyclotétraphosphazène de formule

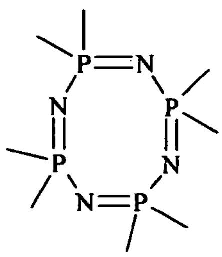 .

**51.** Dendrimères selon la revendication 50, dans lesquels les chaînes de génération sont choisies parmi toute chaîne hydrocarbonée de 1 à 12 chaînons, linéaire, ramifiée ou cyclique, contenant éventuellement une ou plusieurs doubles ou triples liaisons, chacun desdits chaînons pouvant éventuellement être choisi parmi un hétéroatome, ledit hétéroatome étant préférentiellement choisi parmi l'azote, l'oxygène, le soufre, le phosphore ou le silicium, un groupement aryle de 6 à 24 atomes de carbone, un groupement hétéroaryle de 1 à 24 atomes de carbone, l'hétéroélément étant préférentiellement choisi parmi l'oxygène, l'azote ou le soufre, un groupement carboxyle, un groupement >C=NR, chaque chaînon pouvant éventuellement être substitué par au moins un substituant choisi parmi un groupement

alkyle de 1 à 16 atomes de carbone, un atome d'halogène, un groupement $-NO_2$ un groupement - RR', un groupement -CN, un groupement $-CF_3$, un groupement hydroxyle, un groupement alkoxy de 1 à 16 atomes de carbone, un groupement aryle de 6 à 24 atomes de carbone, ou un groupement aralkyle de 7 à 16 atomes de carbone, R et R' représentant indépendamment l'un de l'autre H ou un groupement alkyle de 1 à 16 atomes de carbone, aryle de 6 à 24 atomes de carbone, ou aralkyle de 7 à 16 atomes de carbone.

**52.** Dendrimères selon la revendication 50 ou 51, dans lesquels les chaînes intermédiaires sont choisies parmi les groupes correspondant à la formule :

-J-K-L-

où

- J représente un atome d'oxygène, de soufre, ou un groupement -NR- ;
- K représente un groupement aryle de 6 à 24 atomes de carbone, hétéroaryle de 1 à 24 atomes de carbone, l'hétéroélément étant préférentiellement choisi parmi l'oxygène, l'azote ou le soufre, alkyle de 1 à 16 atomes de carbone, chacun pouvant être éventuellement substitué par un atome d'halogène ou un groupement $-NO_2$, -NRR', - CN, $-CF_3$, -OH, alkyle de 1 à 16 atomes de carbone, aryle de 6 à 24 atomes de carbone, aralkyle de 7 à 16 atomes de carbone ;
- L représente une chaîne hydrocarbonée de 0 à 10 chaînons, linéaire, ramifiée ou cyclique, contenant éventuellement une ou plusieurs doubles ou triples liaisons, chacun desdits chaînons pouvant éventuellement être un hétéroatome, ledit hétéroatome étant préférentiellement choisi parmi l'oxygène, le soufre, l'azote, le phosphore, le silicium chaque chaînon pouvant être éventuellement substitué par au moins un substituant choisi parmi un groupement alkyle de 1 à 16 atomes de carbone, un halogène, un atome d'oxygène, $-NO_2$, -NRR', -CN, $-CF_3$, -OH, un groupement alkoxy de 1 à 16 atomes de carbone, un groupement aryle de 6 à 24 atomes de carbone, un groupement aralkyle de 7 à 16 atomes de carbone ;

R et R' représentant indépendamment l'un de l'autre H ou un groupement alkyle de 1 à 16 atomes de carbone, aryle de 6 à 24 atomes de carbone, ou aralkyle de 7 à 16 atomes de carbone.

**53.** Dendrimères selon l'une des revendications 50 à 52, de structure PMMH, PAMAM, ou DAB.

**54.** Dendrimères selon l'une des revendications 50 à 52, à terminaisons bisphosphoniques correspondant à la formule générale (7) suivante :

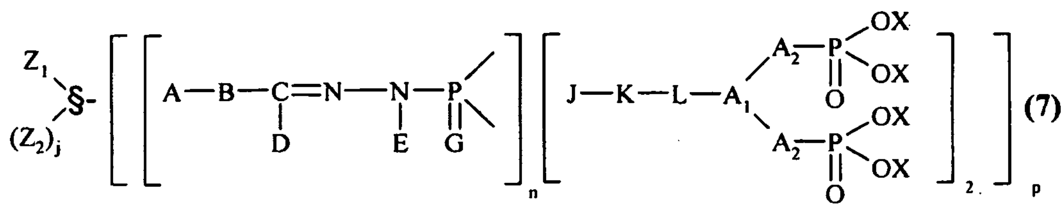

(7)

où n représente un entier de 0 à 3, m représente 3, 6 ou 8, p représente m - 1 ou m - 2, et j représente 0 lorsque p représente m - 1 et 1 lorsque p représente m - 2, à savoir :

- lorsque p = m - 1, la formule (7) correspond à la formule (8) suivante :

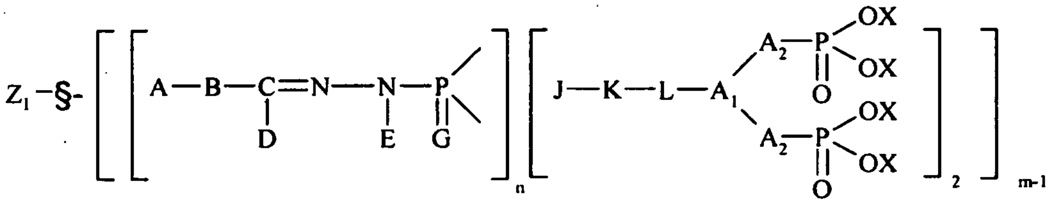

(8)

- lorsque p = m - 2, la formule (7) correspond à la formule (9) suivante

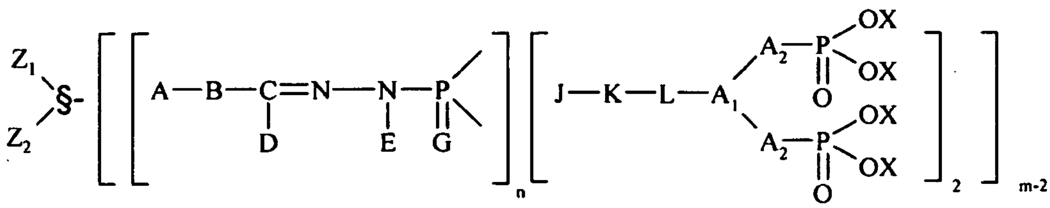

**(9)**

et dans lesquelles formules :

• le noyau central § est choisi parmi les groupes suivants :

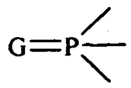 , 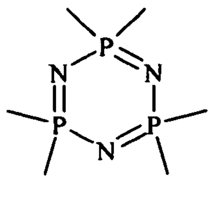 , 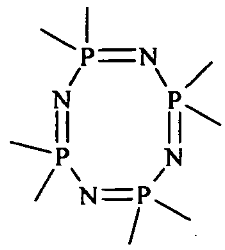 ;

• la chaîne de génération correspond à la formule :

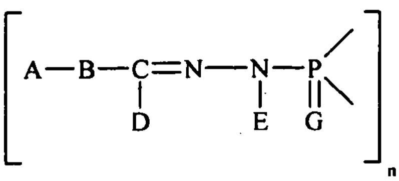

où

- A représente un atome d'oxygène, de soufre, de phosphore ou un groupement -NR-;
- B représente un groupement aryle de 6 à 24 atomes de carbone, hétéroaryle de 1 à 24 atomes de carbone, alkyle de 1 à 16 atomes de carbone, chacun pouvant être éventuellement substitué par un atome d'halogène ou un groupement $-NO_2$, -NRR', - CN, $-CF_3$, -OH, alkyle de 1 à 16 atomes de carbone, aryle de 6 à 24 atomes de carbone, aralkyle de 7 à 16 atomes de carbone ;
- D représente un atome d'hydrogène, un groupement alkyle de 1 à 16 atomes de carbone, alkoxy de 1 à 16 atomes de carbone, aryle de 6 à 24 atomes de carbone, aralkyle de 7 à 16 atomes de carbone, chacun pouvant être éventuellement substitué par un atome d'halogène ou un groupement $-NO_2$, -NRR', -CN, $-CF_3$, -OH, alkyle de 1 à 16 atomes de carbone, aryle de 6 à 24 atomes de carbone, aralkyle de 7 à 16 atomes de carbone ;
- E représente un atome d'hydrogène, un groupement alkyle de 1 à 16 atomes de carbone, alkoxy de 1 à 16 atomes de carbone, aryle de 6 à 24 atomes de carbone, aralkyle de 7 à 16 atomes de carbone, chacun pouvant être éventuellement substitué par un atome d'halogène ou un groupement $-NO_2$, -NRR', -CN, $-CF_3$, -OH, alkyle de 1 à 16 atomes de carbone, aryle de 6 à 24 atomes de carbone, aralkyle de 7 à 16 atomes de carbone ;
- G représente un atome d'oxygène, d'azote, de soufre, de sélénium, de tellure ou un groupement =NR ;

R et R' représentant indépendamment l'un de l'autre H ou un groupement alkyle de 1 à 16 atomes de carbone, aryle de 6 à 24 atomes de carbone, ou aralkyle de 7 à 16 atomes de carbone ;

•la chaîne intermédiaire correspond à la formule :

-J-K-L-

où

- J représente un atome d'oxygène, de soufre, ou un groupement -NR- ;
- K représente un groupement aryle de 6 à 24 atomes de carbone, hétéroaryle de 1 à 24 atomes de carbone,

l'hétéroélément étant préférentiellement choisi parmi l'oxygène, l'azote ou le soufre, alkyle de 1 à 16 atomes de carbone, chacun pouvant être éventuellement substitué par un atome d'halogène ou un groupement $-NO_2$, -NRR', - CN, $-CF_3$, -OH, alkyle de 1 à 16 atomes de carbone, aryle de 6 à 24 atomes de carbone, aralkyle de 7 à 16 atomes de carbone ;
- L représente une chaîne hydrocarbonée de 0 à 10 chaînons, linéaire, ramifiée ou cyclique, contenant éventuellement une ou plusieurs doubles ou triples liaisons, chacun desdits chaînons pouvant éventuellement être un hétéroatome, ledit hétéroatome étant préférentiellement choisi parmi l'oxygène, le soufre, l'azote, le phosphore, le silicium, chaque chaînon pouvant être éventuellement substitué par au moins un substituant choisi parmi un groupement alkyle de 1 à 16 atomes de carbone, un halogène, un atome d'oxygène, $-NO_2$, -NRR', -CN, $-CF_3$, -OH, un groupement alkoxy de 1 à 16 atomes de carbone, un groupement aryle de 6 à 24 atomes de carbone, un groupement aralkyle de 7 à 16 atomes de carbone ;
R et R' représentant indépendamment l'un de l'autre H ou un groupement alkyle de 1 à 16 atomes de carbone, aryle de 6 à 24 atomes de carbone, ou aralkyle de 7 à 16 atomes de carbone ;

• le groupe terminal correspond à la formule :

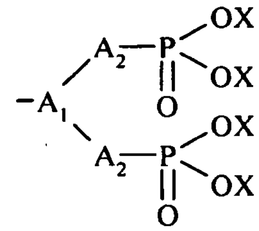

où $A_1$, $A_2$ et X ont été définis précédemment, chacun des X étant identique ou différent ;

$Z_1$ et $Z_2$ étant identiques ou différents, éventuellement liés ensemble, notamment par l'intermédiaire d'une liaison covalente, et représentant H ou une chaîne hydrocarbonée de 1 à 16 chaînons, linéaire, ramifiée ou cyclique, contenant éventuellement une ou plusieurs doubles ou triples liaisons, chacun desdits chaînons pouvant éventuellement être choisi parmi un hétéroatome, ledit hétéroatome étant préférentiellement choisi parmi un atome d'azote, d'oxygène, de phosphore, de silicium ou de soufre, un groupement aryle de 6 à 24 atomes de carbone, un groupement hétéroaryle de 1 à 24 atomes de carbone, un groupement carboxyle, un groupement >C=NR, chaque chaînon pouvant être éventuellement substitué par au moins un substituant choisi parmi un groupement hydroxyle, un groupement -NR"R"', un groupement alkoxy de 1 à 16 atomes de carbone, un groupement alkyle de 1 à 16 atomes de carbone, un atome d'halogène, un groupement $-NO_2$, un groupement -CN, un groupement - $CF_3$, un groupement aryle de 6 à 24 atomes de carbone, un groupement aralkyle de 7 à 16 atomes de carbone, R" et R"' représentant indépendamment l'un de l'autre H ou un groupement alkyle de 1 à 16 atomes de carbone, aryle de 6 à 24 atomes de carbone ou aralkyle de 7 à 16 atomes de carbone, le premier chaînon de ladite chaîne hydrocarbonée étant de préférence un oxygène ou un azote.

**55.** Dendrimères selon la revendication 54, de structure PMMH, correspondant à la formule générale (8), dans laquelle

§ représente

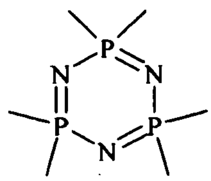 ;

m représente 6 ;
p représente 5 ;
n représente 0, 1, ou 2 ;
A représente un atome d'oxygène ;
B représente un groupement benzénique ;

D représente un hydrogène ;
E représente un groupement méthyle ;
G représente un atome de soufre ;
J représente un atome d'oxygène ;
K représente un groupement benzénique ;
L représente une chaîne hydrocarbonée saturée linéaire non substituée à deux atomes de carbone ;
$A_1$ représente un atome d'azote ;
$A_2$ représente un groupement $CH_2$ ;
X représente un groupement méthyle, ou un atome d'hydrogène ou de sodium ;
$Z_1$ représente un groupement phényloxy ;

ledit dendrimère étant désigné GCn', n étant défini ci-dessus.

**56.** Dendrimères selon l'une des revendications 50 à 55, de formules suivantes :

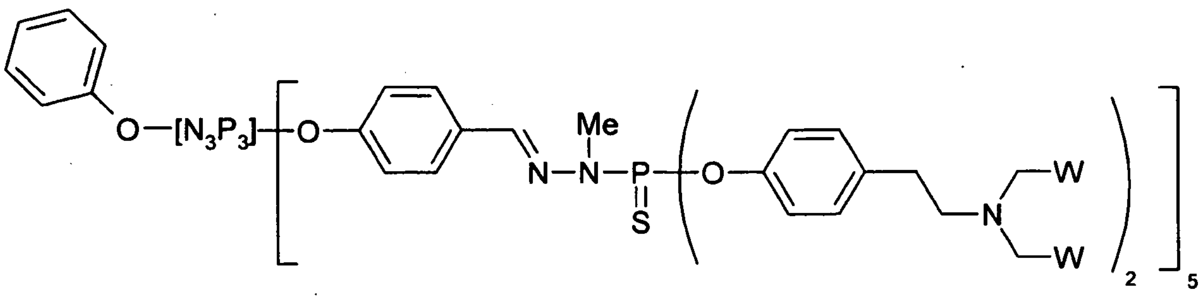

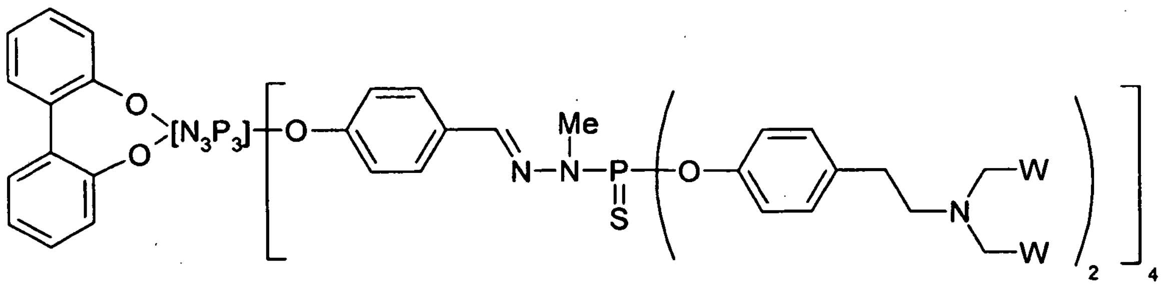

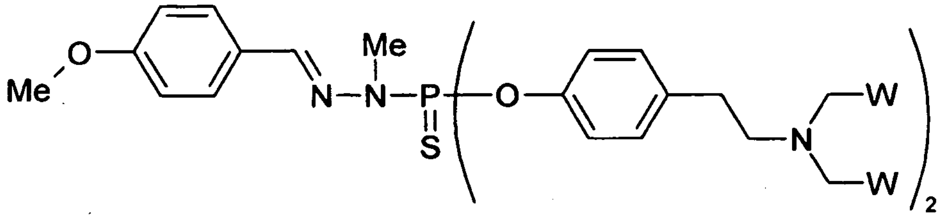

dans lesquelles W représente $PO_3Me_2$, $PO_3HNa$, ou $PO_3H_2$, lesdits dendrimères correspondant notamment au composé GC1' de formule (10) suivante :

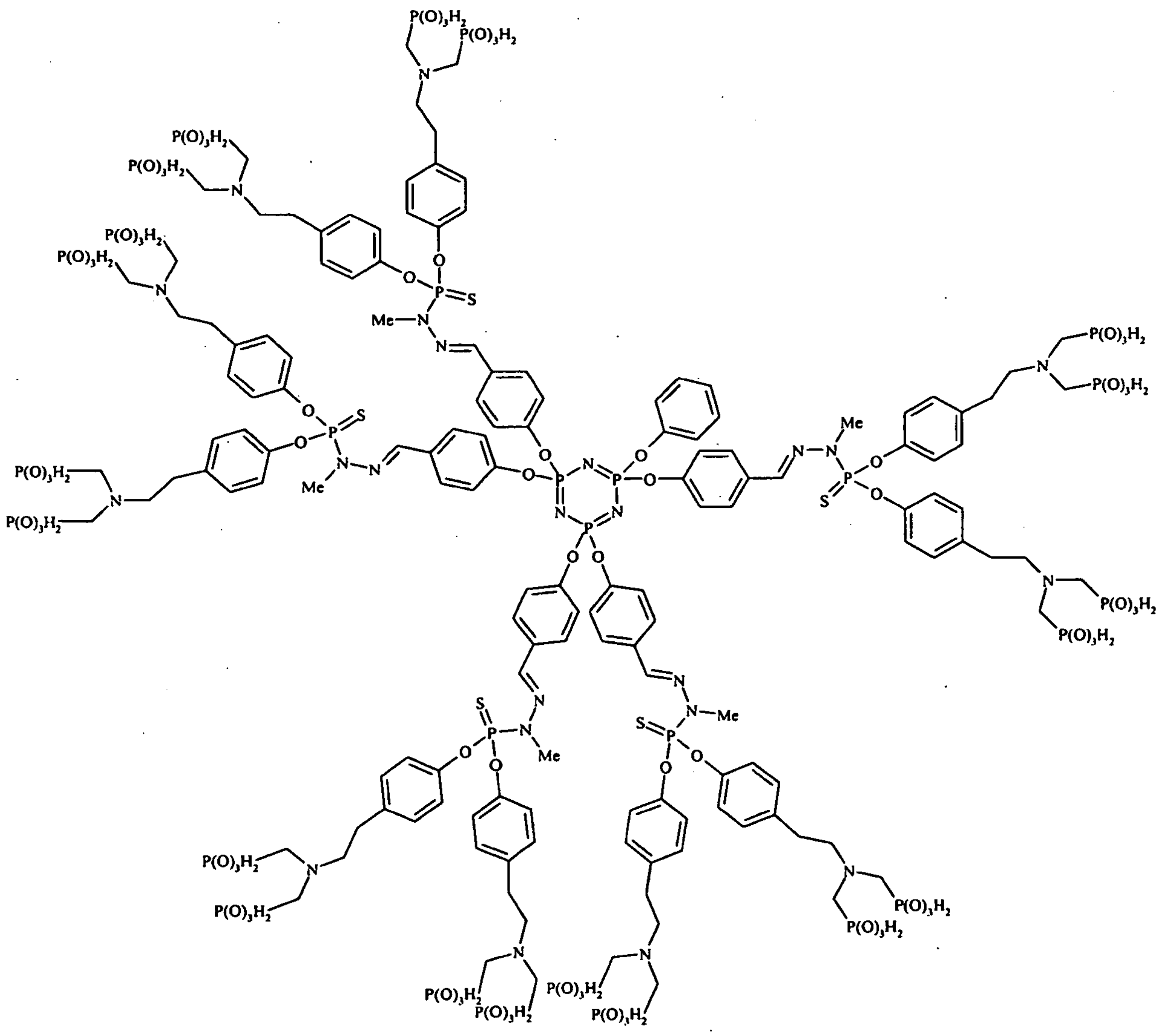

**(10)**

**57.** Dendrimères selon l'une des revendications 50 à 54, de formule suivante :

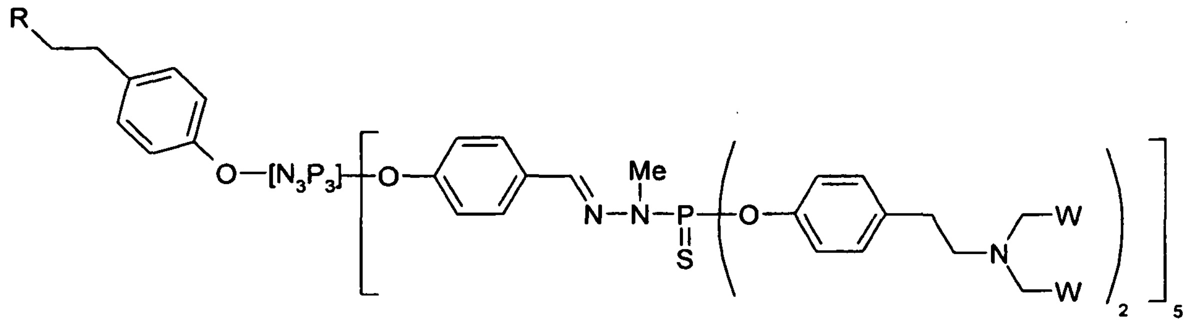

dans laquelle W représente $PO_3Me_2$, $PO_3HNa$, ou $PO_3H_2$ et R représente un groupe fluorescent choisi parmi :

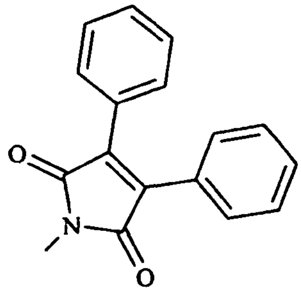 , 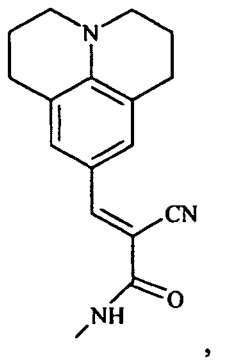 ,

lesdits dendrimères correspondant notamment aux composés de formules suivantes :

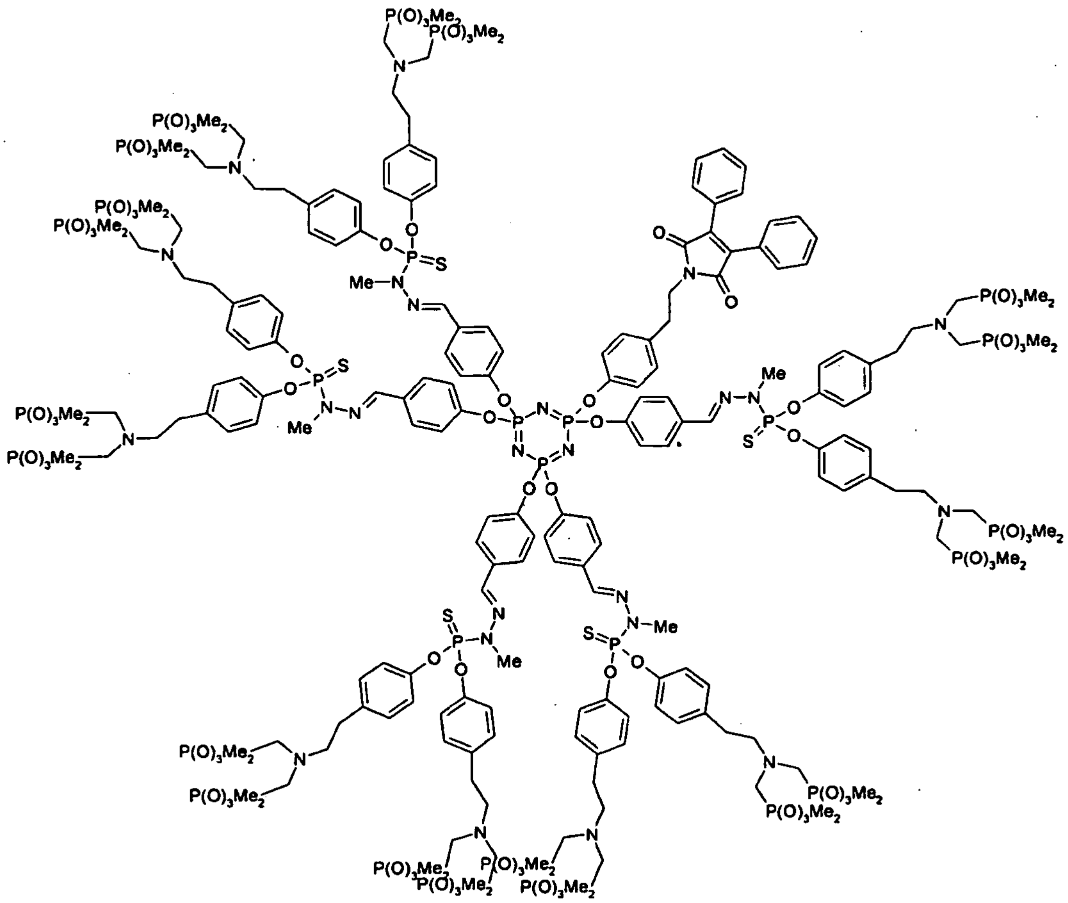

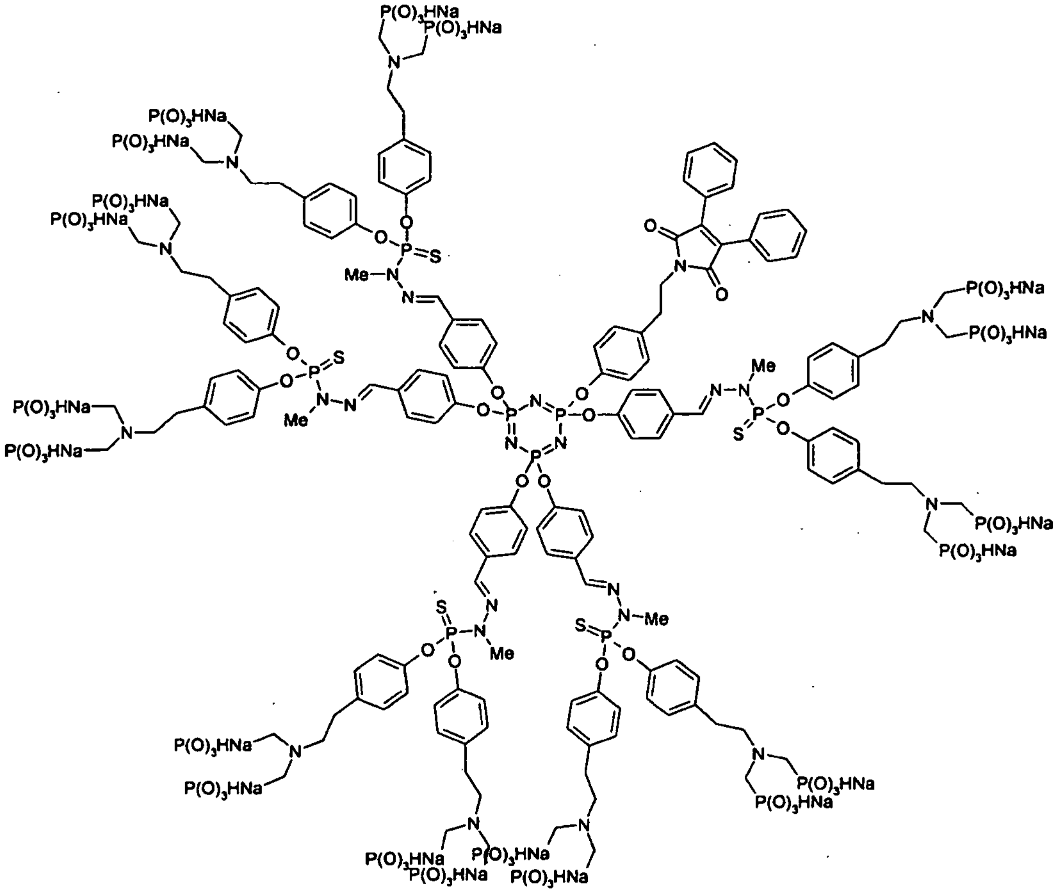

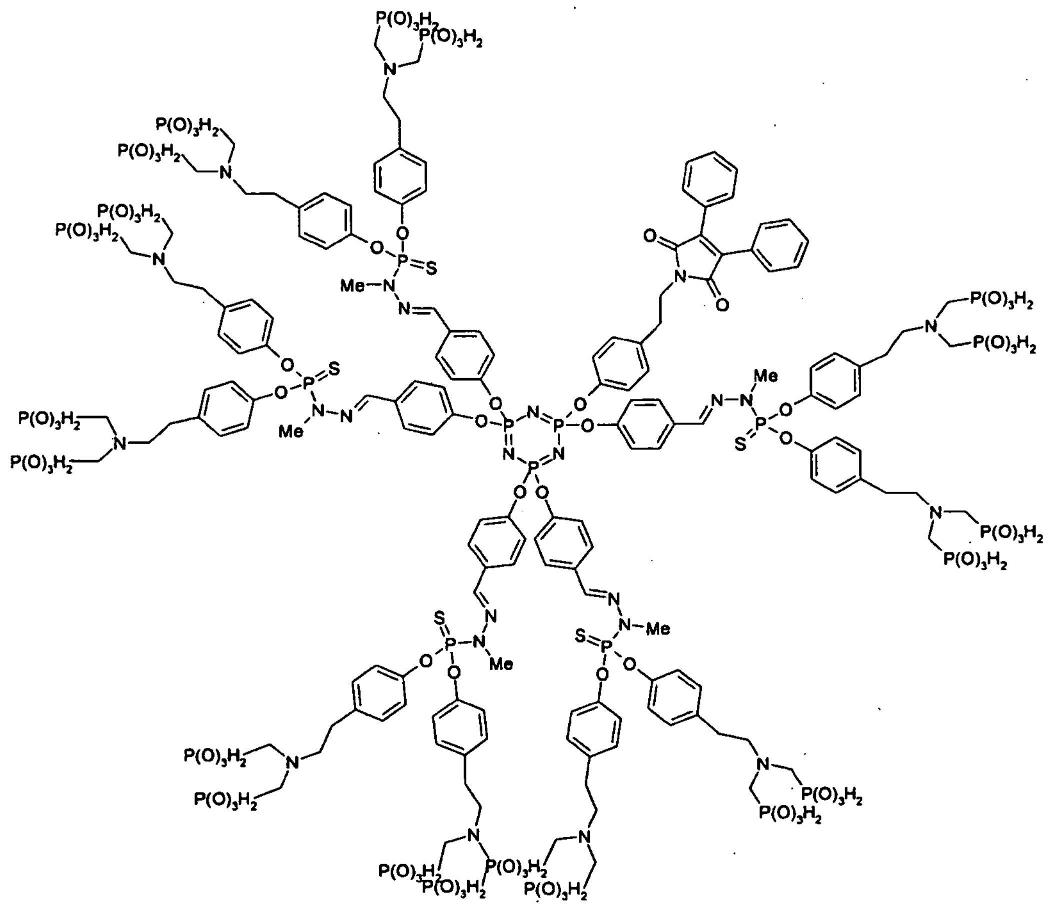

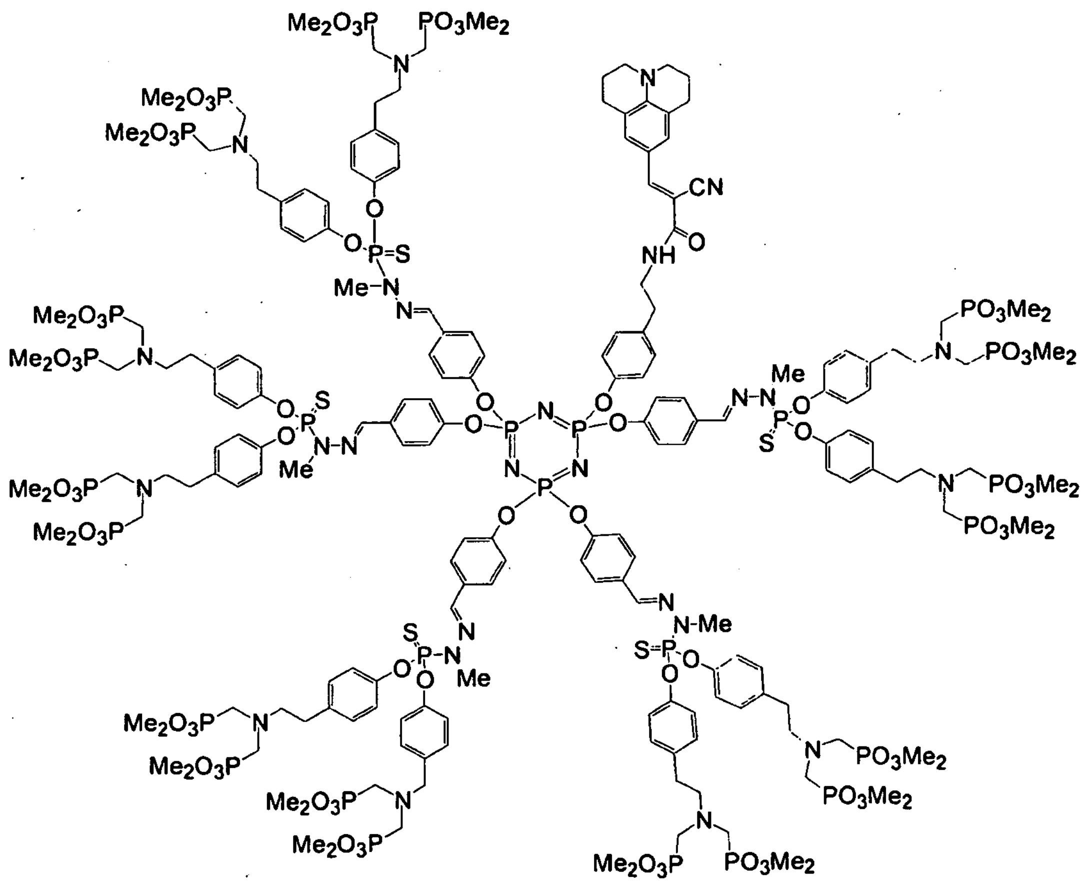

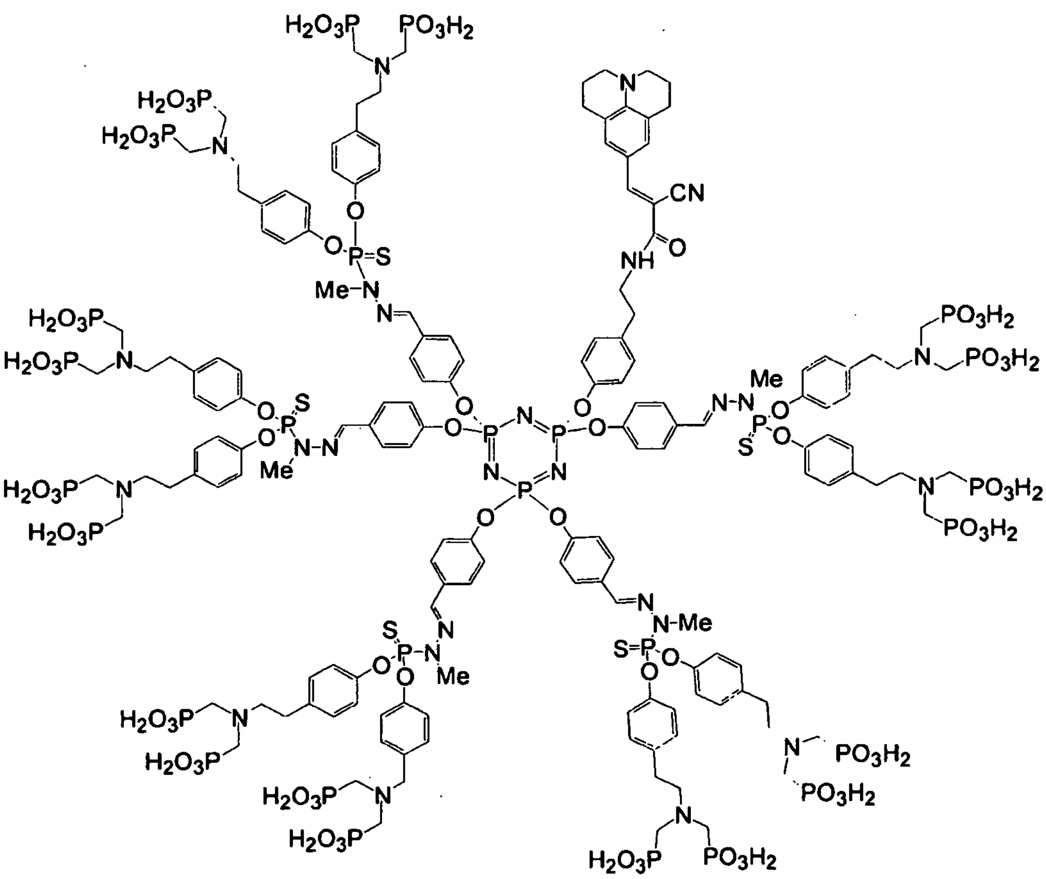

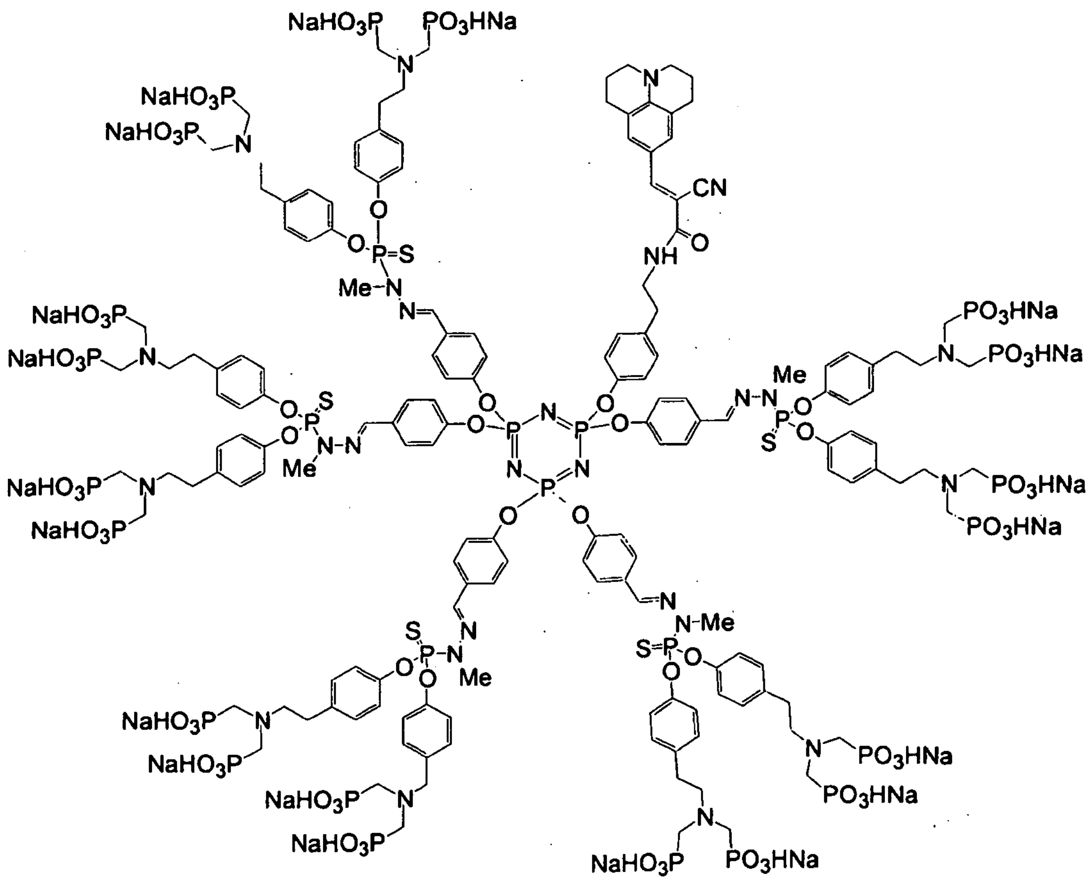

**58.** Dendrimères à terminaisons bisphophoniques selon la revendication 50, de formule suivante :

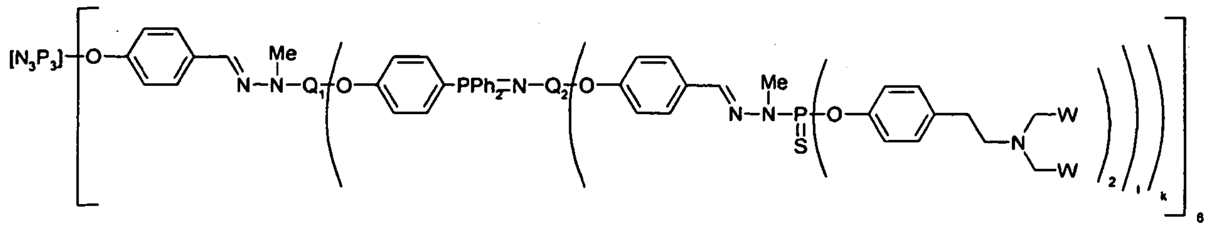

dans laquelle W représente $PO_3Me_2$, $PO_3HNa$, ou $PO_3H_2$, $Q_1$ et $Q_2$, identiques ou différents, représentent P=S ou le cyclotriphosphazène ($N_3P_3$), 1 représente 2 lorsque $Q_2$ représente P=S ou 5 lorsque $Q_2$ représente $N_3P_3$ et k représente 2 lorsque $Q_1$ représente P=S ou 5 lorsque $Q_1$ représente $N_3P_3$, lesdits dendrimères étant notamment représentés par les formules suivantes :

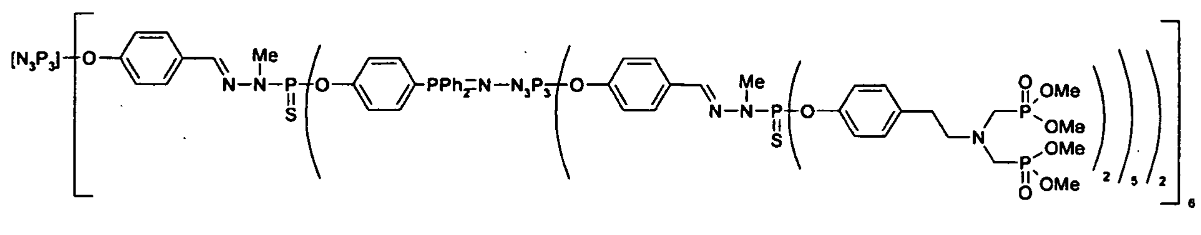

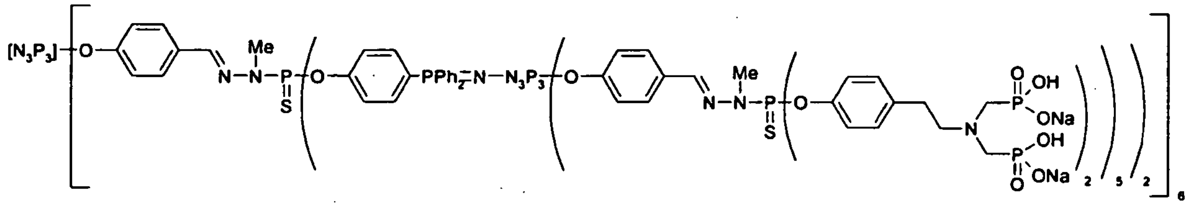

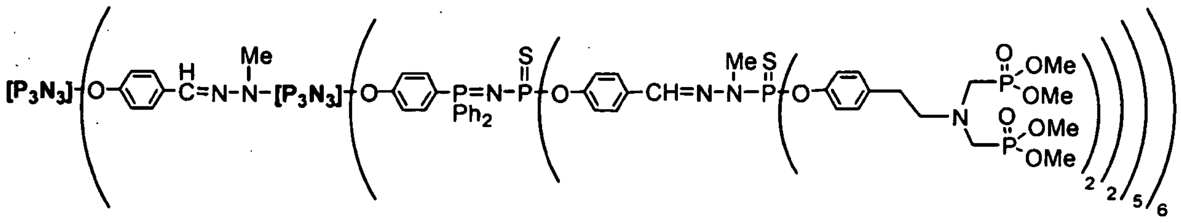

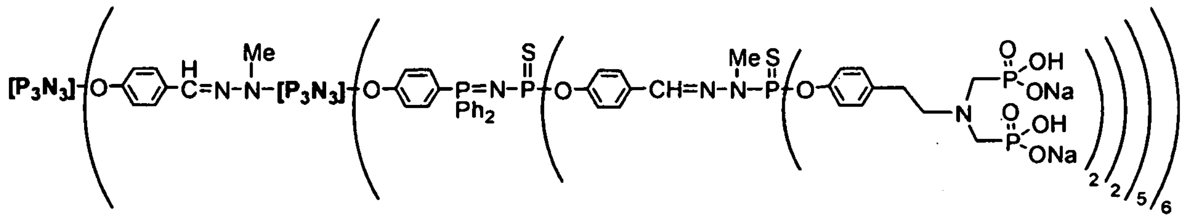

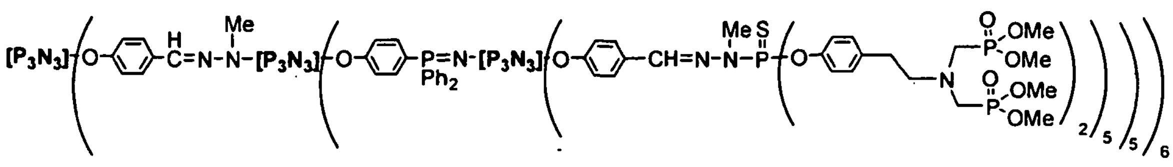

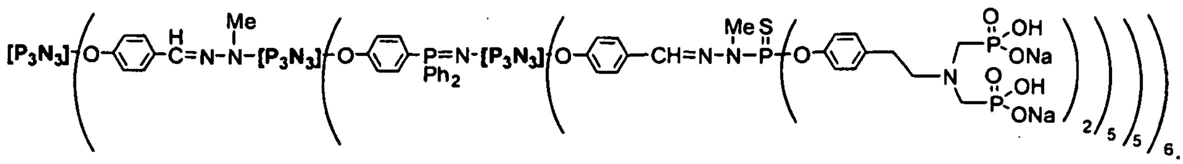

**59.** Dendrimères à terminaisons monophosphoniques ou bisphosphoniques selon la revendication 50, de formule suivante :

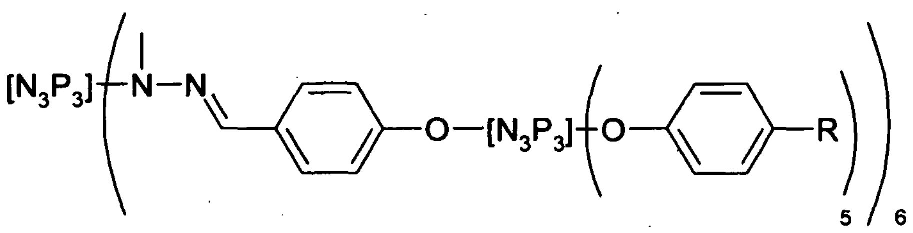

dans laquelle R représente un groupe choisi parmi

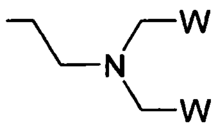 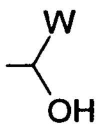

où W représente $PO_3Me_2$, $PO_3HNa$, ou $PO_3H_2$.

**60.** Dendrimères à terminaisons bisphosphoniques selon la revendication 50, de formule suivante :

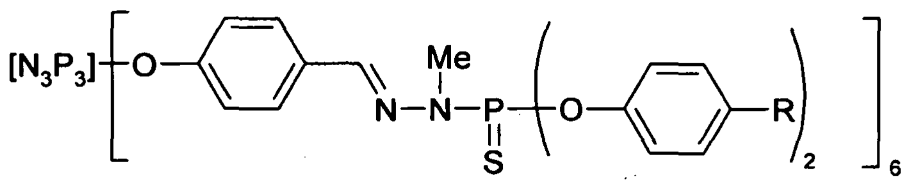

dans laquelle R représente un groupe choisi parmi :

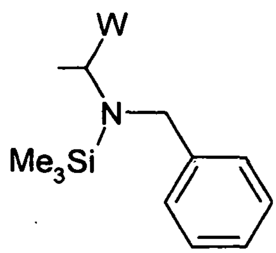 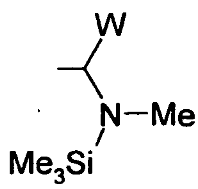 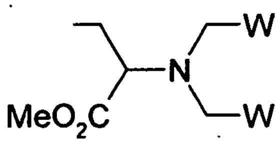 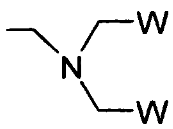

où W représente $PO_3Si_2Me_6$, $PO_3Me_2$, $PO_3HNa$, ou $PO_3H_2$.

**61.** Dendrimères à terminaisons monophosphoniques selon la revendication 50, de formule suivante :

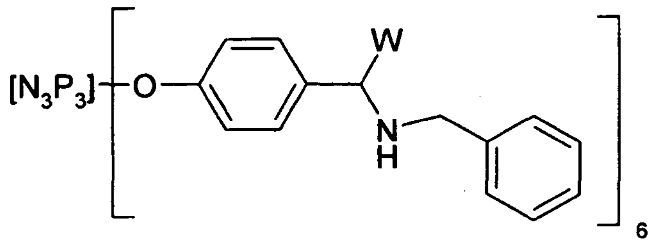

dans laquelle W représente $PO_3Me_2$, $PO_3HNa$, ou $PO_3H_2$.

**62.** Dendrimères à terminaisons bisphosphoniques selon la revendication 50, de formule suivante :

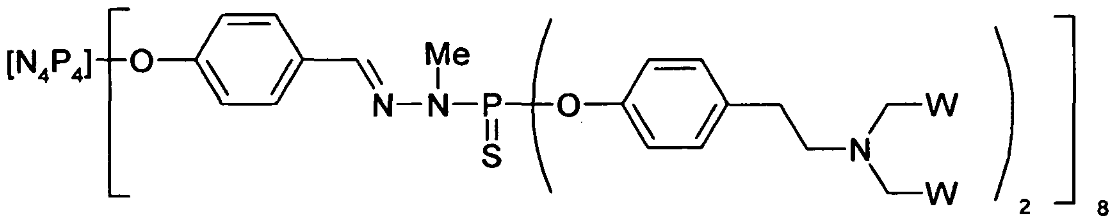

dans laquelle W représente $PO_3Me_2$, $PO_3HNa$, ou $PO_3H_2$.

**63.** Dendrimères à terminaisons bisphosphoniques selon la revendication 50, de formule suivante :

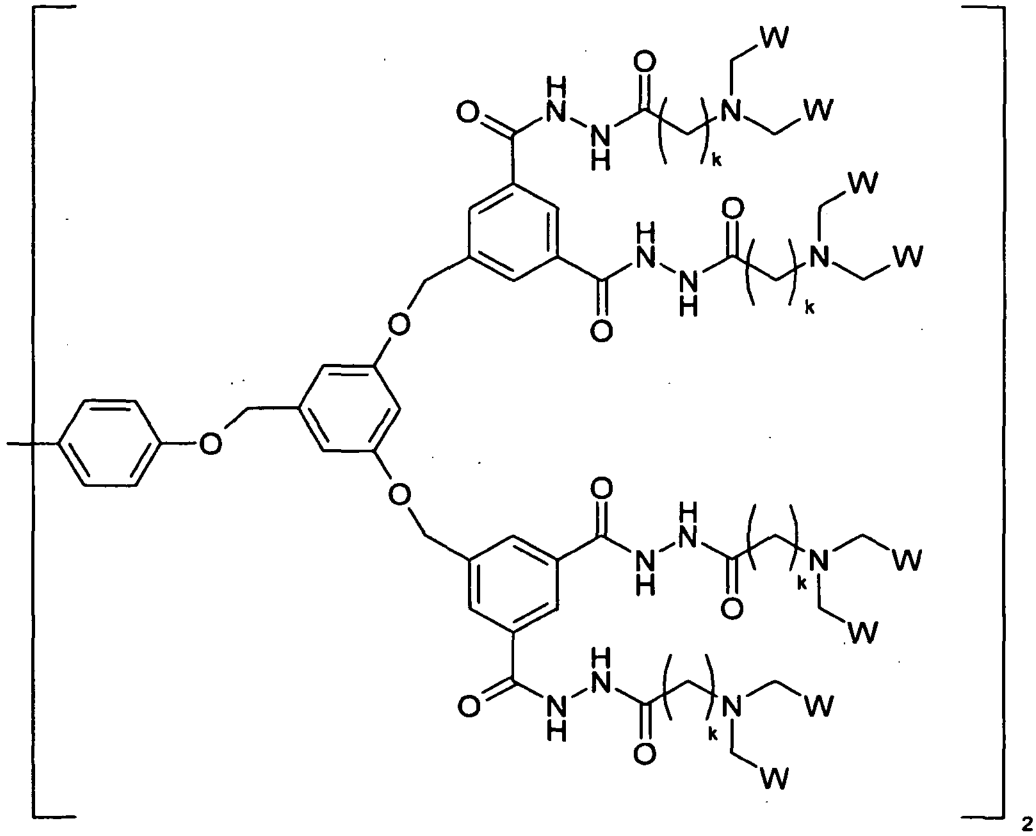

dans laquelle W représente $PO_3Me_2$, $PO_3HNa$, ou $PO_3H_2$ et k représente 1, 2 ou 3

**64.** Dendrimères à terminaisons bisphosphoniques selon la revendication 50, de formule suivante :

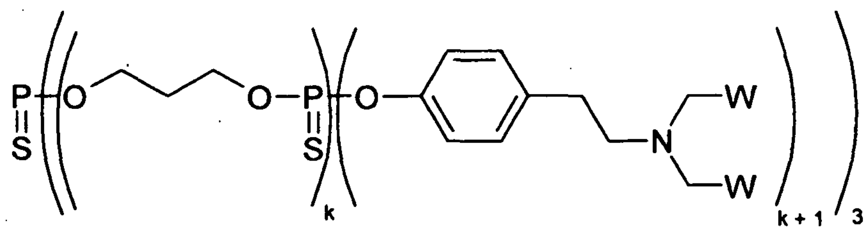

dans laquelle W représente $PO_3Me_2$, $PO_3HNa$, ou $PO_3H_2$ et k représente 0 ou 1.

**65.** Dendrimères à terminaisons bisphosphoniques de formule suivante :

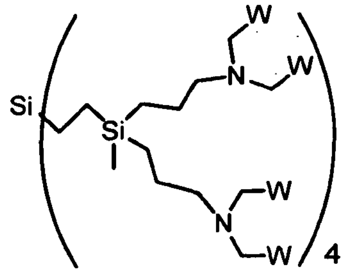

dans laquelle W représente $PO_3Me_2$, $PO_3HNa$, ou $PO_3H_2$.

**66.** Dendrimères à terminaisons bisphosphoniques de formule suivante :

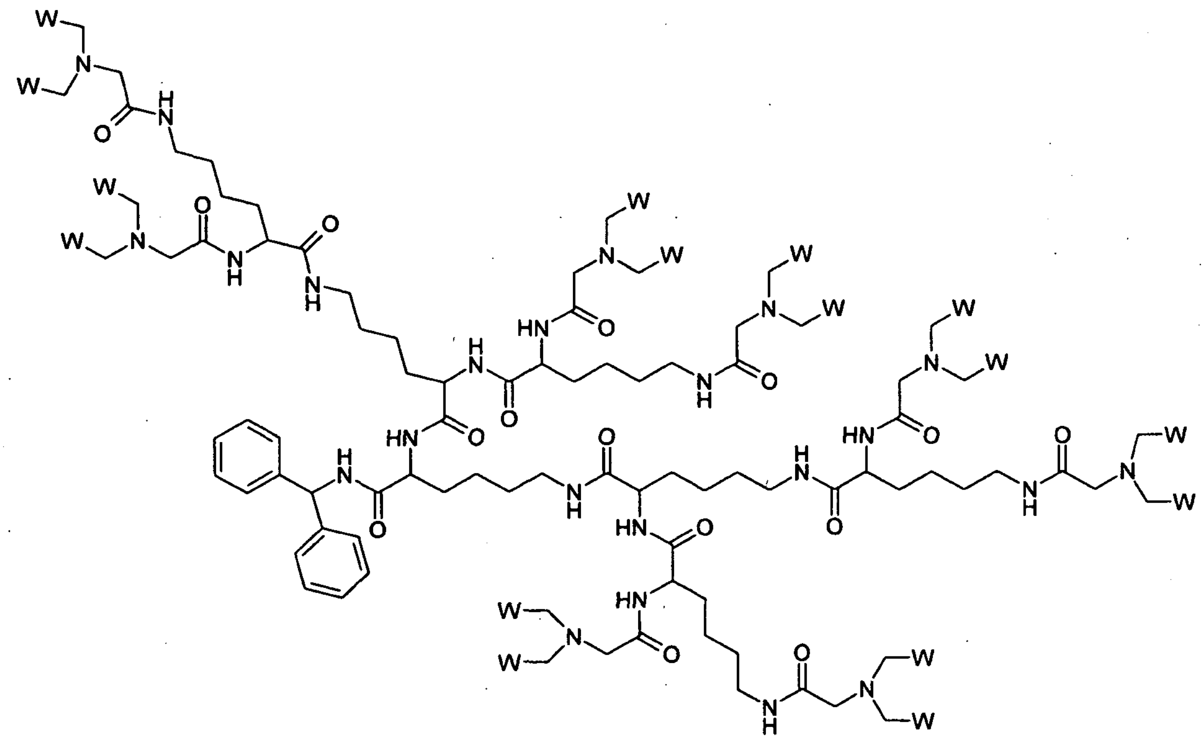

dans laquelle W représente $PO_3Me_2$, $PO_3HNa$, ou $PO_3H_2$.

## Claims

1. Use of dendrimers with monophosphonic or bisphosphonic terminations to stimulate the growth of cell cultures or to activate cells in culture.

2. Use according to claim 1, in which the cells are hematopoietic cells.

3. Use according to claim 1 or 2, of dendrimers with monophosphonic or bisphosphonic terminations for the preparation of cell compositions enriched with cells of the lymphoid line expressing the receptor NKG2D, from biological samples:

4. Use according to one of claims 1 to 3, in which the dendrimers with monophosphonic or bisphosphonic terminations are of generation n and include a central core § with a valency of m which can establish m-2 bonds, providing that m is greater than 2, or m-1 bonds, providing that m is greater than 1, or m bonds with linkage chains, preferably identical to each other, m representing an integer from 1 to 20, in particular of 1 to 10 and more particularly of 1 to

8, and n representing an integer from 0 to 12, which linkage chains are constituted by:

• generation chains attached in a tree-like structure around the core on each of the bonds, when n is greater than or equal to 1, a generation chain of a given generation being linked to

- a generation chain of the generation immediately below the given generation, or to the core when the given generation is 1, and to
- at least 2 generation chains of the generation immediately above the given generation, or optionally to at least one intermediate chain when the given generation is n,
a terminal group being attached to the end of each generation chain of generation n, or if appropriate to the end of each intermediate chain, or

• intermediate chains attached around the core on each of the bonds, when n is 0, a terminal group being attached to the end of each intermediate chain;
said terminal group being represented by the formula:

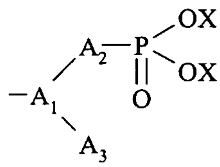

where
$A_1$ represents N; a P=Y group, where Y represents O, S, or any atom; an N-R group; or a C-R group; R representing H or a linear, branched or cyclic hydrocarbon chain with 1 to 16 members, optionally containing one or more heteroatoms, said heteroatoms being preferably chosen from oxygen, sulphur, nitrogen, phosphorus, silicon and/or one or more double or triple bonds, each of said members being optionally substituted by at least one substituent chosen from a hydroxyl group, an -NR'R" group, an alkoxy group of 1 to 16 carbon atoms, an alkyl group of 1 to 16 carbon atoms, a halogen atom, an $-NO_2$ group, a -CN group, a -CF3 group, an aryl group of 6 to 24 carbon atoms, an aralkyl group of 7 to 16 carbon atoms, R' and R" representing independently of each other H or an alkyl group of 1 to 16 carbon atoms, an aryl group of 6 to 24 carbon atoms or an aralkyl group of 7 to 16 carbon atoms;
$A_2$ represents a single bond or a linear, branched or cyclic hydrocarbon chain with 1 to 6 members, each of said members optionally containing one or more heteroatoms, said heteroatoms being preferably chosen from a sulphur, oxygen, phosphorus or nitrogen atom, more preferably nitrogen, and being optionally substituted by at least one substituent chosen from H, an alkyl group of 1 to 16 carbon atoms, a halogen, an $-NO_2$ group, an -NRR' group, a -CN group, a $-CF_3$ group, a hydroxyl group, an alkoxy group of 1 to 16 carbon atoms, an aryl or heteroaryl group of 1 to 24 carbon atoms, the heteroelement being preferably chosen from oxygen, nitrogen or sulphur, an aralkyl group of 7 to 16 carbon atoms, R and R' representing independently of each other H or a linear, branched or cyclic hydrocarbon chain with 1 to 16 members, optionally containing one or more heteroatoms, said heteroatoms being preferably chosen from oxygen, sulphur, nitrogen, phosphorus, silicon and/or one or more double or triple bonds, each of said members being optionally substituted by at least one substituent chosen from a hydroxyl group, an -NR"R"' group, an alkoxy group of 1 to 16 carbon atoms, an alkyl group of 1 to 16 carbon atoms, a halogen atom, an $-NO_2$ group, a -CN group, a $-CF_3$ group, an aryl group of 6 to 24 carbon atoms, an aralkyl group of 7 to 16 carbon atoms, R" and R"' representing independently of each other H or an alkyl group of 1 to 16 carbon atoms, an aryl group of 6 to 24 carbon atoms or an aralkyl group of 7 to 16 carbon atoms;
$A_3$ represents H, or a linear, branched or cyclic hydrocarbon chain with 1 to 6 members, each of said members being optionally chosen from a heteroatom, said heteroatom being preferably chosen from sulphur, nitrogen, phosphorus or silicon, more preferably nitrogen, each member being able to be optionally substituted by at least one group chosen from a hydroxyl group, an -NR"R"' group, an alkoxy group of 1 to 16 carbon atoms, an alkyl group of 1 to 16 carbon atoms, a halogen atom, an $-NO_2$ group, a -CN group, a $-CF_3$ group, an aryl group of 6 to 24 carbon atoms, an aralkyl group of 7 to 16 carbon atoms, R" and R'" representing independently of each other H or an alkyl group of 1 to 16 carbon atoms, an aryl group of 6 to 24 carbon atoms, or an aralkyl group of 7 to 16 carbon atoms or ,
in particular $A_3$ can represent

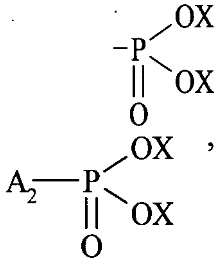

each $A_2$ being identical or different;
each OX, identical or different for each phosphonic group, represents OH, Oalkyl, where the alkyl group comprises from 1 to 16 carbon atoms, Oaryl, where the aryl group comprises from 6 to 24 carbon atoms, Oaralkyl, where the aralkyl group comprises from 7 to 24 carbon atoms, Oalkylaryl, where the alkylaryl group comprises from 7 to 24 carbon atoms, $OSiR'_1R'_2R'_3$, where $R'_1$, $R'_2$ and $R'_3$, identical or different, represent an alkyl group of 1 to 16 carbon atoms, or $O^-M^+$, where $M^+$ is a cation of elements of group IA, IB, IIA, IIB or IIIA, IIIB of the periodic table of the elements, preferably $M^+$ is chosen from the cations of the sodium, potassium, copper, calcium, barium, zinc, magnesium, lithium and aluminium atoms, or hydrocarbon groups of 1 to 100 carbon atoms, or nitrogenous groups of 0 to 100 carbon atoms, such as $NR_1R_2R_3R_4^+$, where, independently of each other $R_1$, $R_2$, $R_3$ and $R_4$ represent H or a linear, branched or cyclic hydrocarbon chain with 1 to 16 members, optionally containing one or more heteroatoms, said heteroatoms being preferably chosen from oxygen, sulphur, nitrogen, phosphorus, silicon and/or one or more double or triple bonds, each of said members being optionally substituted by at least one substituent chosen from a hydroxyl group, an -NR"R"' group, an alkoxy group of 1 to 16 carbon atoms, an alkyl group of 1 to 16 carbon atoms, a halogen atom, an $-NO_2$ group, a -CN group, a $-CF_3$ group, an aryl group of 6 to 24 carbon atoms, an aralkyl group of 7 to 16 carbon atoms, R" and R"' representing independently of each other H or an alkyl group of 1 to 16 carbon atoms, an aryl group of 6 to 24 carbon atoms or an aralkyl group of 7 to 16 carbon atoms;
said central core § representing a group comprising from 1 to 500 atoms, and optionally containing one or more heteroatoms, said heteroatoms being preferably chosen from oxygen, sulphur, nitrogen, phosphorus or silicon.

**5.** Use according to claim 4, in which the core § establishes m bonds with m identical linkage chains constituted:

• either by generation chains attached in a tree-like structure around the core on each of the bonds, the end of each chain generation furthest from the central core being attached either to a terminal group or to an intermediate chain, the end of each intermediate chain being attached to a terminal group,
• or by intermediate chains attached around the core on each of the bonds, the end of each intermediate chain being attached to a terminal group.

**6.** Use according to claim 4, in which the core establishes m-2 or m-1 bonds, m representing an integer from 3 to 20, in particular from 3 to 10 and more particularly from 3 to 8, with respectively m-2 or m-1 identical linkage chains constituted:

• either by generation chains attached in a tree-like structure around the core on each of the bonds, the end of each chain generation furthest from the central core being attached either to a terminal group or to an intermediate chain, and the end of each intermediate chain being attached to a terminal group,
• or by intermediate chains attached around the core on each of the bonds, the end of each intermediate chain being attached to a terminal group;
the 1 or 2 remaining bonds being attached to linkage groups, identical or different, optionally linked together, in particular by means of a covalent bond, constituted:

- either by part of the linkage chains defmed above,
- or by a hydrogen atom,
- or by hydrocarbon groups comprising from 1 to 500 carbon atoms, said hydrocarbon groups being in particular constituted by H or by a linear, branched or cyclic hydrocarbon chain with 1 to 200 members, optionally containing one or more double or triple bonds, each of said members being optionally chosen from a heteroatom, said heteroatom being preferably chosen from a nitrogen, oxygen, phosphorus, silicon or sulphur atom, an aryl group of 6 to 24 carbon atoms, a heteroaryl group of 1 to 24 carbon atoms, a

carboxyl group, a >C=NR group, each member being able to be optionally substituted by at least one substituent chosen from a hydroxyl group, an -NR''R''' group, an alkoxy group of 1 to 16 carbon atoms, an alkyl group of 1 to 16 carbon atoms, a halogen atom, an $-NO_2$ group, a -CN group, a $-CF_3$ group, an aryl group of 6 to 24 carbon atoms, an aralkyl group of 7 to 16 carbon atoms, R'' and R''' representing independently of each other H or an alkyl group of 1 to 16 carbon atoms, an aryl group of 6 to 24 carbon atoms or an aralkyl group of 7 to 16 carbon atoms, the first member of said hydrocarbon chain preferably being oxygen or nitrogen.

**7.** Use according to one of claims 4 to 6, in which the generation chains are chosen from any linear, branched or cyclic hydrocarbon chain with 1 to 12 members, optionally containing one or more double or triple bonds, each of said members being optionally chosen from a heteroatom, said heteroatom being preferably chosen from nitrogen, oxygen, sulphur, phosphorus or silicon, an aryl group of 6 to 24 carbon atoms, a heteroaryl group of 1 to 24 carbon atoms, the heteroelement being preferably chosen from oxygen, nitrogen or sulphur, a carboxyl group, a >C=NR group, each member being optionally substituted by at least one substituent chosen from an alkyl group of 1 to 16 carbon atoms, a halogen atom, an $-NO_2$ group, an -NRR' group, a -CN group, a $-CF_3$ group, a hydroxyl group, an alkoxy group of 1 to 16 carbon atoms, an aryl group of 6 to 24 carbon atoms, or an aralkyl group of 7 to 16 carbon atoms, R and R' representing independently of each other H or an alkyl group of 1 to 16 carbon atoms, an aryl group of 6 to 24 carbon atoms, or an aralkyl group of 7 to 16 carbon atoms.

**8.** Use according to one of claims 4 to 7, in which the intermediate chains are chosen from the groups corresponding to the formula:

-J-K-L-

where

- J represents an oxygen or sulphur atom, or an -NR- group;
- K represents an aryl group of 6 to 24 carbon atoms, a heteroaryl group of 1 to 24 carbon atoms, the heteroelement being preferably chosen from oxygen, nitrogen or sulphur, an alkyl group of 1 to 16 carbon atoms, each being able to be optionally substituted by a halogen atom or an $-NO_2$, -NRR', -CN, $-CF_3$, -OH group, an alkyl group of 1 to 16 carbon atoms, an aryl group of 6 to 24 carbon atoms, an aralkyl group of 7 to 16 carbon atoms;
- L represents a linear, branched or cyclic hydrocarbon chain with 0 to 10 members, optionally containing one or more double or triple bonds, each of said members optionally being able to be a heteroatom, said heteroatom being preferably chosen from oxygen, sulphur, nitrogen, phosphorus, silicon, each member being able to be optionally substituted by at least one substituent chosen from an alkyl group of 1 to 16 carbon atoms, a halogen, an oxygen atom, $-NO_2$, -NRR', -CN, $-CF_3$, -OH, an alkoxy group of 1 to 16 carbon atoms, an aryl group of 6 to 24 carbon atoms, an aralkyl group of 7 to 16 carbon atoms;

R and R' representing independently of each other H or an alkyl group of 1 to 16 carbon atoms, an aryl group of 6 to 24 carbon atoms, or an aralkyl group of 7 to 16 carbon atoms.

**9.** Use according to one of claims 4 to 8, in which the core is chosen from:

- a nitrogen or silicon atom;
- a group of formula

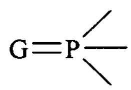,

in which G represents an oxygen, nitrogen, sulphur, selenium, tellurium atom or an =NR group, R representing H or an alkyl group of 1 to 16 carbon atoms, an aryl group of 6 to 24 carbon atoms, or an aralkyl group of 7 to 16 carbon atoms, such as the thiophosphoryl group of formula

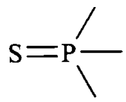

;

- a bis-phenyloxy group of formula

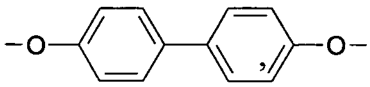

- a 1,2-diamino-ethane group of formula

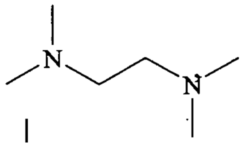

- a 1,4-diamino-butane group of formula

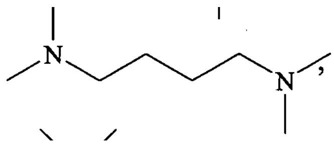

- a cyclotriphosphazene group of formula

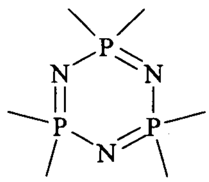

,

also denoted $N_3P_3$ or $P_3N_3$,
- a cyclotetraphosphazene group of formula

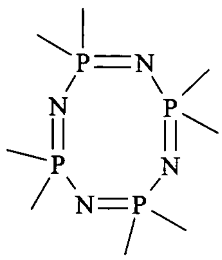

,

also denoted $N_4P_4$ or $P_4N_4$.

**10.** Use according to one of claims 1 to 9, of dendrimers of structure PAMAM, DAB or PMMH.

**11.** Use according to one of claims 4 to 5 and 7 to 9, of dendrimers with monophosphonic or bisphosphonic terminations corresponding to the following general formula (1a):

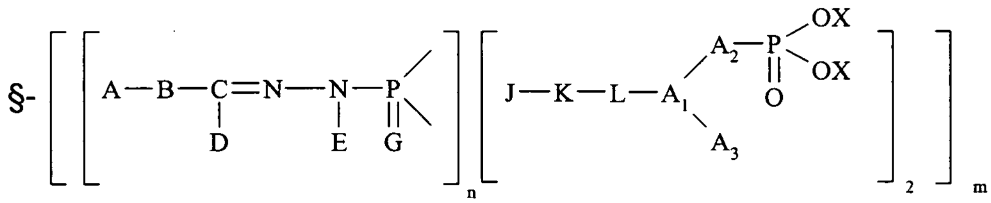

(1a)

where n represents an integer from 0 to 3, namely:

- when n = 0, formula (1a) corresponds to the following formula (2a),

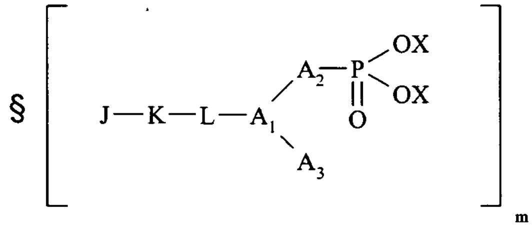

(2a)

- when n = 1, formula (1a) corresponds to the following formula (3 a),

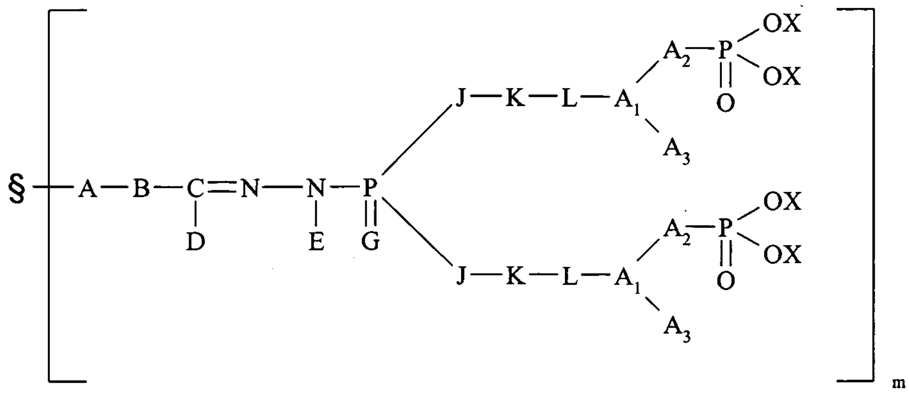

(3a)

- when n = 2, formula (1a) corresponds to the following formula (4a),

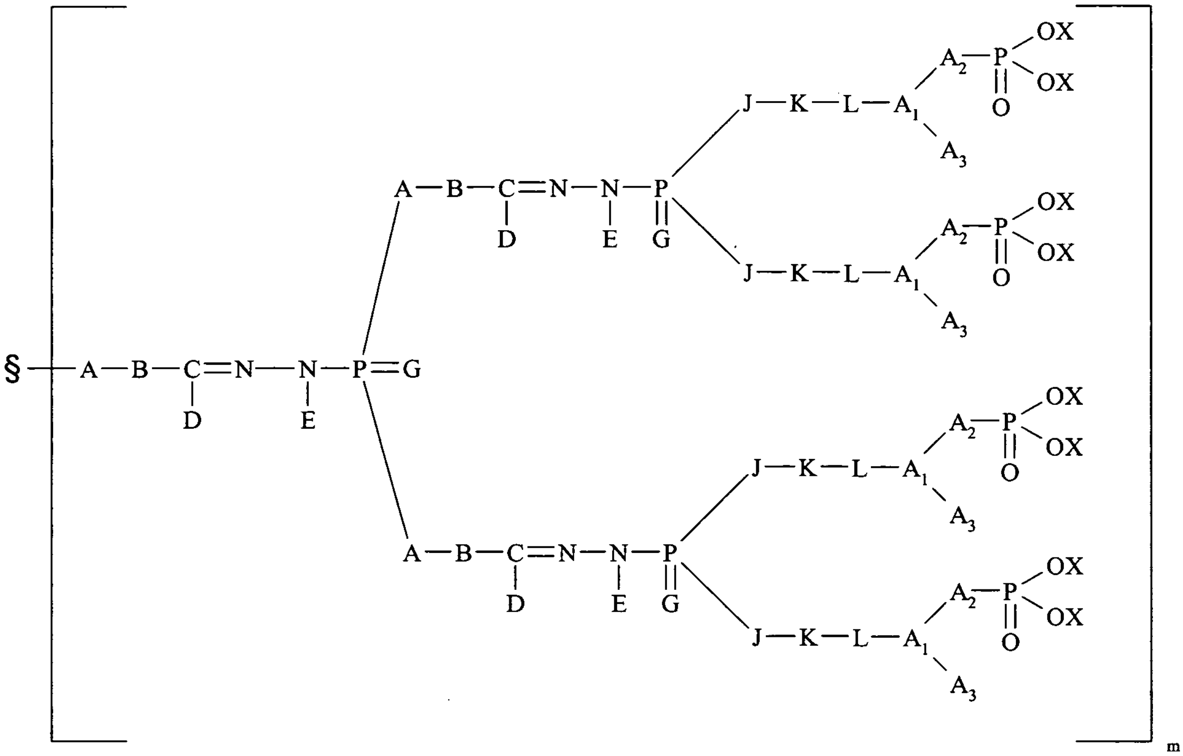

**(4a)**

- and when n = 3, formula (1a) corresponds to the following formula (5a),

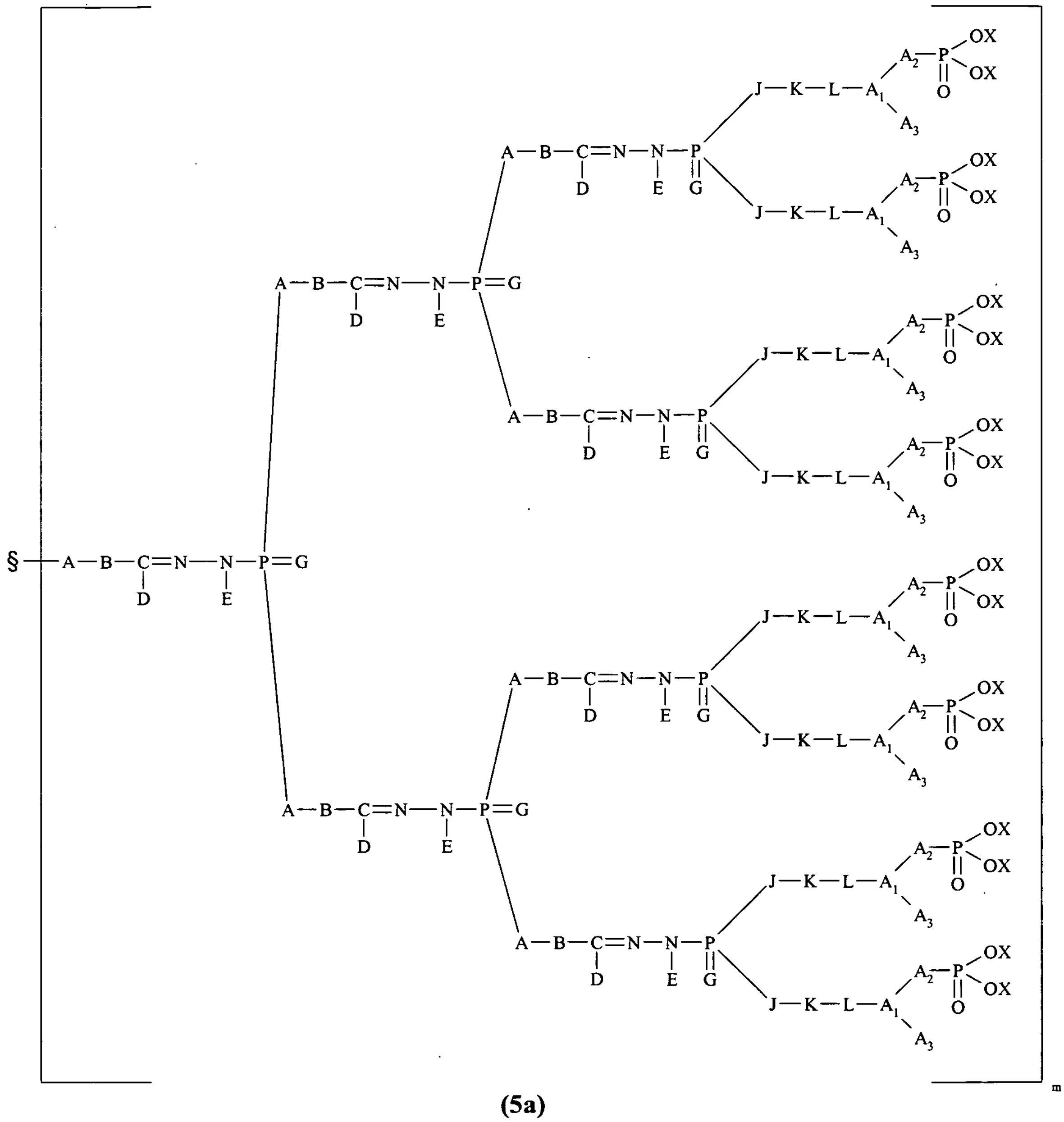

**(5a)**

and in which formulae:

- the central core § is chosen from the following groups:

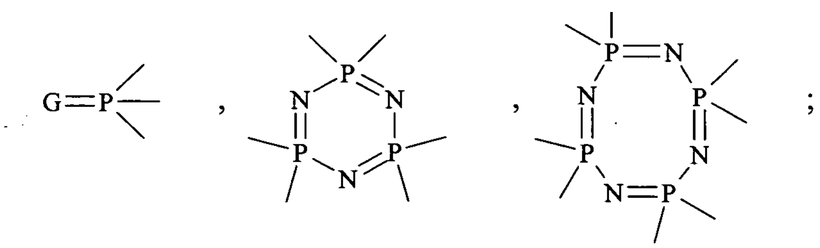

- m represents 3, 6 or 8;
- the generation chain corresponds to the formula:

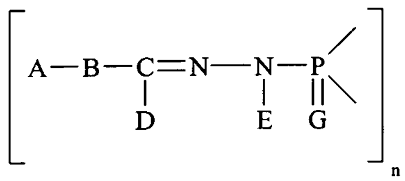

where
- A represents an oxygen, sulphur, phosphorus atom or an -NR- group;
- B represents an aryl group of 6 to 24 carbon atoms, a heteroaryl group of 1 to 24 carbon atoms, an alkyl group of 1 to 16 carbon atoms, each being able to be optionally substituted by a halogen atom or an $-NO_2$, -NRR', -CN, $-CF_3$, -OH group, an alkyl group of 1 to 16 carbon atoms, an aryl group of 6 to 24 carbon atoms, an aralkyl group of 7 to 16 carbon atoms;
- D represents a hydrogen atom, an alkyl group of 1 to 16 carbon atoms, an alkoxy group of 1 to 16 carbon atoms, an aryl group of 6 to 24 carbon atoms, an aralkyl group of 7 to 16 carbon atoms, each being able to be optionally substituted by a halogen atom or an $-NO_2$, -NRR', -CN, $-CF_3$, -OH group, an alkyl group of 1 to 16 carbon atoms, an aryl group of 6 to 24 carbon atoms, an aralkyl group of 7 to 16 carbon atoms;
- E represents a hydrogen atom, an alkyl group of 1 to 16 carbon atoms, an alkoxy group of 1 to 16 carbon atoms, an aryl group of 6 to 24 carbon atoms, an aralkyl group of 7 to 16 carbon atoms, each being able to be optionally substituted by a halogen atom or an - $NO_2$, -NRR', -CN, $-CF_3$, -OH group, an alkyl group of 1 to 16 carbon atoms, an aryl group of 6 to 24 carbon atoms, an aralkyl group of 7 to 16 carbon atoms;
- G represents an oxygen, nitrogen, sulphur, selenium, tellurium atom or an =NR group; R and R' representing independently of each other H or an alkyl group of 1 to 16 carbon atoms, an aryl group of 6 to 24 carbon atoms, or an aralkyl group of 7 to 16 carbon atoms;

• the intermediate chain corresponds to the formula:

-J-K-L-

where

- J represents an oxygen, sulphur atom, or an -NR- group;
- K represents an aryl group of 6 to 24 carbon atoms, a heteroaryl group of 1 to 24 carbon atoms, the heteroelement being preferably chosen from oxygen, nitrogen or sulphur, an alkyl group of 1 to 16 carbon atoms, each being able to be optionally substituted by a halogen atom or an $-NO_2$, -NRR', -CN, $-CF_3$, -OH group, an alkyl group of 1 to 16 carbon atoms, an aryl group of 6 to 24 carbon atoms, an aralkyl group of 7 to 16 carbon atoms;
- L represents a linear, branched or cyclic hydrocarbon chain with 0 to 10 members, optionally containing one or more double or triple bonds, each of said members being able to be optionally a heteroatom, said heteroatom being preferably chosen from oxygen, sulphur, nitrogen, phosphorus, silicon, each member being able to be optionally substituted by at least one substituent chosen from an alkyl group of 1 to 16 carbon atoms, a halogen, an oxygen atom, $-NO_2$, -NRR', -CN, $-CF_3$, -OH, an alkoxy group of 1 to 16 carbon atoms, an aryl group of 6 to 24 carbon atoms, an aralkyl group of 7 to 16 carbon atoms;
R and R' representing independently of each other H or an alkyl group of 1 to 16 carbon atoms, an aryl group of 6 to 24 carbon atoms, or an aralkyl group of 7 to 16 carbon atoms;

• the terminal group corresponds to the formula:

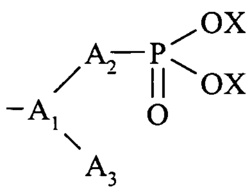

where $A_1$, $A_3$ and X have been defined previously, each X being identical or different.

**12.** Use according to claim 11, of a dendrimer of general formula (1a) in which $A_3$ represents:

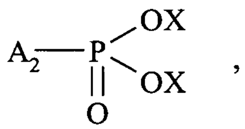

said general formula (1a) then corresponding to the following general formula (1):

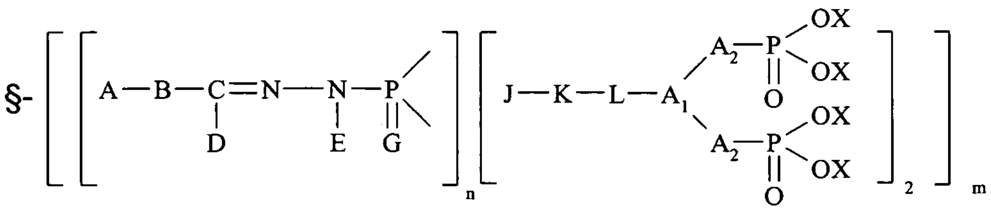

(1)

§, A, B, C, D, E, G, J, K, L, $A_1$, $A_2$, X, m and n being as defined in claim 11.

**13.** Use according to claim 12, of a dendrimer of general formula (1) of structure PMMH, in which

§ represents

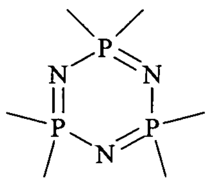

;

m represents 6;
n represents 0, 1, or 2;
A represents an oxygen atom;
B represents a benzene group;
D represents hydrogen;
E represents a methyl group;
G represents a sulphur atom;
J represents an oxygen atom;
K represents a benzene group;
L represents a non-substituted linear saturated hydrocarbon chain with two carbon atoms;
$A_1$ represents a nitrogen atom;
$A_2$ represents a $CH_2$ group;
X represents a methyl group, or a hydrogen or sodium atom;

said dendrimer being designated GCn, n being defined above.

**14.** Use according to one of claims 4, 5 and 7 to 13, of the compounds of the following formulae:

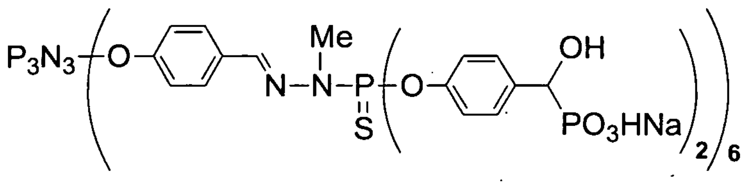

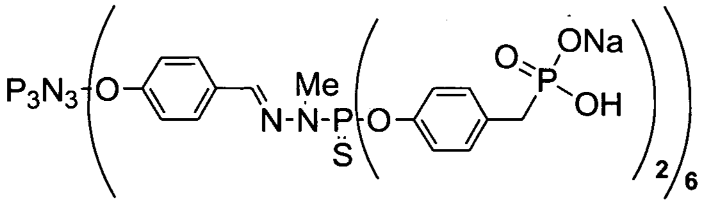

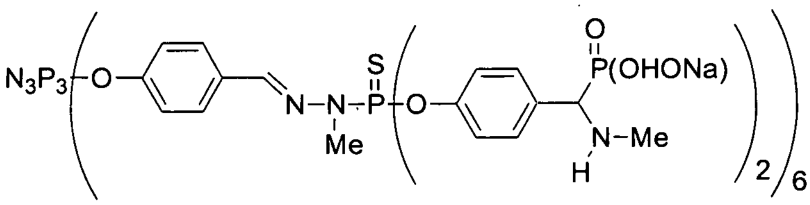

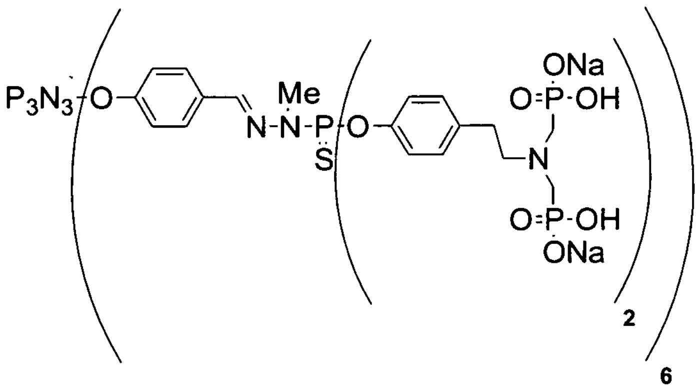

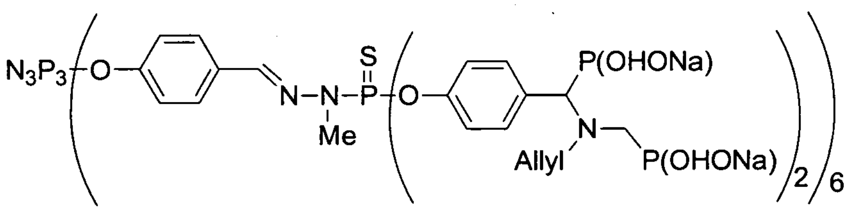

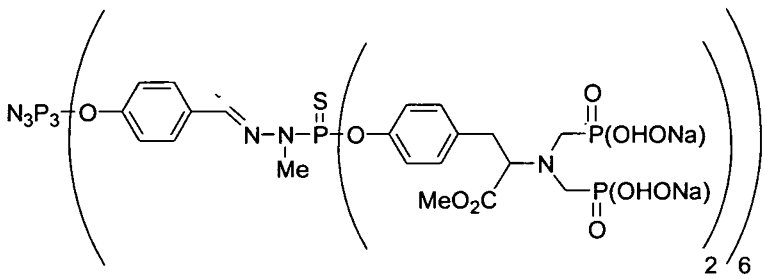

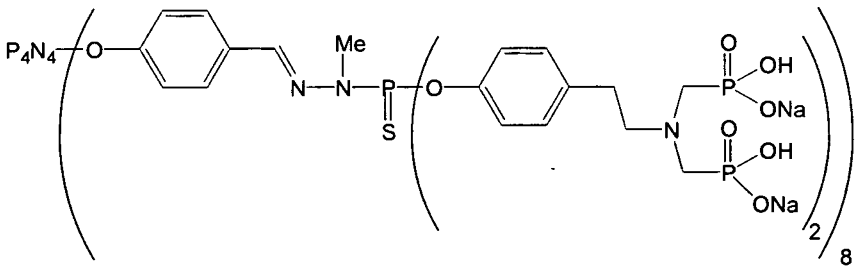

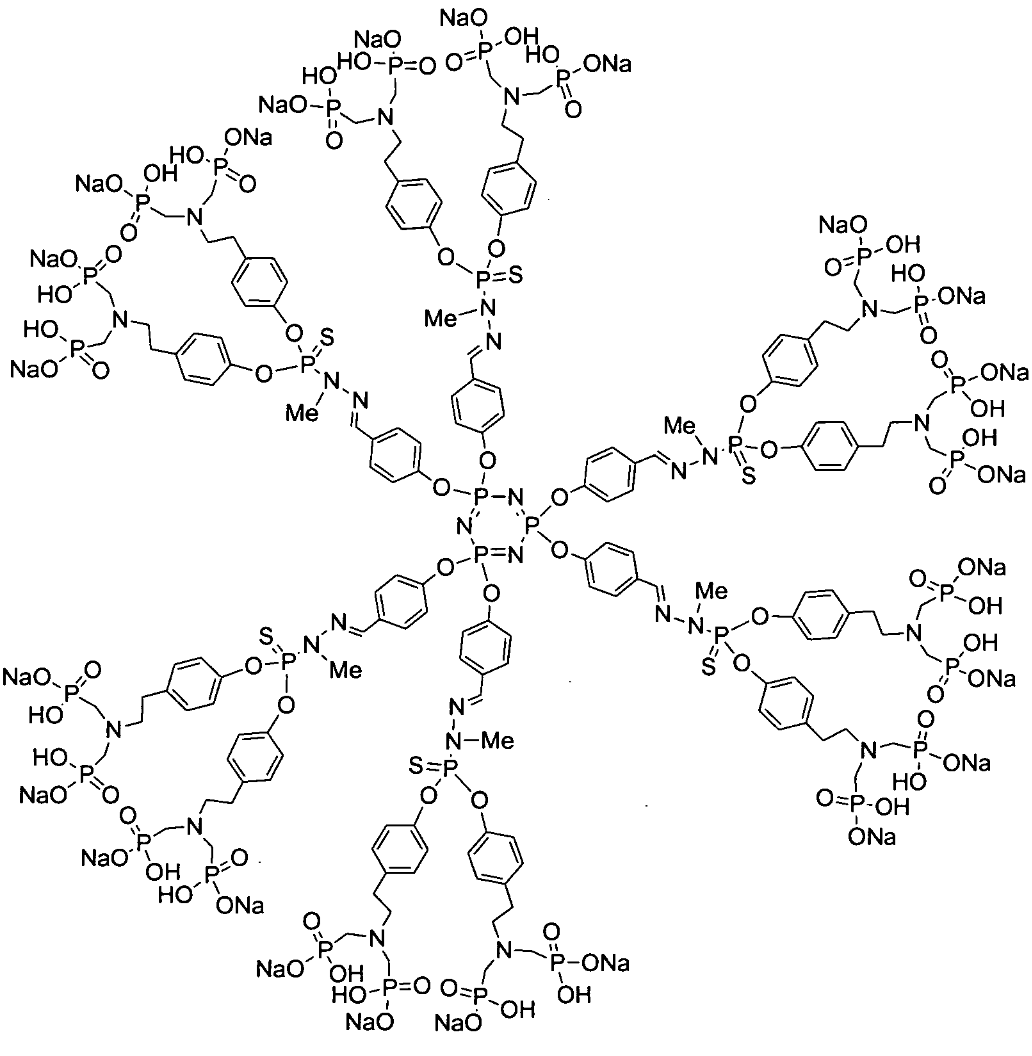

**15.** Use according to one of claims 4 and 6 to 9, of dendrimers with bisphosphonic terminations corresponding to the following general formula (7):

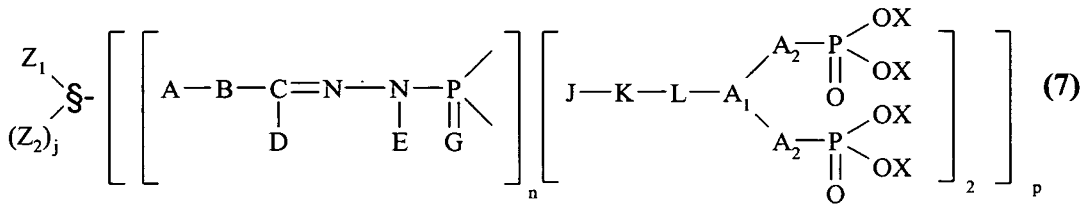

where n represents an integer from 0 to 3, m represents 3, 6 or 8, p represents m - 1 or m - 2, and j represents 0 when p represents m - 1 and 1 when p represents m - 2, namely:

- when p = m - 1, formula (7) corresponds to the following formula (8):
or of the following formulae GC1:

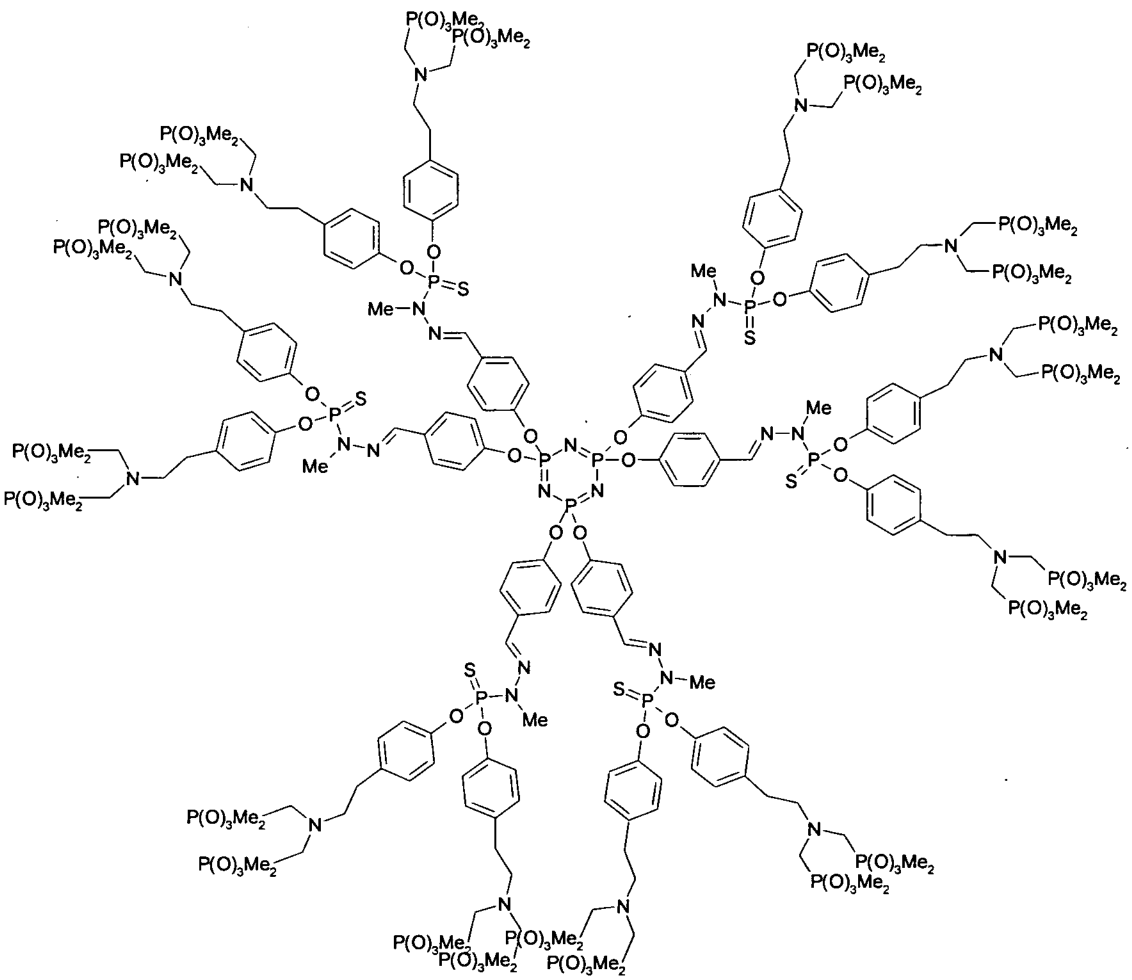

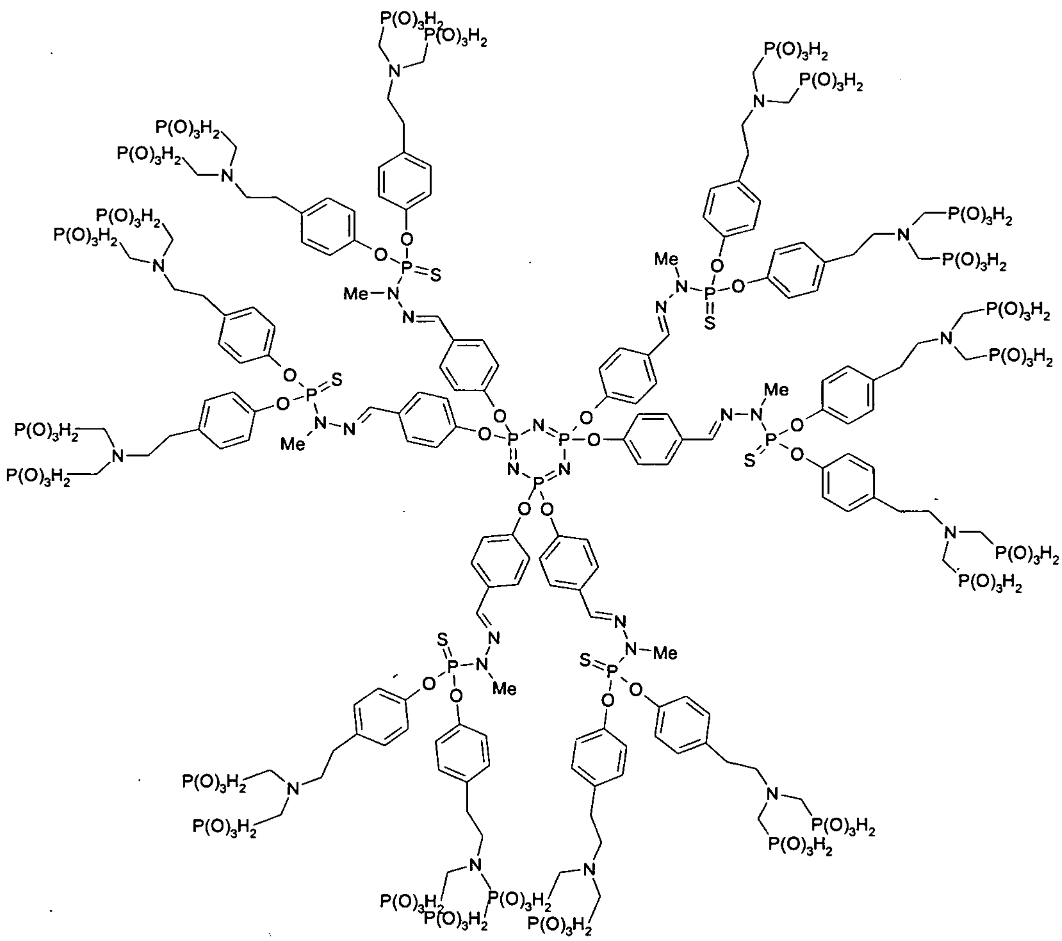

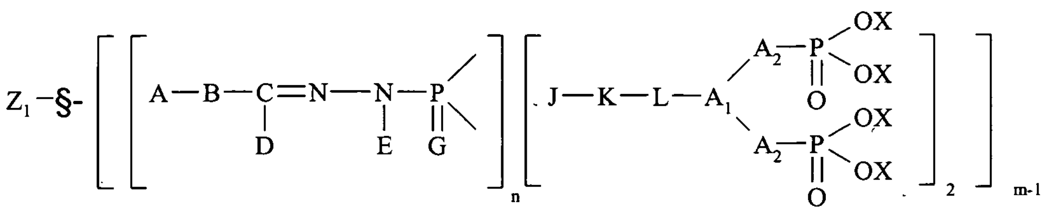

(8)

- when p = m - 2, formula (7) corresponds to the following formula (9)

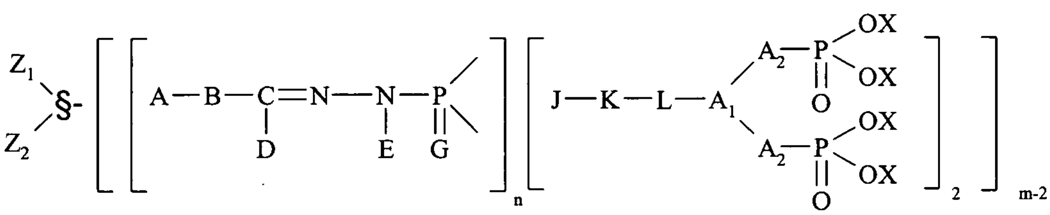

(9)

and in which formulae:

- the central core § is chosen from the following groups:

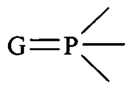 , 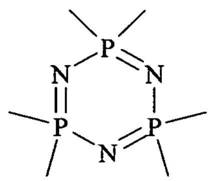 , 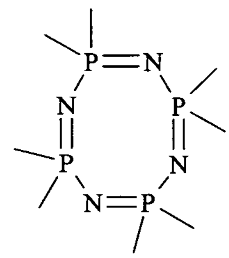 ;

• the generation chain corresponds to the formula:

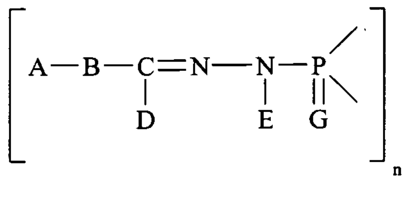

where
- A represents an oxygen, sulphur, phosphorus atom or an -NR- group;
- B represents an aryl group of 6 to 24 carbon atoms, a heteroaryl group of 1 to 24 carbon atoms, an alkyl group of 1 to 16 carbon atoms, each being able to be optionally substituted by a halogen atom or an $-NO_2$, -NRR', -CN, $-CF_3$, -OH group, an alkyl group of 1 to 16 carbon atoms, an aryl group of 6 to 24 carbon atoms, an aralkyl group of 7 to 16 carbon atoms;
- D represents a hydrogen atom, an alkyl group of 1 to 16 carbon atoms, an alkoxy group of 1 to 16 carbon atoms, an aryl group of 6 to 24 carbon atoms, an aralkyl group of 7 to 16 carbon atoms, each being able to be optionally substituted by a halogen atom or an $-NO_2$, -NRR', -CN, $-CF_3$, -OH group, an alkyl group of 1 to 16 carbon atoms, an aryl group of 6 to 24 carbon atoms, an aralkyl group of 7 to 16 carbon atoms;
- E represents a hydrogen atom, an alkyl group of 1 to 16 carbon atoms, an alkoxy group of 1 to 16 carbon atoms, an aryl group of 6 to 24 carbon atoms, an aralkyl group of 7 to 16 carbon atoms, each being able to be optionally substituted by a halogen atom or an $-NO_2$, -NRR', -CN, $-CF_3$, -OH group, an alkyl group of 1 to 16 carbon atoms, an aryl group of 6 to 24 carbon atoms, an aralkyl group of 7 to 16 carbon atoms;
- G represents an oxygen, nitrogen, sulphur, selenium, tellurium atom or an =NR group; R and R' representing independently of each other H or an alkyl group of 1 to 16 carbon atoms, an aryl group of 6 to 24 carbon atoms, or an aralkyl group of 7 to 16 carbon atoms;

• the intermediate chain corresponds to the formula:

-J-K-L-

where

- J represents an oxygen, sulphur atom, or an -NR- group;
- K represents an aryl group of 6 to 24 carbon atoms, a heteroaryl group of 1 to 24 carbon atoms, the heteroelement preferably being chosen from oxygen, nitrogen or sulphur, an alkyl group of 1 to 16 carbon atoms, each being able to be optionally substituted by a halogen atom or an $-NO_2$, -NRR', -CN, $-CF_3$, -OH group, an alkyl group of 1 to 16 carbon atoms, an aryl group of 6 to 24 carbon atoms, an aralkyl group of 7 to 16 carbon atoms;
- L represents a linear, branched or cyclic hydrocarbon chain with 0 to 10 members, optionally containing one or more double or triple bonds, each of said members optionally being a heteroatom, said heteroatom preferably being chosen from oxygen, sulphur, nitrogen, phosphorus, silicon, each member being able to be optionally substituted by at least one substituent chosen from an alkyl group of 1 to 16 carbon atoms, a halogen, an oxygen atom, $-NO_2$, -NRR', -CN, $-CF_3$, -OH, an alkoxy group of 1 to 16 carbon atoms, an aryl group of 6 to 24 carbon atoms, an aralkyl group of 7 to 16 carbon atoms;
R and R' representing independently of each other H or an alkyl group of 1 to 16 carbon atoms, an aryl group of 6 to 24 carbon atoms, or an aralkyl group of 7 to 16 carbon atoms;

• the terminal group corresponds to the formula:

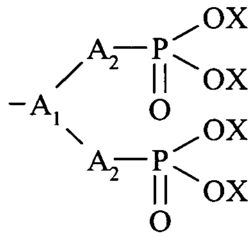

where A1, A2 and X have been defined previously, each X being identical or different;

$Z_1$ and $Z_2$ being identical or different, optionally linked together, in particular by a covalent bond, and representing H or a linear, branched or cyclic hydrocarbon chain with 1 to 16 members, optionally containing one or more double or triple bonds, each of said members optionally being chosen from a heteroatom, said heteroatom preferably being chosen from a nitrogen, oxygen, phosphorus, silicon or sulphur atom, an aryl group of 6 to 24 carbon atoms, a heteroaryl group of 1 to 24 carbon atoms, a carboxyl group, a >C=NR group, each member being able to be optionally substituted by at least one substituent chosen from a hydroxyl group, an -NR"R"' group, an alkoxy group of 1 to 16 carbon atoms, an alkyl group of 1 to 16 carbon atoms, a halogen atom, an $-NO_2$ group, a -CN group, a $-CF_3$ group, an aryl group of 6 to 24 carbon atoms, an aralkyl group of 7 to 16 carbon atoms, R" and R"' representing independently of each other H or an alkyl group of 1 to 16 carbon atoms, an aryl group of 6 to 24 carbon atoms or an aralkyl group of 7 to 16 carbon atoms, the first member of said hydrocarbon chain preferably being oxygen or nitrogen.

**16.** Use according to claim 15, of a dendrimer of general formula (8) of structure PMMH, in which

§ represents

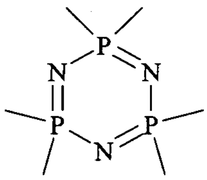 ;

m represents 6;
p represents 5;
n represents 0, 1, or 2;
A represents an oxygen atom;
B represents a benzene group;
D represents hydrogen;
E represents a methyl group;
G represents a sulphur atom;
J represents an oxygen atom;
K represents a benzene group;
L represents a non substituted saturated linear hydrocarbon chain with two carbon atoms;
$A_1$ represents a nitrogen atom;
$A_2$ represents a $CH_2$ group;
X represents a methyl group, or a hydrogen or sodium atom;
$Z_1$ represents a phenyloxy group;

said dendrimer being designated GCn', n being defined above.

**17.** Use according to one of claims 4, 6 to 9 or 15 to 16,

- of the compounds of the following formulae:

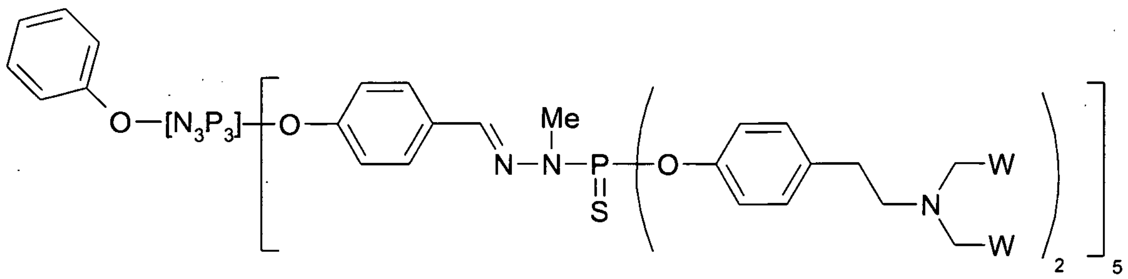

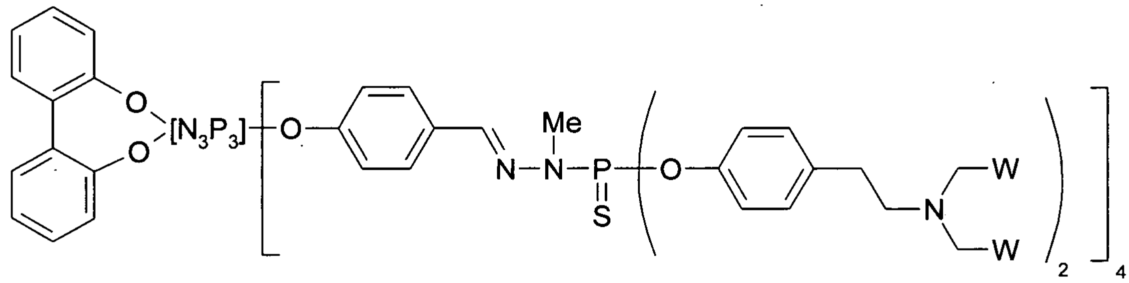

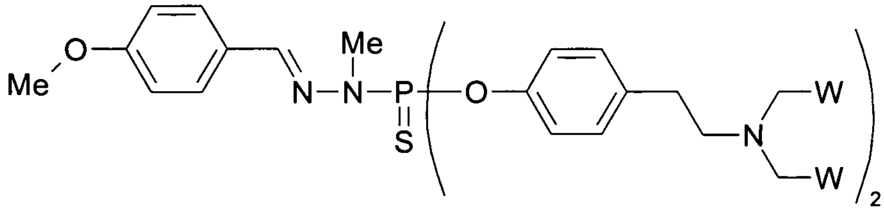

in which W represents $PO_3Me_2$, $PO_3HNa$, $PO_3H_2$ the said compounds corresponding, in particular, to compound GC1' of the following formula (10):

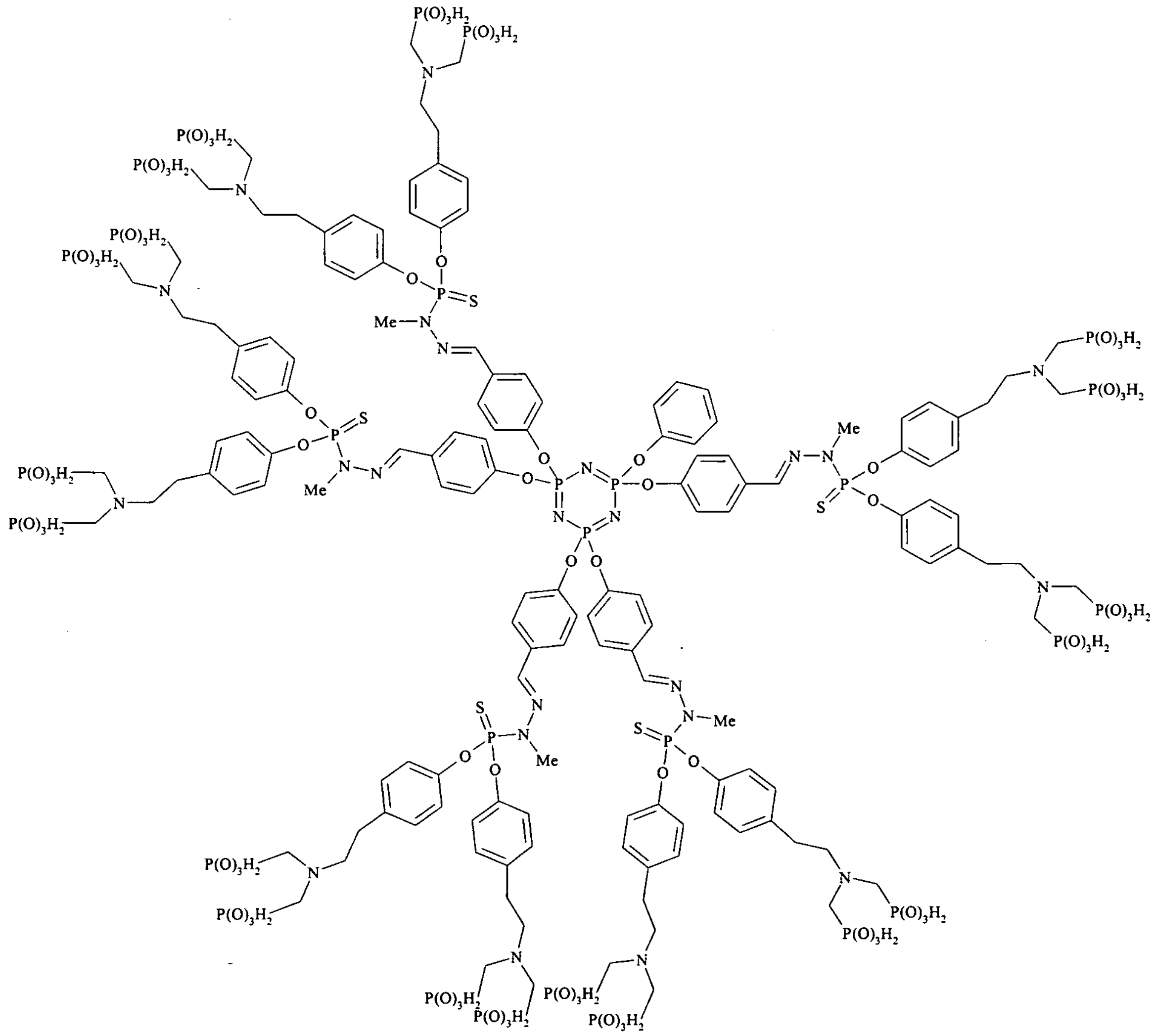

(10)

- or the compounds of the following formula:

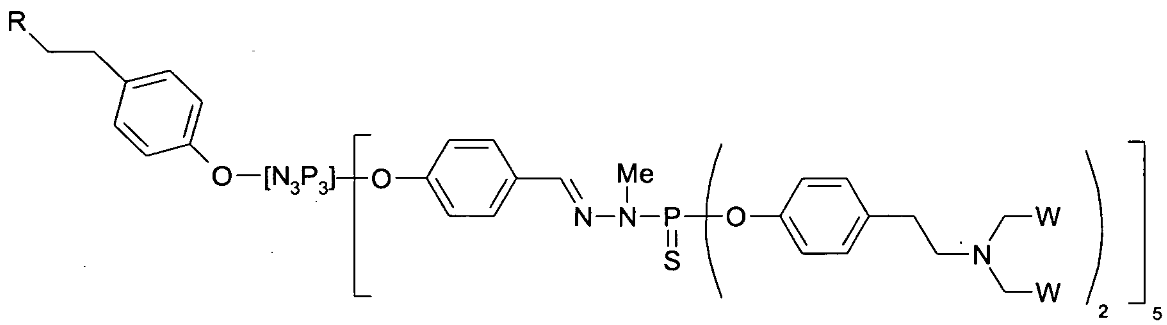

in which W represents $PO_3Me_2$, $PO_3HNa$, or $PO_3H_2$ and R represents a fluorescent group chosen from:

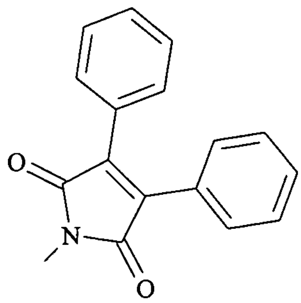 , 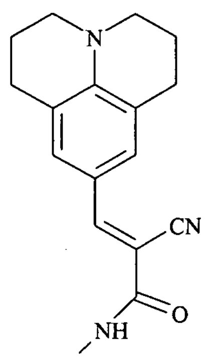 ,

said compounds corresponding in particular to the compounds of the following formulae:

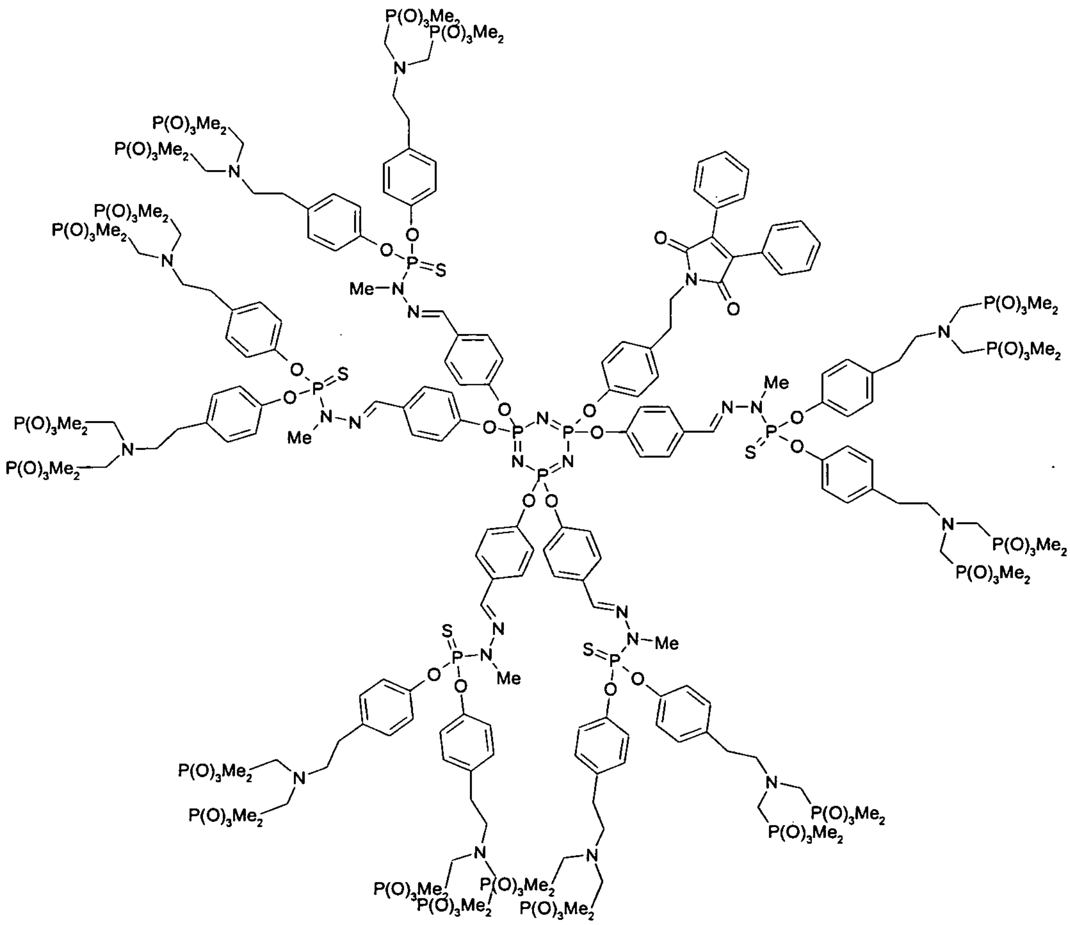

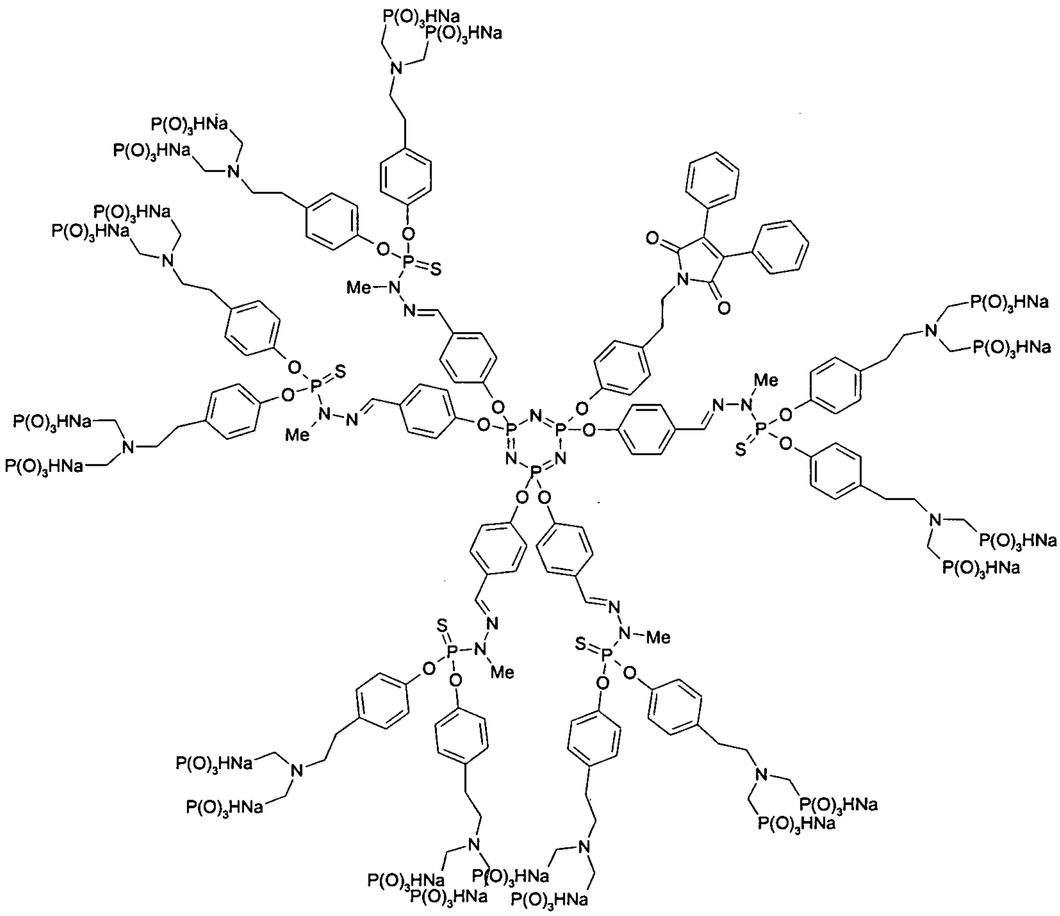

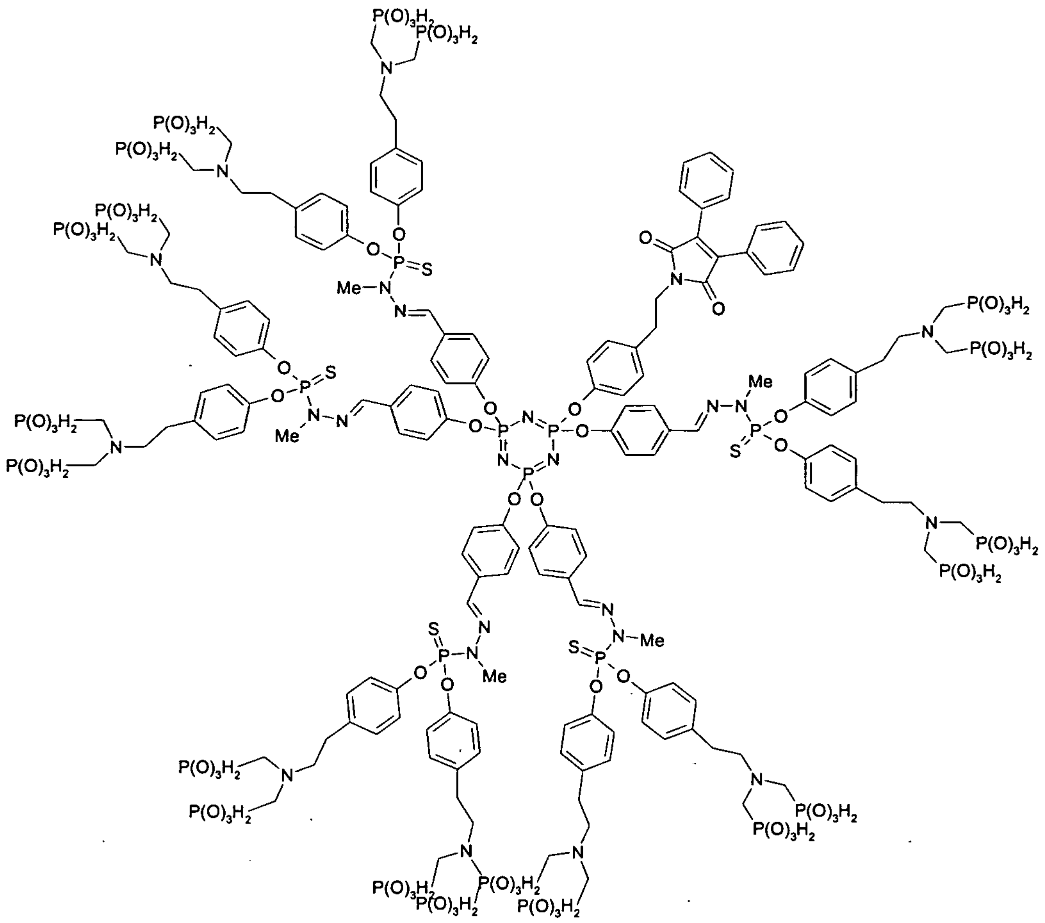

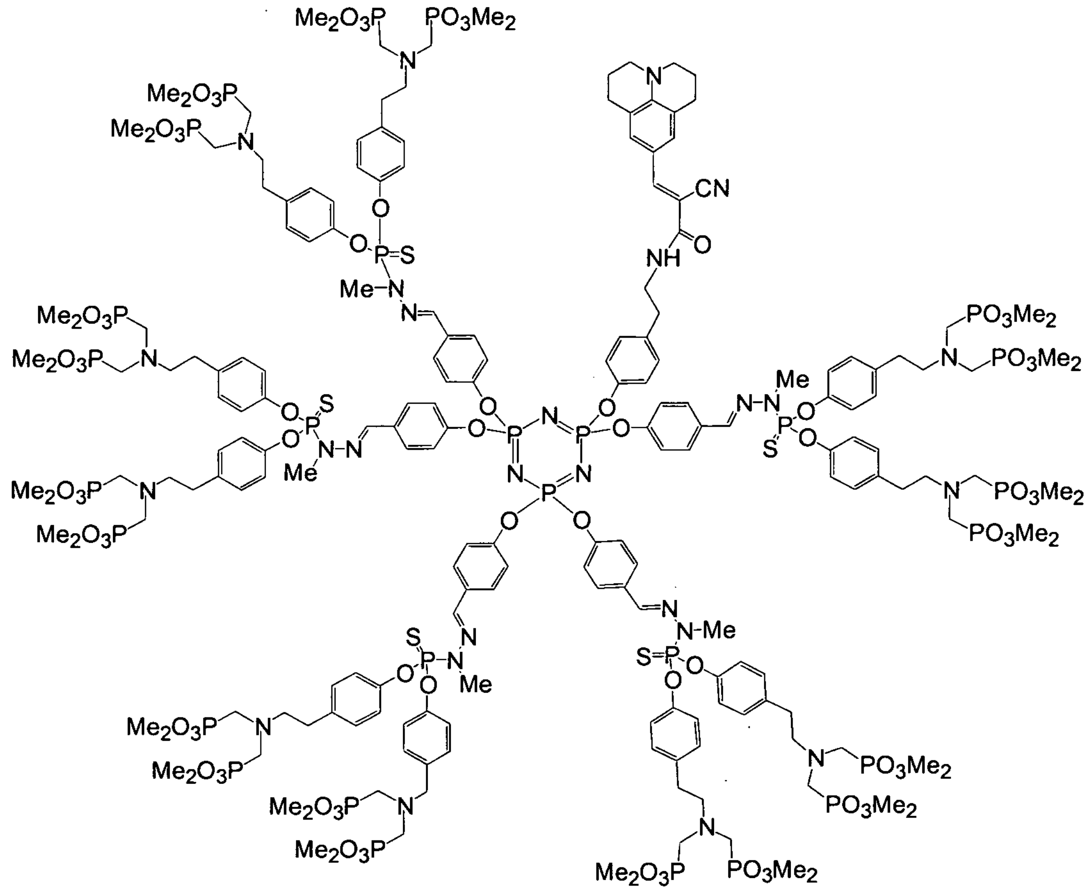

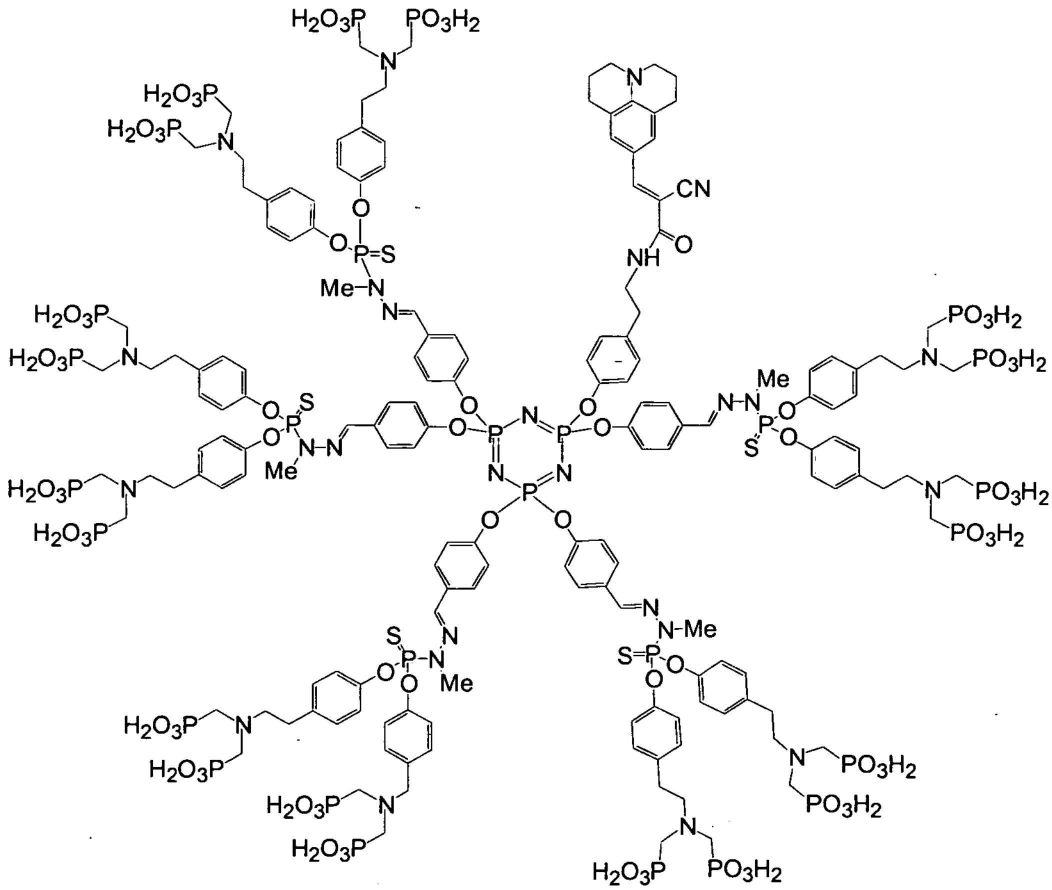

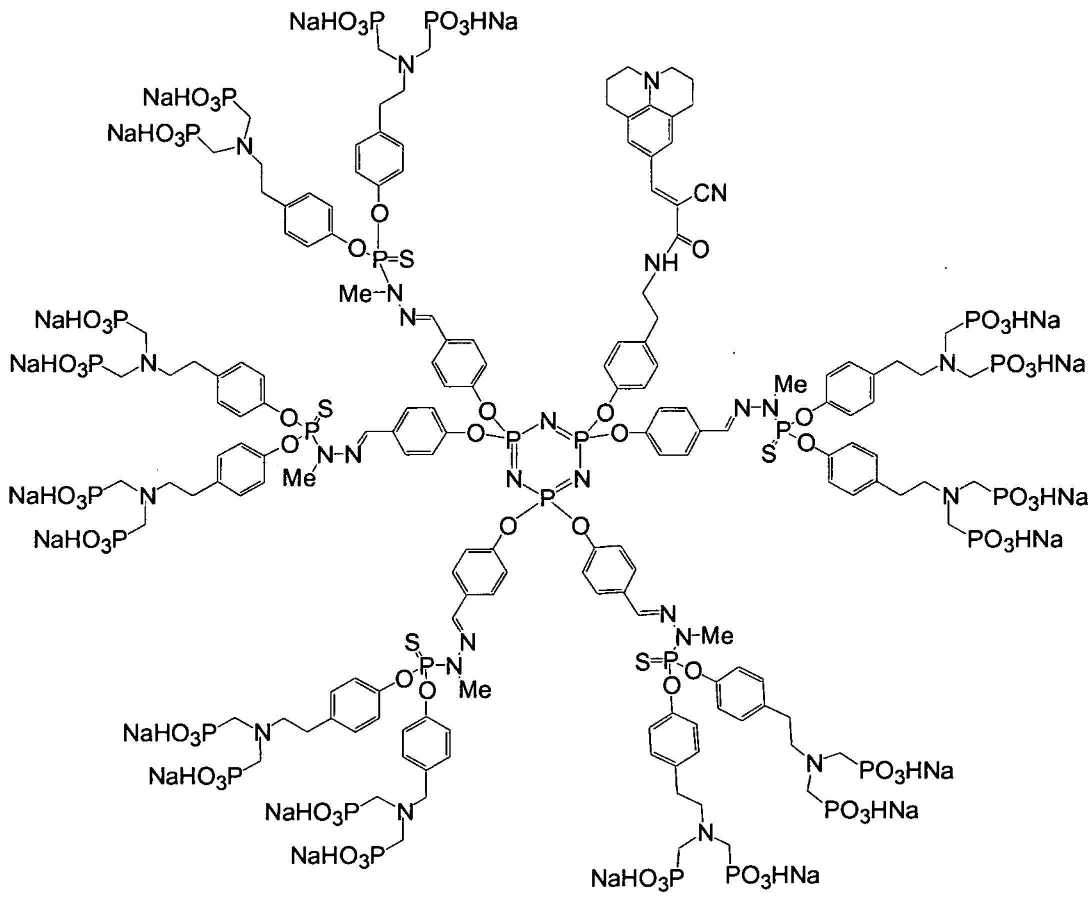

**18.** Use according to one of claims 1 to 17, in which the cells of the lymphoid line expressing the receptor NKG2D are derived from NK cells, $CD8^+$ αβ T lymphocytes or γδ T lymphocytes, in particular ofNK cells.

**19.** Use according to one of claims 1 to 17, in which cells of the monocyte line in culture are activated by the dendrimers with monophosphonic or bisphosphonic terminations, the activation of the cells of the monocyte line corresponding in particular:

- to an increase in the size of the activated cells compared with the non-activated cells, and/or
- to a reduction in the expression of MHC class I and class II molecules, or of the molecule CD14 compared with the non-activated cells, and/or
- to an increase in the nuclear translocation of the factor NFκB.

**20.** Use according to claim 19, in which the cells of the monocyte line in culture exhibit reduced apoptosis compared with cells of the monocyte line cultured in the absence of dendrimers with monophosphonic or bisphosphonic terminations.

**21.** Cell culture medium, **characterized in that** it contains at least one dendrimeric compound with monophosphonic or bisphosphonic terminations.

**22.** Culture medium according to claim 21, **characterized in that** it comprises moreover at least one growth and/or NK cell activation factor.

**23.** Culture medium according to claim 21 or 22, **characterized in that** it comprises at least one interleukin chosen from the group comprising: IL-2, IL-7, IL-12, IL-15, IL-18, or IL-21.

**24.** Culture medium according to one of claims 21 to 23, **characterized in that** it comprises at least one dendrimeric compound with monophosphonic or bisphosphonic terminations in combination with IL-2.

**25.** Culture medium according to one of claims 21 to 24, **characterized in that** the dendrimeric compound corresponds to the dendrimer as defined in one of claims 4 to 17.

**26.** Culture medium according to one of claims 21 to 24, **characterized in that** the dendrimeric compound corresponds to the dendrimers defined in claim 14, or to GC0 or GC2, in particular to GC1.

**27.** Culture medium according to one of claims 21 to 26, **characterized in that** it comprises the compound GC at a concentration of approximately 10 to approximately 50 μM, in particular approximately 20 μM, in combination with IL-2 at a concentration of approximately 100 to approximately 1000 units per ml, corresponding to a concentration of approximately 4 to approximately 40 ng per ml, in particular approximately 400 units per ml, corresponding to approximately 16 ng/ml, and **in that** the IL-2 corresponds to human recombinant IL-2.

**28.** Process for the preparation of cell compositions enriched with cells of the lymphoid line expressing the receptor NKG2D, in particular NK cells, **characterized in that** it comprises a stage of bringing a biological sample together with a dendrimer with monophosphonic or bisphosphonic terminations.

**29.** Process according to claim 28 **characterized in that** it comprises cells of the lymphoid line and/or cells of the monocyte line.

**30.** Process according to claim 28 or 29, **characterized in that** the biological sample is constituted by human blood, in particular by a mononuclear cell fraction of a sample of human peripheral blood.

**31.** Process according to one of claims 28 to 30, **characterized in that** the dendrimer with monophosphonic or bisphosphonic terminations corresponds to the dendrimers as defined in one of claims 4 to 17.

**32.** Process according to one of claims 28 to 31, **characterized in that** the dendrimer with monophosphonic or bisphosphonic terminations corresponds to the dendrimers defined in claim 14, or to GC0 or GC2, in particular to GC1.

**33.** Cell compositions enriched with cells of the lymphoid line expressing the receptor NKG2D, in particular NK cells, as obtained by the process according to one of claims 28 to 32.

**34.** Cell compositions according to claim 33, **characterized in that** they also comprise a dendrimer with monophosphonic or bisphosphonic terminations, in particular GC1.

**35.** Process for the preparation of activated monocytes or of cell compositions comprising activated monocytes, **characterized in that** it comprises a stage of bringing a biological sample comprising monocytes together with a dendrimer with monophosphonic or bisphosphonic terminations.

**36.** Process according to claim 35, **characterized in that** the biological sample is constituted by human blood, in particular by a mononuclear cell fraction of a sample of human peripheral blood.

**37.** Process according to claim 35 or 36, **characterized in that** the dendrimer with monophosphonic or bisphosphonic terminations corresponds to the dendrimers as defined in one of claims 3 to 16.

**38.** Process according to one of claims 35 to 37, **characterized in that** the dendrimer with monophosphonic or bisphosphonic terminations corresponds to the dendrimers defined in claim 14, or to GC0 or GC2, in particular to GC1.

**39.** Activated monocytes or cell compositions comprising activated monocytes as obtained by the process according to one of claims 35 to 38.

**40.** Pharmaceutical composition **characterized in that** it comprises as active ingredient, lymphoid cells expressing the receptor NKG2D, in particular NK cells, and at least one dendrimer with monophosphonic or bisphosphonic terminations, in combination with a pharmaceutically acceptable vehicle.

**41.** Pharmaceutical composition according to claim 40, **characterized in that** the dendrimer with monophosphonic or bisphosphonic terminations corresponds to the dendrimers as defined in one of claims 4 to 17.

**42.** Pharmaceutical composition according to claim 40 or 41, **characterized in that** the dendrimer with monophosphonic

or bisphosphonic terminations corresponds to the dendrimers defined in claim 14, or to GC0 or GC2, in particular to GC1.

**43.** Pharmaceutical composition, **characterized in that** it contains as active ingredient a cell composition as defined in claim 33 and/or activated monocytes or a cell composition comprising activated monocytes as defined in claim 39, in combination with a pharmaceutically acceptable vehicle.

**44.** Pharmaceutical composition according to one of claims 40 to 43, **characterized in that** it is suitable for the administration to an individual of a single dose of approximately $10^5$ to approximately $5.10^9$ lymphoid cells expressing the receptor NKG2D, in particular NK cells.

**45.** Use of lymphoid cells expressing the receptor NKG2D, in particular NK cells, and of GC1 for the preparation of medicaments intended for the treatment and/or prevention of cancers, including hematopoietic tissue tumors, such as myeloid leukemia or anaplastic lymphomas, and melanomas.

**46.** Use of a cell composition as defined in claim 33 and/or of activated monocytes or of a cell composition comprising activated monocytes as defined in claim 39, for the preparation of medicaments intended for the treatment and/or prevention of cancers, including hematopoietic tissue tumors, such as myeloid leukemia or anaplastic lymphomas, and melanomas.

**47.** Pharmaceutical composition containing as active ingredient at least one dendrimer with monophosphonic or bisphosphonic terminations in combination with a pharmaceutically acceptable vehicle.

**48.** Pharmaceutical composition according to claim 47, **characterized in that** the dendrimer with monophosphonic or bisphosphonic terminations corresponds to the dendrimers was defined in one of claims 4 to 14.

**49.** Pharmaceutical composition according to claim 47 or 48, **characterized in that** the dendrimer with monophosphonic or bisphosphonic terminations corresponds to the dendrimers defined in claim 14, or to GC0 or GC2, in particular to GC1.

**50.** Dendrimers with monophosphonic or bisphosphonic terminations of generation n, n representing an integer from 0 to 12, comprising a central core § of valency m, m representing an integer from 3 to 20, in particular 3 to 10 and more particularly 3 to 8, the core establishing m-2 or m-1 bonds with respectively m-2 or m-1 identical linkage chains constituted by:

• generation chains attached in a tree-like structure around the core on each of the bonds, when n is greater than or equal to 1, a generation chain of a given generation being linked to

- a generation chain of the generation immediately below the given generation, or to the core when the given generation is 1, and to
- at least 2 generation chains of the generation immediately above the given generation, or optionally to at least one intermediate chain when the given generation is n,

a terminal group being attached to the outside of each generation chain, or if appropriate to the end of each intermediate chain, or

• intermediate chains attached around the core on each of the bonds, when n is 0, a terminal group being attached to the end of each intermediate chain;
said terminal group being represented by the formula:

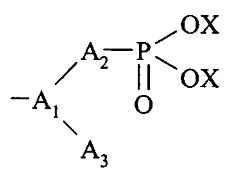

where

$A_1$ represents N; a P=Y group, where Y represents O, S, or any atom; an N-R group; or a C-R group; R representing H or a linear, branched or cyclic hydrocarbon chain with 1 to 16 members optionally containing one or more heteroatoms, said heteroatoms preferably being chosen from oxygen, sulphur, nitrogen, phosphorus, silicon and/or one or more double or triple bonds, each of said members optionally being substituted by at least one substituent chosen from a hydroxyl group, an -NR'R" group, an alkoxy group of 1 to 16 carbon atoms, an alkyl group of 1 to 16 carbon atoms, a halogen atom, an - $NO_2$ group, a -CN group, a -$CF_3$ group, an aryl group of 6 to 24 carbon atoms, an aralkyl group of 7 to 16 carbon atoms, R' and R" representing independently of each other H or an alkyl group of 1 to 16 carbon atoms, an aryl group of 6 to 24 carbon atoms or an aralkyl group of 7 to 16 carbon atoms;

$A_2$ represents a single bond or a linear, branched or cyclic hydrocarbon chain with 1 to 6 members, each of said members optionally containing one or more heteroatoms, said heteroatoms preferably being chosen from a sulphur, oxygen, phosphorus or nitrogen atom, more preferably nitrogen, and optionally being substituted by at least one substituent chosen from H, an alkyl group of 1 to 16 carbon atoms, a halogen, an -$NO_2$ group, an -NRR' group, a -CN group, a -$CF_3$ group, a hydroxyl group, an alkoxy group of 1 to 16 carbon atoms, an aryl or heteroaryl group of 1 to 24 carbon atoms, the heteroelement preferably being chosen from oxygen, nitrogen or sulphur, an aralkyl group of 7 to 16 carbon atoms, R and R' representing independently of each other H or a linear, branched or cyclic hydrocarbon chain with 1 to 16 members, optionally containing one or more heteroatoms, said heteroatoms preferably being chosen from oxygen, sulphur, nitrogen, phosphorus, silicon and/or one or more double or triple bonds, each of said members optionally being substituted by at least one substituent chosen from a hydroxyl group, an -NR"R"' group, an alkoxy group of 1 to 16 carbon atoms, an alkyl group of 1 to 16 carbon atoms, a halogen atom, an -$NO_2$ group, a -CN group, a -$CF_3$ group, an aryl group of 6 to 24 carbon atoms, an aralkyl group of 7 to 16 carbon atoms, R" and R"' representing independently of each other H or an alkyl group of 1, to 16 carbon atoms, an aryl group of 6 to 24 carbon atoms or an aralkyl group of 7 to 16 carbon atoms;

$A_3$ represents H, or a linear, branched or cyclic hydrocarbon chain with 1 to 6 members, each of said members optionally being chosen from a heteroatom, said heteroatom preferably being chosen from sulphur, nitrogen, phosphorus or silicon, more preferably nitrogen, each member being able to be optionally substituted by at least one group chosen from a hydroxyl group, an -NR"R"' group, an alkoxy group of 1 to 16 carbon atoms, an alkyl group of 1 to 16 carbon atoms, a halogen atom, an -$NO_2$ group, a -CN group, a -$CF_3$ group, an aryl group of 6 to 24 carbon atoms, an aralkyl group of 7 to 16 carbon atoms, R" and R"' representing independently of each other H or an alkyl group of 1 to 16 carbon atoms, an aryl group of 6 to 24 carbon atoms, or an aralkyl group of 7 to 16 carbon atoms or

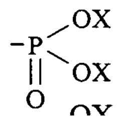
,

in particular $A_3$ can represent

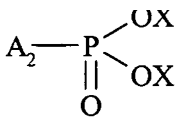
,

each $A_2$ being identical or different;

each OX, identical or different for each phosphonic group, represents OH, Oalkyl, where the alkyl group comprises from 1 to 16 carbon atoms, Oaryl, where the aryl group comprises from 6 to 24 carbon atoms, Oaralkyl, where the aralkyl group comprises from 7 to 24 carbon atoms, Oalkylaryl, where the alkylaryl group comprises from 7 to 24 carbon atoms, $OSiR'_1R'_2R'_3$, where $R'_1$, $R'_2$ and $R'_3$, identical or different, represent an alkyl group of 1 to 16 carbon atoms, or $O^-M^+$, where $M^+$ is a cation of elements of the group IA, IB, IIA, IIB or IIIA, IIIB of the periodic table of the elements, $M^+$ is preferably chosen from the cations of sodium, potassium, copper, calcium, barium, zinc, magnesium, lithium and aluminium atoms, or from hydrocarbon groups of 1 to 100 carbon atoms, or nitrogenous groups of 0 to 100 carbon atoms, such as $NR_1R_2R_3R_4^+$, where, independently of one another $R_1$, $R_2$, $R_3$ and $R_4$ represent H or a linear, branched or cyclic hydrocarbon chain with 1 to 16 members, optionally containing one or more heteroatoms, said heteroatoms preferably being chosen from oxygen, sulphur, nitrogen, phosphorus, silicon and/or one or more double or triple bonds, each of said members optionally being

substituted by at least one substituent chosen from a hydroxyl group, an -NR"R"' group, an alkoxy group of 1 to 16 carbon atoms, an alkyl group of 1 to 16 carbon atoms, a halogen atom, an $-NO_2$ group, a -CN group, a $-CF_3$ group, an aryl group of 6 to 24 carbon atoms, an aralkyl group of 7 to 16 carbon atoms, R" and R"' representing independently of each other H or an alkyl group of 1 to 16 carbon atoms, an aryl group of 6 to 24 carbon atoms or an aralkyl group of 7 to 16 carbon atoms;

the 1 or 2 remaining bonds being attached to the identical or different linkage groups, optionally linked together, in particular by a covalent bond, constituted either on the one hand by linkage chains defined above,
or by a hydrogen atom,
or by hydrocarbon groups comprising from 1 to 500 carbon atoms,
said hydrocarbon groups being constituted in particular by H or a linear, branched or cyclic hydrocarbon chain with 1 to 16 members, optionally containing one or more double or triple bonds, each of said members optionally being chosen from a heteroatom, said heteroatom preferably being chosen from a nitrogen, oxygen, phosphorus, silicon or sulphur atom, an aryl group of 6 to 24 carbon atoms, a heteroaryl group of 1 to 24 carbon atoms, a carboxyl group, a >C=NR group, each member being able to be optionally substituted by at least one substituent chosen from a hydroxyl group, an -NR"R"' group, an alkoxy group of 1 to 16 carbon atoms, an alkyl group of 1 to 16 carbon atoms, a halogen atom, an $-NO_2$ group, a -CN group, a $-CF_3$ group, an aryl group of 6 to 24 carbon atoms, an aralkyl group of 7 to 16 carbon atoms, R" and R"' representing independently of each other H or an alkyl group of 1 to 16 carbon atoms, an aryl group of 6 to 24 carbon atoms or an aralkyl group of 7 to 16 carbon atoms, the first member of said hydrocarbon chain preferably being oxygen or nitrogen;
said central core § being chosen among:

- a nitrogen or silicon atom,
- a group of formula

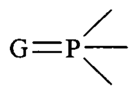 ,

wherein G represents an atom of oxygen, nitrogen, sulfur, selenium, tellurium or a group =NR, R representing H or a alkyl group from 1 to 16 carbon atoms, aryl from 6 to 24 carbon atoms, or aralkyl from 7 to 16 carbon atoms, such as the thiophosphoryle group of formula

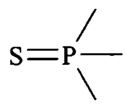 ;

- a group bis-phenyloxy of formula

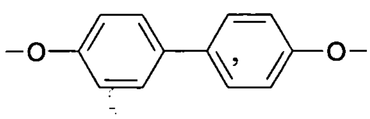

- a group 1,2-diamino-ethane of formula

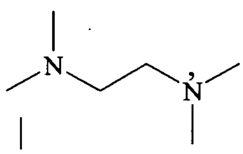

- a group 1,4-diamino-butane of formula

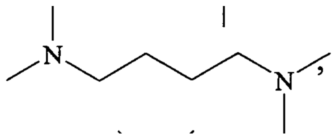

- a group cyclotriphosphazene of formula

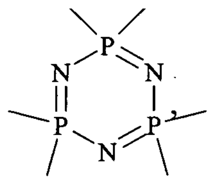

- a group cyclotetraphosphazene of formula

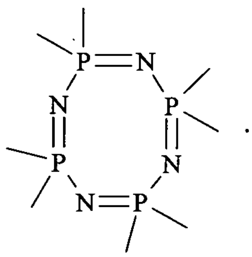

**51.** Dendrimers according to claim 50, in which the generation chains are chosen from any linear, branched or cyclic hydrocarbon chain with 1 to 12 members, optionally containing one or more double or triple bonds, each of said members being optionally chosen from a heteroatom, said heteroatom being preferably chosen from nitrogen, oxygen, sulphur, phosphorus or silicon, an aryl group of 6 to 24 carbon atoms, a heteroaryl group of 1 to 24 carbon atoms, the heteroelement being preferably chosen from oxygen, nitrogen or sulphur, a carboxyl group, a >C=NR group, each member being optionally substituted by at least one substituent chosen from an alkyl group of 1 to 16 carbon atoms, a halogen atom, an $-NO_2$ group, a -RR' group, a -CN group, a $-CF_3$ group, a hydroxyl group, an alkoxy group of 1 to 16 carbon atoms, an aryl group of 6 to 24 carbon atoms, or an aralkyl group of 7 to 16 carbon atoms, R and R' representing independently of each other H or an alkyl group of 1 to 16 carbon atoms, an aryl group of 6 to 24 carbon atoms, or an aralkyl group of 7 to 16 carbon atoms.

**52.** Dendrimers according to claim 50 or 51, in which the intermediate chains are chosen from the groups corresponding to the formula:

-J-K-L-

where

- J represents an oxygen or sulphur atom, or an -NR- group;
- K represents an aryl group of 6 to 24 carbon atoms, a heteroaryl group of 1 to 24 carbon atoms, the heteroelement being preferably chosen from oxygen, nitrogen or sulphur, an alkyl group of 1 to 16 carbon atoms, each being able to be optionally substituted by a halogen atom or an $-NO_2$, -NRR', -CN, $-CF_3$, -OH group, an alkyl group of 1 to 16 carbon atoms, an aryl group of 6 to 24 carbon atoms, an aralkyl group of 7 to 16 carbon atoms;
- L represents a linear, branched or cyclic hydrocarbon chain with 0 to 10 members, optionally containing one or more double or triple bonds, each of said members optionally being able to be a heteroatom, said heteroatom being preferably chosen from oxygen, sulphur, nitrogen, phosphorus, silicon, each member being able to be optionally substituted by at least one substituent chosen from an alkyl group of 1 to 16 carbon atoms, a halogen, an oxygen atom, $-NO_2$, -NRR', -CN, $-CF_3$, -OH, an alkoxy group of 1 to 16 carbon atoms, an aryl group of 6 to 24 carbon atoms, an aralkyl group of 7 to 16 carbon atoms;

R and R' representing independently of each other H or an alkyl group of 1 to 16 carbon atoms, an aryl group of 6 to 24 carbon atoms, or an aralkyl group of 7 to 16 carbon atoms.

**53.** Dendrimers according to one of claims 50 to 52, of structure PMMH, PAMAM, or DAB.

**54.** Dendrimers according to one of claims 50 to 52, with bisphosphonic terminations corresponding to the following general formula (7):

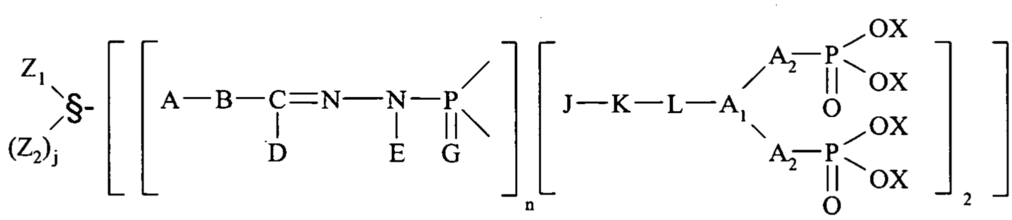

**(7)**

where n represents an integer from 0 to 3, m represents 3, 6 or 8, p represents m - 1 or m - 2, and j represents 0 when p represents m - 1 and 1 when p represents m - 2, namely:

- when p = m - 1, formula (7) corresponds to the following formula (8):

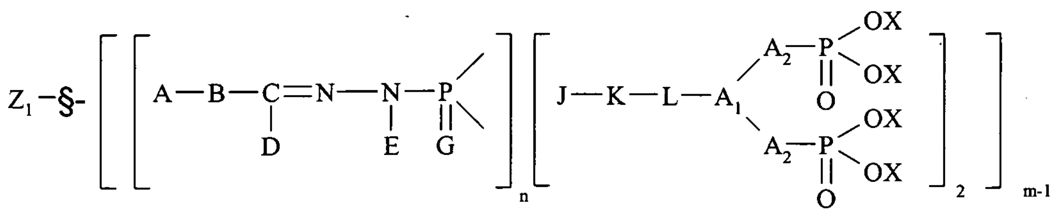

**(8)**

- when p = m - 2, formula (7) corresponds to the following formula (9)

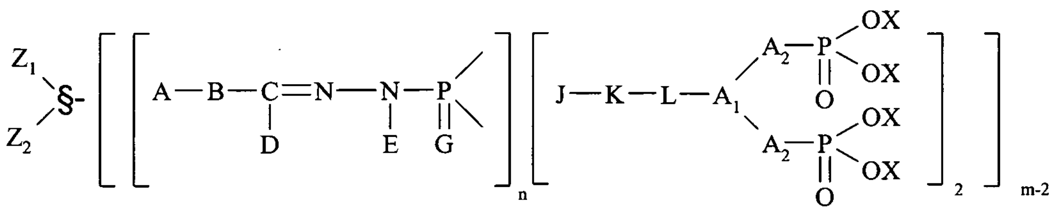

**(9)**

and in which formulae:

• the central core § is chosen from the following groups:

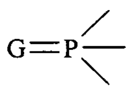

,

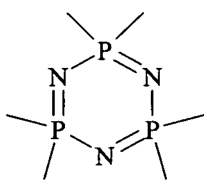

,

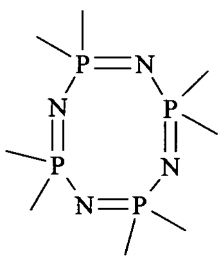

;

• the generation chain corresponds to the formula:

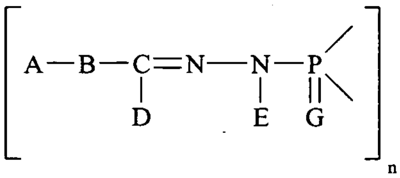

where
- A represents an oxygen, sulphur, phosphorus atom or an -NR- group;
- B represents an aryl group of 6 to 24 carbon atoms, a heteroaryl group of 1 to 24 carbon atoms, an alkyl group of 1 to 16 carbon atoms, each being able to be optionally substituted by a halogen atom or an $-NO_2$, -NRR', -CN, $-CF_3$, -OH group, an alkyl group of 1 to 16 carbon atoms, an aryl group of 6 to 24 carbon atoms, an aralkyl group of 7 to 16 carbon atoms;
- D represents a hydrogen atom, an alkyl group of 1 to 16 carbon atoms, an alkoxy group of 1 to 16 carbon atoms, an aryl group of 6 to 24 carbon atoms, an aralkyl group of 7 to 16 carbon atoms, each being able to be optionally substituted by a halogen atom or an $-NO_2$, -NRR', -CN, $-CF_3$, -OH group, an alkyl group of 1 to 16 carbon atoms, an aryl group of 6 to 24 carbon atoms, an aralkyl group of 7 to 16 carbon atoms;
- E represents a hydrogen atom, an alkyl group of 1 to 16 carbon atoms, an alkoxy group of 1 to 16 carbon atoms, an aryl group of 6 to 24 carbon atoms, an aralkyl group of 7 to 16 carbon atoms, each being able to be optionally substituted by a halogen atom or an
$-NO_2$, -NRR', -CN, $-CF_3$, -OH group, an alkyl group of 1 to 16 carbon atoms, an aryl group of 6 to 24 carbon atoms, an aralkyl group of 7 to 16 carbon atoms;
- G represents an oxygen, nitrogen, sulphur, selenium, tellurium atom or an =NR group; R and R' representing independently of each other H or an alkyl group of 1 to 16 carbon atoms, an aryl group of 6 to 24 carbon atoms, or an aralkyl group of 7 to 16 carbon atoms;

• the intermediate chain corresponds to the formula:

-J-K-L-

where

- J represents an oxygen, sulphur atom, or an -NR- group;
- K represents an aryl group of 6 to 24 carbon atoms, a heteroaryl group of 1 to 24 carbon atoms, the heteroelement preferably being chosen from oxygen, nitrogen or sulphur, an alkyl group of 1 to 16 carbon atoms, each being able to be optionally substituted by a halogen atom or an $-NO_2$, -NRR', -CN, $-CF_3$, -OH group, an alkyl group of 1 to 16 carbon atoms, an aryl group of 6 to 24 carbon atoms, an aralkyl group of 7 to 16 carbon atoms;
- L represents a linear, branched or cyclic hydrocarbon chain with 0 to 10 members, optionally containing one or more double or triple bonds, each of said members optionally being a heteroatom, said heteroatom preferably being chosen from oxygen, sulphur, nitrogen, phosphorus, silicon, each member being able to be optionally substituted by at least one substituent chosen from an alkyl group of 1 to 16 carbon atoms, a halogen, an oxygen atom, $-NO_2$, -NRR', -CN, $-CF_3$, -OH, an alkoxy group of 1 to 16 carbon atoms, an aryl group of 6 to 24 carbon atoms, an aralkyl group of 7 to 16 carbon atoms;
R and R' representing independently of each other H or an alkyl group of 1 to 16 carbon atoms, an aryl group of 6 to 24 carbon atoms, or an aralkyl group of 7 to 16 carbon atoms;

• the terminal group corresponds to the formula:

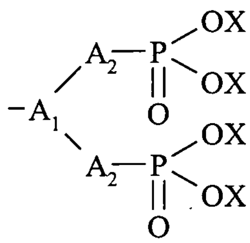

where $A_1$, $A_2$ and X have been defined previously, each X being identical or different;

$Z_1$ and $Z_2$ being identical or different, optionally linked together, in particular by a covalent bond, and representing H or a linear, branched or cyclic hydrocarbon chain with 1 to 16 members, optionally containing one or more double or triple bonds, each of said members optionally being chosen from a heteroatom, said heteroatom preferably being chosen from a nitrogen, oxygen, phosphorus, silicon or sulphur atom, an aryl group of 6 to 24 carbon atoms, a heteroaryl group of 1 to 24 carbon atoms, a carboxyl group, a >C=NR group, each member being able to be optionally substituted by at least one substituent chosen from a hydroxyl group, an -NR"R"' group, an alkoxy group of 1 to 16 carbon atoms, an alkyl group of 1 to 16 carbon atoms, a halogen atom, an $-NO_2$ group, a -CN group, a $-CF_3$ group, an aryl group of 6 to 24 carbon atoms, an aralkyl group of 7 to 16 carbon atoms, R" and R"' representing independently of each other H or an alkyl group of 1 to 16 carbon atoms, an aryl group of 6 to 24 carbon atoms or an aralkyl group of 7 to 16 carbon atoms, the first member of said hydrocarbon chain preferably being oxygen or nitrogen.

**55.** Dendrimers according to claim 54, of structure PMMH, corresponding to the general formula (8), in which

§ represents

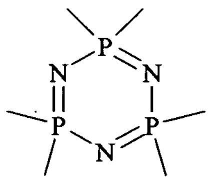

;

m represents 6;
p represents 5;
n represents 0, 1, or 2;
A represents an oxygen atom;
B represents a benzene group;
D represents hydrogen;
E represents a methyl group;
G represents a sulphur atom;
J represents an oxygen atom;
K represents a benzene group;
L represents a non substituted saturated linear hydrocarbon chain with two carbon atoms;
$A_1$ represents a nitrogen atom;
$A_2$ represents a $CH_2$ group;
X represents a methyl group, or a hydrogen or sodium atom;
$Z_1$ represents a phenyloxy group;

said dendrimer being designated GCn', n being defined above.

**56.** Dendrimers according to one of claims 50 to 55, of the following formulae:

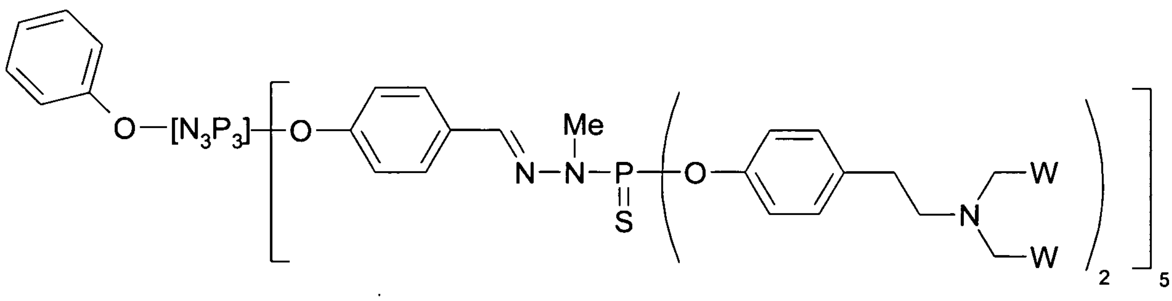

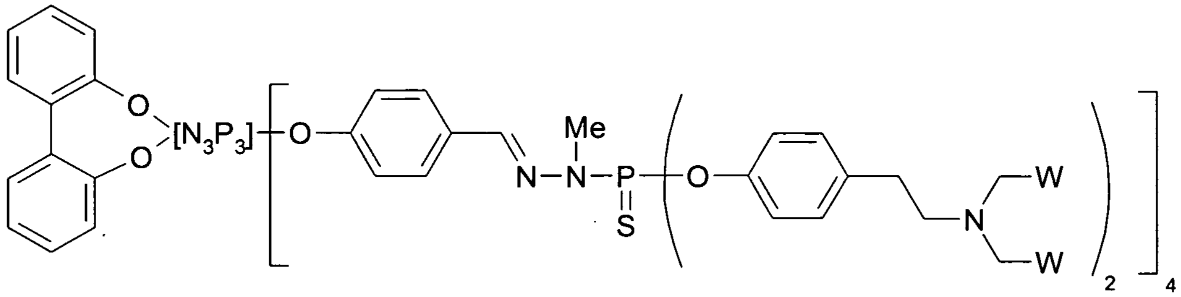

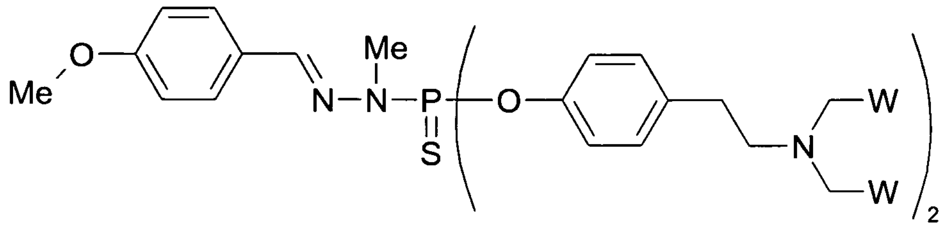

in which W represents $PO_3Me_2$, $PO_3HNa$, or $PO_3H_2$, said dendrimers corresponding in particular to compound GC1' of the following formula (10):

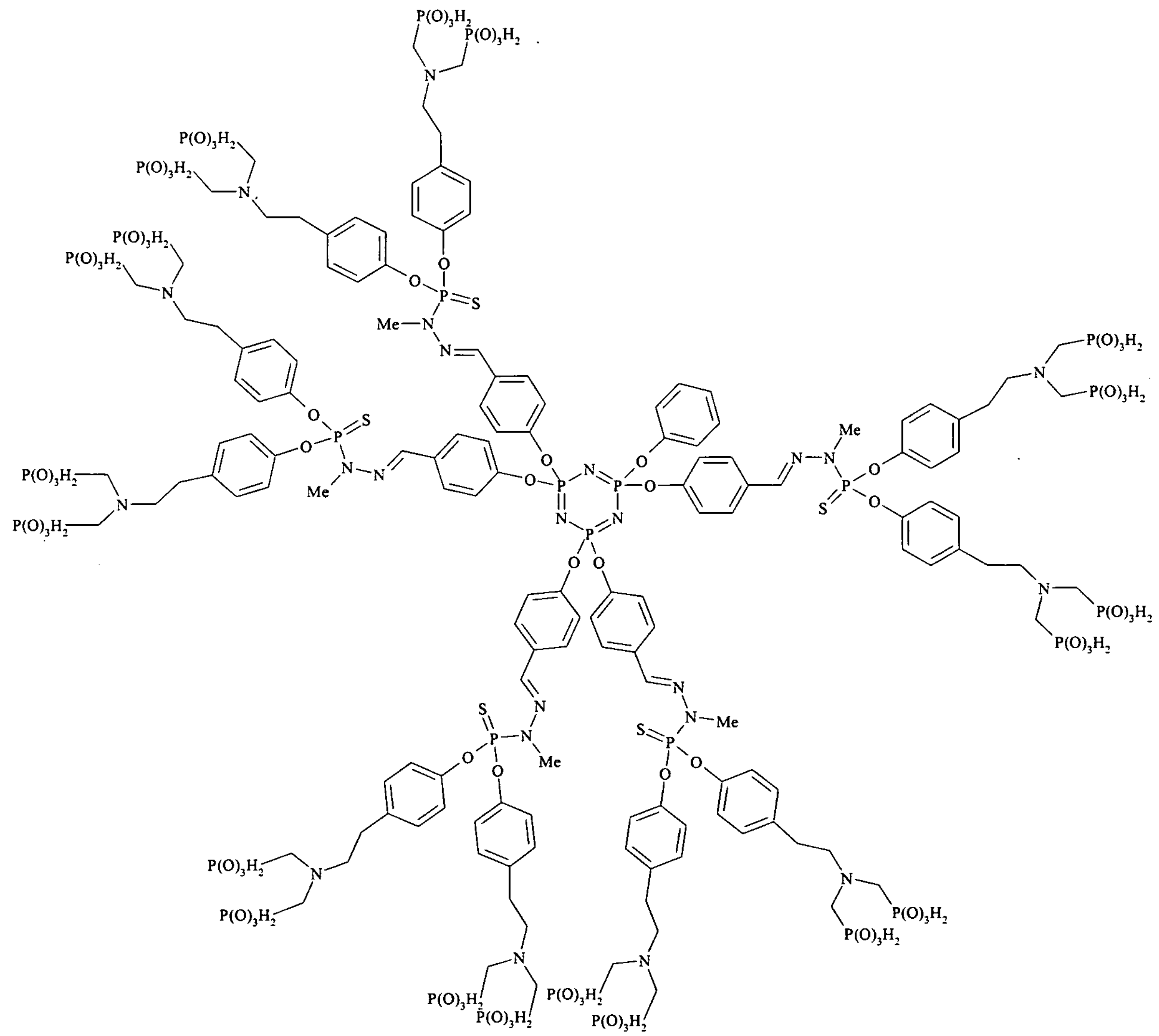

**(10)**

**57.** Dendrimers according to one of claims 50 to 54, of the following formula :

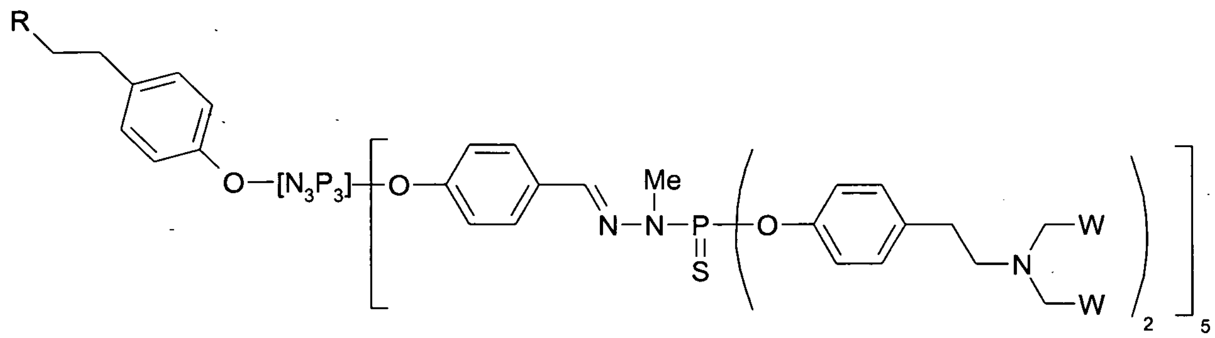

in which W represents $PO_3Me_2$, $PO_3HNa$, or $PO_3H_2$ and R represents a fluorescent group chosen from:

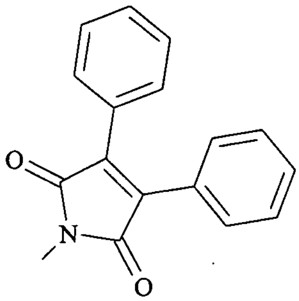 , 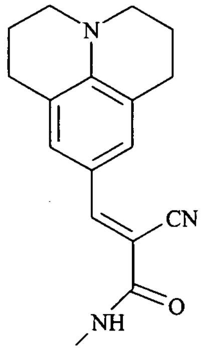 ,

said dendrimers corresponding in particular to the compounds of the following formulae:

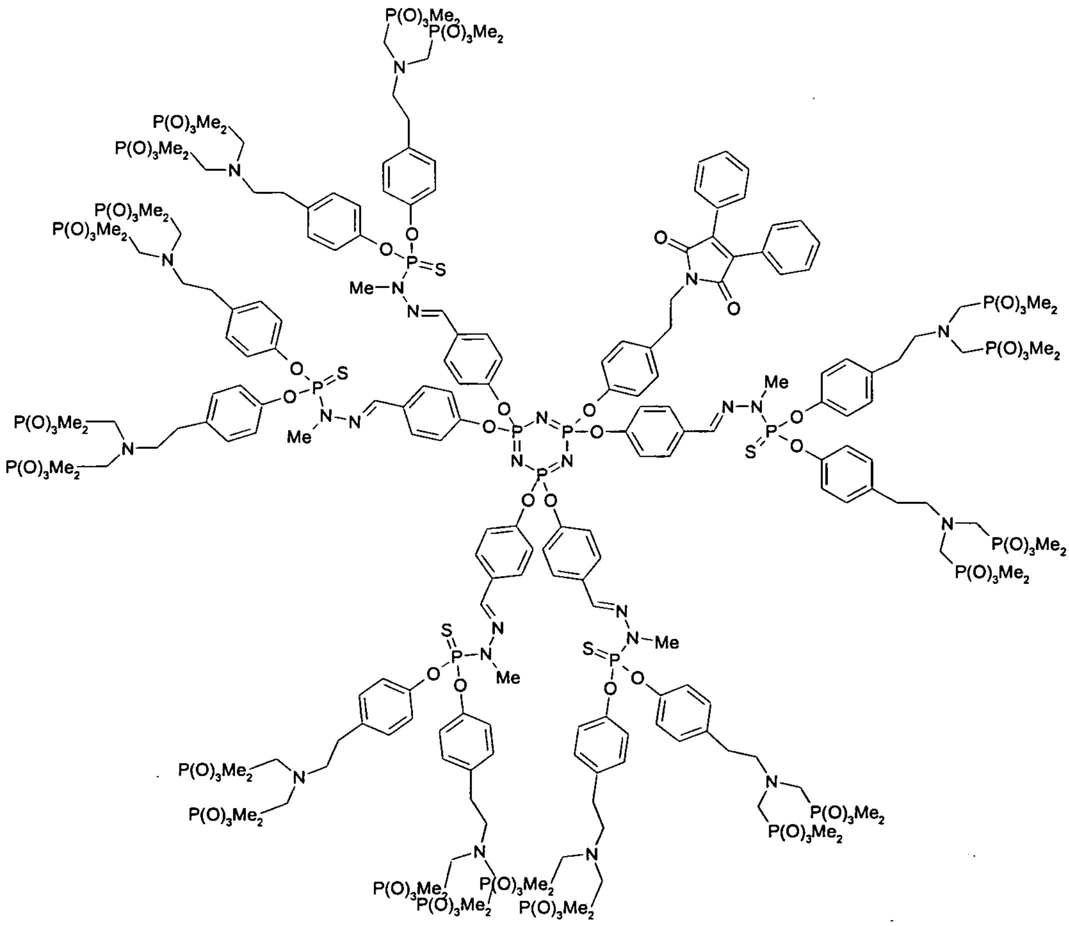

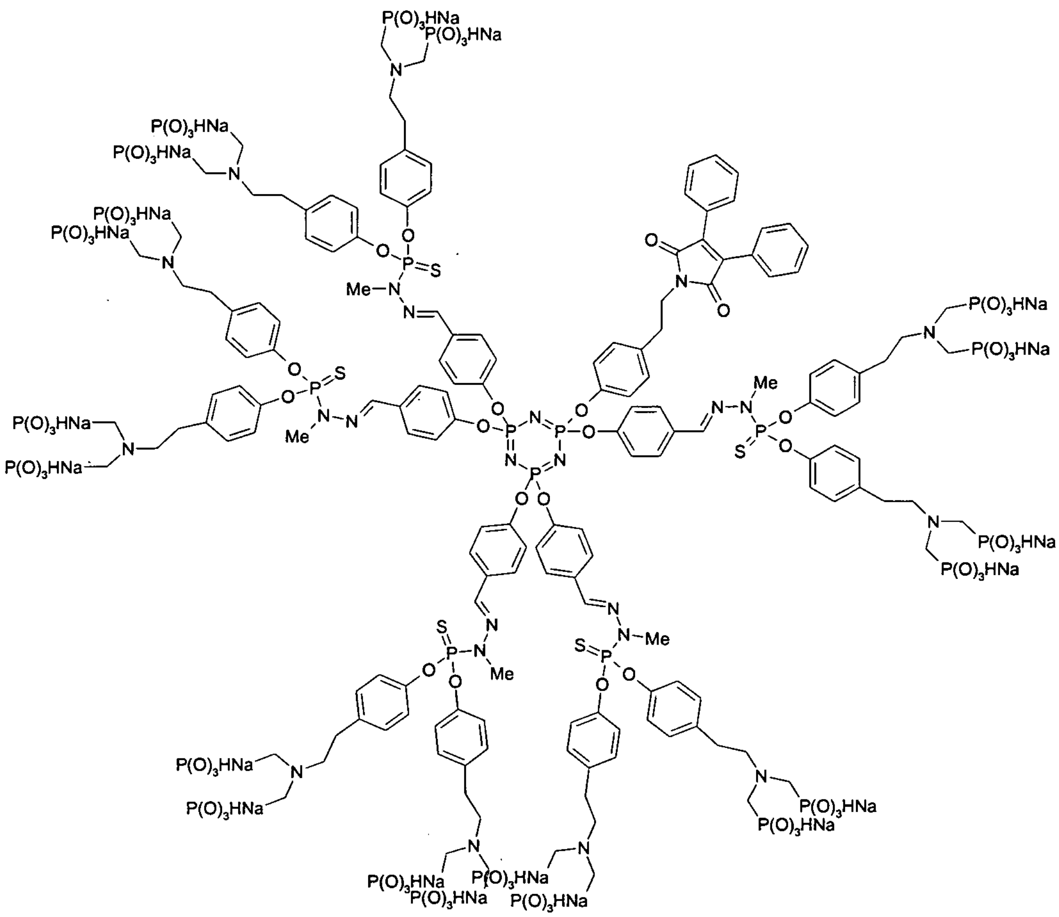

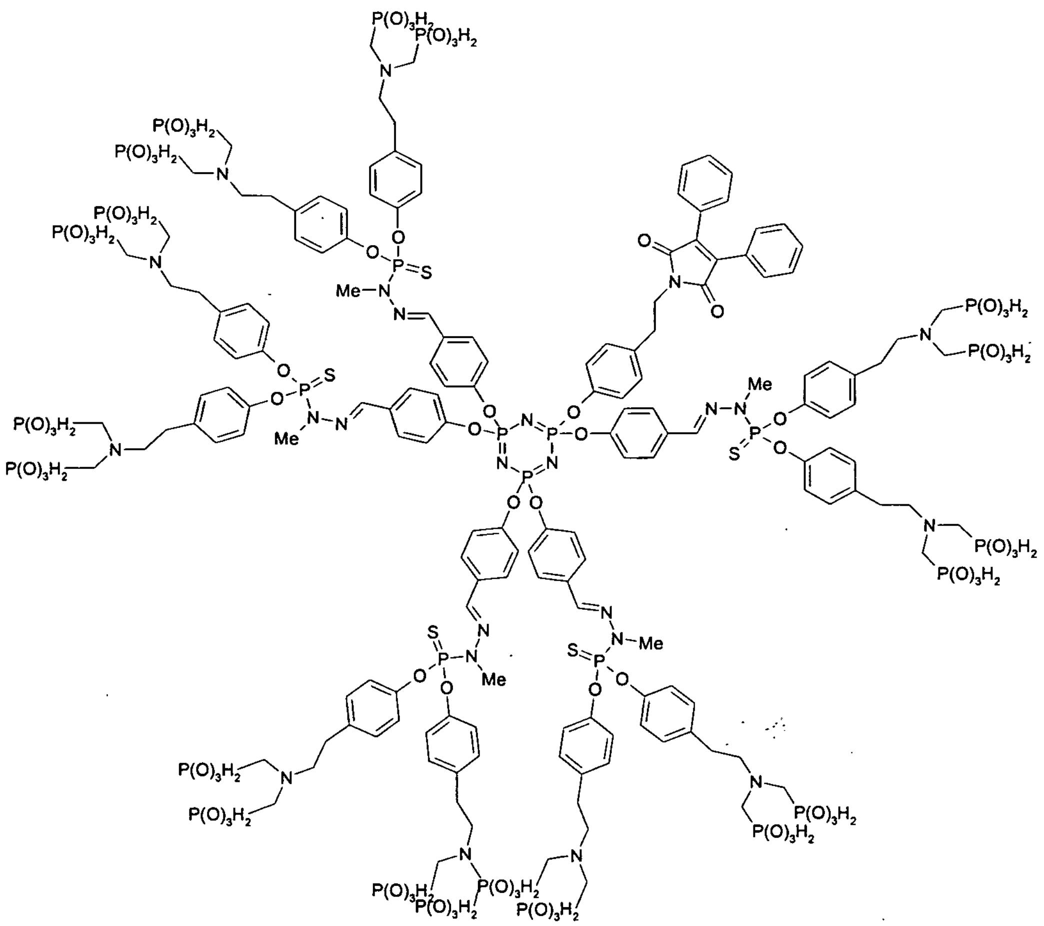

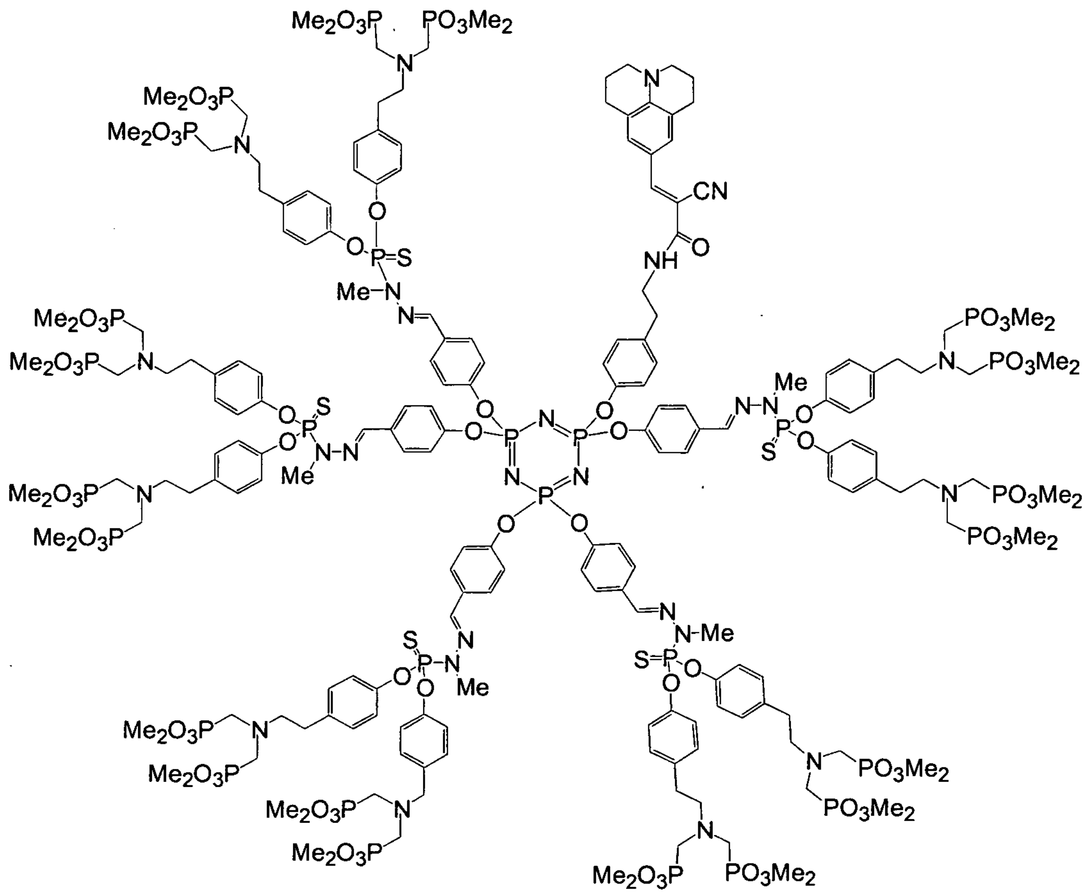

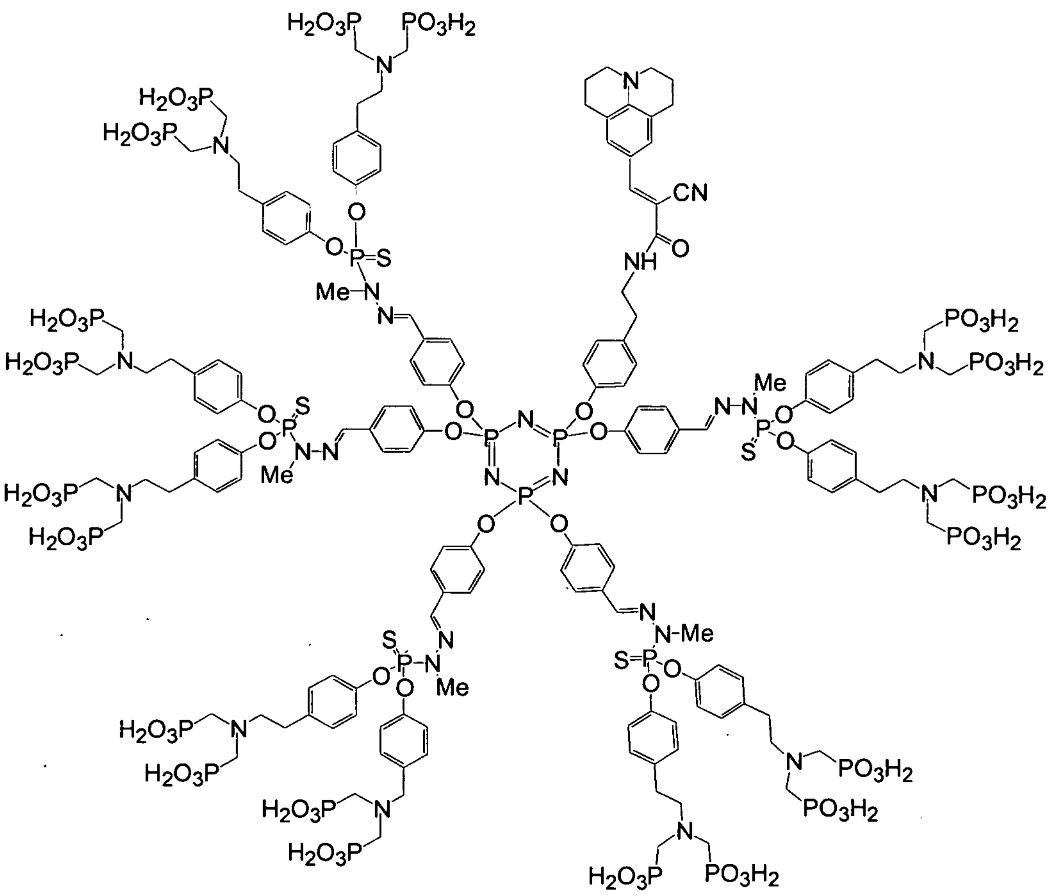

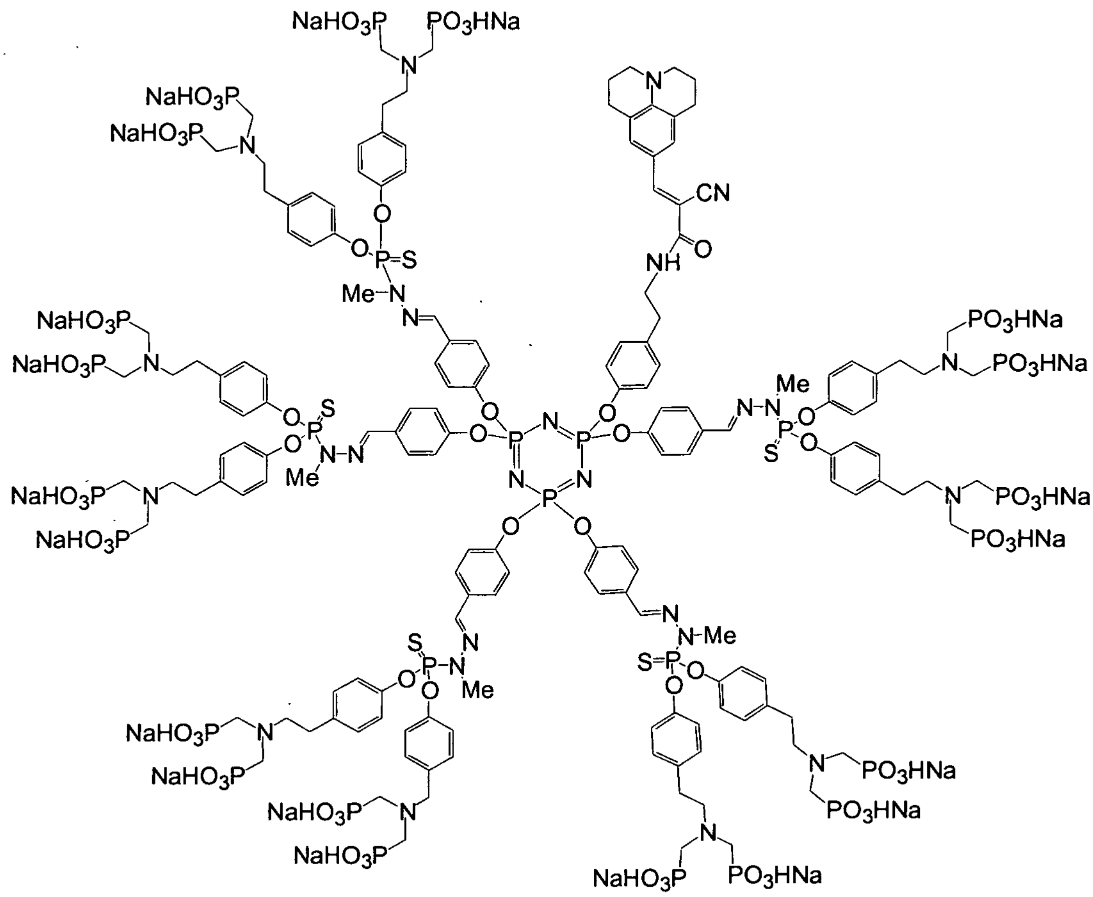

**58.** Dendrimers with bisphophonic terminations according to claim 50, of the following formula :

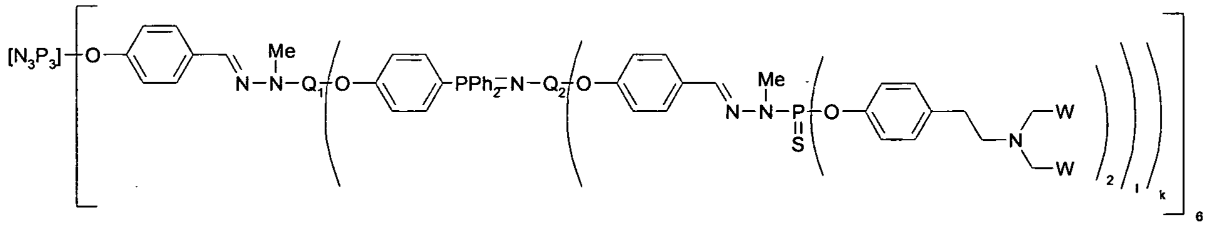

in which W represents $PO_3Me_2$, $PO_3HNa$, or $PO_3H_2$, $Q_1$ and $Q_2$, identical or different, represent P=S or the cyclotriphosphazene ($N_3P_3$), 1 represents 2 when $Q_2$ represents P=S or 5 when $Q_2$ represents $N_3P_3$ and k represents 2 when $Q_1$ represents P=S or 5 when $Q_1$ represents $N_3P_3$, said dendrimers being represented in particular by the following formulae:

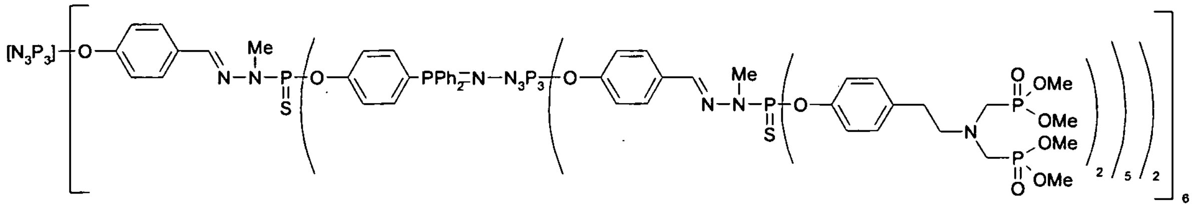

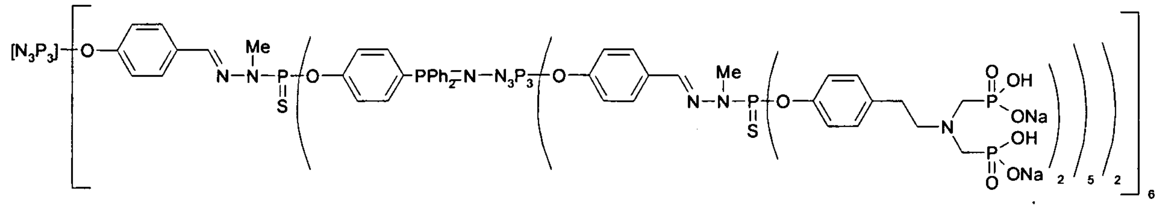

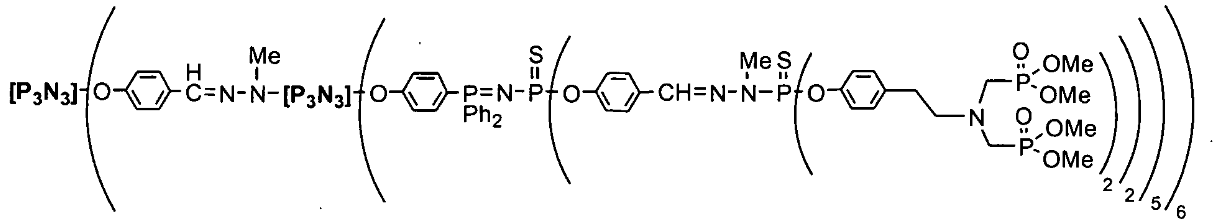

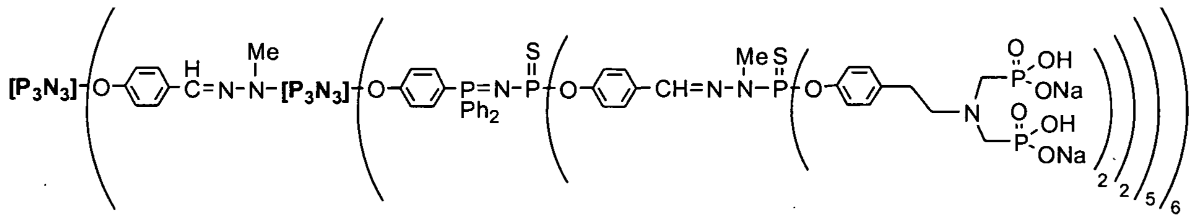

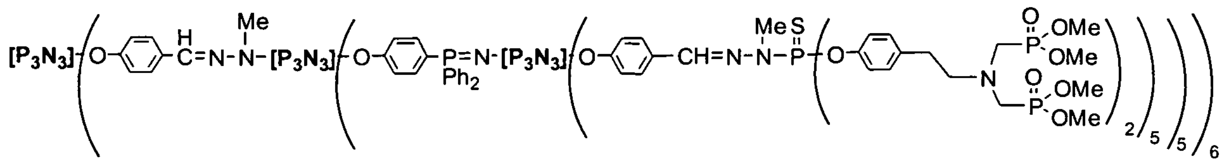

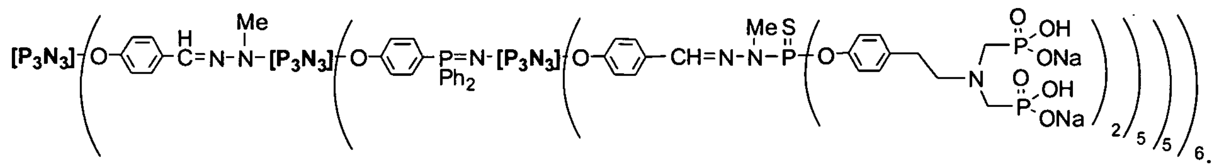

**59.** Dendrimers with monophosphonic or bisphosphonic terminations according to claim 50, of the following formula :

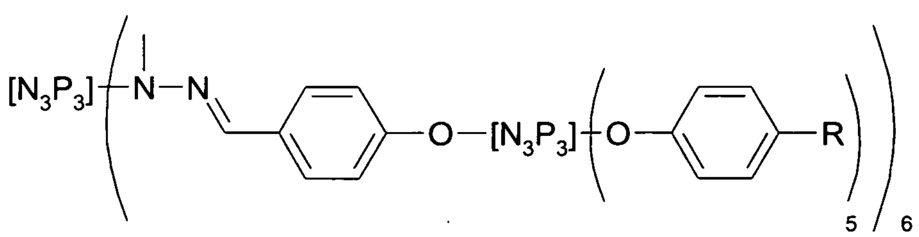

in which R represents a group chosen from

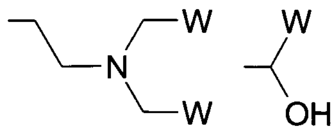

where W represents $PO_3Me_2$, $PO_3HNa$, or $PO_3H_2$.

**60.** Dendrimers with bisphosphonic terminations according to claim 50, of the following formula :

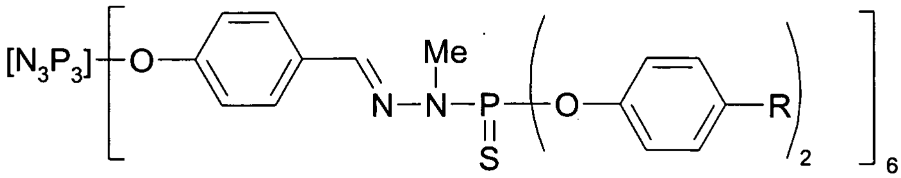

in which R represents a group chosen from:

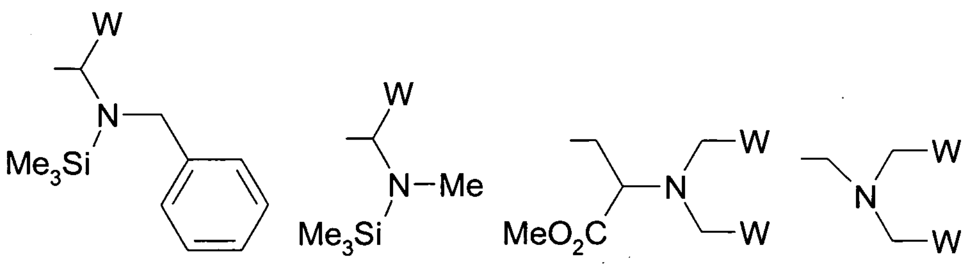

where W represents $PO_3Si_2Me_6$, $PO_3Me_2$, $PO_3HNa$, or $PO_3H_2$.

**61.** Dendrimers with monophosphonic terminations according to claim 50, of the following formula :

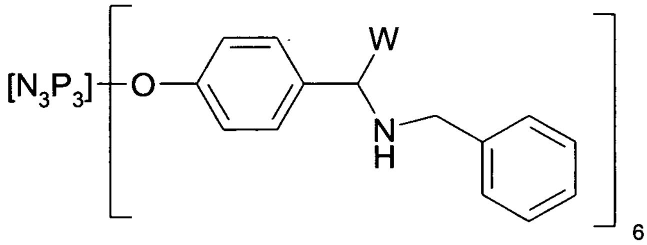

in which W represents $PO_3Me_2$, $PO_3HNa$, or $PO_3H_2$.

**62.** Dendrimers with bisphosphonic terminations according to claim 50, of the following formula :

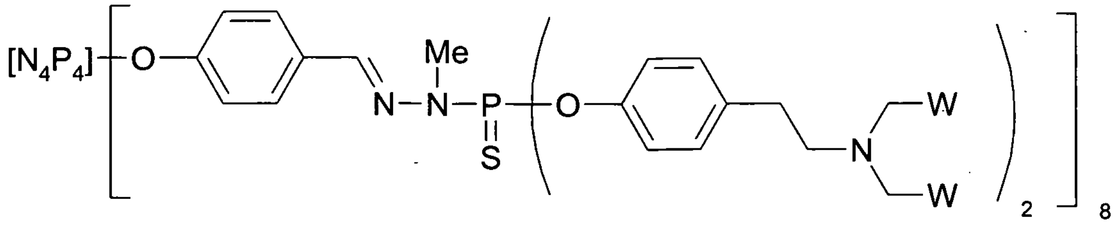

in which W represents $PO_3Me_2$, $PO_3HNa$, or $PO_3H_2$.

**63.** Dendrimers with bisphosphonic terminations according to claim 50, of the following formula :

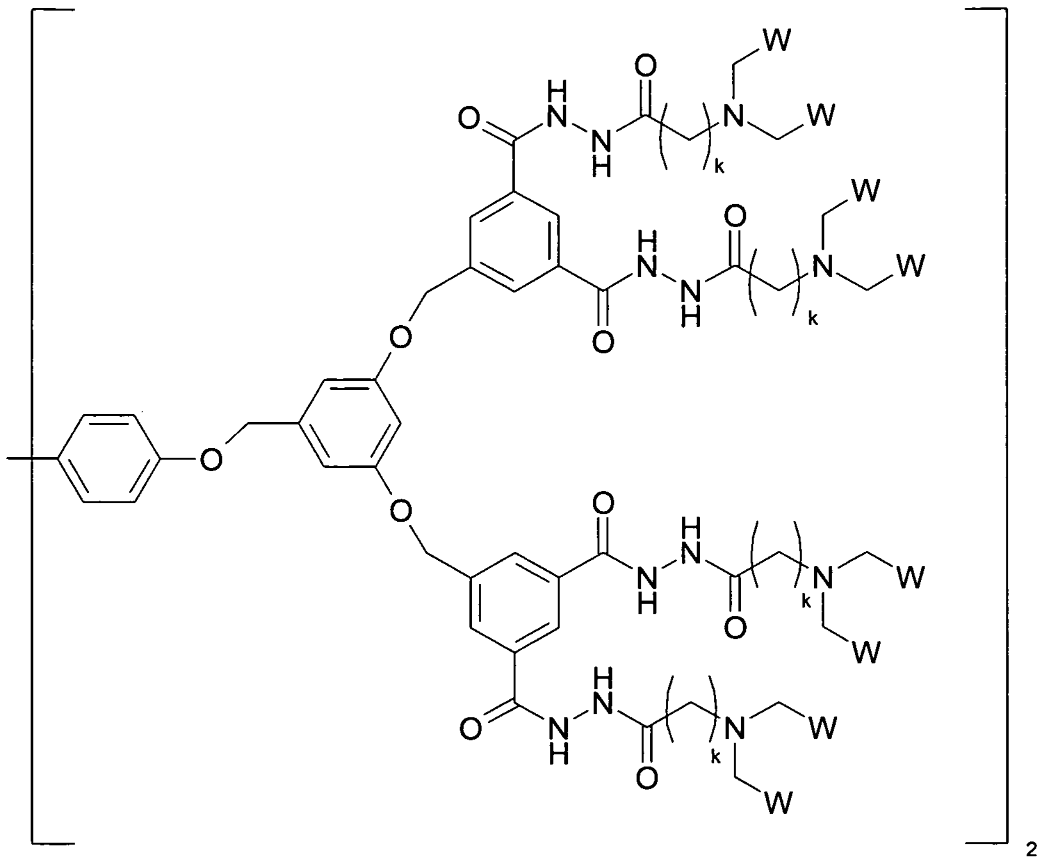

in which W represents $PO_3Me_2$, $PO_3HNa$, or $PO_3H_2$ and k represents 1, 2 or 3

**64.** Dendrimers with bisphosphonic terminations according to claim 50, of the following formula :

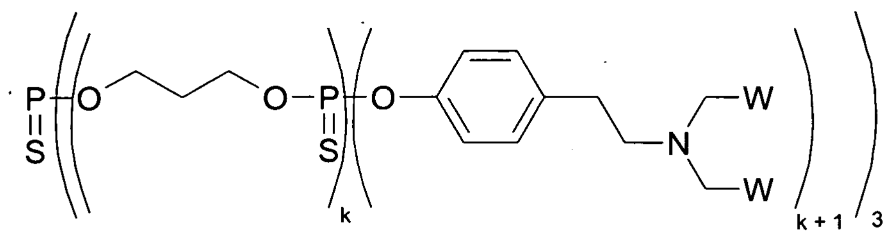

in which W represents $PO_3Mez$, $PO_3HNa$, or $PO_3H_2$ and k represents 0 or 1.

**65.** Dendrimers with bisphosphonic terminations according to claim 50, of the following formula :

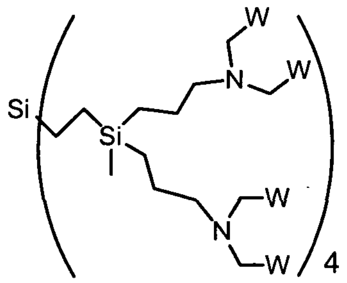

in which W represents $PO_3Me_2$, $PO_3HNa$, or $PO_3H_2$.

**66.** Dendrimers with bisphosphonic terminations according to claim 50, of the following formula:

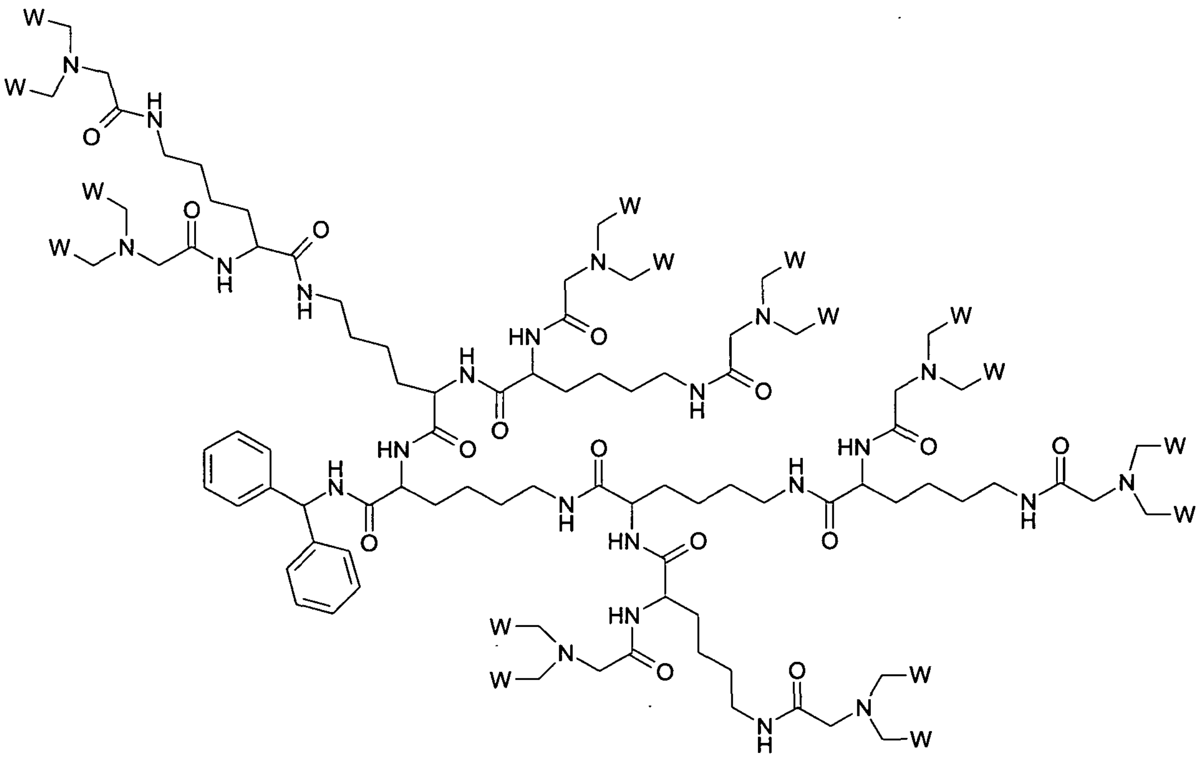

in which W represents $PO_3Me_2$, $PO_3HNa$, or $PO_3H_2$.

## Patentansprüche

1. Verwendung von Monophosphon- oder Bisphosphon-terminierten Dendrimeren, um das Wachstum von Zellkulturen zu stimulieren oder um Zellen in Kultur zu aktivieren.

2. Verwendung nach Anspruch 1, wobei die Zellen hämatopoetische Zellen sind.

3. Verwendung nach Anspruch 1 oder 2 von Monophosphon-oder Bisphosphon-terminierten Dendrimeren zur Herstellung von Zellzusammensetzungen, angereichert mit Zellen der Lymphoidlinie, die den Rezeptor NKG2D exprimieren, aus biologischen Proben.

**4.** Verwendung nach einem der Ansprüche 1 bis 3, wobei die Monophosphon- oder Bisphosphon-terminierten Dendrimere aus der Generation n stammen und einen zentralen Kern § mit Valenz m umfassen, der m-2 Bindungen aufbauen kann, unter der Voraussetzung, dass m größer als 2 ist, oder m-1 Bindungen, unter der Voraussetzung, dass m größer als 1 ist, oder m Bindungen mit Verbindungsketten, die untereinander bevorzugt identisch sind, wobei m für eine ganze Zahl von 1 bis 20, bevorzugt von 1 bis 10 und besonders bevorzugt von 1 bis 8 steht, und n für eine ganze Zahl von 0 bis 12 steht, wobei die Verbindungsketten bestehen aus:

• Generationsketten, die in baumähnlicher Struktur um den Kern herum auf jeder der Bindungen befestigt sind, wenn n größer oder gleich 1 ist, wobei eine Generationskette einer gegebenen Generation, verbunden ist mit

- einer Generationskette der Generation ummittelbar unterhalb der gegebenen Generation, oder dem Kern, wenn die gegebene Generation gleich 1 ist, und mit
- mindestens 2 Generationsketten der Generation unmittelbar über der gegebenen Generation, oder eventuell mit mindestens einer Intermediärkette, wenn die gegebene Generation gleich n ist,

einer terminalen Gruppe, die am Ende jeder der Generationsketten der Generation n oder gegebenenfalls am Ende jeder Intermediärkette befestigt ist, oder

• Intermediärketten, die um den Kern auf jeder der Bindungen befestigt sind, wenn n gleich 0 ist, wobei eine terminale Gruppe am Ende jeder Intermediärkette befestigt ist;
wobei die terminale Gruppe durch die folgende Formel dargestellt wird:

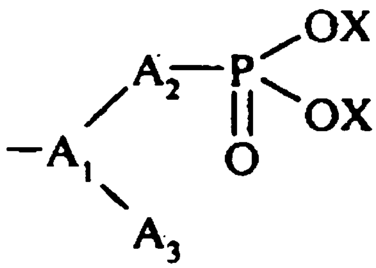

worin $A_1$ für N; eine Gruppe P=Y steht, worin Y für O, S oder kein Atom; eine Gruppe N-R; oder eine Gruppe C-R steht; R für H oder eine Kohlenwasserstoffkette mit 1 bis 16 linearen, verzweigten oder cyclischen Glieder steht, die eventuell ein oder mehrere Heteroatome enthalten, wobei die Heteroatome bevorzugt ausgewählt sind aus Sauerstoff, Schwefel, Stickstoff, Phosphor, Silicium und/oder einer oder mehreren Doppel- oder Dreifachbindungen, wobei jedes der Glieder eventuell mit mindestens einem Substituenten substituiert ist, ausgewählt aus einer Hydroxylgruppe, einer -NR'R''-Gruppe, einer Alkoxy-Gruppe mit 1 bis 16 Kohlenstoffatomen, einer Alkyl-Gruppe mit 1 bis 16 Kohlenstoffatomen, einem Halogenatom, einer -$NO_2$ Gruppe, einer -CN-Gruppe, einer -$CF_3$-Gruppe, einer Aryl-Gruppe mit 6 bis 24 Kohlenstoffatomen, einer Aralkyl-Gruppe mit 7 bis 16 Kohlenstoffatomen, wobei R' und R" unabhängig voneinander für H oder -eine Alkyl-Gruppe mit 1 bis 16 Kohlenstoffatomen, eine Aryl-Gruppe mit 6 bis. 24 Kohlenstoffatomen oder eine Aralkyl-Gruppe mit 7 bis 16 Kohlenstoffatomen stehen;
$A_2$ für eine einfache Bindung oder eine Kohlenwasserstoffkette mit 1 bis 6 linearen, verzweigten oder cyclischen Gliedern steht, wobei jedes der Glieder eventuell ein oder mehrere Heteroatome enthält, wobei die Heteroatome bevorzugt ausgewählt sind aus einem Schwefel-, Sauerstoff-, Phosphor- oder Stickstoffatom, am stärksten bevorzugt aus einem Stickstoffatom, und eventuell mit mindestens einem Substituenten substituiert sind, ausgewählt aus H, einer Alkyl-Gruppe mit 1 bis 16 Kohlenstoffatomen, einem Halogen, einer -$NO_2$-Gruppe, einer -NRR'-Gruppe, einer -CN-Gruppe, einer -$CF_3$-Gruppe, einer Hydroxyl-Gruppe, einer Alkoxy-Gruppe mit 1 bis 16 Kohlenstoffatomen, einer Aryl- oder Heteroaryl-Gruppe mit 1 bis 24 Kohlenstoffatomen, wobei das Heteroelement bevorzugt ausgewählt ist aus Sauerstoff, Stickstoff oder Schwefel, einer Aralkyl-Gruppe mit 7 bis 16 Kohlenstoffatomen, wobei R und R' unabhängig voneinander für H oder eine Kohlenwasserstoffkette mit 1 bis 16 linearen, verzweigten oder cyclischen Gliedern stehen, die eventuell ein oder mehrere Heteroatome enthalten, wobei die Heteroatome bevorzugt ausgewählt sind aus Sauerstoff, Schwefel, Stickstoff, -Phosphor, Silicium und/oder einer oder mehreren Doppel- oder Dreifachbindungen, wobei jedes der Glieder eventuell mit mindestens einem Substituenten substituiert ist, ausgewählt aus einer Hydroxyl-Gruppe, einer -NR''R'''-Gruppe, einer

Alkoxy-Gruppe mit 1 bis 16 Kohlenstoffatomen, einer Alkyl-Gruppe mit 1 bis 16 Kohlenstoffatomen, einem Halogenatom, einer - $NO_2$-Gruppe, einer -CN-Gruppe, einer -$CF_3$-Gruppe, einer Aryl-Gruppe mit 6 bis 24 Kohlenstoffatomen, einer Aralkyl-Gruppe mit 7 bis 16 Kohlenstoffatomen, wobei R" und R''' unabhängig voneinander für H oder eine Alkylgruppe mit 1 bis 16 Kohlenstoffatomen, eine Aryl-Gruppe mit 6 bis 24 Kohlenstoffatomen oder eine Aralkylgruppe mit 7 bis 16 Kohlenstoffatomen stehen;

$A_3$ für H oder eine Kohlenwasserstoffkette mit 1 bis 6 linearen, verzweigten oder cyclischen Gliedern steht, wobei jedes der Glieder eventuell aus einem Heteroatom ausgewählt sein kann, wobei das Heteroatom bevorzugt ausgewählt ist aus Schwefel, Stickstoff, Phosphor oder Silicium, am stärksten bevorzugt Stickstoff, wobei jedes der Glieder eventuell mit mindestens einer Gruppe substituiert sein kann, ausgewählt aus einer Hydroxyl-Gruppe, einer -NR''R'''-Gruppe, einer Alkoxy-Gruppe mit 1 bis 16 Kohlenstoffatomen, einer Alkyl-Gruppe mit 1 bis 16 Kohlenstoffatomen, einem Halogenatom, einer -$NO_2$-Gruppe, einer -CN-Gruppe, einer -$CF_3$-Gruppe, einer Aryl-Gruppe mit 6 bis 24 Kohlenstoffatomen, einer Aralkyl-Gruppe mit 7 bis 16 Kohlenstoffatomen, wobei R'' und R''' unabhängig voneinander für H oder eine Alkyl-Gruppe mit 1 bis 16 Kohlenstoffatomen, eine Aryl-Gruppe mit 6 bis 24 Kohlenstoffatomen, oder eine Aralkylgruppe mit 7 bis 16 Kohlenstoffatomen oder

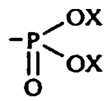

stehen,

wobei $A_3$ insbesondere für

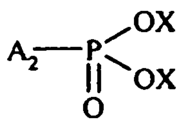

stehen kann, wobei jedes $A_2$ gleich oder verschieden ist;

wobei jedes OX, für jede Phosphon-Gruppe gleich oder verschieden, für OH, OAlkyl, worin die Alkyl-Gruppe 1 bis 16 Kohlenstoffatome umfasst, OAryl, worin, die Aryl-Gruppe 6 bis 24 Kohlenstoffatome umfasst, $O_A$ralkyl, worin die Aralkyl-Gruppe 7 bis 24 Kohlenstoffatome umfasst, OAlkylaryl, worin die Alkylaryl-Gruppe 7 bis 24 Kohlenstoffatome umfasst, $OSiR'_1R'_2R'_3$, worin $R'_1$, $R'_2$ und $R'_3$, gleich oder verschieden, für eine Alkyl-Gruppe mit 1 bis 16 Kohlenstoffatomen stehen, oder $O^-M^+$ steht, worin $M^+$ ein Kation der Elemente der Gruppe IA, IB, IIA, IIB oder IIIA, IIIB des Periodensystems der Elemente ist, wobei $M^+$ bevorzugt ausgewählt ist aus den Kationen der Natrium-, Kalium-, Kupfer-, Calcium-, Barium-, Zink-, Magnesium-, Lithium- und Aluminiumatome, oder aus Kohlenwasserstoffgruppen mit 1 bis 100 Kohlenstoffatomen, oder Stickstoffgruppen mit 0 bis 100 Kohlenstoffatomen, wie etwa $NR,R_2R_3R_4^+$, worin unabhängig voneinander $R_1$, $R_2$, $R_3$ und $R_4$ für H oder eine Kohlenwasserstoffkette mit 1 bis 16 linearen, verzweigten oder cyclischen Gliedern stehen, die eventuell ein oder mehrere Heteroatome enthalten, wobei die Heteroatome bevorzugt ausgewählt sind aus Sauerstoff, Schwefel, Stickstoff, Phosphor, Silicium und/oder einer oder mehreren Doppel- oder Dreifachbindungen, wobei jedes der Glieder eventuell mit mindestens einem Substituenten substituiert ist, ausgewählt aus einer Hydroxyl-Gruppe, einer -NR"R'''-Gruppe, einer Alkoxy-Gruppe mit 1 bis 16 Kohlenstoffatomen, einer Alkyl-Gruppe mit 1 bis 16 Kohlenstoffatomen, einem Halogenatom, einer -$NO_2$-Gruppe, einer -CN-Gruppe, einer -$CF_3$-Gruppe, einer Aryl-Gruppe mit 6 bis 24 Kohlenstoffatomen, einer Aralkyl-Gruppe mit 7 bis 16 Kohlenstoffatomen, wobei R'' und R''' unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen;

wobei der zentrale Kern § für eine Gruppe steht, die 1 bis 500 Atome umfasst und eventuell ein oder mehrere Heteroatome enthält, wobei die Heteroatome bevorzugt ausgewählt sind aus Sauerstoff, Schwefel, Stickstoff, Phosphor oder Silicium.

**5.** Verwendung nach Anspruch 4, wobei der Kern m Bindungen mit m identischen Verbindungsketten aufbaut, bestehend:

• entweder aus Generationsketten, die in baumähnlicher Struktur um den Kern herum auf jeder der Bindungen befestigt sind, wobei das Ende jeder am weitesten vom zentralen Kern entfernten Generationskette, entweder an einer terminalen Gruppe oder an einer Intermediärkette befestigt ist, und das Ende jeder Intermediärkette

an einer terminalen Gruppe befestigt ist,
• oder aus Intermediärketten, die um den Kern auf jeder der Bindungen befestigt sind, wobei das Ende jeder Intermediärkette an einer terminalen Gruppe befestigt ist.

**6.** Verwendung nach Anspruch 4, wobei der Kern m-2 oder m-1 Bindungen aufbaut, wobei m für eine ganze Zahl von 3 bis 20, bevorzugt von 3 bis 10 und besonders bevorzugt von 3 bis 8 steht, mit m-2 oder m-1 identischen Verbindungsketten, bestehend:

• entweder aus Generationsketten, die in baumähnlicher Struktur um den Kern herum auf jeder der Bindungen befestigt sind, wobei das Ende jeder am weitesten vom zentralen Kern entfernten Generationskette, entweder an einer terminalen Gruppe oder an einer Intermediärkette befestigt ist, und das Ende jeder Intermediärkette an einer terminalen Gruppe befestigt ist,
• oder aus Intermediärketten, die um den Kern auf jeder der Bindungen befestigt sind, wobei das Ende jeder Intermediärkette an einer terminalen Gruppe befestigt ist;
wobei die 1 oder 2 verbleibenden Bindungen an Verbindungsgruppen, gleich oder verschieden, befestigt sind, die eventuell, insbesondere mittels einer kovalenten Bindung, miteinander verbunden sind, bestehend:

- aus entweder einem Teil der oben definierten Verbindungsketten,
- oder einem Wasserstoffatom,
- oder aus Kohlenwasserstoffgruppen, umfassend 1 bis 500 Kohlenstoffatome, wobei die Kohlenwasserstoffgruppen insbesondere aus H oder einer Kohlenwasserstoffkette mit 1 bis 200 linearen, verzweigten oder cyclischen Gliedern bestehen, die eventuell eine oder mehrere Doppel-oder Dreifachbindungen enthalten, wobei jedes der Glieder eventuell aus einem Heteroatom ausgewählt sein kann, wobei das Heteroatom bevorzugt ausgewählt werden kann aus einem Stickstoff-, Sauerstoff-, Phosphor-, silicium- oder Schwefelatom, einer Aryl-Gruppe mit 6 bis 24 Kohlenstoffatomen, einer Heteroaryl-Gruppe mit 1 bis 24 Kohlenstoffatomen, einer Carboxylgruppe, einer >C=NR-Gruppe, wobei jedes Glied eventuell mit mindestens einem Substituenten substituiert sein kann, ausgewählt aus einer Hydroxyl-Gruppe, einer -NR' 'R" '-Gruppe, einer Alkoxy-Gruppe mit 1 bis 16 Kohlenstoffatomen, einer Alkyl-Gruppe mit 1 bis 16 Kohlenstoffatomen, einem Halogenatom, einer -$NO_2$-Gruppe, einer -CN-Gruppe, einer -$CF_3$-Gruppe, einer Aryl-Gruppe mit 6 bis 24 Kohlenstoffatomen, einer Aralkyl-Gruppe mit 7 bis 16 Kohlenstoffatomen, wobei R'' und R''' unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen, wobei das erste Glied der Kohlenwasserstoffkette bevorzugt ein Sauerstoff oder ein Stickstoff ist.

**7.** Verwendung nach einem der Ansprüche 4 bis 6, wobei die Generationsketten ausgewählt sind aus jeder Kohlenwasserstoffkette mit 1 bis 12 linearen, verzweigten oder cyclischen Gliedern, die eventuell eine oder mehrere Doppel- oder Dreifachbindungen enthalten, wobei jedes der Glieder eventuell aus einem Heteroatom ausgewählt sein kann, wobei das Heteroatom bevorzugt ausgewählt ist aus Stickstoff, Sauerstoff, Schwefel, Phosphor oder Silicium, einer Aryl-Gruppe mit 6 bis 24 Kohlenstoffatomen, einer Heteoaryl-Gruppe mit 1 bis 24 Kohlenstoffatomen, wobei das Heteroelement bevorzugt ausgewählt ist aus Sauerstoff, Stickstoff oder Schwefel, einer Carboxyl-Gruppe, einer >C=NR-Gruppe, wobei jedes Glied eventuell mit mindestens einem Substituenten substituiert sein kann, ausgewählt aus einer Alkyl-Gruppe mit 1 bis 16 Kohlenstoffatomen, einem Halogenatom, einer $NO_2$-Gruppe, einer -NRR'-Gruppe, einer -CN-Gruppe, einer - $CF_3$-Gruppe, einer Hydroxyl-Gruppe, einer Alkoxy-Gruppe mit 1 bis 16 Kohlenstoffatomen, einer Aryl-Gruppe mit 6 bis 24 Kohlenstoffatomen oder einer Aralkyl-Gruppe mit 7 bis 16 Kohlenstoffatomen, wobei R und R' unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen.

**8.** Verwendung nach einem der Ansprüche 4 bis 7, wobei die Intermediärketten ausgewählt sind aus den Gruppen, die der folgenden Formel entsprechen:

-J-K-L-

worin

- J für ein Sauerstoffatom, ein Schwefelatom oder eine [-NR-] -Gruppe steht;
- K für eine Aryl-Gruppe mit 6 bis 24 Kohlenstoffatomen, eine Heteroaryl-Gruppe mit 1 bis 24 Kohlenstoffatomen, wobei das Heteroelement bevorzugt ausgewählt ist aus Sauerstoff, Stickstoff oder Schwefel, eine Alkyl-Gruppe mit 1 bis 16 Kohlenstoffatomen steht, wobei jede eventuell mit einem Halogenatom oder einer -$NO_2$-Gruppe,

-NRR'-Gruppe ,-CN-Gruppe, $-CF_3$-Gruppe, - OH-Gruppe, einer Alkyl-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen substituiert sein kann;

- L für eine Kohlenwasserstoffkette mit 0 bis 10 linearen, verzweigten oder cyclischen Gliedern steht, die eventuell eine oder mehrere Doppel-oder Dreifachbindungen enthalten, wobei jedes der Glieder eventuell ein Heteroatom sein kann, wobei das Heteroatom bevorzugt ausgewählt ist aus Sauerstoff, Schwefel, Stickstoff, Phosphor, Silicium, wobei jedes Glied eventuell mit mindestens einem Substituenten substituiert sein kann, ausgewählt aus einer Alkyl-Gruppe mit 1 bis 16 Kohlenstoffatomen, einem Halogen, einem Sauerstoffatom, $-NO_2$, -NRR' , -CN, $-CF_3$, -OH, einer Alkoxy-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen;

R und R' unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen.

**9.** Verwendung nach einem der Ansprüche 4 bis 8, wobei der Kern ausgewählt ist aus:

- einem Stickstoff- oder einem Siliciumatom;
- einer Gruppe mit der Formel

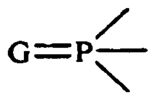

,

worin G für ein Sauerstoff-, Stickstoff-, Schwefel-, Selen-, Telluratom oder eine =NR-Gruppe steht, wobei R für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen steht, wie etwa der Thiophosphoryl-Gruppe mit der Formel

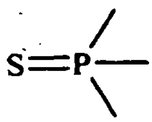

;

- einer bis-Phenyloxy-Gruppe mit der Formel

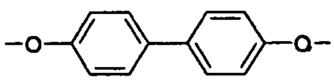

- einer 1,2-Diaminoethan-Gruppe mit der Formel

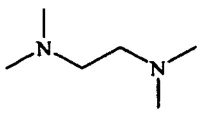

,

- einer 1,4-Diaminobutan-Gruppe mit der Formel

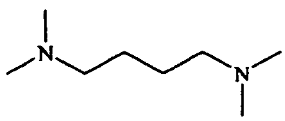

,

- einer Cyclotriphosphazen-Gruppe mit der Formel

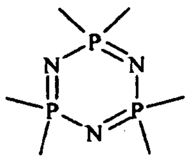,

die auch als $N_3P_3$ oder $P_3N_3$ notiert wird,

- einer Cyclotetraphosphazen-Gruppe mit der Formel

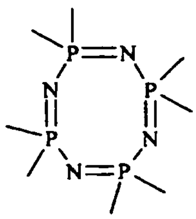,

die auch als $N_4P_4$ oder $P_4N_4$ notiert wird.

**10.** Verwendung nach einem der Ansprüche 1 bis 9 von Dendrimeren mit PAMAM-, DAB- oder PMMH-Struktur.

**11.** Verwendung nach einem der Ansprüche 4 bis 5 und 7 bis 9, von Monophosphon- oder Bisphosphon-terminierten Dendrimeren, die der folgenden allgemeinen Formel (1a) entsprechen:

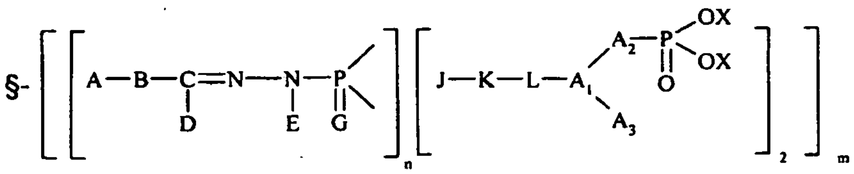

**(1a)**

worin n für eine ganze Zahl von 0 bis 3 steht, und zwar:

- wenn n = 0, entspricht die Formel (1a) der folgenden Formel (2a),

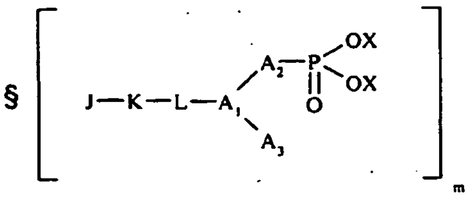

**(2a)**

- wenn n = 1, entspricht die Formel (1a) der folgenden Formel (3a),

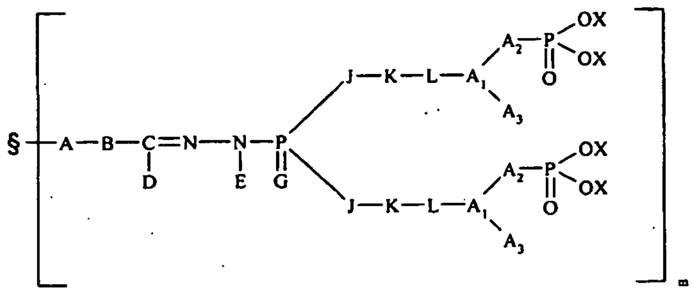

(3a)

- wenn n = 2, entspricht die Formel (1a) der folgenden Formel (4a),

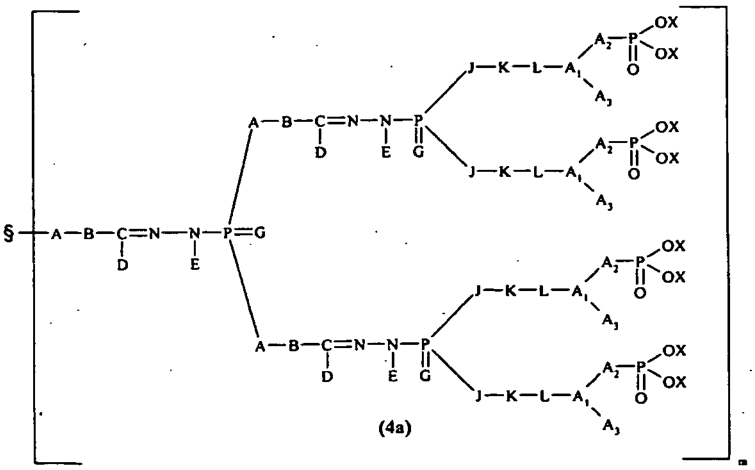

(4a)

- und wenn n = 3, entspricht die Formel (1a) der folgenden Formel (5a),

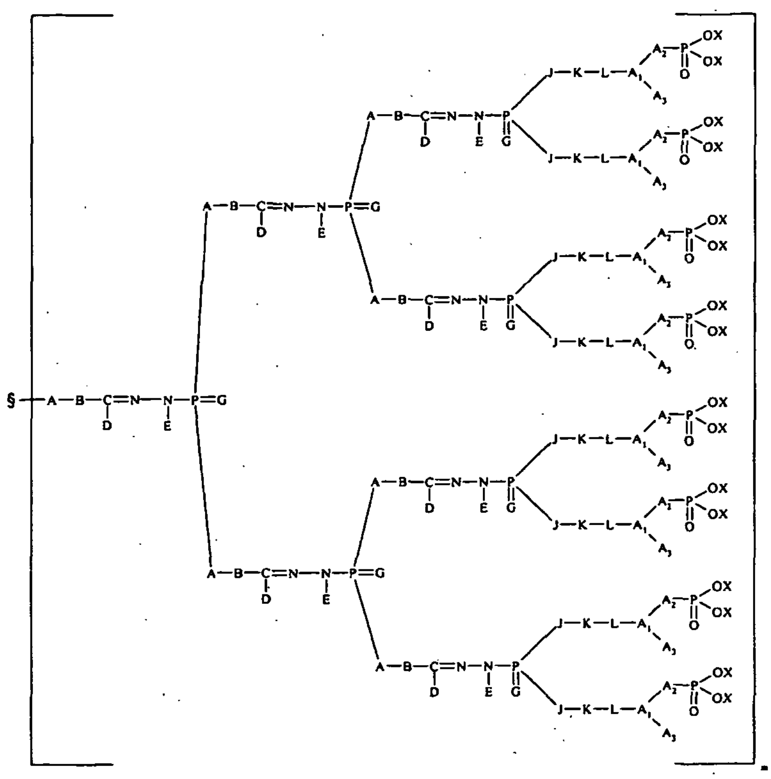

(5a)

und in diesen Formeln:

- ist der zentrale Kern § aus den folgenden Gruppen ausgewählt:

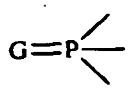 , 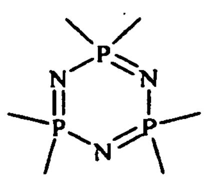 , 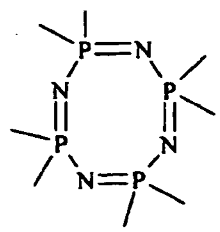 ;

- m steht für 3, 6 oder 8;
- die Generationskette entspricht der Formel:

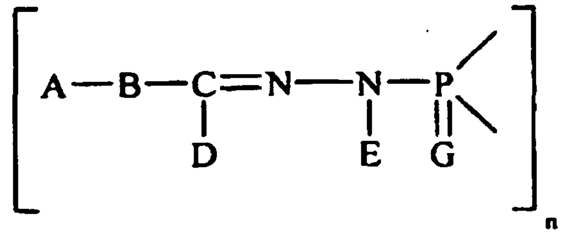

worin

- A für ein Sauerstoffatom, ein Schwefelatom, ein Phosphoratom oder eine [-NR-]-Gruppe steht;
- B für eine Aryl-Gruppe mit 6 bis 24 Kohlenstoffatomen, eine Heteroaryl-Gruppe mit 1 bis 24 Kohlenstoffatomen,

eine Alkyl-Gruppe mit 1 bis 16 Kohlenstoffatomen steht, wobei jede eventuell mit einem Halogenatom oder einer $-NO_2$ Gruppe, -NRR'-Gruppe ,-CN-Gruppe, $-CF_3$-Gruppe, - OH-Gruppe, Alkyl-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen substituiert sein kann;
- D für ein Wasserstoffatom, eine Alkylgruppe mit 1 bis 16 Kohlenstoffatomen, eine Alkoxy-Gruppe mit 1 bis 16 Kohlenstoffatomen, eine Aryl-Gruppe mit 6 bis 24 Kohlenstoffatomen, eine Aralkyl-Gruppe mit 7 bis 16 Kohlenstoffatomen steht, wobei jede eventuell mit einem Halogenatom oder einer $-N0_2$-Gruppe, -NRR'-Gruppe, -CN-Gruppe, $-CF_3$-Gruppe, - OH-Gruppe, einer Alkyl-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen substituiert sein kann;
- E für ein Wasserstoffatom, eine Alkylgruppe mit 1 bis 16 Kohlenstoffatomen, eine Alkoxy-Gruppe mit 1 bis 16 Kohlenstoffatomen, eine Aryl-Gruppe mit 6 bis 24 Kohlenstoffatomen, eine Aralkyl-Gruppe mit 7 bis 16 Kohlenstoffatomen steht, wobei jede eventuell mit einem Halogenatom oder einer $-NO_2$-Gruppe -NRR'-Gruppe, -CN-Gruppe, $-CF_3$-Gruppe, - OH-Gruppe, einer Alkyl-Gruppe mit 1 bis 16 Kohlenstoffatomen, einer Aryl-Gruppe mit 6 bis 24 Kohlenstoffatomen, einer Aralkyl-Gruppe mit 7 bis 16 Kohleristoffatomen substituiert sein kann;
- G für ein Sauerstoffatom, ein Stickstoffatom, ein Schwefelatom, ein Selenatom, ein Telluratom oder eine =NR-Gruppe steht;
R und R' unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen;

• die Intermediärkette entspricht der Formel:

-J-K-L-

worin
- J für ein Sauerstoffatom, ein Schwefelatom oder eine [-NR-] -Gruppe steht;
- K für eine Aryl-Gruppe mit 6 bis 24 Kohlenstoffatomen, eine Heteroaryl-Gruppe mit 1 bis 24 Kohlenstoffatomen, wobei das Heteroelement bevorzugt ausgewählt ist aus Sauerstoff, Stickstoff oder Schwefel, eine Alkyl-Gruppe mit 1 bis 16 Kohlenstoffatomen steht, wobei jede eventuell mit einem Halogenatom oder einer $-NO_2$-Gruppe, -NRR'-Gruppe ,-CN-Gruppe, $-CF_3$-Gruppe, - OH-Gruppe, einer Alkyl-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen substituiert sein kann;
- L für eine Kohlenwasserstoffkette mit 0 bis 10 linearen, verzweigten oder cyclischen Gliedern steht, die eventuell eine oder mehrere Doppel-oder Dreifachbindungen enthalten, wobei jedes der Glieder eventuell ein Heteroatom sein kann, wobei das Heteroatom bevorzugt ausgewählt ist aus Sauerstoff, Schwefel, Stickstoff, Phosphor, Silicium, wobei jedes Glied eventuell mit mindestens einem Substituenten substituiert sein kann, ausgewählt aus einer Alkyl-Gruppe mit 1 bis 16 Kohlenstoffatomen, einem Halogen, einem Sauerstoffatom, $-NO_2$, -NRR', -CN, -CF3, -OH, einer Alkoxy-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen;
R und R' unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen;

• die terminale Gruppe entspricht der Formel:

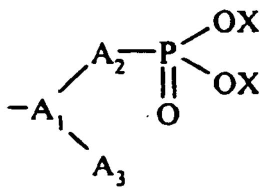

worin $A_1$, $A_3$ und X zuvor definiert wurden, wobei jedes X gleich oder verschieden ist.

**12.** Verwendung nach Anspruch 11 eines Dendrimers mit der allgemeinen Formel (1a), wobei $A_3$ für:

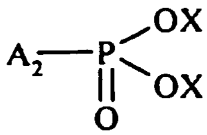,

steht, wobei die allgemeine Formel (Ia) dann der folgenden allgemeinen Formel (1) entspricht:

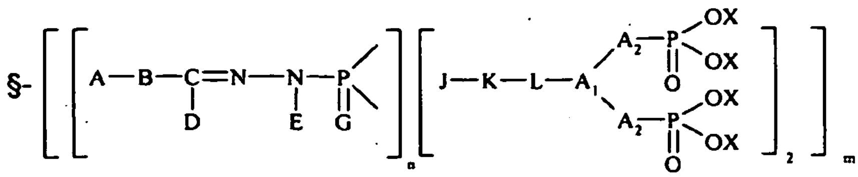 (1)

wobei §, A, B, C, D, E, G, J, K, L, $A_1$, $A_2$ und X so sind, wie in Anspruch 11 definiert.

**13.** Verwendung nach Anspruch 12 eines Dendrimers mit der allgemeinen Formel (1) mit PMMH-Struktur, wobei:

§ für

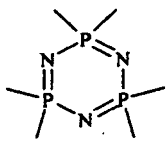

steht;
m für 6 steht;
n für 0, 1 oder 2 steht;
A für ein Sauerstoffatom steht;
B für eine Benzen-Gruppe steht;
D für einen Wasserstoff steht;
E für eine Methyl-Gruppe steht;
G für ein Schwefelatom steht;
J für ein Sauerstoffatom steht;
K für eine Benzen-Gruppe steht;
L für eine nicht substituierte, gesättigte lineare Kohlenwasserstoffkette mit zwei Kohlenstoffatomen steht;
$A_1$ für ein Stickstoffatom steht;
$A_2$ für eine $CH_2$-Gruppe steht;
X für eine Methyl-Gruppe oder ein Wasserstoffatom oder ein Natriumatom steht;
wobei das Dendrimer als GCn bezeichnet wird, wobei n oben definiert wird.

**14.** Verwendung nach einem der Ansprüche 4, 5 und 7 bis 13 von Verbindungen mit den folgenden Formeln:

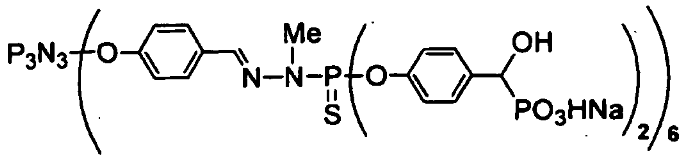

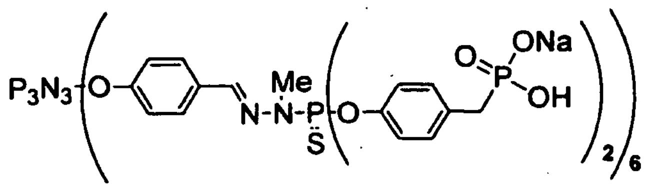

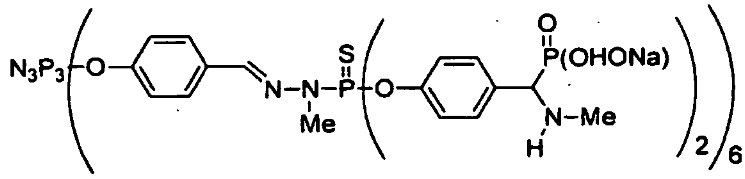

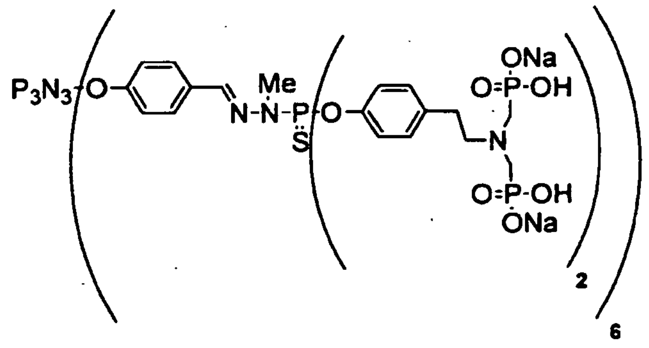

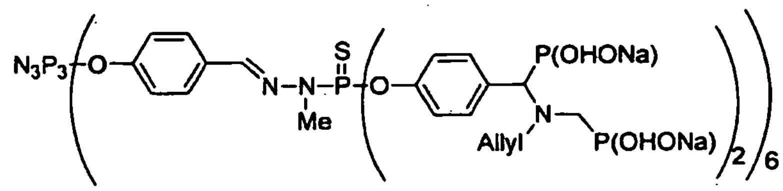

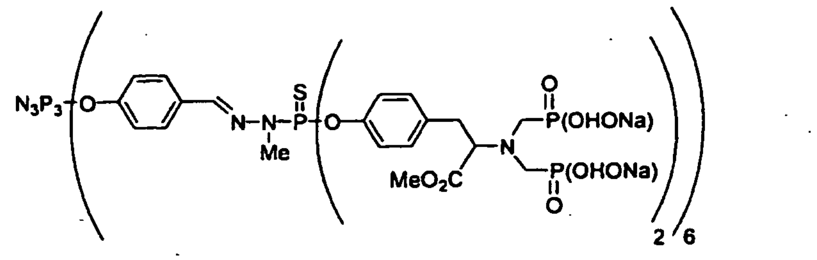

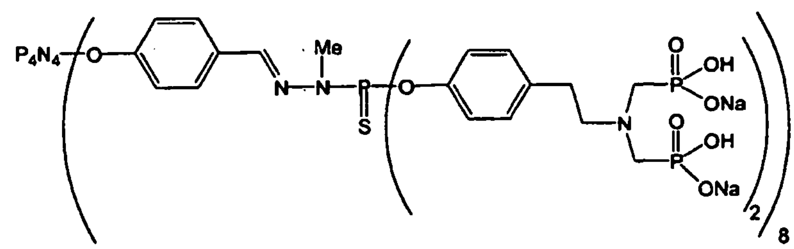

o der mit folgenden GC1-Formeln:

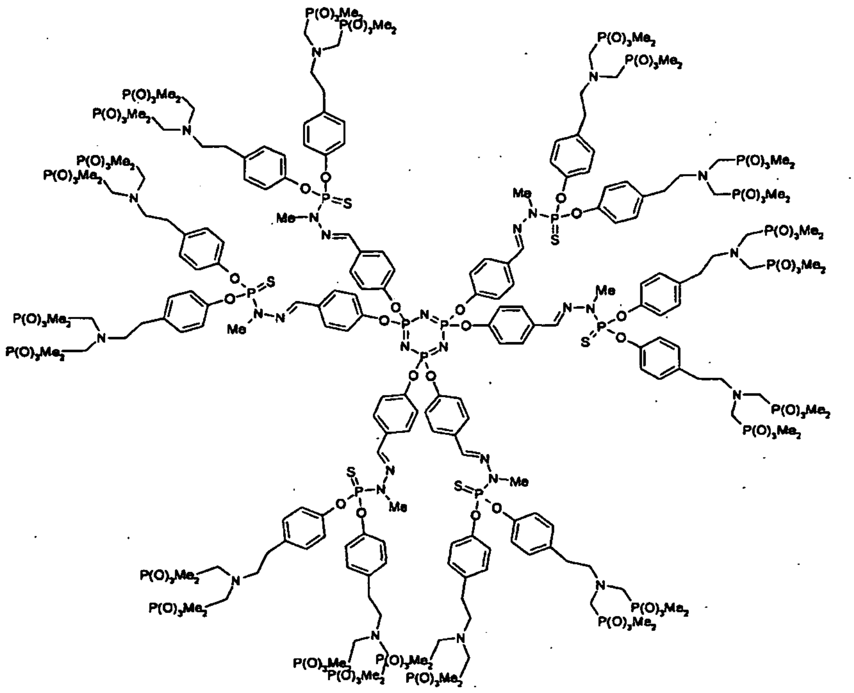

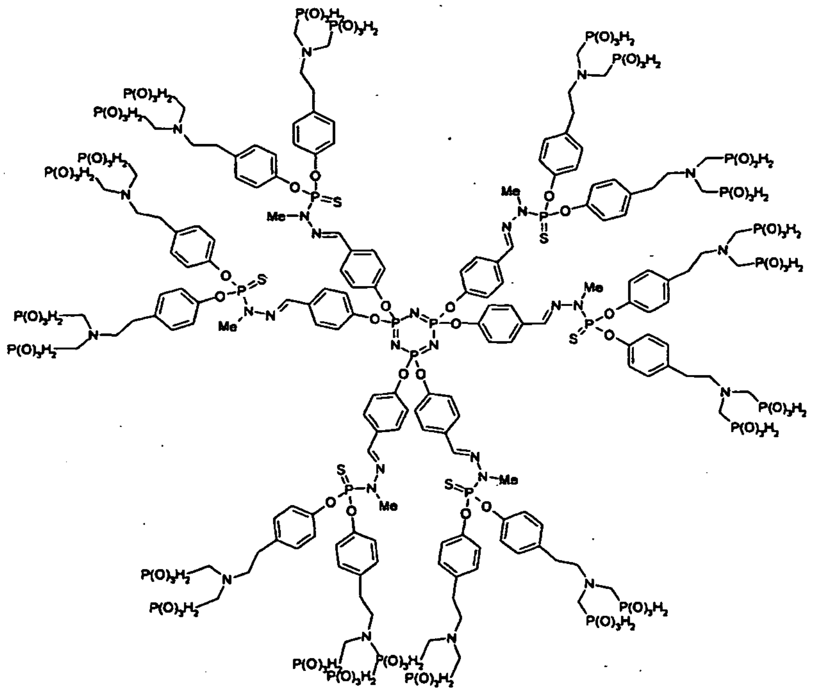

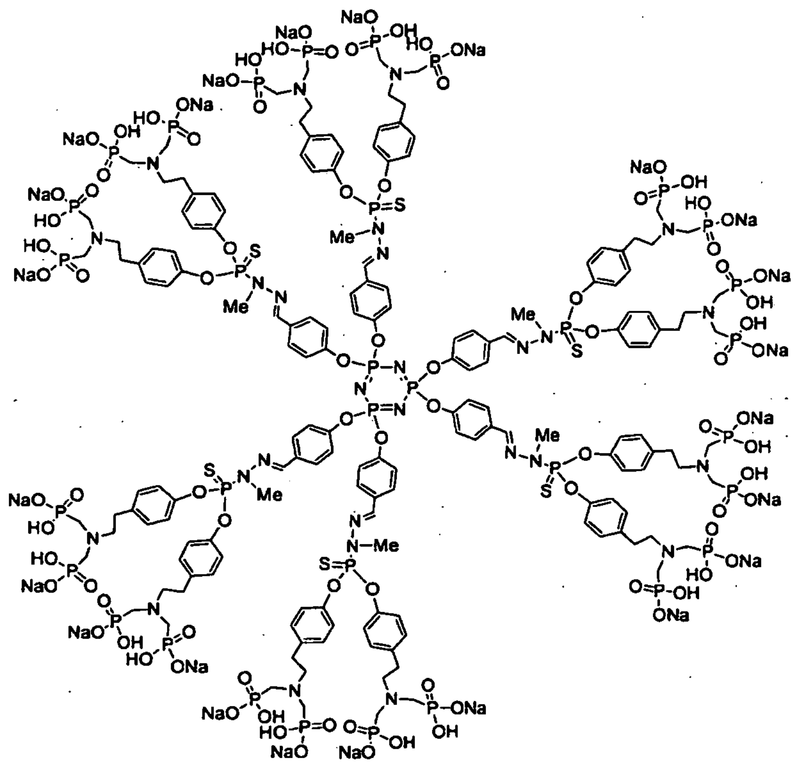

**15.** Verwendung nach einem der Ansprüche 4 und 6 bis 9 von Bisphosphon-terminierten Dendrimeren, die der folgenden allgemeinen Formel (7) entsprechen:

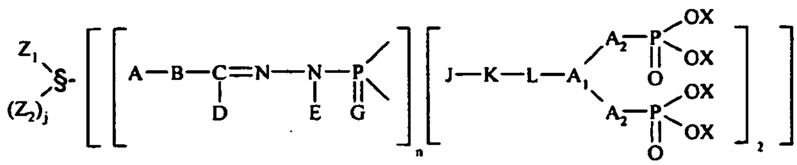

(7)

worin n für eine ganze Zahl von 0 bis 3 steht, m für 3, 6 oder 8 steht, p für m-1 oder m-2 steht, und j für 0 steht, wenn p für m-1 steht, und für 1 steht, wenn p für m-2 steht, und zwar:

- wenn p = m-1, entspricht die Formel (7) der folgenden Formel (8):

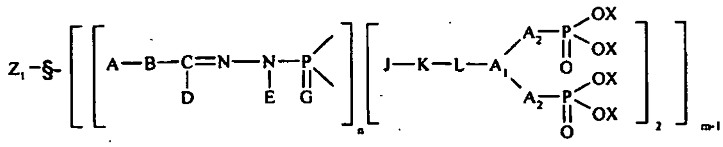

(8)

- wenn p = m-2, entspricht die Formel (7) der folgenden Formel (9):

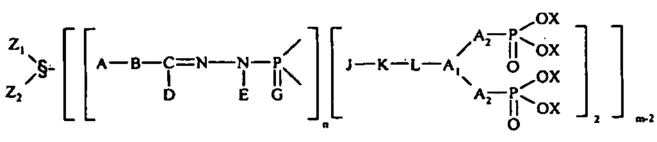

(9)

und in diesen Formeln:

• ist der zentrale Kern § aus den folgenden Gruppen ausgewählt:

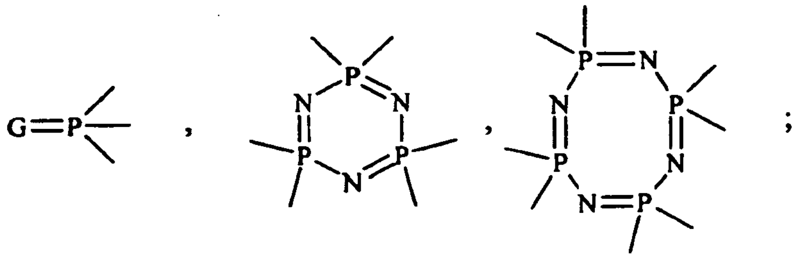

• die Generationskette entspricht der Formel:

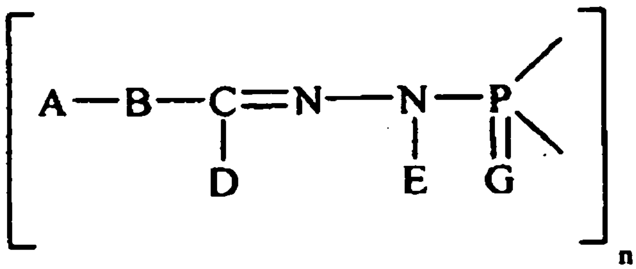

worin

- A für ein Sauerstoffatom, ein Schwefelatom, ein Phosphoratom oder eine [-NR-]-Gruppe steht;
- B für eine Aryl-Gruppe mit 6 bis 24 Kohlenstoffatomen, eine Heteroaryl-Gruppe mit 1 bis 24 Kohlenstoffatomen, eine Alkyl-Gruppe mit 1 bits 16 Kohlenstoffatomen steht, wobei jede eventuell mit einem Halogenatom oder einer -$No_2$-Gruppe, -NRR'-Gruppe ,-CR-Gruppe, -$CF_3$-Gruppe, - OH-Gruppe, Alkyl-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen substituiert sein kann;
- D für ein 4dasserstoffatom, eine Alkylgruppe mit 1 bis 16 Kohlenstoffatomen, eine Alkoxy-Gruppe mit 1 bis 16 Kohlenstoffatomen, eine Aryl-Gruppe mit 6 bis 24 Kohlenstoffatomen, eine Aralkyl-Gruppe mit 7 bis 16 Kohlenstoffatomen steht, wobei jede eventuell mit einem Halogenatom oder einer -$NO_2$-Gruppe -NRR'-Gruppe, -CN-Gruppe, -$CF_3$-Gruppe, - OH-Gruppe, einer Alkyl-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen substituiert sein kann;
- E für ein Wasserstoffatom, eine Alkylgruppe mit 1 bis 16 Kohlenstoffatomen, eine Alkoxy-Gruppe mit 1 bis 16 Kohlenstoffatomen, eine Aryl-Gruppe mit 6 bis 24 Kohlenstoffatomen, eine Aralkyl-Gruppe mit 7 bis 16 Kohlenstoffatomen steht, wobei jede eventuell mit einem Halogenatom oder einer -$NO_2$-Gruppe -NRR'-Gruppe, -CN-Gruppe, -$CF_3$-Gruppe, - OH-Gruppe, einer Alkyl-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen substituiert sein kann;
- G für ein Sauerstoffatom, ein Stickstoffatom, ein Schwefelatom, ein Selenatom, ein Telluratom oder eine =NR-Gruppe steht;

R und R' unabhängig voneinander für H oder eine Alkyl-Gruppe mit 1 bis 16 Kohlenstoffatomen, eine Aryl-Gruppe mit 6 bis 24 Kohlenstoffatomen oder eine. Aralkyl-Gruppe mit 7 bis 16 Kohlenstoffatomen stehen;

• die Intermediärkette entspricht der Formel:

-J-K-L-

worin

- J für ein Sauerstoffatom, ein Schwefelatom oder eine [-NR-] -Gruppe steht;
- K für eine Aryl-Gruppe mit 6 bis 24 Kohlenstoffatomen, eine Heteroaryl-Gruppe mit 1 bis 24 Kohlenstoffatomen, wobei das Heteroelement bevorzugt ausgewählt ist aus Sauerstoff, Stickstoff oder Schwefel, eine Alkyl-Gruppe mit 1 bis 16 Kohlenstoffatomen steht, wobei jede eventuell mit einem Halogenatom oder einer -$NO_2$-Gruppe,

-NRR'-Gruppe ,-CN-Gruppe, $-CF_3$-Gruppe, - OH-Gruppe, einer Alkyl-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen substituiert sein kann;

- L für eine Kohlenwasserstoffkette mit 0 bis 10 linearen, verzweigten oder cyclischen Gliedern steht, die eventuell eine oder mehrere Doppel-oder Dreifachbindungen enthalten, wobei jedes der Glieder eventuell ein Heteroatom sein kann, wobei das Heteroatom bevorzugt ausgewählt ist aus Sauerstoff, Schwefel, Stickstoff, Phosphor, Silicium, wobei jedes Glied eventuell mit mindestens einem Substituenten substituiert sein kann, ausgewählt aus einer Alkyl-Gruppe mit 1 bis 16 Kohlenstoffatomen, einem Halogen, einem Sauerstoffatom, $-NO_2$, -NRR', -CN, $-CF_3$, -OH, einer Alkoxy-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen;

R und R' unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen;

• die terminale Gruppe entspricht der Formel:

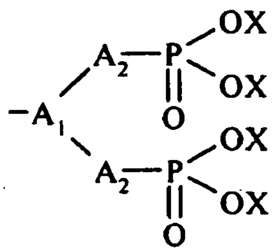

worin $A_1$, $A_2$ und X zuvor definiert wurden, wobei jedes X gleich oder verschieden ist;

$Z_1$ und $Z_2$ gleich oder verschieden, eventuell mittels einer kovalenten Bindung miteinander verbunden sind, und für H oder eine Kohlenwasserstoffkette mit 1 bis 16 linearen, verzweigten oder cyclischen Gliedern stehen, die eventuell eine oder mehrere Doppel- oder Dreifachbindungen enthalten, wobei jedes der Glieder eventuell aus einem Heteroatom ausgewählt sein kann, wobei das Heteroatom bevorzugt ausgewählt werden kann aus einem Stickstoff-, Sauerstoff-, Phosphor-, Silicium- oder Schwefelatom, einer Aryl-Gruppe mit 6 bis 24 Kohlenstoffatomen, einer Heteroaryl-Gruppe mit 1 bis 24 Kohlenstoffatomen, einer Carboxylgruppe, einer >C=NR-Gruppe, wobei jedes Glied eventuell mit mindestens einem Substituenten substituiert sein kann, ausgewählt aus einer Hydroxyl-Gruppe, einer -NR"R'''-Gruppe, einer Alkoxy-Gruppe mit 1 bis 16 Kohlenstoffatomen, einer Alkyl-Gruppe mit 1 bis 16 Kohlenstoffatomen, einem Halogenatom, einer $-NO_2$ Gruppe, einer -CN-Gruppe, einer $-CF_3$-Gruppe, einer Aryl-Gruppe mit 6 bis 24 Kohlenstoffatomen, einer Aralkyl-Gruppe mit 7 bis 16 Kohlenstoffatomen, wobei R" und R''' unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen, wobei das erste Glied der Kohlenwasserstoffkette bevorzugt ein Sauerstoff oder ein Stickstoff ist.

**16.** Verwendung nach Anspruch 15 eines Dendrimers mit der allgemeinen Formel (8) mit PMMH-Struktur, wobei:

§ für

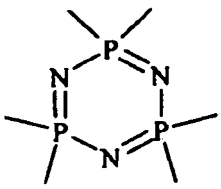

steht;
m für 6 steht;
p für 5 steht;
n für 0, 1 oder 2 steht;

A für ein Sauerstoffatom steht;
B für eine Benzen-Gruppe steht;
D für einen Wasserstoff steht;
E für eine Methyl-Gruppe steht;
G für ein Schwefelatom steht;
J für ein Sauerstoffatom steht;
K für eine Benzen-Gruppe steht;
L für eine nicht substituierte, gesättigte lineare Kohlenwasserstoffkette mit zwei Kohlenstoffatomen steht;
$A_l$ für ein Stickstoffatom steht;
$A_2$ für eine $CH_2$-Gruppe steht;
X für eine Methyl-Gruppe oder ein Wasserstoffatom oder ein Natriumatom steht;
$Z_1$ für eine Phenyloxy-Gruppe steht;
wobei das Dendrimer als GCn' bezeichnet wird, wobei n oben definiert wird.

**17.** Verwendung nach einem der Ansprüche 4, 6 bis 9 oder 15 bis 16,

- von Verbindungen mit den folgenden Formeln:

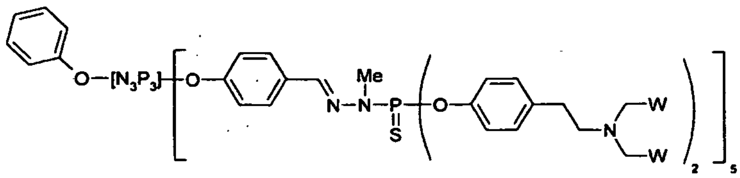

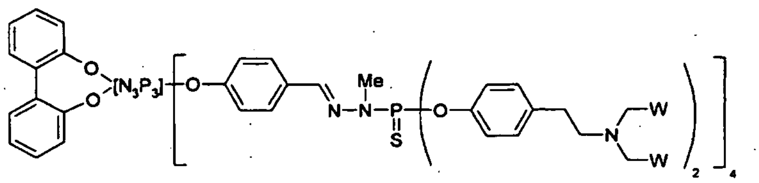

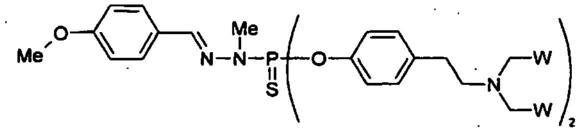

wobei W für $PO_3Me_2$, $PO_3HNa$, $PO_3H_2$ steht, wobei die Verbindungen insbesondere der Verbindung GCl' mit der folgenden Formel (10):

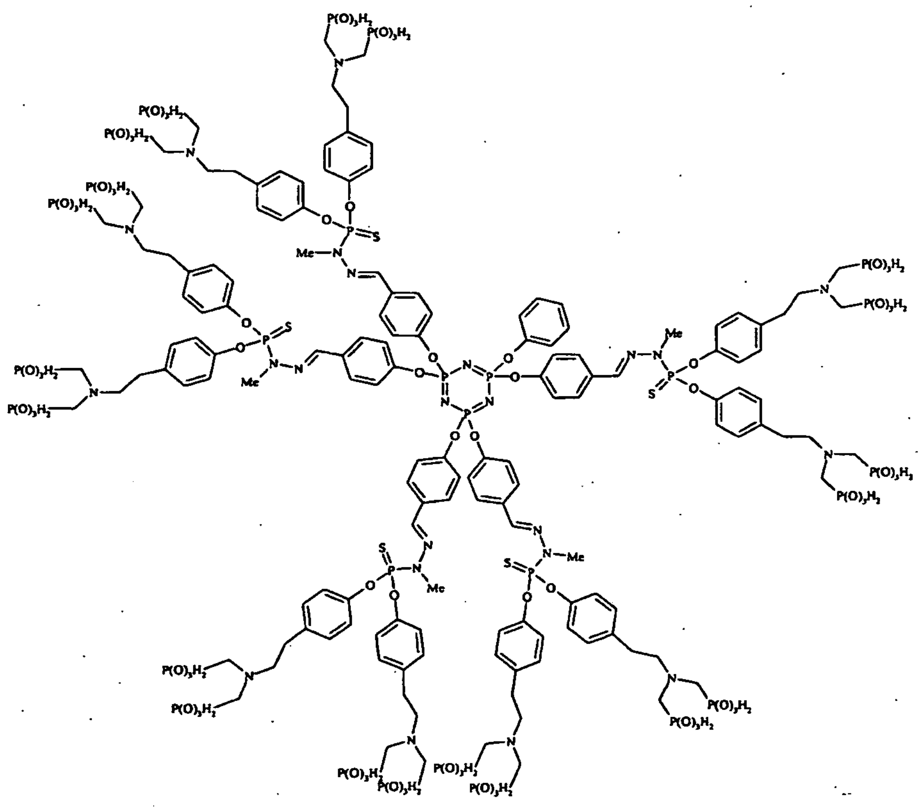

(10)

- oder Verbindungen mit der folgenden Formel entsprechen:

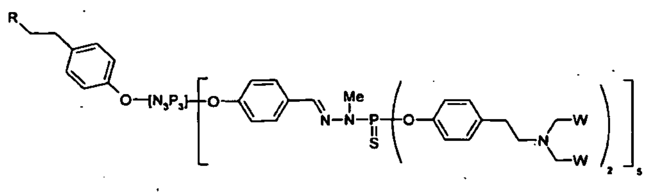

wobei W für $PO_3Me_2$ $PO_3HN$ oder $PO_3H_2$ steht und R für eine fluoreszierende Gruppe steht, ausgewählt aus:

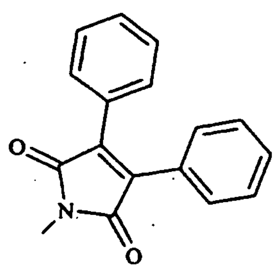

,

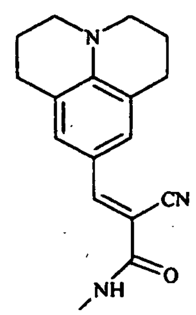

,

wobei die Verbindungen insbesondere den Verbindungen mit den folgenden Formeln entsprechen:

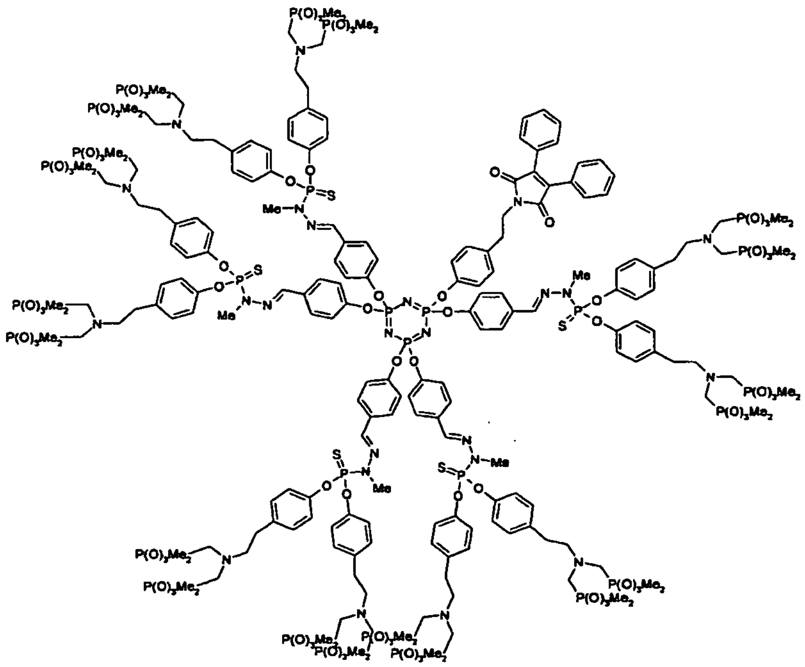

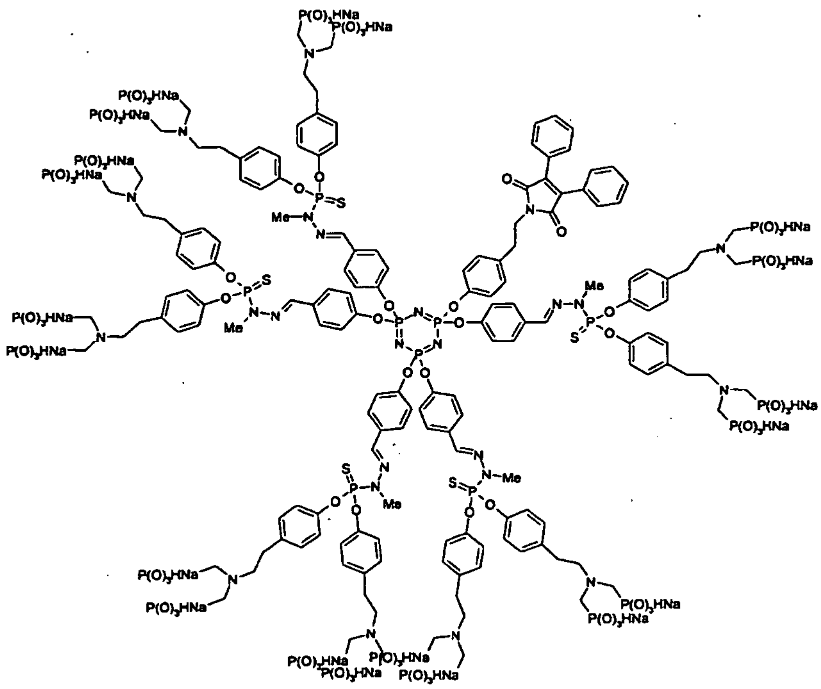

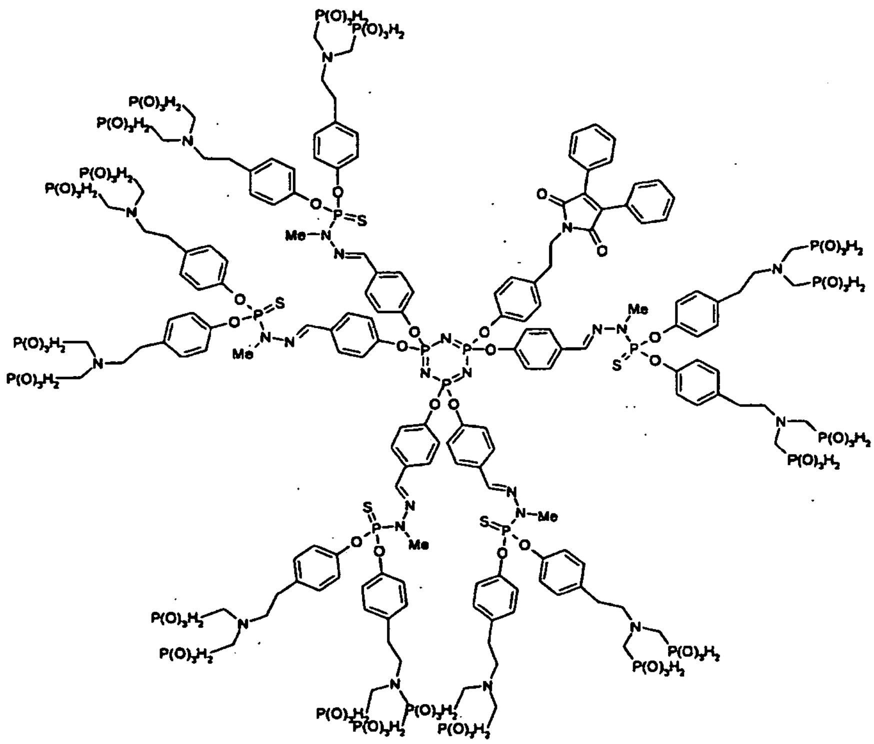

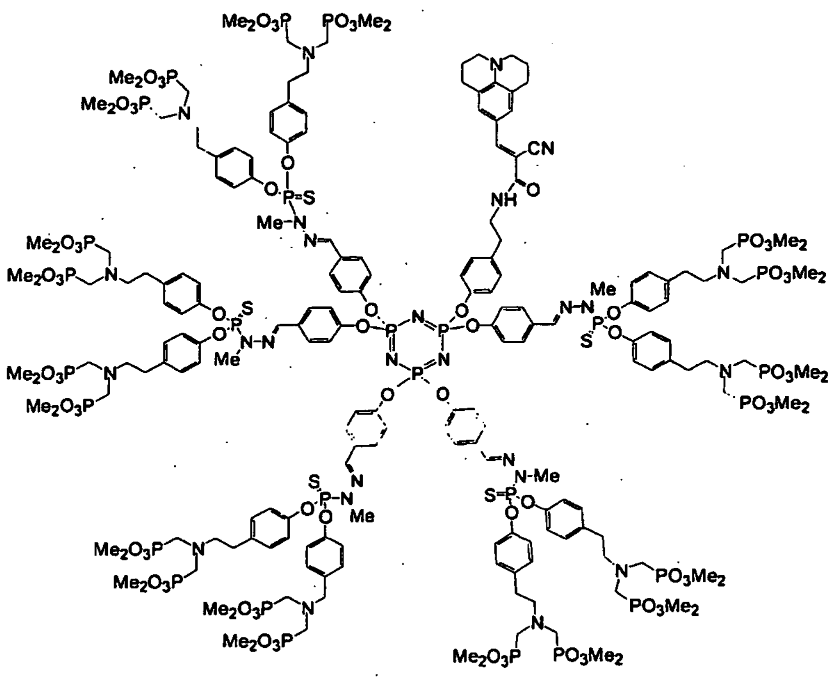

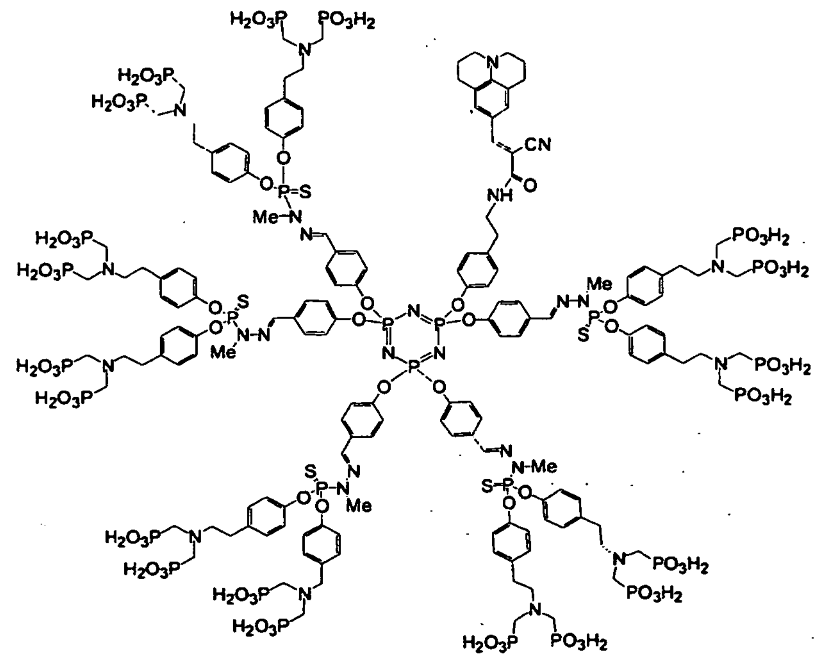

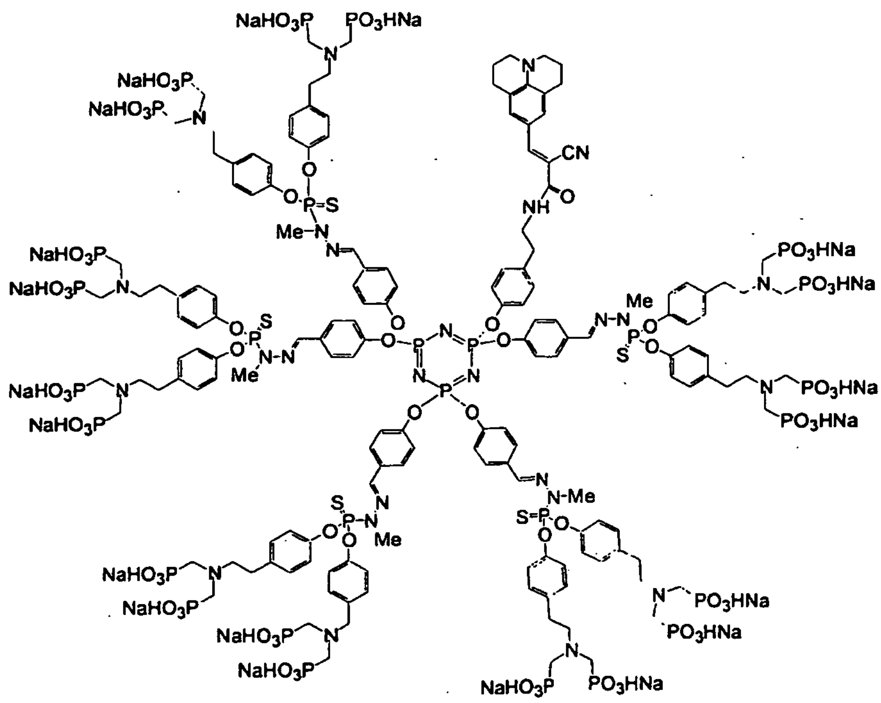

**18.** Verwendung nach einem der Ansprüche 1 bis 17, wobei die Zellen der Lymphoidlinie, die den Rezeptor NKG2D exprimieren, von NK-Zellen, von αβ-$CD8^+$-T-Lymphozyten oder von γδ-T-Lymphozyten, insbesondere von NK-Zellen, abgeleitet sind.

**19.** Verwendung nach einem der Ansprüche 1 bis 17, wobei Kulturen der Monozytenlinie in Kultur durch die Monophosphon- oder Bisphosphon-terminierten Dendrimere aktiviert werden, wobei die Aktivierung der Zellen der Monozytenlinie insbesondere:

- einer Erhöhung der Größe der aktivierten Zellen im Vergleich mit nicht aktivierten Zellen, und/oder
- einer Verringerung der Expression von Molekülen des MHC der Klasse I und der Klasse II, oder des CD14-Moleküls, im Vergleich mit nicht aktivierten Zellen, und/oder
- einer Erhöhung der nukleären Translokation des Faktors NFκB entspricht.

**20.** Verwendung nach Anspruch 19, wobei die Zellen der Monozytenlinie in Kultur im Vergleich mit Zellen der Monozytenlinie, die in Abwesenheit von Monophosphon-oder Bisphosphon-terminiertem Dendrimer kultiviert werden, eine reduzierte Apoptose aufweisen.

**21.** Zellkulturmedium, **dadurch gekennzeichnet, dass** es mindestens eine Monophosphon- oder Bisphosphon-terminierte Dendrimerverbindung enthält.

**22.** Kulturmedium nach Anspruch 21, **dadurch gekennzeichnet, dass** es ferner mindestens einen Wachstum- und/oder und Aktivierungsfaktor der NK-Zellen umfasst.

**23.** Kulturmedium nach Anspruch 21 oder 22, **dadurch gekennzeichnet, dass** es mindestens ein Interleukin umfasst, ausgewählt aus der Gruppe, umfassend: IL-2, IL-7, IL-12, IL-15, IL-18 oder IL-21.

**24.** Kulturmedium nach einem der Ansprüche 21 bis 23, **dadurch gekennzeichnet, dass** es mindestens eine Monophosphon- oder Bisphosphon-terminierte Dendrimerverbindung in Assoziation mit IL-2 umfasst.

**25.** Kulturmedium nach einem der Ansprüche 21 bis 24, **dadurch gekennzeichnet, dass** die Dendrimerverbindung den Dendrimeren, die in einem der Ansprüche 4 bis 17 definiert wurden, entspricht.

**26.** Kulturmedium nach einem der Ansprüche 21 bis 24, **dadurch gekennzeichnet, dass** die Dendrimerverbindung den Dendrimeren, die in Anspruch 14 definiert wurden, oder GC0 oder GC2, insbesondere GC1 entspricht.

**27.** Kulturmedium nach einem der Ansprüche 21 bis 26, **dadurch gekennzeichnet, dass** es die Verbindung GC1 in der Konzentration von etwa 10 bis etwa 50 μM, insbesondere etwa 20 μM, in Assoziation mit IL-2 in der Konzentration von etwa 100 bis etwa 1.000 Einheiten je ml umfasst, was einer Konzentration von etwa 4 bis etwa 40 ng je ml, insbesondere etwa 400 Einheiten je ml entspricht, was etwa 16 ng/ml entspricht, und dadurch, dass das IL-2 rekombinantem humanem IL-2 entspricht.

**28.** Verfahren zur Herstellung von Zellzusammensetzungen, angereichert mit Zellen der Lymphoidlinie, die den Rezeptor NKG2D exprimieren, insbesondere mit NK-Zellen, **dadurch gekennzeichnet, dass** es einen Schritt zum Inkontaktbringen einer biologischen Probe mit einem Monophosphon- oder Bisphosphon-terminierten Dendrimer umfasst.

**29.** Verfahren nach Anspruch 28, **dadurch gekennzeichnet, dass** es Zellen der Lymphoidlinie und/oder Zellen der Monozytenlinie umfasst.

**30.** Verfahren nach Anspruch 28 oder 29, **dadurch gekennzeichnet, dass** die biologische Probe aus humanem Blut besteht, insbesondere aus einer Fraktion mononukleärer Zellen einer Probe von humanem peripherem Blut.

**31.** Verfahren nach einem der Ansprüche 28 bis 30, **dadurch gekennzeichnet, dass** das Monophosphon- oder Bisphosphon-terminierte Dendrimer den Dendrimeren entspricht, wie in einem der Ansprüche 4 bis 17 definiert.

**32.** Verfahren nach einem der Ansprüche 28 bis 31, **dadurch gekennzeichnet, dass** das Monophosphon- oder Bisphosphon-terminierten Dendrimer den Dendrimeren, die in Anspruch 14 definiert wurden, oder GC0 oder GC2, insbesondere GC1, entspricht.

**33.** Zellzusammensetzungen, angereichert mit Zellen der Lymphoidlinie, die den Rezeptor NKG2D exprimieren, insbesondere mit NK-Zellen, wie durch das Verfahren nach einem der Ansprüche 28 bis 32 erhalten.

**34.** Zellzusammensetzungen nach Anspruch 33, **dadurch gekennzeichnet, dass** sie auch ein Monophosphon- oder Bisphosphon-terminiertes Dendrimer, insbesondere GC1, umfassen.

**35.** Verfahren zur Herstellung von aktivierten Monozyten oder von Zellzusammensetzungen, die aktivierte Monozyten umfassen, **dadurch gekennzeichnet, dass** es einen Schritt zum Inkontaktbringen einer biologischen Probe, die

Monozyten umfasst, mit einem Monophosphon-oder Bisphosphon-terminierten Dendrimer umfasst.

**36.** Verfahren nach Anspruch 35, **dadurch gekennzeichnet, dass** die biologische Probe aus humanem Blut besteht, insbesondere aus einer Fraktion mononukleärer Zellen einer Probe von humanem peripherem Blut.

**37.** Verfahren nach Anspruch 35 oder 36, **dadurch gekennzeichnet, dass** das Monophosphon- oder Bisphosphon-terminierte Dendrimer den Dendrimeren entspricht, wie in einem der Ansprüche 3 bis 16 definiert.

**38.** Verfahren nach einem der Ansprüche 35 bis 37, **dadurch gekennzeichnet, dass** das Monophosphon- oder Bisphosphon-terminierte Dendrimer den Dendrimeren, die in Anspruch 14 definiert wurden, oder GC0 oder GC2, insbesondere GC1, entspricht.

**39.** Aktivierte Monozyten oder Zellzusammensetzungen, die aktivierte Monozyten umfassen, wie durch das Verfahren nach einem der Ansprüche 35 bis 38 erhalten.

**40.** Pharmazeutische Zusammensetzung, **dadurch gekennzeichnet, dass** sie als Wirkstoff Lymphoidzellen, die den Rezeptor NKG2D exprimieren, insbesondere NK-Zellen, und mindestens ein Monophosphon- oder Bisphosphon-terminiertes Dendrimer in Assoziation mit einem pharmazeutisch verträglichen Vehikel umfasst.

**41.** Pharmazeutische Zusammensetzung nach Anspruch 40, **dadurch gekennzeichnet, dass** das Monophosphon- oder Bisphosphon-terminierte Dendrimer den Dendrimeren entspricht, wie in einem der Ansprüche 4 bis 17 definiert.

**42.** Pharmazeutische Zusammensetzung nach Anspruch 40 oder 41, **dadurch gekennzeichnet, dass** das Monophosphon-oder Bisphosphon-terminierte Dendrimer den Dendrimeren, die in Anspruch 14 definiert wurden, oder GC0 oder GC2, insbesondere GC1, entspricht.

**43.** Pharmazeutische Zusammensetzung, **dadurch gekennzeichnet, dass** sie als Wirkstoff eine Zellzusammensetzung, wie in Anspruch 33 definiert, und/oder aktivierte Monozyten oder eine Zellzusammensetzung, die aktivierte Monozyten umfasst, wie in Anspruch 39 definiert, in Assoziation mit einem pharmazeutisch verträglichen Vehikel enthält.

**44.** Pharmazeutische Zusammensetzung nach einem der Ansprüche 40 bis 43, **dadurch gekennzeichnet, dass** sie sich zur Verabreichung an ein Individuum in einer Einheitsdosis von etwa $10^5$ bis etwa $5.10^9$ Lymphoidzellen, die den Rezeptor NKG2D exprimieren, insbesondere NK-Zellen, eignet.

**45.** Verwendung von Lymphoidzellen, die den Rezeptor NKG2D exprimieren, insbesondere NK-Zellen, und von GC1 zur Herstellung von Medikamenten, die zur Behandlung und/oder Vorbeugung von Krebs, darunter Tumore der blutbildenden Gewebe, wie etwa myeloische Leukämien oder anaplastische Lymphome, und Melanome bestimmt sind.

**46.** Verwendung einer Zellzusammensetzung, wie in Anspruch 33 definiert, und/oder aktivierten Monozyten oder einer Zellzusammensetzung, die aktivierte Monozyten umfasst, wie in Anspruch 39 definiert, zur Herstellung von Medikamenten, die zur Behandlung und/oder Vorbeugung von Krebs, darunter Tumore der blutbildenden Gewebe, wie etwa myeloische Leukämien oder anaplastische Lymphome, und Melanome bestimmt sind.

**47.** Pharmazeutische Zusammensetzung, die als Wirkstoff mindestens ein Monophosphon- oder Bisphosphon-terminiertes Dendrimer in Assoziation mit einem pharmazeutisch verträglichen Vehikel enthält.

**48.** Pharmazeutische Zusammensetzung nach Anspruch 47, **dadurch gekennzeichnet, dass** das Monophosphon- oder Bisphosphon-terminierte Dendrimer den Dendrimeren entspricht, wie in einem der Ansprüche 4 bis 14 definiert.

**49.** Pharmazeutische Zusammensetzung nach Anspruch 47 oder 48, **dadurch gekennzeichnet, dass** das Monophosphon-oder Bisphosphon-terminierte Dendrimer den Dendrimeren, die in Anspruch 14 definiert wurden, oder GC0 oder GC2, insbesondere GC1, entspricht.

**50.** Monophosphon- oder Bisphosphon-terminierte Dendrimere der Generation n, wobei n für eine ganze Zahl von 0 bis 12 steht, umfassend einen zentralen Kern § mit Valenz m, wobei m für eine ganze Zahl von 3 bis 20, bevorzugt von 3 bis 10 und besonders bevorzugt von 3 bis 8 steht, wobei der Kern m-2- oder m-1-Verbindungen jeweils mit

m-2 oder m-1 identischen Verbindungsketten aufbaut, bestehend:

• aus Generationsketten, die in baumähnlicher Struktur um den Kern herum auf jeder der Bindungen befestigt sind, wenn n größer oder gleich 1 ist, wobei eine Generationskette einer gegebenen Generation verbunden ist mit

- einer Generationskette der Generation unmittelbar unter der gegebenen Generation, oder mit dem Kern, wenn die gegebene Generation gleich 1 ist, und mit
- mindestens 2 Generationsketten der Generation unmittelbar über der gegebenen Generation, oder eventuell mit mindestens einer Intermediärkette, wenn die gegebene Generation gleich n ist,
einer terminalen Gruppe, die am Ende jeder Generationskette oder gegebenenfalls am Ende jeder Intermediärkette befestigt ist, oder

• Intermediärketten, die um den Kern auf jeder der Bindungen befestigt sind, wenn n gleich 0 ist, wobei eine terminale Gruppe am Ende jeder Intermediärkette befestigt ist;
wobei die terminale Gruppe durch die folgende Formel dargestellt wird:

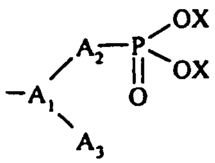

worin
$A_1$ für N; eine Gruppe P=Y steht, worin Y für O, S oder kein Atom; eine Gruppe N-R; oder eine Gruppe C-R steht; R für H oder eine Kohlenwasserstoffkette mit 1 bis 16 linearen, verzweigten oder cyclischen Glieder steht, die eventuell ein oder mehrere Heteroatome enthalten, wobei die Heteroatome bevorzugt ausgewählt sind aus Sauerstoff, Schwefel, Stickstoff, Phosphor, Silicium und/oder einer oder mehreren Doppel- oder Dreifachbindungen, wobei jedes der Glieder eventuell mit mindestens einem Substituenten substituiert ist, ausgewählt aus einer Hydroxylgruppe, einer -NR'R''-Gruppe, einer Alkoxy-Gruppe mit 1 bis 16 Kohlenstoffatomen, einer Alkyl-Gruppe mit 1 bis 16 Kohlenstoffatomen, einem Halogenatom, einer -$NO_2$-Gruppe, einer -CN-Gruppe, einer -$CF_3$-Gruppe, einer Aryl-Gruppe mit 6 bis 24 Kohlenstoffatomen, einer Aralkyl-Gruppe mit 7 bis 16 Kohlenstoffatomen, wobei R' und R" unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen;
$A_2$ für eine einfache Bindung oder eine Kohlenwasserstoffkette mit 1 bis 6 linearen, verzweigten oder cyclischen Gliedern steht, wobei jedes der Glieder eventuell ein oder mehrere Heteroatome enthält, wobei die Heteroatome bevorzugt ausgewählt sind aus einem Schwefel-, Sauerstoff-, Phosphor- oder Stickstoffatom, am stärksten bevorzugt aus einem Stickstoffatom, und eventuell mit mindestens einem Substituenten substituiert sind, ausgewählt aus H, einer Alkyl-Gruppe mit 1 bis 16 Kohlenstoffatomen, einem Halogen, einer -$NO_2$-Gruppe, einer -NRR'-Gruppe, einer -CN-Gruppe, einer -$CF_3$-Gruppe, einer Hydroxyl-Gruppe, einer Alkoxy-Gruppe mit 1 bis 16 Kohlenstoffatomen, einer Aryl- oder Heteroaryl-Gruppe mit 1 bis 24 Kohlenstoffatomen, wobei das Heteroelement bevorzugt ausgewählt ist aus Sauerstoff, Stickstoff oder Schwefel, einer Aralkyl-Gruppe mit 7 bis 16 Kohlenstoffatomen, wobei R und R' unabhängig voneinander für H oder eine Kohlenwasserstoffkette mit 1 bis 16 linearen, verzweigten oder cyclischen Gliedern stehen, die eventuell ein oder mehrere Heteroatome enthalten, wobei die Heteroatome bevorzugt ausgewählt sind aus Sauerstoff, Schwefel, Stickstoff Phosphor, Silicium und/oder einer oder mehreren Doppel- oder Dreifachbindungen, wobei jedes der Glieder eventuell mit mindestens einem Substituenten substituiert ist, ausgewählt aus einer Hydroxyl-Gruppe, einer -NR''R'''-Gruppe, einer Alkoxy-Gruppe mit 1 bis 16 Kohlenstoffatomen, einer Alkyl-Gruppe mit 1 bis 16 Kohlenstoffatomen, einem Halogenatom, einer - $NO_2$-Gruppe, einer -CN-Gruppe, einer -$CF_3$-Gruppe, einer Aryl-Gruppe mit 6 bis 24 Kohlenstoffatomen, einer Aralkyl-Gruppe mit 7 bis 16 Kohlenstoffatomen, wobei R'' und R''' unabhängig voneinander für H oder eine Alkylgruppe mit 1 bis 16 Kohlenstoffatomen, eine Aryl-Gruppe mit 6 bis 24 Kohlenstoffatomen oder eine Aralkylgruppe mit 7 bis 16 Kohlenstoffatomen stehen;
$A_3$ für H oder eine Kohlenwasserstoffkette mit 1 bis 6 linearen, verzweigten oder cyclischen Gliedern steht, wobei jedes der Glieder eventuell aus einem Heteroatom ausgewählt sein kann, wobei das Heteroatom bevorzugt ausgewählt ist aus Schwefel, Stickstoff, Phosphor oder Silicium, am stärksten bevorzugt Stickstoff, wobei jedes der Glieder eventuell mit mindestens einer Gruppe substituiert sein kann, ausgewählt aus einer Hydroxyl-Gruppe, einer -NR''R'''-Gruppe, einer Alkoxy-Gruppe mit 1 bis 16 Kohlenstoffatomen, einer Alkyl-Gruppe mit 1 bis 16 Kohlenstoffatomen, einem Halogenatom, einer -$NO_2$-Gruppe, einer -CN-Gruppe, einer -$CF_3$-Gruppe, einer Aryl-Gruppe mit 6 bis 24 Kohlenstoffatomen, einer Aralkyl-Gruppe mit 7 bis 16 Kohlenstoffatomen, wobei R'' und R''' unabhängig voneinander für H oder eine Alkyl-Gruppe mit 1 bis 16 Kohlenstoffatomen, eine Aryl-Gruppe mit 6 bis 24 Kohlenstoffatomen, oder eine Aralkylgruppe mit 7 bis 16 Kohlenstoffatomen oder

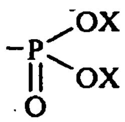 ,

wobei $A_3$ insbesondere für

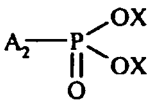

stehen kann, wobei jedes $A_2$ gleich oder verschieden ist;

wobei jedes OX, für jede Phosphon-Gruppe gleich oder verschieden, für OH, OAlkyl, worin die Alkyl-Gruppe 1 bis 16 Kohlenstoffatome umfasst, OAryl, worin, die Aryl-Gruppe 6 bis 24 Kohlenstoffatome umfasst, OAralkyl, worin die Aralkyl-Gruppe 7 bis 24 Kohlenstoffatome umfasst, OAlkylaryl, worin die Alkylaryl-Gruppe 7 bis 24 Kohlenstoffatome umfasst, $OSiR'_1R'_2R'_3$, worin $R'_1$, $R'_2$ und $R'_3$, gleich oder verschieden, für eine Alkyl-Gruppe mit 1 bis 16 Kohlenstoffatomen stehen, oder $O^-M^+$ steht, worin $M^+$ ein Kation der Elemente der Gruppe IA, IB, IIA, IIB oder IIIA, IIIB des Periodensystems der Elemente ist, wobei $M^+$ bevorzugt ausgewählt ist aus den Kationen der Natrium-, Kalium-, Kupfer-, Calcium-, Barium-, Zink-, Magnesium-, Lithium- und Aluminiumatome, oder aus Kohlenwasserstoffgruppen mit 1 bis 100 Kohlenstoffatomen, oder Stickstoffgruppen mit 0 bis 100 Kohlenstoffatomen, wie etwa $NR_1R_2R_3R_4^+$, worin unabhängig voneinander $R_1$, $R_2$, $R_3$ und $R_4$ für H oder eine Kohlenwasserstoffkette mit 1 bis 16 linearen, verzweigten oder cyclischen Gliedern stehen, die eventuell ein oder mehrere Heteroatome enthalten, wobei die Heteroatome bevorzugt ausgewählt sind aus Sauerstoff, Schwefel, Stickstoff, Phosphor, Silicium und/oder einer oder mehreren Doppel- oder Dreifachbindungen, wobei jedes der Glieder eventuell mit mindestens einem Substituenten substituiert ist, ausgewählt aus einer Hydroxyl-Gruppe, einer -NR''R'''-Gruppe, einer Alkoxy-Gruppe mit 1 bis 16 Kohlenstoffatomen, einer Alkyl-Gruppe mit 1 bis 16 Kohlenstoffatomen, einem Halogenatom, einer -$NO_2$-Gruppe, einer -CN-Gruppe, einer -$CF_3$-Gruppe, einer Aryl-Gruppe mit 6 bis 24 Kohlenstoffatomen, einer Aralkyl-Gruppe mit 7 bis 16 Kohlenstoffatomen, wobei R'' und R''' unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen;

wobei die 1 oder 2 verbleibenden Bindungen an Verbindungsgruppen, gleich oder verschieden, befestigt sind, die eventuell, insbesondere mittels einer kovalenten Bindung, miteinander verbunden sind, bestehend:

aus entweder einem Teil der oben definierten Verbindungsketten,

oder einem Wasserstoffatom,

oder aus Kohlenwasserstoffgruppen, die 1 bis 500 Kohlenstoffatome umfassen,

wobei die Kohlenwasserstoffgruppen insbesondere aus H oder einer Kohlenwasserstoffkette mit 1 bis 16 linearen, verzweigten oder cyclischen Gliedern bestehen, die eventuell eine oder mehrere Doppel- oder Dreifachbindungen enthalten, wobei jedes der Glieder eventuell aus einem Heteroatom ausgewählt sein kann, wobei das Heteroatom bevorzugt ausgewählt werden kann aus einem Stickstoff-, Sauerstoff-, Phosphor-, Silicium- oder Schwefelatom, einer Aryl-Gruppe mit 6 bis 24 Kohlenstoffatomen, einer Heteroaryl-Gruppe mit 1 bis 24 Kohlenstoffatomen, einer Carboxylgruppe, einer >C=NR-Gruppe, wobei jedes Glied eventuell mit mindestens einem Substituenten substituiert sein kann, ausgewählt aus einer Hydroxyl-Gruppe, einer -NR''R'''-Gruppe, einer Alkoxy-Gruppe mit 1 bis 16 Kohlenstoffatomen, einer Alkyl-Gruppe mit 1 bis 16 Kohlenstoffatomen, einem Halogenatom, einer -$NO_2$-Gruppe, einer -CN-Gruppe, einer -$CF_3$-Gruppe, einer Aryl-Gruppe mit 6 bis 24 Kohlenstoffatomen, einer Aralkyl-Gruppe mit 7 bis 16 Kohlenstoffatomen, wobei R'' und R''' unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen, wobei das erste Glied der Kohlenwasserstoffkette bevorzugt ein Sauerstoff oder ein Stickstoff ist; wobei der zentrale Kern §

ausgewählt ist aus:

- einem Stickstoffatom oder einem Siliciumatom;
- einer Gruppe mit der Formel

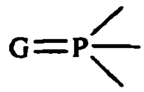

,

worin G für ein Sauerstoff-, Stickstoff-, Schwefel-, Selen-, Telluratom oder eine =NR-Gruppe steht, wobei R für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen steht, wie etwa der Thiophosphoryl-Gruppe mit der Formel

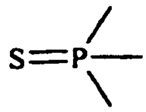

;

- einer bis-Phenyloxy-Gruppe mit der Formel

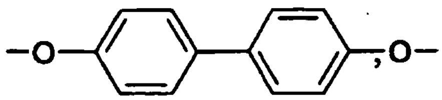

- einer 1,2-Diaminoethan-Gruppe mit der Formel

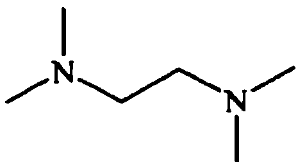

,

- einer 1,4-Diaminobutan-Gruppe mit der Formel

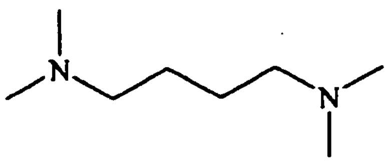

,

- einer Cyclotriphosphazen-Gruppe mit der Formel

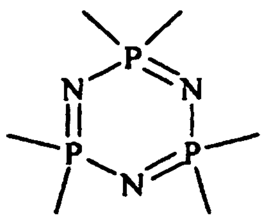

,

- einer Cyclotetraphosphazen-Gruppe mit der Formel

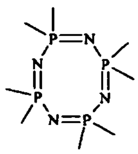

**51.** Dendrimere nach Anspruch 50, wobei die Generationsketten ausgewählt sind aus jeder Kohlenwasserstoffkette mit 1 bis 12 linearen, verzweigten oder cyclischen Gliedern, die eventuell eine oder mehrere Doppel- oder Dreifachbindungen enthalten, wobei jedes der Glieder eventuell aus einem Heteroatom ausgewählt sein kann, wobei das Heteroatom bevorzugt ausgewählt ist aus Stickstoff, Sauerstoff, Schwefel, Phosphor oder Silicium, einer Aryl-Gruppe mit 6 bis 24 Kohlenstoffatomen, einer Heteoaryl-Gruppe mit 1 bis 24 Kohlenstoffatomen, wobei das Heteroelement bevorzugt ausgewählt ist aus Sauerstoff, Stickstoff oder Schwefel, einer Carboxyl-Gruppe, einer >C=NR-Gruppe, wobei jedes Glied eventuell mit mindestens einem Substituenten substituiert sein kann, ausgewählt aus einer Alkyl-Gruppe mit 1 bis 16 Kohlenstoffatomen, einem Halogenatom, einer $NO_2$-Gruppe, einer -RR'-Gruppe, einer -CN-Gruppe, einer - $CF_3$-Gruppe, einer Hydroxyl-Gruppe, einer Alkoxy-Gruppe mit 1 bis 16 Kohlenstoffatomen, einer Aryl-Gruppe mit 6 bis 24 Kohlenstoffatomen oder einer Aralkyl-Gruppe mit 7 bis 16 Kohlenstoffatomen, wobei R und R' unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen.

**52.** Dendrimere nach Anspruch 50 oder 51, wobei die Intermediärketten ausgewählt sind aus den Gruppen, die der folgenden Formel entsprechen:

-J-K-L-

worin

- J für ein Sauerstoffatom, ein Schwefelatom oder eine [-NR-]-Gruppe steht;
- K für eine Aryl-Gruppe mit 6 bis 24 Kohlenstoffatomen, eine Heteroaryl-Gruppe mit 1 bis 24 Kohlenstoffatomen, wobei das Heteroelement bevorzugt ausgewählt ist aus Sauerstoff, Stickstoff oder Schwefel, eine Alkyl-Gruppe mit 1 bis 16 Kohlenstoffatomen steht, wobei jede eventuell mit einem Halogenatom oder einer -$NO_2$-Gruppe, -NRR'-Gruppe ,-CN-Gruppe, -$CF_3$-Gruppe, - OH-Gruppe, einer Alkyl-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen substituiert sein kann;
- L für eine Kohlenwasserstoffkette mit 0 bis 10 linearen, verzweigten oder cyclischen Gliedern steht, die eventuell eine oder mehrere Doppel-oder Dreifachbindungen enthalten, wobei jedes der Glieder eventuell ein Heteroatom sein kann, wobei das Heteroatom bevorzugt ausgewählt ist aus Sauerstoff, Schwefel, Stickstoff, Phosphor, Silicium, wobei jedes Glied eventuell mit mindestens einem Substituenten substituiert sein kann, ausgewählt aus einer Alkyl-Gruppe mit 1 bis 16 Kohlenstoffatomen, einem Halogen, einem Sauerstoffatom, -$NO_2$, -NRR', -CN, -$CF_3$, -OH, einer Alkoxy-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen;

R und R' unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen.

**53.** Dendrimere nach einem der Ansprüche 50 bis 52 mit PAMAM-, DAB- oder PMMH-Struktur.

**54.** Bisphosphon-terminierte Dendrimere nach einem der Ansprüche 50 und 52, die der folgenden allgemeinen Formel (7) entsprechen:

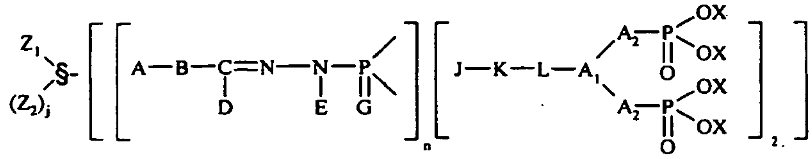
(7)

worin n für eine ganze Zahl von 0 bis 3 steht, m für 3, 6 oder 8 steht, p für m-1 oder m-2 steht, und j für 0 steht, wenn p für m-1 steht, und für 1 steht, wenn p für m-2 steht, und zwar:

- wenn p = m-1, entspricht die Formel (7) der folgenden Formel (8):

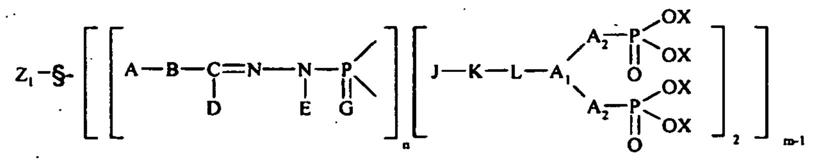 (8)

- wenn p = m-2, entspricht die Formel (7) der folgenden Formel (9):

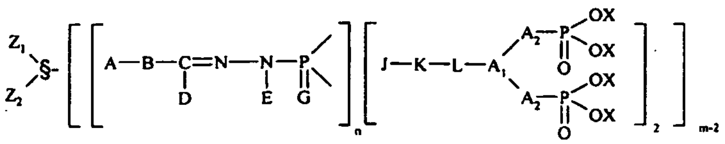 (9)

und in diesen Formeln:

• ist der zentrale Kern § aus den folgenden Gruppen ausgewählt:

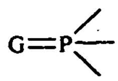 , 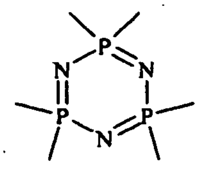 , 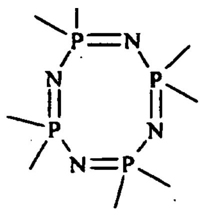 ;

• die Generationskette entspricht der Formel:

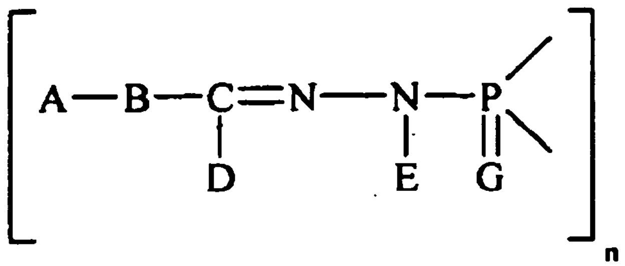

worin

- A für ein Sauerstoffatom, ein Schwefelatom, ein Phosphoratom oder eine [-NR-]-Gruppe steht;
- B für eine Aryl-Gruppe mit 6 bis 24 Kohlenstoffatomen, eine Heteroaryl-Gruppe mit 1 bis 24 Kohlenstoffatomen, eine Alkyl-Gruppe mit 1 bis 16 Kohlenstoffatomen steht, wobei jede eventuell mit einem Halogenatom oder einer -$NO_2$-Gruppe, -NRR'-Gruppe ,-CN-Gruppe, -$CF_3$-Gruppe, - OH-Gruppe, Alkyl-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen substituiert sein kann;
- D für ein Wasserstoffatom, eine Alkylgruppe mit 1 bis 16 Kohlenstoffatomen, eine Alkoxy-Gruppe mit 1 bis 16 Kohlenstoffatomen, eine Aryl-Gruppe mit 6 bis 24 Kohlenstoffatomen, eine Aralkyl-Gruppe mit 7 bis 16 Kohlenstoffatomen steht, wobei jede eventuell mit einem Halogenatom oder einer -$NO_2$-Gruppe- NRR'-Gruppe, -CN-Gruppe, -$CF_3$-Gruppe, - OH-Gruppe, einer Alkyl-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen substituiert sein kann;
- E für ein Wasserstoffatom, eine Alkylgruppe mit 1 bis 16 Kohlenstoffatomen, eine Alkoxy-Gruppe mit 1 bis 16 Kohlenstoffatomen, eine Aryl-Gruppe mit 6 bis 24- Kohlenstoffatomen, eine Aralkyl-Gruppe mit 7 bis 16 Kohlenstoffatomen steht, wobei jede eventuell mit einem Halogenatom oder einer $-NO_2$-Gruppe -NRR'-Gruppe, -CN-Gruppe, $-CF_3$-Gruppe, - OH-Gruppe, einer Alkyl-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen substituiert sein kann;
- G für ein Sauerstoffatom, ein Stickstoffatom, ein Schwefelatom, ein Selenatom, ein Telluratom oder eine =NR-Gruppe steht;
R und R' unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen;

• die Intermediärkette entspricht der Formel:

-J-K-L-

worin
- J für ein Sauerstoffatom, ein Schwefelatom oder eine [-NR-]-Gruppe steht;
- K für eine Aryl-Gruppe mit 6 bis 24 Kohlenstoffatomen, eine Heteroaryl-Gruppe mit 1 bis 24 Kohlenstoffatomen, wobei das Heteroelement bevorzugt ausgewählt ist aus Sauerstoff, Stickstoff oder Schwefel, eine Alkyl-Gruppe mit 1 bis 16 Kohlenstoffatomen steht, wobei jede eventuell mit einem Halogenatom oder einer -NO2-Gruppe,-NRR'-Gruppe ,-CN-Gruppe, -CF3-Gruppe, - OH-Gruppe, einer Alkyl-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen substituiert sein kann;
- L für eine Kohlenwasserstoffkette mit 0 bis 10 linearen, verzweigten oder cyclischen Gliedern steht, die eventuell eine oder mehrere Doppel-oder Dreifachbindungen enthalten, wobei jedes der Glieder eventuell ein Heteroatom sein kann, wobei das Heteroatom bevorzugt ausgewählt ist aus Sauerstoff, Schwefel, Stickstoff, Phosphor, Silicium, wobei jedes Glied eventuell mit mindestens einem Substituenten substituiert sein kann, ausgewählt aus einer Alkyl-Gruppe mit 1 bis 16 Kohlenstoffatomen, einem Halogen, einem Sauerstoffatom, -NO2, -NRR', -CN, -CF3, -OH, einer Alkoxy-Gruppe mit 1 bis 16 Kohlenstoffatomen, einer Aryl-Gruppe mit 6 bis 24 Kohlenstoffatomen, einer Aralkyl-Gruppe mit 7 bis 16 Kohlenstoffatomen;
R und R' unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen;

• die terminale Gruppe entspricht der Formel:

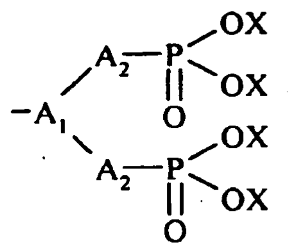

worin $A_1$, $A_2$ und X zuvor definiert wurden, wobei jedes X gleich oder verschieden ist;
$Z_1$ und $Z_2$ gleich oder verschieden, eventuell mittels einer kovalenten Bindung miteinander verbunden sind, und für H oder eine Kohlenwasserstoffkette mit 1 bis 16 linearen, verzweigten oder cyclischen Gliedern stehen, die eventuell eine oder mehrere Doppel- oder Dreifachbindungen enthalten, wobei jedes der Glieder eventuell aus einem Heteroatom ausgewählt sein kann, wobei das Heteroatom bevorzugt ausgewählt werden kann aus einem Stickstoff-, Sauerstoff-, Phosphor-, Silicium- oder Schwefelatom, einer Aryl-Gruppe mit 6 bis 24 Kohlenstoffatomen, einer Heteroaryl-Gruppe mit 1 bis 24 Kohlenstoffatomen, einer Carboxylgruppe, einer >C=NR-Gruppe, wobei jedes Glied eventuell mit mindestens einem Substituenten substituiert sein kann, ausgewählt aus einer Hydroxyl-Gruppe, einer -NR''R'''-Gruppe, einer Alkoxy-Gruppe mit 1 bis 16 Kohlenstoffatomen, einer Alkyl-Gruppe mit 1 bis 16 Kohlenstoffatomen, einem Halogenatom, einer $-NO_2$-Gruppe, einer -CN-Gruppe, einer $-CF_3$-Gruppe, einer Aryl-Gruppe mit 6 bis 24 Kohlenstoffatomen, einer Aralkyl-Gruppe mit 7 bis 16 Kohlenstoffatomen, wobei R" und R''' unabhängig voneinander für H oder eine Alkyl-Gruppe mit 1 bis 16 Kohlenstoffatomen, eine Aryl-Gruppe mit 6 bis 24 Kohlenstoffatomen oder eine Aralkyl-Gruppe mit 7 bis 16 Kohlenstoffatomen stehen, wobei das erste Glied der Kohlenwasserstoffkette bevorzugt ein Sauerstoff oder ein Stickstoff ist.

**55.** Dendrimere nach Anspruch 54 mit PMMH-Struktur, die der allgemeinen Formel (8) entsprechen, wobei

§ für

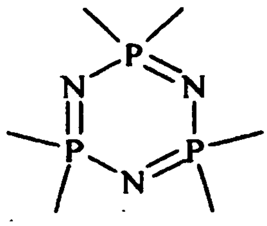

steht;
m für 6 steht;
p für 5 steht;
n für 0, 1 oder 2 steht;
A für ein Sauerstoffatom steht;
B für eine Benzen-Gruppe steht;
D für einen Wasserstoff steht;
E für eine Methylgruppe steht;
G für ein Schwefelatom steht;
J für ein Sauerstoffatom steht;
K für eine Benzen-Gruppe steht;
L für eine nicht substituierte, gesättigte lineare Kohlenwasserstoffkette mit zwei Kohlenstoffatomen steht;
$A_1$ für ein Stickstoffatom steht;
$A_2$ für eine $CH_2$-Gruppe steht;
X für eine Methyl-Gruppe oder ein Wasserstoffatom oder ein Natriumatom steht;
$Z_1$ für eine Phenyloxy-Gruppe steht;

wobei das Dendrimer als GCn' bezeichnet wird, wobei n oben definiert wird.

**56.** Dendrimere nach einem der Ansprüche 50 bis 55 mit den folgenden Formeln:

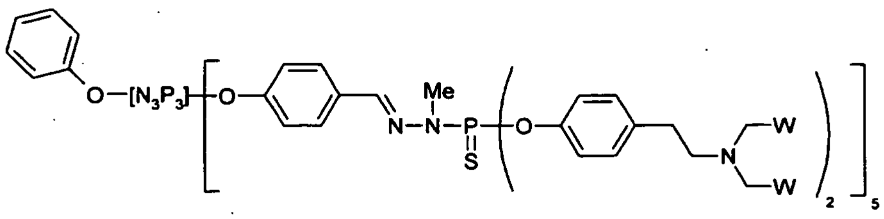

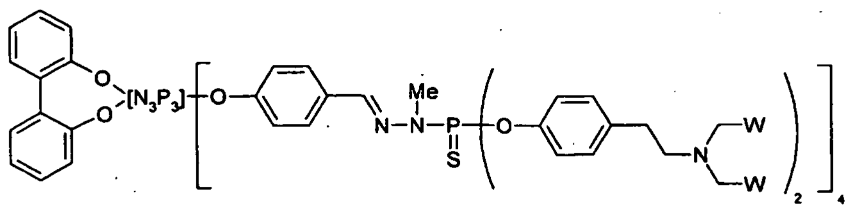

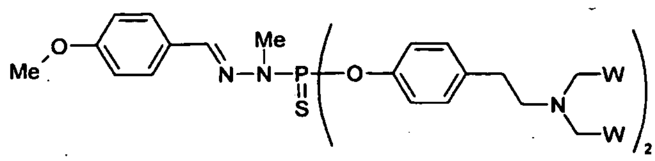

wobei W für $PO_3Me_2$, $PO_3HNa$ oder $PO_3H_2$ steht, wobei die Dendrimere insbesondere der Verbindung GC1' mit der folgenden Formel (10):

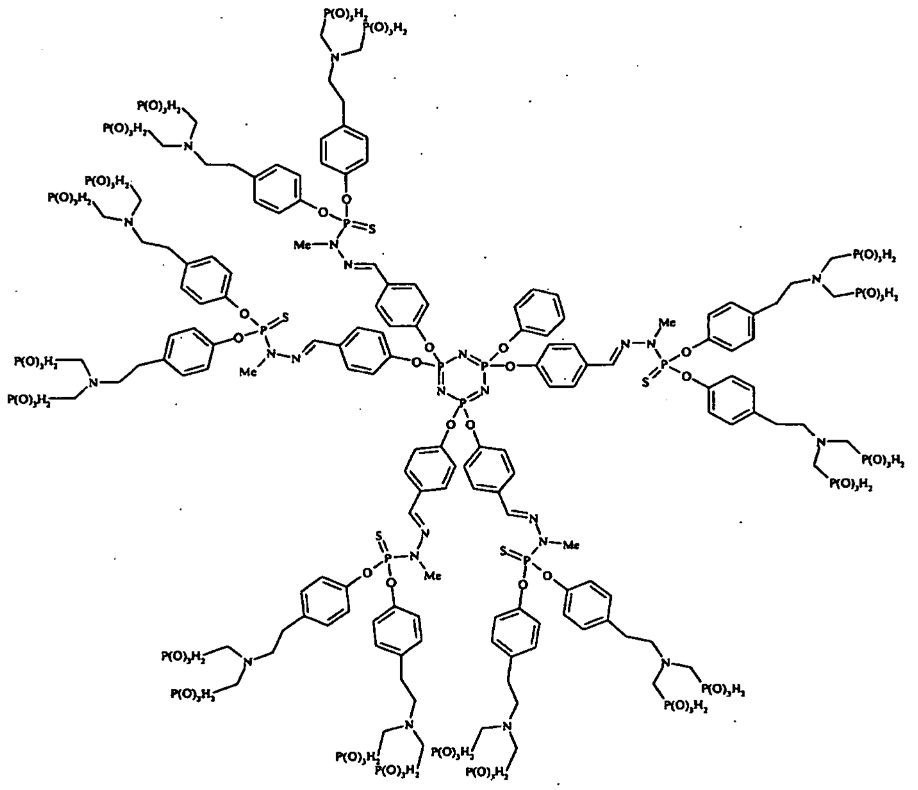

(10)

entsprechen.

**57.** Dendrimere nach einem der Ansprüche 50 bis 54 mit der folgenden Formel:

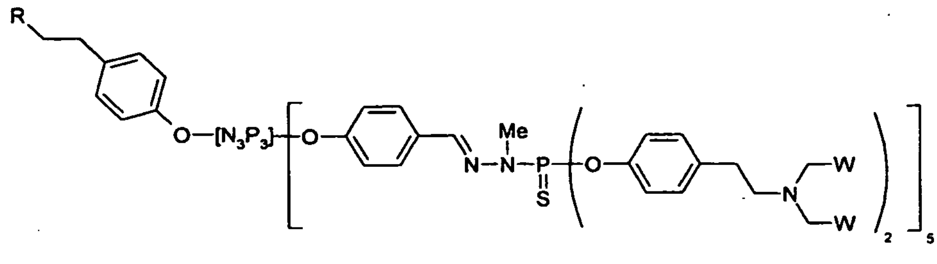

wobei W für $PO_3Me_2$, $PO_3HNa$ oder $PO_3H_2$ steht und R für eine fluoreszierende Gruppe steht, ausgewählt aus:

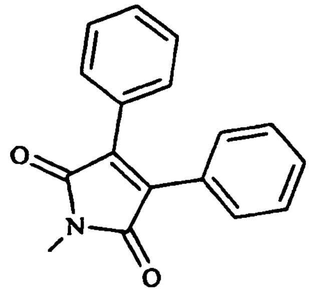 , 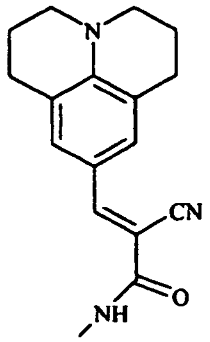 ,

wobei die Dendrimere insbesondere den Verbindungen mit den folgenden Formeln entsprechen:

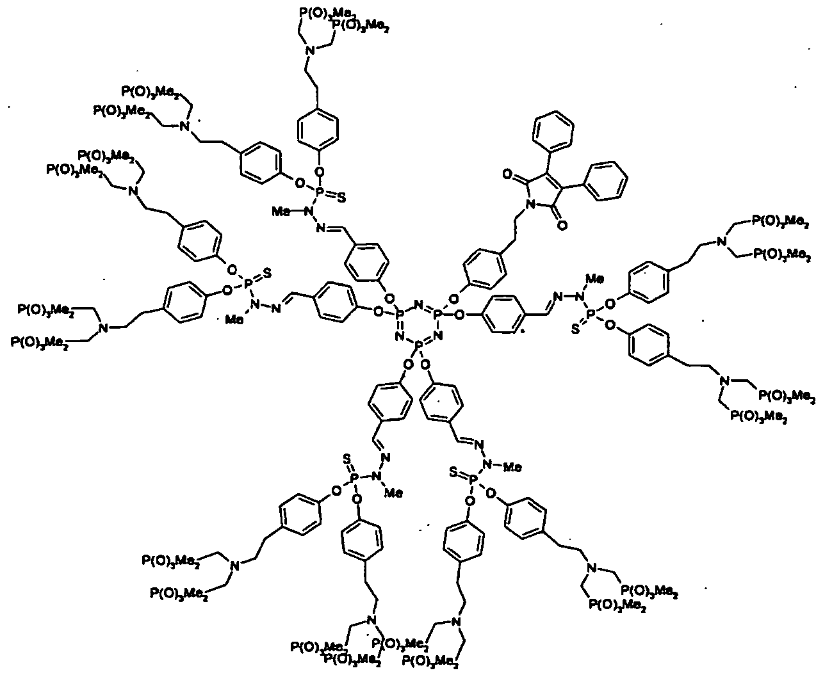

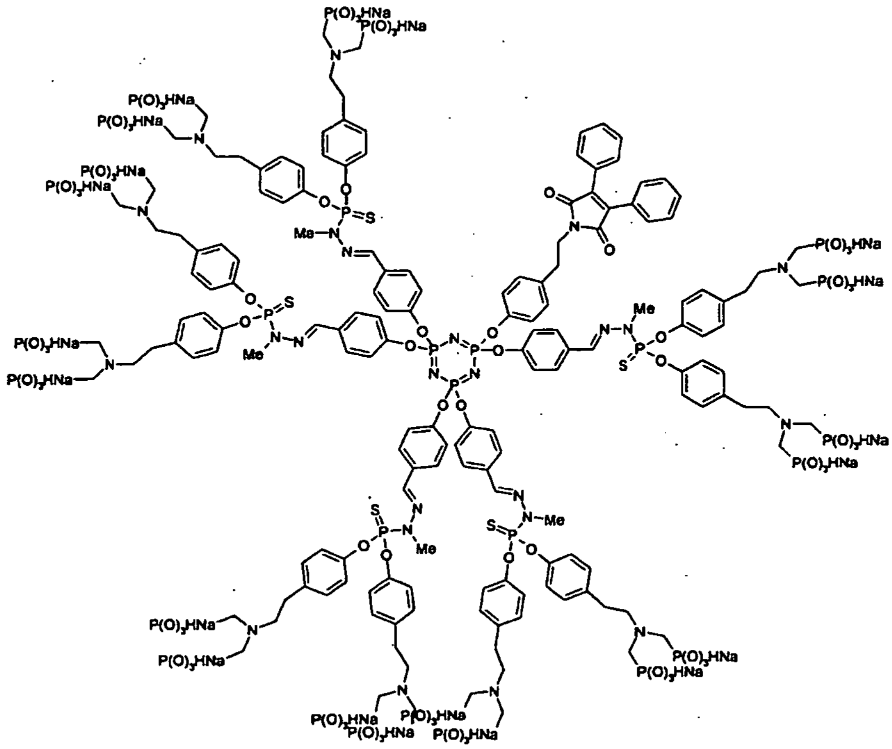

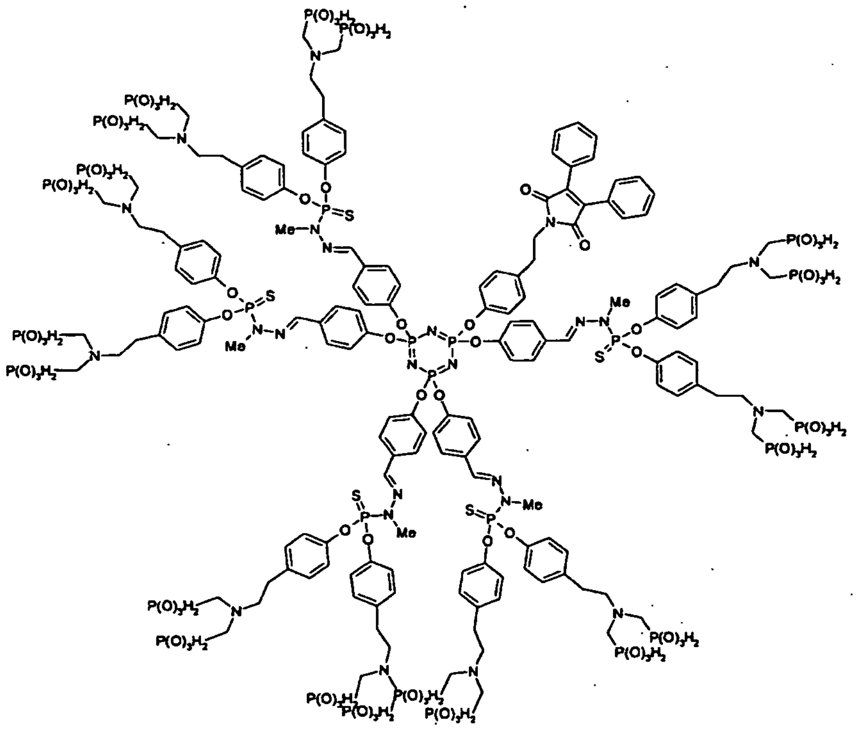

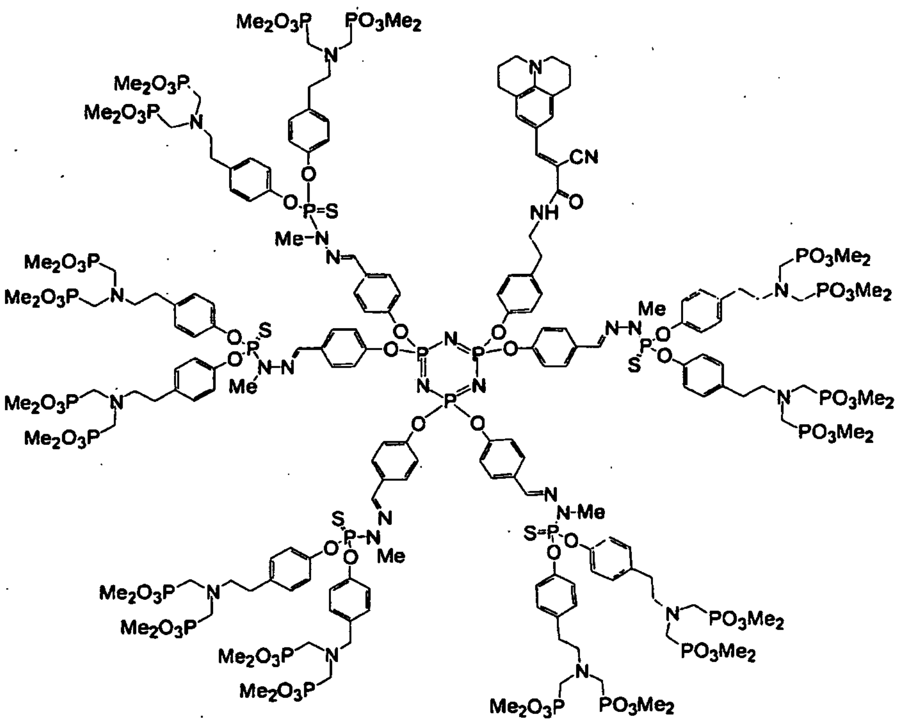

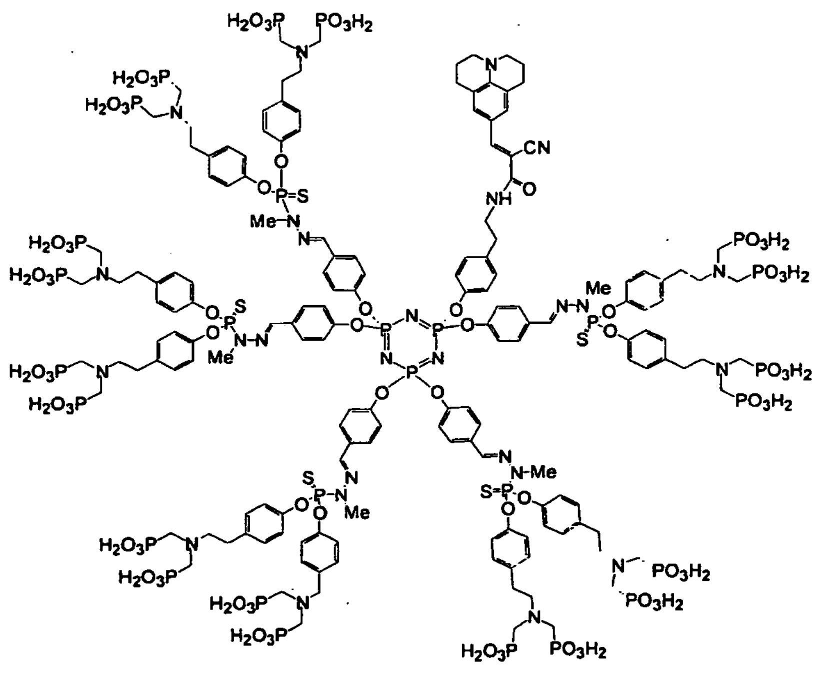

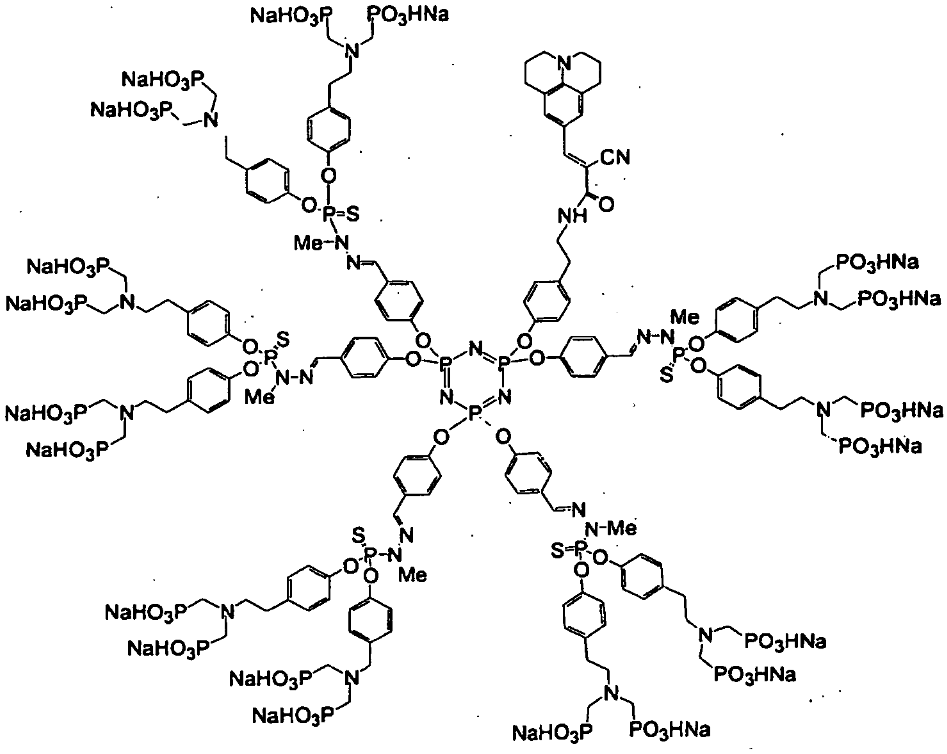

**58.** Bisphosphon-terminierte Dendrimere nach Anspruch 50 mit der folgenden Formel:

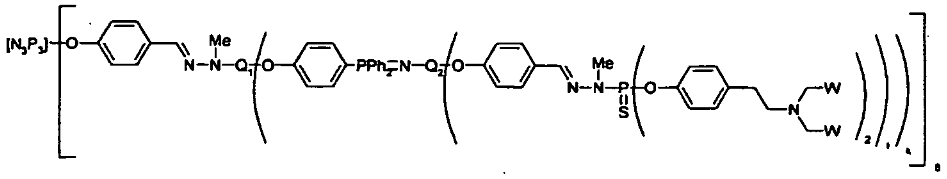

wobei W für $PO_3Me_2$, $PO_3HNa$ oder $PO_3H_2$ steht, $Q_1$ und $Q_2$, gleich oder verschieden, für P=S oder Cyclotriphosphazen ($N_3P_3$) stehen, 1 für 2 steht, wenn $Q_2$ für P=S steht, oder für 5, wenn $Q_2$ für $N_3P_3$ steht, und k für 2 steht, wenn $Q_1$ für P=S steht, oder für 5, wenn $Q_1$ für $N_3P_3$ steht, wobei die Dendrimere insbesondere durch die folgenden Formeln dargestellt werden:

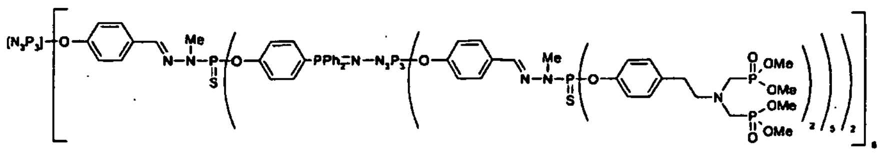

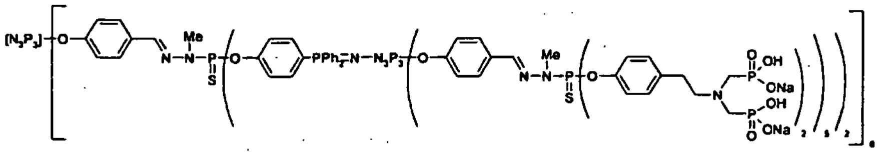

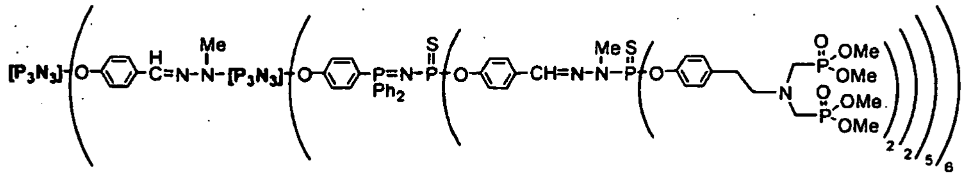

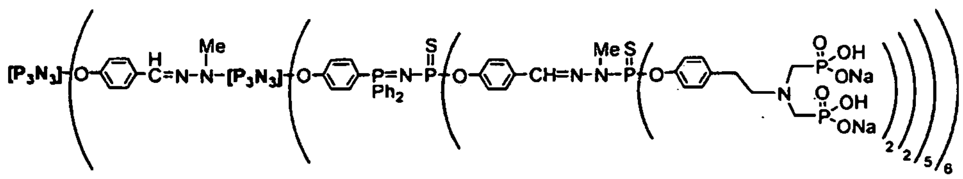

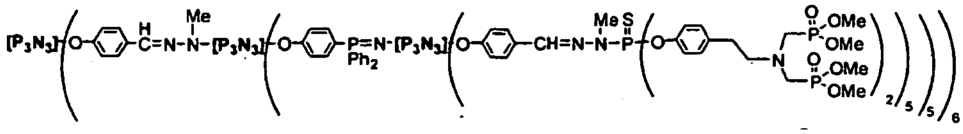

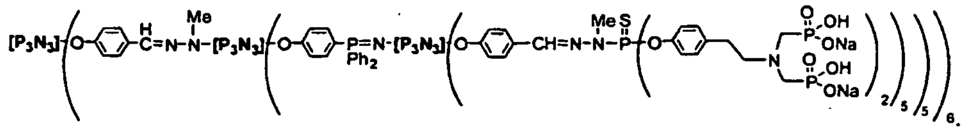

**59.** Monophosphon- oder Bisphosphon-terminierte Dendrimere nach Anspruch 50 mit der folgenden Formel:

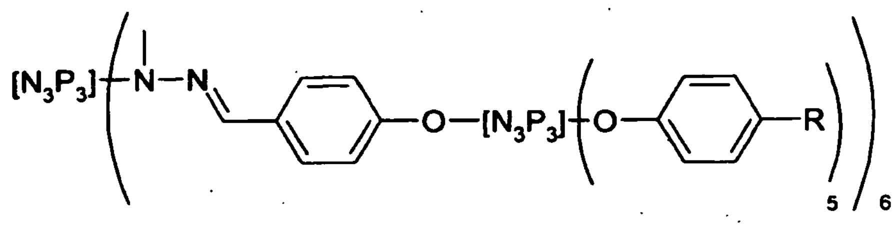

wobei R für eine Gruppe steht, ausgewählt aus

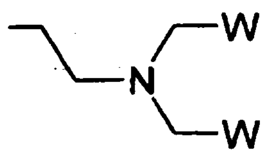

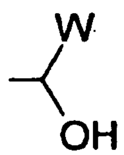

worin W für $PO_3Me_2$, $PO_3HN$ oder $PO_3H_2$ steht.

**60.** Bisphosphon-terminierte Dendrimere nach Anspruch 50 mit der folgenden Formel:

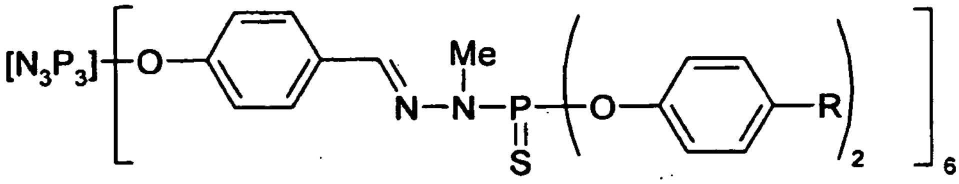

wobei R für eine Gruppe steht, ausgewählt aus:

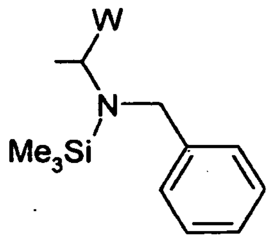
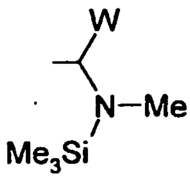
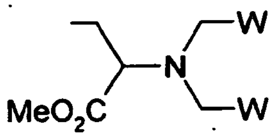
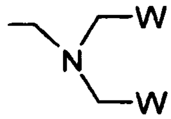

worin W für $PO_3Si_2Me_6$, $PO_3Me_2$, $PO_3HNa$ oder $PO_3H_2$ steht.

**61.** Monophosphon-terminierte Dendrimere nach Anspruch 50 mit der folgenden Formel:

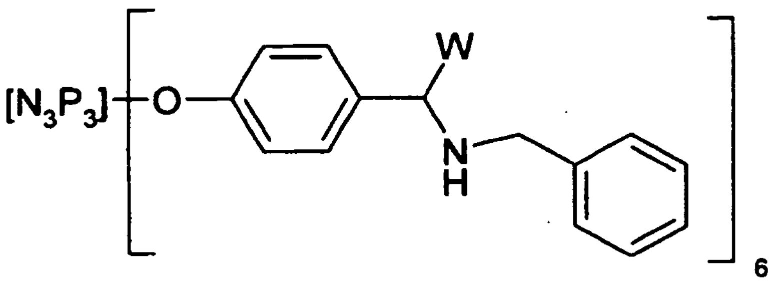

wobei W für $PO_3Me_2$ $PO_3HNa$ oder $PO_3H_2$ steht.

**62.** Bisphosphon-terminierte Dendrimere nach Anspruch 50 mit der folgenden Formel:

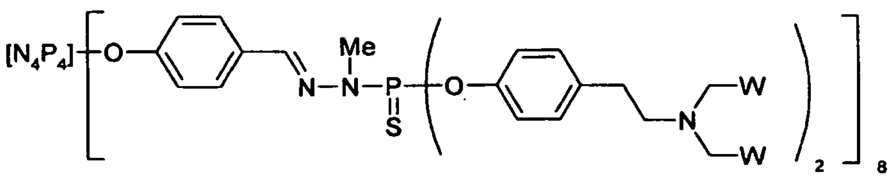

wobei W für $PO_3Me_2$, $PO_3HNa$ oder $PO_3H_2$ steht.

**63.** Bisphosphon-terminierte Dendrimere nach Anspruch 50 mit der folgenden Formel:

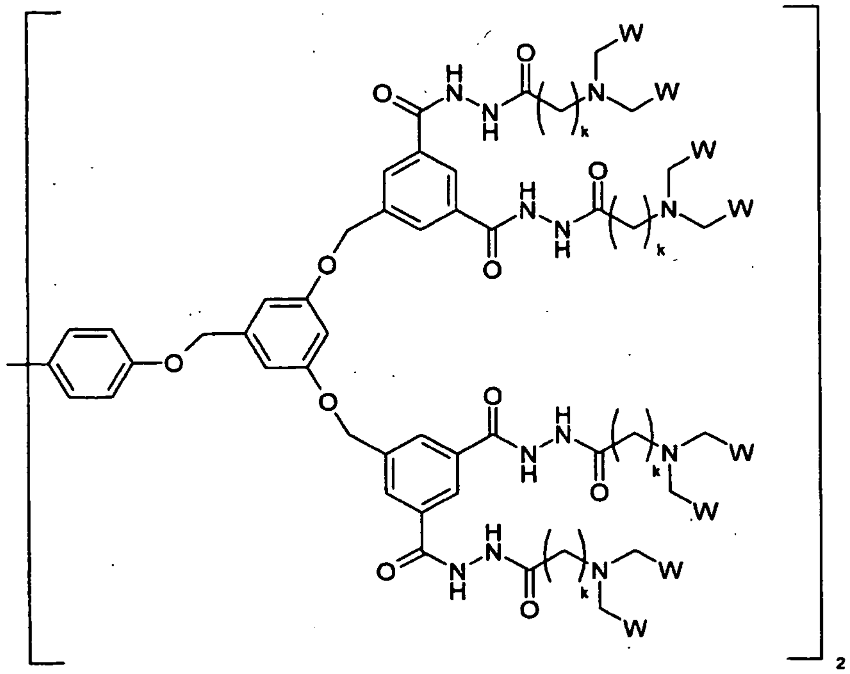

wobei W für $PO_3Me_2$, $PO_3HNa$ oder $PO_3H_2$ steht und k für 1, 2 oder 3 steht.

**64.** Bisphosphon-terminierte Dendrimere nach Anspruch 50 mit der folgenden Formel:

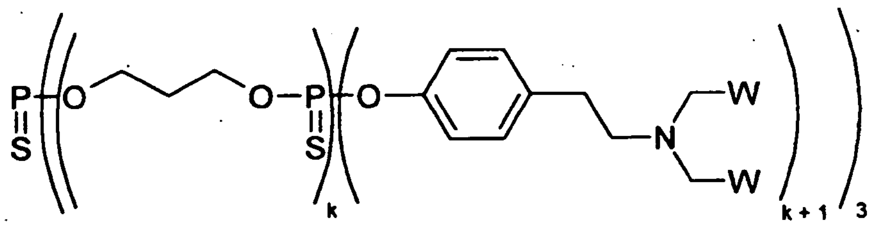

wobei W für $PO_3Me_2$, $PO_3HNa$ oder $PO_3H_2$ steht und k für 0 oder 1 steht.

**65.** Bisphosphon-terminierte Dendrimere mit der folgenden Formel:

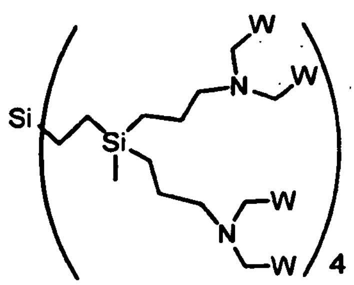

wobei W für $PO_3Me_2$, $PO_3HNa$ oder $PO_3H_2$ steht.

**66.** Bisphosphon-terminierte Dendrimere mit der folgenden Formel:

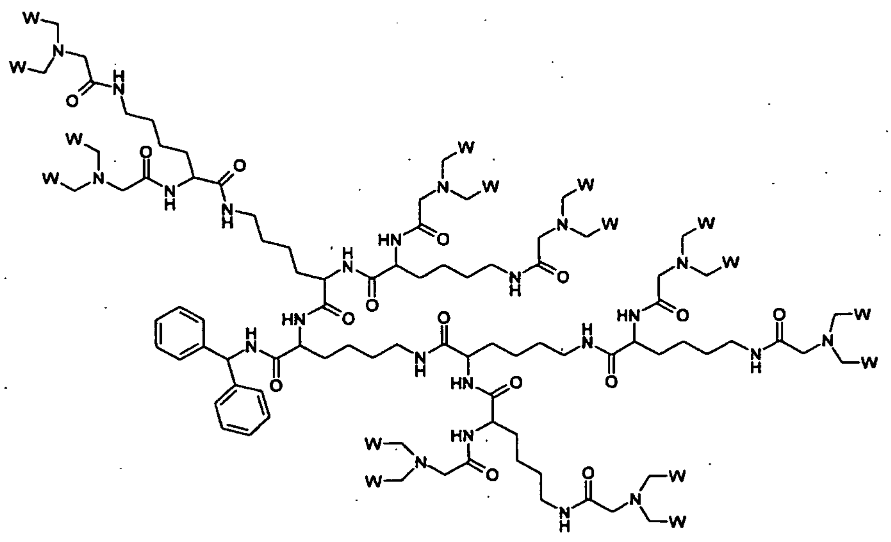

wobei W für $PO_3Me_2$, $PO_3HNa$ oder $PO_3H_2$ steht.

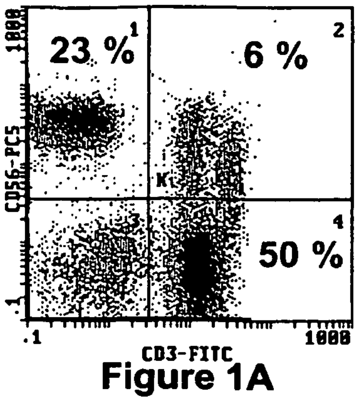


Figure 1A

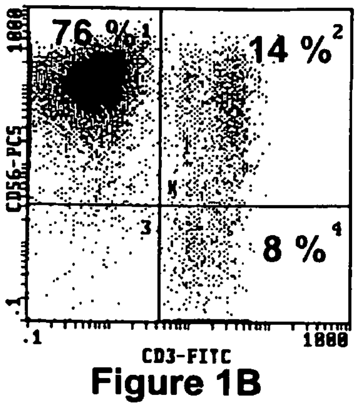


Figure 1B

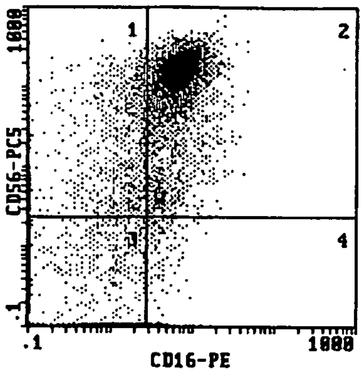


Figure 1C

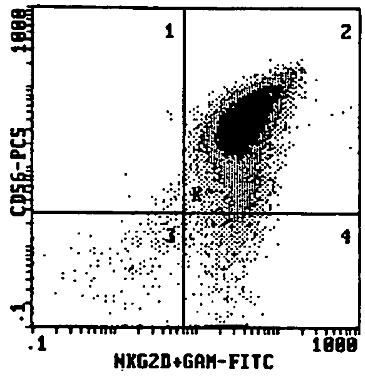


Figure 1D

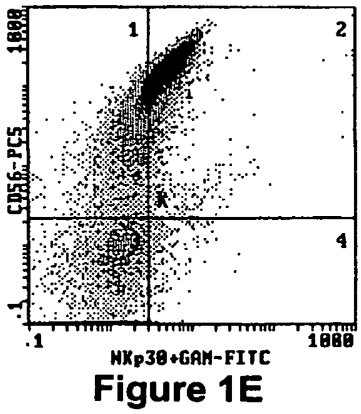


Figure 1E

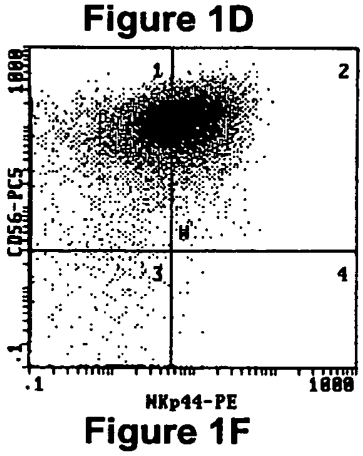


Figure 1F

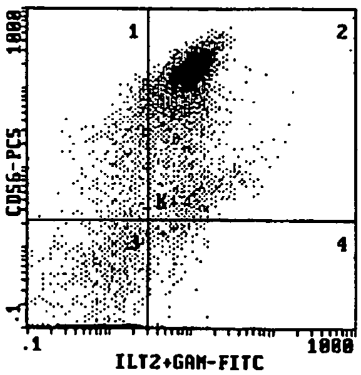


Figure 1G

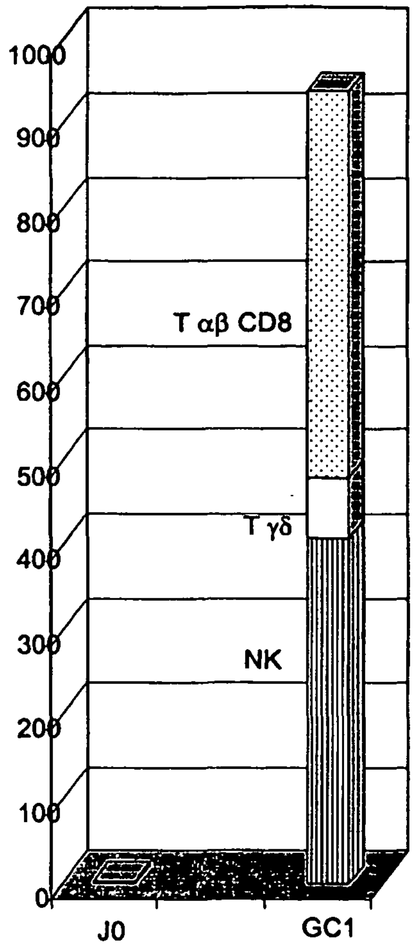


Figure 2A

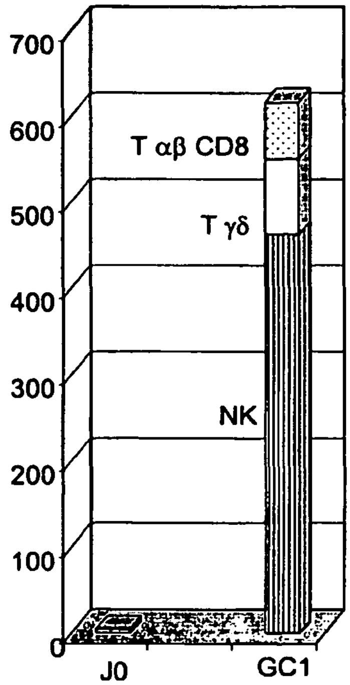


Figure 2B

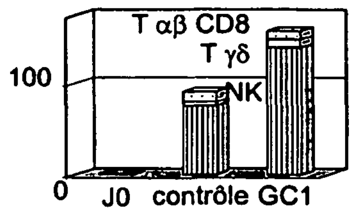


Figure 2C

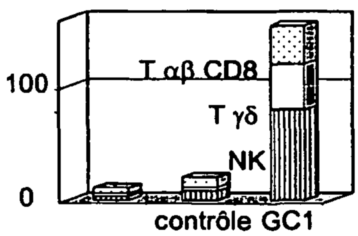


Figure 2D

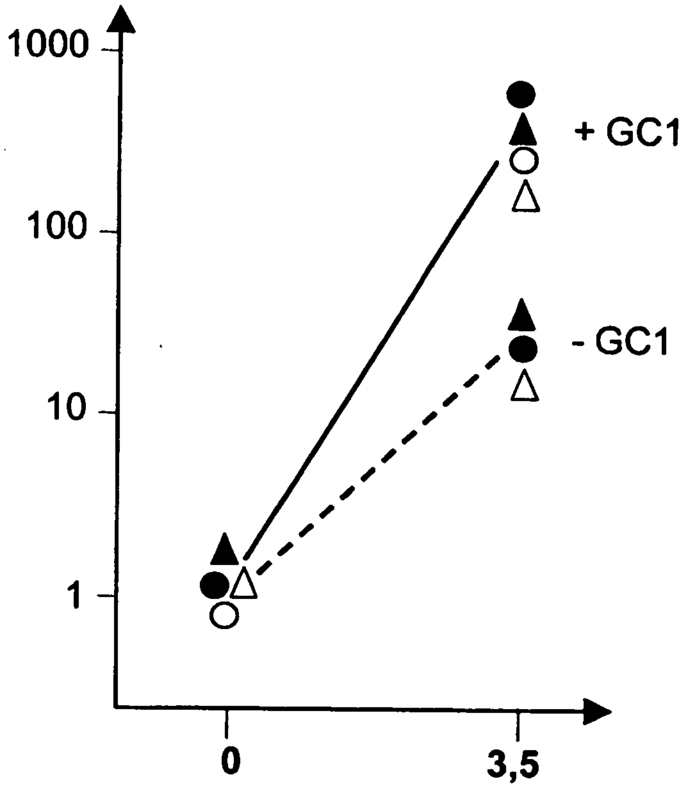


Figure 3

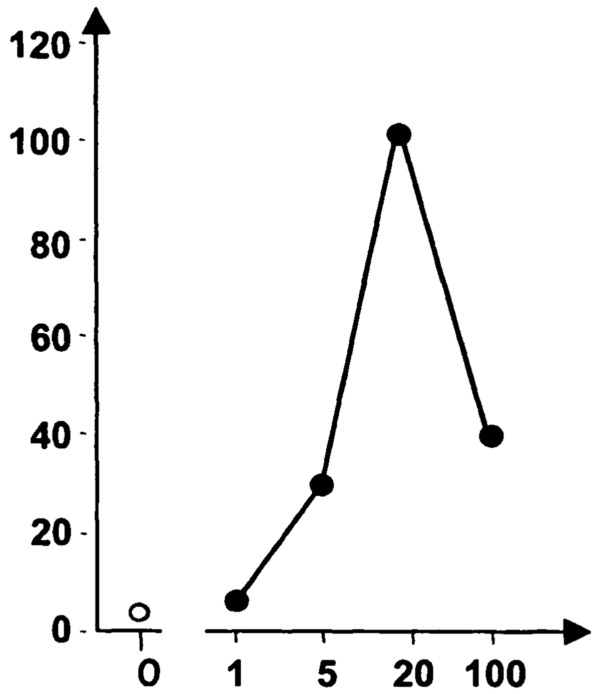


Figure 4A

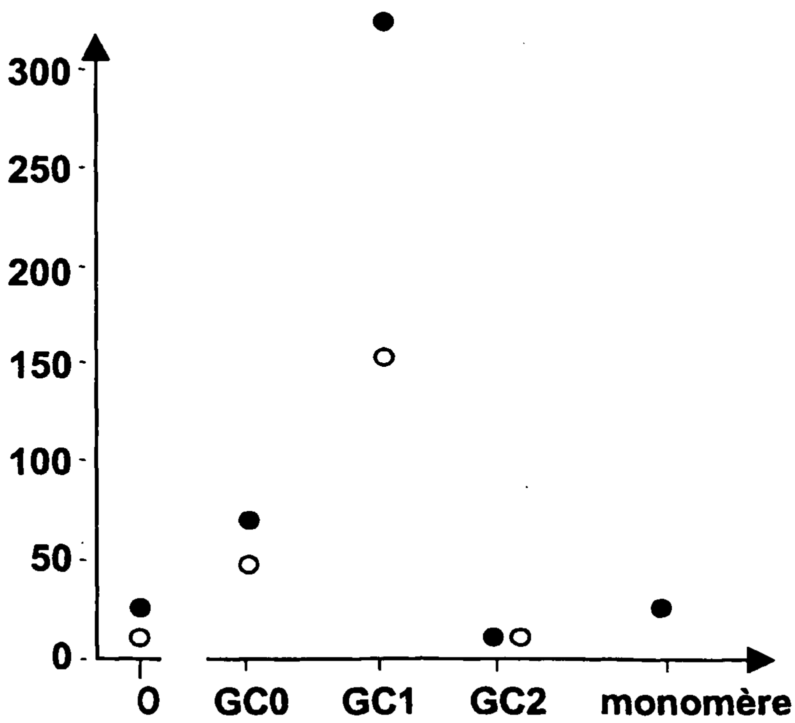


Figure 4B

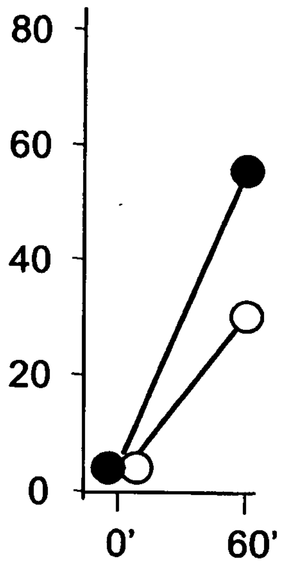


**Figure 5A**

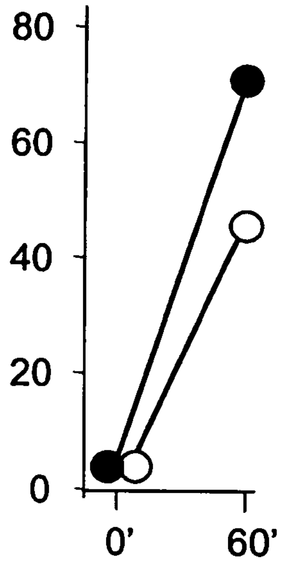


**Figure 5B**

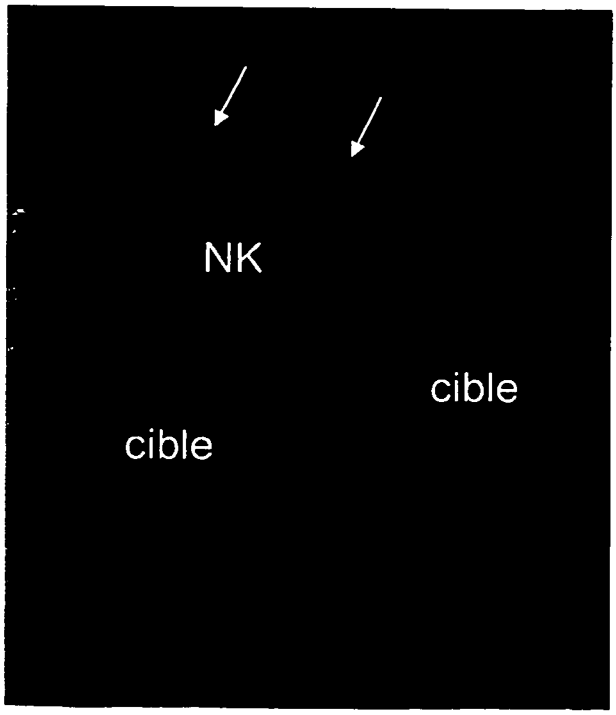


**Figure 5C**

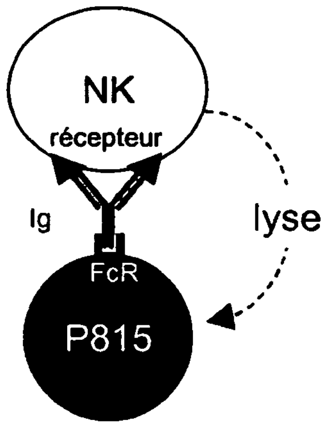


**Figure 6A**

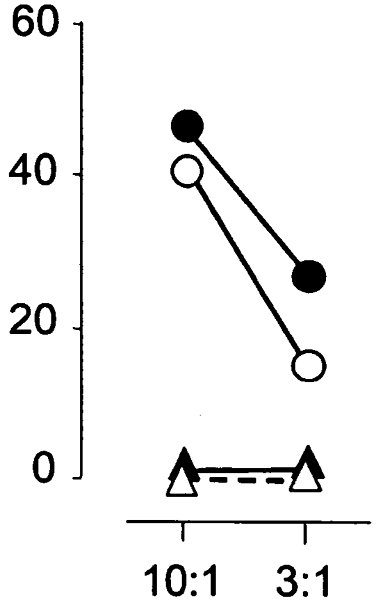


**Figure 6B**

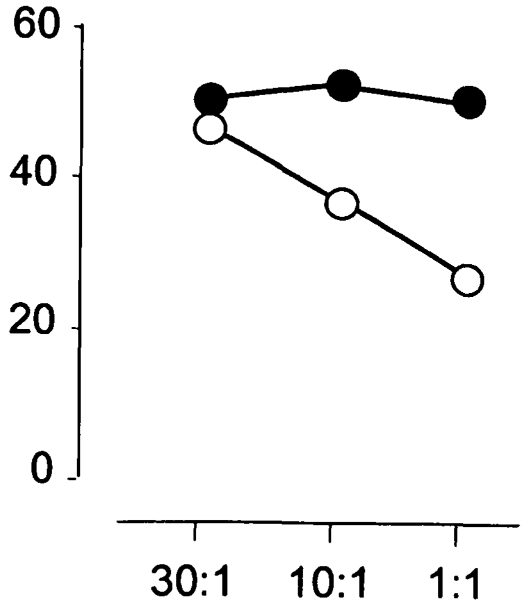


**Figure 7A**

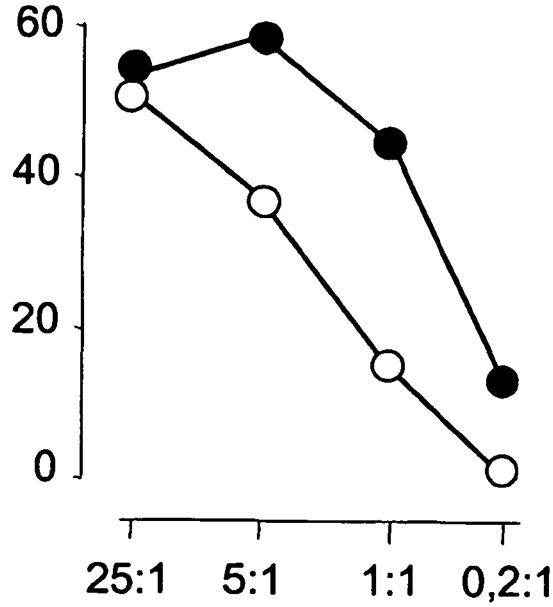


**Figure 7B**

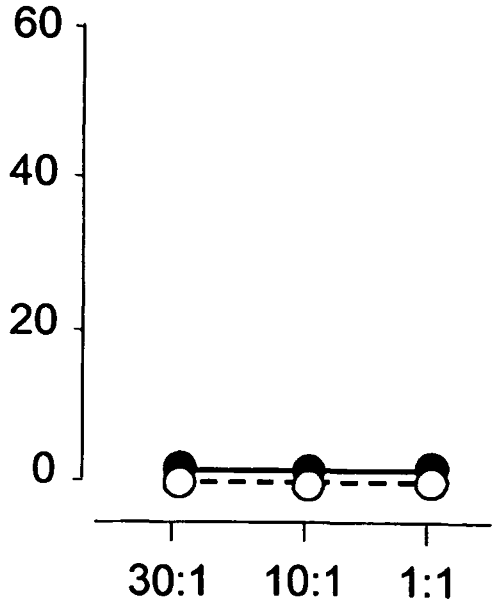


**Figure 7C**

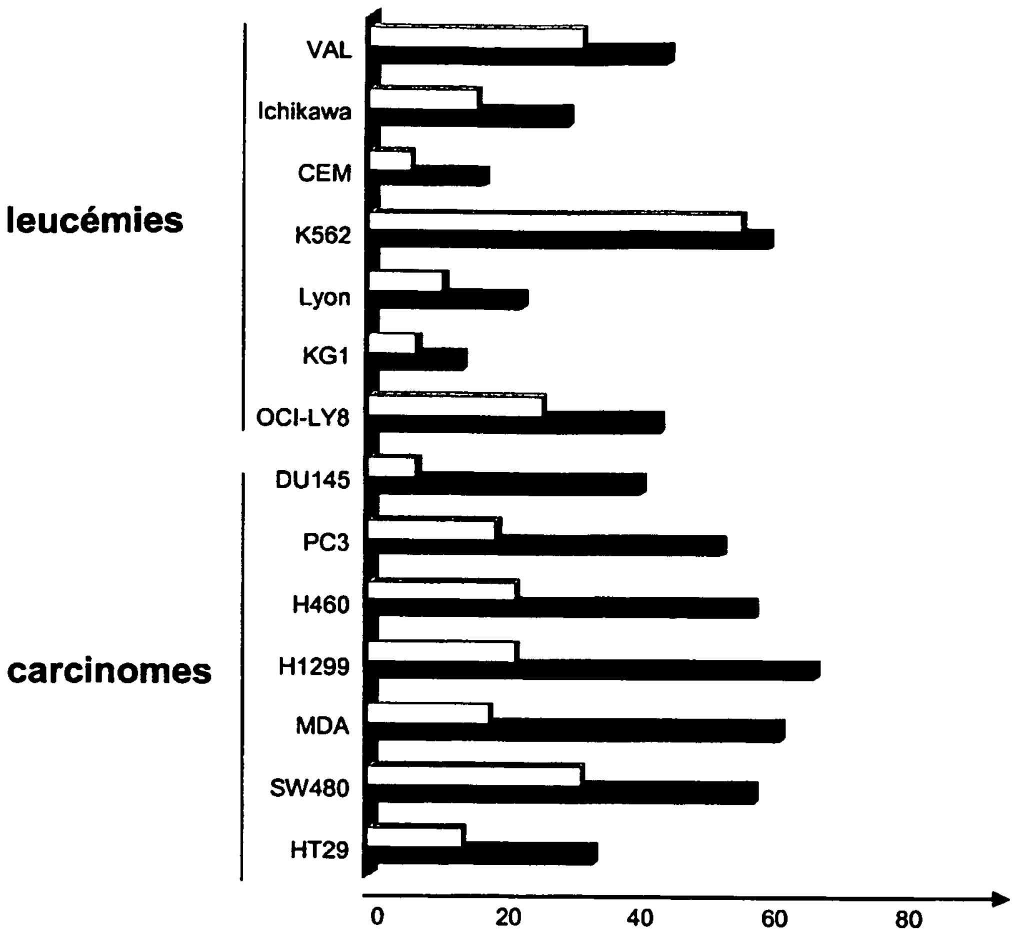


Figure 8

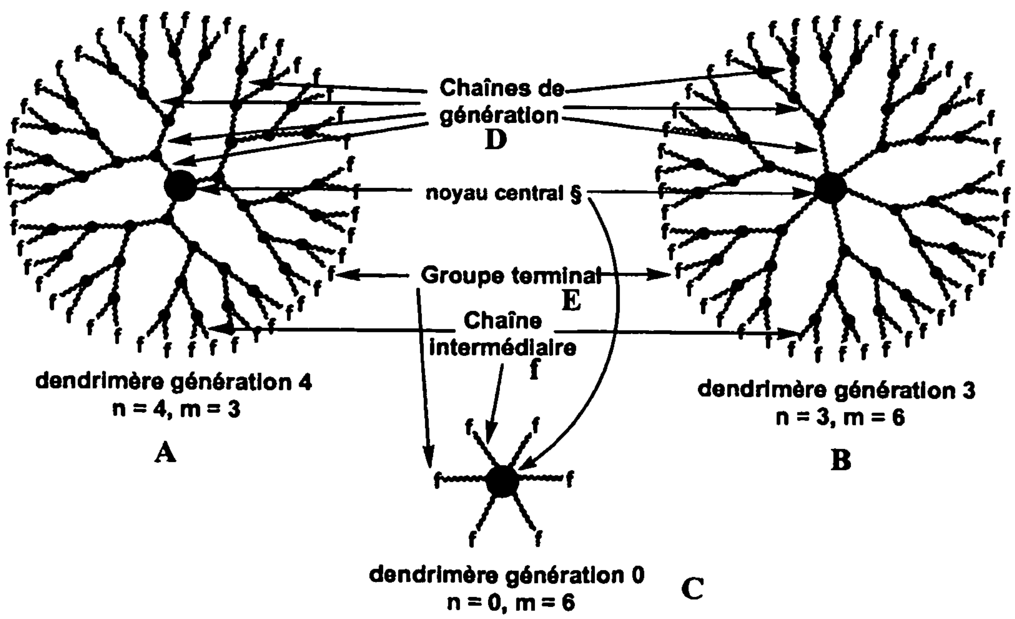


Figure 9

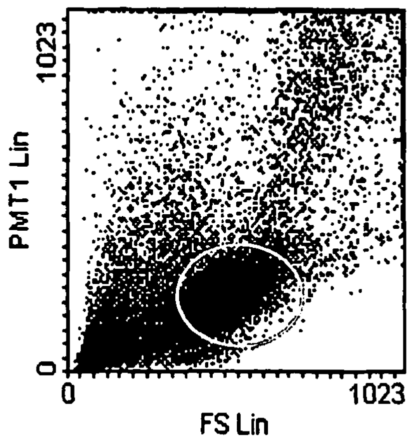


**Figure 10A**

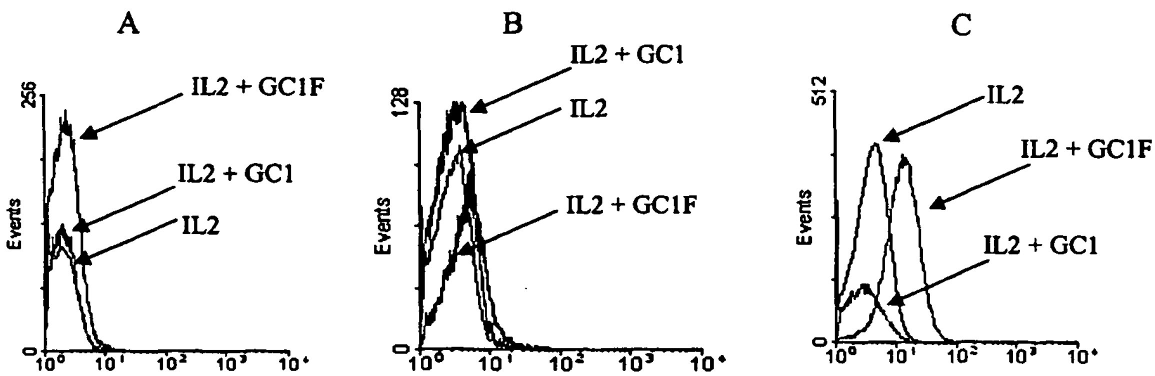


**Figure 10B**

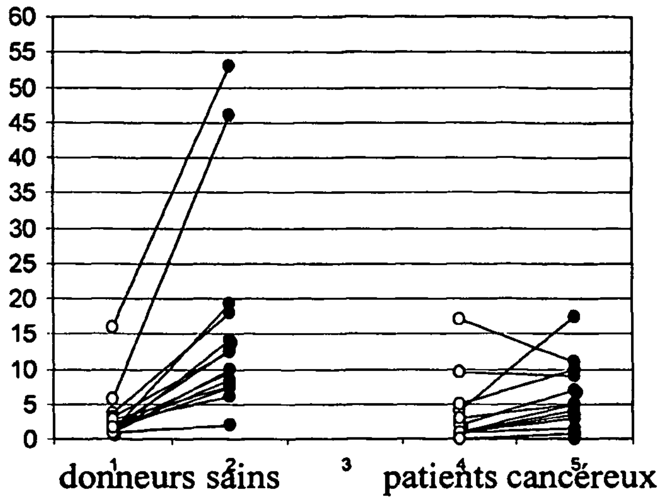


**Figure 11**

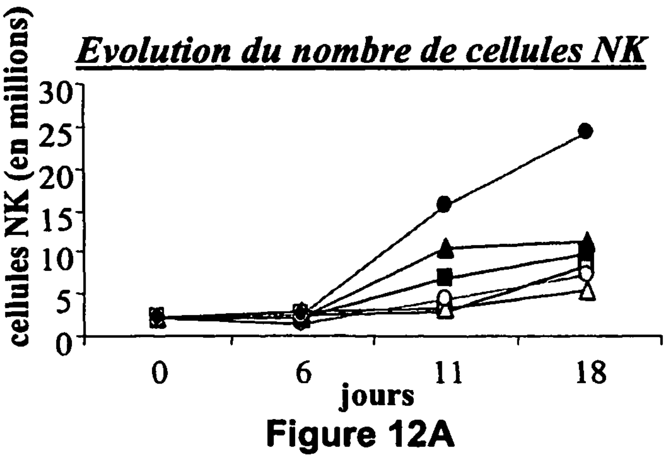


**Figure 12A**

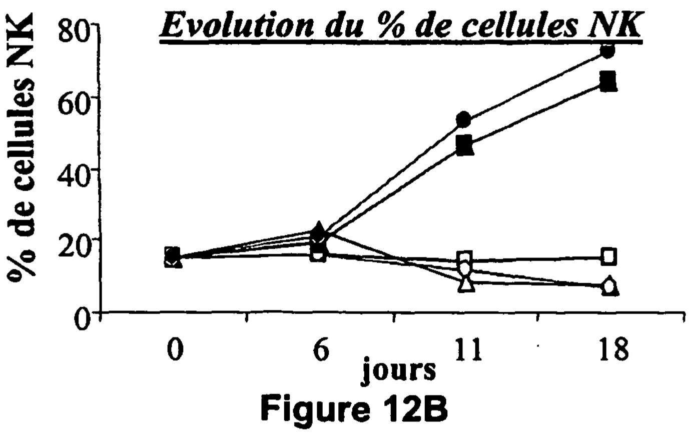


**Figure 12B**

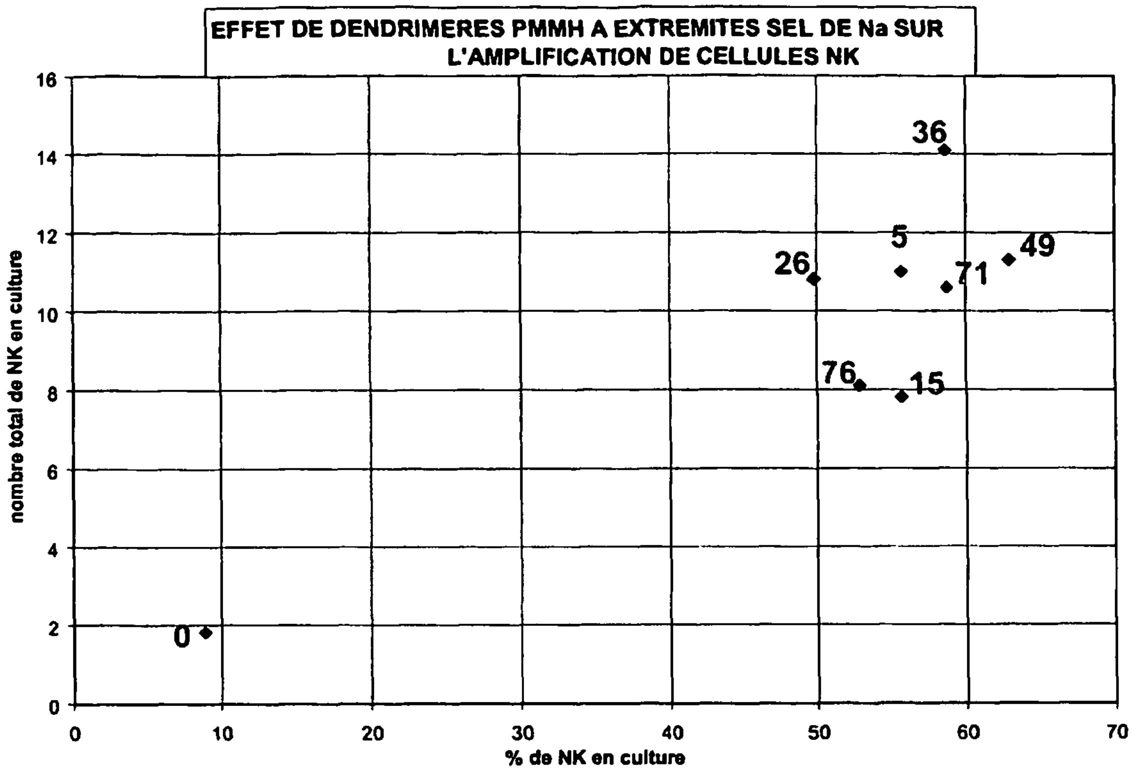
EFFET DE DENDRIMERES PMMH A EXTREMITES SEL DE Na SUR L'AMPLIFICATION DE CELLULES NK
nombre total de NK en culture
% de NK en culture
0
2
4
6
8
10
12
14
16
0
10
20
30
40
50
60
70
36
5
26
71
49
76
15
0

Figure 13

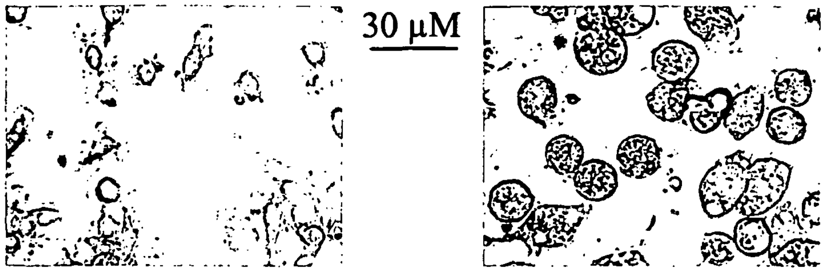


monocytes après 6 jours de culture
sans GC1 avecGC1

Figure 14A

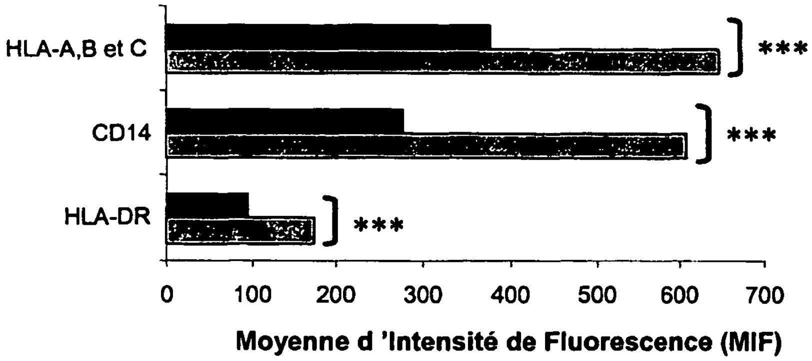


Figure 14B

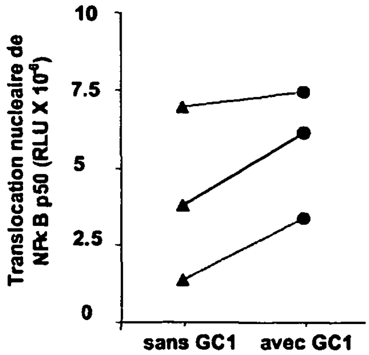


Figure 14C

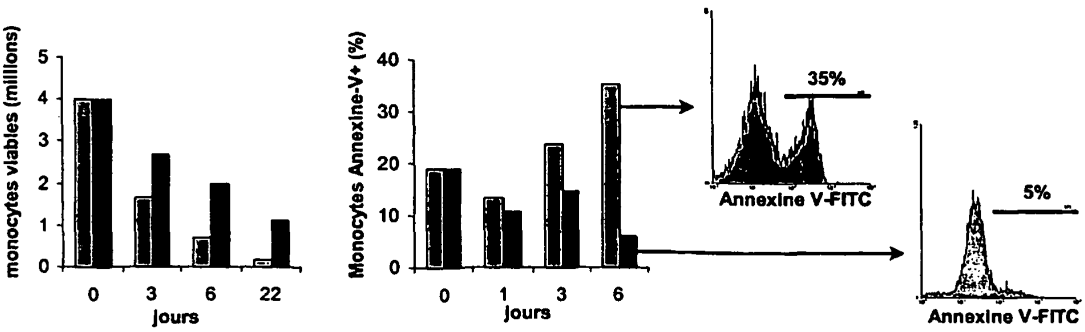


Figure 14D

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 02067508 A
- WO 0191816 A
- WO 0138335 A
- WO 030913304 A
- WO 20050052032 A
- WO 20050052031 A


### Littérature non-brevet citée dans la description


- **Launay et al.** *Angew. Chem. Int. Ed. Engl.,* 1994, vol. 33 (15/16), 1589-1592
- **Launay et al.** *Journal of Organometallic Chemistry,* 1997, vol. 529, 51-58
- **Vivier et al.** Immunologie des Cancers. 2003
- *J. Org. Chem.,* 1997, vol. 62 (14), 4834-4841
- **Muraki et al.** *Polymer Degradation and Stability,* 2004, vol. 84 (1), 87-93
- **S. Bauer et al.** *Science,* 1999, vol. 285, 727-729
- **J. Wu.** *Science,* 1999, vol. 285, 730-732
- **D.A. Tomalia ; H. Baker ; J. Dewald ; M. Hall ; G. Kallos ; S. Martin ; J. Roeck ; J. Ryder ; P.S. Smith.** *Polym. J. (Tokyo),* 1985, vol. 17, 117
- **D.A. Tomalia ; H. Baker ; J. Dewald ; M. Hall ; G. Kallos ; S. Martin ; J. Roeck ; J. Ryder ; P.S. Smith.** *Macromolecules,* 1986, vol. 19, 2466
- **E.M.M. de Brabander-van den Berg ; E.W. Meijer.** *Angew. Chem. Int. Ed. Engl.,* 1993, vol. 32, 1308
- **Launay N. ; Caminade A.M. ; Lahana R. ; Majoral J.P.** A general synthetic strategy for neutral phosphorus containing dendrimers. *Angew. Chem.,* 1994, vol. 106, 1682
- *Angew. Chem. Int. Ed. Engl.,* 1994, vol. 33, 1589
- **Launay N. ; Caminade A.M. ; Majoral J.P.** Synthesis of bowl-shaped dendrimers from generation 1 to generation 8. *J. Organomet. Chem.,* 1997, vol. 529, 51
- **V. Maraval et al.** *Angew. Chem. Int. Ed.,* 2003, vol. 42, 1822
- **Larock.** Comprehensive Organic Transformations. VCH Pub, 1989
- **T.W. Green ; P.G.M. Wuts.** Protective Groups in Organic Chemistry. John Wiley and Sons, 1991
- **J.F.W. McOmie.** Protective Groups in Organic Chemistry. Plenum Press, 1973
- *J. Org. Chem.,* 1997, vol. 62, 4834
- **B.A. Arbuzow.** *Pure appl. Chem.,* 1964, vol. 9, 307
- **Olszewski et al.** *J. Org. Chem.,* 1994, vol. 59, 4285-4296
- **I. Linzaga et al.** *Tetrahedron,* 2002, vol. 58, 8973-8978
- **Perrin et al.** Purification of Laboratory Chemicals. 1988
- **J.D. Olsjewski et al.** *J. Org. Chem.,* 1994, vol. 59, 4285-4296
- **D.A. Tomalia ; H. Baker ; J. Dewald ; M. Hall ; G. Kallos ; S. Martin ; J. Roeck ; J. Ryder ; P.S. Smith.** *Polym. J.,* 1985, vol. 17, 117
- **J.P. Majoral et al.** *Angew. Chem. Int. Ed. Engl.,* 1993, vol. 32, 1477
- *Inorg. Chem.,* 1994, vol. 33, 6351
- **M.A. Haidekk et al.** *Chemistry and Biology,* 2001, vol. 8, 123-131
- **R. Pelc et al.** *Phosphorus, Sulfur and Silicon,* 1990, vol. 47, 375-382
- *Tetrahedron Lett.,* 2000, vol. 41, 1643
- **G.M. Salamonczyk.** *Tetrahedron Lett.,* 2000, vol. 41, 1643
- **L.-L. Zhou ; J. Roovers.** *Macromolecules,* 1993, vol. 26, 963
- **D. Seyferth ; D. Y. Son.** *Organometallics,* 1994, vol. 13, 2682